(19) Europäisches Patentamt
European Patent Office
Office européen des brevets

(11) EP 2 067 472 A1

(12) EUROPEAN PATENT APPLICATION

(43) Date of publication:
**10.06.2009 Bulletin 2009/24**

(51) Int Cl.:
***A61K 31/00*** *(2006.01)* ***A61P 35/00*** *(2006.01)*
***A61K 47/48*** *(2006.01)*

(21) Application number: **08018727.1**

(22) Date of filing: **30.12.2002**

(84) Designated Contracting States:
**AT BE BG CH CY CZ DE DK EE ES FI FR GB GR HU IE IT LI LU MC NL PT RO SE SI SK TR**

(30) Priority:
**02.01.2002 US 345444 P**
**25.01.2002 US 351885 P**
**25.02.2002 US 360066 P**
**05.03.2002 US 362004 P**
**20.03.2002 US 366869 P**
**21.03.2002 US 366284 P**
**28.03.2002 US 368679 P**
**19.08.2002 US 404809 P**
**21.08.2002 US 405645 P**

(62) Document number(s) of the earlier application(s) in accordance with Art. 76 EPC:
**02806285.9 / 1 575 571**

(71) Applicant: **Genentech, Inc.**
**South San Francisco CA 94080-4990 (US)**

(72) Inventors:
- **Frantz, Gretchen**
**San Francisco CA 94127 (US)**
- **Hillan, Kenneth**
**San Francisco CA 94114 (US)**
- **Phillips, Heidi**
**Palo Alto CA 94303 (US)**
- **Polakis, Paul**
**Burlingame CA 97303 (US)**
- **Smith, Victoria**
**Burlingame CA 94010 (US)**
- **Spencer, Susan D.**
**Tiburon CA 94920 (US)**
- **Williams, P. Mickey**
**Emerald Hills, CA 94062 (US)**
- **Wu, Thomas D.**
**San Francisco, CA 94110 (US)**
- **Zhang, Zemin**
**Foster City CA 94404 (US)**

(74) Representative: **Sampson, Catherine et al**
**Mewburn Ellis LLP**
**33 Gutter Lane**
**London**
**EC2V 8AS (GB)**

Remarks:
This application was filed on 27-10-2008 as a divisional application to the application mentioned under INID code 62.

# (54) Compositions and methods for the diagnosis and treatment of tumor

(57) The present invention is directed to composition of matter useful for the diagnosis and treatment of tumor in mammals and to methods of using those compositions of matter for the same.

EP 2 067 472 A1

**Description**

FIELD OF THE INVENTION

The present invention is directed to compositions of matter useful for the diagnosis and treatment of tumor in mammals and to methods of using those compositions of matter for the same.

BACKGROUND OF THE INVENTION

Malignant tumors (cancers) are the second leading cause of death in the United States, after heart disease (Boring et al., CA Cancel J. Clin. 43:7 (1993)). Cancer is characterized by the increase in the number of abnormal, or neoplastic, cells derived from a normal tissue which proliferate to form a tumor mass, the invasion of adjacent tissues by these neoplastic tumor cells, and the generation of malignant cells which eventually spread via the blood or lymphatic system to regional lymph nodes and to distant sites via a process called metastasis. In a cancerous state, a cell proliferates under conditions in which normal cells would not grow. Cancer manifests itself in a wide variety of forms, characterized by different degrees of invasiveness and aggressiveness.

In attempts to discover effective cellular targets for cancer diagnosis and therapy, researchers have sought to identify transmembrane or otherwise membrane-associated polypeptides that are specifically expressed on the surface of one or more particular type(s) of cancer cell as compared to on one or more normal non-cancerous cell(s). Often, such membrane-associated polypeptides are more abundantly expressed on the surface of the cancer cells as compared to on the surface of the non-cancerous cells. The identification of such tumor-associated cell surface antigen polypeptides has given rise to the ability to specifically target cancer cells for destruction via antibody-based therapies. In this regard, it is noted that antibody-based therapy has proved very effective in the treatment of certain cancers. For example, HERCEPTIN® and RITUXAN® (both from Genentech Inc., South San Francisco, California) are antibodies that have been used successfully to treat breast cancer and non-Hodgkin's lymphoma, respectively. More specifically, HERCEPTIN® is a recombinant DNA-derived humanized monoclonal antibody that selectively binds to the extracellular domain of the human epidermal growth factor receptor 2 (HER2) proto-oncogene. HER2 protein overexpression is observed in 25-30% of primary breast cancers. RITUXAN® is a genetically engineered chimeric murine/human monoclonal antibody directed against the CD20 antigen found on the surface of normal and malignant B lymphocytes. Both these antibodies are recombinantly produced in CHO cells.

In other attempts to discover effective cellular targets for cancer diagnosis and therapy, researchers have sought to identify (1) non-membrane-associated polypeptides that are specifically produced by one or more particular type(s) of cancer cell(s) as compared to by one or more particular type(s) of non-cancerous normal cell(s), (2) polypeptides that are produced by cancer cells at an expression level that is significantly higher than that of one or more normal non-cancerous cell(s), or (3) polypeptides whose expression is specifically limited to only a single (or very limited number of different) tissue type(s) in both the cancerous and non-cancerous state (e.g., normal prostate and prostate tumor tissue). Such polypeptides may remain intracellularly located or may be secreted by the cancer cell. Moreover, such polypeptides may be expressed not by the cancer cell itself, but rather by cells which produce and/or secrete polypeptides having a potentiating or growth-enhancing effect on cancer cells. Such secreted polypeptides are often proteins that provide cancer cells with a growth advantage over normal cells and include such things as, for example, angiogenic factors, cellular adhesion factors, growth factors, and the like. Identification of antagonists of such non-membrane associated polypeptides would be expected to serve as effective therapeutic agents for the treatment of such cancers. Furthermore, identification of the expression pattern of such polypeptides would be useful for the diagnosis of particular cancers in mammals.

Despite the above identified advances in mammalian cancer therapy, there is a great need for additional diagnostic and therapeutic agents capable of detecting the presence of tumor in a mammal and for effectively inhibiting neoplastic cell growth, respectively. Accordingly, it is an objective of the present invention to identify: (1) cell membrane-associated polypeptides that are more abundantly expressed on one or more type(s) of cancer cell(*s*) as compared to on normal cells or on other different cancer cells, (2) non-membrane-associated polypeptides that are specifically produced by one or more particular type(s) of cancer cell(s) (or by other cells that produce polypeptides having a potentiating effect on the growth of cancer cells) as compared to by one or more particular type(s) of non-cancerous normal cell(s), (3) non-membrane-associated polypeptides that are produced by cancer cells at an expression level that is significantly higher than that of one or more normal non-cancerous cell(s), or (4) polypeptides whose expression is specifically limited to only a single (or very limited number of different) tissue type(s) in both a cancerous and non-cancerous state (e.g., normal prostate and prostate tumor tissue), and to use those polypeptides, and their encoding nucleic acids, to produce compositions of matter useful in the therapeutic treatment and diagnostic detection of cancer in mammals. It is also an objective of the present invention to identify cell membrane-associated, secreted or intracellular polypeptides whose expression is limited to a single or very limited number of tissues, and to use those polypeptides, and their encoding nucleic acids, to produce compositions of matter useful in the therapeutic treatment and diagnostic detection of cancer in mammals.

## SUMMARY OF THE INVENTION

### A. Embodiments

In the present specification, Applicants describe for the first time the identification of various cellular polypeptides (and their encoding nucleic acids or fragments thereof) which are expressed to a greater degree on the surface of or by one or more types of cancer cell(s) as compared to on the surface of or by one or more types of normal non-cancer cells. Alternatively, such polypeptides are expressed by cells which produce and/or secrete polypeptides having a potentiating or growth-enhancing effect on cancer cells. Again alternatively, such polypeptides may not be overexpressed by tumor cells as compared to normal cells of the same tissue type, but rather may be specifically expressed by both tumor cells and normal cells of only a single or very limited number of tissue types (preferably tissues which are not essential for life, e.g., prostate, etc.). All of the above polypeptides are herein referred to as Tumor-associated Antigenic Target polypeptides ("TAT" polypeptides) and are expected to serve as effective targets for cancer therapy and diagnosis in mammals.

Accordingly, in one embodiment of the present invention, the invention provides an isolated nucleic acid molecule having a nucleotide sequence that encodes a tumor-associated antigenic target polypeptide or fragment thereof (a "TAT" polypeptide).

In certain aspects, the isolated nucleic acid molecule comprises a nucleotide sequence having at least about 80% nucleic acid sequence identity, alternatively at least about 81%, 82%, 83%, 84%, 85%, 86%, 87%, 88%, 89%, 90%, 91%, 92%, 93%, 94%, 95%, 96%, 97%, 98%, 99% or 100% nucleic acid sequence identity, to (a) a DNA molecule encoding a full-length TAT polypeptide having an amino acid sequence as disclosed herein, a TAT polypeptide amino acid sequence lacking the signal peptide as disclosed herein, an extracellular domain of a transmembrane TAT polypeptide, with or without the signal peptide, as disclosed herein or any other specifically defined fragment of a full-length TAT polypeptide amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81%, 82%, 83%, 84%, 85%, 86%, 87%, 88%, 89%, 90%, 91%, 92%, 93%, 94%, 95%, 96%, 97%, 98%, 99% or 100% nucleic acid sequence identity, to (a) a DNA molecule comprising the coding sequence of a full-length TAT polypeptide cDNA as disclosed herein, the coding sequence of a TAT polypeptide lacking the signal peptide as disclosed herein, the coding sequence of an extracellular domain of a transmembrane TAT polypeptide, with or without the signal peptide, as disclosed herein or the coding sequence of any other specifically defined fragment of the full-length TAT polypeptide amino acid sequence as disclosed herein, or (b) the complement of the DNA molecule of (a).

In further aspects, the invention concerns an isolated nucleic acid molecule comprising a nucleotide sequence having at least about 80% nucleic acid sequence identity, alternatively at least about 81%, 82%, 83%, 84%, 85%, 86%, 87%, 88%, 89%, 90%, 91%, 92%, 93%, 94%, 95%, 96%, 97%, 98%, 99% or 100% nucleic acid sequence identity, to (a) a DNA molecule that encodes the same mature polypeptide encoded by the full-length coding region of any of the human protein cDNAs deposited with the ATCC as disclosed herein, or (b) the complement of the DNA molecule of (a).

Another aspect of the invention provides an isolated nucleic acid molecule comprising a nucleotide sequence encoding a TAT polypeptide which is either transmembrane domain-deleted or transmembrane domain-inactivated, or is complementary to such encoding nucleotide sequence, wherein the transmembrane domain(s) of such polypeptide(s) are disclosed herein. Therefore, soluble extracellular domains of the herein described TAT polypeptides are contemplated.

In other aspects, the present invention is directed to isolated nucleic acid molecules which hybridize to (a) a nucleotide sequence encoding a TAT polypeptide having a full-length amino acid sequence as disclosed herein, a TAT polypeptide amino acid sequence lacking the signal peptide as disclosed herein, an extracellular domain of a transmembrane TAT polypeptide, with or without the signal peptide, as disclosed herein or any other specifically defined fragment of a full-length TAT polypeptide amino acid sequence as disclosed herein, or (b) the complement of the nucleotide sequence of (a). In this regard, an embodiment of the present invention is directed to fragments of a full-length TAT polypeptide coding sequence, or the complement thereof, as disclosed herein, that may find use as, for example, hybridization probes useful as, for example, diagnostic probes, antisense oligonucleotide probes, or for encoding fragments of a full-length TAT polypeptide that may optionally encode a polypeptide comprising a binding site for an anti-TAT polypeptide antibody, a TAT binding oligopeptide or other small organic molecule that binds to a TAT polypeptide. Such nucleic acid fragments are usually at least about 5 nucleotides in length, alternatively at least about 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510,

520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides in length, wherein in this context the term "about" means the referenced nucleotide sequence length plus or minus 10% of that referenced length. It is noted that novel fragments of a TAT polypeptide-encoding nucleotide sequence may be determined in a routine manner by aligning the TAT polypeptide-encoding nucleotide sequence with other known nucleotide sequences using any of a number of well known sequence alignment programs and determining which TAT polypeptide-encoding nucleotide sequence fragment(*s*) are novel. All of such novel fragments of TAT polypeptide-encoding nucleotide sequences are contemplated herein. Also contemplated are the TAT polypeptide fragments encoded by these nucleotide molecule fragments, preferably those TAT polypeptide fragments that comprise a binding site for an anti-TAT antibody, a TAT binding oligopeptide or other small organic molecule that binds to a TAT polypeptide.

In another embodiment, the invention provides isolated TAT polypeptides encoded by any of the isolated nucleic acid sequences hereinabove identified

In a certain aspect, the invention concerns an isolated TAT polypeptide, comprising an amino acid sequence having at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, 99% or 100% amino acid sequence identity, to a TAT polypeptide having a full-length amino acid sequence as disclosed herein, a TAT polypeptide amino acid sequence lacking the signal peptide as disclosed herein, an extracellular domain of a transmembrane TAT polypeptide protein, with or without the signal peptide, as disclosed herein, an amino acid sequence encoded by any of the nucleic acid sequences disclosed herein or any other specifically defined fragment of a full-length TAT polypeptide amino acid sequence as disclosed herein.

In a further aspect, the invention concerns an isolated TAT polypeptide comprising an amino acid sequence having at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% amino acid sequence identity, to an amino acid sequence encoded by any of the human protein cDNAs deposited with the ATCC as disclosed herein.

In a specific aspect, the invention provides an isolated TAT polypeptide without the N-terminal signal sequence and/or without the initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule under conditions suitable for expression of the TAT polypeptide and recovering the TAT polypeptide from the cell culture.

Another aspect of the invention provides an isolated TAT polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the TAT polypeptide and recovering the TAT polypeptide from the cell culture.

In other embodiments of the present invention, the invention provides vectors comprising DNA encoding any of the herein described polypeptides. Host cells comprising any such vector are also provided. By way of example, the host cells may be CHO cells, E. *coli* cells, or yeast cells. A process for producing any of the herein described polypeptides is further provided and comprises culturing host cells under conditions suitable for expression of the desired polypeptide and recovering the desired polypeptide from the cell culture.

In other embodiments, the invention provides isolated chimeric polypeptides comprising any of the herein described TAT polypeptides fused to a heterologous (non-TAT) polypeptide. Example of such chimeric molecules comprise any of the herein described TAT polypeptides fused to a heterologous polypeptide such as, for example, an epitope tag sequence or a Fc region of an immunoglobulin.

In another embodiment, the invention provides an antibody which binds, preferably specifically, to any of the above or below described polypeptides. Optionally, the antibody is a monoclonal antibody, antibody fragment, chimeric antibody, humanized antibody, single-chain antibody or antibody that competitively inhibits the binding of an anti-TAT polypeptide antibody to its respective antigenic epitope. Antibodies of the present invention may optionally be conjugated to a growth inhibitory agent or cytotoxic agent such as a toxin, including, for example, a maytansinoid or calicheamicin, an antibiotic, a radioactive isotope, a nucleolytic enzyme, or the like. The antibodies of the present invention may optionally be produced in CHO cells or bacterial cells and preferably induce death of a cell to which they bind. For diagnostic purposes, the antibodies of the present invention may be detectably labeled, attached to a solid support, or the like.

In other embodiments of the present invention, the invention provides vectors comprising DNA encoding any of the herein described antibodies. Host cell comprising any such vector are also provided. By way of example, the host cells may be CHO cells, E. *coli* cells, or yeast cells. A process for producing any of the herein described antibodies is further provided and comprises culturing host cells under conditions suitable for expression of the desired antibody and recovering the desired antibody from the cell culture.

In another embodiment, the invention provides oligopeptides ("TAT binding oligopeptides") which bind, preferably specifically, to any of the above or below described TAT polypeptides. Optionally, the TAT binding oligopeptides of the present invention may be conjugated to a growth inhibitory agent or cytotoxic agent such as a toxin, including, for example, a maytansinoid or calicheamicin, an antibiotic, a radioactive isotope, a nucleolytic enzyme, or the like. The TAT binding oligopeptides of the present invention may optionally be produced in CHO cells or bacterial cells and preferably induce death of a cell to which they bind. For diagnostic purposes, the TAT binding oligopeptides of the present invention may be detectably labeled, attached to a solid support, or the like.

In other embodiments of the present invention, the invention provides vectors comprising DNA encoding any of the herein described TAT binding oligopeptides. Host cell comprising any such vector are also provided. By way of example, the host cells may be CHO cells, E. *coli* cells, or yeast cells. A process for producing any of the herein described TAT binding oligopeptides is further provided and comprises culturing host cells under conditions suitable for expression of the desired oligopeptide and recovering the desired oligopeptide from the cell culture.

In another embodiment, the invention provides small organic molecules ("TAT binding organic molecules") which bind, preferably specifically, to any of the above or below described TAT polypeptides. Optionally, the TAT binding organic molecules of the present invention may be conjugated to a growth inhibitory agent or cytotoxic agent such as a toxin, including, for example, a maytansinoid or calicheamicin, an antibiotic, a radioactive isotope, a nucleolytic enzyme, or the like. The TAT binding organic molecules of the present invention preferably induce death of a cell to which they bind. For diagnostic purposes, the TAT binding organic molecules of the present invention may be detectably labeled, attached to a solid support, or the like.

In a still further embodiment, the invention concerns a composition of matter comprising a TAT polypeptide as described herein, a chimeric TAT polypeptide as described herein, an anti-TAT antibody as described herein, a TAT binding oligopeptide as described herein, or a TAT binding organic molecule as described herein, in combination with a carrier. Optionally, the carrier is a pharmaceutically acceptable carrier.

In yet another embodiment, the invention concerns an article of manufacture comprising a container and a composition of matter contained within the container, wherein the composition of matter may comprise a TAT polypeptide as described herein, a chimeric TAT polypeptide as described herein, an anti-TAT antibody as described herein, a TAT binding oligopeptide as described herein, or a TAT binding organic molecule as described herein. The article may further optionally comprise a label affixed to the container, or a package insert included with the container, that refers to the use of the composition of matter for the therapeutic treatment or diagnostic detection of a tumor.

Another embodiment of the present invention is directed to the use of a TAT polypeptide as described herein, a chimeric TAT polypeptide as described herein, an anti-TAT polypeptide antibody as described herein, a TAT binding oligopeptide as described herein, or a TAT binding organic molecule as described herein, for the preparation of a medicament useful in the treatment of a condition which is responsive to the TAT polypeptide, chimeric TAT polypeptide, anti-TAT polypeptide antibody, TAT binding oligopeptide, or TAT binding organic molecule.

B. Additional Embodiments

Another embodiment of the present invention is directed to a method for inhibiting the growth of a cell that expresses a TAT polypeptide, wherein the method comprises contacting the cell with an antibody, an oligopeptide or a small organic molecule that binds to the TAT polypeptide, and wherein the binding of the antibody, oligopeptide or organic molecule to the TAT polypeptide causes inhibition of the growth of the cell expressing the TAT polypeptide. In preferred embodiments, the cell is a cancer cell and binding of the antibody, oligopeptide or organic molecule to the TAT polypeptide causes death of the cell expressing the TAT polypeptide. Optionally, the antibody is a monoclonal antibody, antibody fragment, chimeric antibody, humanized antibody, or single-chain antibody. Antibodies, TAT binding oligopeptides and TAT binding organic molecules employed in the methods of the present invention may optionally be conjugated to a growth inhibitory agent or cytotoxic agent such as a toxin, including, for example, a maytansinoid or calicheamicin, an antibiotic, a radioactive isotope, a nucleolytic enzyme, or the like. The antibodies and TAT binding oligopeptides employed in the methods of the present invention may optionally be produced in CHO cells or bacterial cells.

Yet another embodiment of the present invention is directed to a method of therapeutically treating a mammal having a cancerous tumor comprising cells that express a TAT polypeptide, wherein the method comprises administering to the mammal a therapeutically effective amount of an antibody, an oligopeptide or a small organic molecule that binds to the TAT polypeptide, thereby resulting in the effective therapeutic treatment of the tumor. Optionally, the antibody is a monoclonal antibody, antibody fragment, chimeric antibody, humanized antibody, or single-chain antibody. Antibodies, TAT binding oligopeptides and TAT binding organic molecules employed in the methods of the present invention may optionally be conjugated to a growth inhibitory agent or cytotoxic agent such as a toxin, including, for example, a maytansinoid or calicheamicin, an antibiotic, a radioactive isotope, a nucleolytic enzyme, or the like. The antibodies and oligopeptides employed in the methods of the present invention may optionally be produced in CHO cells or bacterial cells.

Yet another embodiment of the present invention is directed to a method of determining the presence of a TAT polypeptide in a sample suspected of containing the TAT polypeptide, wherein the method comprises exposing the sample to an antibody, oligopeptide or small organic molecule that binds to the TAT polypeptide and determining binding of the antibody, oligopeptide or organic molecule to the TAT polypeptide in the sample, wherein the presence of such binding is indicative of the presence of the TAT polypeptide in the sample. Optionally, the sample may contain cells (which may be cancer cells) suspected of expressing the TAT polypeptide. The antibody, TAT binding oligopeptide or TAT binding organic molecule employed in the method may optionally be detectably labeled, attached to a solid support, or the like.

A further embodiment of the present invention is directed to a method of diagnosing the presence of a tumor in a mammal, wherein the method comprises detecting the level of expression of a gene encoding a TAT polypeptide (a) in a test sample of tissue cells obtained from said mammal, and (b) in a control sample of known normal non-cancerous cells of the same tissue origin or type, wherein a higher level of expression of the TAT polypeptide in the test sample, as compared to the control sample, is indicative of the presence of tumor in the mammal from which the test sample was obtained.

Another embodiment of the present invention is directed to a method of diagnosing the presence of a tumor in a mammal, wherein the method comprises (a) contacting a test sample comprising tissue cells obtained from the mammal with an antibody, oligopeptide or small organic molecule that binds to a TAT polypeptide and (b) detecting the formation of a complex between the antibody, oligopeptide or small organic molecule and the TAT polypeptide in the test sample, wherein the formation of a complex is indicative of the presence of a tumor in the mammal. Optionally, the antibody, TAT binding oligopeptide or TAT binding organic molecule employed is detectably labeled, attached to a solid support, or the like, and/or the test sample of tissue cells is obtained from an individual suspected of having a cancerous tumor.

Yet another embodiment of the present invention is directed to a method for treating or preventing a cell proliferative disorder associated with altered, preferably increased, expression or activity of a TAT polypeptide, the method comprising administering to a subject in need of such treatment an effective amount of an antagonist of a TAT polypeptide. Preferably, the cell proliferative disorder is cancer and the antagonist of the TAT polypeptide is an anti-TAT polypeptide antibody, TAT binding oligopeptide, TAT binding organic molecule or antisense oligonucleotide. Effective treatment or prevention of the cell proliferative disorder may be a result of direct killing or growth inhibition of cells that express a TAT polypeptide or by antagonizing the cell growth potentiating activity of a TAT polypeptide.

Yet another embodiment of the present invention is directed to a method of binding an antibody, oligopeptide or small organic molecule to a cell that expresses a TAT polypeptide, wherein the method comprises contacting a cell that expresses a TAT polypeptide with said antibody, oligopeptide or small organic molecule under conditions which are suitable for binding of the antibody, oligopeptide or small organic molecule to said TAT polypeptide and allowing binding therebetween.

Other embodiments of the present invention are directed to the use of (a) a TAT polypeptide, (b) a nucleic acid encoding a TAT polypeptide or a vector or host cell comprising that nucleic acid, (c) an anti-TAT polypeptide antibody, (d) a TAT-binding oligopeptide, or (e) a TAT-binding small organic molecule in the preparation of a medicament useful for (i) the therapeutic treatment or diagnostic detection of a cancer or tumor, or (ii) the therapeutic treatment or prevention of a cell proliferative disorder.

Another embodiment of the present invention is directed to a method for inhibiting the growth of a cancer cell, wherein the growth of said cancer cell is at least in part dependent upon the growth potentiating effect(s) of a TAT polypeptide (wherein the TAT polypeptide may be expressed either by the cancer cell itself or a cell that produces polypeptide(s) that have a growth potentiating effect on cancer cells), wherein the method comprises contacting the TAT polypeptide with an antibody, an oligopeptide or a small organic molecule that binds to the TAT polypeptide, thereby antagonizing the growth-potentiating activity of the TAT polypeptide and, in turn, inhibiting the growth of the cancer cell. Preferably the growth of the cancer cell is completely inhibited. Even more preferably, binding of the antibody, oligopeptide or small organic molecule to the TAT polypeptide induces the death of the cancer cell. Optionally, the antibody is a monoclonal antibody, antibody fragment, chimeric antibody, humanized antibody, or single-chain antibody. Antibodies, TAT binding oligopeptides and TAT binding organic molecules employed in the methods of the present invention may optionally be conjugated to a growth inhibitory agent or cytotoxic agent such as a toxin, including, for example, a maytansinoid or calicheamicin, an antibiotics, a radioactive isotope, a nucleolytic enzyme, or the like. The antibodies and TAT binding oligopeptides employed in the methods of the present invention may optionally be produced in CHO cells or bacterial cells.

Yet another embodiment of the present invention is directed to a method of therapeutically treating a tumor in a mammal, wherein the growth of said tumor is at least in part dependent upon the growth potentiating effect(*s*) of a TAT polypeptide, wherein the method comprises administering to the mammal a therapeutically effective amount of an antibody, an oligopeptide or a small organic molecule that binds to the TAT polypeptide, thereby antagonizing the growth potentiating activity of said TAT polypeptide and resulting in the effective therapeutic treatment of the tumor. Optionally, the antibody is a monoclonal antibody, antibody fragment, chimeric antibody, humanized antibody, or single-chain antibody. Antibodies, TAT binding oligopeptides and TAT binding organic molecules employed in the methods of the present invention may optionally be conjugated to a growth inhibitory agent or cytotoxic agent such as a toxin, including, for example, a maytansinoid or calicheamicin, an antibiotic, a radioactive isotope, a nucleolytic enzyme, or the like. The antibodies and oligopeptides employed in the methods of the present invention may optionally be produced in CHO cells or bacterial cells.

C. Further Additional Embodiments

In yet further embodiments, the invention is directed to the following set of potential claims for this application:

1. Isolated nucleic acid having a nucleotide sequence that has at least 80% nucleic acid sequence identity to:

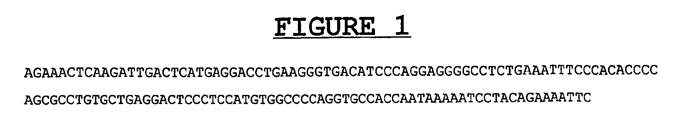

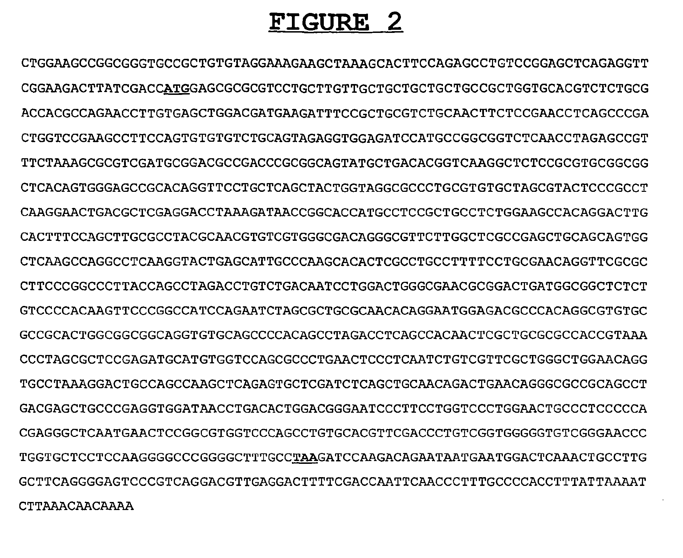

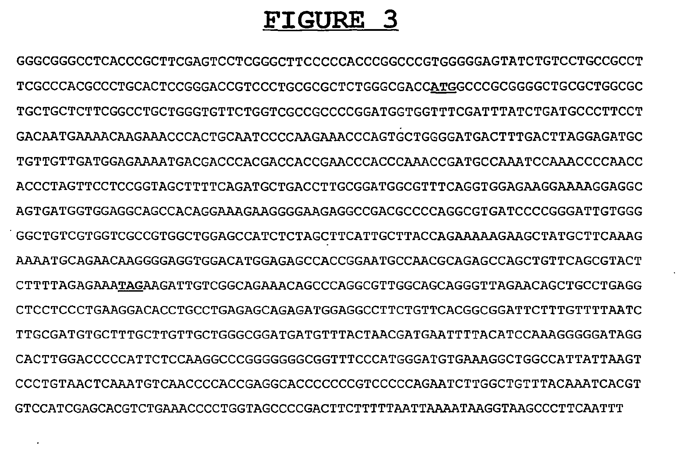

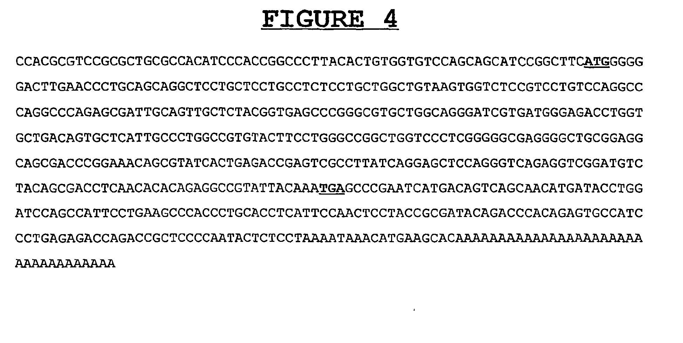

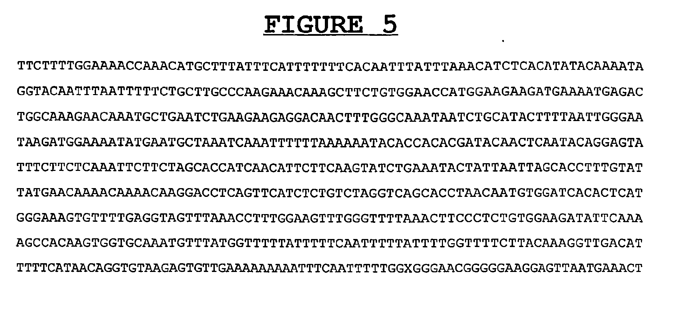

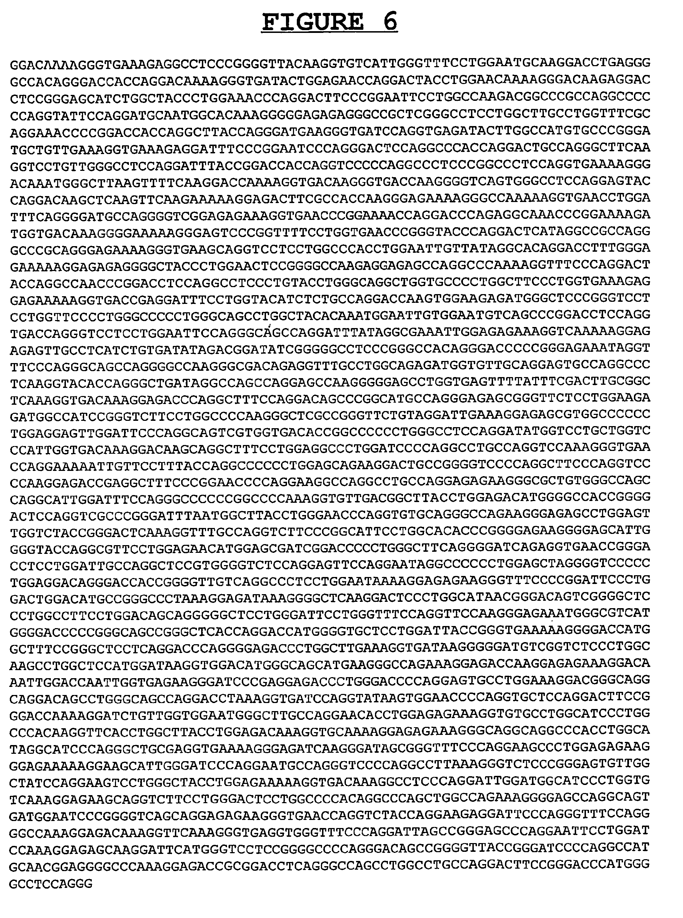

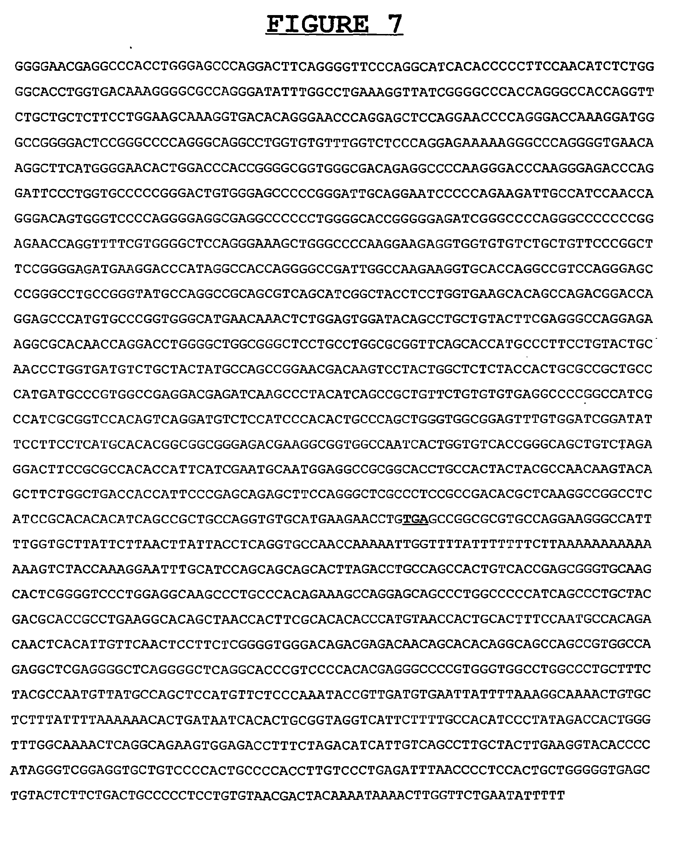

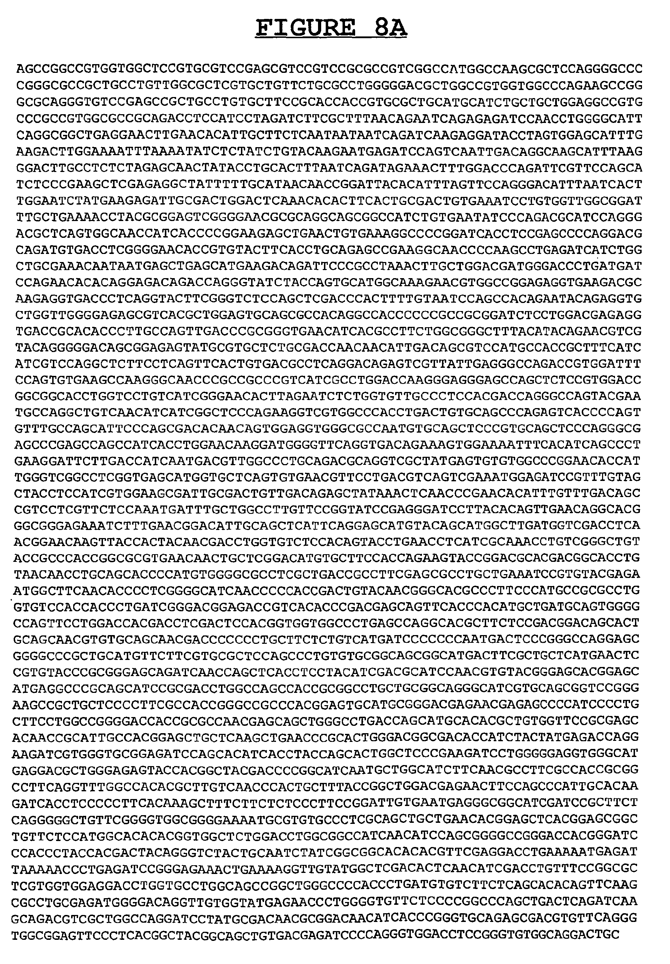

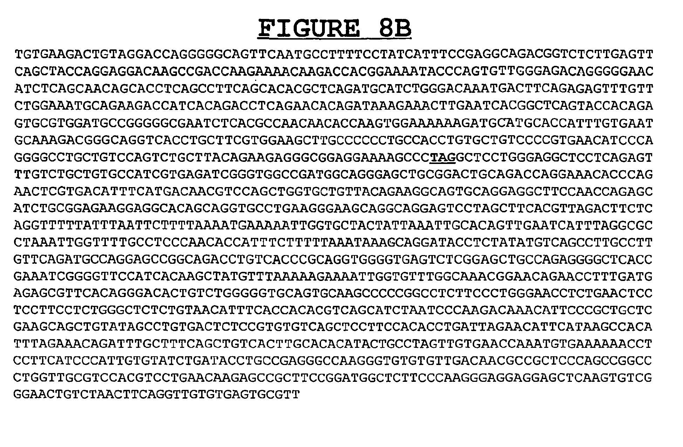

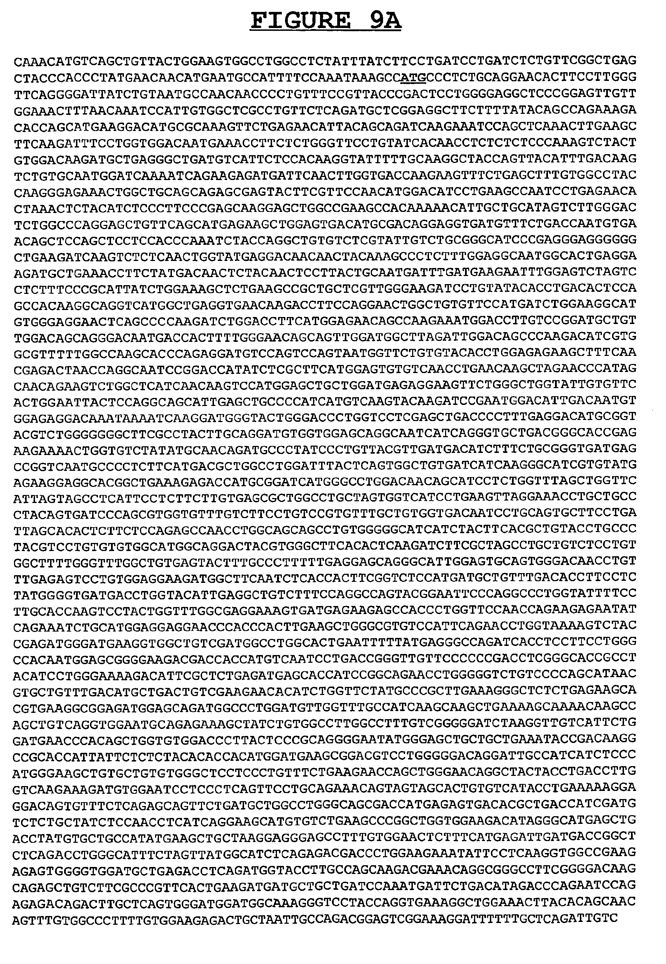

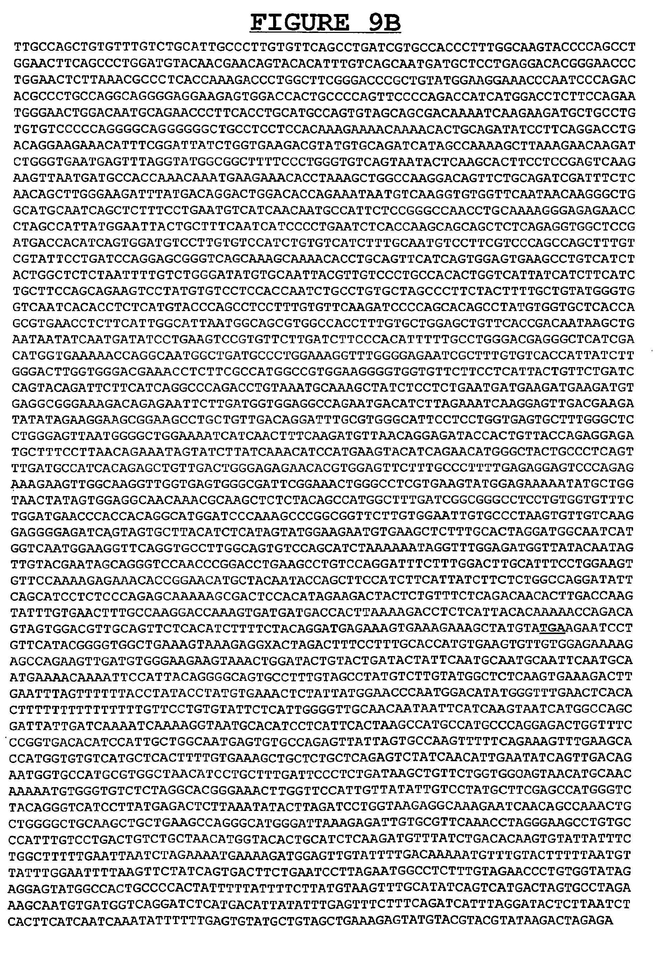

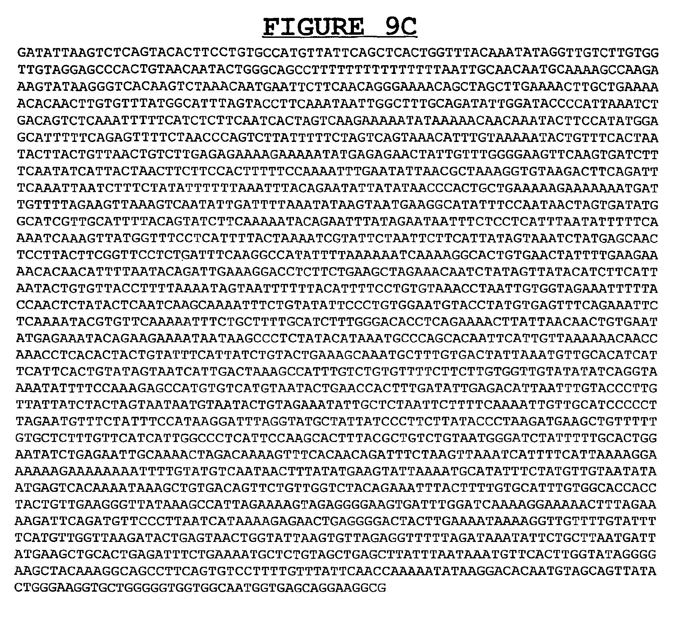

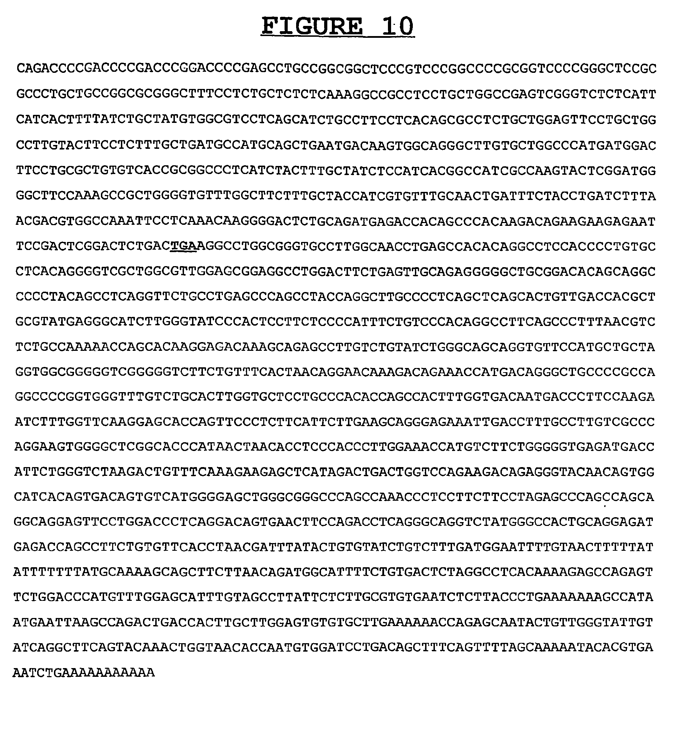

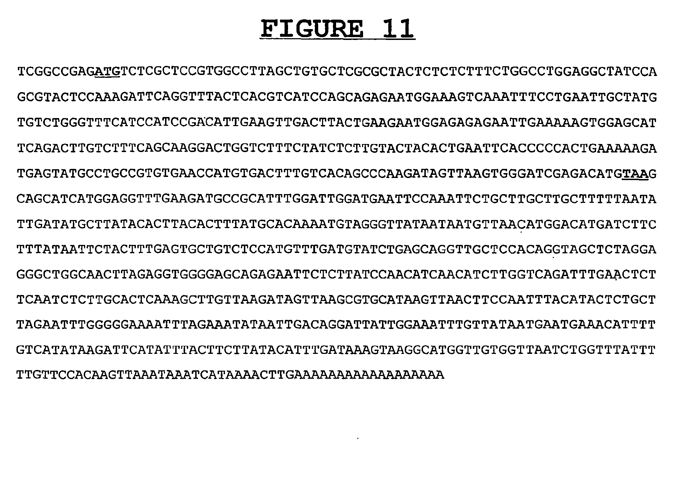

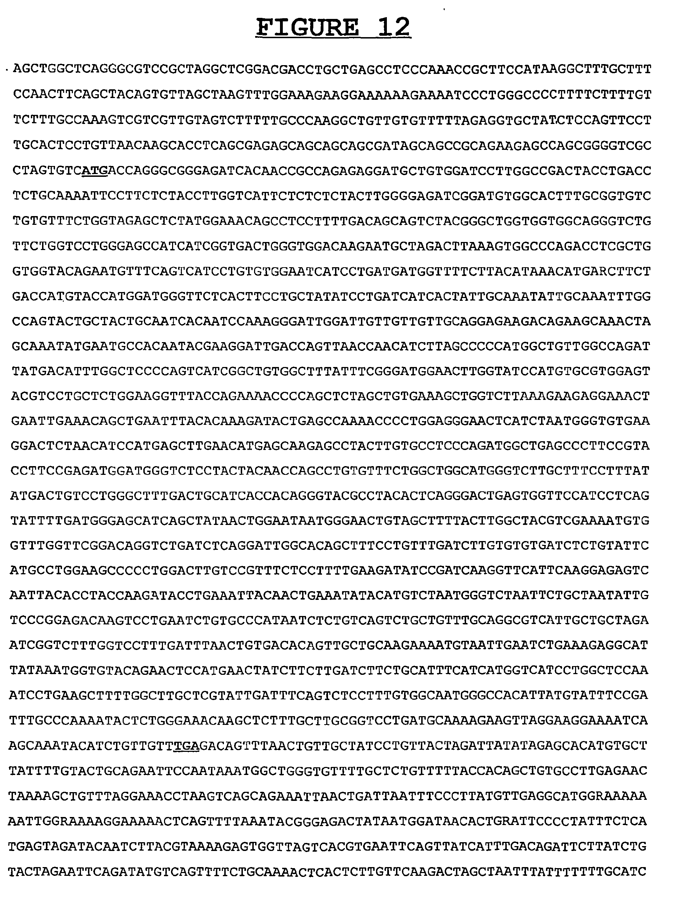

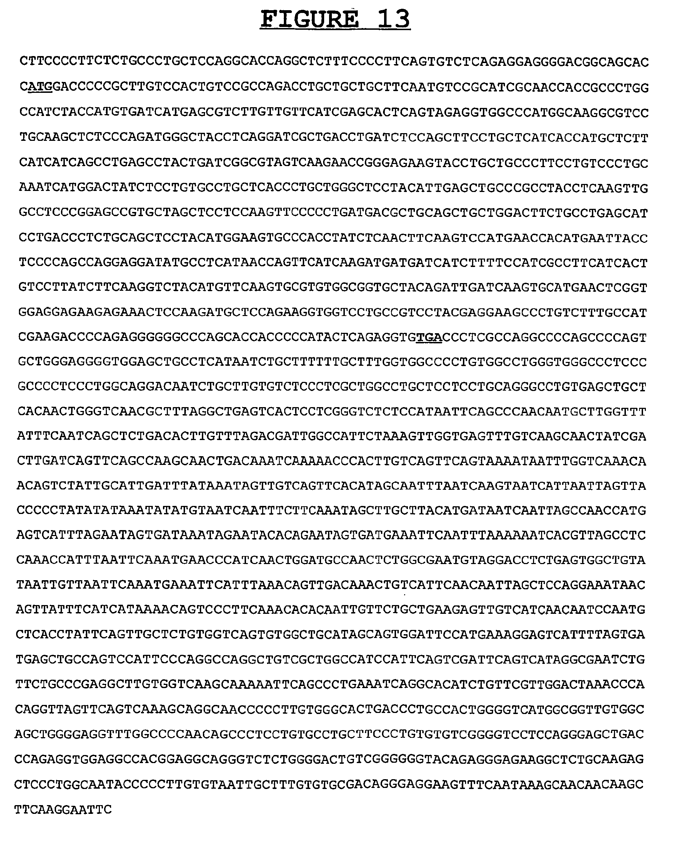

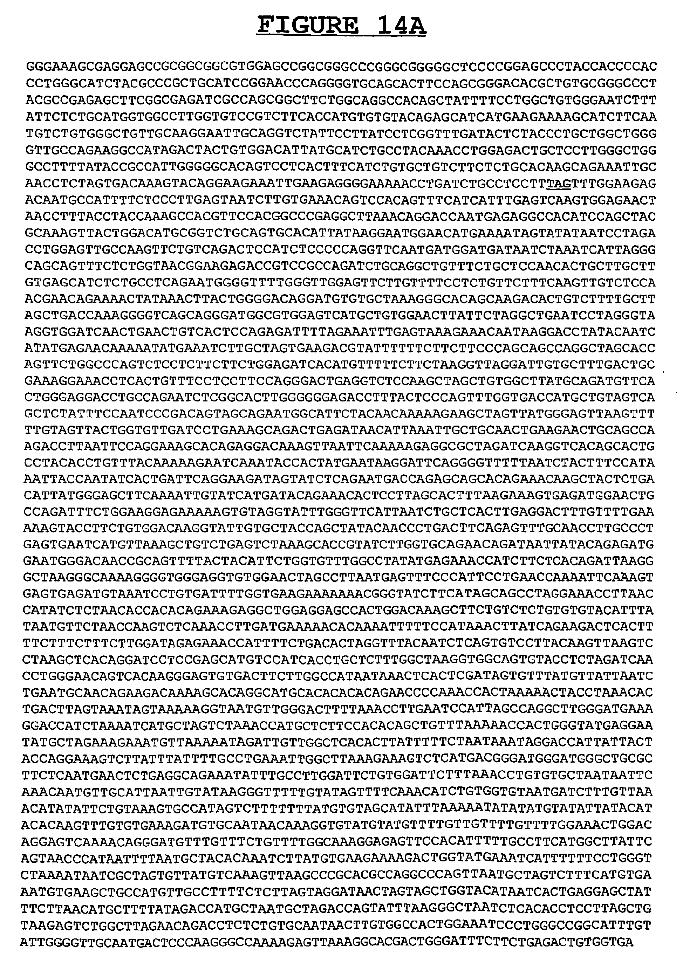

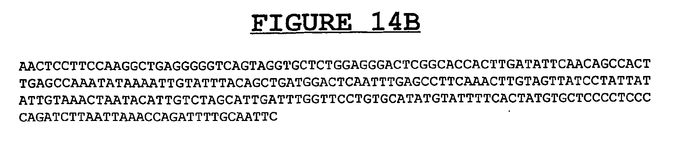

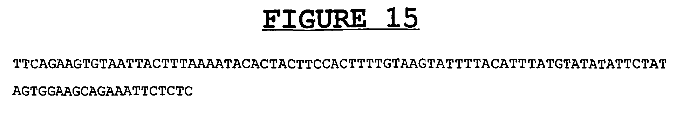

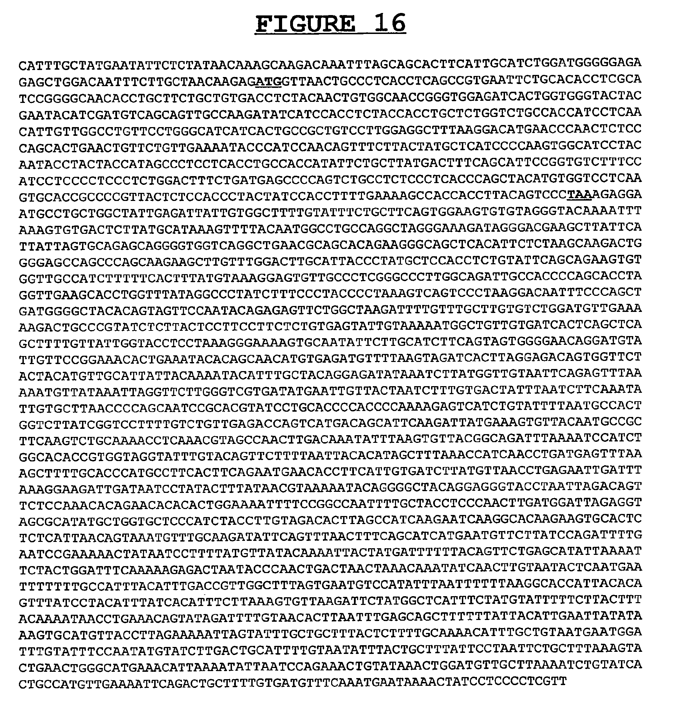

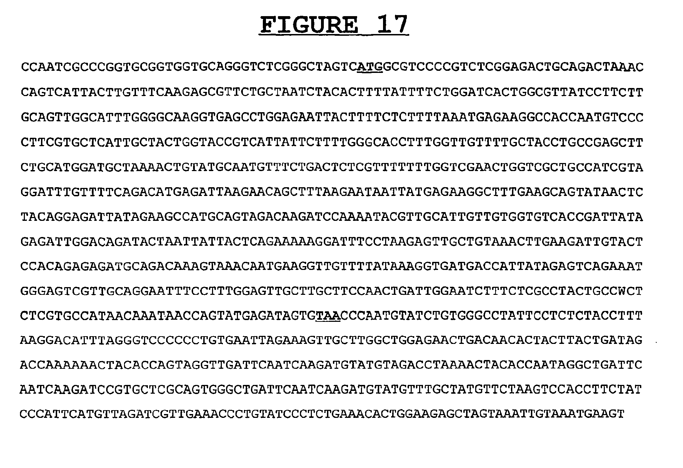

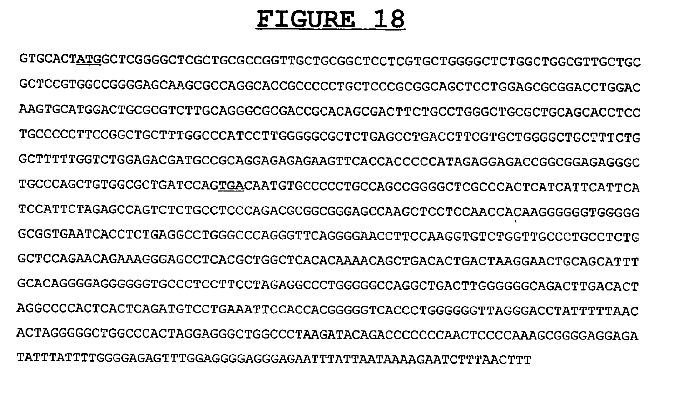

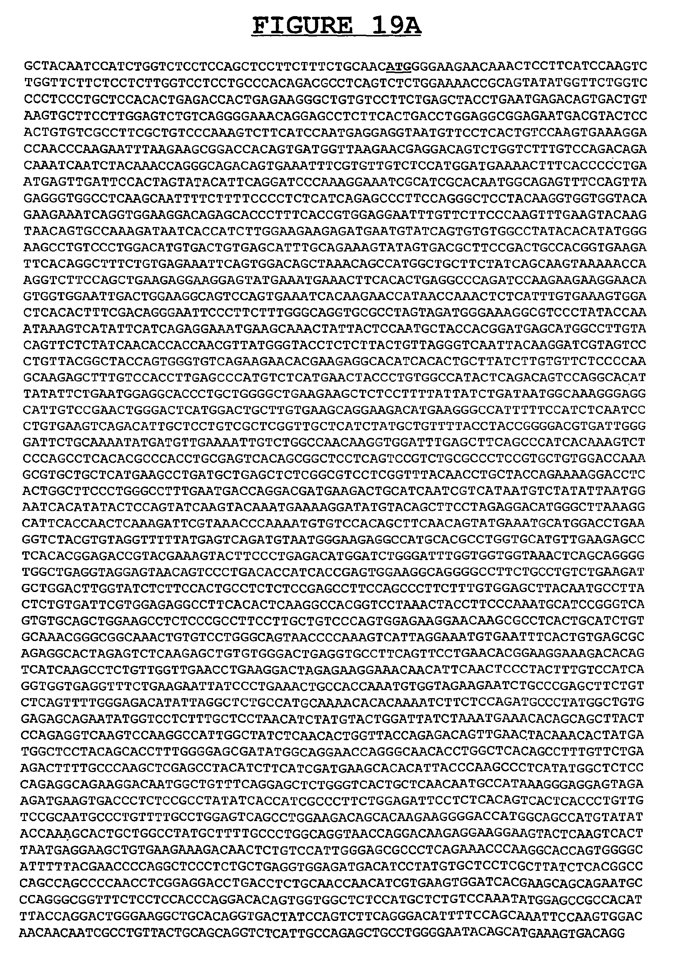

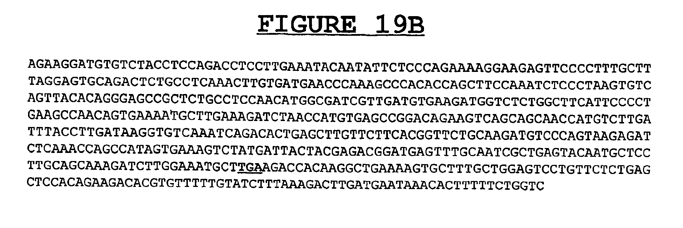

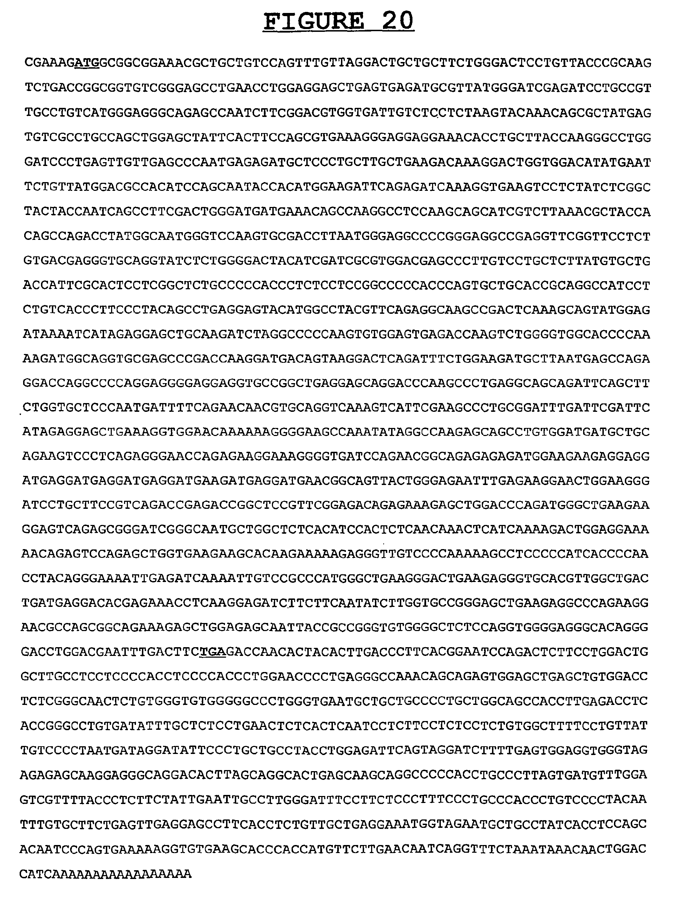

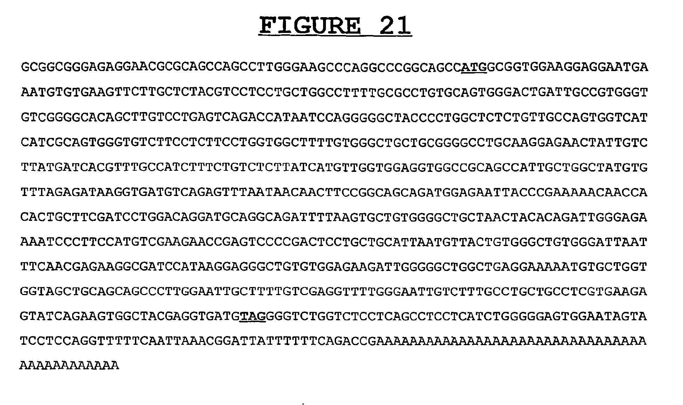

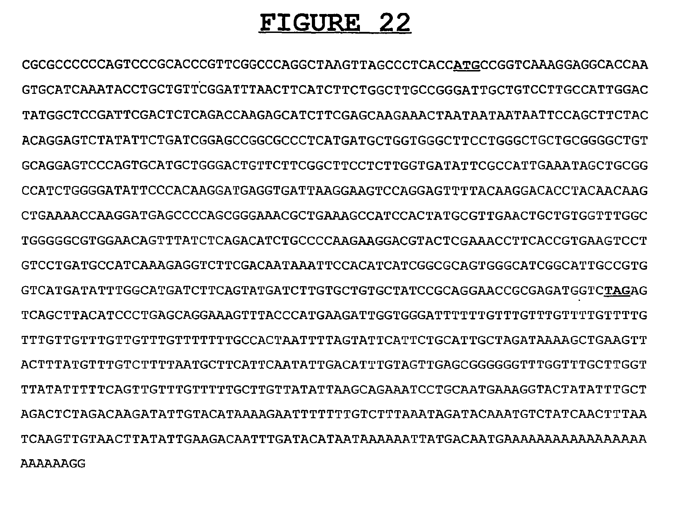

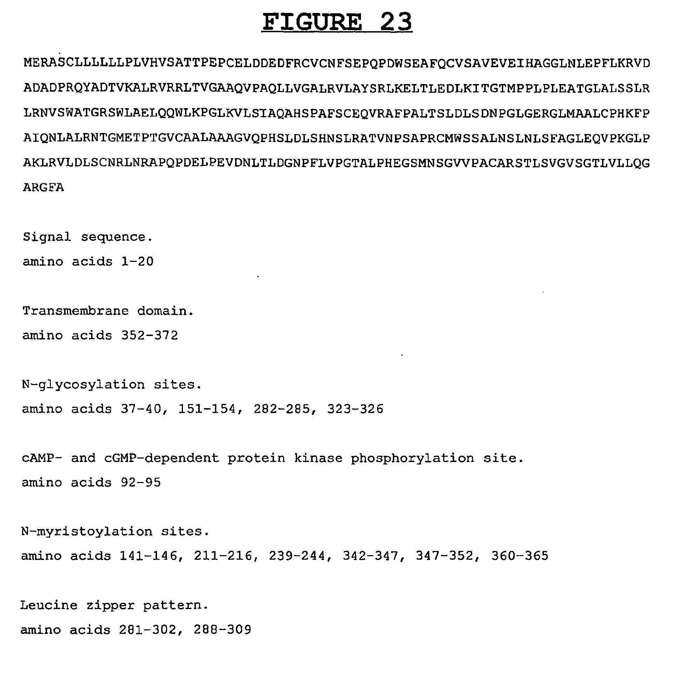

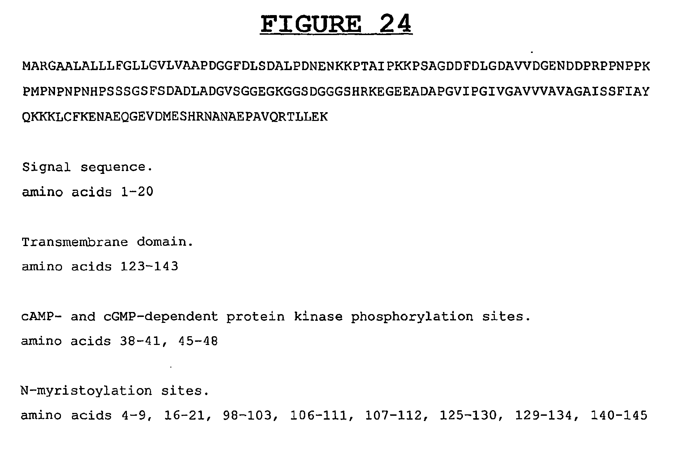

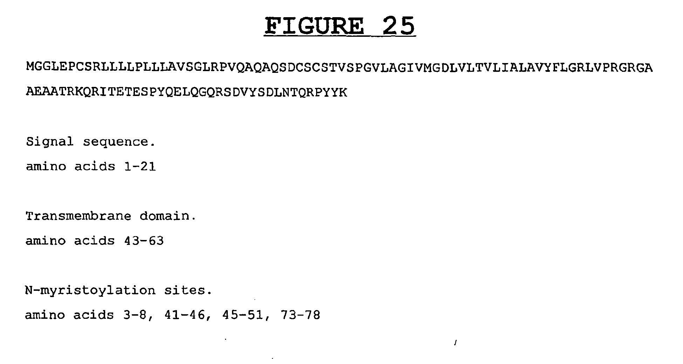

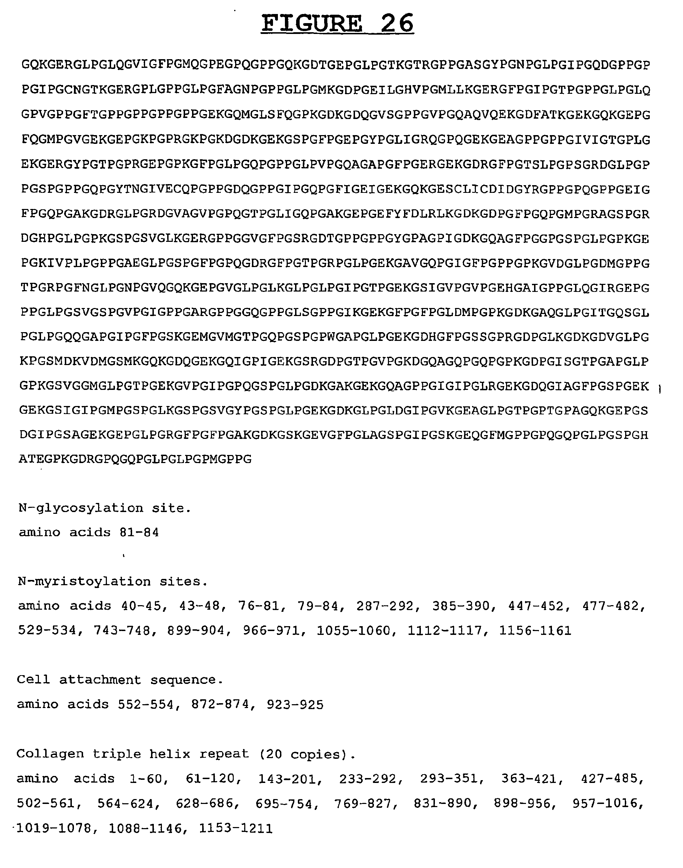

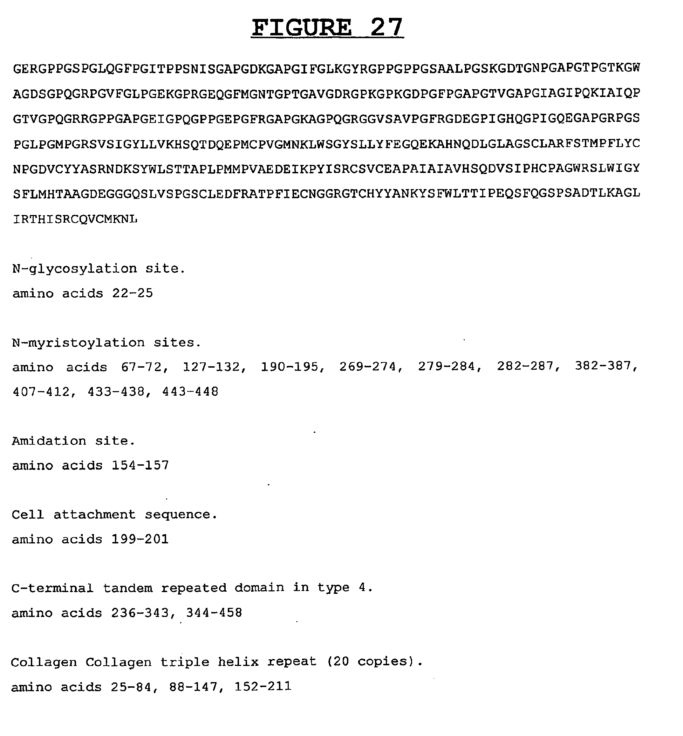

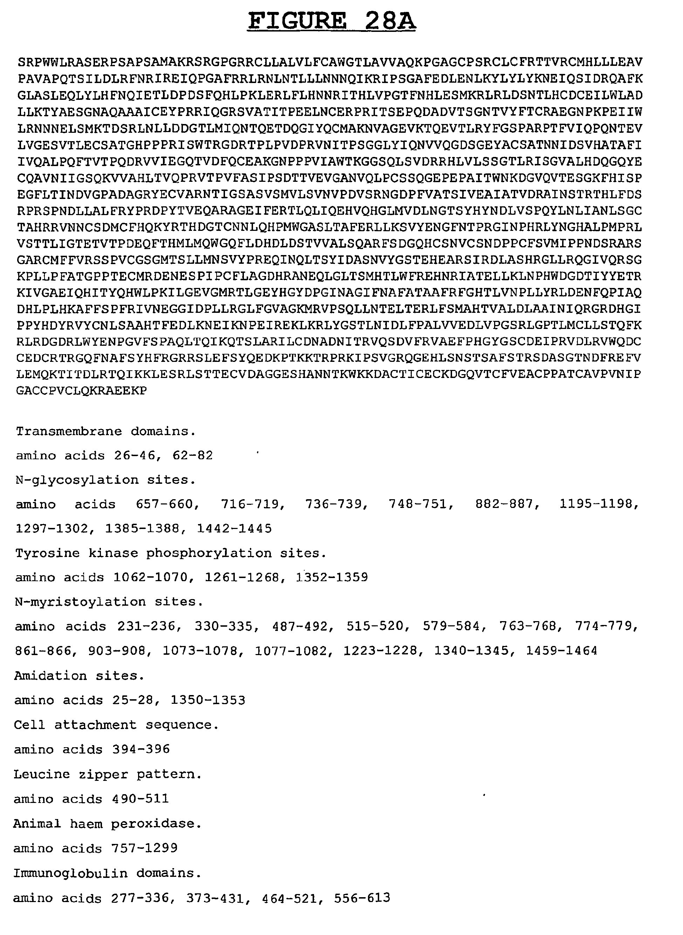

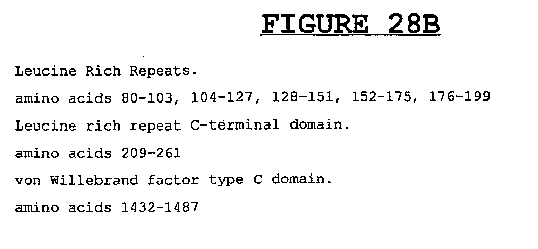

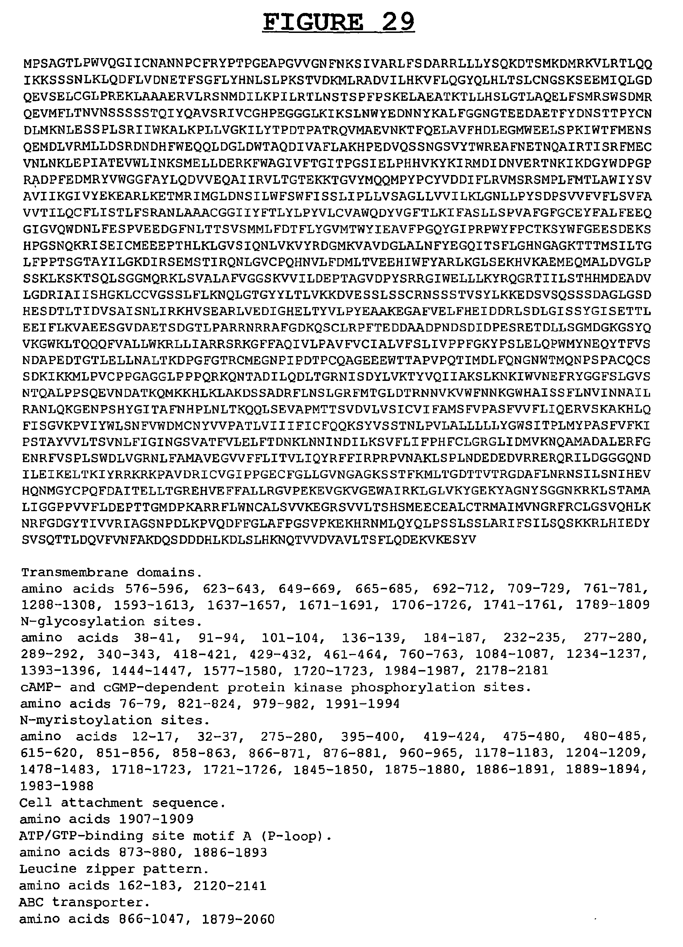

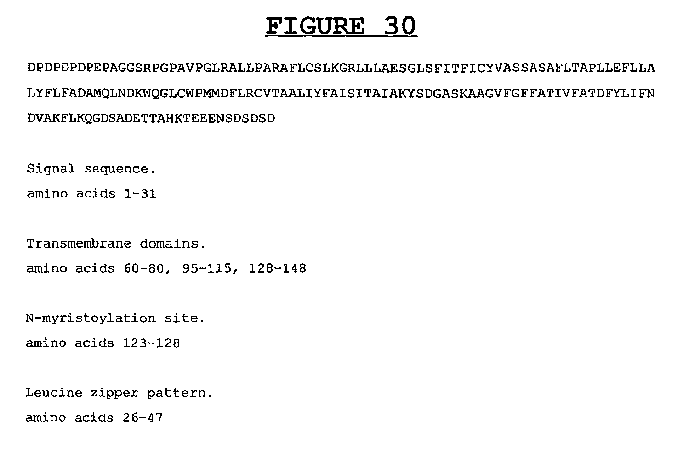

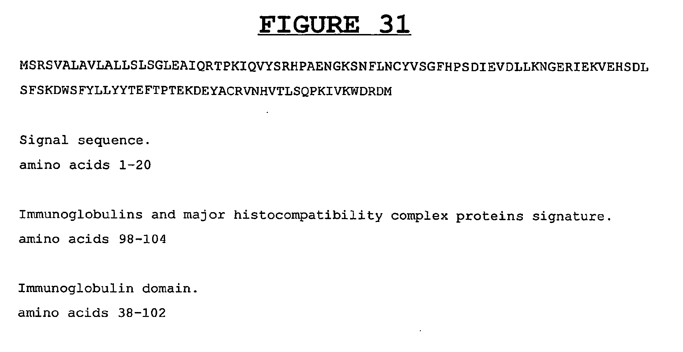

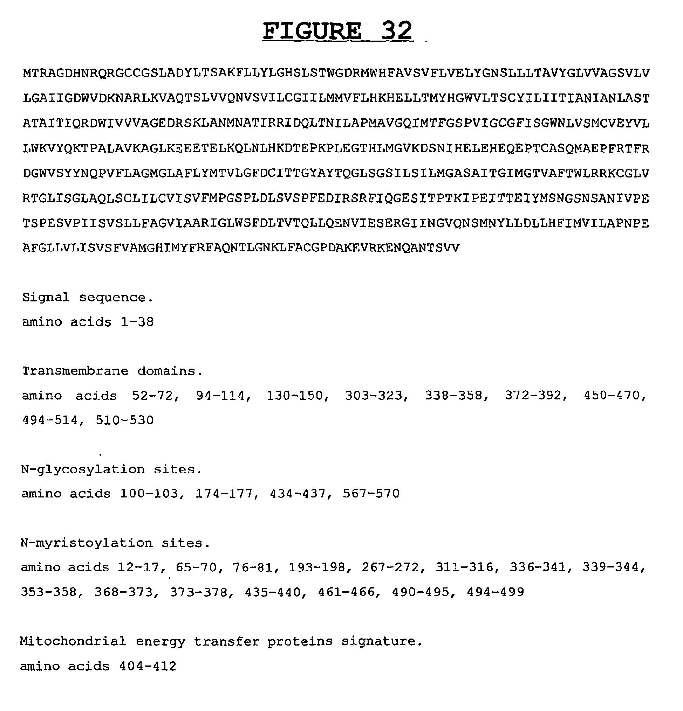

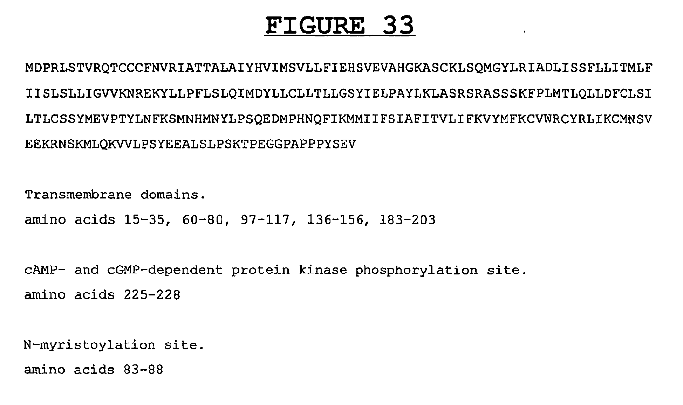

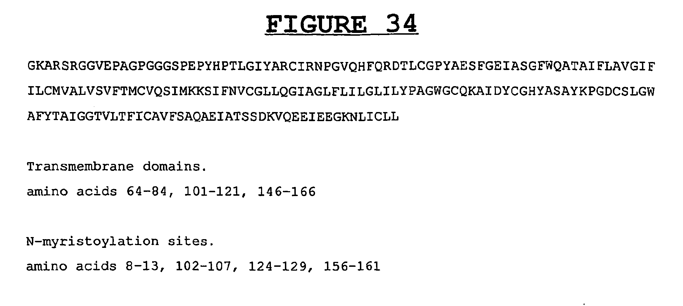

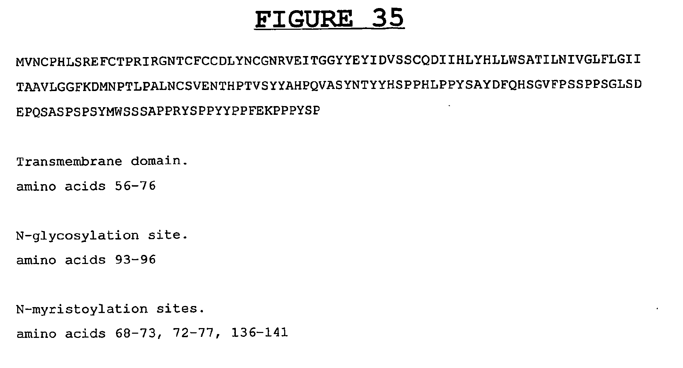

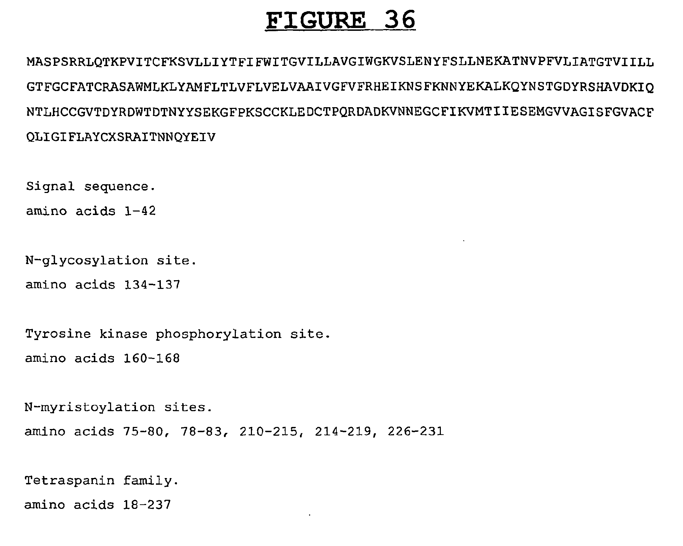

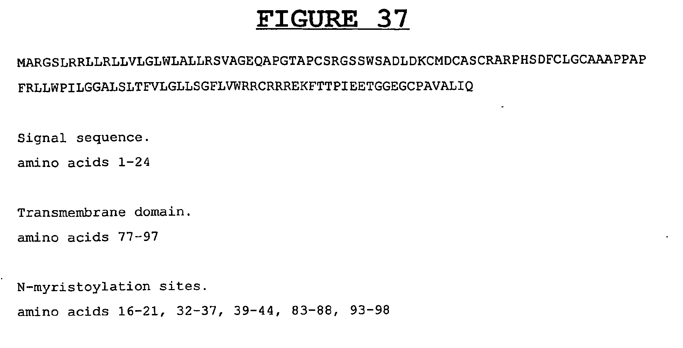

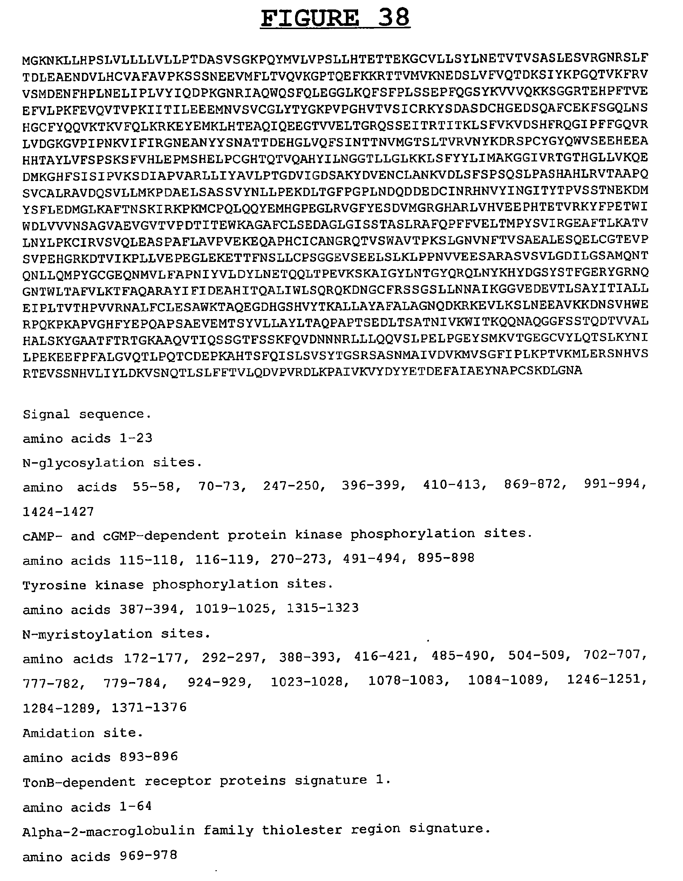

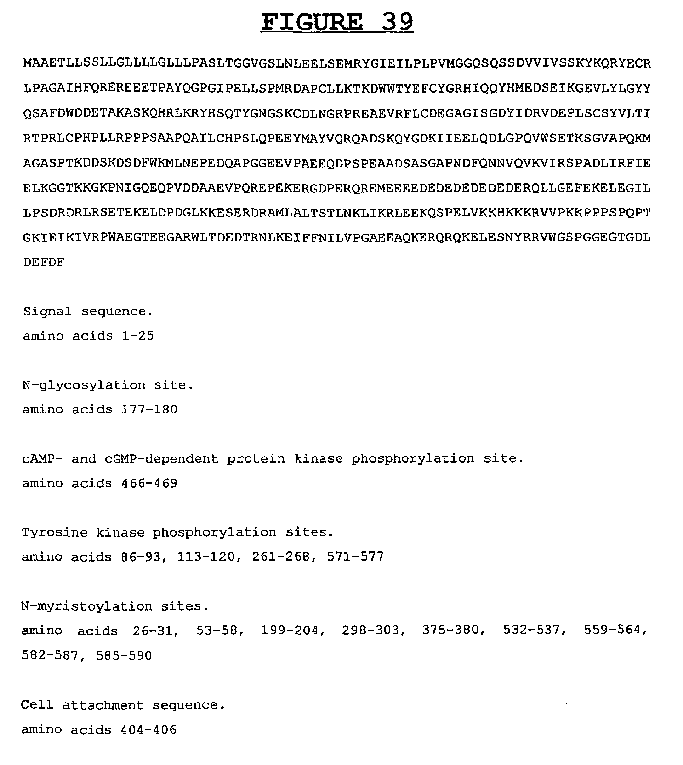

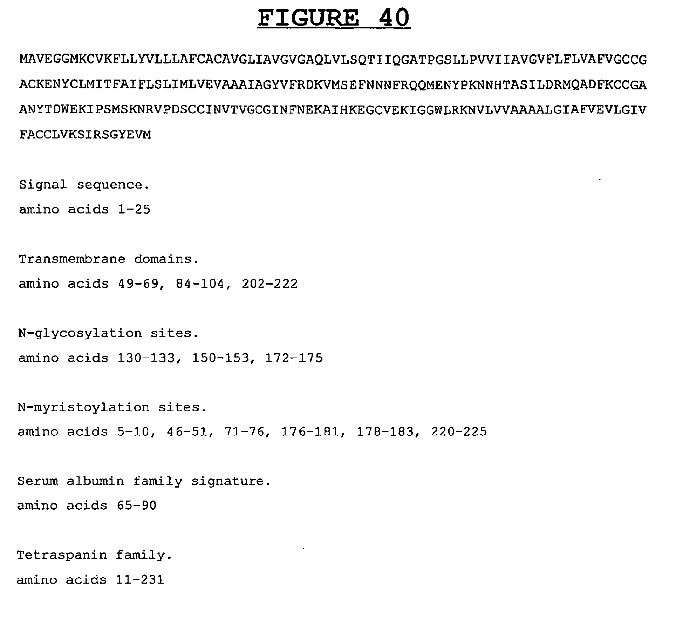

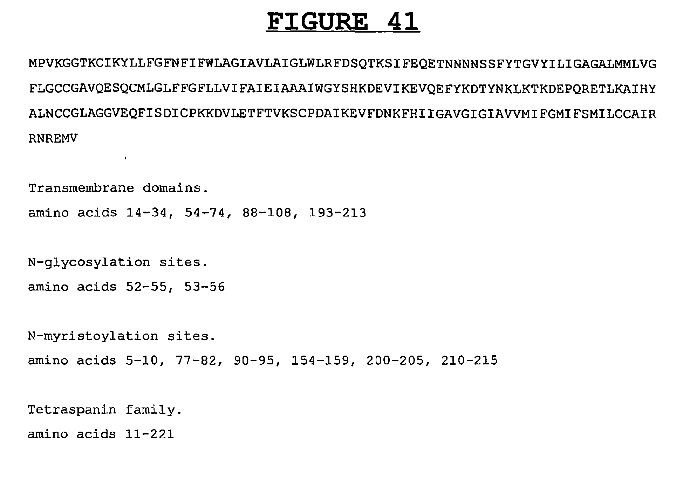

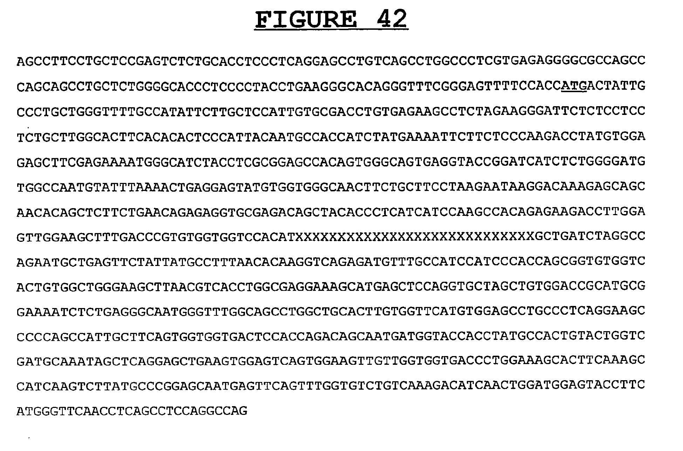

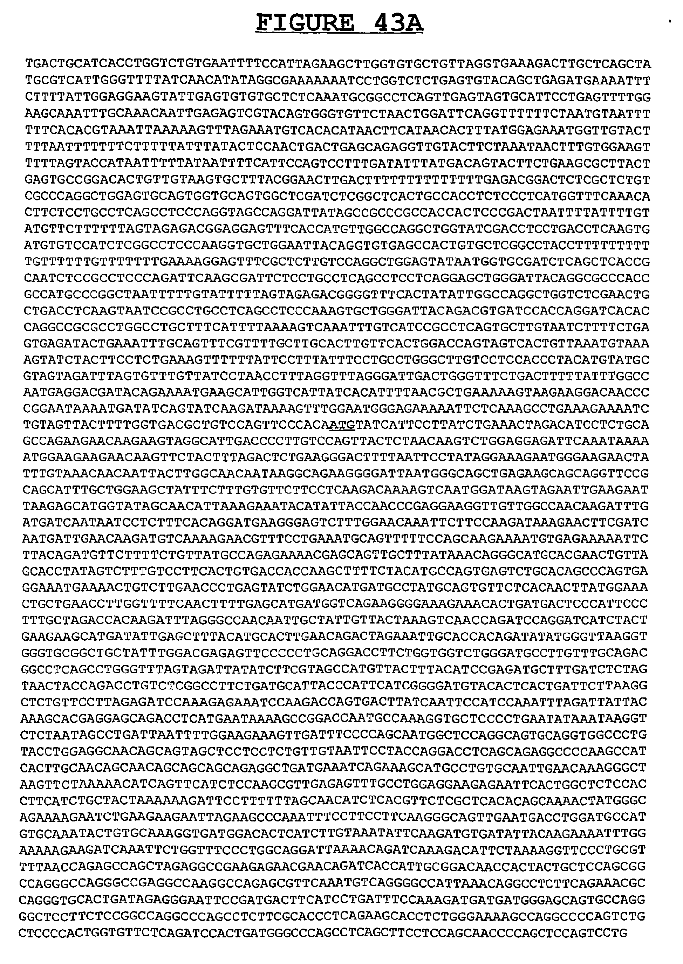

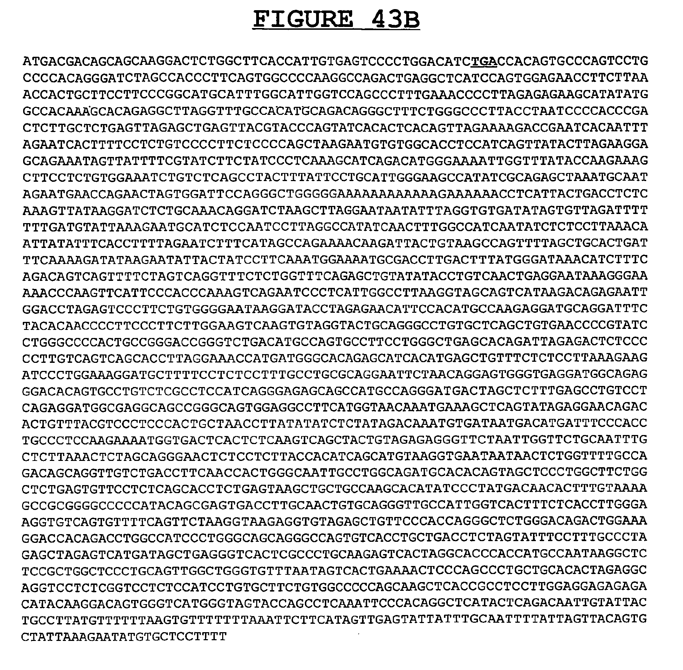

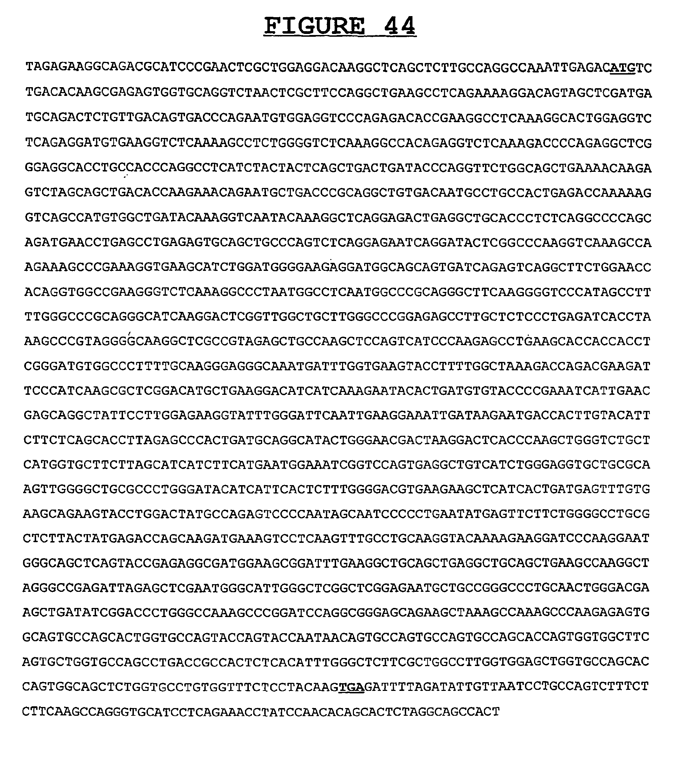

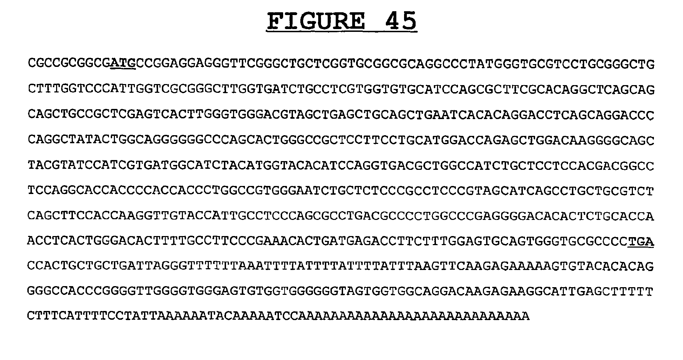

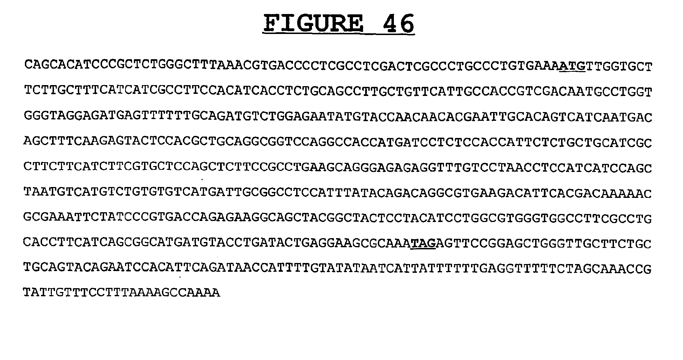

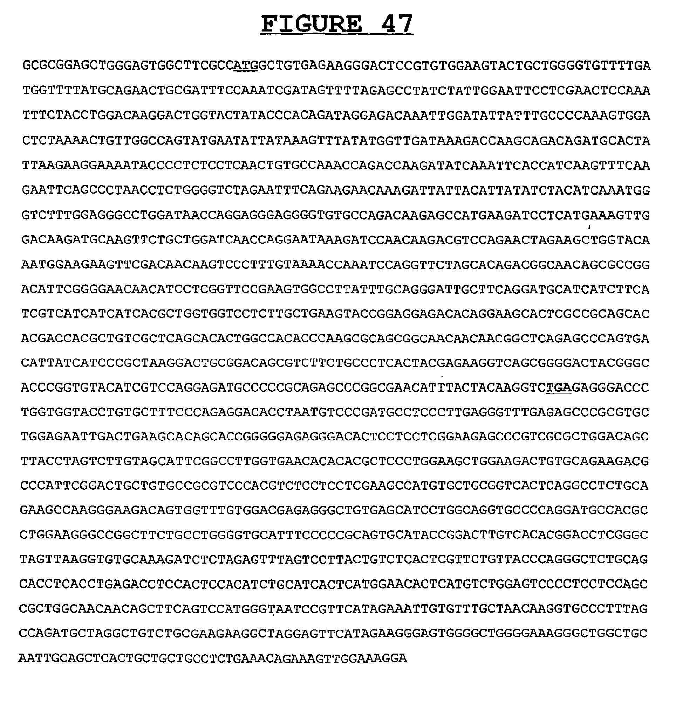

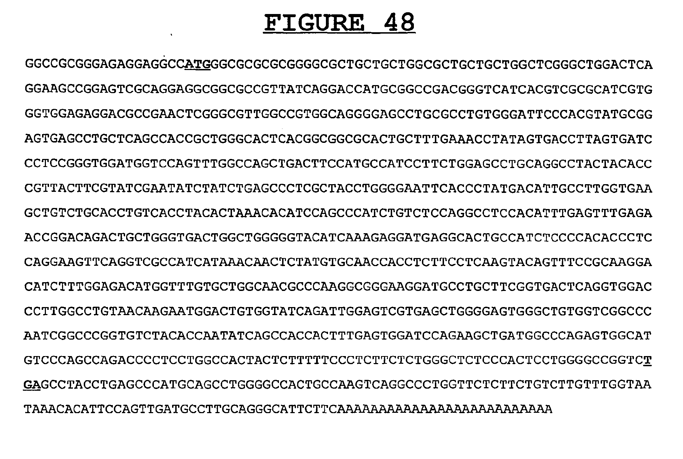

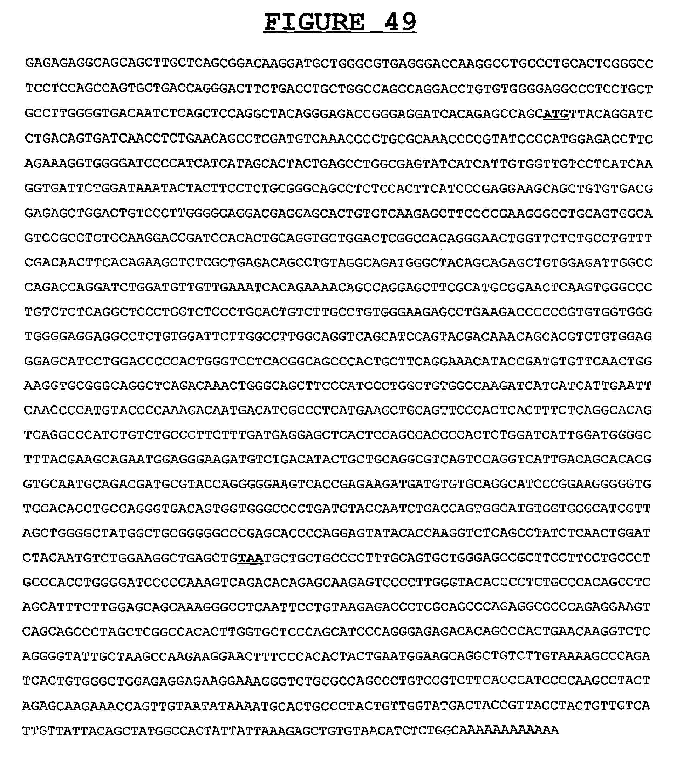

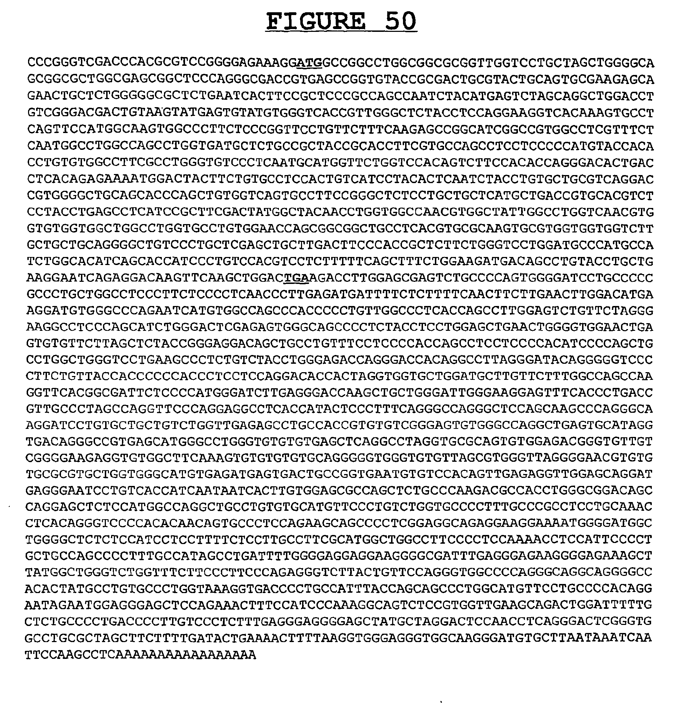

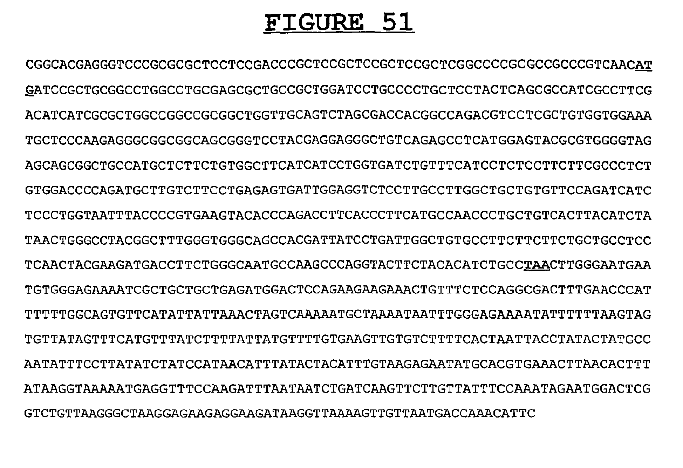

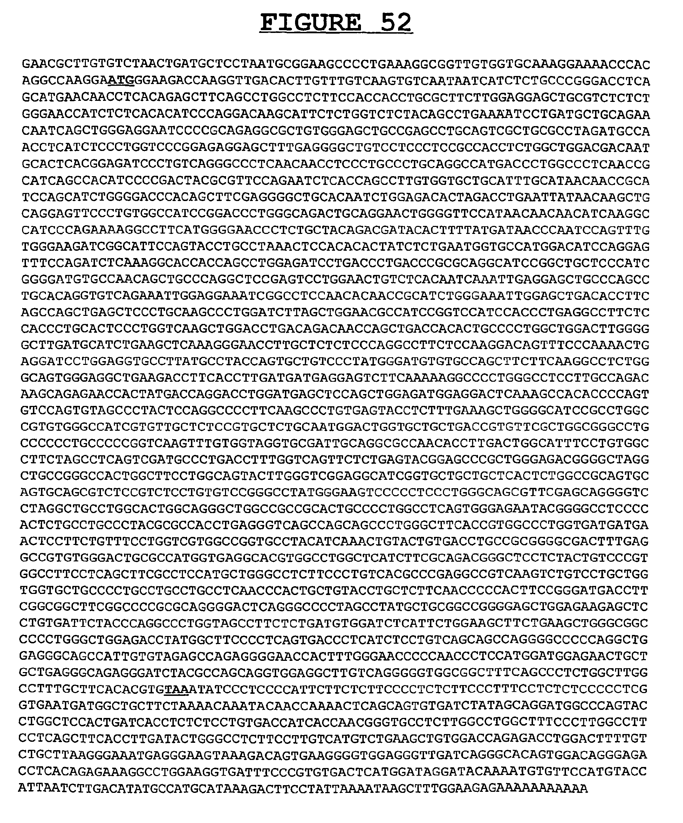

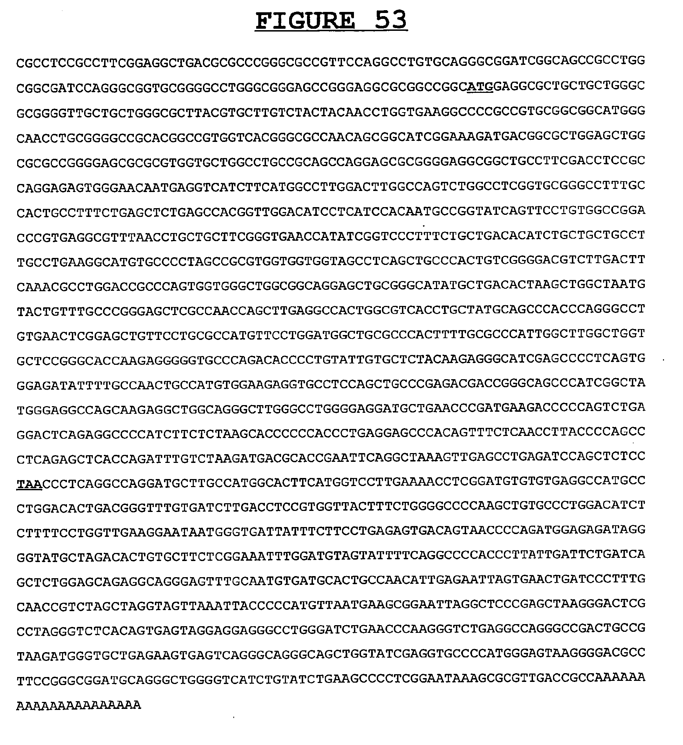

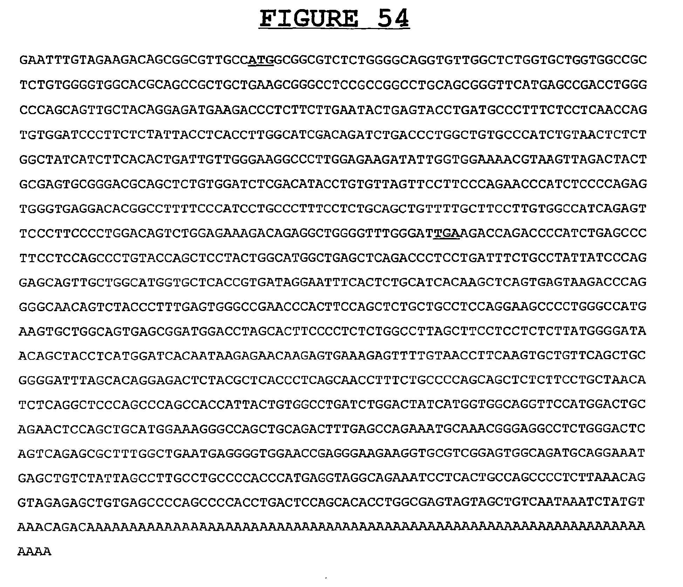

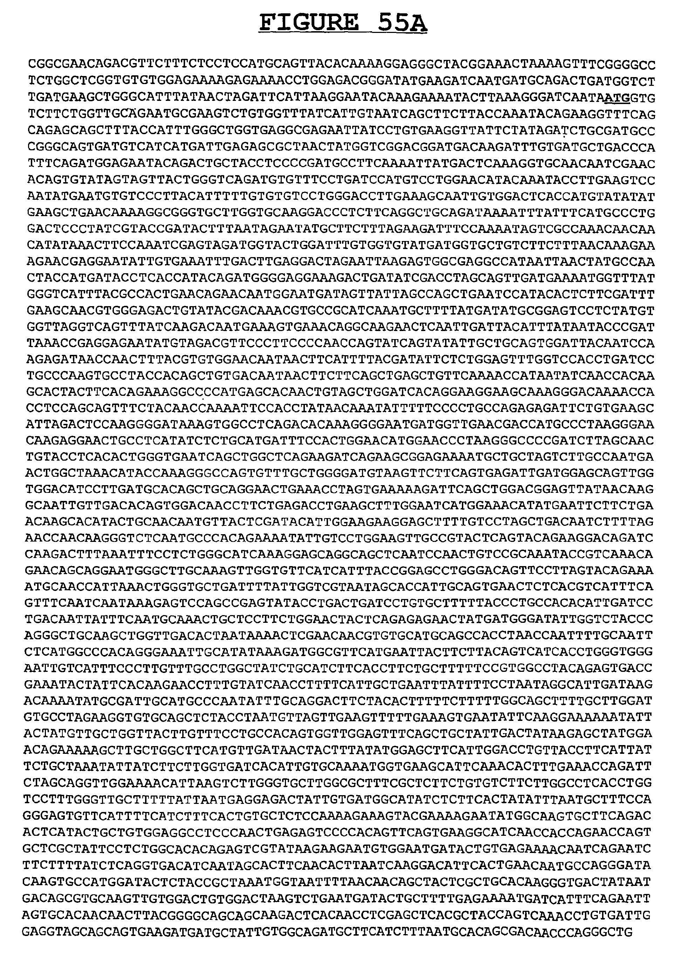

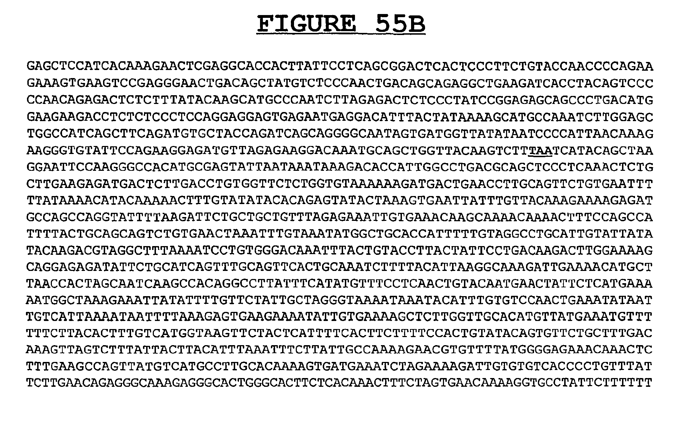

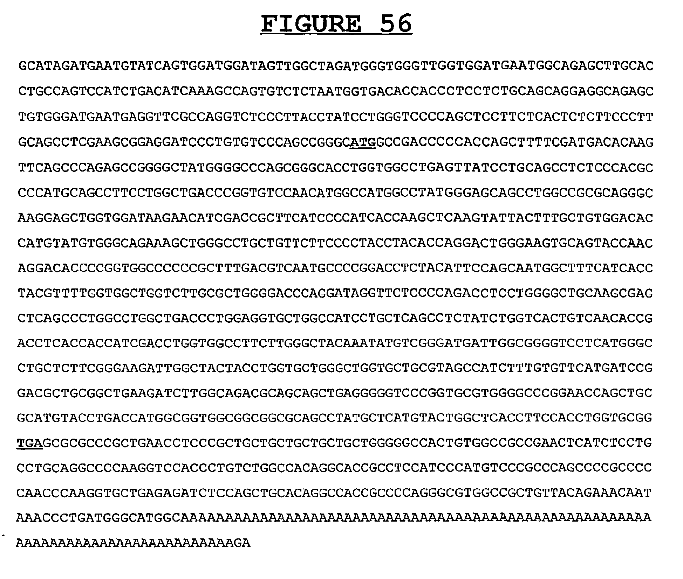

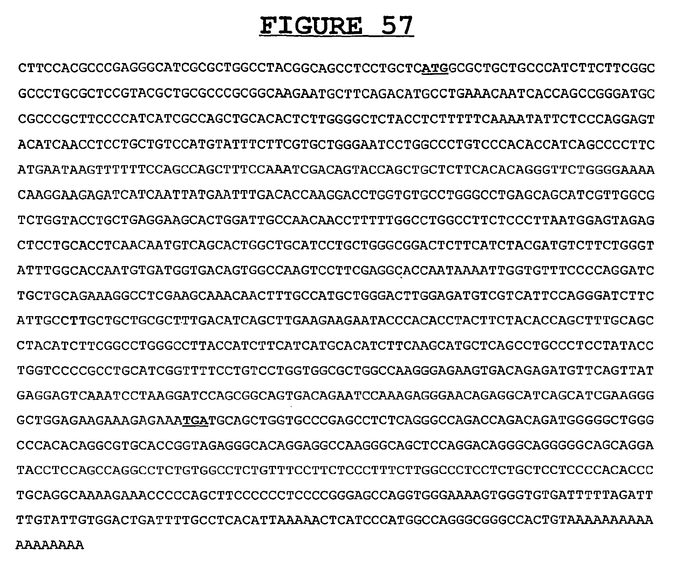

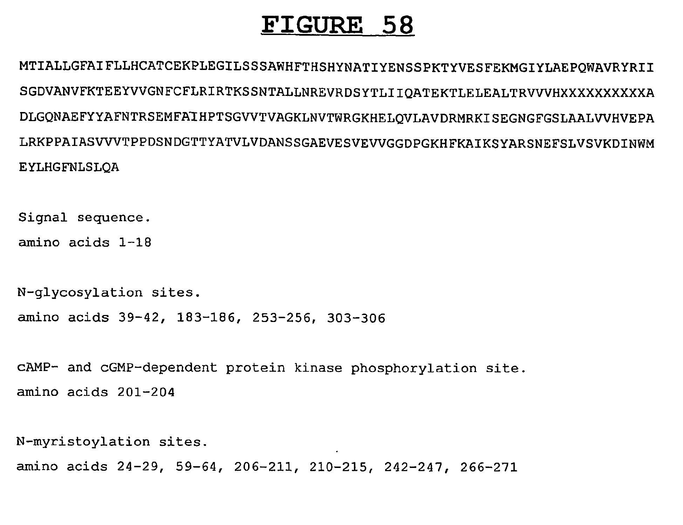

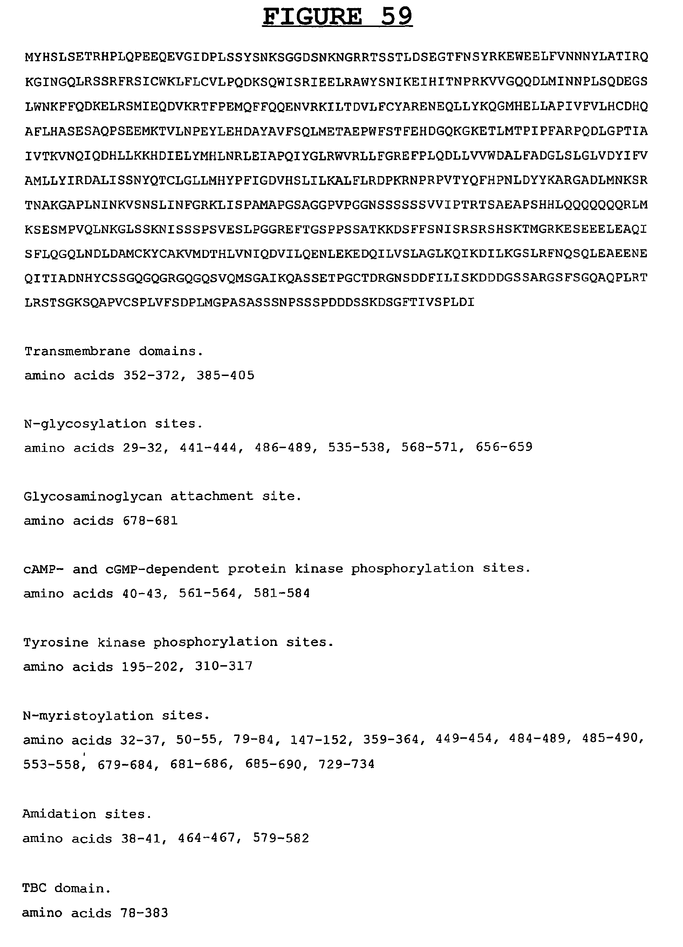

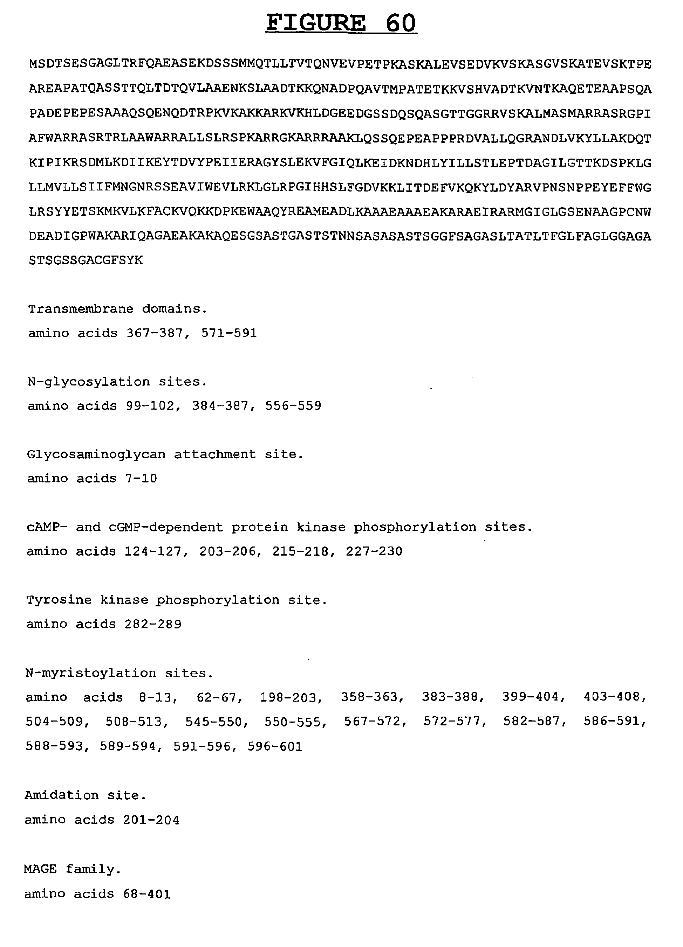

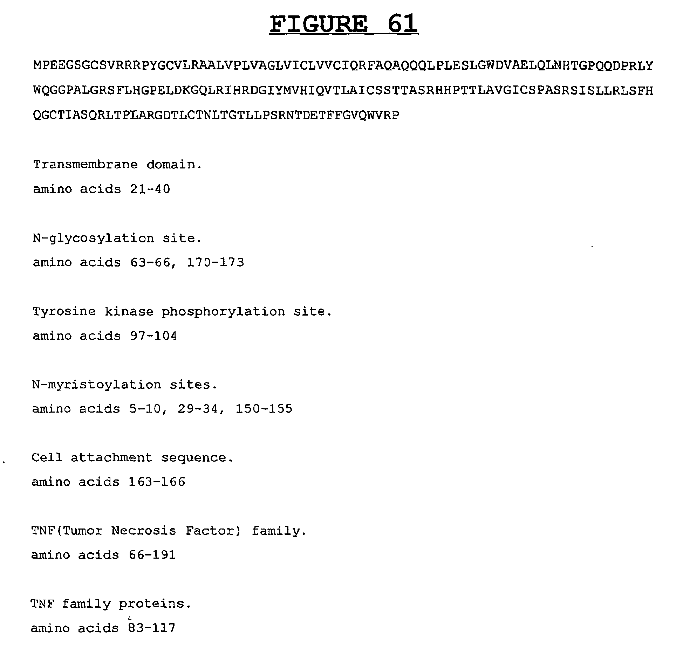

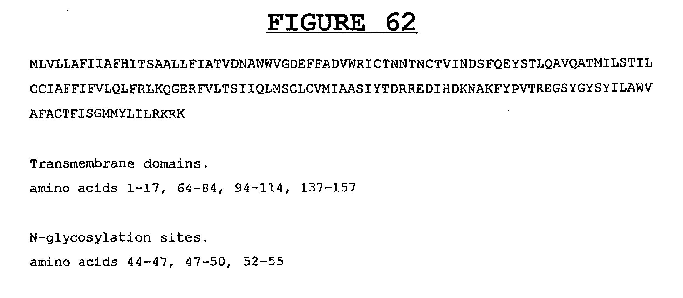

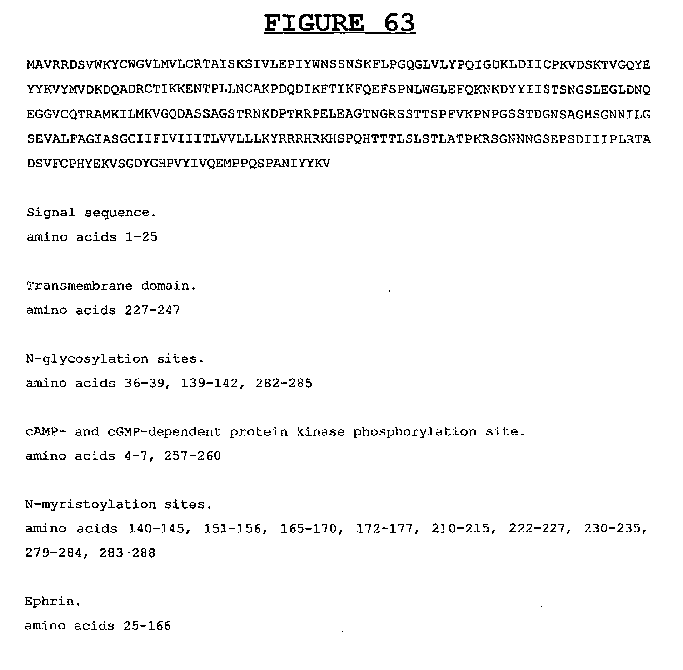

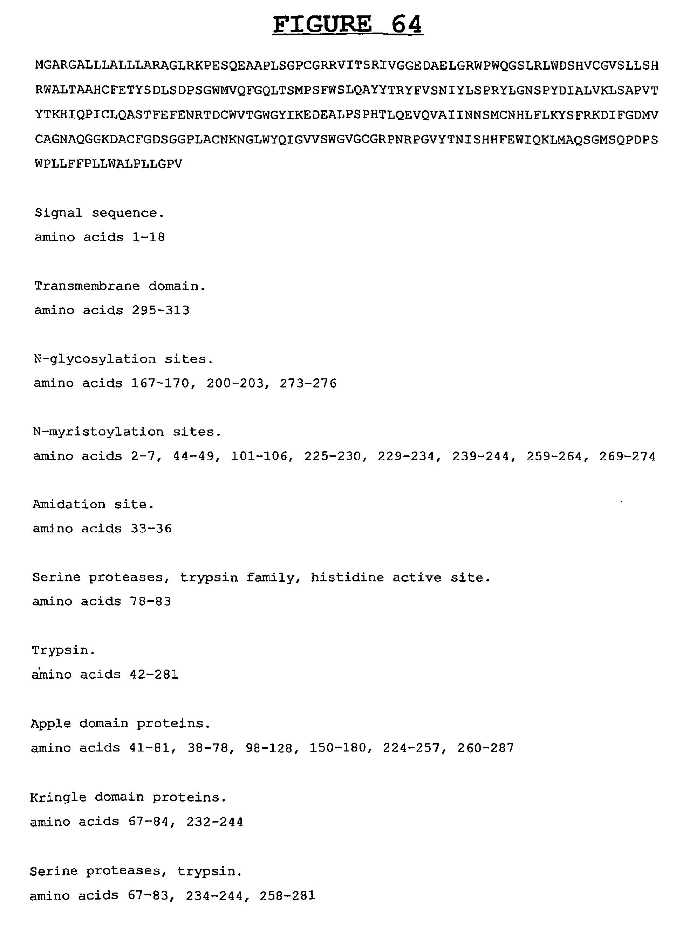

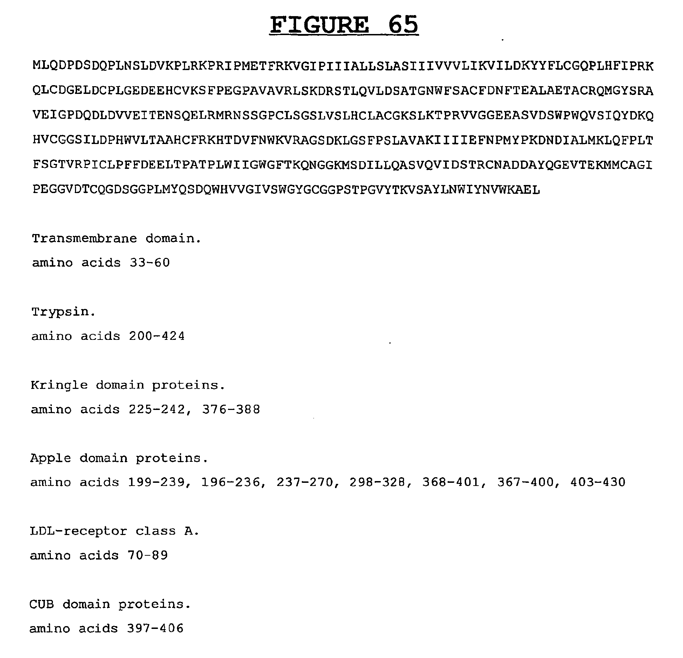

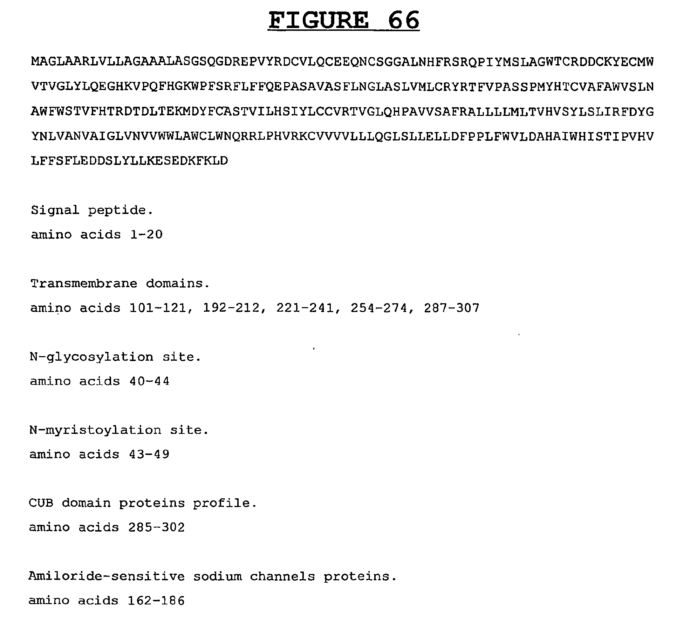

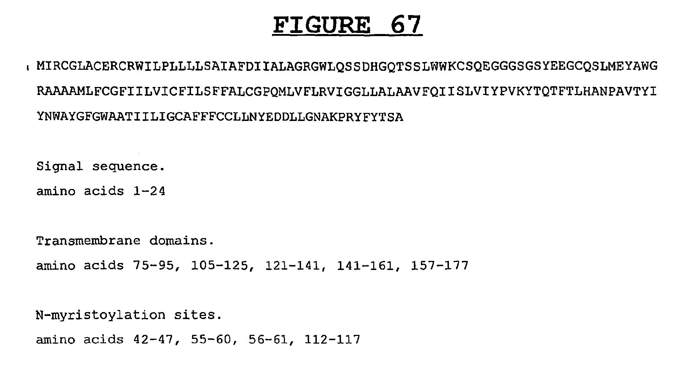

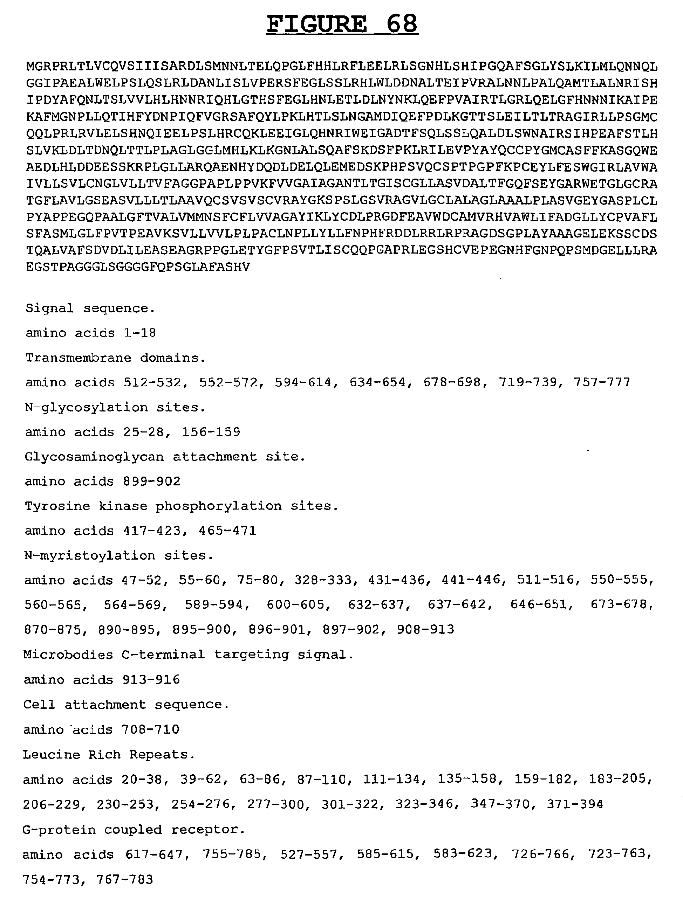

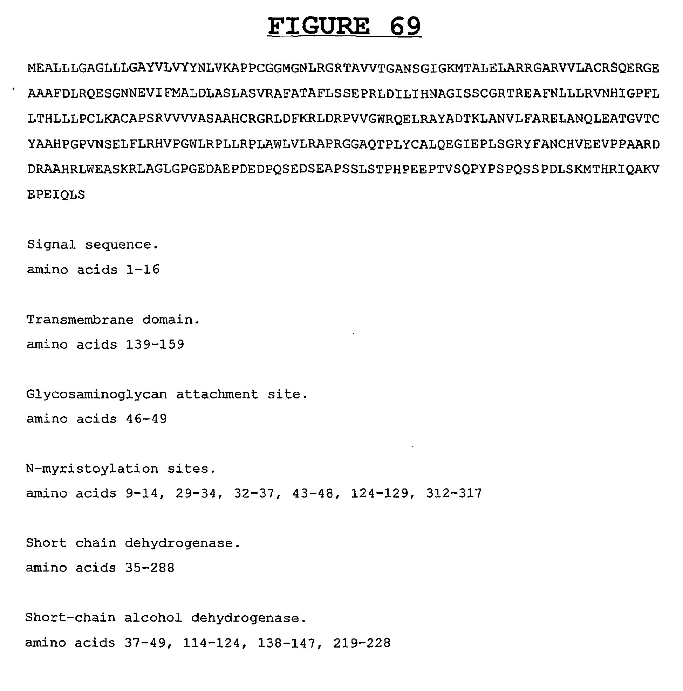

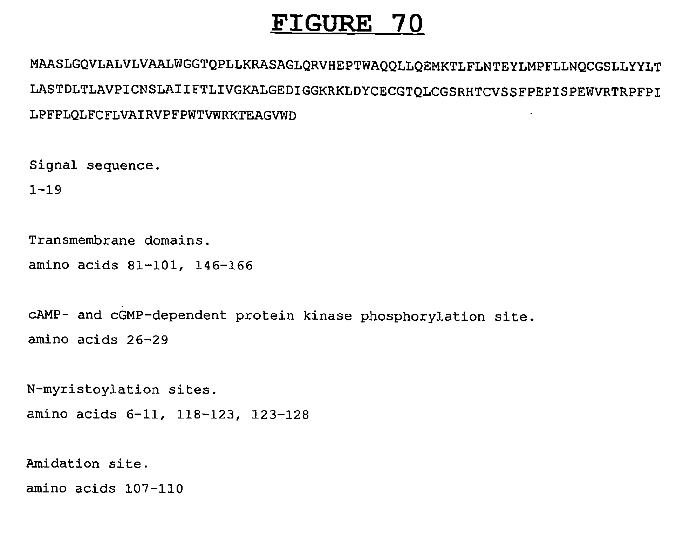

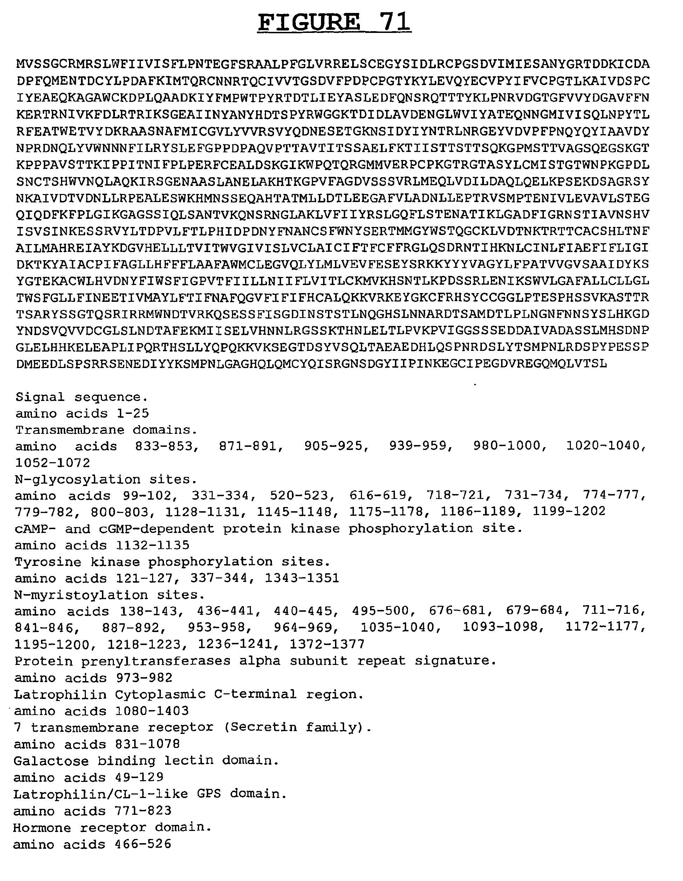

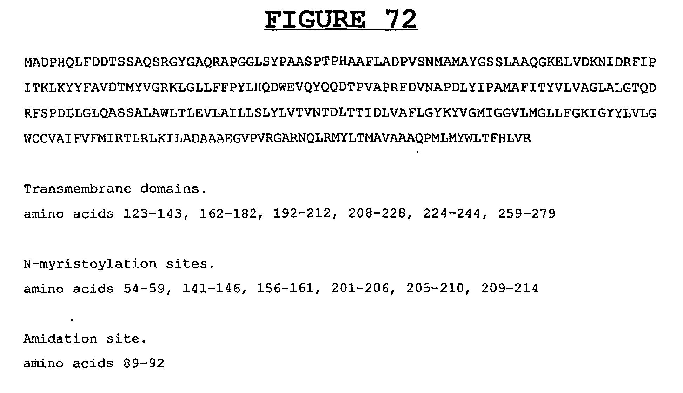

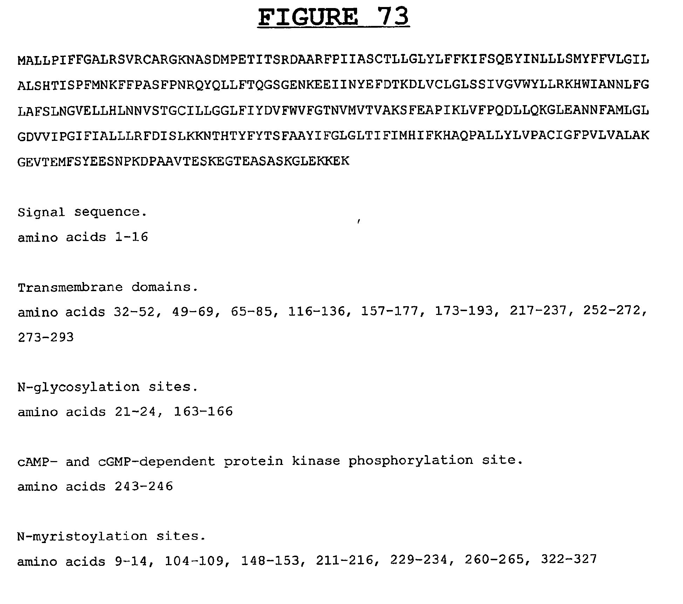

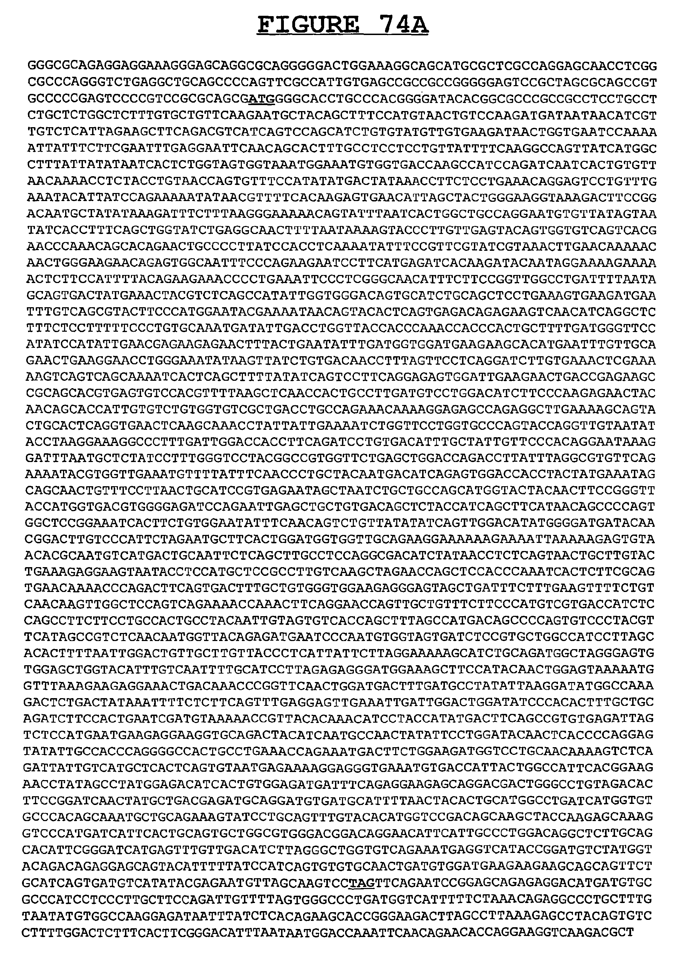

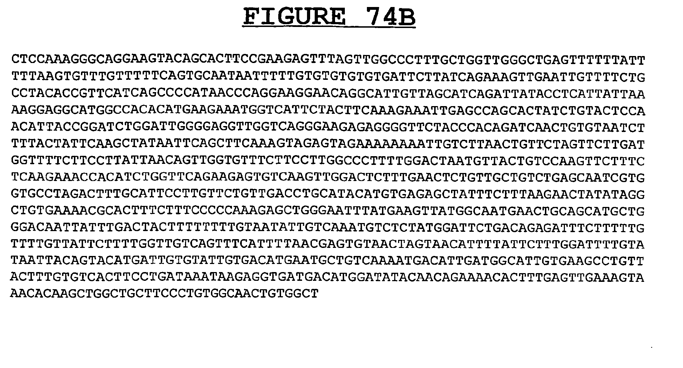

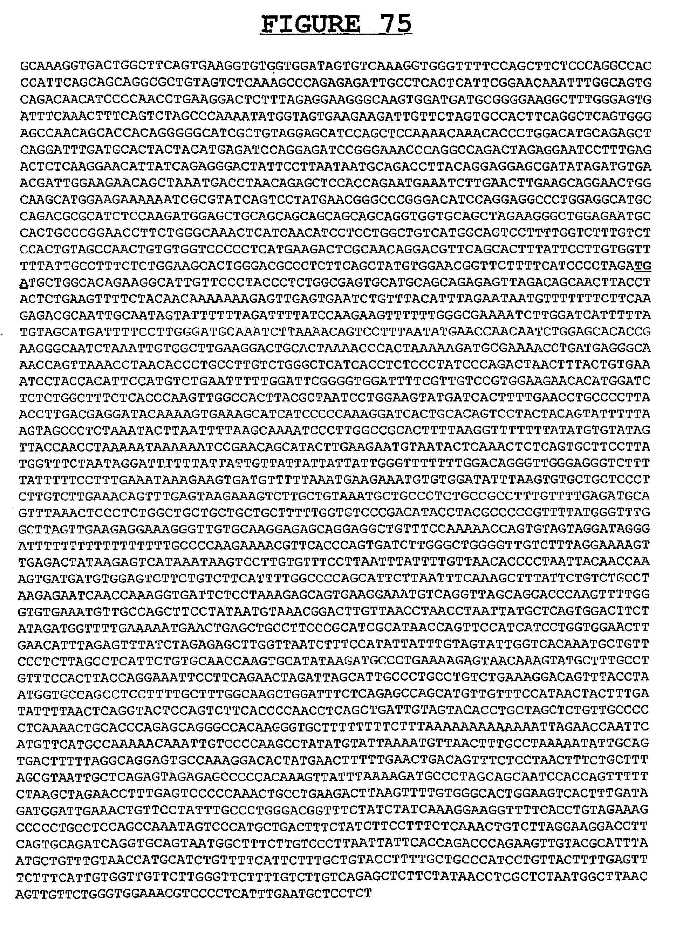

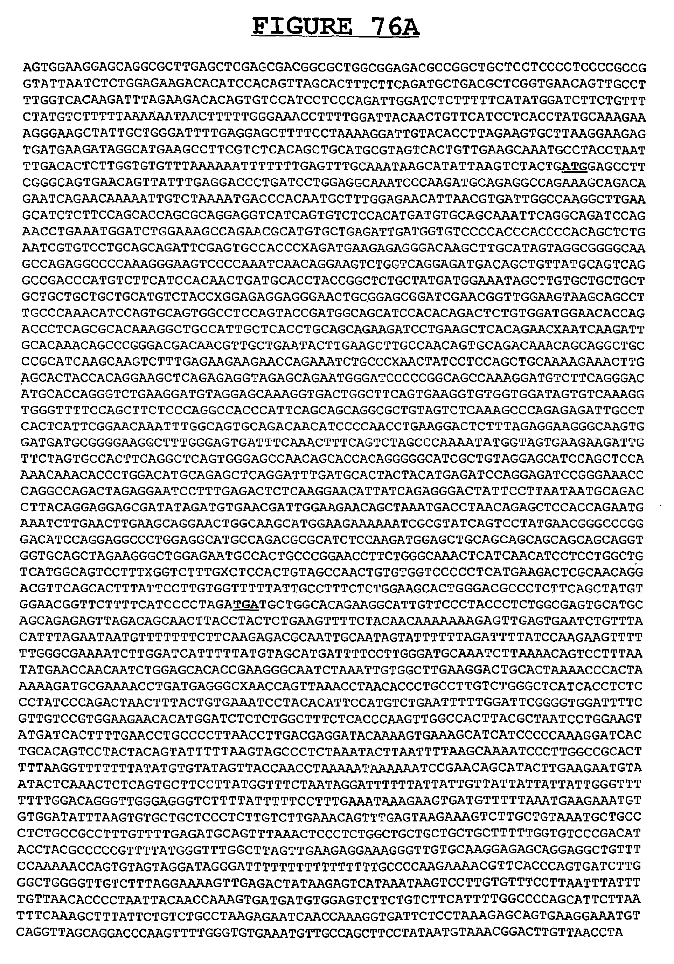

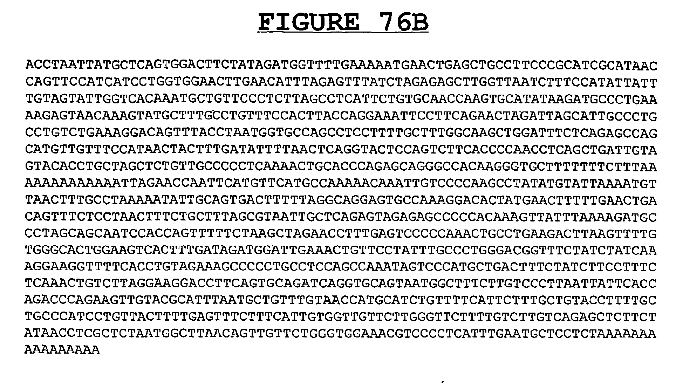

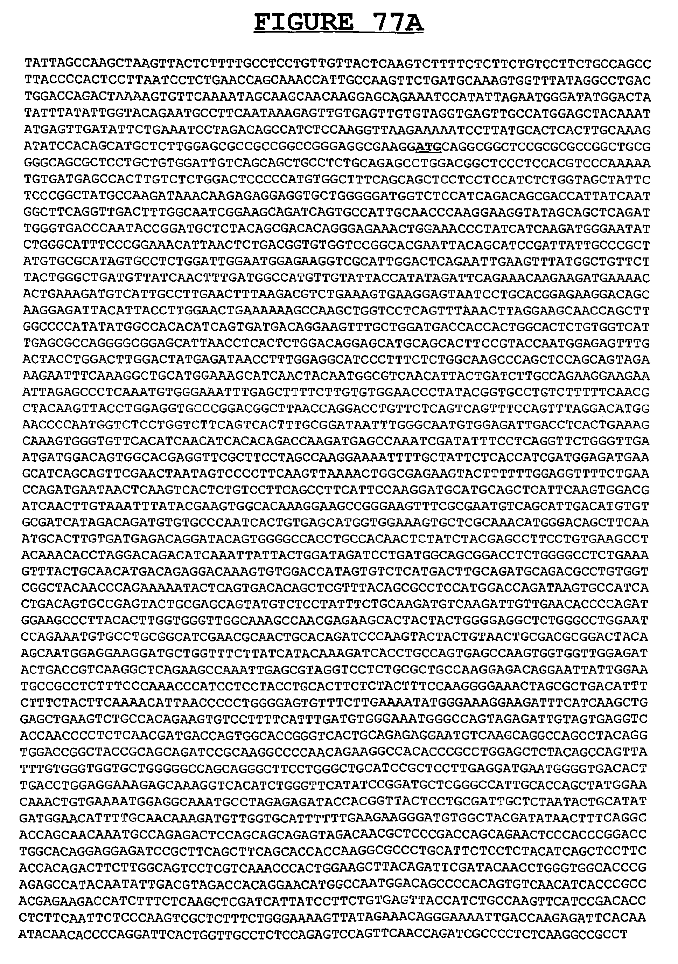

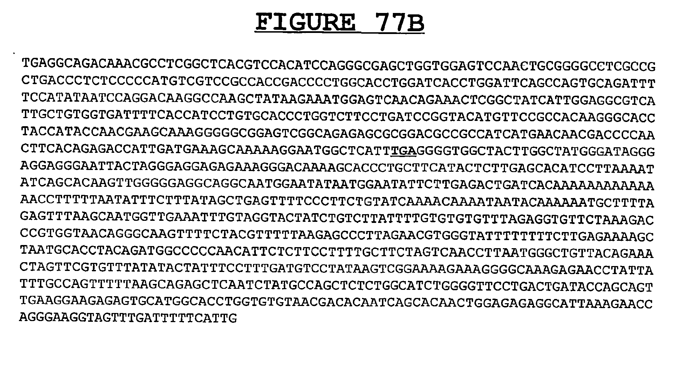

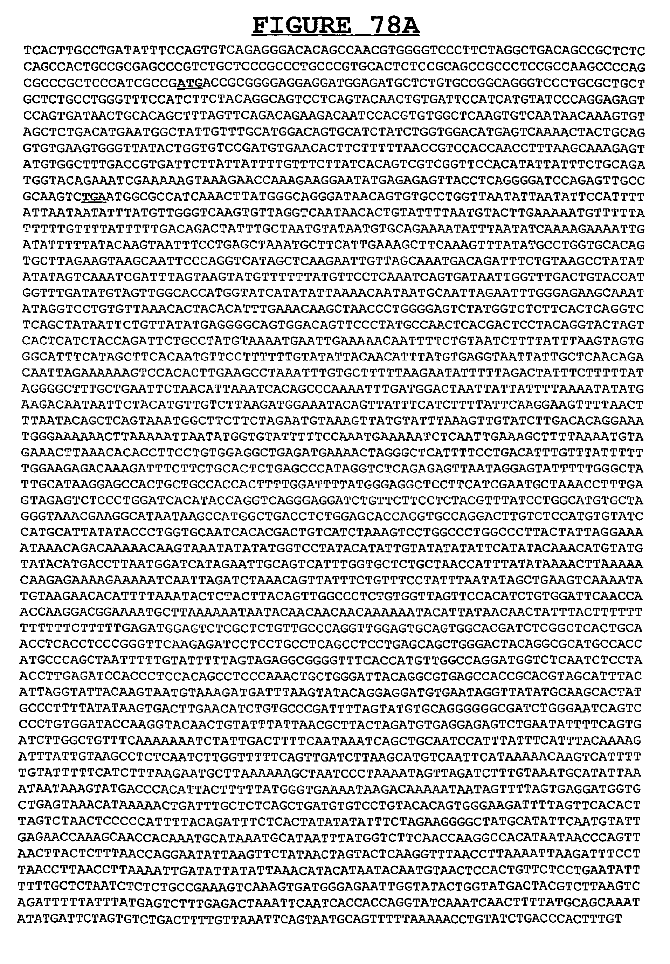

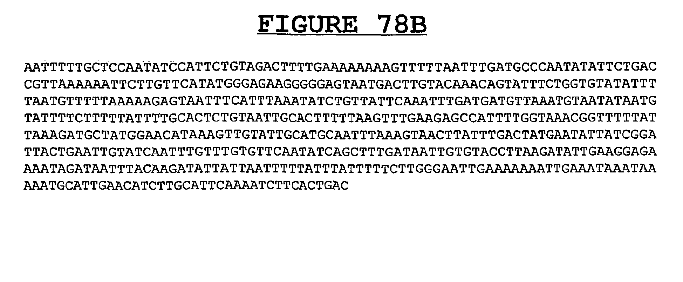

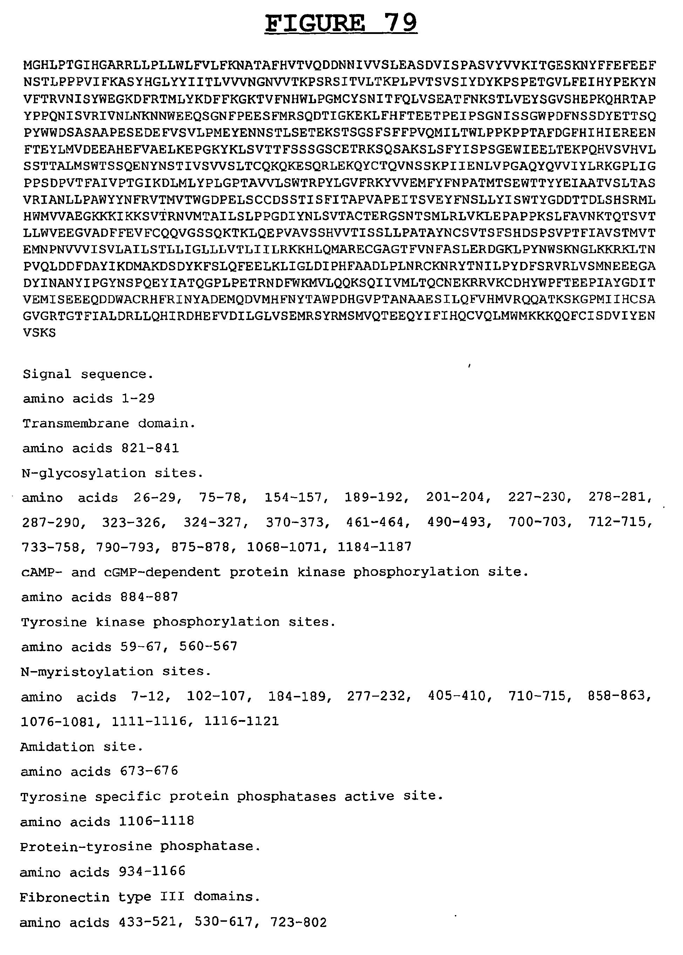

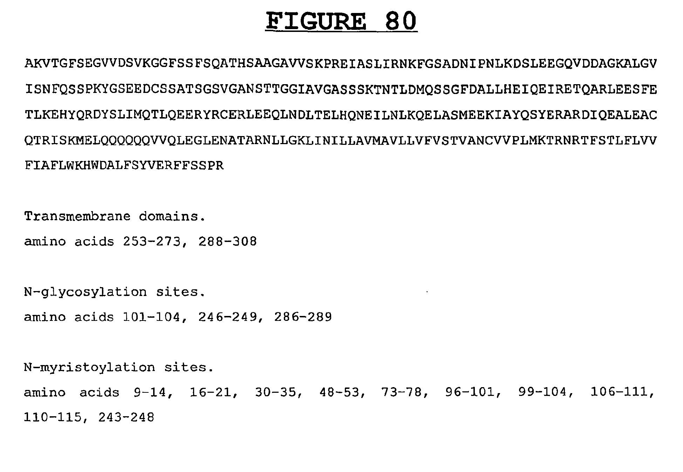

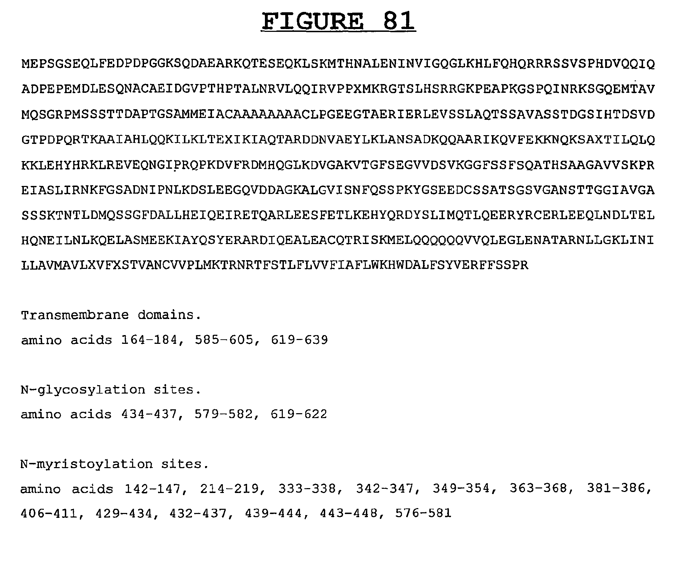

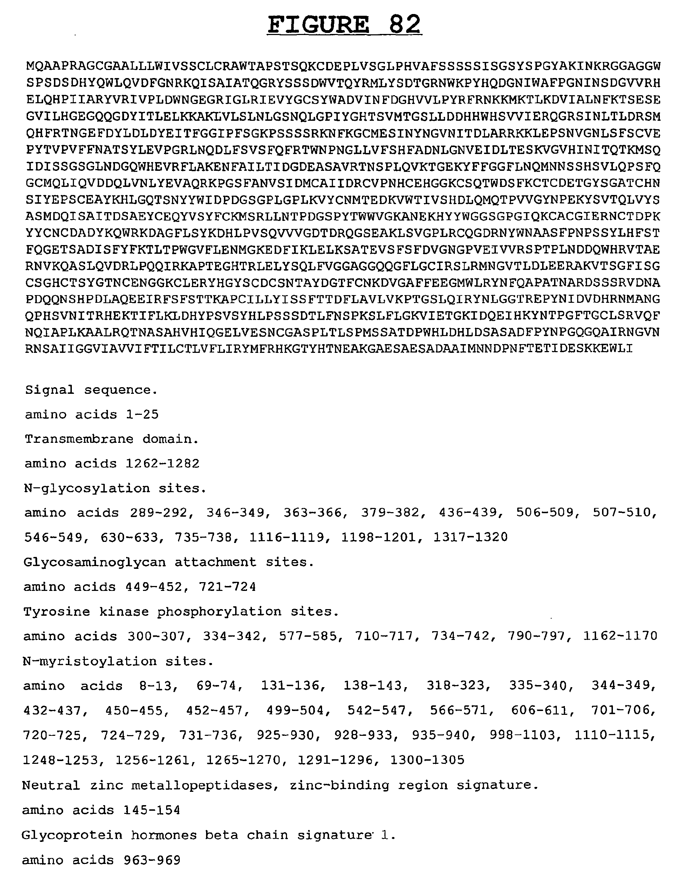

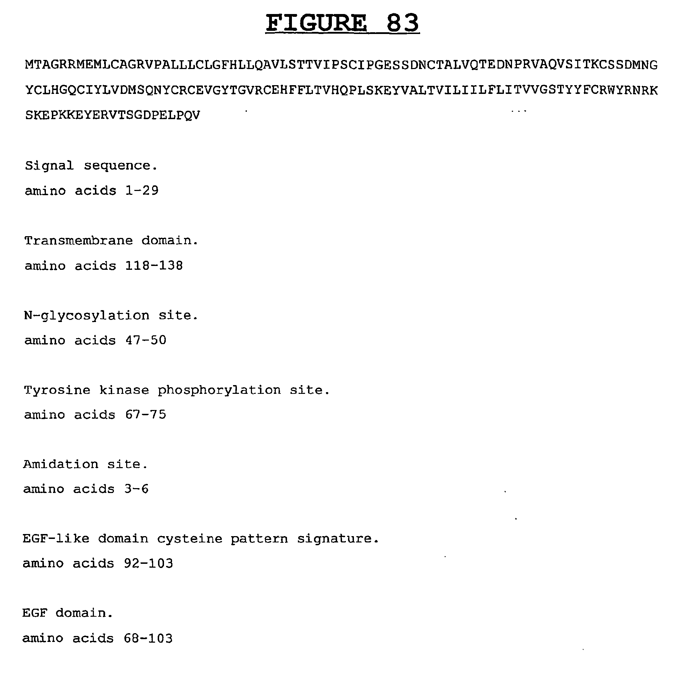

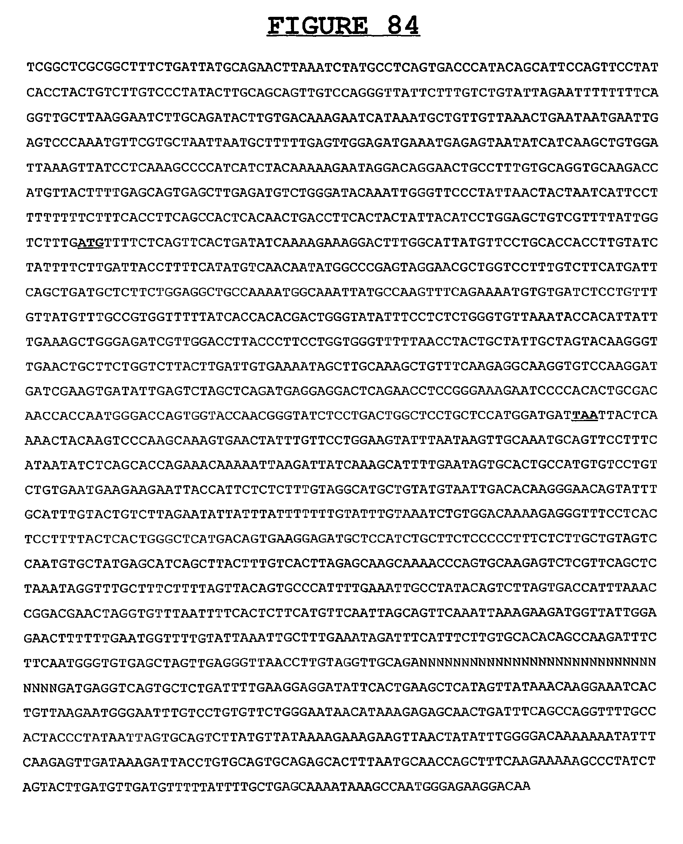

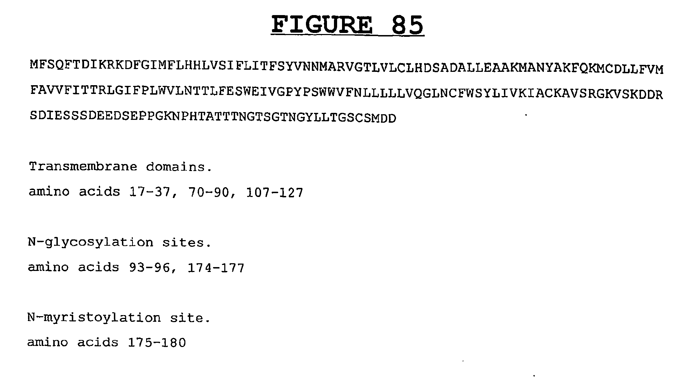

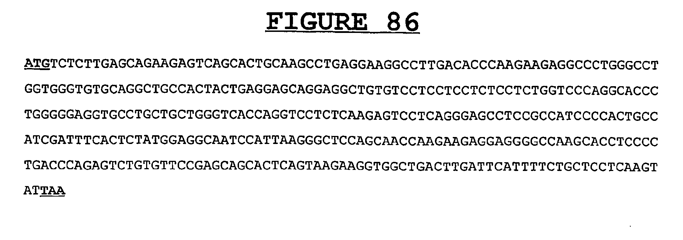

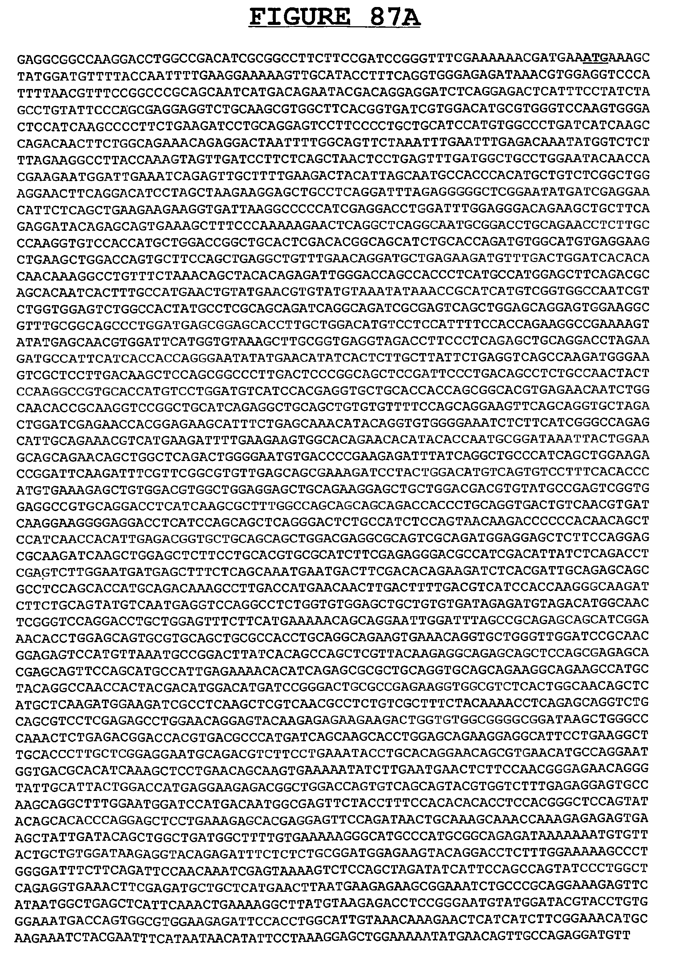

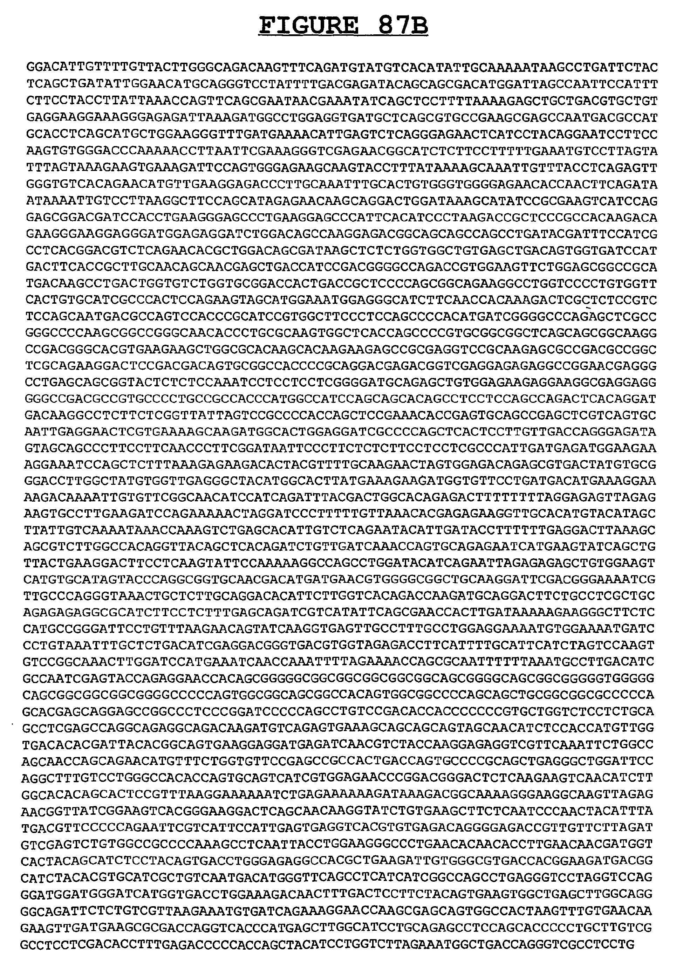

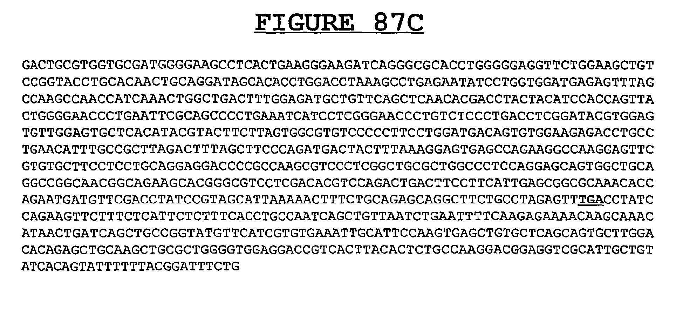

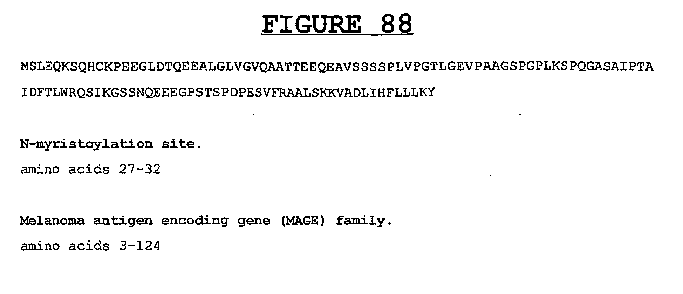

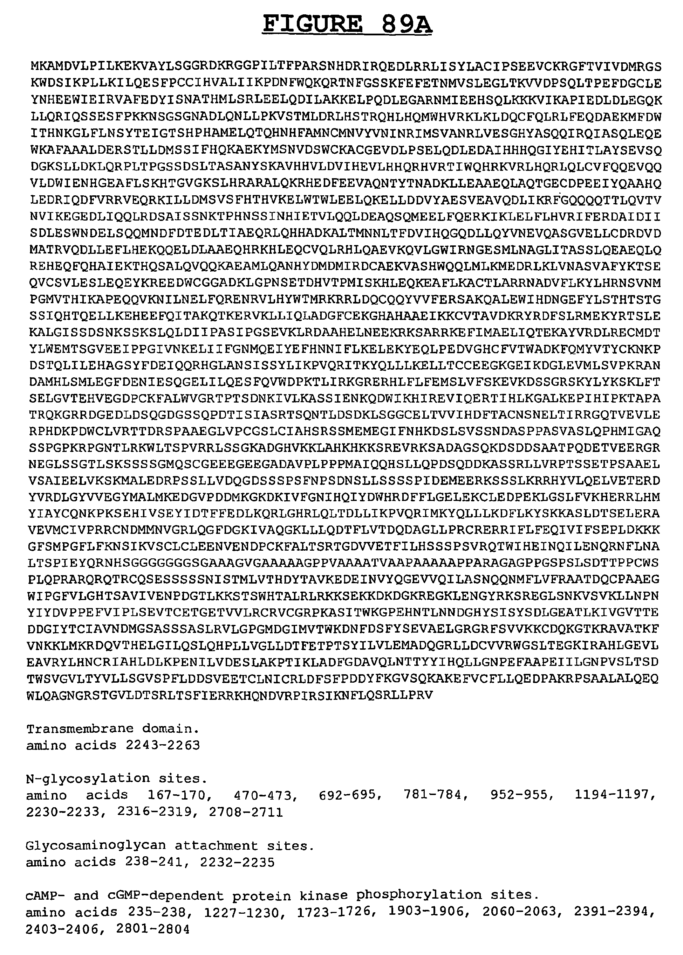

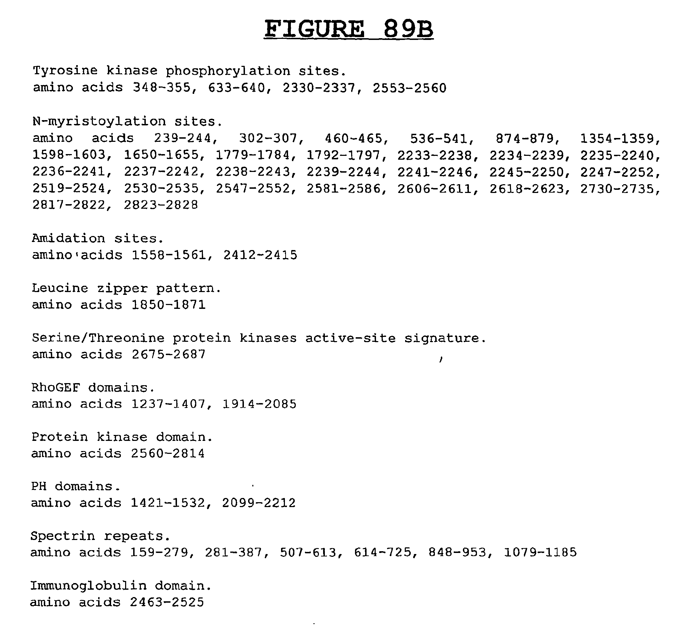

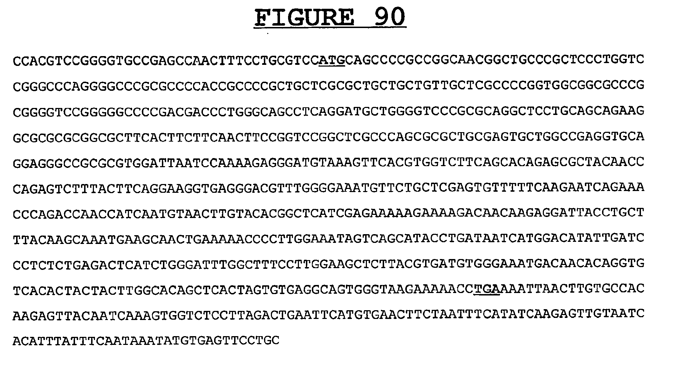

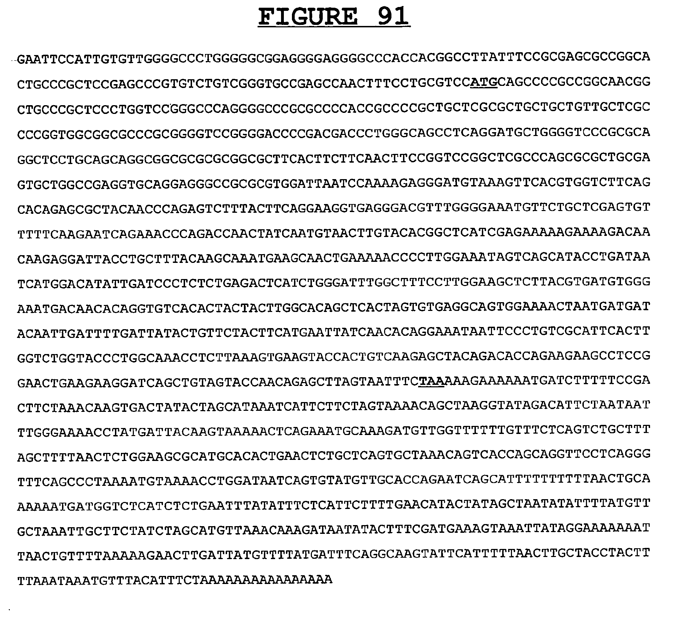

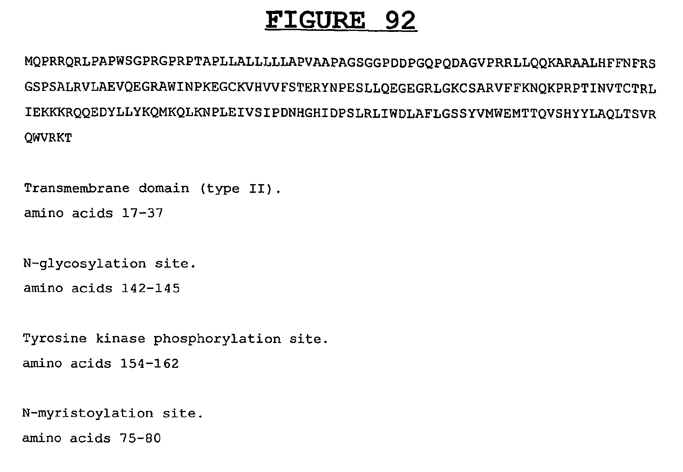

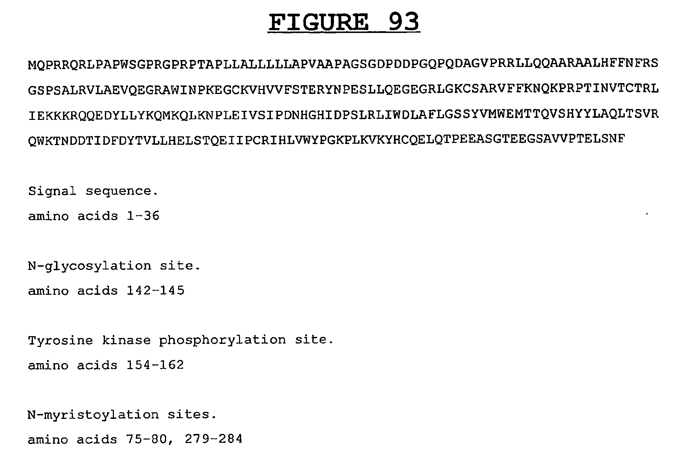

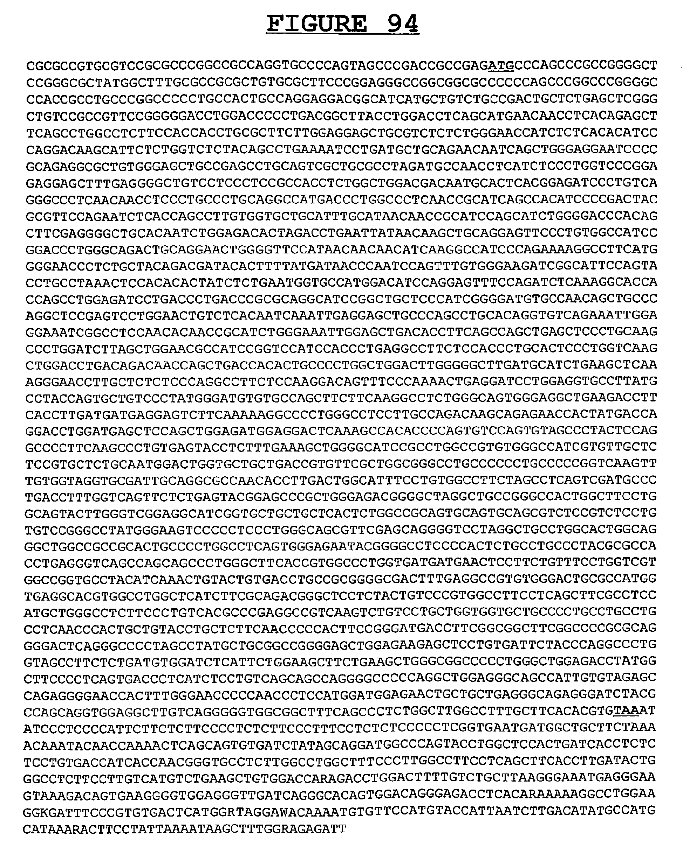

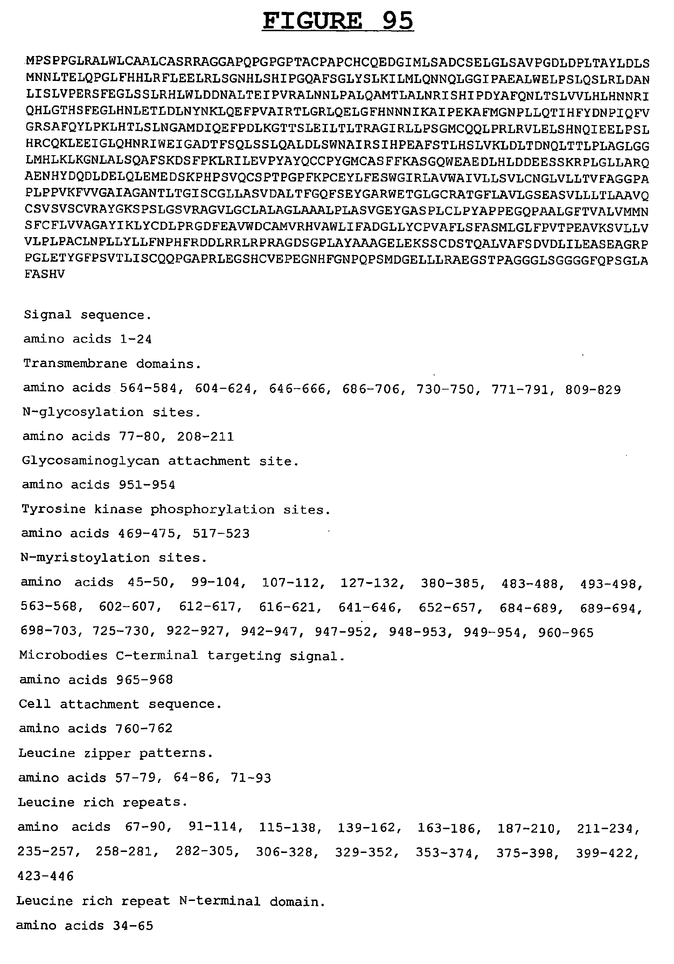

(a) a DNA molecule encoding the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95);
(b) a DNA molecule encoding the amino acid sequence shown in any one of Figures 23-41, 58-73, 79-83, 85,88,89,92,93 or 95 (SEQ ID NOS:23-41, 58-73, 79-83, 85, 88, 89, 92, 93 or 95), lacking its associated signal peptide;
(c) a DNA molecule encoding an extracellular domain of the polypeptide shown in any one of Figures 23-41,58-73,79-83,85,88,89,92,93 or 95 (SEQ ID NOS:23-41, 58-73, 79-83, 85, 88, 89, 92, 93 or 95), with its associated signal peptide;
(d) a DNA molecule encoding an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(e) the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94);
(f) the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(g) the complement of (a), (b), (c), (d), (e) or (f).

2. Isolated nucleic acid having:

(a) a nucleotide sequence that encodes the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95);
(b) a nucleotide sequence that encodes the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(c) a nucleotide sequence that encodes an extracellular domain of the polypeptide shown in any one of Figures 23-41, 58-73, 79-83, 85, 88, 89, 92, 93 or 95 (SEQ IDNOS:23-41, 58-73, 79-83, 85, 88, 89, 92, 93 or 95), with its associated signal peptide;
(d) a nucleotide sequence that encodes an extracellular domain of the polypeptide shown in any one of Figures 23-41,58-73,79-83,85,88,89,92,93 or 95 (SEQ ID NOS:23-41, 58-73, 79-83, 85, 88, 89, 92, 93 or 95), lacking its associated signal peptide;
(e) the nucleotide sequence shown in any one of Figures 1-22,42-57,74-78,84,86,87,90,91 or 94 (SEQ ID NOS: 1-22, 42-57, 74-78, 84, 86, 87, 90, 91 or 94);
(f) the full-length coding region of the nucleotide sequence shown in any one of Figures 1-22, 42-57, 74-78, 84, 86, 87, 90, 91 or 94 (SEQ ID NOS: 1-22, 42-57, 74-78, 84, 86, 87, 90, 91 or 94); or
(g) the complement of (a), (b), (c), (d), (e) or (f).

3. Isolated nucleic acid that hybridizes to:

(a) a nucleic acid that encodes the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95);
(b) a nucleic acid that encodes the amino acid sequence shown in any one of Figures 23-41, 58-73, 79-83, 85,88,89,92,93 or 95 (SEQ ID NOS:23-41, 58-73, 79-83, 85, 88, 89, 92, 93 or 95), lacking its associated signal peptide;
(c) a nucleic acid that encodes an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide;
(d) a nucleic acid that encodes an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(e) the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94);
(f) the full-length coding region of the nucleotide sequence shown in any one of Figures 1-22, 42-57, 74-78, 84,

86, 87, 90, 91 or 94 (SEQ ID NOS:1-22, 42-57, 74-78, 84, 86, 87, 90, 91 or 94); or
(g) the complement of (a), (b), (c), (d), (e) or (f).

4. The nucleic acid of Claim 3, wherein the hybridization occurs under stringent conditions.
5. The nucleic acid of Claim 3 which is at least about 5 nucleotides in length.
6. An expression vector comprising the nucleic acid of Claim 1, 2 or 3.
7. The expression vector of Claim 6, wherein said nucleic acid is operably linked to control sequences recognized by a host cell transformed with the vector.
8. A host cell comprising the expression vector of Claim 7.
9. The host cell of Claim 8 which is a CHO cell, an E. *coli* cell or a yeast cell.
10. A process for producing a polypeptide comprising culturing the host cell of Claim 8 under conditions suitable for expression of said polypeptide and recovering said polypeptide from the cell culture.
11. An isolated polypeptide having at least 80% amino acid sequence identity to:

(a) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95);
(b) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95), lacking its associated signal peptide;
(c) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide;
(d) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(e) a polypeptide encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) a polypeptide encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

12. An isolated polypeptide having:

(a) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95);
(b) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(c) an amino acid sequence of an extracellular domain of the polypeptide shown in any one of Figures 23-41,58-73,79-83,85,88,89,92,93 or 95 (SEQ ID NOS:23-41, 58-73, 79-83, 85, 88, 89, 92, 93 or 95), with its associated signal peptide sequence;
(d) an amino acid sequence of an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(e) an amino acid sequence encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) an amino acid sequence encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

13. A chimeric polypeptide comprising the polypeptide of Claim 11 or 12 fused to a heterologous polypeptide.
14. The chimeric polypeptide of Claim 13, wherein said heterologous polypeptide is an epitope tag sequence or an Fc region of an immunoglobulin.
15. An isolated antibody that binds to a polypeptide having at least 80% amino acid sequence identity to:

(a) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95);
(b) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95), lacking its associated signal peptide;
(c) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide;
(d) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;

(e) a polypeptide encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) a polypeptide encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

16. An isolated antibody that binds to a polypeptide having:

(a) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95);
(b) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(c) an amino acid sequence of an extracellular domain of the polypeptide shown in any one of Figures 23-41,58-73,79-83,85,88,89,92,93 or 95 (SEQ ID NOS:23-41, 8-73, 79-83, 85, 88, 89, 92, 93 or 5), with its associated signal peptide sequence;
(d) an amino acid sequence of an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(e) an amino acid sequence encoded by the nucleotide sequence shown in any one of Figures 1-22,42-57, 74-78, 84, 86, 87, 90, 91 or 94 (SEQ ID NOS:1-22, 42-57, 74-78, 84, 86, 87, 90, 91 or 94); or
(f) an amino acid sequence encoded by the full-length coding region of the nucleotide sequence shown inanyoneofFigures 1-22, 42-57, 74-78, 84, 86, 87, 90, 91 or 94 (SEQ ID NOS:1-22, 42-57, 74-78, 84, 86, 87, 90, 91 or 94).

17. The antibody of Claim 15 or 16 which is a monoclonal antibody.
18. The antibody of Claim 15 or 16 which is an antibody fragment.
19. The antibody of Claim 15 or 16 which is a chimeric or a humanized antibody.
20. The antibody of Claim 15 or 16 which is conjugated to a growth inhibitory agent.
21. The antibody of Claim 15 or 16 which is conjugated to a cytotoxic agent.
22. The antibody of Claim 21, wherein the cytotoxic agent is selected from the group consisting of toxins, antibiotics, radioactive isotopes and nucleolytic enzymes.
23. The antibody of Claim 21, wherein the cytotoxic agent is a toxin.
24. The antibody of Claim 23, wherein the toxin is selected from the group consisting of maytansinoid and calicheamicin.
25. The antibody of Claim 23, wherein the toxin is a maytansinoid.
26. The antibody of Claim 15 or 16 which is produced in bacteria.
27. The antibody of Claim 15 or 16 which is produced in CHO cells.
28. The antibody of Claim 15 or 16 which induces death of a cell to which it binds.
29. The antibody of Claim 15 or 16 which is detectably labeled.
30. An isolated nucleic acid having a nucleotide sequence that encodes the antibody of Claim 15 or 16.
31. An expression vector comprising the nucleic acid of Claim 30 operably linked to control sequences recognized by a host cell transformed with the vector.
32. A host cell comprising the expression vector of Claim 31.
33. The host cell of Claim 32 which is a CHO cell, an E. *coli* cell or a yeast cell.
34. A process for producing an antibody comprising culturing the host cell of Claim 32 under conditions suitable for expression of said antibody and recovering said antibody from the cell culture.
35. An isolated oligopeptide that binds to a polypeptide having at least 80% amino acid sequence identity to:

(a) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95);
(b) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95), lacking its associated signal peptide;
(c) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide;
(d) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(e) a polypeptide encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) a polypeptide encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

36. An isolated oligopeptide that binds to a polypeptide having:

(a) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95);
(b) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(c) an amino acid sequence of an extracellular domain of the polypeptide shown in any one of Figures 23-41,58-73,79-83,85,88,89,92,93 or 95 (SEQ ID NOS:23-41, 58-73, 79-83, 85, 88, 89, 92, 93 or 95), with its associated signal peptide sequence;
(d) an amino acid sequence of an extracellular domain of the polypeptide shown in any one of Figures 23-41,58-73,79-83,85,88,89,92,93 or 95 (SEQ ID NOS:23-41, 58-73, 79-83, 85, 88, 89, 92, 93 or 95), lacking its associated signal peptide sequence;
(e) an amino acid sequence encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) an amino acid sequence encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

37. The oligopeptide of Claim 35 or 36 which is conjugated to a growth inhibitory agent.
38. The oligopeptide of Claim 35 or 36 which is conjugated to a cytotoxic agent.
39. The oligopeptide of Claim 3 8, wherein the cytotoxic agent is selected from the group consisting of toxins, antibiotics, radioactive isotopes and nucleolytic enzymes.
40. The oligopeptide of Claim 38, wherein the cytotoxic agent is a toxin.
41. The oligopeptide of Claim 40, wherein the toxin is selected from the group consisting of maytansinoid and calicheamicin.
42. The oligopeptide of Claim 40, wherein the toxin is a maytansinoid.
43. The oligopeptide of Claim 35 or 36 which induces death of a cell to which it binds.
44. The oligopeptide of Claim 35 or 36 which is detectably labeled.
45. A TAT binding organic molecule that binds to a polypeptide having at least 80% amino acid sequence identity to:

(a) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95);
(b) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95), lacking its associated signal peptide;
(c) an extracellular domain of the polypeptide shown in any one of Figures 23-41,58-73,79-83,85,88,89, 92, 93 or 95 (SEQ ID NOS:23-41, 58-73, 79-83, 85, 88, 89, 92, 93 or 95), with its associated signal peptide;
(d) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(e) a polypeptide encoded by the nucleotide sequence shown in any one of Figures 1-22, 42-57, 74-78, 84, 86, 87, 90, 91 or 94 (SEQ ID NOS: 1-22, 42-57, 74-78, 84, 86, 87, 90, 91 or 94); or
(f) a polypeptide encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

46. The organic molecule of Claim 45 that binds to a polypeptide having:

(a) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95);
(b) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(c) an amino acid sequence of an extracellular domain of the polypeptide shown in any one of Figures 23-41,58-73,79-83,85,88,89,92,93 or 95 (SEQ ID NOS:23-41, 58-73, 79-83, 85, 88, 89, 92, 93 or 95), with its associated signal peptide sequence;
(d) an amino acid sequence of an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(e) an amino acid sequence encoded by the nucleotide sequence shown in any one of Figures 1-22, 42-57,

74-78, 84, 86, 87, 90, 91 or 94 (SEQ ID NOS: 1-22, 42-57, 74-78, 84, 86, 87, 90, 91 or 94); or

(f) an amino acid sequence encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

47. The organic molecule of Claim 45 or 46 which is conjugated to a growth inhibitory agent.
48. The organic molecule of Claim 45 or 46 which is conjugated to a cytotoxic agent.
49. The organic molecule of Claim 48, wherein the cytotoxic agent is selected from the group consisting of toxins, antibiotics, radioactive isotopes and nucleolytic enzymes.
50. The organic molecule of Claim 48, wherein the cytotoxic agent is a toxin.
51. The organic molecule of Claim 50, wherein the toxin is selected from the group consisting of maytansinoid and calicheamicin.
52. The organic molecule of Claim 50, wherein the toxin is a maytansinoid.
53. The organic molecule of Claim 45 or 46 which induces death of a cell to which it binds.
54. The organic molecule of Claim 45 or 46 which is detectably labeled.
55. A composition of matter comprising:

(a) the polypeptide of Claim 11;
(b) the polypeptide of Claim 12;
(c) the chimeric polypeptide of Claim 13;
(d) the antibody of Claim 15;
(e) the antibody of Claim 16;
(f) the oligopeptide of Claim 35;
(g) the oligopeptide of Claim 36;
(h) the TAT binding organic molecule of Claim 45; or
(i) the TAT binding organic molecule of Claim 46; in combination with a carrier.

56. The composition of matter of Claim 55, wherein said carrier is a pharmaceutically acceptable carrier.
57. An article of manufacture comprising:

(a) a container; and
(b) the composition of matter of Claim 55 contained within said container.

58. The article of manufacture of Claim 57 further comprising a label affixed to said container, or a package insert included with said container, referring to the use of said composition of matter for the therapeutic treatment of or the diagnostic detection of a cancer.
59. A method of inhibiting the growth of a cell that expresses a protein having at least 80% amino acid sequence identity to:

(a) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95);
(b) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95), lacking its associated signal peptide;
(c) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide;
(d) an extracellular domain of the polypeptide shown in any one of Figures 23-41, 58-73,79-83, 85, 88, 89, 92, 93 or 95 (SEQ ID NOS:23-41, 58-73, 79-83, 85, 88, 89, 92, 93 or 95), lacking its associated signal peptide;
(e) a polypeptide encoded by the nucleotide sequence shown in any one of Figures 1-22,42-57, 74-78, 84, 86, 87, 90, 91 or 94 (SEQ ID NOS:1-22, 42-57, 74-78, 84, 86, 87, 90, 91 or 94); or
(f) a polypeptide encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94), said method comprising contacting said cell with an antibody, oligopeptide or organic molecule that binds to said protein, the binding of said antibody, oligopeptide or organic molecule to said protein thereby causing an inhibition of growth of said cell.

60. The method of Claim 59, wherein said antibody is a monoclonal antibody.
61. The method of Claim 59, wherein said antibody is an antibody fragment.
62. The method of Claim 59, wherein said antibody is a chimeric or a humanized antibody.

63. The method of Claim 59, wherein said antibody, oligopeptide or organic molecule is conjugated to a growth inhibitory agent.

64. The method of Claim 59, wherein said antibody, oligopeptide or organic molecule is conjugated to a cytotoxic agent.

65. The method of Claim 64, wherein said cytotoxic agent is selected from the group consisting of toxins, antibiotics, radioactive isotopes and nucleolytic enzymes.

66. The method of Claim 64, wherein the cytotoxic agent is a toxin.

67. The method of Claim 66, wherein the toxin is selected from the group consisting ofmaytansinoid and calicheamicin.

68. The method of Claim 66, wherein the toxin is a maytansinoid.

69. The method of Claim 59, wherein said antibody is produced in bacteria.

70. The method of Claim 59, wherein said antibody is produced in CHO cells.

71. The method of Claim 59, wherein said cell is a cancer cell.

72. The method of Claim 71, wherein said cancer cell is further exposed to radiation treatment or a chemotherapeutic agent.

73. The method of Claim 71, wherein said cancer cell is selected from the group consisting of a breast cancer cell, a colorectal cancer cell, a lung cancer cell, an ovarian cancer cell, a central nervous system cancer cell, a liver cancer cell, a bladder cancer cell, a pancreatic cancer cell, a cervical cancer cell, a melanoma cell and a leukemia cell.

74. The method of Claim 71, wherein said protein is more abundantly expressed by said cancer cell as compared to a normal cell of the same tissue origin.

75. The method of Claim 59 which causes the death of said cell.

76. The method of Claim 59, wherein said protein has:

(a) the amino acid sequence shown in any one of Figures 23-41,58-73,79-83,85,88,89,92,93 or 95 (SEQ ID NOS:23-41, 58-73, 79-83, 85, 88, 89, 92, 93 or 95);

(b) the amino acid sequence shown in any one of Figures 23-41, 58-73, 79-83, 85, 88, 89, 92,93 or 95 (SEQ ID NOS:23-41, 58-73, 79-83, 85, 88, 89, 92, 93 or 95), lacking its associated signal peptide sequence;

(c) an amino acid sequence of an extracellular domain of the polypeptide shown in any one of Figures 23-41,58-73,79-83,85,88,89,92,93 or 95 (SEQ ID NOS:23-41, 58-73, 79-83, 85, 88, 89, 92, 93 or 95), with its associated signal peptide sequence;

(d) an amino acid sequence of an extracellular domain of the polypeptide shown in any one of Figures 23-41,58-73,79-83,85,88,89,92,93 or 95 (SEQ ID NOS:23-41, 58-73, 79-83, 85, 88, 89, 92, 93 or 95), lacking its associated signal peptide sequence;

(e) an amino acid sequence encoded by the nucleotide sequence shown in any one of Figures 1-22,42-57, 74-78, 84, 86, 87, 90, 91 or 94 (SEQ ID NOS:1-22, 42-57, 74-78, 84, 86, 87, 90, 91 or 94); or

(f) an amino acid sequence encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

77. A method of therapeutically treating a mammal having a cancerous tumor comprising cells that express a protein having at least 80% amino acid sequence identity to:

(a) the polypeptide shown in any one of Figures 23-41, 58-73, 79-83, 85, 88, 89, 92, 93 or 95 (SEQ YID NOS: 23-4.1, 58-73, 79-83, 85, 88, 89, 92, 93 or 95);

(b) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95), lacking its associated signal peptide;

(c) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide;

(d) an extracellular domain of the polypeptide shown in any one of Figures 23-41, 58-73, 79-83, 85, 88, 89, 92,93 or 95 (SEQ ID NOS:23-41, 58-73, 79-83, 85, 88, 89, 92, 93 or 95), lacking its associated signal peptide;

(e) a polypeptide encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or

(f) a polypeptide encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94), said method comprising administering to said mammal a therapeutically effective amount of an antibody, oligopeptide or organic molecule that binds to said protein, thereby effectively treating said mammal.

78. The method of Claim 77, wherein said antibody is a monoclonal antibody.

79. The method of Claim 77, wherein said antibody is an antibody fragment.

80. The method of Claim 77, wherein said antibody is a chimeric or a humanized antibody.
81. The method of Claim 77, wherein said antibody, oligopeptide or organic molecule is conjugated to a growth inhibitory agent.
82. The method of Claim 77, wherein said antibody, oligopeptide or organic molecule is conjugated to a cytotoxic agent.
83. The method of Claim 82, wherein said cytotoxic agent is selected from the group consisting of toxins, antibiotics, radioactive isotopes and nucleolytic enzymes.
84. The method of Claim 82, wherein the cytotoxic agent is a toxin.
85. The method of Claim 84, wherein the toxin is selected from the group consisting of maytansinoid and calicheamicin.
86. The method of Claim 84, wherein the toxin is a maytansinoid.
87. The method of Claim 77, wherein said antibody is produced in bacteria.
88. The method of Claim 77, wherein said antibody is produced in CHO cells.
89. The method of Claim 77, wherein said tumor is further exposed to radiation treatment or a chemotherapeutic agent.
90. The method of Claim 77, wherein said tumor is a breast tumor, a colorectal tumor, a lung tumor, an ovarian tumor, a central nervous system tumor, a liver tumor, a bladder tumor, a pancreatic tumor, or a cervical tumor.
91. The method of Claim 77, wherein said protein is more abundantly expressed by the cancerous cells of said tumor as compared to a normal cell of the same tissue origin.
92. The method of Claim 77, wherein said protein has:

(a) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95);
(b) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(c) an amino acid sequence of an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide sequence;
(d) an amino acid sequence of an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(e) an amino acid sequence encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) an amino acid sequence encoded by the full-length coding region of the nucleotide sequence shown in any one ofFigures 1-22, 42-57, 74-78, 84, 86, 87, 90, 91 or 94 (SEQ ID NOS:1-22, 42-57, 74-78, 84, 86, 87, 90, 91 or 94).

93. A method of determining the presence of a protein in a sample suspected of containing said protein, wherein said protein has at least 80% amino acid sequence identity to:

(a) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95);
(b) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95), lacking its associated signal peptide;
(c) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide;
(d) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(e) a polypeptide encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) a polypeptide encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94), said method comprising exposing said sample to an antibody, oligopeptide or organic molecule that binds to said protein and determining binding of said antibody, oligopeptide or organic molecule to said protein in said sample,

wherein binding of the antibody, oligopeptide or organic molecule to said protein is indicative of the presence of said protein in said sample.
94. The method of Claim 93, wherein said sample comprises a cell suspected of expressing said protein.

95. The method of Claim 94, wherein said cell is a cancer cell.
96. The method of Claim 93, wherein said antibody, oligopeptide or organic molecule is detectably labeled.
97. The method of Claim 93, wherein said protein has:

(a) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95);
(b) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(c) an amino acid sequence of an extracellular domain of the polypeptide shown in any one of Figures 23-41,58-73,79-83,85,88,89,92,93 or 95 (SEQ ID NOS:23-41, 58-73, 79-83, 85, 88, 89, 92, 93 or 95), with its associated signal peptide sequence;
(d) an amino acid sequence of an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(e) an amino acid sequence encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) an amino acid sequence encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

98. A method of diagnosing the presence of a tumor in a mammal, said method comprising determining the level of expression of a gene encoding a protein having at least 80% amino acid sequence identity to:

(a) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95);
(b) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95), lacking its associated signal peptide;
(c) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide;
(d) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(e) a polypeptide encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) a polypeptide encoded by the full-length coding region of the nucleotide sequence shown in any one of Figures 1-22,42-57,74-78,84,86,87,90,91 or 94 (SEQ ID NOS:1-22, 42-57, 74-78, 84, 86, 87, 90, 91 or 94), in a test sample of tissue cells obtained from said mammal and in a control sample of known normal cells of the same tissue origin, wherein a higher level of expression of said protein in the test sample, as compared to the control sample, is indicative of the presence of tumor in the mammal from which the test sample was obtained.

99. The method of Claim 98, wherein the step of determining the level of expression of a gene encoding said protein comprises employing an oligonucleotide in an *in situ* hybridization or RT-PCR analysis.
100. The method of Claim 98, wherein the step determining the level of expression of a gene encoding said protein comprises employing an antibody in an immunohistochemistry or Western blot analysis.
101. The method of Claim 98, wherein said protein has:

(a) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95);
(b) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(c) an amino acid sequence of an extracellular domain of the polypeptide shown in any one of Figures 23-41,58-73,79-83,85,88,89,92,93 or 95 (SEQ ID NOS:23-41, 58-73, 79-83, 85, 88, 89, 92, 93 or 95), with its associated signal peptide sequence;
(d) an amino acid sequence of an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(e) an amino acid sequence encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) an amino acid sequence encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

102. A method of diagnosing the presence of a tumor in a mammal, said method comprising contacting a test sample of tissue cells obtained from said mammal with an antibody, oligopeptide or organic molecule that binds to a protein having at least 80% amino acid sequence identity to:

(a) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95);
(b) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95), lacking its associated signal peptide;
(c) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide;
(d) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(e) a polypeptide encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) a polypeptide encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94), and detecting the formation of a complex between said antibody, oligopeptide or organic molecule and said protein in the test sample, wherein the formation of a complex is indicative of the presence of a tumor in said mammal.

103. The method of Claim 102, wherein said antibody, oligopeptide or organic molecule is detectably labeled.
104. The method of Claim 102, wherein said test sample of tissue cells is obtained from an individual suspected of having a cancerous tumor.
105. The method of Claim 102, wherein said protein has:

(a) the amino acid sequence shown in any one of Figures 23-41, 58-73, 79-83, 85, 88, 89, 92, 93 or 95 (SEQ IDNOS:23-41, 58-73, 79-83, 85, 88, 89, 92, 93 or 95);
(b) the amino acid sequence shown in any one of Figures 23-41, 58-73, 79-83, 85, 88, 89, 92, 93 or 95 (SEQ IDNOS:23-41,58-73,79-83, 85, 88, 89,92, 93 or 95), lacking its associated signal peptide sequence;
(c) an amino acid sequence of an extracellular domain of the polypeptide shown in any one of Figures 23-41,58-73,79-83,85,88,89,92,93 or 95 (SEQ IDNOS:23-41,58-73,79-83, 85,88,89,92,93 or 95), with its associated signal peptide sequence;
(d) an amino acid sequence of an extracellular domain of the polypeptide shown in any one of Figures 23-41,58-73,79-83,85,88,89,92,93 or 95 (SEQ ID NOS:23-41, 58-73, 79-83, 85, 88, 89, 92, 93 or 95), lacking its associated signal peptide sequence;
(e) an amino acid sequence encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) an amino acid sequence encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

106. A method for treating or preventing a cell proliferative disorder associated with increased expression or activity of a protein having at least 80% amino acid sequence identity to:

(a) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95);
(b) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95), lacking its associated signal peptide;
(c) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide;
(d) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(e) a polypeptide encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) a polypeptide encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94), said method comprising administering to a subject in need of such treatment an effective amount of an antagonist of said protein, thereby effectively treating or preventing said cell proliferative disorder.

107. The method of Claim 106, wherein said cell proliferative disorder is cancer.
108. The method of Claim 106, wherein said antagonist is an anti-TAT polypeptide antibody, TAT binding oligopeptide, TAT binding organic molecule or antisense oligonucleotide.
109. A method of binding an antibody, oligopeptide or organic molecule to a cell that expresses a protein having at least 80% amino acid sequence identity to:

(a) the polypeptide shown in any one of Figures 23-41, 58-73, 79-83, 85, 88, 89, 92, 93 or 95 (SEQ ID NOS: 23-41, 58-73,79-83, 85, 88, 89, 92,93 or 95);
(b) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95), lacking its associated signal peptide;
(c) an extracellular domain of the polypeptide shown in any one of Figures 23-41, 58-73, 79-83; 85, 88, 89, 92, 93 or 95 (SEQ ID NOS:23-41, 58-73, 79-83, 85, 88, 89, 92, 93 or 95), with its associated signal peptide;
(d) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(e) a polypeptide encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) a polypeptide encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94), said method comprising contacting said cell with an antibody, oligopeptide or organic molecule that binds to said protein and allowing the binding of the antibody, oligopeptide or organic molecule to said protein to occur, thereby binding said antibody, oligopeptide or organic molecule to said cell.

110. The method of Claim 109, wherein said antibody is a monoclonal antibody.
111. The method of Claim 109, wherein said antibody is an antibody fragment.
112. The method of Claim 109, wherein said antibody is a chimeric or a humanized antibody.
113. The method of Claim 109, wherein said antibody, oligopeptide or organic molecule is conjugated to a growth inhibitory agent.
114. The method of Claim 109, wherein said antibody, oligopeptide or organic molecule is conjugated to a cytotoxic agent.
115. The method of Claim 114, wherein said cytotoxic agent is selected from the group consisting of toxins, antibiotics, radioactive isotopes and nucleolytic enzymes.
116. The method of Claim 114, wherein the cytotoxic agent is a toxin.
117. The method of Claim 116, wherein the toxin is selected from the group consisting of maytansinoid and calicheamicin.
118. The method of Claim 116, wherein the toxin is a maytansinoid.
119. The method of Claim 109, wherein said antibody is produced in bacteria.
120. The method of Claim 109, wherein said antibody is produced in CHO cells.
121. The method of Claim 109, wherein said cell is a cancer cell.
122. The method of Claim 121, wherein said cancer cell is further exposed to radiation treatment or a chemotherapeutic agent.
123. The method of Claim 121, wherein said cancer cell is selected from the group consisting of a breast cancer cell, a colorectal cancer cell, a lung cancer cell, an ovarian cancer cell, a central nervous system cancer cell, a liver cancer cell, a bladder cancer cell, a pancreatic cancer cell, a cervical cancer cell, a melanoma cell and a leukemia cell.
124. The method of Claim 123, wherein said protein is more abundantly expressed by said cancer cell as compared to a normal cell of the same tissue origin.
125. The method of Claim 109 which causes the death of said cell.
126. Use of a nucleic acid as claimed in any of Claims 1 to 5 or 30 in the preparation of a medicament for the therapeutic treatment or diagnostic detection of a cancer.
127. Use of a nucleic acid as claimed in any of Claims 1 to 5 or 30 in the preparation of a medicament for treating a tumor.
128. Use of a nucleic acid as claimed in any of Claims 1 to 5 or 30 in the preparation of a medicament for treatment or prevention of a cell proliferative disorder.
129. Use of an expression vector as claimed in any of Claims 6, 7 or 31 in the preparation of a medicament for the therapeutic treatment or diagnostic detection of a cancer.

130. Use of an expression vector as claimed in any of Claims 6, 7 or 31 in the preparation of medicament for treating a tumor.
131. Use of an expression vector as claimed in any of Claims 6, 7 or 31 in the preparation of a medicament for treatment or prevention of a cell proliferative disorder.
132. Use of a host cell as claimed in any of Claims 8, 9, 32, or 33 in the preparation of a medicament for the therapeutic treatment or diagnostic detection of a cancer.
133. Use of a host cell as claimed in any of Claims 8, 9, 32 or 33 in the preparation of a medicament for treating a tumor.
134. Use of a host cell as claimed in any of Claims 8, 9, 32 or 33 in the preparation of a medicament for treatment or prevention of a cell proliferative disorder.
135. Use of a polypeptide as claimed in any of Claims 11 to 14 in the preparation of a medicament for the therapeutic treatment or diagnostic detection of a cancer.
136. Use of a polypeptide as claimed in any of Claims 11 to 14 in the preparation of a medicament for treating a tumor.
137. Use of a polypeptide as claimed in any of Claims 11 to 14 in the preparation of a medicament for treatment or prevention of a cell proliferative disorder.
138. Use of an antibody as claimed in any of Claims 15 to 29 in the preparation of a medicament for the therapeutic treatment or diagnostic detection of a cancer.
139. Use of an antibody as claimed in any of Claims 15 to 29 in the preparation of a medicament for treating a tumor.
140. Use of an antibody as claimed in any of Claims 15 to 29 in the preparation of a medicament for treatment or prevention of a cell proliferative disorder.
141. Use of an oligopeptide as claimed in any of Claims 35 to 44 in the preparation of a medicament for the therapeutic treatment or diagnostic detection of a cancer.
142. Use of an oligopeptide as claimed in any of Claims 35 to 44 in the preparation of a medicament for treating a tumor.
143. Use of an oligopeptide as claimed in any of Claims 35 to 44 in the preparation of a medicament for treatment or prevention of a cell proliferative disorder.
144. Use of a TAT binding organic molecule as claimed in any of Claims 45 to 54 in the preparation of a medicament for the therapeutic treatment or diagnostic detection of a cancer.
145. Use of a TAT binding organic molecule as claimed in any of Claims 45 to 54 in the preparation of a medicament for treating a tumor.
146. Use of a TAT binding organic molecule as claimed in any of Claims 45 to 54 in the preparation of a medicament for treatment.or prevention of a cell proliferative disorder.
147. Use of a composition of matter as claimed in any of Claims 55 or 56 in the preparation of a medicament for the therapeutic treatment or diagnostic detection of a cancer.
148. Use of a composition of matter as claimed in any of Claims 55 or 56 in the preparation of a medicament for treating a tumor.
149. Use of a composition of matter as claimed in any of Claims 55 or 56 in the preparation of a medicament for treatment or prevention of a cell proliferative disorder.
150. Use of an article of manufacture as claimed in any of Claims 57 or 58 in the preparation of a medicament for the therapeutic treatment or diagnostic detection of a cancer.
151. Use of an article of manufacture as claimed in any of Claims 57 or 58 in the preparation of a medicament for treating a tumor.
152. Use of an article of manufacture as claimed in any of Claims 57 or 58 in the preparation of a medicament for treatment or prevention of a cell proliferative disorder.
153. A method for inhibiting the growth of a cell, wherein the growth of said cell is at least in part dependent upon a growth potentiating effect of a protein having at least 80% amino acid sequence identity to:

(a) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95);
(b) the polypeptide shown in any one of Figures 23-41, 58-73, 79-83, 85, 88, 89, 92, 93 or 95 (SEQ LID NOS: 23-41, 58-73, 79-83, 85, 88, 89, 92, 93 or 95), lacking its associated signal peptide;
(c) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide;
(d) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(e) a polypeptide encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) a polypeptide encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94),
said method comprising contacting said protein with an antibody, oligopeptide or organic molecule that binds to said protein, there by inhibiting the growth of said cell.

154. The method of Claim 153, wherein said cell is a cancer cell.
155. The method of Claim 153, wherein said protein is expressed by said cell.
156. The method of Claim 153, wherein the binding of said antibody, oligopeptide or organic molecule to said protein antagonizes a cell growth-potentiating activity of said protein.
157. The method of Claim 153, wherein the binding of said antibody, oligopeptide or organic molecule to said protein induces the death of said cell.
158. The method of Claim 153, wherein said antibody is a monoclonal antibody.
159. The method of Claim 153, wherein said antibody is an antibody fragment.
160. The method of Claim 153, wherein said antibody is a chimeric or a humanized antibody.
161. The method of Claim 153, wherein said antibody, oligopeptide or organic molecule is conjugated to a growth inhibitory agent.
162. The method of Claim 153, wherein said antibody, oligopeptide or organic molecule is conjugated to a cytotoxic agent.
163. The method of Claim 162, wherein said cytotoxic agent is selected from the group consisting of toxins, antibiotics, radioactive isotopes and nucleolytic enzymes.
164. The method of Claim 162, wherein the cytotoxic agent is a toxin.
165. The method of Claim 164, wherein the toxin is selected from the group consisting of maytansinoid and calicheamicin.
166. The method of Claim 164, wherein the toxin is a maytansinoid.
167. The method of Claim 153, wherein said antibody is produced in bacteria.
168. The method of Claim 153, wherein said antibody is produced in CHO cells.
169. The method of Claim 153, wherein said protein has:

(a) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95);
(b) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(c) an amino acid sequence of an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide sequence;
(d) an amino acid sequence of an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;
(e) an amino acid sequence encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) an amino acid sequence encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

170. A method of therapeutically treating a tumor in a mammal, wherein the growth of said tumor is at least in part dependent upon a growth potentiating effect of a protein having at least 80% amino acid sequence identity to:

(a) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95);
(b) the polypeptide shown in any one of Figures 23-41, 58-73, 79-83, 85, 88, 89, 92, 93 or 95 (SEQ ID NOS: 23-41, 58-73, 79-83, 85, 88, 89, 92, 93 or 95), lacking its associated signal peptide;
(c) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), with its associated signal peptide;
(d) an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide;
(e) a polypeptide encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or
(f) a polypeptide encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94), said method comprising contacting said protein with an antibody, oligopeptide or organic molecule that binds to said protein, thereby effectively treating said tumor.

171. The method of Claim 170, wherein said protein is expressed by cells of said tumor.

172. The method of Claim 170, wherein the binding of said antibody, oligopeptide or organic molecule to said protein antagonizes a cell growth-potentiating activity of said protein.

173. The method of Claim 170, wherein said antibody is a monoclonal antibody.

174. The method of Claim 170, wherein said antibody is an antibody fragment.

175. The method of Claim 170, wherein said antibody is a chimeric or a humanized antibody.

176. The method of Claim 170, wherein said antibody, oligopeptide or organic molecule is conjugated to a growth inhibitory agent.

177. The method of Claim 170, wherein said antibody, oligopeptide or organic molecule is conjugated to a cytotoxic agent.

178. The method of Claim 177, wherein said cytotoxic agent is selected from the group consisting of toxins, antibiotics, radioactive isotopes and nucleolytic enzymes.

179. The method of Claim 177, wherein the cytotoxic agent is a toxin.

180. The method of Claim 179, wherein the toxin is selected from the group consisting of maytansinoid and calicheamicin.

181. The method of Claim 179, wherein the toxin is a maytansinoid.

182. The method of Claim 170, wherein said antibody is produced in bacteria.

183. The method of Claim 170, wherein said antibody is produced in CHO cells.

184. The method of Claim 170, wherein said protein has:

(a) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95);

(b) the amino acid sequence shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;

(c) an amino acid sequence of an extracellular domain of the polypeptide shown in any one of Figures 23-41,58-73,79-83,85,88,89,92,93 or 95 (SEQ ID NOS:23-41, 58-73, 79-83, 85, 88, 89, 92, 93 or 95), with its associated signal peptide sequence;

(d) an amino acid sequence of an extracellular domain of the polypeptide shown in any one of Figures 23-41, 58-73, 79-83, 85, 88, 89, 92, 93 or 95 (SEQ ID NOS:23-41, 58-73, 79-83, 85, 88, 89, 92, 93 or 95), lacking its associated signal peptide sequence;

(e) an amino acid sequence encoded by the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94); or

(f) an amino acid sequence encoded by the full-length coding region of the nucleotide sequence shown in any one of Figures 1-22, 42-57, 74-78, 84, 86, 87, 90, 91 or 94 (SEQ ID NOS:1-22, 42-57, 74-78, 84, 86, 87, 90, 91 or 94).

Yet further embodiments of the present invention will be evident to the skilled artisan upon a reading of the present specification.

## BRIEF DESCRIPTION OF THE DRAWINGS

Figure 1 shows a nucleotide sequence (SEQ ID NO:1) of a TAT257 cDNA, wherein SEQ ID NO:1 is a clone designated herein as "DNA274297".

Figure 2 shows a nucleotide sequence (SEQ ID NO:2) of a TAT258 cDNA, wherein SEQ ID NO:2 is a clone designated herein as "DNA47369".

Figure 3 shows a nucleotide sequence (SEQ ID NO:3) of a TAT259 cDNA, wherein SEQ ID NO:3 is a clone designated herein as "DNA226027".

Figure 4 shows a nucleotide sequence (SEQ ID NO:4) of a TAT260 cDNA, wherein SEQ ID NO:4 is a clone designated herein as "DNA226713".

Figure 5 shows a nucleotide sequence (SEQ ID NO:5) ofa TAT261 cDNA, wherein SEQ ID NO:5 is a clone designated herein as "DNA86517".

Figure 6 shows a nucleotide sequence (SEQ ID NO:6) ofa TAT262 cDNA, wherein SEQ ID NO:6 is a clone designated herein as "DNA88126".

Figure 7 shows a nucleotide sequence (SEQ ID NO:7) of a TAT263 cDNA, wherein SEQ ID NO:7 is a clone designated herein as "DNA103464".

Figures 8A-B show a nucleotide sequence (SEQ ID NO:8) of a TAT264 cDNA, wherein SEQ ID NO:8 is a clone designated herein as "DNA194776".

Figures 9A-C show a nucleotide sequence (SEQ ID NO:9) of a TAT265 cDNA, wherein SEQ ID NO:9 is a clone designated herein as "DNA288204".

Figure 10 shows a nucleotide sequence (SEQ ID NO:10) of a TAT266 cDNA, wherein SEQ ID NO:10 is a clone designated herein as "DNA257354".

Figure 11 shows a nucleotide sequence (SEQ ID NO:11) of a TAT267 cDNA, wherein SEQ ID NO:11 is a clone designated herein as "DNA98566".

Figure 12 shows a nucleotide sequence (SEQ ID NO:12) of a TAT268 cDNA, wherein SEQ ID NO:12 is a clone designated herein as "DNA227212".

Figure 13 shows a nucleotide sequence (SEQ ID NO: 13) of a TAT269 cDNA, wherein SEQ ID NO: 13 is a clone designated herein as "DNA227461".

Figures 14A-B show a nucleotide sequence (SEQ ID NO: 14) ofa TAT270 cDNA, wherein SEQ ID NO: 14 is a clone designated herein as "DNA150762".

Figure 15 shows a nucleotide sequence (SEQ ID NO:15) of a TAT271 cDNA, wherein SEQ ID NO:15 is a clone designated herein as "DNA86382".

Figure 16 shows a nucleotide sequence (SEQ ID NO:16) of a TAT272 cDNA, wherein SEQ ID NO:16 is a clone designated herein as "DNA256608".

Figure 17 shows a nucleotide sequence (SEQ ID NO:17) of a TAT273 cDNA, wherein SEQ ID NO:17 is a clone designated herein as "DNA19902".

Figure 18 shows a nucleotide sequence (SEQ ID NO:18) of a TAT274 cDNA, wherein SEQ ID NO:18 is a clone designated herein as "DNA182764".

Figures 19A-B show a nucleotide sequence (SEQ ID NO:19) of a TAT275 cDNA, wherein SEQ ID NO:19 is a clone designated herein as "DNA225727".

Figure 20 shows a nucleotide sequence (SEQ ID NO:20) of a TAT276 cDNA, wherein SEQ ID NO:20 is a clone designated herein as "DNA119500".

Figure 21 shows a nucleotide sequence (SEQ ID NO:21) of a TAT277 cDNA, wherein SEQ ID NO:21 is a clone designated herein as "DNA19362".

Figure 22 shows a nucleotide sequence (SEQ ID NO:22) of a TAT278 cDNA, wherein SEQ ID NO:22 is a clone designated herein as "DNA226446".

Figure 23 shows the amino acid sequence (SEQ ID NO:23) derived from the coding sequence of SEQ ID NO:2 shown in Figure 2.

Figure 24 shows the amino acid sequence (SEQ ID NO:24) derived from the coding sequence of SEQ ID NO:3 shown in Figure 3.

Figure 25 shows the amino acid sequence (SEQ ID NO:25) derived from the coding sequence of SEQ ID NO:4 shown in Figure 4.

Figure 26 shows the amino acid sequence (SEQ ID NO:26) derived from the coding sequence of SEQ ID NO:6 shown in Figure 6.

Figure 27 shows the amino acid sequence (SEQ ID NO:27) derived from the coding sequence of SEQ ID NO:7 shown in Figure 7.

Figures 28A-B show the amino acid sequence (SEQ ID NO:28) derived from the coding sequence of SEQ ID NO: 8 shown in Figures 8A-B.

Figure 29 shows the amino acid sequence (SEQ ID NO:29) derived from the coding sequence of SEQ ID NO:9 shown in Figures 9A-C.

Figure 30 shows the amino acid sequence (SEQ ID NO:30) derived from the coding sequence of SEQ ID NO:10 shown in Figure 10.

Figure 31 shows the amino acid sequence (SEQ ID NO:31) derived from the coding sequence of SEQ ID NO:11 shown in Figure 11.

Figure 32 shows the amino acid sequence (SEQ ID NO:32) derived from the coding sequence of SEQ ID NO:12 shown in Figure 12.

Figure 33 shows the amino acid sequence (SEQ ID NO:33) derived from the coding sequence of SEQ ID NO:13 shown in Figure 13.

Figure 34 shows the amino acid sequence (SEQ ID NO:34) derived from the coding sequence of SEQ ID NO:14 shown in Figures 14A-B.

Figure 35 shows the amino acid sequence (SEQ ID NO:35) derived from the coding sequence of SEQ ID NO:16 shown in Figure 16.

Figure 36 shows the amino acid sequence (SEQ ID NO:36) derived from the coding sequence of SEQ ID NO:17 shown in Figure 17.

Figure 37 shows the amino acid sequence (SEQ ID NO:37) derived from the coding sequence of SEQ ID NO:18 shown in Figure 18.

Figure 38 shows the amino acid sequence (SEQ ID NO:38) derived from the coding sequence of SEQ ID NO:19 shown in Figures 19A-B.
Figure 39 shows the amino acid sequence (SEQ ID NO:39) derived from the coding sequence of SEQ ID NO:20 shown in Figure 20.
Figure 40 shows the amino acid sequence (SEQ ID NO:40) derived from the coding sequence of SEQ ID NO:21 shown in Figure 21.
Figure 41 shows the amino acid sequence (SEQ ID NO:41) derived from the coding sequence of SEQ ID NO:22 shown in Figure 22.
Figure 42 shows a nucleotide sequence (SEQ ID NO:42) of a TAT240 cDNA, wherein SEQ ID NO:42 is a clone designated herein as "DNA172363".
Figures 43A-B show a nucleotide sequence (SEQ ID NO:43) of a TAT241 cDNA, wherein SEQ ID NO:43 is a clone designated herein as "DNA227465".
Figure 44 shows a nucleotide sequence (SEQ ID NO:44) of a TAT242 cDNA, wherein SEQ ID NO:44 is a clone designated herein as "DNA227943".
Figure 45 shows a nucleotide sequence (SEQ ID NO:45) of a TAT243 cDNA, wherein SEQ ID NO:45 is a clone designated herein as "DNA82306".
Figure 46 shows a nucleotide sequence (SEQ ID NO:46) of a TAT244 cDNA, wherein SEQ ID NO:46 is a clone designated herein as "DNA227019".
Figure 47 shows a nucleotide sequence (SEQ BD NO:47) of a TAT245 cDNA, wherein SEQ ID NO:47 is a clone designated herein as "DNA96942".
Figure 48 shows a nucleotide sequence (SEQ ID NO:48) of a TAT246 cDNA, wherein SEQ ID NO:48 is a clone designated herein as "DNA42551".
Figure 49 shows a nucleotide sequence (SEQ ID NO:49) of a TAT135 cDNA, wherein SEQ ID NO:49 is a clone designated herein as "DNA68885".
Figure 50 shows a nucleotide sequence (SEQ ID NO:50) of a TAT249 cDNA, wherein SEQ ID NO:50 is a clone designated herein as "DNA59619".
Figure 51 shows a nucleotide sequence (SEQ ID NO:51) of a TAT250 cDNA, wherein SEQ ID NO:512 is a clone designated herein as "DNA227205".
Figure 52 shows a nucleotide sequence (SEQ ID NO:52) of a TAT251 cDNA, wherein SEQ ID NO:52 is a clone designated herein as "DNA175959".
Figure 53 shows a nucleotide sequence (SEQ ID NO:53) of a TAT2S2 cDNA, wherein SEQ ID NO:53 is a clone designated herein as "DNA48227".
Figure 54 shows a nucleotide sequence (SEQ ID NO:54) of a TAT253 cDNA, wherein SEQ ID NO:54 is a clone designated herein as "DNA59612".
Figures 55A-B show a nucleotide sequence (SEQ ID NO:55) of a TAT254 cDNA, wherein SEQ ID NO:55 is a clone designated herein as "DNA226917".
Figure 56 shows a nucleotide sequence (SEQ ID NO:56) of a TAT2S5 cDNA, wherein SEQ ID NO:56 is a clone designated herein as "DNA125219".
Figure 57 shows a nucleotide sequence (SEQ ID NO:57) of a TAT256 cDNA, wherein SEQ ID NO:57 is a clone designated herein as "DNA151291".
Figure 58 shows the amino acid sequence (SEQ ID NO:58) derived from the coding sequence of SEQ LID NO:42 shown in Figure 42.
Figure 59 shows the amino acid sequence (SEQ ID NO:59) derived from the coding sequence of SEQ ID NO:43 shown in Figures 43A-B.
Figure 60 shows the amino acid sequence (SEQ ID NO:60) derived from the coding sequence of SEQ ID NO:44 shown in Figure 44.
Figure 61 shows the amino acid sequence (SEQ ID NO:61) derived from the coding sequence of SEQ ID NO:45 shown in Figure 45.
Figure 62 shows the amino acid sequence (SEQ ID NO:62) derived from the coding sequence of SEQ ID NO:46 shown in Figure 46.
Figure 63 shows the amino acid sequence (SEQ ID NO:63) derived from the coding sequence of SEQ ID NO:47 shown in Figure 47.
Figure 64 shows the amino acid sequence (SEQ ID NO:64) derived from the coding sequence of SEQ ID NO:48 shown in Figure 48.
Figure 65 shows the amino acid sequence (SEQ ID NO:65) derived from the coding sequence of SEQ ID NO:49 shown in Figure 49.
Figure 66 shows the amino acid sequence (SEQ ID NO:66) derived from the coding sequence of SEQ ID NO:50 shown in Figure 50.

Figure 67 shows the amino acid sequence (SEQ ID NO:67) derived from the coding sequence of SEQ ID NO:51 shown in Figure 51.
Figure 68 shows the amino acid sequence (SEQ ID NO:68) derived from the coding sequence of SEQ ID NO:52 shown in Figure 52.
Figure 69 shows the amino acid sequence (SEQ ID NO:69) derived from the coding sequence of SEQ ID NO:53 shown in Figure 53.
Figure 70 shows the amino acid sequence (SEQ ID NO:70) derived from the coding sequence of SEQ ID NO:54 shown in Figure 54.
Figure 71 shows the amino acid sequence (SEQ ID NO:71) derived from the coding sequence of SEQ ID NO:55 shown in Figures 55A-B.
Figure 72 shows the amino acid sequence (SEQ ID NO:72) derived from the coding sequence of SEQ ID NO:56 shown in Figure 56.
Figure 73 shows the amino acid sequence (SEQ ID NO:73) derived from the coding sequence of SEQ ID NO:57 shown in Figure 57.
Figures 74A-B show a nucleotide sequence (SEQ ID NO:74) of a TAT279 cDNA, wherein SEQ ID NO:74 is a clone designated herein as "DNA227583".
Figure 75 shows a nucleotide sequence (SEQ ID NO:75) of a TAT280 cDNA, wherein SEQ ID NO:75 is a clone designated herein as "DNA194838".
Figures 76A-B show a nucleotide sequence (SEQ ID NO:76) of a TAT290 cDNA, wherein SEQ ID NO:76 is a clone designated herein as "DNA290924".
Figures 77A-B show a nucleotide sequence (SEQ ID NO:77) of a TAT281 cDNA, wherein SEQ ID NO:77 is a clone designated herein as "DNA227708".
Figures 78A-B show a nucleotide sequence (SEQ ID NO:78) of a TAT282 cDNA, wherein SEQ ID NO:78 is a clone designated herein as "DNA226859".
Figure 79 shows the amino acid sequence (SEQ ID NO:79) derived from the coding sequence of SEQ ID NO:74 shown in Figures 74A-B.
Figure 80 shows the amino acid sequence (SEQ ID NO:80) derived from the coding sequence of SEQ ID NO:75 shown in Figure 75.
Figure 81 shows the amino acid sequence (SEQ ID NO:81) derived from the coding sequence of SEQ ID NO:76 shown in Figures 76A-B.
Figure 82 shows the amino acid sequence (SEQ ID NO:82) derived from the coding sequence of SEQ ID NO:77 shown in Figures 77A-B.
Figure 83 shows the amino acid sequence (SEQ ID NO:83) derived from the coding sequence of SEQ ID NO:78 shown in Figures 78A-B.
Figure 84 shows a nucleotide sequence (SEQ ID NO:84) of a TAT283 cDNA, wherein SEQ ID NO:84 is a clone designated herein as "DNA290812".
Figure 85 shows the amino acid sequence (SEQ ID NO:85) derived from the coding sequence of SEQ ID NO:84 shown in Figure 84.
Figure 86 shows a nucleotide sequence (SEQ ID NO:86) of a TAT286 cDNA, wherein SEQ ID NO:86 is a clone designated herein as "DNA292996".
Figures 87A-C show a nucleotide sequence (SEQ ID NO:87) of a TAT288 cDNA, wherein SEQ ID NO:87 is a clone designated herein as "DNA254932".
Figure 88 shows the amino acid sequence (SEQ ID NO:88) derived from the coding sequence of SEQ ID NO:86 shown in Figure 86.
Figures 89A-B show the amino acid sequence (SEQ ID NO:89) derived from the coding sequence of SEQ ID NO: 87 shown in Figures 87A-C.
Figure 90 shows a nucleotide sequence (SEQ ID NO:90) of a TAT287 cDNA, wherein SEQ ID NO:90 is a clone designated herein as "DNA254340".
Figure 91 shows a nucleotide sequence (SEQ ID NO:91) of a TAT373 cDNA, wherein SEQ ID NO:91 is a clone designated herein as "DNA299882".
Figure 92 shows the amino acid sequence (SEQ ID NO:92) derived from the coding sequence of SEQ ID NO:90 shown in Figure 90.
Figure 93 shows the amino acid sequence (SEQ ID NO:93) derived from the coding sequence of SEQ ID NO:91 shown in Figure 91.
Figure 94 shows a nucleotide sequence (SEQ ID NO:94) of a TAT289 cDNA, wherein SEQ ID NO:94 is a clone designated herein as "DNA288313".
Figure 95 shows the amino acid sequence (SEQ ID NO:95) derived from the coding sequence of SEQ ID NO:94 shown in Figure 94.

DETAILED DESCRIPTION OF THE PREFERRED EMBODIMENTS

I. Definitions

The terms "TAT polypeptide" and "TAT" as used herein and when immediately followed by a numerical designation, refer to various polypeptides, wherein the complete designation (i.e.,TAT/number) refers to specific polypeptide sequences as described herein. The terms "TAT/number polypeptide" and "TAT/number" wherein the term "number" is provided as an actual numerical designation as used herein encompass native sequence polypeptides, polypeptide variants and fragments of native sequence polypeptides and polypeptide variants (which are further defined herein). The TAT polypeptides described herein may be isolated from a variety of sources, such as from human tissue types or from another source, or prepared by recombinant or synthetic methods. The term "TAT polypeptide" refers to each individual TAT/number polypeptide disclosed herein. All disclosures in this specification which refer to the "TAT polypeptide" refer to each of the polypeptides individually as well as jointly. For example, descriptions of the preparation of, purification of, derivation of, formation of antibodies to or against, formation of TAT binding oligopeptides to or against, formation of TAT binding organic molecules to or against, administration of, compositions containing, treatment of a disease with, etc., pertain to each polypeptide of the invention individually. The term "TAT polypeptide" also includes variants of the TAT/number polypeptides disclosed herein.

A "native sequence TAT polypeptide" comprises a polypeptide having the same amino acid sequence as the corresponding TAT polypeptide derived from nature. Such native sequence TAT polypeptides can be isolated from nature or can be produced by recombinant or synthetic means. The term "native sequence TAT polypeptide" specifically encompasses naturally-occurring truncated or secreted forms of the specific TAT polypeptide (*e.g.,* an extracellular domain sequence), naturally-occurring variant forms (*e.g.*, alternatively spliced forms) and naturally-occurring allelic variants of the polypeptide. In certain embodiments of the invention, the native sequence TAT polypeptides disclosed herein are mature or full-length native sequence polypeptides comprising the full-length amino acids sequences shown in the accompanying figures. Start and stop codons (if indicated) are shown in bold font and underlined in the figures. Nucleic acid residues indicated as "N" in the accompanying figures are any nucleic acid residue. However, while the TAT polypeptides disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the TAT polypeptides.

The TAT polypeptide "extracellular domain" or "ECD" refers to a form of the TAT polypeptide which is essentially free of the transmembrane and cytoplasmic domains. Ordinarily, a TAT polypeptide ECD will have less than 1% of such transmembrane and/or cytoplasmic domains and preferably, will have less than 0.5% of such domains. It will be understood that any transmembrane domains identified for the TAT polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a TAT polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the various TAT polypeptides disclosed herein may be shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and vonHeinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"TAT polypeptide variant" means a TAT polypeptide, preferably an active TAT polypeptide, as defined herein having at least about 80% amino acid sequence identity with a full-length native sequence TAT polypeptide sequence as disclosed herein, a TAT polypeptide sequence lacking the signal peptide as disclosed herein, an extracellular domain of a TAT polypeptide, with or without the signal peptide, as disclosed herein or any other fragment of a full-length TAT polypeptide sequence as disclosed herein (such as those encoded by a nucleic acid that represents only a portion of the complete coding sequence for a full-length TAT polypeptide). Such TAT polypeptide variants include, for instance, TAT polypeptides wherein one or more amino acid residues are added, or deleted, at the N- or C-terminus of the full-length native amino acid sequence. Ordinarily, a TAT polypeptide variant will have at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% amino acid sequence identity, to a full-length native sequence TAT polypeptide sequence as disclosed herein, a TAT polypeptide sequence lacking the signal peptide as disclosed herein, an extracellular domain of a TAT polypeptide, with or without the signal peptide, as disclosed herein or any other specifically defined fragment of a full-length TAT polypeptide sequence as disclosed herein. Ordinarily, TAT variant polypeptides are at least about 10 amino acids in length, alternatively at least about 20, 30, 40, 50, 60, 70, 80, 90, 100, 110, 120, 130 ,140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 amino acids in length, ormore. Optionally, TAT variant polypeptides will have no more than one conservative amino acid substitution as compared to the native TAT polypeptide sequence, alternatively no more than 2, 3, 4, 5, 6, 7, 8, 9, or 10 conservative amino acid substitution as compared to the native TAT polypeptide sequence.

"Percent (%) amino acid sequence identity" with respect to the TAT polypeptide sequences identified herein is defined as the percentage of amino acid residues in a candidate sequence that are identical with the amino acid residues in the specific TAT polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "TAT", wherein "TAT" represents the amino acid sequence of a hypothetical TAT polypeptide of interest, "Comparison Protein" represents the amino acid sequence of a polypeptide against which the "TAT" polypeptide of interest is being compared, and "X, "Y" and "Z" each represent different hypothetical amino acid residues. Unless specifically stated otherwise, all % amino acid sequence identity values used herein are obtained as described in the immediately preceding paragraph using the ALIGN-2 computer program.

"TAT variant polynucleotide" or "TAT variant nucleic acid sequence" means a nucleic acid molecule which encodes a TAT polypeptide, preferably an active TAT polypeptide, as defined herein and which has at least about 80% nucleic acid sequence identity with a nucleotide acid sequence encoding a full-length native sequence TAT polypeptide sequence as disclosed herein, a full-length native sequence TAT polypeptide sequence lacking the signal peptide as disclosed herein, an extracellular domain of a TAT polypeptide, with or without the signal peptide, as disclosed herein or any other fragment of a full-length TAT polypeptide sequence as disclosed herein (such as those encoded by a nucleic acid that represents only a portion of the complete coding sequence for a full-length TAT polypeptide). Ordinarily, a TAT variant polynucleotide will have at least about 80% nucleic acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% nucleic acid sequence identity with a nucleic acid sequence encoding a full-length native sequence TAT polypeptide sequence as disclosed herein, a full-length native sequence TAT polypeptide sequence lacking the signal peptide as disclosed herein, an extracellular domain of a TAT polypeptide, with or without the signal sequence, as disclosed herein or any other fragment of a full-length TAT polypeptide sequence as disclosed herein. Variants do not encompass the native nucleotide sequence.

Ordinarily, TAT variant polynucleotides are at least about 5 nucleotides in length, alternatively at least about 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 210, 220, 230, 240, 250, 260, 270, 280, 290, 300, 310, 320, 330, 340, 350, 3 60, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides in length, wherein in this context the term "about" means the referenced nucleotide sequence length plus or minus 10% of that referenced length.

"Percent (%) nucleic acid sequence identity" with respect to TAT-encoding nucleic acid sequences identified herein is defined as the percentage of nucleotides in a candidate sequence that are identical with the nucleotides in the TAT nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

**100 times the fraction W/Z**

where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity ofD to C. As examples of % nucleic acid sequence identity calculations, Tables 4 and 5, demonstrate how to calculate the % nucleic acid sequence identity of the nucleic acid sequence designated "Comparison DNA" to the nucleic acid sequence designated "TAT-DNA", wherein "TAT-DNA" represents a hypothetical TAT-encoding nucleic acid sequence of interest, "Comparison DNA" represents the nucleotide sequence of a nucleic acid molecule against which the "TAT-DNA" nucleic acid molecule of interest is being compared, and "N", "L" and "V" each represent different hypothetical nucleotides. Unless specifically stated otherwise, all % nucleic acid sequence identity values used herein are obtained as described in the immediately preceding paragraph using the ALIGN-2 computer program.

In other embodiments, TAT variant polynucleotides are nucleic acid molecules that encode a TAT polypeptide and which are capable of hybridizing, preferably under stringent hybridization and wash conditions, to nucleotide sequences encoding a full-length TAT polypeptide as disclosed herein. TAT variant polypeptides may be those that are encoded by a TAT variant polynucleotide.

The term "full-length coding region" when used in reference to a nucleic acid encoding a TAT polypeptide refers to the sequence of nucleotides which encode the full-length TAT polypeptide of the invention (which is often shown between start and stop codons, inclusive thereof, in the accompanying figures). The term "full-length coding region" when used in reference to an ATCC deposited nucleic acid refers to the TAT polypeptide-encoding portion of the cDNA that is inserted into the vector deposited with the ATCC (which is often shown between start and stop codons, inclusive thereof, in the accompanying figures).

"Isolated," when used to describe the various TAT polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or nonproteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the TAT polypeptide natural environment will not be present. Ordinarily, however, isolated polypeptide will be prepared by at least one purification step.

An "isolated" TAT polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.001 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42 °C; or (3) overnight hybridization in a solution that employs 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 $\mu$g/ml), 0.1% SDS, and 10% dextran sulfate at 42°C, with a 10 minute wash at 42°C in 0.2 x SSC (sodium chloride/sodium citrate) followed by a 10 minute high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a TAT polypeptide or anti-TAT antibody fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

"Active" or "activity" for the purposes herein refers to form(s) of a TAT polypeptide which retain a biological and/or an immunological activity of native or naturally-occurring TAT, wherein "biological" activity refers to a biological function (either inhibitory or stimulatory) caused by a native or naturally-occurring TAT other than the ability to induce the production of an antibody against an antigenic epitope possessed by a native or naturally-occurring TAT and an "immunological" activity refers to the ability to induce the production of an antibody against an antigenic epitope possessed by a native or naturally-occurring TAT.

The term "antagonist" is used in the broadest sense, and includes any molecule that partially or fully blocks, inhibits, or neutralizes a biological activity of a native TAT polypeptide disclosed herein. In a similar manner, the term "agonist" is used in the broadest sense and includes any molecule that mimics a biological activity of a native TAT polypeptide disclosed herein. Suitable agonist or antagonist molecules specifically include agonist or antagonist antibodies or antibody fragments, fragments or amino acid sequence variants of native TAT polypeptides, peptides, antisense oligonucleotides, small organic molecules, etc. Methods for identifying agonists or antagonists of a TAT polypeptide may comprise contacting a TAT polypeptide with a candidate agonist or antagonist molecule and measuring a detectable change in one or more biological activities normally associated with the TAT polypeptide.

"Treating" or "treatment" or "alleviation" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented. A subject or mammal is successfully "treated" for a TAT polypeptide-expressing cancer if, after receiving a therapeutic amount of an anti-TAT antibody, TAT binding oligopeptide or TAT binding organic molecule according to the methods of the present invention, the patient shows observable and/or measurable reduction in or absence of one or more of the following: reduction in the number of cancer cells or absence of the cancer cells; reduction in the tumor size; inhibition (i.e., slow to some extent and preferably stop) of cancer cell infiltration into peripheral organs including the spread of cancer into soft tissue and bone; inhibition (i.e., slow to some extent and preferably stop) of tumor metastasis; inhibition, to some extent, of tumor growth; and/or relief to some extent, one or more of the symptoms associated with the specific cancer; reduced morbidity and mortality, and improvement in quality of life issues. To the extent the anti-TAT antibody or TAT binding oligopeptide may prevent growth and/or kill existing cancer cells, it may be cytostatic and/or cytotoxic. Reduction of these signs or symptoms may also be felt by the patient.

The above parameters for assessing successful treatment and improvement in the disease are readily measurable by routine procedures familiar to a physician. For cancer therapy, efficacy can be measured, for example, by assessing the time to disease progression (TTP) and/or determining the response rate (RR). Metastasis can be determined by staging tests and by bone scan and tests for calcium level and other enzymes to determine spread to the bone. CT scans can also be done to look for spread to the pelvis and lymph nodes in the area. Chest X-rays and measurement of liver enzyme levels by known methods are used to look for metastasis to the lungs and liver, respectively. Other routine methods for monitoring the disease include transrectal ultrasonography (TRUS) and transrectal needle biopsy (TRNB).

For bladder cancer, which is a more localized cancer, methods to determine progress of disease include urinary cytologic evaluation by cystoscopy, monitoring for presence of blood in the urine, visualization of the urothelial tract by sonography or an intravenous pyelogram, computed tomography (CT) and magnetic resonance imaging (MRI). The presence of distant metastases can be assessed by CT of the abdomen, chest x-rays, or radionuclide imaging of the skeleton.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of the treatment of, alleviating the symptoms of or diagnosis of a cancer refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN®, polyethylene glycol (PEG), and PLURONICS®.

By "solid phase" or "solid support" is meant a non-aqueous matrix to which an antibody, TAT binding oligopeptide or TAT binding organic molecule of the present invention can adhere or attach. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a TAT polypeptide, an antibody thereto or a TAT binding oligopeptide) to a mammal. The components of the liposome are commonly arranged in a bilayer formation, similar to the lipid arrangement of biological membranes.

A "small" molecule or "small" organic molecule is defined herein to have a molecular weight below about 500 Daltons.

An "effective amount" of a polypeptide, antibody, TAT binding oligopeptide, TAT binding organic molecule or an agonist or antagonist thereof as disclosed herein is an amount sufficient to carry out a specifically stated purpose. An "effective amount" may be determined empirically and in a routine manner, in relation to the stated purpose.

The term "therapeutically effective amount" refers to an amount of an antibody, polypeptide, TAT binding oligopeptide, TAT binding organic molecule or other drug effective to "treat" a disease or disorder in a subject or mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. See the definition herein of "treating". To the extent the drug may prevent growth and/or kill existing cancer cells, it may be cytostatic and/or cytotoxic.

A "growth inhibitory amount" of an anti-TAT antibody, TAT polypeptide, TAT binding oligopeptide or TAT binding organic molecule is an amount capable of inhibiting the growth of a cell, especially tumor, e.g., cancer cell, either *in vitro* or *in vivo.* A "growth inhibitory amount" of an anti-TAT antibody, TAT polypeptide, TAT binding oligopeptide or TAT binding organic molecule for purposes of inhibiting neoplastic cell growth may be determined empirically and in a routine manner.

A "cytotoxic amount" of an anti-TAT antibody, TAT polypeptide, TAT binding oligopeptide or TAT binding organic molecule is an amount capable of causing the destruction of a cell, especially tumor, e.g., cancer cell, either *in vitro* or *in vivo.* A "cytotoxic amount" of an anti-TAT antibody, TAT polypeptide, TAT binding oligopeptide or TAT binding organic molecule for purposes of inhibiting neoplastic cell growth may be determined empirically and in a routine manner.

The term "antibody" is used in the broadest sense and specifically covers, for example, single anti-TAT monoclonal antibodies (including agonist, antagonist, and neutralizing antibodies), anti-TAT antibody compositions with polyepitopic specificity, polyclonal antibodies, single chain anti-TAT antibodies, and fragments of anti-TAT antibodies (see below) as long as they exhibit the desired biological or immunological activity. The term "immunoglobulin" (Ig) is used interchangeable with antibody herein.

An "isolated antibody" is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The basic 4-chain antibody unit is a heterotetrameric glycoprotein composed of two identical light (L) chains and two identical heavy (H) chains (an IgM antibody consists of 5 of the basic heterotetramer unit along with an additional polypeptide called J chain, and therefore contain 10 antigen binding sites, while secreted IgA antibodies can polymerize to form polyvalent assemblages comprising 2-5 of the basic 4-chain units along with J chain). In the case of IgGs, the 4-chain unit is generally about 150,000 daltons. Each L chain is linked to a H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain ($V_H$) followed by three constant domains ($C_H$) for each of the $\alpha$ and $\gamma$ chains and four $C_H$ domains for $\mu$ and $\varepsilon$ isotypes. Each L chain has at the N-terminus, a variable domain ($V_L$) followed by a constant domain ($C_L$) at its other end. The $V_L$ is aligned with the $V_H$ and the $C_L$ is aligned with the first constant domain of the heavy chain ($C_H1$). Particular amino acid residues are believed to form an interface between the light chain and heavy chain variable domains. The pairing of a $V_H$ and $V_L$ together forms a single antigen-binding site. For the structure and properties of the different classes of antibodies, see, e.g., Basic and Clinical Immunology, 8th edition, Daniel P. Stites, Abba I. Terr and Tristram G. Parslow (eds.), Appleton & Lange, Norwalk, CT, 1994, page 71 and Chapter 6.

The L chain from any vertebrate species can be assigned to one of two clearly distinct types, called kappa and lambda, based on the amino acid sequences of their constant domains. Depending on the amino acid sequence of the constant domain of their heavy chains ($C_H$), immunoglobulins can be assigned to different classes or isotypes. There are five classes of immunoglobulins: IgA, IgD, IgE, IgG, and IgM, having heavy chains designated α, δ, ε, γ, and μ, respectively. The γ and α classes are further divided into subclasses on the basis of relatively minor differences in $C_H$ sequence and function, e.g., humans express the following subclasses: IgG1, IgG2, IgG3, IgG4, IgA1, and IgA2.

The term "variable" refers to the fact that certain segments of the variable domains differ extensively in sequence among antibodies. The V domain mediates antigen binding and define specificity of a particular antibody for its particular antigen. However, the variability is not evenly distributed across the 110-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody which are responsible for antigen-binding. The hypervariable region generally comprises amino acid residues from a "complementarity determining region" or "CDR" (e.g. around about residues 24-34 (L1), 50-56 (L2) and 89-97 (L3) in the $V_L$, and around about 1-35 (H1), 50-65 (H2) and 95-102 (H3) in the $V_H$; Kabat et al., Sequences of Proteins of Immunological Interest, 5th Ed. Public Health Service, National Institutes of Health, Bethesda, MD. (1991)) and/or those residues from a "hypervariable loop" (*e.g.* residues 26-32 (L1), 50-52 (L2) and 91-96 (L3) in the $V_L$, and 26-32 (H1), 53-55 (H2) and 96-101 (H3) in the $V_H$; Chothia and Lesk J. Mol_Biol. 196:901-917 (1987)).

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" is not to be construed as requiring production of the antibody by any particular method. For example, the monoclonal antibodies useful in the present invention may be prepared by the hybridoma methodology first described by Kohler et al., Nature, 256:495 (1975), or may be made using recombinant DNA methods in bacterial, eukaryotic animal or plant cells (see, e.g., U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see U.S. Patent No. 4,816,567; and Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (*e.g.* Old World Monkey, Ape etc), and human constant region sequences.

An "intact" antibody is one which comprises an antigen-binding site as well as a $C_L$ and at least heavy chain constant domains, $C_H1$, $C_H2$ and $C_H3$. The constant domains may be native sequence constant domains (*e.g.* human native sequence constant domains) or amino acid sequence variant thereof. Preferably, the intact antibody has one or more effector functions.

"Antibody fragments" comprise a portion of an intact antibody, preferably the antigen binding or variable region of the intact antibody. Examples of antibody fragments include Fab, Fab', $F(ab')_2$, and Fv fragments; diabodies; linear antibodies (see U.S. PatentNo. 5,641,870, Example 2; Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, and a residual "Fc" fragment, a designation reflecting the ability to crystallize readily. The Fab fi-agment consists of an entire L chain along with the variable region domain of the H chain ($V_H$), and the first constant domain of one heavy chain ($C_H1$). Each Fab fragment is monovalent with respect to antigen binding, i.e., it has a single antigen-binding site. Pepsin treatment of an antibody yields a single large $F(ab')_2$ fragment which roughly corresponds to two disulfide linked Fab fragments having divalent antigen-binding activity and is still capable of cross-linking antigen. Fab' fragments differ from Fab fragments by having additional few residues at the carboxy terminus of the $C_H1$ domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known. The Fc fragment comprises the carboxy-terminal portions of both H chains held together by disulfides. The effector functions of antibodies are determined by sequences in the Fc region, which region is also the part recognized by Fc receptors (FcR) found on certain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv" also abbreviated as "sFv" or "scFv" are antibody fragments that comprise the $V_H$ and $V_L$ antibody domains connected into a single polypeptide chain. Preferably, the sFv polypeptide further comprises a polypeptide linker between the $V_H$ and $V_L$ domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994); Borrebaeck 1995, infra.

The term "diabodies" refers to small antibody fragments prepared by constructing sFv fragments (see preceding paragraph) with short linkers (about 5-10 residues) between the $V_H$ and $V_L$ domains such that inter-chain but not intra-chain pairing of the V domains is achieved, resulting in a bivalent fragment, i.e., fragment having two antigen-binding sites. Bispecific diabodies are heterodimers of two "crossover" sFv fragments in which the $V_H$ and $V_L$ domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).

"Humanized" forms of non-human (*e.g.*, rodent) antibodies are chimeric antibodies that contain minimal sequence derived from the non-human antibody. For the most part, humanized antibodies are human immunoglobulins (recipient antibody) in which residues from a hypervariable region of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

A "species-dependent antibody," e.g., a mammalian anti-human IgE antibody, is an antibody which has a stronger binding affinity for an antigen from a first mammalian species than it has for a homologue of that antigen from a second mammalian species. Normally, the species-dependent antibody "bind specifically" to a human antigen (i.e., has a binding affinity (Kd) value of no more than about $1 \times 10^{-7}$ M, preferably no more than about $1 \times 10^{-8}$ and most preferably no more than about $1 \times 10^{-9}$ M) but has a binding affinity for a homologue of the antigen from a second non-human mammalian species which is at least about 50 fold, or at least about 500 fold, or at least about 1000 fold, weaker than its binding affinity for the human antigen. The species-dependent antibody can be of any of the various types of antibodies as defined above, but preferably is a humanized or human antibody.

A "TAT binding oligopeptide" is an oligopeptide that binds, preferably specifically, to a TAT polypeptide as described herein. TAT binding oligopeptides may be chemically synthesized using known oligopeptide synthesis methodology or may be prepared and purified using recombinant technology. TAT binding oligopeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length ormore, wherein such oligopeptides that are capable of binding, preferably specifically, to a TAT polypeptide as described herein. TAT binding oligopeptides may be identified without undue experimentation using well known techniques. In this regard, it is noted that techniques for screening oligopeptide libraries for oligopeptides that are capable of specifically binding to a polypeptide target are well known in the art (see, e.g., U.S. PatentNos. 5,556,762,5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci. U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J. Immunol.,

140:611-616 (1988), Cwirla, S. E. et al. (1990) Proc. Natl. Acad. Sci. USA, 87:6378; Lowman, H.B. etal. (1991) Biochemistry, 30:10832; Clackson, T. etal. (1991)Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668).

A "TAT binding organic molecule" is an organic molecule other than an oligopeptide or antibody as defined herein that binds, preferably specifically, to a TAT polypeptide as described herein. TAT binding organic molecules may be identified and chemically synthesized using known methodology (see, e.g., PCT PublicationNos. WO00/00823 and WO00/39585). TAT binding organic molecules are usually less than about 2000 daltons in size, alternatively less than about 1500, 750, 500, 250 or 200 daltons in size, wherein such organic molecules that are capable of binding, preferably specifically, to a TAT polypeptide as described herein may be identified without undue experimentation using well known techniques. In this regard, it is noted that techniques for screening organic molecule libraries for molecules that are capable of binding to a polypeptide target are well known in the art (see, e.g., PCT Publication Nos. WO00/00823 and WO00/39585).

An antibody, oligopeptide or other organic molecule "which binds" an antigen of interest, e.g. a tumor-associated polypeptide antigen target, is one that binds the antigen with sufficient affinity such that the antibody, oligopeptide or other organic molecule is useful as a diagnostic and/or therapeutic agent in targeting a cell or tissue expressing the antigen, and does not significantly cross-react with other proteins. In such embodiments, the extent of binding of the antibody, oligopeptide or other organic molecule to a "non-target" protein will be less than about 10% of the binding of the antibody, oligopeptide or other organic molecule to its particular target protein as determined by fluorescence activated cell sorting (FACS) analysis or radioimmunoprecipitation (RIA). With regard to the binding of an antibody, oligopeptide or other organic molecule to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target, for example, an excess of non-labeled target. In this case, specific binding is indicated if the binding of the labeled target to a probe is competitively inhibited by excess unlabeled target. The term "specific binding" or "specifically binds to" or is "specific for" a particular polypeptide or an epitope on a particular polypeptide target as used herein can be exhibited, for example, by a molecule having a Kd for the target of at least about $10^{-4}$ M, alternatively at least about $10^{-5}$ M, alternatively at least about $10^{-6}$ M, alternatively at least about $10^{-7}$ M, alternatively at least about $10^{-8}$ M, alternatively at least about $10^{-9}$ M, alternatively at least about $10^{-10}$ M, alternatively at least about $10^{-11}$ M, alternatively at least about $10^{-12}$ M, or greater. In one embodiment, the term "specific binding" refers to binding where a molecule binds to a particular polypeptide or epitope on a particular polypeptide without substantially binding to any other polypeptide or polypeptide epitope.

An antibody, oligopeptide or other organic molecule that "inhibits the growth of tumor cells expressing a TAT polypeptide" or a "growth inhibitory" antibody, oligopeptide or other organic molecule is one which results in measurable growth inhibition of cancer cells expressing or overexpressing the appropriate TAT polypeptide. The TAT polypeptide may be a transmembrane polypeptide expressed on the surface of a cancer cell or may be a polypeptide that is produced and secreted by a cancer cell. Preferred growth inhibitory anti-TAT antibodies, oligopeptides or organic molecules inhibit growth of TAT-expressing tumor cells by greater than 20%, preferably from about 20% to about 50%, and even more preferably, by greater than 50% (e.g., from about 50% to about 100%) as compared to the appropriate control, the control typically being tumor cells not treated with the antibody, oligopeptide or other organic molecule being tested. In one embodiment, growth inhibition can be measured at an antibody concentration of about 0.1 to 30 μg/ml or about 0.5 nM to 200 nM in cell culture, where the growth inhibition is determined 1-10 days after exposure of the tumor cells to the antibody. Growth inhibition of tumor cells *in vivo* can be determined in various ways such as is described in the Experimental Examples section below. The antibody is growth inhibitory in vivo if administration of the anti-TAT antibody at about 1 μg/kg to about 100 mg/kg body weight results in reduction in tumor size or tumor cell proliferation within about 5 days to 3 months from the first administration of the antibody, preferably within about 5 to 30 days.

An antibody, oligopeptide or other organic molecule which "induces apoptosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies). The cell is usually one which overexpresses a TAT polypeptide. Preferably the cell is a tumor cell, e.g., a prostate, breast, ovarian, stomach, endometrial, lung, kidney, colon, bladder cell. Various methods are available for evaluating the cellular events associated with apoptosis. For example, phosphatidyl serine (PS) translocation can be measured by annexin binding; DNA fragmentation can be evaluated through DNA laddering; and nuclear/chromatin condensation along with DNA fragmentation can be evaluated by any increase in hypodiploid cells. Preferably, the antibody, oligopeptide or other organic molecule which induces apoptosis is one which results in about 2 to 50 fold, preferably about 5 to 50 fold, and most preferably about 10 to 50 fold, induction of annexin binding relative to untreated cell in an annexin binding assay.

Antibody "effector functions" refer to those biological activities attributable to the Fc region (a native sequence Fc region or amino acid sequence variant Fc region) of an antibody, and vary with the antibody isotype. Examples of antibody effector functions include: Clq binding and complement dependent cytotoxicity; Fc receptor binding; antibody-dependent cell-mediated cytotoxicity (ADCC); phagocytosis; down regulation of cell surface receptors (e.g., B cell receptor); and B cell activation.

"Antibody-dependent cell-mediated cytotoxicity" or "ADCC" refers to a form of cytotoxicity in which secreted Ig bound onto Fc receptors (FcRs) present on certain cytotoxic cells (e.g., Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are absolutely required for such killing. The primary cells for mediating ADCC, NKcells, express FcγRIII only, whereas monocytes express FcγRI, FcγRII and FcγRIII. FcR expression on hematopoietic cells is summarized in Table 3 on page 464 ofRavetch and Kinet, Annu. Rev. Immunol. 9: 457-92 (1991). To assess ADCC activity of a molecule of interest, an in vitro ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. (USA) 95:652-656 (1998).

"Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcγRI, FcγRII and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differprimarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see review M. in Daëron, Annu. Rev. Immunol. 15:203-234 (1997)). FcRs are reviewed in Ravetch and Kinet, Annu. Rev. Immunol. 9:457-492 (1991); Capel et al., Immunomethods 4:25-34 (1994); and de Haas et al., J. Lab. Clin. Med. 126:330-41 (1995). OtherFcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source, e.g., from blood.

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass) which are bound to their cognate antigen.. To assess complement activation, a CDC assay, e.g., as described in Gazzano-Santoro et al., J. Immunol. Methods 202:163 (1996), may be performed.

The terms "cancer" and "cancerous" refer to or describe the physiological condition in mammals that is typically characterized by unregulated cell growth. Examples of cancer include, but are not limited to, carcinoma, lymphoma, blastoma, sarcoma, and leukemia or lymphoid malignancies. More particular examples of such cancers include squamous cell cancer (e.g., epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma, brain, as well as head and neck cancer, and associated metastases.

The terms "cell proliferative disorder" and "proliferative disorder" refer to disorders that are associated with some degree of abnormal cell proliferation. In one embodiment, the cell proliferative disorder is cancer.

"Tumor", as used herein, refers to all neoplastic cell growth and proliferation, whether malignant or benign, and all pre-cancerous and cancerous cells and tissues.

An antibody, oligopeptide or other organic molecule which "induces cell death" is one which causes a viable cell to become nonviable. The cell is one which expresses a TAT polypeptide, preferably a cell that overexpresses a TAT polypeptide as compared to a normal cell of the same tissue type. The TAT polypeptide may be a transmembrane polypeptide expressed on the surface of a cancer cell or may be a polypeptide that is produced and secreted by a cancer cell. Preferably, the cell is a cancer cell, e.g., a breast, ovarian, stomach, endometrial, salivary gland, lung, kidney, colon, thyroid, pancreatic or bladder cell. Cell death *in vitro* may be determined in the absence of complement and immune effector cells to distinguish cell death induced by antibody-dependent cell-mediated cytotoxicity (ADCC) or complement dependent cytotoxicity (CDC). Thus, the assay for cell death may be performed using heat inactivated serum (i.e., in the absence of complement) and in the absence of immune effector cells. To determine whether the antibody, oligopeptide or other organic molecule is able to induce cell death, loss of membrane integrity as evaluated by uptake of propidium iodide (PI), trypan blue (see Moore et al. Cytotechnology 17:1-11 (1995)) or 7AAD can be assessed relative to untreated cells. Preferred cell death-inducing antibodies, oligopeptides or other organic molecules are those which induce PI uptake in the PI uptake assay in BT474 cells.

A "TAT-expressing cell" is a cell which expresses an endogenous or transfected TAT polypeptide either on the cell surface or in a secreted form. A "TAT-expressing cancer" is a cancer comprising cells that have a TAT polypeptide present on the cell surface or that produce and secrete a TAT polypeptide. A "TAT-expressing cancer" optionally produces sufficient levels of TAT polypeptide on the surface of cells thereof, such that an anti-TAT antibody, oligopeptide ot other organic molecule can bind thereto and have a therapeutic effect with respect to the cancer. In another embodiment, a "TAT-expressing cancer" optionally produces and secretes sufficient levels of TAT polypeptide, such that an anti-TAT antibody, oligopeptide ot other organic molecule antagonist can bind thereto and have a therapeutic effect with respect to the cancer. With regard to the latter, the antagonist may be an antisense oligonucleotide which reduces, inhibits or prevents production and secretion of the secreted TAT polypeptide by tumor cells. A cancer which "overexpresses" a TAT polypeptide is one which has significantly higher levels of TAT polypeptide at the cell surface thereof, or produces and secretes, compared to a noncancerous cell of the same tissue type. Such overexpression may be caused by gene amplification or by increased transcription or translation. TAT polypeptide overexpression may be determined in a diagnostic or prognostic assay by evaluating increased levels of the TAT protein present on the surface of a cell, or secreted by the cell (e.g., via an immunohistochemistry assay using anti-TAT antibodies prepared against an isolated TAT polypeptide which may be prepared using recombinant DNA technology from an isolated nucleic acid encoding the TAT polypeptide; FACS analysis, etc.). Alternatively, or additionally, one may measure levels of TAT polypeptide-encoding nucleic acid or mRNA in the cell, e.g., via fluorescent *in situ* hybridization using a nucleic acid based probe corresponding to a TAT-encoding nucleic acid or the complement thereof; (FISH; see WO98/45479 published October, 1998), Southern blotting, Northern blotting, or polymerase chain reaction (PCR) techniques, such as real time quantitative PCR (RT-PCR). One may also study TAT polypeptide overexpression by measuring shed antigen in a biological fluid such as serum, e.g, using antibody-based assays (see also, e.g., U.S. PatentNo. 4,933,294 issued June 12,1990; WO91/05264 published April 18, 1991; U.S. Patent 5,401,638 issued March 28, 1995; and Sias et al., J. Immunol. Methods 132:73-80 (1990)). Aside from the above assays, various in *vivo* assays are available to the skilled practitioner. For example, one may expose cells within the body of the patient to an antibody which is optionally labeled with a detectable label, e.g., a radioactive isotope, and binding of the antibody to cells in the patient can be evaluated, e.g., by external scanning for radioactivity or by analyzing a biopsy taken from a patient previously exposed to the antibody.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

The word "label" when used herein refers to a detectable compound or composition which is conjugated directly or indirectly to the antibody, oligopeptide or other organic molecule so as to generate a "labeled" antibody, oligopeptide or other organic molecule. The label may be detectable by itself (e.g. radioisotope labels or fluorescent labels) or, in the case of an enzymatic label, may catalyze chemical alteration of a substrate compound or composition which is detectable.

The term "cytotoxic agent" as used herein refers to a substance that inhibits or prevents the function of cells and/or causes destruction of cells. The term is intended to include radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), chemotherapeutic agents e.g. methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "growth inhibitory agent" when used herein refers to a compound or composition which inhibits growth of a cell, especially a TAT-expressing cancer cell, either *in vitro* or *in vivo*. Thus, the growth inhibitory agent may be one which significantly reduces the percentage of TAT-expressing cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

"Doxorubicin" is an anthracycline antibiotic. The full chemical name of doxorubicin is (8S-cis)-10-[(3-amino-2,3,6-trideoxy- α- L- lyxo- hexapyranosyl) oxy]- 7,8,9,10- tetrahydro- 6,8,11- trihydroxy- 8-(hydroxyacetyl)- 1- methoxy- 5,12- naphthacenedione.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor, transforming growth factors (TGFs) such as TGF-α and TGF- β; insulin-like growth factor-I and -II; erythropoietin (EPO); osteoinductive factors; interferons such as interferon -α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1a, IL-2, BL-3, IL-4, IL-5, IL-6, IL-7, IL-8, IL-9, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "package insert" is used to refer to instructions customarily included in commercial packages of therapeutic products, that contain information about the indications, usage, dosage, administration, contraindications and/or warnings concerning the use of such therapeutic products.

## Table 1

```
/*
 *
 * C-C increased from 12 to 15
 * Z is average of EQ
 * B is average of ND
 * match with stop is _M; stop-stop = 0; J (joker) match = 0
 */
#define  _M      -8        /* value of a match with a stop */

int      _day[26][26] = {
/*    A B C D E F G H I J K L M N O P Q R S T U V W X Y Z */
/* A */   { 2, 0,-2, 0, 0,-4, 1,-1,-1, 0,-1,-2,-1, 0,_M, 1, 0,-2, 1, 1, 0, 0,-6, 0,-3, 0},
/* B */   { 0, 3,-4, 3, 2,-5, 0, 1,-2, 0, 0,-3,-2, 2,_M,-1, 1, 0, 0, 0, 0,-2,-5, 0,-3, 1},
/* C */   {-2,-4,15,-5,-5,-4,-3,-3,-2, 0,-5,-6,-5,-4,_M,-3,-5,-4, 0,-2, 0,-2,-8, 0, 0,-5},
/* D */   { 0, 3,-5, 4, 3,-6, 1, 1,-2, 0, 0,-4,-3, 2,_M,-1, 2,-1, 0, 0, 0,-2,-7, 0,-4, 2},
/* E */   { 0, 2,-5, 3, 4,-5, 0, 1,-2, 0, 0,-3,-2, 1,_M,-1, 2,-1, 0, 0, 0,-2,-7, 0,-4, 3},
/* F */   {-4,-5,-4,-6,-5, 9,-5,-2, 1, 0,-5, 2, 0,-4,_M,-5,-5,-4,-3,-3, 0,-1, 0, 0, 7,-5},
/* G */   { 1, 0,-3, 1, 0,-5, 5,-2,-3, 0,-2,-4,-3, 0,_M,-1,-1,-3, 1, 0, 0,-1,-7, 0,-5, 0},
/* H */   {-1, 1,-3, 1, 1,-2,-2, 6,-2, 0, 0,-2,-2, 2,_M, 0, 3, 2,-1,-1, 0,-2,-3, 0, 0, 2},
/* I */   {-1,-2,-2,-2,-2, 1,-3,-2, 5, 0,-2, 2, 2,-2,_M,-2,-2,-2,-1, 0, 0, 4,-5, 0,-1,-2},
/* J */   { 0, 0, 0, 0, 0, 0, 0, 0, 0, 0, 0, 0, 0, 0,_M, 0, 0, 0, 0, 0, 0, 0, 0, 0, 0, 0},
/* K */   {-1, 0,-5, 0, 0,-5,-2, 0,-2, 0, 5,-3, 0, 1,_M,-1, 1, 3, 0, 0, 0,-2,-3, 0,-4, 0},
/* L */   {-2,-3,-6,-4,-3, 2,-4,-2, 2, 0,-3, 6, 4,-3,_M,-3,-2,-3,-3,-1, 0, 2,-2, 0,-1,-2},
/* M */   {-1,-2,-5,-3,-2, 0,-3,-2, 2, 0, 0, 4, 6,-2,_M,-2,-1, 0,-2,-1, 0, 2,-4, 0,-2,-1},
/* N */   { 0, 2,-4, 2, 1,-4, 0, 2,-2, 0, 1,-3,-2, 2,_M,-1, 1, 0, 1, 0, 0,-2,-4, 0,-2, 1},
/* O */   {_M,_M,_M,_M,_M,_M,_M,_M,_M,_M,_M,_M,_M,_M, 0,_M,_M,_M,_M,_M,_M,_M,_M,_M,_M,_M},
/* P */   { 1,-1,-3,-1,-1,-5,-1, 0,-2, 0,-1,-3,-2,-1,_M, 6, 0, 0, 1, 0, 0,-1,-6, 0,-5, 0},
/* Q */   { 0, 1,-5, 2, 2,-5,-1, 3,-2, 0, 1,-2,-1, 1,_M, 0, 4, 1,-1,-1, 0,-2,-5, 0,-4, 3},
/* R */   {-2, 0,-4,-1,-1,-4,-3, 2,-2, 0, 3,-3, 0, 0,_M, 0, 1, 6, 0,-1, 0,-2, 2, 0,-4, 0},
/* S */   { 1, 0, 0, 0, 0,-3, 1,-1,-1, 0, 0,-3,-2, 1,_M, 1,-1, 0, 2, 1, 0,-1,-2, 0,-3, 0},
/* T */   { 1, 0,-2, 0, 0,-3, 0,-1, 0, 0, 0,-1,-1, 0,_M, 0,-1,-1, 1, 3, 0, 0,-5, 0,-3, 0},
/* U */   { 0, 0, 0, 0, 0, 0, 0, 0, 0, 0, 0, 0, 0, 0,_M, 0, 0, 0, 0, 0, 0, 0, 0, 0, 0, 0},
/* V */   { 0,-2,-2,-2,-2,-1,-1,-2, 4, 0,-2, 2, 2,-2,_M,-1,-2,-2,-1, 0, 0, 4,-6, 0,-2,-2},
/* W */   {-6,-5,-8,-7,-7, 0,-7,-3,-5, 0,-3,-2,-4,-4,_M,-6,-5, 2,-2,-5, 0,-6,17, 0, 0,-6},
/* X */   { 0, 0, 0, 0, 0, 0, 0, 0, 0, 0, 0, 0, 0, 0,_M, 0, 0, 0, 0, 0, 0, 0, 0, 0, 0, 0},
/* Y */   {-3,-3, 0,-4,-4, 7,-5, 0,-1, 0,-4,-1,-2,-2,_M,-5,-4,-4,-3,-3, 0,-2, 0, 0,10,-4},
/* Z */   { 0, 1,-5, 2, 3,-5, 0, 2,-2, 0, 0,-2,-1, 1,_M, 0, 3, 0, 0, 0, 0,-2,-6, 0,-4, 4}
};
```

## Table 1 (cont')

```
/*
 */
#include <stdio.h>
#include <ctype.h>

#define  MAXJMP     16      /* max jumps in a diag */
#define  MAXGAP     24      /* don't continue to penalize gaps larger than this */
#define  JMPS       1024    /* max jmps in an path */
#define  MX         4       /* save if there's at least MX-1 bases since last jmp */

#define  DMAT       3       /* value of matching bases */
#define  DMIS       0       /* penalty for mismatched bases */
#define  DINS0      8       /* penalty for a gap */
#define  DINS1      1       /* penalty per base */
#define  PINS0      8       /* penalty for a gap */
#define  PINS1      4       /* penalty per residue */

struct jmp {
        short           n[MAXJMP];      /* size of jmp (neg for dely) */
        unsigned short  x[MAXJMP];      /* base no. of jmp in seq x */
};                                      /* limits seq to 2^16 -1 */

struct diag {
        int             score;          /* score at last jmp */
        long            offset;         /* offset of prev block */
        short           ijmp;           /* current jmp index */
        struct jmp      jp;             /* list of jmps */
};

struct path {
        int     spc;                    /* number of leading spaces */
        short   n[JMPS];/* size of jmp (gap) */
        int     x[JMPS];/* loc of jmp (last elem before gap) */
};

char            *ofile;                 /* output file name */
char            *namex[2];              /* seq names: getseqs() */
char            *prog;                  /* prog name for err msgs */
char            *seqx[2];               /* seqs: getseqs() */
int             dmax;                   /* best diag: nw() */
int             dmax0;                  /* final diag */
int             dna;                    /* set if dna: main() */
int             endgaps;                /* set if penalizing end gaps */
int             gapx, gapy;             /* total gaps in seqs */
int             len0, len1;             /* seq lens */
int             ngapx, ngapy;           /* total size of gaps */
int             smax;                   /* max score: nw() */
int             *xbm;                   /* bitmap for matching */
long            offset;                 /* current offset in jmp file */
struct  diag    *dx;                    /* holds diagonals */
struct  path    pp[2];                  /* holds path for seqs */

char            *calloc(), *malloc(), *index(), *strcpy();
char            *getseq(), *g_calloc();
```

## Table 1 (cont')

```
/* Needleman-Wunsch alignment program
 *
 * usage: progs file1 file2
 *   where file1 and file2 are two dna or two protein sequences.
 *   The sequences can be in upper- or lower-case an may contain ambiguity
 *   Any lines beginning with ';', '>' or '<' are ignored
 *   Max file length is 65535 (limited by unsigned short x in the jmp struct)
 *   A sequence with 1/3 or more of its elements ACGTU is assumed to be DNA
 *   Output is in the file "align.out"
 *
 * The program may create a tmp file in /tmp to hold info about traceback.
 * Original version developed under BSD 4.3 on a vax 8650
 */
#include "nw.h"
#include "day.h"

static    _dbval[26] = {
          1,14,2,13,0,0,4,11,0,0,12,0,3,15,0,0,0,5,6,8,8,7,9,0,10,0
};

static    _pbval[26] = {
          1, 2|(1<<('D'-'A'))|(1<<('N'-'A')), 4, 8, 16, 32, 64,
          128, 256, 0xFFFFFFF, 1<<10, 1<<11, 1<<12, 1<<13, 1<<14,
          1<<15, 1<<16, 1<<17, 1<<18, 1<<19, 1<<20, 1<<21, 1<<22,
          1<<23, 1<<24, 1<<25|(1<<('E'-'A'))|(1<<('Q'-'A'))
};

main(ac, av)                                                              main
          int     ac;
          char    *av[];
{
          prog = av[0];
          if (ac != 3) {
                    fprintf(stderr,"usage: %s file1 file2\n", prog);
                    fprintf(stderr,"where file1 and file2 are two dna or two protein sequences.\n");
                    fprintf(stderr,"The sequences can be in upper- or lower-case\n");
                    fprintf(stderr,"Any lines beginning with ';' or '<' are ignored\n");
                    fprintf(stderr,"Output is in the file \"align.out\"\n");
                    exit(1);
          }
          namex[0] = av[1];
          namex[1] = av[2];
          seqx[0] = getseq(namex[0], &len0);
          seqx[1] = getseq(namex[1], &len1);
          xbm = (dna)? _dbval : _pbval;

          endgaps = 0;              /* 1 to penalize endgaps */
          ofile = "align.out";      /* output file */

          nw();             /* fill in the matrix, get the possible jmps */
          readjmps();       /* get the actual jmps */
          print();          /* print stats, alignment */

          cleanup(0);       /* unlink any tmp files */}
```

## Table 1 (cont')

```
/* do the alignment, return best score: main()
 * dna: values in Fitch and Smith, PNAS, 80, 1382-1386, 1983
 * pro: PAM 250 values
 * When scores are equal, we prefer mismatches to any gap, prefer
 * a new gap to extending an ongoing gap, and prefer a gap in seqx
 * to a gap in seq y.
 */
nw()                                                                    nw
{
        char            *px, *py;           /* seqs and ptrs */
        int             *ndely, *dely;      /* keep track of dely */
        int             ndelx, delx;        /* keep track of delx */
        int             *tmp;               /* for swapping row0, row1 */
        int             mis;                /* score for each type */
        int             ins0, ins1;         /* insertion penalties */
        register        id;                 /* diagonal index */
        register        ij;                 /* jmp index */
        register        *col0, *col1;       /* score for curr, last row */
        register        xx, yy;             /* index into seqs */

        dx = (struct diag *)g_calloc("to get diags", len0+len1+1, sizeof(struct diag));
        ndely = (int *)g_calloc("to get ndely", len1+1, sizeof(int));
        dely = (int *)g_calloc("to get dely", len1+1, sizeof(int));
        col0 = (int *)g_calloc("to get col0", len1+1, sizeof(int));
        col1 = (int *)g_calloc("to get col1", len1+1, sizeof(int));
        ins0 = (dna)? DINS0 : PINS0;
        ins1 = (dna)? DINS1 : PINS1;
        smax = -10000;
        if (endgaps) {
                for (col0[0] = dely[0] = -ins0, yy = 1; yy <= len1; yy++) {
                        col0[yy] = dely[yy] = col0[yy-1] - ins1;
                        ndely[yy] = yy;
                }
                col0[0] = 0;        /* Waterman Bull Math Biol 84 */
        }
        else
                for (yy = 1; yy <= len1; yy++)
                        dely[yy] = -ins0;
        /* fill in match matrix
         */
        for (px = seqx[0], xx = 1; xx <= len0; px++, xx++) {
                /* initialize first entry in col
                 */
                if (endgaps) {
                        if (xx == 1)
                                col1[0] = delx = -(ins0+ins1);
                        else
                                col1[0] = delx = col0[0] - ins1;
                        ndelx = xx;
                }
                else {
                        col1[0] = 0;
                        delx = -ins0;
                        ndelx = 0;
                }
```

## Table 1 (cont')

...nw

```
for (py = seqx[1], yy = 1; yy <= len1; py++, yy++) {
        mis = col0[yy-1];
        if (dna)
                mis += (xbm[*px-'A']&xbm[*py-'A'])? DMAT : DMIS;
        else
                mis += _day[*px-'A'][*py-'A'];

        /* update penalty for del in x seq;
         * favor new del over ongong del
         * ignore MAXGAP if weighting endgaps
         */
        if (endgaps || ndely[yy] < MAXGAP) {
                if (col0[yy] - ins0 >= dely[yy]) {
                        dely[yy] = col0[yy] - (ins0+ins1);
                        ndely[yy] = 1;
                } else {
                        dely[yy] -= ins1;
                        ndely[yy]++;
                }
        } else {
                if (col0[yy] - (ins0+ins1) >= dely[yy]) {
                        dely[yy] = col0[yy] - (ins0+ins1);
                        ndely[yy] = 1;
                } else
                        ndely[yy]++;
        }

        /* update penalty for del in y seq;
         * favor new del over ongong del
         */
        if (endgaps || ndelx < MAXGAP) {
                if (col1[yy-1] - ins0 >= delx) {
                        delx = col1[yy-1] - (ins0+ins1);
                        ndelx = 1;
                } else {
                        delx -= ins1;
                        ndelx++;
                }
        } else {
                if (col1[yy-1] - (ins0+ins1) >= delx) {
                        delx = col1[yy-1] - (ins0+ins1);
                        ndelx = 1;
                } else
                        ndelx++;
        }

        /* pick the maximum score; we're favoring
         * mis over any del and delx over dely
         */
```

...nw

```
        id = xx - yy + len1 - 1;
        if (mis >= delx && mis >= dely[yy])
                col1[yy] = mis;
```

## Table 1 (cont')

```
                    else if (delx >= dely[yy]) {
                            col1[yy] = delx;
                            ij = dx[id].ijmp;
                            if (dx[id].jp.n[0] && (!dna || (ndelx >= MAXJMP
                            && xx > dx[id].jp.x[ij]+MX) || mis > dx[id].score+DINS0)) {
                                    dx[id].ijmp++;
                                    if (++ij >= MAXJMP) {
                                            writejmps(id);
                                            ij = dx[id].ijmp = 0;
                                            dx[id].offset = offset;
                                            offset += sizeof(struct jmp) + sizeof(offset);
                                    }
                            }
                            dx[id].jp.n[ij] = ndelx;
                            dx[id].jp.x[ij] = xx;
                            dx[id].score = delx;
                    }
                    else {
                            col1[yy] = dely[yy];
                            ij = dx[id].ijmp;
if (dx[id].jp.n[0] && (!dna || (ndely[yy] >= MAXJMP
                            && xx > dx[id].jp.x[ij]+MX) || mis > dx[id].score+DINS0)) {
                                    dx[id].ijmp++;
                                    if (++ij >= MAXJMP) {
                                            writejmps(id);
                                            ij = dx[id].ijmp = 0;
                                            dx[id].offset = offset;
                                            offset += sizeof(struct jmp) + sizeof(offset);
                                    }
                            }
                            dx[id].jp.n[ij] = -ndely[yy];
                            dx[id].jp.x[ij] = xx;
                            dx[id].score = dely[yy];
                    }
                    if (xx == len0 && yy < len1) {
                            /* last col
                             */
                            if (endgaps)
                                    col1[yy] -= ins0+ins1*(len1-yy);
                            if (col1[yy] > smax) {
                                    smax = col1[yy];
                                    dmax = id;
                            }
                    }
            }
            if (endgaps && xx < len0)
                    col1[yy-1] -= ins0+ins1*(len0-xx);
            if (col1[yy-1] > smax) {
                    smax = col1[yy-1];
                    dmax = id;
            }
            tmp = col0; col0 = col1; col1 = tmp;                    }
(void) free((char *)ndely);
(void) free((char *)dely);
(void) free((char *)col0);
(void) free((char *)col1);                    }
```

## Table 1 (cont')

```
/*
 *
 * print() -- only routine visible outside this module
 *
 * static:
 * getmat() -- trace back best path, count matches: print()
 * pr_align() -- print alignment of described in array p[]: print()
 * dumpblock() -- dump a block of lines with numbers, stars: pr_align()
 * nums() -- put out a number line: dumpblock()
 * putline() -- put out a line (name, [num], seq, [num]): dumpblock()
 * stars() - -put a line of stars: dumpblock()
 * stripname() -- strip any path and prefix from a seqname
 */

#include "nw.h"

#define SPC      3
#define P_LINE   256     /* maximum output line */
#define P_SPC    3       /* space between name or num and seq */

extern   _day[26][26];
int      olen;           /* set output line length */
FILE     *fx;            /* output file */

print()                                                              print
{
        int     lx, ly, firstgap, lastgap;      /* overlap */

        if ((fx = fopen(ofile, "w")) == 0) {
                fprintf(stderr,"%s: can't write %s\n", prog, ofile);
                cleanup(1);
        }
        fprintf(fx, "<first sequence: %s (length = %d)\n", namex[0], len0);
        fprintf(fx, "<second sequence: %s (length = %d)\n", namex[1], len1);
        olen = 60;
        lx = len0;
        ly = len1;
        firstgap = lastgap = 0;
        if (dmax < len1 - 1) {          /* leading gap in x */
                pp[0].spc = firstgap = len1 - dmax - 1;
                ly -= pp[0].spc;
        }
        else if (dmax > len1 - 1) {     /* leading gap in y */
                pp[1].spc = firstgap = dmax - (len1 - 1);
                lx -= pp[1].spc;
        }
        if (dmax0 < len0 - 1) {         /* trailing gap in x */
                lastgap = len0 - dmax0 -1;
                lx -= lastgap;
        }
        else if (dmax0 > len0 - 1) {    /* trailing gap in y */
                lastgap = dmax0 - (len0 - 1);
                ly -= lastgap;
        }
        getmat(lx, ly, firstgap, lastgap);
        pr_align();             }
```

Table 1 (cont')

```
/*
 * trace back the best path, count matches
 */
static
getmat(lx, ly, firstgap, lastgap)                                                    getmat
        int     lx, ly;                     /* "core" (minus endgaps) */
        int     firstgap, lastgap;          /* leading trailing overlap */
{
        int             nm, i0, i1, siz0, siz1;
        char            outx[32];
        double          pct;
        register        n0, n1;
        register char   *p0, *p1;
        /* get total matches, score
         */
        i0 = i1 = siz0 = siz1 = 0;
        p0 = seqx[0] + pp[1].spc;
        p1 = seqx[1] + pp[0].spc;
        n0 = pp[1].spc + 1;
        n1 = pp[0].spc + 1;
        nm = 0;
        while ( *p0 && *p1 ) {
                if (siz0) {
                        p1++;
                        n1++;
                        siz0--;
                }
                else if (siz1) {
                        p0++;
                        n0++;
                        siz1--;
                }
                else {
                        if (xbm[*p0-'A']&xbm[*p1-'A'])
                                nm++;
                        if (n0++ == pp[0].x[i0])
                                siz0 = pp[0].n[i0++];
                        if (n1++ == pp[1].x[i1])
                                siz1 = pp[1].n[i1++];
                        p0++;
                        p1++;
                }
        }

        /* pct homology:
         * if penalizing endgaps, base is the shorter seq
         * else, knock off overhangs and take shorter core
         */
        if (endgaps)
                lx = (len0 < len1)? len0 : len1;
        else
                lx = (lx < ly)? lx : ly;
        pct = 100.*(double)nm/(double)lx;
        fprintf(fx, "\n");
        fprintf(fx, "<%d match%s in an overlap of %d: %.2f percent similarity\n",
                nm, (nm == 1)? "" : "es", lx, pct);
```

## Table 1 (cont')

...getmat

```
		fprintf(fx, "<gaps in first sequence: %d", gapx);
		if (gapx) {
			(void) sprintf(outx, " (%d %s%s)",
				ngapx, (dna)? "base":"residue", (ngapx == 1)? "":"s");
			fprintf(fx,"%s", outx);
		fprintf(fx, ", gaps in second sequence: %d", gapy);
		if (gapy) {
			(void) sprintf(outx, " (%d %s%s)",
				ngapy, (dna)? "base":"residue", (ngapy == 1)? "":"s");
			fprintf(fx,"%s", outx);
		}
		if (dna)
			fprintf(fx,
			"\n<score: %d (match = %d, mismatch = %d, gap penalty = %d + %d per base)\n",
			smax, DMAT, DMIS, DINS0, DINS1);
		else
			fprintf(fx,
			"\n<score: %d (Dayhoff PAM 250 matrix, gap penalty = %d + %d per residue)\n",
			smax, PINS0, PINS1);
		if (endgaps)
			fprintf(fx,
			"<endgaps penalized. left endgap: %d %s%s, right endgap: %d %s%s\n",
			firstgap, (dna)? "base" : "residue", (firstgap == 1)? "" : "s",
			lastgap, (dna)? "base" : "residue", (lastgap == 1)? "" : "s");
		else
			fprintf(fx, "<endgaps not penalized\n");
}
 static		nm;		/* matches in core -- for checking */
 static		lmax;		/* lengths of stripped file names */
 static		ij[2];		/* jmp index for a path */
 static		nc[2];		/* number at start of current line */
 static		ni[2];		/* current elem number -- for gapping */
 static		siz[2];
 static char	*ps[2];		/* ptr to current element */
 static char	*po[2];		/* ptr to next output char slot */
 static char	out[2][P_LINE];	/* output line */
 static char	star[P_LINE];	/* set by stars() */
/*
 * print alignment of described in struct path pp[]
 */
static
pr_align()
{
	int		nn;	/* char count */
	int		more;
	register	i;

	for (i = 0, lmax = 0; i < 2; i++) {
		nn = stripname(namex[i]);
		if (nn > lmax)
			lmax = nn;
		nc[i] = 1;
		ni[i] = 1;
		siz[i] = ij[i] = 0;
		ps[i] = seqx[i];
		po[i] = out[i];		}
```

pr_align

Table 1 (cont')

...pr_align

```
    for (nn = nm = 0, more = 1; more; ) {
        for (i = more = 0; i < 2; i++) {
            /*
             * do we have more of this sequence?
             */
            if (!*ps[i])
                continue;
            more++;
            if (pp[i].spc) {    /* leading space */
                *po[i]++ = ' ';
                pp[i].spc--;
            }
            else if (siz[i]) {  /* in a gap */
                *po[i]++ = '-';
                siz[i]--;
            }
            else {              /* we're putting a seq element
                                 */
                *po[i] = *ps[i];
                if (islower(*ps[i]))
                    *ps[i] = toupper(*ps[i]);
                po[i]++;
                ps[i]++;
                /*
                 * are we at next gap for this seq?
                 */
                if (ni[i] == pp[i].x[ij[i]]) {
                    /*
                     * we need to merge all gaps
                     * at this location
                     */
                    siz[i] = pp[i].n[ij[i]++];
                    while (ni[i] == pp[i].x[ij[i]])
                        siz[i] += pp[i].n[ij[i]++];
                }
                ni[i]++;
            }
        }
        if (++nn == olen || !more && nn) {
            dumpblock();
            for (i = 0; i < 2; i++)
                po[i] = out[i];
            nn = 0;
        }
    }
}
/*
 * dump a block of lines, including numbers, stars: pr_align()
 */
static
dumpblock()
{
    register i;
    for (i = 0; i < 2; i++)
        *po[i]-- = '\0';
```

dumpblock

Table 1 (cont')

...dumpblock

```
        (void) putc('\n', fx);
        for (i = 0; i < 2; i++) {
                if (*out[i] && (*out[i] != ' ' || *(po[i]) != ' ')) {
                        if (i == 0)
                                nums(i);
                        if (i == 0 && *out[1])
                                stars();
                        putline(i);
                        if (i == 0 && *out[1])
                                fprintf(fx, star);
                        if (i == 1)
                                nums(i);
                }
        }
}
/*
 * put out a number line: dumpblock()
 */
static
nums(ix)                                                                nums
        int     ix;     /* index in out[] holding seq line */
{
        char            nline[P_LINE];
        register        i, j;
        register char   *pn, *px, *py;
        for (pn = nline, i = 0; i < lmax+P_SPC; i++, pn++)
                *pn = ' ';
        for (i = nc[ix], py = out[ix]; *py; py++, pn++) {
                if (*py == ' ' || *py == '-')
                        *pn = ' ';
                else {
                        if (i%10 == 0 || (i == 1 && nc[ix] != 1)) {
                                j = (i < 0)? -i : i;
                                for (px = pn; j; j /= 10, px--)
                                        *px = j%10 + '0';
                                if (i < 0)
                                        *px = '-';
                        }
                        else
                                *pn = ' ';
                        i++;
                }
        }
        *pn = '\0';
        nc[ix] = i;
        for (pn = nline; *pn; pn++)
                (void) putc(*pn, fx);
        (void) putc('\n', fx);
}
/*
 * put out a line (name, [num], seq, [num]): dumpblock()
 */
static
putline(ix)                                                             putline
        int     ix;                     {
```

## Table 1 (cont')

...putline

```
        int                 i;
        register char       *px;

        for (px = namex[ix], i = 0; *px && *px != ':'; px++, i++)
                (void) putc(*px, fx);
        for (; i < lmax+P_SPC; i++)
                (void) putc(' ', fx);

        /* these count from 1:
         * ni[] is current element (from 1)
         * nc[] is number at start of current line
         */
        for (px = out[ix]; *px; px++)
                (void) putc(*px&0x7F, fx);
        (void) putc('\n', fx);
}


/*
 * put a line of stars (seqs always in out[0], out[1]): dumpblock()
 */
static
stars()
{
        int                 i;
        register char       *p0, *p1, cx, *px;

        if (!*out[0] || (*out[0] == ' ' && *(po[0]) == ' ') ||
           !*out[1] || (*out[1] == ' ' && *(po[1]) == ' '))
                return;
        px = star;
        for (i = lmax+P_SPC; i; i--)
                *px++ = ' ';

        for (p0 = out[0], p1 = out[1]; *p0 && *p1; p0++, p1++) {
                if (isalpha(*p0) && isalpha(*p1)) {

                        if (xbm[*p0-'A']&xbm[*p1-'A']) {
                                cx = '*';
                                nm++;
                        }
                        else if (!dna && _day[*p0-'A'][*p1-'A'] > 0)
                                cx = '.';
                        else
                                cx = ' ';
                }
                else
                        cx = ' ';
                *px++ = cx;
        }
        *px++ = '\n';
        *px = '\0';
}
```

stars

## Table 1 (cont')

stripname

```
/*
 * strip path or prefix from pn, return len: pr_align()
 */
static
stripname(pn)
        char    *pn;    /* file name (may be path) */
{
        register char   *px, *py;

        py = 0;
        for (px = pn; *px; px++)
                if (*px == '/')
                        py = px + 1;
        if (py)
                (void) strcpy(pn, py);
        return(strlen(pn));
}
```

## Table 1 (cont')

```
/*
 * cleanup() -- cleanup any tmp file
 * getseq() -- read in seq, set dna, len, maxlen
 * g_calloc() -- calloc() with error checkin
 * readjmps() -- get the good jmps, from tmp file if necessary
 * writejmps() -- write a filled array of jmps to a tmp file: nw()
 */
#include "nw.h"
#include <sys/file.h>

char    *jname = "/tmp/homgXXXXXX";         /* tmp file for jmps */
FILE    *fj;
int     cleanup();                          /* cleanup tmp file */
long    lseek();
/*
 * remove any tmp file if we blow
 */
cleanup(i)                                                          cleanup
        int     i;
{
        if (fj)
                (void) unlink(jname);
        exit(i);
}
/*
 * read, return ptr to seq, set dna, len, maxlen
 * skip lines starting with ';', '<', or '>'
 * seq in upper or lower case
 */
char    *
getseq(file, len)                                                   getseq
        char    *file;  /* file name */
        int     *len;   /* seq len */
{
        char            line[1024], *pseq;
        register char   *px, *py;
        int             natgc, tlen;
        FILE            *fp;
        if ((fp = fopen(file,"r")) == 0) {
                fprintf(stderr,"%s: can't read %s\n", prog, file);
                exit(1);
        }
        tlen = natgc = 0;
        while (fgets(line, 1024, fp)) {
                if (*line == ';' || *line == '<' || *line == '>')
                        continue;
                for (px = line; *px != '\n'; px++)
                        if (isupper(*px) || islower(*px))
                                tlen++;
        }
        if ((pseq = malloc((unsigned)(tlen+6))) == 0) {
                fprintf(stderr,"%s: malloc() failed to get %d bytes for %s\n", prog, tlen+6, file);
                exit(1);
        }
        pseq[0] = pseq[1] = pseq[2] = pseq[3] = '\0';
```

## Table 1 (cont')

**...getseq**

```
        py = pseq + 4;
        *len = tlen;
        rewind(fp);
        while (fgets(line, 1024, fp)) {
                if (*line == ';' || *line == '<' || *line == '>')
                        continue;
                for (px = line; *px != '\n'; px++) {
                        if (isupper(*px))
                                *py++ = *px;
                        else if (islower(*px))
                                *py++ = toupper(*px);
                        if (index("ATGCU",*(py-1)))
                                natgc++;
                }
        }
        *py++ = '\0';
        *py = '\0';
        (void) fclose(fp);
        dna = natgc > (tlen/3);
        return(pseq+4);
}
char    *
g_calloc(msg, nx, sz)
        char    *msg;           /* program, calling routine */
        int     nx, sz;         /* number and size of elements */
{
        char            *px, *calloc();
        if ((px = calloc((unsigned)nx, (unsigned)sz)) == 0) {
                if (*msg) {
                        fprintf(stderr, "%s: g_calloc() failed %s (n=%d, sz=%d)\n", prog, msg, nx, sz);
                        exit(1);
                }
        }
        return(px);
}
```

**g_calloc**

```
/*
 * get final jmps from dx[] or tmp file, set pp[], reset dmax: main()
 */
readjmps()
{
        int             fd = -1;
        int             siz, i0, i1;
        register  i, j, xx;
        if (fj) {
                (void) fclose(fj);
                if ((fd = open(jname, O_RDONLY, 0)) < 0) {
                        fprintf(stderr, "%s: can't open() %s\n", prog, jname);
                        cleanup(1);
                }
        }
        for (i = i0 = i1 = 0, dmax0 = dmax, xx = len0; ; i++) {
                while (1) {
                        for (j = dx[dmax].ijmp; j >= 0 && dx[dmax].jp.x[j] >= xx; j--)
                                ;
```

**readjmps**

## Table 1 (cont')

**...readjmps**

```
                        if (j < 0 && dx[dmax].offset && fj) {
                                (void) lseek(fd, dx[dmax].offset, 0);
                                (void) read(fd, (char *)&dx[dmax].jp, sizeof(struct jmp));
                                (void) read(fd, (char *)&dx[dmax].offset, sizeof(dx[dmax].offset));
                                dx[dmax].ijmp = MAXJMP-1;                       }
                        else
                                break;          }
                if (i >= JMPS) {
                        fprintf(stderr, "%s: too many gaps in alignment\n", prog);
                        cleanup(1);
                }
                if (j >= 0) {
                        siz = dx[dmax].jp.n[j];
                        xx = dx[dmax].jp.x[j];
                        dmax += siz;
                        if (siz < 0) {                  /* gap in second seq */
                                pp[1].n[i1] = -siz;
                                xx += siz;
                                /* id = xx - yy + len1 - 1                      */
                                pp[1].x[i1] = xx - dmax + len1 - 1;
                                gapy++;
                                ngapy -= siz;
/* ignore MAXGAP when doing endgaps */
                                siz = (-siz < MAXGAP || endgaps)? -siz : MAXGAP;
                                i1++;
                        }
                        else if (siz > 0) {     /* gap in first seq */
                                pp[0].n[i0] = siz;
                                pp[0].x[i0] = xx;
                                gapx++;
                                ngapx += siz;
/* ignore MAXGAP when doing endgaps */
                                siz = (siz < MAXGAP || endgaps)? siz : MAXGAP;
                                i0++;
                        }
                }
                else
                        break;
        }
        /* reverse the order of jmps    */
        for (j = 0, i0--; j < i0; j++, i0--) {
                i = pp[0].n[j]; pp[0].n[j] = pp[0].n[i0]; pp[0].n[i0] = i;
                i = pp[0].x[j]; pp[0].x[j] = pp[0].x[i0]; pp[0].x[i0] = i;
        }
        for (j = 0, i1--; j < i1; j++, i1--) {
                i = pp[1].n[j]; pp[1].n[j] = pp[1].n[i1]; pp[1].n[i1] = i;
                i = pp[1].x[j]; pp[1].x[j] = pp[1].x[i1]; pp[1].x[i1] = i;
        }
        if (fd >= 0)
                (void) close(fd);
        if (fj) {
                (void) unlink(jname);
                fj = 0;
                offset = 0;
        }                               }
```

## Table 1 (cont')

```
/*
 * write a filled jmp struct offset of the prev one (if any): nw()
 */
writejmps(ix)                                                    writejmps
        int     ix;
{
        char    *mktemp();

        if (!fj) {
                if (mktemp(jname) < 0) {
                        fprintf(stderr, "%s: can't mktemp() %s\n", prog, jname);
                        cleanup(1);
                }
                if ((fj = fopen(jname, "w")) == 0) {
                        fprintf(stderr, "%s: can't write %s\n", prog, jname);
                        exit(1);
                }
        }
        (void) fwrite((char *)&dx[ix].jp, sizeof(struct jmp), 1, fj);
        (void) fwrite((char *)&dx[ix].offset, sizeof(dx[ix].offset), 1, fj);
}
```

**Table 2**

| | | |
|---|---|---|
| TAT | XXXXXXXXXXXXXXX | (Length = 15 amino acids) |
| Comparison Protein | XXXXXYYYYYYY | (Length = 12 amino acids) |

% amino acid sequence identity =
(the number of identically matching amino acid residues between the two polypeptide sequences as determined by ALIGN-2) divided by (the total number of amino residues of the TAT polypeptide) =
5 divided by 15 = 33.3%

**Table 3**

| | | |
|---|---|---|
| TAT | XXXXXXXXXX | (Length = 10 amino acids) |
| Comparison Protein | XXXXXYYYYYYZZYZ | (Length = 15 amino acids) |

% amino acid sequence identity =
(the number of identically matching amino acid residues between the two polypeptide sequences as determined by ALIGN-2) divided by (the total number of amino residues of the TAT polypeptide) =
5 divided by 15 = 50%

**Table 4**

| | | |
|---|---|---|
| TAT-DNA | NNNNNNNNNNNNNN | (Length = 14 nucleotides) |
| Comparison DNA | NNNNNNLLLLLLLLLL | (Length = 16 nucleotides) |

% amino acid sequence identity =
(the number of identically matching nucleotides between the two nucleic acid sequences as determined by ALIGN-2) divided by (the total number of nucleotides of the TAT-DNA nucleic acid sequence) =
6 divided by 14 = 42.9%

**Table 5**

| | | |
|---|---|---|
| TAT-DNA | NNNNNNNNNNNN | (Length =12 nucleotides) |
| Comparison DNA | NNNNLLLVV | (Length = 9 nucleotides) |

% nucleic acid sequence identity =
(the number of identically matching nucleotides between the two nucleic acid sequences as determined by ALIGN-2) divided by (the total number of nucleotides of the TAT-DNA nucleic acid sequence) =
4 divided by 12 = 33.3%

II. Compositions and Methods of the Invention

A. Anti-TAT Antibodies

In one embodiment, the present invention provides anti-TAT antibodies which may find use herein as therapeutic and/or diagnostic agents. Exemplary antibodies include polyclonal, monoclonal, humanized, bispecific, and heteroconjugate antibodies.

1. Polyclonal Antibodies

Polyclonal antibodies are preferably raised in animals by multiple subcutaneous (sc) or intraperitoneal (ip) injections of the relevant antigen and an adjuvant. It may be useful to conjugate the relevant antigen (especially when synthetic peptides are used) to a protein that is immunogenic in the species to be immunized. For example, the antigen can be conjugated to keyhole limpet hemocyanin (KLH), serum albumin, bovine thyroglobulin, or soybean trypsin inhibitor, using a bifunctional or derivatizing agent, e.g., maleimidobenzoyl sulfosuccinimide ester (conjugation through cysteine residues), N-hydroxysuccinimide (through lysine residues), glutaraldehyde, succinic anhydride, $SOCl_2$, or $R^1N=C=NR$, where R and $R^1$ are different alkyl groups.
Animals are immunized against the antigen, immunogenic conjugates, or derivatives by combining, e.g., 100 μg or 5 μg of the protein or conjugate (for rabbits or mice, respectively) with 3 volumes of Freund's complete adjuvant and injecting the solution intradermally at multiple sites. One month later, the animals are boosted with 1/5 to 1/10 the original amount of peptide or conjugate in Freund's complete adjuvant by subcutaneous injection at multiple sites. Seven to 14 days later, the animals are bled and the serum is assayed for antibody titer. Animals are boosted until the titer plateaus. Conjugates also can be made in recombinant cell culture as protein fusions. Also, aggregating agents such as alum are suitably used to enhance the immune response.

2. Monoclonal Antibodies

Monoclonal antibodies may be made using the hybridoma method first described by Kohler et al., Nature, 256: 495 (1975), or may be made by recombinant DNA methods (U.S. Patent No. 4,816,567).
In the hybridoma method, a mouse or other appropriate host animal, such as a hamster, is immunized as described above to elicit lymphocytes that produce or are capable of producing antibodies that will specifically bind to the protein used for immunization. Alternatively, lymphocytes may be immunized *in vitro.* After immunization, lymphocytes are isolated and then fused with a myeloma cell line using a suitable fusing agent, such as polyethylene glycol, to form a hybridoma cell (Goding, Monoclonal Antibodies: Principles and Practice, pp.59-103 (Academic Press, 1986)).
The hybridoma cells thus prepared are seeded and grown in a suitable culture medium which medium preferably contains one or more substances that inhibit the growth or survival of the unfused, parental myeloma cells (also referred to as fusion partner). For example, if the parental myeloma cells lack the enzyme hypoxanthine guanine phosphoribosyl transferase (HGPRT or HPRT), the selective culture medium for the hybridomas typically will include hypoxanthine, aminopterin, and thymidine (HAT medium), which substances prevent the growth of HGPRT-deficient cells.
Preferred fusion partner myeloma cells are those that fuse efficiently, support stable high-level production of antibody by the selected antibody-producing cells, and are sensitive to a selective medium that selects against the unfused parental cells. Preferred myeloma cell lines are murine myeloma lines, such as those derived from MOPC-21 and MPC-11 mouse tumors available from the Salk Institute Cell Distribution Center, San Diego, California USA, and SP-2 and derivatives e.g., X63-Ag8-653 cells available from the American Type Culture Collection, Manassas, Virginia, USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunosorbent assay (ELISA).

The binding affinity of the monoclonal antibody can, for example, be determined by the Scatchard analysis described in Munson et al., Anal. Biochem., 107:220 (1980).

Once hybridoma cells that produce antibodies of the desired specificity, affinity, and/or activity are identified, the clones may be subcloned by limiting dilution procedures and grown by standard methods (Goding, Monoclonal Antibodies: Principles and Practice, pp.59-103 (Academic Press,1986)). Suitable culture media for this purpose include, for example, D-MEM or RPMI-1640 medium. In addition, the hybridoma cells may be grown in *vivo* as ascites tumors in an animal e.g,, by i.p. injection of the cells into mice.

The monoclonal antibodies secreted by the subclones are suitably separated from the culture medium, ascites fluid, or serum by conventional antibody purification procedures such as, for example, affinity chromatography (e.g., using protein A or protein G-Sepharose) or ion-exchange chromatography, hydroxylapatite chromatography, gel electrophoresis, dialysis, etc.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Plückthun, Immunol. Revs. 130:151-188 (1992).

In a further embodiment, monoclonal antibodies or antibody fragments can be isolated from antibody phage libraries generated using the techniques described in McCafferty et al., Nature, 348:552-554(1990). Clackson et al., Nature, 352: 624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res. 21: 2265-2266 (1993)). Thus, these techniques are viable alternatives to traditional monoclonal antibody hybridoma techniques for isolation of monoclonal antibodies.

The DNA that encodes the antibody may be modified to produce chimeric or fusion antibody polypeptides, for example, by substituting human heavy chain and light chain constant domain ($C_H$ and $C_L$) sequences for the homologous murine sequences (U.S. Patent No. 4,816,567; and Morrison, et al., Proc. Natl Acad. Sci. USA, 81:6851 (1984)), or by fusing the immunoglobulin coding sequence with all or part of the coding sequence for a non-immunoglobulin polypeptide (heterologous polypeptide). The non-immunoglobulin polypeptide sequences can substitute for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

3. Human and Humanized Antibodies

The anti-TAT antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity and HAMA response (human anti-mouse antibody) when the antibody is intended for human therapeutic use. According to the so-called "best-fit" method, the sequence of the variable domain of a rodent antibody is screened against the entire library of known human variable domain sequences. The human V domain sequence which is closest to that of the rodent is identified and the human framework region (FR) within it accepted for the humanized antibody (Sims et al., J. Immunol. 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol. 151:2623 (1993)).

It is further important that antibodies be humanized with retention of high binding affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Various forms of a humanized anti-TAT antibody are contemplated. For example, the humanized antibody may be an antibody fragment, such as a Fab, which is optionally conjugated with one or more cytotoxic agent(s) in order to generate an immunoconjugate. Alternatively, the humanized antibody may be an intact antibody, such as an intact IgG1 antibody.

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region ($J_H$) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, e.g., Jakobovits et al., Proc. Natl. Acad. Sci. USA, 90:2551 (1993); Jakobovits et al., Nature, 362:255-258 (1993); Bruggemann et al., Year in Immuno. 7:33 (1993); U.S. PatentNos. 5,545,806, 5,569,825, 5,591,669 (all of GenPharm); 5,545,807; and WO 97/17852.

Alternatively, phage display technology (McCafferty et al., Nature 348:552-553 [1990]) can be used to produce human antibodies and antibody fragments *in vitro*, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats, reviewed in, e.g., Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J.12:725-734 (1993). See, also, U.S. Patent Nos. 5,565,332 and 5,573,905.

As discussed above, human antibodies may also be generated by *in vitro* activated B cells (see U.S. Patents 5,567,610 and 5,229,275).

4. Antibody fragments

In certain circumstances there are advantages of using antibody fragments, rather than whole antibodies. The smaller size of the fragments allows for rapid clearance, and may lead to improved access to solid tumors. Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from *E. coli*, thus allowing the facile production of large amounts of these fragments. Antibody fragments can be isolated from the antibody phage libraries discussed above. Alternatively, Fab'-SH fragments can be directly recovered from *E. coli* and chemically coupled to form $F(ab')_2$ fragments (Carter et al., Bio/Technology 10:163-167 (1992)). According to another approach, $F(ab')_2$ fragments can be isolated directly from recombinant host cell culture. Fab and $F(ab')_2$ fragment with increased in vivo half-life comprising a salvage receptor binding epitope residues are described in U.S. Patent No. 5,869,046. Other techniques for the production of antibody fragments will be apparent to the skilled practitioner. In other embodiments, the antibody of choice is a single chain Fv fragment (scFv). See WO 93/16185; U.S. Patent No. 5,571,894; and U.S. Patent No. 5,587,458. Fv and sFv are the only species with intact combining sites that are devoid of constant regions; thus, they are suitable for reduced nonspecific binding during in vivo use. sFv fusion proteins may be constructed to yield fusion of an effector protein at either the amino or the carboxy terminus of an sFv. See Antibody Engineering, ed. Borrebaeck, supra. The antibody fragment may also be a "linear antibody", e.g., as described in U.S. Patent 5,641,870 for example. Such linear antibody fragments may be monospecific or bispecific.

5. Bispecific Antibodies

Bispecific antibodies are antibodies that have binding specificities for at least two different epitopes. Exemplary bispecific antibodies may bind to two different epitopes of a TAT protein as described herein. Other such antibodies may combine a TAT binding site with a binding site for another protein. Alternatively, an anti-TAT arm may be combined with an arm which binds to a triggering molecule on a leukocyte such as a T-cell receptor molecule (e.g. CD3), or Fc receptors for IgG (FcγR), such as FcγRI (CD64), FcγRII (CD32) and FcγRIII (CD16), so as to focus and localize cellular defense mechanisms to the TAT-expressing cell. Bispecific antibodies may also be used to localize cytotoxic agents to cells which express TAT. These antibodies possess a TAT-binding arm and an arm which binds the cytotoxic agent (e.g., saporin, anti-interferon-α, vinca alkaloid, ricin A chain, methotrexate or radioactive isotope hapten). Bispecific antibodies can be prepared as full length antibodies or antibody fragments (e.g., $F(ab')_2$ bispecific antibodies). WO 96/16673 describes a bispecific anti-ErbB2/anti-FcγRIII antibody and U.S. Patent No. 5,837,234 discloses a bispecific anti-ErbB2/anti-FcγRI antibody. A bispecific anti-ErbB2/Fcα antibody is shown in WO98/02463. U.S. Patent No. 5,821,337 teaches a bispecific anti-ErbB2/anti-CD3 antibody. Methods for making bispecific antibodies are known in the art. Traditional production of full length bispecific antibodies is based on the co-expression of two immunoglobulin heavy chain-light chain pairs, where the two chains have different specificities (Millstein et al., Nature 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J. 10:3655-3659 (1991). According to a different approach, antibody variable domains with the desired binding specificities (antibody-antigen combining sites) are fused to immunoglobulin constant domain sequences. Preferably, the fusion is with an Ig heavy chain constant domain, comprising at least part of the hinge, $C_H2$, and $C_H3$ regions. It is preferred to have the first heavy-chain constant region ($C_H1$) containing the site necessary for light chain bonding, present in at least one of the fusions. DNAs encoding the immunoglobulin heavy chain fusions and, if desired, the immunoglobulin light chain, are inserted into separate expression vectors, and are co-transfected into a suitable host cell. This provides for greater flexibility in adjusting the mutual proportions of the three polypeptide fragments in embodiments when unequal ratios of the three polypeptide chains used in the construction provide the optimum yield of the desired bispecific antibody. It is, however, possible to insert the coding sequences for two or all three polypeptide chains into a single expression vector when the expression of at least two polypeptide chains in equal ratios results in high yields or when the ratios have no significant affect on the yield of the desired chain combination. In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology 121:210 (1986).
According to another approach described in U.S. Patent No. 5,731,168, the interface between a pair of antibody molecules can be engineered to maximize the percentage of heterodimers which are recovered from recombinant cell culture. The preferred interface comprises at least a part of the $C_H3$ domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers.
Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.
Techniques for generating bispecific antibodies from antibody fragments have also been described in the literature. For example, bispecific antibodies can be prepared using chemical linkage. Brennan et al., Science 229:81 (1985) describe a procedure wherein intact antibodies are proteolytically cleaved to generate $F(ab')_2$ fragments. These fragments are reduced in the presence of the dithiol complexing agent, sodium arsenite, to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.
Recent progress has facilitated the direct recovery of Fab'-SH fragments from E. *coli,* which can be chemically coupled to form bispecific antibodies. Shalaby et al., J. Exp. Med. 175: 217-225 (1992) describe the production of a fully humanized bispecific antibody $F(ab')_2$ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets. Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a $V_H$ connected to a $V_L$ by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).
Antibodies with more than two valencies are contemplated. For example, trispecific antibodies can be prepared. Tutt et al., J. Immunol. 147:60 (1991).

6. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. PatentNo. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

7. Multivalent Antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody will comprise an Fc region and three or more antigen binding sites amino-terminal to the Fc region. The preferred multivalent antibody herein comprises (or consists of) three to about eight, but preferably four, antigen binding sites. The multivalent antibody comprises at least one polypeptide chain (and preferably two polypeptide chains), wherein the polypeptide chain(s) comprise two or more variable domains. For instance, the polypeptide chain(s) may comprise VD1-$(X1)_n$-VD2-$(X2)_n$-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

8. Effector Function Engineering

It may be desirable to modify the antibody of the invention with respect to effector function, e.g., so as to enhance antigen-dependent cell-mediated cyotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med.176:1191-1195 (1992) and Shopes, B. J. Immunol. 148:2918-2922 (1992). Homodimeric antibodies with enhanced anti-tumor activity may also be prepared using heterobifunctional cross-linkers as described in Wolff et al., Cancer Research 53:2560-2565 (1993). Alternatively, an antibody can be engineered which has dual Fc regions and may thereby have enhanced complement lysis and ADCC capabilities. See Stevenson et al., Anti-Cancer Drug Design 3:219-230 (1989). To increase the serum half life of the antibody, one may incorporate a salvage receptor binding epitope into the antibody (especially an antibody fragment) as described in U.S. Patent 5,739,277, for example. As used herein, the term "salvage receptor binding epitope" refers to an epitope of the Fc region of an IgG molecule (e.g., $IgG_1$, $IgG_2$, $IgG_3$, or $IgG_4$) that is responsible for increasing the *in vivo* serum half-life of the IgG molecule.

9. Immunoconjugates

The invention also pertains to immunoconjugates comprising an antibody conjugated to a cytotoxic agent such as a chemotherapeutic agent, a growth inhibitory agent, a toxin (e.g., an enzymatically active toxin of bacterial, fungal, plant, or animal origin, or fragments thereof), or a radioactive isotope (*i.e.*, a radioconjugate).
Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include $^{212}Bi$, $^{131}I$, $^{131}In$, $^{90}Y$, and $^{186}Re$. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.
Conjugates of an antibody and one or more small molecule toxins, such as a calicheamicin, maytansinoids, a trichothene, and CC1065, and the derivatives of these toxins that have toxin activity, are also contemplated herein.

Maytansine and maytansinoids

In one preferred embodiment, an anti-TAT antibody (full length or fragments) of the invention is conjugated to one or more maytansinoid molecules.
Maytansinoids are mitototic inhibitors which act by inhibiting tubulin polymerization. Maytansine was first isolated from the east African shrub *Maytenus serrata* (U.S. Patent No. 3,896,111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. PatentNos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533, the disclosures of which are hereby expressly incorporated by reference.

Maytansinoid-antibody conjugates

In an attempt to improve their therapeutic index, maytansine and maytansinoids have been conjugated to antibodies specifically binding to tumor cell antigens. Immunoconjugates containing maytansinoids and their therapeutic use are disclosed, for example, in U.S. Patent Nos. 5,208,020, 5,416,064 and European Patent EP 0 425 235 B1, the disclosures of which are hereby expressly incorporated by reference. Liu et al., Proc. Natl. Acad. Sci. USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an *in vivo* tumor growth assay. Chari et al., Cancer Research 52:127-131 (1992) describe immunoconjugates in which a maytansinoid was conjugated via a disulfide linker to the murine antibody A7 binding to an antigen on human colon cancer cell lines, or to another murine monoclonal antibody TA.1 that binds the HER-2/*neu* oncogene. The cytotoxicity of the TA.1-maytansonoid conjugate was tested *in vitro* on the human breast cancer cell line SK-BR-3, which expresses 3 x $10^5$ HER-2 surface antigens per cell. The drug conjugate achieved a degree of cytotoxicity similar to the free maytansonid drug, which could be increased by increasing the number of maytansinoid molecules per antibody molecule. The A7-maytansinoid conjugate showed low systemic cytotoxicity in mice.

Anti-TAT polypeptide antibody-maytansinoid conjugates (immunoconjugates)

Anti-TAT antibody-maytansinoid conjugates are prepared by chemically linking an anti-TAT antibody to a maytansinoid molecule without significantly diminishing the biological activity of either the antibody or the maytansinoid molecule. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Patent No. 5,208,020 and in the other patents and nonpatent publications referred to hereinabove. Preferred maytansinoids are maytansinol and maytansinol analogues modified in the aromatic ring or at other positions of the maytansinol molecule, such as various maytansinol esters.
There are many linking groups known in the art for making antibody-maytansinoid conjugates, including, for example, those disclosed in U.S. Patent No. 5,208,020 or EP Patent 0 425 235 B1, and Chari et al., Cancer Research 52:127-131 (1992). The linking groups include disufide groups, thioether groups, acid labile groups, photolabile groups, peptidase labile groups, or esterase labile groups, as disclosed in the above-identified patents, disulfide and thioether groups being preferred.
Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particularly preferred coupling agents include N-succinimidyl-3-(2-pyridyldithio) propionate (SPDP) (Carlsson et al., Biochem. J. 173:723-737 [1978]) and N-succinimidyl-4-(2-pyridylthio)pentanoate (SPP) to provide for a disulfide linkage.
The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hyrdoxymethyl, the C-15 position modified with a hydroxyl group, and the C-20 position having a hydroxyl group. In a preferred embodiment, the linkage is formed at the C-3 position of maytansinol or a maytansinol analogue.

Calicheamicin

Another immunoconjugate of interest comprises an anti-TAT antibody conjugated to one or more calicheamicin molecules. The calicheamicin family of antibiotics are capable of producing double-stranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, $\gamma_1^I$, $\alpha_2^I$, $\alpha_3^I$, N-acetyl-$\gamma_1^I$, PSAG and $\theta^I_1$ (Hinman et al., Cancer Research 53:3336-3342 (1993), Lode et al., Cancer Research 58:2925-2928 (1998) and the aforementioned U.S. patents to American Cyanamid). Another anti-tumor drug that the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

Other cytotoxic agents

Other antitumor agents that can be conjugated to the anti-TAT antibodies of the invention include BCNU, streptozoicin, vincristine and 5-fluorouracil, the family of agents known collectively LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain *(fromPseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates an immunoconjugate formed between an antibody and a compound with nucleolytic activity (e.g., a ribonuclease or a DNA endonuclease such as a deoxyribonuclease; DNase).

For selective destruction of the tumor, the antibody may comprise a highly radioactive atom. A variety of radioactive isotopes are available for the production of radioconjugated anti-TAT antibodies. Examples include $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu. When the conjugate is used for diagnosis, it may comprise a radioactive atom for scintigraphic studies, for example $tc^{99m}$ or $I^{123}$, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as $tc^{99m}$ or $I^{123}$, $Re^{186}$, $Re^{188}$ and $In^{111}$ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintigraphy" (Chatal,CRC Press 1989) describes other methods in detail.

Conjugates of the antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radio nucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Patent No. 5,208,020) may be used.

Alternatively, a fusion protein comprising the anti-TAT antibody and cytotoxic agent may be made, e.g., by recombinant techniques or peptide synthesis. The length of DNA may comprise respective regions encoding the two portions of the conjugate either adjacent one another or separated by a region encoding a linker peptide which does not destroy the desired properties of the conjugate.

In yet another embodiment, the antibody may be conjugated to a "receptor" (such streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a radionucleotide).

10. Immunoliposomes

The anti-TAT antibodies disclosed herein may also be formulated as immunoliposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA 82:3688 (1985); Hwang et al., Proc. Natl Acad. Sci. USA 77:4030 (1980); U.S. Pat. Nos. 4,485,045 and 4,544,545; and WO97/38731 published October 23, 1997. Liposomes with enhanced circulation time are disclosed in U.S. Patent No. 5,013,556.
Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem. 257:286-288 (1982) via a disulfide interchange reaction. A chemotherapeutic agent is optionally contained within the liposome. See Gabizon et al., J. National Cancer Inst. 81(19):1484 (1989).

B. TAT Binding Oligopeptides

TAT binding oligopeptides of the present invention are oligopeptides that bind, preferably specifically, to a TAT polypeptide as described herein. TAT binding oligopeptides may be chemically synthesized using known oligopeptide synthesis methodology or may be prepared and purified using recombinant technology. TAT binding oligopeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, S5, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such oligopeptides that are capable of binding, preferably specifically, to a TAT polypeptide as described herein. TAT binding oligopeptides may be identified without undue experimentation using well known techniques. In this regard, it is noted that techniques for screening oligopeptide libraries for oligopeptides that are capable of specifically binding to a polypeptide target are well known in the art (see, e.g., U.S. PatentNos. 5,556,762, 5,750,373, 4,708,871, 4,833,092, 5,223,409, 5,403,484, 5,571,689, 5,663,143; PCTPublicationNos. WO 84/03506 and W084/03564; Geysen et al., Proc. Natl. Acad. Sci. U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. (1990) Proc. Natl. Acad. Sci. USA, 87:6378; Lowman, H.B. et al. (1991) Biochemistry, 30:10832; Clackson, T. et al. (1991) Nature, 352: 624; Marks, J. D. etal. (1991), J. Mol. Biol., 222:581; Kang, A.S. etal. (1991) Proc. Natl. Acad. Sci. USA, 88:8363, and Smith, G. P. (1991) Current Opin. Biotechnol., 2:668).
In this regard, bacteriophage (phage) display is one well known technique which allows one to screen large oligopeptide libraries to identify member(s) of those libraries which are capable of specifically binding to a polypeptide target. Phage display is a technique by which variant polypeptides are displayed as fusion proteins to the coat protein on the surface of bacteriophage particles (Scott, J.K. and Smith, G. P. (1990) Science 249: 386). The utility of phage display lies in the fact that large libraries of selectively randomized protein variants (or randomly cloned cDNAs) can be rapidly and efficiently sorted for those sequences that bind to a target molecule with high affinity. Display of peptide (Cwirla, S. E. et al. (1990) Proc. Natl. Acad. Sci. USA, 87:6378) or protein (Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363) libraries on phage have been used for screening millions of polypeptides or oligopeptides for ones with specific binding properties (Smith, G. P. (1991) Current Opin. Biotechnol., 2:668). Sorting phage libraries of random mutants requires a strategy for constructing and propagating a large number of variants, a procedure for affinity purification using the target receptor, and a means of evaluating the results of binding enrichments. U.S. Patent Nos. 5,223,409, 5,403,484, 5,571,689, and 5,663,143.
Although most phage display methods have used filamentous phage, lambdoid phage display systems (WO95/34683; U.S. 5,627,024), T4 phage display systems(Ren, Z-J. et al. (1998) Gene 215:439; Zhu, Z. (1997) CAN 33:534; Jiang, J. metal. (1997) can 128:44380; Ren, Z-J. et al. (1997) CAN 127:215644; Ren, Z-J. (1996) Protein Sci. 5:1833; Efimov, V. P. et al. (1995) Virus Genes 10:173) and T7 phage display systems (Smith, G. P. and Scott, J.K. (1993) Methods in Enzymology, 217,228-257; U.S. 5,766,905) are also known.
Many other improvements and variations of the basic phage display concept have now been developed. These improvements enhance the ability of display systems to screen peptide libraries for binding to selected target molecules and to display functional proteins with the potential of screening these proteins for desired properties. Combinatorial reaction devices for phage display reactions have been developed (WO 98/14277) and phage display libraries have been used to analyze and control bimolecular interactions (WO 98/20169; WO 98/20159) and properties of constrained helical peptides (WO 98/20036). WO 97/35196 describes a method of isolating an affinity ligand in which a phage display library is contacted with one solution in which the ligand will bind to a target molecule and a second solution in which the affinity ligand will not bind to the target molecule, to selectively isolate binding ligands. WO 97/46251 describes a method of biopanning a random phage display library with an affinity purified antibody and then isolating binding phage, followed by a micropanning process using microplate wells to isolate high affinity binding phage. The use of *Staphlylococcus aureus* protein A as an affinity tag has also been reported (Li et al. (1998) Mol Biotech., 9:187). WO 97/47314 describes the use of substrate subtraction libraries to distinguish enzyme specificities using a combinatorial library which may be a phage display library. A method for selecting enzymes suitable for use in detergents using phage display is described in WO 97/09446. Additional methods of selecting specific binding proteins are described in U.S. Patent Nos. 5,498,538, 5,432,018, and WO 98/15833.

Methods of generating peptide libraries and screening these libraries are also disclosed in U.S. Patent Nos. 5,723,286, 5,432,018, 5,580,717, 5,427,908, 5,498,530, 5,770,434, 5,734,018, 5,698,426, 5,763,192, and 5,723,323.

C. TAT Binding Organic Molecules

TAT binding organic molecules are organic molecules other than oligopeptides or antibodies as defined herein that bind, preferably specifically, to a TAT polypeptide as described herein. TAT binding organic molecules may be identified and chemically synthesized using known methodology (see, e.g., PCT Publication Nos. WO00/00823 and WO00/39585). TAT binding organic molecules are usually less than about 2000 daltons in size, alternatively less than about 1500, 750, 500, 250 or 200 daltons in size, wherein such organic molecules that are capable of binding, preferably specifically, to a TAT polypeptide as described herein may be identified without undue experimentation using well known techniques. In this regard, it is noted that techniques for screening organic molecule libraries for molecules that are capable of binding to a polypeptide target are well known in the art (see, e.g., PCT Publication Nos. WO00/00823 and WO00/39585). TAT binding organic molecules may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, acid chlorides, or the like.

D. Screening for Anti-TAT Antibodies, TAT Binding Oligopeptides and TAT Binding Organic Molecules With the Desired Properties

Techniques for generating antibodies, oligopeptides and organic molecules that bind to TAT polypeptides have been described above. One may further select antibodies, oligopeptides or other organic molecules with certain biological characteristics, as desired.

The growth inhibitory effects of an anti-TAT antibody, oligopeptide or other organic molecule of the invention may be assessed by methods known in the art, e.g., using cells which express a TAT polypeptide either endogenously or following transfection with the TAT gene. For example, appropriate tumor cell lines and TAT-transfected cells may treated with an anti-TAT monoclonal antibody, oligopeptide or other organic molecule of the invention at various concentrations for a few days (e.g., 2-7) days and stained with crystal violet or MTT or analyzed by some other colorimetric assay. Another method of measuring proliferation would be by comparing $^3$H-thymidine uptake by the cells treated in the presence or absence an anti-TAT antibody, TAT binding oligopeptide or TAT binding organic molecule of the invention. After treatment, the cells are harvested and the amount of radioactivity incorporated into the DNA quantitated in a scintillation counter. Appropriate positive controls include treatment of a selected cell line with a growth inhibitory antibody known to inhibit growth of that cell line. Growth inhibition of tumor cells *in vivo* can be determined in various ways known in the art. Preferably, the tumor cell is one that overexpresses a TAT polypeptide. Preferably, the anti-TAT antibody, TAT binding oligopeptide or TAT binding organic molecule will inhibit cell proliferation of a TAT-expressing tumor cell *in vitro* or *in vivo* by about 25-100% compared to the untreated tumor cell, more preferably, by about 30-100%, and even more preferably by about 50-100% or 70-100%, in one embodiment, at an antibody concentration of about 0.5 to 30 μg/ml. Growth inhibition can be measured at an antibody concentration of about 0.5 to 30 μg/ml or about 0.5 nM to 200 nM in cell culture, where the growth inhibition is determined 1-10 days after exposure of the tumor cells to the antibody. The antibody is growth inhibitory *in vivo* if administration of the anti-TAT antibody at about 1 μg/kg to about 100 mg/kg body weight results in reduction in tumor size or reduction of tumor cell proliferation within about 5 days to 3 months from the first administration of the antibody, preferably within about 5 to 30 days.

To select for an anti-TAT antibody, TAT binding oligopeptide or TAT binding organic molecule which induces cell death, loss of membrane integrity as indicated by, e.g., propidium iodide (PI), trypan blue or 7AAD uptake may be assessed relative to control. A PI uptake assay can be performed in the absence of complement and immune effector cells. TAT polypeptide-expressing tumor cells are incubated with medium alone or medium containing the appropriate anti-TAT antibody (e.g, at about 10μg/ml), TAT binding oligopeptide or TAT binding organic molecule. The cells are incubated for a 3 day time period. Following each treatment, cells are washed and aliquoted into 35 mm strainer-capped 12 x 75 tubes (1ml per tube, 3 tubes per treatment group) for removal of cell clumps. Tubes then receive PI (10μg/ml). Samples may be analyzed using a FACSCAN® flow cytometer and FACSCONVERT® CellQuest software (Becton Dickinson). Those anti-TAT antibodies, TAT binding oligopeptides or TAT binding organic molecules that induce statistically significant levels of cell death as determined by PI uptake may be selected as cell death-inducing anti-TAT antibodies, TAT binding oligopeptides or TAT binding organic molecules.

To screen for antibodies, oligopeptides or other organic molecules which bind to an epitope on a TAT polypeptide bound by an antibody of interest, a routine cross-blocking assay such as that described in Antibodies, A Laboratory Manual, Cold Spring Harbor Laboratory, Ed Harlow and David Lane (1988), can be performed. This assay can be used to determine if a test antibody, oligopeptide or other organic molecule binds the same site or epitope as a known anti-TAT antibody. Alternatively, or additionally, epitope mapping can be performed by methods known in the art. For example, the antibody sequence can be mutagenized such as by alanine scanning, to identify contact residues. The mutant antibody is initailly tested for binding with polyclonal antibody to ensure proper folding. In a different method, peptides corresponding to different regions of a TAT polypeptide can be used in competition assays with the test antibodies or with a test antibody and an antibody with a characterized or known epitope.

E. Antibody Dependent Enzyme Mediated Prodrug Therapy (ADEPT)

The antibodies of the present invention may also be used in ADEPT by conjugating the antibody to a prodrug-activating enzyme which converts a prodrug (e.g., a peptidyl chemotherapeutic agent, see WO81/01145) to an active anti-cancer drug. See, for example, WO 88/07378 and U.S. Patent No. 4,975,278.

The enzyme component of the immunoconjugate useful for ADEPT includes any enzyme capable of acting on a prodrug in such a way so as to covert it into its more active, cytotoxic form.

Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galactosidase and neuraminidase useful for converting glycosylated prodrugs into free drugs; β-lactamase useful for converting drugs derivatized with β- lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs (see, e.g., Massey, Nature 328:457-458 (1987)). Antibody-abzyme conjugates can be prepared as described herein for delivery of the abzyme to a tumor cell population.

The enzymes of this invention can be covalently bound to the anti-TAT antibodies by techniques well known in the art such as the use of the heterobifunctional crosslinking reagents discussed above. Alternatively, fusion proteins comprising at least the antigen binding region of an antibody of the invention linked to at least a functionally active portion of an enzyme of the invention can be constructed using recombinant DNA techniques well known in the art (see, e.g., Neuberger et al., Nature 312:604-608 (1984).

F. Full-Length TAT Polypeptides

The present invention also provides newly identified and isolated nucleotide sequences encoding polypeptides referred to in the present application as TAT polypeptides. In particular, cDNAs (partial and full-length) encoding various TAT polypeptides have been identified and isolated, as disclosed in further detail in the Examples below.

As disclosed in the Examples below, various cDNA clones have been deposited with the ATCC. The actual nucleotide sequences of those clones can readily be determined by the skilled artisan by sequencing of the deposited clone using routine methods in the art. The predicted amino acid sequence can be determined from the nucleotide sequence using routine skill. For the TAT polypeptides and encoding nucleic acids described herein, in some cases, Applicants have identified what is believed to be the reading frame best identifiable with the sequence information available at the time.

G. Anti-TAT Antibody and TAT Polypeptide Variants

In addition to the anti-TAT antibodies and full-length native sequence TAT polypeptides described herein, it is contemplated that anti-TAT antibody and TAT polypeptide variants can be prepared. Anti-TAT antibody and TAT polypeptide variants can be prepared by introducing appropriate nucleotide changes into the encoding DNA, and/or by synthesis of the desired antibody or polypeptide. Those skilled in the art will appreciate that amino acid changes may alter post-translational processes of the anti-TAT antibody or TAT polypeptide, such as changing the number or position of glycosylation sites or altering the membrane anchoring characteristics.

Variations in the anti-TAT antibodies and TAT polypeptides described herein, can be made, for example, using any of the techniques and guidelines for conservative and non-conservative mutations set forth, for instance, in U.S. Patent No. 5,364,934. Variations may be a substitution, deletion or insertion of one or more codons encoding the antibody or polypeptide that results in a change in the amino acid sequence as compared with the native sequence antibody or polypeptide. Optionally the variation is by substitution of at least one amino acid with any other amino acid in one or more of the domains of the anti-TAT antibody or TAT polypeptide. Guidance in determining which amino acid residue may be inserted, substituted or deleted without adversely affecting the desired activity may be found by comparing the sequence of the anti-TAT antibody or TAT polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

Anti-TAT antibody and TAT polypeptide fragments are provided herein. Such fragments may be truncated at the N-terminus or C-terminus, or may lack internal residues, for example, when compared with a full length native antibody or protein. Certain fragments lack amino acid residues that are not essential for a desired biological activity of the anti-TAT antibody or TAT polypeptide.

Anti-TAT antibody and TAT polypeptide fragments may be prepared by any of a number of conventional techniques. Desired peptide fragments may be chemically synthesized. An alternative approach involves generating antibody or polypeptide fragments by enzymatic digestion, e.g., by treating the protein with an enzyme known to cleave proteins at sites defined by particular amino acid residues, or by digesting the DNA with suitable restriction enzymes and isolating the desired fragment Yet another suitable technique involves isolating and amplifying a DNA fragment encoding a desired antibody or polypeptide fragment, by polymerase chain reaction (PCR). Oligonucleotides that define the desired termini of the DNA fragment are employed at the 5' and 3' primers in the PCR. Preferably, anti-TAT antibody and TAT polypeptide fragments share at least one biological and/or immunological activity with the native anti-TAT antibody or TAT polypeptide disclosed herein.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

Table 6

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |

(continued)

| Original Residue | Exemplary Substitutions | Preferred Substitutions |
|---|---|---|
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the anti-TAT antibody or TAT polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res.,13:4331 (1986); Zoller et al., Nucl. Acids Res., 10:6487 (1987)], cassette mutagenesis [Wells etal., Gene, 34:315 (1985)], restriction selection mutagenesis [Wells et al., Philos. Trans. R Soc. London SerA, 317:415 (1986)] or other known techniques can be performed on the cloned DNA to produce the anti-TAT antibody or TAT polypeptide variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244:1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

Any cysteine residue not involved in maintaining the proper conformation of the anti-TAT antibody or TAT polypeptide also may be substituted, generally with serine, to improve the oxidative stability of the molecule and prevent aberrant crosslinking. Conversely, cysteine bond(s) may be added to the anti-TAT antibody or TAT polypeptide to improve its stability (particularly where the antibody is an antibody fragment such as an Fv fragment).

A particularly preferred type of substitutional variant involves substituting one or more hypervariable region residues of a parent antibody (e.g., a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (e.g., 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (e.g., binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and human TAT polypeptide. Such contact residues and neighboring residues are candidates for substitution according to the techniques elaborated herein. Once such variants are generated, the panel of variants is subjected to screening as described herein and antibodies with superior properties in one or more relevant assays may be selected for further development.
Nucleic acid molecules encoding amino acid sequence variants of the anti-TAT antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the anti-TAT antibody.

H. Modifications of Anti-TAT Antibodies and TAT Polypeptides

Covalent modifications of anti-TAT antibodies and TAT polypeptides are included within the scope of this invention. One type of covalent modification includes reacting targeted amino acid residues of an anti-TAT antibody or TAT polypeptide with an organic derivatizing agent that is capable of reacting with selected side chains or the N- or C-terminal residues of the anti-TAT antibody or TAT polypeptide. Derivatization with bifunctional agents is useful, for instance, for crosslinking anti-TAT antibody or TAT polypeptide to a water-insoluble support matrix or surface for use in the method for purifying anti-TAT antibodies, and vice-versa. Commonly used crosslinking agents include, e.g., 1,1-bis (diazoacetyl)-2-phenylethane, glutaraldehyde, N-hydroxysuccinimide esters, for example, esters with4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.
Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the $\alpha$-amino groups of lysine, arginine, and histidine side chains [T.E. Creighton, Proteins: Structure and Molecular Properties, W.H. Freeman & Co., SanFrancisco, pp. 79-86 (1983)], acetylation of the N-terminal amine, and amidation of any C-terminal carboxyl group.
Another type of covalent modification of the anti-TAT antibody or TAT polypeptide included within the scope of this invention comprises altering the native glycosylation pattern of the antibody or polypeptide. "Altering the native glycosylation pattern" is intended for purposes herein to mean deleting one or more carbohydrate moieties found in native sequence anti-TAT antibody or TAT polypeptide (either by removing the underlying glycosylation site or by deleting the glycosylation by chemical and/or enzymatic means), and/or adding one or more glycosylation sites that are not present in the native sequence anti-TAT antibody or TAT polypeptide. In addition, the phrase includes qualitative changes in the glycosylation of the native proteins, involving a change in the nature and proportions of the various carbohydrate moieties present.
Glycosylation of antibodies and other polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.
Addition of glycosylation sites to the anti-TAT antibody or TAT polypeptide is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original anti-TAT antibody or TAT polypeptide (for O-linked glycosylation sites). The anti-TAT antibody or TAT polypeptide amino acid sequence may optionally be altered through changes at the DNA level, particularly by mutating the DNA encoding the anti-TAT antibody or TAT polypeptide at preselected bases such that codons are generated that will translate into the desired amino acids.
Another means of increasing the number of carbohydrate moieties on the anti-TAT antibody or TAT polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).
Removal of carbohydrate moieties present on the anti-TAT antibody or TAT polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).
Another type of covalent modification of anti-TAT antibody or TAT polypeptide comprises linking the antibody or polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337. The antibody or polypeptide also may be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, 16th edition, Oslo, A., Ed., (1980).

The anti-TAT antibody or TAT polypeptide of the present invention may also be modified in a way to form chimeric molecules comprising an anti-TAT antibody or TAT polypeptide fused to another, heterologous polypeptide or amino acid sequence.

In one embodiment, such a chimeric molecule comprises a fusion of the anti-TAT antibody or TAT polypeptide with a tag polypeptide which provides an epitope to which an anti-tag antibody can selectively bind. The epitope tag is generally placed at the amino- or carboxyl- terminus of the anti-TAT antibody or TAT polypeptide. The presence of such epitope-tagged forms of the anti-TAT antibody or TAT polypeptide can be detected using an antibody against the tag polypeptide. Also, provision of the epitope tag enables the anti-TAT antibody or TAT polypeptide to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the fluHA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnoloey, 6:1204-1210 (1988)]; the KT3 epitope peptide [Martin et a]., Science 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the anti-TAT antibody or TAT polypeptide with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of an anti-TAT antibody or TAT polypeptide in place of at least one variable region within an Ig molecule. In a particularly preferred embodiment, the immunoglobulin fusion includes the hinge, $CH_2$ and $CH_3$, or the hinge, $CH_1$, $CH_2$ and $CH_3$ regions of an IgG1 molecule. For the production of immunoglobulin fusions see also US Patent No. 5,428,130 issued June 27,1995.

I. Preparation of Anti-TAT Antibodies and TAT Polypeptides

The description below relates primarily to production of anti-TAT antibodies and TAT polypeptides by culturing cells transformed or transfected with a vector containing anti-TAT antibody- and TAT polypeptide-encoding nucleic acid. It is, of course, contemplated that alternative methods, which are well known in the art, may be employed to prepare anti-TAT antibodies and TAT polypeptides. For instance, the appropriate amino acid sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the anti-TAT antibody or TAT polypeptide may be chemically synthesized separately and combined using chemical or enzymatic methods to produce the desired anti-TAT antibody or TAT polypeptide.

1. Isolation of DNA Encoding Anti-TAT Antibody or TAT Polypeptide

DNA encoding anti-TAT antibody or TAT polypeptide may be obtained from a cDNA library prepared from tissue believed to possess the anti-TAT antibody or TAT polypeptide mRNA and to express it at a detectable level. Accordingly, human anti-TAT antibody or TAT polypeptide DNA can be conveniently obtained from a cDNA library prepared from human tissue. The anti-TAT antibody- or TAT polypeptide-encoding gene may also be obtained from a genomic library or by known synthetic procedures (e.g., automated nucleic acid synthesis).

Libraries can be screened with probes (such as oligonucleotides of at least about 20-80 bases) designed to identify the gene of interest or the protein encoded by it Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding anti-TAT antibody or TAT polypeptide is to use PCR methodology [Sambrook et al., supra; Dieffenbach et al., PCR Primer: A Laboratory Manual (Cold Spring Harbor Laboratory Press, 1995)].

Techniques for screening a cDNA library are well known in the art. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like $^{32}P$-labeled ATP, biotinylation or enzyme labeling. Hybridization conditions, including moderate stringency and high stringency, are provided in Sambrook et al., supra.
Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.
Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

2. Selection and Transformation of Host Cells

Host cells are transfected or transformed with expression or cloning vectors described herein for anti-TAT antibody or TAT polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.
Methods of eukaryotic cell transfection and prokaryotic cell transformation are known to the ordinarily skilled artisan, for example, $CaCl_2$, $CaPO_4$, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. Formammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).
Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as E. *coli.* Various E. *coli* strains are publicly available, such as *E. coli* K12 strain MM294 (ATCC 31,446); E. *coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including E. *coli* W3110 strain 1 A2, which has the complete genotype *tonA ; E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT* $kan^r$; *E. coli* W3110 strain 37D6, which has the complete genotype *tonA ptr3 phoA E15 (argF-lac)169 degP ompT rbs7 ilvG* $kan^r$; *E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.
Full length antibody, antibody fragments, and antibody fusion proteins can be produced in bacteria, in particular when glycosylation and Fc effector function are not needed, such as when the therapeutic antibody is conjugated to a cytotoxic agent (e.g., a toxin) and the immunoconjugate by itself shows effectiveness in tumor cell destruction. Full length antibodies have greater half life in circulation. Production in E. coli is faster and more cost efficient. For expression of antibody fragments and polypeptides in bacteria, see, e.g., U.S. 5,648,237 (Carter et. al.), U.S. 5,789,199 (Joly et al.), and U.S. 5,840,523 (Simmons et al.) which describes translation initiation regio (TIR) and signal sequences for optimizing expression and secretion, these patents incorporated herein by reference. After expression, the antibody is isolated from the E. coli cell paste in a soluble fraction and can be purified through, e.g., a protein A or G column depending on the isotype. Final purification can be carried out similar to the process for purifying antibody expressed e.g" in CHO cells.
In addition to prokaryotes, eukaryotic microbes such as filamentous fungi or yeast are suitable cloning or expression hosts for anti-TAT antibody- or TAT polypeptide-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/TechnologY, 9:968-975 (1991)) such as, e.g., *K. lactis* (MW98-8C, CBS683, CBS4574; Louvencourt et al., J. Bacteriol., 154 (2):737-742 [1983]), *K fragilis* (ATCC *12,424), K bulgaricus* (ATCC 16,045), *K. wickeramii* (ATCC 24,178), *K. waltii* (ATCC 56,500), *K. drosophilarum* (ATCC 36,906; Van den Berg et al., Bio/Technoiogy, 8:135 (1990)), *K. thermotolerans,* and *K. marxianus; yarrowia* (EP 402,226); *Pichia pastoris* (EP 183,070; Sreekrishna et al., J. Basic Microbiol., 28: 265-278 [1988]); *Candida; Trichodenna reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such *as A. nidulans* (Ballance et al., Biochem. Bionhys. Res. Commun., 112:284-289 [1983]; Tilbum et al., Gene, 26:205-221 [1983]; Yelton et al., Proc. Natl. Acad. Sci. USA, 81: 1470-1474 [1984]) andA. *niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).
Suitable host cells for the expression of glycosylated anti-TAT antibody or TAT polypeptide are derived from multicellular organisms. Examples of invertebrate cells include insect cells such as Drosophila S2 and Spodoptera Sf9, as well as plant cells, such as cell cultures of cotton, corn, potato, soybean, petunia, tomato, and tobacco. Numerous baculoviral strains and variants and corresponding permissive insect host cells from hosts such as *Spodoptera frugiperda* (caterpillar), *Aedes aegypti* (mosquito), *Andes albopictus* (mosquito), *Drosophila melanogaster* (fruitfly), and *Bombyx mori* have been identified. A variety of viral strains for transfection are publicly available, e.g., the L-1 variant of *Autographa californica* NPV and the Bm-5 strain of *Bombyx mori* NPV, and such viruses may be used as the virus herein according to the present invention, particularly for transfection of *Spodoptera frugiperda* cells.
However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamsterovary cells/-DHFR(CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383: 44-68 (1982)); MRC 5 cells; FS4 cells; and a human hepatoma line (Hep G2).
Host cells are transformed with the above-described expression or cloning vectors for anti-TAT antibody or TAT polypeptide production and cultured in conventional nutrient media modified as appropriate for inducing promoters, selecting Transformants, or amplifying the genes encoding the desired sequences.

3. Selection and Use of a Replicable Vector

The nucleic acid (e.g., cDNA or genomic DNA) encoding anti-TAT antibody or TAT polypeptide may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.
The TAT may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the anti-TAT antibody- or TAT polypeptide-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2μ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the anti-TAT antibody- or TAT polypeptide-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemperet al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85: 12 (1977)].

Expression and cloning vectors usually contain a promoter operably linked to the anti-TAT antibody-or TAT polypeptide-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding anti-TAT antibody or TAT polypeptide.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

Anti-TAT antibody or TAT polypeptide transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the anti-TAT antibody or TAT polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the anti-TAT antibody or TAT polypeptide coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding anti-TAT antibody or TAT polypeptide.

Still other methods, vectors, and host cells suitable for adaptation to the synthesis of anti-TAT antibody or TAT polypeptide in recombinant vertebrate cell culture are described in Gething et al., Nature, 293:620-625 (1981); Mantei et al., Nature, 281:40-46 (1979); EP 117,060; and EP 117,058.

4. Culturing the Host Cells

The host cells used to produce the anti-TAT antibody or TAT polypeptide of this invention may be cultured in a variety of media. Commercially available media such as Ham's F10 (Sigma), Minimal Essential Medium ((MEM), (Sigma), RPMI-1640 (Sigma), and Dulbecco's ModifiedEagle's Medium ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

5. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77: 5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes orDNA-protein duplexes. The antibodies in turn may be labeled and the assay may be carried out where the duplex is bound to a surface, so that upon the formation of duplex on the surface, the presence of antibody bound to the duplex can be detected.
Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence TAT polypeptide or against a synthetic peptide based on the DNA sequences provided herein or against exogenous sequence fused to TAT DNA and encoding a specific antibody epitope.

6. Purification of Anti-TAT Antibody and TAT Polypeptide

Forms of anti-TAT antibody and TAT polypeptide may be recovered from culture medium or from host cell lysates. If membrane-bound, it can be released from the membrane using a suitable detergent solution (e.g. Triton-X 100) or by enzymatic cleavage. Cells employed in expression of anti-TAT antibody and TAT polypeptide can be disrupted by various physical or chemical means, such as freeze-thaw cycling, sonication, mechanical disruption, or cell lysing agents.
It may be desired to purify anti-TAT antibody and TAT polypeptide from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the anti-TAT antibody and TAT polypeptide. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular anti-TAT antibody or TAT polypeptide produced.
When using recombinant techniques, the antibody can be produced intracellularly, in the periplasmic space, or directly secreted into the medium. If the antibody is produced intracellularly, as a first step, the particulate debris, either host cells or lysed fragments, are removed, for example, by centrifugation or ultrafiltration. Carter et al., Bio/Teclmology 10: 163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.
The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human γ1, γ2 or γ4 heavy chains (Lindmark et al., J. Immunol. Meth. 62:1-13 (1983)). Protein G is recommended for all mouse isotypes and for human γ3 (Guss et al., EMBO J. 5:15671575 (1986)). The matrix to which the affinity ligand is attached is most often agarose, but other matrices are available. Mechanically stable matrices such as controlled pore glass or poly (styrenedivinyl)benzene allow for faster flow rates and shorter processing times than can be achieved with agarose. Where the antibody comprises a $C_H3$ domain, the Bakerbond ABX™resin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.
Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (e.g., from about 0-0.25M salt).

J. Pharmaceutical Formulations

Therapeutic formulations of the anti-TAT antibodies, TAT binding oligopeptides, TAT binding organic molecules and/or TAT polypeptides used in accordance with the present invention are prepared for storage by mixing the antibody, polypeptide, oligopeptide or organic molecule having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as acetate, Tris, phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; tonicifiers such as trehalose and sodium chloride; sugars such as sucrose, mannitol, trehalose or sorbitol; surfactant such as polysorbate; salt-forming counter-ions such as sodium; metal complexes (e.g., Zn-protein complexes); and/or non-ionic surfactants such as TWEEN®, PLURONICS® or polyethylene glycol (PEG). The antibody preferably comprises the antibody at a concentration of between 5-200 mg/ml, preferably between 10-100 mg/ml.
The formulations herein may also contain more than one active compound as necessary for the particular indication being treated, preferably those with complementary activities that do not adversely affect each other. For example, in addition to an anti-TAT antibody, TAT binding oligopeptide, or TAT binding organic molecule, it may be desirable to include in the one formulation, an additional antibody, e.g., a second anti-TAT antibody which binds a different epitope on the TAT polypeptide, or an antibody to some other target such as a growth factor that affects the growth of the particular cancer. Alternatively, or additionally, the composition may further comprise a chemotherapeutic agent, cytotoxic agent, cytokine, growth inhibitory agent, anti-hormonal agent, and/or cardioprotectant. Such molecules are suitably present in combination in amounts that are effective for the purpose intended.
The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. Ed. (1980).
Sustained-release preparations may be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRONDEPOT® (injectable microspheres composed of lactic acid-glycolic acid copolymer and leuprolide acetate), and poly-D-(-)-3-hydroxybutyric acid.
The formulations to be used for *in* vivo administration must be sterile. This is readily accomplished by filtration through sterile filtration membranes.

K. Diagnosis and Treatment with Anti-TAT Antibodies, TAT Binding Oligopeptides and TAT Binding Organic Molecules

To determine TAT expression in the cancer, various diagnostic assays are available. In one embodiment, TAT polypeptide overexpression may be analyzed by immunohistochemistry (IHC). Parrafin embedded tissue sections from a tumor biopsy may be subjected to the IHC assay and accorded a TAT protein staining intensity criteria as follows:
Score 0 - no staining is observed or membrane staining is observed in less than 10% of tumor cells.
Score 1+ - a faint/barely perceptible membrane staining is detected in more than 10% of the tumor cells. The cells are only stained in part of their membrane.
Score 2+ - a weak to moderate complete membrane staining is observed in more than 10% of the tumor cells.
Score 3+ - a moderate to strong complete membrane staining is observed in more than 10% of the tumor cells.
Those tumors with 0 or 1+ scores for TAT polypeptide expression may be characterized as not overexpressing TAT, whereas those tumors with 2+ or 3+ scores may be characterized as overexpressing TAT.
Alternatively, or additionally, FISH assays such as the INFORM® (sold by Ventana, Arizona) or PATHVISION® (Vysis, Illinois) may be carried out on formalin-fixed, paraffin-embedded tumor tissue to determine the extent (if any) of TAT overexpression in the tumor.
TAT overexpression or amplification may be evaluated using an in *vivo* diagnostic assay, e.g., by administering a molecule (such as an antibody, oligopeptide or organic molecule) which binds the molecule to be detected and is tagged with a detectable label (e.g., a radioactive isotope or a fluorescent label) and externally scanning the patient for localization of the label.
As described above, the anti-TAT antibodies, oligopeptides and organic molecules of the invention have various non-therapeutic applications. The anti-TAT antibodies, oligopeptides and organic molecules of the present invention can be useful for diagnosis and staging of TAT polypeptide-expressing cancers (e.g., in radioimaging). The antibodies, oligopeptides and organic molecules are also useful for purification or immunoprecipitation of TAT polypeptide from cells, for detection and quantitation of TAT polypeptide *in vitro,* e.g., in an ELISA or a Western blot, to kill and eliminate TAT-expressing cells from a population of mixed cells as a step in the purification of other cells.
Currently, depending on the stage of the cancer, cancer treatment involves one or a combination of the following therapies: surgery to remove the cancerous tissue, radiation therapy, and chemotherapy. Anti-TAT antibody, oligopeptide or organic molecule therapy may be especially desirable in elderly patients who do not tolerate the toxicity and side effects of chemotherapy well and in metastatic disease where radiation therapy has limited usefulness. The tumor targeting anti-TAT antibodies, oligopeptides and organic molecules of the invention are useful to alleviate TAT-expressing cancers upon initial diagnosis of the disease or during relapse. For therapeutic applications, the anti-TAT antibody, oligopeptide or organic molecule can be used alone, or in combination therapy with, e.g., hormones, antiangiogens, or radiolabelled compounds, or with surgery, cryotherapy, and/or radiotherapy. Anti-TAT antibody, oligopeptide or organic molecule treatment can be administered in conjunction with other forms of conventional therapy, either consecutively with, pre- or post-conventional therapy. Chemotherapeutic drugs such as TAXOTERE® (docetaxel), TAXOL® (palictaxel), estramustine and mitoxantrone are used in treating cancer, in particular, in good risk patients. In the present method of the invention for treating or alleviating cancer, the cancer patient can be administered anti-TAT antibody, oligopeptide or organic molecule in conjuction with treatment with the one or more of the preceding chemotherapeutic agents. In particular, combination therapy with palictaxel and modified derivatives (see, e.g., EP0600517) is contemplated. The anti-TAT antibody, oligopeptide or organic molecule will be administered with a therapeutically effective dose of the chemotherapeutic agent. In another embodiment, the anti-TAT antibody, oligopeptide or organic molecule is administered in conjunction with chemotherapy to enhance the activity and efficacy of the chemotherapeutic agent, e.g., paclitaxel. The Physicians' Desk Reference (PDR) discloses dosages of these agents that have been used in treatment of various cancers. The dosing regimen and dosages of these aforementioned chemotherapeutic drugs that are therapeutically effective will depend on the particular cancer being treated, the extent of the disease and other factors familiar to the physician of skill in the art and can be determined by the physician.
In one particular embodiment, a conjugate comprising an anti-TAT antibody, oligopeptide or organic molecule conjugated with a cytotoxic agent is administered to the patient. Preferably, the immunoconjugate bound to the TAT protein is internalized by the cell, resulting in increased therapeutic efficacy of the immunoconjugate in killing the cancer cell to which it binds. In a preferred embodiment, the cytotoxic agent targets or interferes with the nucleic acid in the cancer cell. Examples of such cytotoxic agents are described above and include maytansinoids, calicheamicins, ribonucleases and DNA endonucleases.
The anti-TAT antibodies, oligopeptides, organic molecules or toxin conjugates thereof are administered to a human patient, in accord with known methods, such as intravenous administration, e.g." as a bolus or by continuous infusion over a period of time, by intramuscular, intraperitoneal, intracerobrospinal, subcutaneous, intra-articular, intrasynovial, intrathecal, oral, topical, or inhalation routes. Intravenous or subcutaneous administration of the antibody, oligopeptide or organic molecule is preferred.

Other therapeutic regimens may be combined with the administration of the anti-TAT antibody, oligopeptide or organic molecule. The combined administration includes co-administration, using separate formulations or a single pharmaceutical formulation, and consecutive administration in either order, wherein preferably there is a time period while both (or all) active agents simultaneously exert their biological activities. Preferably such combined therapy results in a synergistic therapeutic effect.

It may also be desirable to combine administration of the anti-TAT antibody or antibodies, oligopeptides or organic molecules, with administration of an antibody directed against another tumor antigen associated with the particular cancer. In another embodiment, the therapeutic treatment methods of the present invention involves the combined administration of an anti-TAT antibody (or antibodies), oligopeptides or organic molecules and one or more chemotherapeutic agents or growth inhibitory agents, including co-administration of cocktails of different chemotherapeutic agents. Chemotherapeutic agents include estramustine phosphate, prednimustine, cisplatin, 5-fluorouracil, melphalan, cyclophosphamide, hydroxyurea and hydroxyureataxanes (such as paclitaxel and doxetaxel) and/or anthracycline antibiotics. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992).

The antibody, oligopeptide or organic molecule may be combined with an anti-hormonal compound; e.g., an anti-estrogen compound such as tamoxifen; an anti-progesterone such as onapristone (see, EP 616 812); or an anti-androgen such as flutamide, in dosages known for such molecules. Where the cancer to be treated is androgen independent cancer, the patient may previously have been subjected to anti-androgen therapy and, after the cancer becomes androgen independent, the anti-TAT antibody, oligopeptide or organic molecule (and optionally other agents as described herein) may be administered to the patient

Sometimes, it may be beneficial to also co-administer a cardioprotectant (to prevent or reduce myocardial dysfunction associated with the therapy) or one or more cytokines to the patient. In addition to the above therapeutic regimes, the patient may be subjected to surgical removal of cancer cells and/or radiation therapy, before, simultaneously with, or post antibody, oligopeptide or organic molecule therapy. Suitable dosages for any of the above co-administered agents are those presently used and may be lowered due to the combined action (synergy) of the agent and anti-TAT antibody, oligopeptide or organic molecule.

For the prevention or treatment of disease, the dosage and mode of administration will be chosen by the physician according to known criteria. The appropriate dosage of antibody, oligopeptide or organic molecule will depend on the type of disease to be treated, as defined above, the severity and course of the disease, whether the antibody, oligopeptide or organic molecule is administered for preventive or therapeutic purposes, previous therapy, the patient's clinical history and response to the antibody, oligopeptide or organic molecule, and the discretion of the attending physician. The antibody, oligopeptide or organic molecule is suitably administered to the patient at one time or over a series of treatments. Preferably, the antibody, oligopeptide or organic molecule is administered by intravenous infusion or by subcutaneous injections. Depending on the type and severity of the disease, about 1 $\mu$g/kg to about 50 mg/kg body weight (e.g., about 0.1-15mg/kg/dose) of antibody can be an initial candidate dosage for administration to the patient, whether, for example, by one or more separate administrations, or by continuous infusion. A dosing regimen can comprise administering an initial loading dose of about 4 mg/kg, followed by a weekly maintenance dose of about 2 mg/kg of the anti-TAT antibody. However, other dosage regimens may be useful. A typical daily dosage might range from about 1 $\mu$g/kg to 100 mg/kg or more, depending on the factors mentioned above. For repeated administrations over several days or longer, depending on the condition, the treatment is sustained until a desired suppression of disease symptoms occurs. The progress of this therapy can be readily monitored by conventional methods and assays and based on criteria known to the physician or other persons of skill in the art.

Aside from administration of the antibody protein to the patient, the present application contemplates administration of the antibody by gene therapy. Such administration of nucleic acid encoding the antibody is encompassed by the expression "administering a therapeutically effective amount of an autibody". See, for example, WO96/07321 published March 14, 1996 concerning the use of gene therapy to generate intracellular antibodies.

There are two major approaches to getting the nucleic acid (optionally contained in a vector) into the patient's cells; *in vivo* and *ex vivo*. For *in vivo* delivery the nucleic acid is injected directly into the patient, usually at the site where the antibody is required. For *ex vivo* treatment, the patient's cells are removed, the nucleic acid is introduced into these isolated cells and the modified cells are administered to the patient either directly or, for example, encapsulated within porous membranes which are implanted into the patient (see, e.g., U.S. Patent Nos. 4,892,538 and 5,283,187). There are a variety of techniques available for introducing nucleic acids into viable cells. The techniques vary depending upon whether the nucleic acid is transferred into cultured cells *in vitro,* or in *vivo* in the cells of the intended host Techniques suitable for the transfer of nucleic acid into mammalian cells *in vitro* include the use of liposomes, electroporation, microinjection, cell fusion, DEAE-dextran, the calcium phosphate precipitation method, etc. A commonly used vector for ex *vivo* delivery of the gene is a retroviral vector.

The currently preferred *in vivo* nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). For review of the currently known gene marking and gene therapy protocols see Anderson et al., Science 256:808-813 (1992). See also WO 93/25673 and the references cited therein.

The anti-TAT antibodies of the invention can be in the different forms encompassed by the definition of "antibody" herein. Thus, the antibodies include full length or intact antibody, antibody fragments, native sequence antibody or amino acid variants, humanized, chimeric or fusion antibodies, immunoconjugates, and functional fragments thereof. In fusion antibodies an antibody sequence is fused to a heterologous polypeptide sequence. The antibodies can be modified in the Fc region to provide desired effector functions. As discussed in more detail in the sections herein, with the appropriate Fc regions, the naked antibody bound on the cell surface can induce cytotoxicity, e.g., via antibody-dependent cellular cytotoxicity (ADCC) or by recruiting complement in complement dependent cytotoxicity, or some other mechanism. Alternatively, where it is desirable to eliminate or reduce effector function, so as to minimize side effects or therapeutic complications, certain other Fc regions may be used.

In one embodiment, the antibody competes for binding or bind substantially to, the same epitope as the antibodies of the invention. Antibodies having the biological characteristics of the present anti-TAT antibodies of the invention are also contemplated, specifically including the *in vivo* tumor targeting and any cell proliferation inhibition or cytotoxic characteristics.

Methods of producing the above antibodies are described in detail herein.

The present anti-TAT antibodies, oligopeptides and organic molecules are useful for treating a TAT-expressing cancer or alleviating one or more symptoms of the cancer in a mammal. Such a cancer includes prostate cancer, cancer of the urinary tract, lung cancer, breast cancer, colon cancer and ovarian cancer, more specifically, prostate adenocarcinoma, renal cell carcinomas, colorectal adenocarcinomas, lung adenocarcinomas, lung squamous cell carcinomas, and pleural mesothelioma. The cancers encompass metastatic cancers of any of the preceding. The antibody, oligopeptide or organic molecule is able to bind to at least a portion of the cancer cells that express TAT polypeptide in the mammal. In a preferred embodiment, the antibody, oligopeptide or organic molecule is effective to destroy or kill TAT-expressing tumor cells or inhibit the growth of such tumor cells, *in vitro* or *in vivo,* upon binding to TAT polypeptide on the cell. Such an antibody includes a naked anti-TAT antibody (not conjugated to any agent). Naked antibodies that have cytotoxic or cell growth inhibition properties can be further harnessed with a cytotoxic agent to render them even more potent in tumor cell destruction. Cytotoxic properties can be conferred to an anti-TAT antibody by, e.g., conjugating the antibody with a cytotoxic agent, to form an immunoconjugate as described herein. The cytotoxic agent or a growth inhibitory agent is preferably a small molecule. Toxins such as calicheamicin or a maytansinoid and analogs or derivatives thereof, are preferable.

The invention provides a composition comprising an anti-TAT antibody, oligopeptide or organic molecule of the invention, and a carrier. For the purposes of treating cancer, compositions can be administered to the patient in need of such treatment, wherein the composition can comprise one or more anti-TAT antibodies present as an immunoconjugate or as the naked antibody. In a further embodiment, the compositions can comprise these antibodies, oligopeptides or organic molecules in combination with other therapeutic agents such as cytotoxic or growth inhibitory agents, including chemotherapeutic agents. The invention also provides formulations comprising an anti-TAT antibody, oligopeptide or organic molecule of the invention, and a carrier. In one embodiment, the formulation is a therapeutic formulation comprising a pharmaceutically acceptable carrier.

Another aspect of the invention is isolated nucleic acids encoding the anti-TAT antibodies. Nucleic acids encoding both the H and L chains and especially the hypervariable region residues, chains which encode the native sequence antibody as well as variants, modifications and humanized versions of the antibody, are encompassed.

The invention also provides methods useful for treating a TAT polypeptide-expressing cancer or alleviating one or more symptoms of the cancer in a mammal, comprising administering a therapeutically effective amount of an anti-TAT antibody, oligopeptide or organic molecule to the mammal. The antibody, oligopeptide or organic molecule therapeutic compositions can be administered short term (acute) or chronic, or intermittent as directed by physician. Also provided are methods of inhibiting the growth of, and killing a TAT polypeptide-expressing cell.

The invention also provides kits and articles of manufacture comprising at least one anti-TAT antibody, oligopeptide or organic molecule. Kits containing anti-TAT antibodies, oligopeptides or organic molecules find use, e.g., for TAT cell killing assays, for purification or immunoprecipitation of TAT polypeptide from cells. For example, for isolation and purification of TAT, the kit can contain an anti-TAT antibody, oligopeptide or organic molecule coupled to beads (e.g., sepharose beads). Kits can be provided which contain the antibodies, oligopeptides or organic molecules for detection and quantitation of TAT *in vitro,* e.g., in an ELISA or a Western blot. Such antibody, oligopeptide or organic molecule useful for detection may be provided with a label such as a fluorescent or radiolabel.

L. Articles of Manufacture and Kits

Another embodiment of the invention is an article of manufacture containing materials useful for the treatment of anti-TAT expressing cancer. The article of manufacture comprises a container and a label or package insert on or associated with the container. Suitable containers include, for example, bottles, vials, syringes, etc. The containers may be formed from a variety of materials such as glass or plastic. The container holds a composition which is effective for treating the cancer condition and may have a sterile access port (for example the container may be an intravenous solution bag or a vial having a stopper pierceable by a hypodermic injection needle). At least one active agent in the composition is an anti-TAT antibody, oligopeptide or organic molecule of the invention. The label or package insert indicates that the composition is used for treating cancer. The label or package insert will further comprise instructions for administering the antibody, oligopeptide or organic molecule composition to the cancer patient Additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Kits are also provided that are useful for various purposes , e.g., for TAT-expressing cell killing assays, for purification or immunoprecipitation of TAT polypeptide from cells. For isolation and purification of TAT polypeptide, the kit can contain an anti-TAT antibody, oligopeptide or organic molecule coupled to beads (e.g., sepharose beads). Kits can be provided which contain the antibodies, oligopeptides or organic molecules for detection and quantitation of TAT polypeptide *in vitro,* e.g., in an ELISA or a Western blot. As with the article of manufacture, the kit comprises a container and a label or package insert on or associated with the container. The container holds a composition comprising at least one anti-TAT antibody, oligopeptide or organic molecule of the invention. Additional containers may be included that contain, e.g., diluents and buffers, control antibodies. The label or package insert may provide a description of the composition as well as instructions for the intended in vitro or diagnostic use.

M. Uses for TAT Polypeptides and TAT-Polypeptide Encoding Nucleic Acids

Nucleotide sequences (or their complement) encoding TAT polypeptides have various applications in the art of molecular biology, including uses as hybridization probes, in chromosome and gene mapping and in the generation of anti-sense RNA and DNA probes. TAT-encoding nucleic acid will also be useful for the preparation of TAT polypeptides by the recombinant techniques described herein, wherein those TAT polypeptides may find use, for example, in the preparation of anti-TAT antibodies as described herein.

The full-length native sequence TAT gene, or portions thereof, may be used as hybridization probes for a cDNA library to isolate the full-length TAT cDNA or to isolate still other cDNAs (for instance, those encoding naturally-occurring variants of TAT or TAT from other species) which have a desired sequence identity to the native TAT sequence disclosed herein. Optionally, the length of the probes will be about 20 to about 50 bases. The hybridization probes may be derived from at least partially novel regions of the full length native nucleotide sequence wherein those regions may be determined without undue experimentation or from genomic sequences including promoters, enhancer elements and introns of native sequence TAT. By way of example, a screening method will comprise isolating the coding region of the TAT gene using the known DNA sequence to synthesize a selected probe of about 40 bases. Hybridization probes may be labeled by a variety of labels, including radionucleotides such as $^{32}P$ or $^{35}S$, or enzymatic labels such as alkaline phosphatase coupled to the probe via avidin/biotin coupling systems. Labeled probes having a sequence complementary to that of the TAT gene of the present invention can be used to screen libraries of human cDNA, genomic DNA or mRNA to determine which members of such libraries the probe hybridizes to. Hybridization techniques are described in further detail in the Examples below. Any EST sequences disclosed in the present application may similarly be employed as probes, using the methods disclosed herein.

Other useful fragments of the TAT-encoding nucleic acids include antisense or sense oligonucleotides comprising a singe-stranded nucleic acid sequence (either RNA or DNA) capable of binding to target TAT mRNA (sense) or TAT DNA (antisense) sequences. Antisense or sense oligonucleotides, according to the present invention, comprise a fragment of the coding region of TAT DNA. Such a fragment generally comprises at least about 14 nucleotides, preferably from about 14 to 30 nucleotides. The ability to derive an antisense or a sense oligonucleotide, based upon a cDNA sequence encoding a given protein is described in, for example, Stein and Cohen (Cancer Res. 48:2659, 1988) and van der Krol et al. (BioTechniques 6:958, 1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. Such methods are encompassed by the present invention. The antisense oligonucleotides thus may be used to block expression of TAT proteins, wherein those TAT proteins may play a role in the induction of cancer in mammals. Antisense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences.

Preferred intragenic sites for antisense binding include the region incorporating the translation initiation/start codon (5'-AUG / 5'-ATG) or termination/stop codon (5'-UAA, 5'-UAG and 5-UGA/ 5'-TAA, 5'-TAG and 5'-TGA) of the open reading frame (ORF) of the gene. These regions refer to a portion of the mRNA or gene that encompasses from about 25 to about 50 contiguous nucleotides in either direction (i.e., 5' or 3') from a translation initiation or termination codon. Other preferred regions for antisense binding include: introns; exons; intron-exon junctions; the open reading frame (ORF) or "coding region," which is the region between the translation initiation codon and the translation termination codon; the 5' cap of an mRNA which comprises an N7-methylated guanosine residue joined to the 5'-most residue of the mRNA via a 5'-5' triphosphate linkage and includes 5' cap structure itself as well as the first 50 nucleotides adjacent to the cap; the 5' untranslated region (5'UTR), the portion of an mRNA in the 5' direction from the translation initiation codon, and thus including nucleotides between the 5' cap site and the translation initiation codon of an mRNA or corresponding nucleotides on the gene; and the 3' untranslated region (3'UTR), the portion of an mRNA in the 3' direction from the translation termination codon, and thus including nucleotides between the translation termination codon and 3' end of an mRNA or corresponding nucleotides on the gene.

Specific examples of preferred antisense compounds useful for inhibiting expression of TAT proteins include oligonucleotides containing modified backbones or non-natural intemucleoside linkages. Oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides.

Preferred modified oligonucleotide backbones include, for example, phosphorothioates, chiral phosphorothioates, phosphorodithioates, phosphotriesters, aminoalkylphosphotri-esters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and borano-phosphates having normal 3'-5' linkages, 2'-5' linked analogs of these, and those having inverted polarity wherein one or more internucleotide linkages is a 3' to 3', 5' to 5' or 2' to 2' linkage. Preferred oligonucleotides having inverted polarity comprise a single 3' to 3' linkage at the 3'-most internucleotide linkage i.e. a single inverted nucleoside residue which may be abasic (the nucleobase is missing or has a hydroxyl group in place thereof). Various salts, mixed salts and free acid forms are also included. Representative United States patents that teach the preparation of phosphorus-containing linkages include, but are not limited to, U.S. Pat. Nos.: 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; 5,194,599; 5,565,555; 5,527,899; 5,721,218; 5,672,697 and 5,625,050, each of which is herein incorporated by reference.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH.sub.2 component parts. Representative United States patents that teach the preparation of such oligonucleosides include, but are not limited to,. U.S. Pat. Nos.: 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225;5,596,086; 5,602,240; 5,610,289; 5,602,240; 5,608,046; 5,610,289; 5,618,704; 5,623,070; 5,663,312; 5,633,360; 5,677,437; 5,792,608; 5,646,269 and 5,677,439, each ofwhichis herein incorporated by reference.

In other preferred antisense oligonucleotides, both the sugar and the internucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S. Pat. Nos.: 5,539,082; 5,714,331; and 5,719,262, each of which is herein incorporated by reference. Further teaching of PNA compounds can be found in Nielsen et al., Science, 1991, 254, 1497-1500.

Preferred antisense oligonucleotides incorporate phosphorothioate backbones and/or heteroatom backbones, and in particular-$CH_2$-NH-O-$CH_2$-, -$CH_2$-N($CH_3$)-O-$CH_2$- [known as a methylene (methylimino) or MMI backbone], -$CH_2$-O-N($CH_3$)-$CH_2$-, -$CH_2$-N($CH_3$)-N($CH_3$)-$CH_2$- and -O-N($CH_3$)-$CH_2$-$CH_2$- [wherein the native phosphodiester backbone is represented as -O-P-O-$CH_2$-] described in the above referenced U.S. Pat. No. 5,489,677, and the amide backbones of the above referenced U.S. Pat. No. 5,602,240. Also preferred are antisense oligonucleotides having morpholino backbone structures of the above-referenced U.S. Pat. No. 5,034,506.

Modified oligonucleotides may also contain one or more substituted sugar moieties. Preferred oligonucleotides comprise one of the following at the 2' position: OH; F; O-alkyl, S-alkyl, or N-alkyl; O-alkenyl, S-alkeynyl, orN-alkenyl; O-alkynyl, S-alkynyl or N-alkynyl; or O-alkyl-O-alkyl, wherein the alkyl, alkenyl and alkynyl may be substituted or unsubstituted $C_1$ to $C_{10}$ alkyl or $C_2$ to $C_{10}$ alkenyl and alkynyl. Particularly preferred are $O[(CH_2)_nO]_mCH_3$, $O(CH_2)_nOCH_3$, $O(CH_2)_nNH_2$, $O(CH_2)_nCH_3$, $O(CH_2)_nONH_2$, and $O(CH_2)_nON[(CH_2)_nCH_3)]_2$, where n and m are from 1 to about 10. Other preferred antisense oligonucleotides comprise one of the following at the 2' position: $C_1$ to $C_{10}$ lower alkyl, substituted lower alkyl, alkenyl, alkynyl, alkaryl, aralkyl, O-alkaryl or O-aralkyl, SH, $SCH_3$, OCN, Cl, Br, CN, $CF_3$, $OCF_3$, $SOCH_3$, $SO_2$ $CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy (2'-O-$CH_2CH_2OCH_3$, also known as 2'-O-(2-methoxyethyl) or 2'-MOE) (Martin et al., Helv. Chim. Acta, 1995, 78, 486-504) i.e., an alkoxyalkoxy group. A further preferred modification includes 2'-dimethylaminooxyethoxy, i.e., a $O(CH_2)_2ON(CH_3)_2$ group, also known as 2'-DMAOE, as described in examples hereinbelow, and 2'-dimethylaminoethoxyethoxy (also known in the art as 2'-O-dimethylaminoethoxyethyl or 2'-DMAEOE), i.e., 2'-O-$CH_2$-O-$CH_2$-N($CH_2$).

A further prefered modification includes Locked Nucleic Acids (LNAs) in which the 2'-hydroxyl group is linked to the 3' or 4' carbon atom of the sugar ring thereby forming a bicyclic sugar moiety. The linkage is preferably a methelyne ($-CH_2-)_n$ group bridging the 2' oxygen atom and the 4' carbon atom wherein n is 1 or 2. LNAs and preparation thereof are described in WO 98/39352 and WO 99/14226.

Other preferred modifications include 2'-methoxy (2'-O-$CH_3$), 2'-aminopropoxy (2'-$OCH_2CH_2CH_2$ $NH_2$), 2'-allyl (2'-$CH_2$-CH=$CH_2$), 2'-O-allyl (2'-O-$CH_2$-CH=$CH_2$) and 2'-fluoro (2'-F). The 2'-modification may be in the arabino (up) position or ribo (down) position. A preferred 2'-arabino modification is 2'-F. Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar. Representative United States patents that teach the preparation of such modified sugar structures include, but are not limited to, U.S. Pat. Nos.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; and 5,700,920, each of which is herein incorporated by reference in its entirety.

Oligonucleotides may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-$CH_3$ or -$CH_2$-C≡CH) uracil and cytosine and other alkynyl derivatives ofpyrimidine bases, 6-azo uracil, cytosine and thymine, 5-uracil (pseudouracil), 4-thiouracil, 8-halo, 8-amino, 8-thiol, 8-thioallcyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine(1H-pyrimido[5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in U.S. Pat. No. 3,687,808, those disclosed in The Concise Encyclopedia Of Polymer Science And Engineering, pages 858-859, Kroschwitz, J. I., ed. John Wiley & Sons, 1990, and those disclosed by Englisch et al., Angewandte Chemie, International Edition, 1991, 30, 613. Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2.degree. C. (Sanghvi et al, Antisense Research and Applications, CRC Press, Boca Raton, 1993, pp. 276-278) and are preferred base substitutions, even more particularly when combined with 2'-O-methoxyethyl sugar modifications. Representative United States patents that teach the preparation of modified nucleobases include, but are not limited to: U.S. Pat. No. 3,687,808, as well as U.S. Pat Nos.: 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,645,985; 5,830,653; 5,763,588; 6,005,096; 5,681,941 and5,750,692, each of which is herein incorporated by reference. Another modification of antisense oligonucleotides chemically linking to the oligonucleotide one or more moieties or conjugates which enhance the activity, cellular distribution or cellular uptake of the oligonucleotide. The compounds of the invention can include conjugate groups covalently bound to functional groups such as primary or secondary hydroxyl groups. Conjugate groups of the invention include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugates groups include cholesterols, lipids, cation lipids, phospholipids, cationic phospholipids, biotin, phenazine, folate, phenanthridine, anthraquinone, acridine, fluoresceins, rhodamines, coumarins, and dyes. Groups that enhance the pharmacodynamic properties, in the context of this invention, include groups that improve oligomer uptake, enhance oligomer resistance to degradation, and/or strengthen sequence-specific hybridization with RNA. Groups that enhance the pharmacokinetic properties, in the context of this invention, include groups that improve oligomer uptake, distribution, metabolism or excretion. Conjugate moieties include but are not limited to lipid moieties such as a cholesterol moiety (Letsinger et al., Proc. Natl. Acad. Sci. USA,1989, 86, 6553-6556), cholic acid (Manoharan et al., Bioorg. Med. Chem. Let.,1994, 4,1053-1060), a thioether, e.g., hexyl-S-tritylthiol (Manoharan et al., Ann. N.Y. Acad. Sci.,1992, 660, 306-309; Manoharan et al., Bioorg. Med. Chem. Let.,1993, 3, 2765-2770), a thiocholesterol (Oberhauser et al., Nucl. Acids Res., 1992, 20, 533-538), an aliphatic chain, e.g., dodecandiol or undecyl residues (Saison-Behmoaras et al., EMBO J.,1991,10,1111-1118; Kabanov et al., FEBS Lett.,1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett.,1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Misbra et al., Biochim. Biophys. Acta, 1995, 1264, 229-237), or an octadecylamine or hexylamino-carbonyl-oxycholesterol moiety. Oligonucleotides of the invention may also be conjugated to active drug substances, for example, aspirin, warfarin, phenylbutazone, ibuprofen, suprofen, fenbufen, ketoprofen, (S)-(+)-pranoprofen, carprofen, dansylsarcosine, 2,3,5-triiodobenzoic acid, flufenamic acid, folinic acid, a benzothiadiazide, chlorothiazide, a diazepine, indomethicin, a barbiturate, a cephalosporin, a sulfa drug, an antidiabetic, an antibacterial or an antibiotic. Oligonucleotide-drug conjugates and their preparation are described in U.S. patent application Ser. No. 09/334,130 (filed Jun 15, 1999) and United States patents Nos.: 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203,5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941, each of which is herein incorporated by reference.

It is not necessary for all positions in a given compound to be uniformly modified, and in fact more than one of the aforementioned modifications may be incorporated in a single compound or even at a single nucleoside within an oligonucleotide. The present invention also includes antisense compounds which are chimeric compounds. "Chimeric" antisense compounds or "chimeras," in the context of this invention, are antisense compounds, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Chimeric antisense compounds of the invention may be formed as composite structures of two or more oligonucleotides, modified oligonucleotides, oligonucleosides and/or oligonucleotide mimetics as described above. Preferred chimeric antisense oligonucleotides incorporate at least one 2' modified sugar (preferably 2'-O-$(CH_2)_2$-O-$CH_3$) at the 3' terminal to confer nuclease resistance and a region with at least 4 contiguous 2'-H sugars to confer RNase H activity. Such compounds have also been referred to in the art as hybrids or gapmers. Preferred gapmers have a region of 2' modified sugars (preferably 2'-O-$(CH_2)_2$-O-$CH_3$) at the 3'-teiminal and at the 5' terminal separated by at least one region having at least 4 contiguous 2'-H sugars and preferably incorporate phosphorothioate backbone linkages. Representative United States patents that teach the preparation of such hybrid structures include, but are not limited to, U.S. Pat. Nos. 5,013,830; 5,149,797; 5,220,007; 5,256,775; 5,366,878; 5,403,711; 5,491,133; 5,565,350; 5,623,065; 5,652,355; 5,652,356; and 5,700,922, each of which is herein incorporated by reference in its entirety.

The antisense compounds used in accordance with this invention may be conveniently and routinely made through the well-known technique of solid phase synthesis. Equipment for such synthesis is sold by several vendors including, for example, Applied Biosystems (Foster City, Calif.). Any other means for such synthesis known in the art may additionally or alternatively be employed. It is well known to use similar techniques to prepare oligonucleotides such as the phosphorothioates and alkylated derivatives. The compounds of the invention may also be admixed, encapsulated, conjugated or otherwise associated with other molecules, molecule structures or mixtures of compounds, as for example, liposomes, receptor targeted molecules, oral, rectal, topical or other formulations, for assisting in uptake, distribution and/or absorption. Representative United States patents that teach the preparation of such uptake, distribution and/or absorption assisting formulations include, but are not limited to, U.S. Pat. Nos. 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756, each of which is herein incorporated by reference.

Other examples of sense or antisense oligonucleotides include those oligonucleotides which are covalently linked to organic moieties, such as those described in WO 90/10048, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, $CaPO_4$-mediated DNA transfection, electroporation, or by using gene transfer vectors such as Epstein-Barr virus. In a preferred procedure, an antisense or sense oligonucleotide is inserted into a suitable retroviral vector. A cell containing the target nucleic acid sequence is contacted with the recombinant retroviral vector, either *in vivo* or *ex vivo*. Suitable retroviral vectors include, but are not limited to, those derived from the murine retrovirus M-MuLV, N2 (a retrovirus derived from M-MuLV), or the double copy vectors designated DCT5A, DCT5B and DCT5C (see WO 90/13641).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

Antisense or sense RNA or DNA molecules are generally at least about 5 nucleotides in length, alternatively at least about 6,7,8,9,10,11,12,13,14,15,16,17,18,19,20,21,22,23,24,25,26,27,28,29,30,35,40, 45,50,55,60,65,70,75,80,85,90, 95,100,105,110,115,120,125,130,135,140,145,150,155,160,165,170,175,180, 185,190,195,200,210,220,230,240, 250,260,270,280,290,300,310,320,330,340,350,360,370,380,390,400,410, 420,430,440,450,460,470,480,490,500, 510,520,530,540,550,560,570,580,590,600,610,620,630,640,650,660, 670,680,690,700,710,720,730,740,750,760, 770,780,790,800,810,820,830,840,850,860,870,880,890,900,910, 920,930,940,950,960,970,980,990, or 1000 nucleotides in length, wherein in this context the term "about" means the referenced nucleotide sequence length plus or minus 10% of that referenced length.

The probes may also be employed in PCR techniques to generate a pool of sequences for identification of closely related TAT coding sequences.

Nucleotide sequences encoding a TAT can also be used to construct hybridization probes for mapping the gene which encodes that TAT and for the genetic analysis of individuals with genetic disorders. The nucleotide sequences provided herein may be mapped to a chromosome and specific regions of a chromosome using known techniques, such as *in situ* hybridization, linkage analysis against known chromosomal markers, and hybridization screening with libraries.

When the coding sequences for TAT encode a protein which binds to another protein (example, where the TAT is a receptor), the TAT can be used in assays to identify the other proteins or molecules involved in the binding interaction. By such methods, inhibitors of the receptor/ligand binding interaction can be identified. Proteins involved in such binding interactions can also be used to screen for peptide or small molecule inhibitors or agonists of the binding interaction. Also, the receptor TAT can be used to isolate correlative ligand(s). Screening assays can be designed to find lead compounds that mimic the biological activity of a native TAT or a receptor for TAT. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode TAT or its modified forms can also be used to generate either transgenic animals or "knock out" animals which, in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. In one embodiment, cDNA encoding TAT can be used to clone genomic DNA encoding TAT in accordance with established techniques and the genomic sequences used to generate transgenic animals that contain cells which express DNA encoding TAT. Methods for generating transgenic animals, particularly animals such as mice or rats, have become conventional in the art and are described, for example, in U.S. Patent Nos. 4,736,866 and 4,870,009. Typically, particular cells would be targeted for TAT transgene incorporation with tissue-specific enhancers. Transgenic animals that include a copy of a transgene encoding TAT introduced into the germ line of the animal at an embryonic stage can be used to examine the effect of increased expression of DNA encoding TAT. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition.

Alternatively, non-human homologues of TAT can be used to construct a TAT "knock out" animal which has a defective or altered gene encoding TAT as a result of homologous recombination between the endogenous gene encoding TAT and altered genomic DNA encoding TAT introduced into an embryonic stem cell of the animal. For example, cDNA encoding TAT can be used to clone genomic DNA encoding TAT in accordance with established techniques. A portion of the genomic DNA encoding TAT can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g., a mouse or rat) to form aggregation chimeras [see e.g., Bradley, in Teratocarcinomas and Embryonic Stem Cells: A Practical Approach, E. J. Robertson, ed. (IRL, Oxford, 1987), pp. 113-152]. Acbimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the TAT polypeptide.

Nucleic acid encoding the TAT polypeptides may also be used in gene therapy. In gene therapy applications, genes are introduced into cells in order to achieve *in vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes *in vivo.* It has already been shown that short antisense oligonucleotides can be imported into cells where they act as inhibitors, despite their low intracellular concentrations caused by their restricted uptake by the cell membrane. (Zamecnik et al., Proc. Natl. Acad. Sci. USA 83:4143-4146 [1986]). The oligonucleotides can be modified to enhance their uptake, e.g. by substituting their negatively charged phosphodiester groups by uncharged groups.

There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. The currently preferred in *vivo* gene transfer techniques include transfection with viral (typically retroviral) vectors and viral coat protein-liposome mediated transfection (Dzau et al., Trends in Biotechnology 11, 205-210 [1993]). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, proteins that target intracellular localization and enhance intracellular half-life. The technique of receptor-mediated endocytosis is described, for example, by Wu et al., J. Biol. Chem. 262, 4429-4432 (1987); and Wagner et al., Proc. Natl. Acad. Sci. USA 87, 3410-3414 (1990). For review of gene marking and gene therapy protocols see Anderson et al., Science 256, 808-813 (1992).

The nucleic acid molecules encoding the TAT polypeptides or fragments thereof described herein are useful for chromosome identification. In this regard, there exists an ongoing need to identify new chromosome markers, since relatively few chromosome marking reagents, based upon actual sequence data are presently available. Each TAT nucleic acid molecule of the present invention can be used as a chromosome marker.

The TAT polypeptides and nucleic acid molecules of the present invention may also be used diagnostically for tissue typing, wherein the TAT polypeptides of the present invention may be differentially expressed in one tissue as compared to another, preferably in a diseased tissue as compared to a normal tissue of the same tissue type. TAT nucleic acid molecules will find use for generating probes for PCR, Northern analysis, Southern analysis and Western analysis.

This invention encompasses methods of screening compounds to identify those that mimic the TAT polypeptide (agonists) or prevent the effect of the TAT polypeptide (antagonists). Screening assays for antagonist drug candidates are designed to identify compounds that bind or complex with the TAT polypeptides encoded by the genes identified herein, or otherwise interfere with the interaction of the encoded polypeptides with other cellular proteins, including e.g., inhibiting the expression ofTAT polypeptide from cells. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with a TAT polypeptide encoded by a nucleic acid identified herein under conditions and for a time sufficient to allow these two components to interact.

In binding assays, the interaction is binding and the complex formed can be isolated or detected in the reaction mixture. In a particular embodiment, the TAT polypeptide encoded by the gene identified herein or the drug candidate is immobilized on a solid phase, e.g., on a microtiter plate, by covalent or non-covalent attachments. Non-covalent attachment generally is accomplished by coating the solid surface with a solution of the TAT polypeptide and drying. Alternatively, an immobilized antibody, e.g., a monoclonal antibody, specific for the TAT polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular TAT polypeptide encoded by a gene identified herein, its interaction with that polypeptide can be assayed by methods well known for detecting protein-protein interactions. Such assays include traditional approaches, such as, e.g., cross-linking, co-immunoprecipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a gene encoding a TAT polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the TAT polypeptide may be added to a cell along with the compound to be screened for a particular activity and the ability of the compound to inhibit the activity of interest in the presence of the TAT polypeptide indicates that the compound is an antagonist to the TAT polypeptide. Alternatively, antagonists may be detected by combining the TAT polypeptide and a potential antagonist with membrane-bound TAT polypeptide receptors or recombinant receptors under appropriate conditions for a competitive inhibition assay. The TAT polypeptide can be labeled, such as by radioactivity, such that the number of TAT polypeptide molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the TAT polypeptide and a cDNA library created from this RNA is divided into pools and used to transfect COS cells or other cells that are not responsive to the TAT polypeptide. Transfected cells that are grown on glass slides are exposed to labeled TAT polypeptide. The TAT polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, labeled TAT polypeptide can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro- sequencing would be used to design a set of degenerate oligonucleotide probes to screen a cDNA library to identify the gene encoding the putative receptor.

In another assay for antagonists, mammalian cells or a membrane preparation expressing the receptor would be incubated with labeled TAT polypeptide in the presence of the candidate compound. The ability of the compound to enhance or block this interaction could then be measured.

More specific examples of potential antagonists include an oligonucleotide that binds to the fusions of immunoglobulin with TAT polypeptide, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the TAT polypeptide that recognizes the receptor but imparts no effect, thereby competitively inhibiting the action of the TAT polypeptide.

Another potential TAT polypeptide antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature TAT polypeptides herein, is used to design an antisense RNA oligonucleotide of from about 10 to 40 base pairs in length. A DNA oligonucleotide is designed to be complementary to a region of the gene involved in transcription (triple helix - see Lee et al., Nucl. Acids Res., 6:3073 (1979); Cooney et al., Science, 241: 456 (1988); Dervan et al., Science, 251:1360 (1991)), thereby preventing transcription and the production of the TAT polypeptide. The antisense RNA oligonucleotide hybridizes to the mRNA *in vivo* and blocks translation of the mRNA molecule into the TAT polypeptide (antisense - Okano, Neurochem., 56:560 (1991); Oligodeoxynucleotides as Antisense Inhibitors of Gene Expression (CRC Press: Boca Raton, FL, 1988). The oligonucleotides described above can also be delivered to cells such that the antisense RNA or DNA may be expressed *in vivo* to inhibit production of the TAT polypeptide. When antisense DNA is used, oligodeoxyribonucleotides derived from the translation-initiation site, e.g., between about -10 and +10 positions of the target gene nucleotide sequence, are preferred.

Potential antagonists include small molecules that bind to the active site, the receptor binding site, or growth factor or other relevant binding site of the TAT polypeptide, thereby blocking the normal biological activity of the TAT polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, e.g., Rossi, Current Biology, 4:469-471 (1994), and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, e.g., PCT publication No. WO 97/33551, *supra.*

These small molecules can be identified by any one or more of the screening assays discussed hereinabove and/or by any other screening techniques well known for those skilled in the art.
Isolated TAT polypeptide-encoding nucleic acid can be used herein for recombinantly producing TAT polypeptide using techniques well known in the art and as described herein. In turn, the produced TAT polypeptides can be employed for generating anti-TAT antibodies using techniques well known in the art and as described herein.
Antibodies specifically binding a TAT polypeptide identified herein, as well as other molecules identified by the screening assays disclosed hereinbefore, can be administered for the treatment of various disorders, including cancer, in the form of pharmaceutical compositions.
If the TAT polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, *e.g.*, Marasco et al., Proc. Natl. Acad. Sci. USA, 90: 7889-7893 (1993).
The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.
The following examples are offered for illustrative purposes only, and are not intended to limit the scope of the present invention in any way.
All patent and literature references cited in the present specification are hereby incorporated by reference in their entirety.

## EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1: Tissue Expression Profiling Using GeneExpress®

A proprietary database containing gene expression information (GeneExpress®, Gene Logic Inc., Gaithersburg, MD) was analyzed in an attempt to identify polypeptides (and their encoding nucleic acids) whose expression is significantly upregulated in a particular tumor tissue(s) of interest as compared to other tumor(s) and/or normal tissues. Specifically, analysis of the GeneExpress® database was conducted using either software available through Gene Logic Inc., Gaithersburg, MD, for use with the GeneExpress® database or with proprietary software written and developed at Genentech, Inc. for use with the GeneExpress® database. The rating of positive hits in the analysis is based upon several criteria including, for example, tissue specificity, tumor specificity and expression level in normal essential and/or normal proliferating tissues. The following is a list of molecules whose tissue expression profile as determined from an analysis of the GeneExpress® database evidences high tissue expression and significant upregulation of expression in a specific tumor or tumors as compared to other tumor(s) and/or normal tissues and optionally relatively low expression in normal essential and/or normal proliferating tissues. As such, the molecules listed below are excellent polypeptide targets for the diagnosis and therapy of cancer in mammals.

| Molecule | upregulation of expression in: | as compared to: |
|---|---|---|
| DNA227943 (TAT242) | breast tumor | normal breast tissue |
| DNA227943 (TAT242) | brain tumor | normal brain tissue |
| DNA227019 (TAT244) | breast tumor | normal breast tissue |
| DNA227109 (TAT244) | lung tumor | normal lung tissue |
| DNA227019 (TAT244) | ovarian tumor | normal ovarian tissue |
| DNA227465 (TAT241) | breast tumor | normal breast tissue |
| DNA227465 (TAT241) | lung tumor | normal lung tissue |
| DNA82306 (TAT243) | kidney tumor | normal kidney tissue |
| DNA82306 (TAT243) | lymphoid tumor | normal lymphoid tissue |
| DNA82306 (TAT243) | colon tumor | normal colon tissue |
| DNA42551 (TAT246) | breast tumor | normal breast tissue |
| DNA42551 (TAT246) | lung tumor | normal lung tissue |

(continued)

| Molecule | upregulation of expression in: | as compared to: |
|---|---|---|
| DNA42551 (TAT246) | ovarian tumor | normal ovarian tissue |
| DNA68885 (TAT135) | uterine tumor | normal uterine tissue |
| DNA68885 (TAT135) | lung tumor | normal lung tissue |
| DNA68885 (TAT135) | ovarian tumor | normal ovarian tissue |
| DNA68885 (TAT135) | pancreatic tumor | normal pancreatic tissue |
| DNA68885 (TAT135) | breast tumor | normal breast tissue |
| DNA68885 (TAT135) | cervical tumor | normal cervical tissue |
| DNA68885 (TAT135) | endometrial tumor | normal endometrial tissue |
| DNA68885 (TAT135) | stomach tumor | normal stomach tissue |
| DNA59619 (TAT249) | breast tumor | normal breast tissue |
| DNA59619 (TAT249) | esophageal tumor | normal esophageal tissue |
| DNA59619 (TAT249) | ovarian tumor | normal ovarian tissue |
| DNA59619 (TAT249) | stomach tumor | normal stomach tissue |
| DNA290812 (TAT283) | colon tumor | normal colon tissue |
| DNA290812 (TAT283) | breast tumor | normal breast tissue |
| DNA292996 (TAT286) | lung tumor | normal lung tissue |
| DNA254932 (TAT288) | breast tumor | normal breast tissue |
| DNA254932 (TAT288) | colon tumor | normal colon tissue |
| DNA254932 (TAT288) | ovarian tumor | normal ovarian tissue |
| DNA288313 (TAT289) | colon tumor | normal colon tissue |
| DNA288313 (TAT289) | ovarian tumor | normal ovarian tissue |
| DNA227583 (TAT279) | colon tumor | normal colon tissue |
| DNA227583 (TAT279) | uterus tumor | normal uterus tissue |
| DNA227708 (TAT281) | breast tumor | normal breast tissue |
| DNA227708 (TAT281) | prostate tumor | normal prostate tissue |
| DNA226859 (TAT282) | colon tumor | normal colon tissue |
| DNA194838 (TAT280) | breast tumor | normal breast tissue |
| DNA194838 (TAT280) | colon tumor | normal colon tissue |
| DNA194838 (TAT280) | rectum tumor | normal rectum tissue |
| DNA194838 (TAT280) | endometrial tumor | normal endometrial tissue |
| DNA194838 (TAT280) | kidney tumor | normal kidney tissue |
| DNA194838 (TAT280) | ovarian tumor | normal ovarian tissue |
| DNA290924 (TAT290) | breast tumor | normal breast tissue |
| DNA290924 (TAT290) | colon tumor | normal colon tissue |
| DNA290924 (TAT290) | rectum tumor | normal rectum tissue |
| DNA290924 (TAT290) | endometrial tumor | normal endometrial tissue |
| DNA290924 (TAT290) | kidney tumor | normal kidney tissue |
| DNA290924 (TAT290) | ovarian tumor | normal ovarian tissue |
| DNA299882 (TAT373) | breast tumor | normal breast tissue |
| DNA299882 (TAT373) | colon tumor | normal colon tissue |
| DNA299882 (TAT373) | rectum tumor | normal rectum tissue |
| DNA299882 (TAT373) | uterine tumor | normal uterine tissue |
| DNA299882 (TAT373) | ovarian tumor | normal ovarian tissue |
| DNA299882 (TAT373) | pancreas tumor | normal pancreas tissue |
| DNA299882 (TAT373) | bladder tumor | normal bladder tissue |
| DNA299882 (TAT373) | lung tumor | normal lung tissue |
| DNA299882 (TAT373) | kidney tumor | normal kidney tissue |
| DNA254340 (TAT287) | breast tumor | normal breast tissue |
| DNA254340 (TAT287) | colon tumor | normal colon tissue |
| DNA254340 (TAT287) | rectum tumor | normal rectum tissue |

(continued)

| Molecule | upregulation of expression in: | as compared to: |
|---|---|---|
| DNA254340 (TAT287) | uterine tumor | normal uterine tissue |
| DNA254340 (TAT287) | ovarian tumor | normal ovarian tissue |
| DNA254340 (TAT287) | pancreas tumor | normal pancreas tissue |
| DNA254340 (TAT287) | bladder tumor | normal bladder tissue |
| DNA254340 (TAT287) | lung tumor | normal lung tissue |
| DNA254340 (TAT287) | kidney tumor | normal kidney tissue |
| DNA274297 (TAT257) | glioma tumor | normal brain tissue |
| DNA274297 (TAT257) | breast tumor | normal breast tissue |
| DNA274297 (TAT257) | thyroid tumor | normal thyroid tissue |
| DNA274297-(TAT257) - | stomach tumor | normal stomach tissue |
| DNA274297 (TAT257) | kidney tumor | normal kidney tissue |
| DNA274297 (TAT257) | neuroendocrine tumor | normal neuroendocrine tissue |
| DNA274297 (TAT257) | Hodgkins lymphoma | normal associated tissues |
| DNA274297 (TAT257) | malignant lymphoma | normal associated tissues |
| DNA47369 (TAT258) | glioma tumor | normal brain tissue |
| DNA47369 (TAT258) | benign bone tumor | normal bone tissue |
| DNA226027 (TAT259) | glioma tumor | normal brain tissue |
| DNA226027 (TAT259) | giant cell bone tumor | normal bone tissue |
| DNA226027 (TAT259) | benign bone tumor | normal bone tissue |
| DNA226027 (TAT259) | metastatic bone tumor | normal bone tissue |
| DNA226027 (TAT259) | fibroma tumor | normal fibrous tissue |
| DNA226713 (TAT260) | glioma tumor | normal brain tissue |
| DNA226713 (TAT260) | benign bone tumor | normal bone tissue |
| DNA226713 (TAT260) | giant cell bone tumor | normal bone tissue |
| DNA226713 (TAT260) | Hodgkins lymphoma | normal associated tissue |
| DNA226713 (TAT260) | metastatic lymphoma | normal associated tissue |
| DNA86517 (TAT261) | glioma tumor | normal brain tissue |
| DNA88126 (TAT262) | glioma tumor | normal brain tissue |
| DNA103464 (TAT263) | glioma tumor | normal brain tissue |
| DNA194776 (TAT264) | glioma tumor | normal brain tissue |
| DNA194776 (TAT264) | Wilm’s tumor | normal associated tissue |
| DNA194776 (TAT264) | metastatic kidney tumor | normal kidney tissue |
| DNA194776 (TAT264) | soft tissue tumors | normal soft tissues |
| DNA288204 (TAT265) | glioma tumor | normal brain tissue |
| DNA288204 (TAT265) | soft tissue tumors | normal soft tissues |
| DNA288204 (TAT265) | white blood cells from Wegner’s granulomatosis | normal white blood cells |
| DNA257354 (TAT266) | glioma tumor | normal brain tissue |
| DNA257354 (TAT266) | metastatic ovarian tumor | normal ovarian tissue |
| DNA98566 (TAT267) | glioma tumor | normal brain tissue |
| DNA227212 (TAT268) | glioma tumor | normal brain tissue |
| DNA227212 (TAT268) | breast tumor | normal breast tissue |
| DNA227212 (TAT268) | uterus tumor | normal uterus tissu |
| DNA227461 (TAT269) | glioma tumor | normal brain tissue |
| DNA150762 (TAT270) | glioma tumor | normal brain tissue |
| DNA150762 (TAT270) | kidney tumor | normal kidney tissue |
| DNA150762 (TAT270) | head and neck tumor | normal head and neck tissue |
| DNA150762 (TAT270) | soft tissue tumors | normal soft tissues |
| DNA150762 (TAT270) | breast tumor | normal breast tissue |
| DNA150762 (TAT270) | chronic myeloid leukemia | normal myeloid tissue |
| DNA150762 (TAT270) | Hodgkin’s lymphoma | normal associated tissue |

(continued)

| Molecule | upregulation of expression in: | as compared to: |
|---|---|---|
| DNA150762 (TAT270) | malignant lymphoma | normal associated tissue |
| DNA86382 (TAT271) | glioma tumor | normal brain tissue |
| DNA86382 (TAT271) | white blood cells from Wegner's granulomatosis | normal white blood cells |
| DNA256608 (TAT272) | glioma tumor | normal brain tissue |
| DNA256608 (TAT272) | kidney tumor | normal kidney tissue |
| DNA256608 (TAT272) | neuroendocrine tumor | normal neuroendocrine tissue |
| DNA19902 (TAT273) | glioma tumor | normal brain tissue |
| DNA19902 (TAT273) | colorectal tumor | normal colorectal tissue |
| DNA182764 (TAT274) | glioma tumor | normal brain tissue |
| DNA182764 (TAT274) | ovary tumor | normal ovary tissue |
| DNA225727 (TAT275) | glioma tumor | normal brain tissue |
| DNA119500 (TAT276) | glioma tumor | normal brain tissue |
| DNA19362 (TAT277) | glioma tumor | normal brain tissue |
| DNA19362 (TAT277) | skin tumor | normal skin tissue |
| DNA19362 (TAT277) | bone tumor | normal bone tissue |
| DNA19362 (TAT277) | kidney tumor | normal kidney tissue |
| DNA19362 (TAT277) | soft tissue tumors | normal soft tissues |

EXAMPLE 2: Microarray Analysis to Detect Upregulation of TAT Polypeptides in Cancerous Tumors

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (disease tissue) sample is greater than hybridization signal of a probe from a control (normal tissue) sample, the gene or genes overexpressed in the disease tissue are identified. The implication of this result is that an overexpressed protein in a diseased tissue is useful not only as a diagnostic marker for the presence of the disease condition, but also as a therapeutic target for treatment of the disease condition.

The methodology of hybridization of nucleic acids and microarray technology is well known in the art. In one example, the specific preparation of nucleic acids for hybridization and probes, slides, and hybridization conditions are all detailed inPCT Patent Application SerialNo. PCT/US01/10482, filed on March 30, 2001 and which is herein incorporated by reference.

In the present example, cancerous tumors derived from various human tissues were studied for upregulated gene expression relative to cancerous tumors from different tissue types and/or non-cancerous human tissues in an attempt to identify those polypeptides which are overexpressed in a particular cancerous tumor(s).

In certain experiments, cancerous human tumor tissue and non-cancerous human tumor tissue of the same tissue type (often from the same patient) were obtained and analyzed for TAT polypeptide expression. Additionally, cancerous human tumor tissue from any of a variety of different human tumors was obtained and compared to a "universal" epithelial control sample which was prepared by pooling non-cancerous human tissues of epithelial origin, including liver, kidney, and lung. mRNA isolated from the pooled tissues represents a mixture of expressed gene products from these different tissues. Microarray hybridization experiments using the pooled control samples generated a linear plot in a 2-color analysis. The slope of the line generated in a 2-color analysis was then used to normalize the ratios of (test:control detection) within each experiment. The normalized ratios from various experiments were then compared and used to identify clustering of gene expression. Thus, the pooled "universal control" sample not only allowed effective relative gene expression determinations in a simple 2-sample comparison, it also allowed multi-sample comparisons across several experiments.

In the present experiments, nucleic acid probes derived from the herein described TAT polypeptide-encoding nucleic acid sequences were used in the creation of the microarray and RNA from various tumor tissues were used for the hybridization thereto. Below is shown the results of these experiments, demonstrating that various TAT polypeptides of the present invention are significantly overexpressed in various human tumor tissues as compared to their normal counterpart tissue(s). Moreover, all of the molecules shown below are significantly overexpressed in their specific tumor tissue(s) as compared to in the "universal" epithelial control. As described above, these data demonstrate that the TAT polypeptides of the present invention are useful not only as diagnostic markers for the presence of one or more cancerous tumors, but also serve as therapeutic targets for the treatment of those tumors.

| Molecule | upregulation of expression in: | as compared to: |
|---|---|---|
| DNA68885 (TAT135) | breast tumor | normal breast tissue |
| DNA68885 (TAT135) | rectum tumor | normal rectum tissue |
| DNA68885 (TAT135) | lung tumor | normal lung tissue |
| DNA68885 (TAT135) | ovarian tumor | normal ovarian tissue |
| DNA274297 (TAT257) | glioma tumor | normal glial tissue |
| DNA47369 (TAT258) | glioma tumor | normal glial tissue |
| DNA226027 (TAT259) | glioma tumor | normal glial tissue |
| DNA226713 (TAT260) | glioma tumor | normal glial tissue |
| DNA86517 (TAT261) | glioma tumor | normal glial tissue |
| DNA88126 (TAT262) | glioma tumor | normal glial tissue |
| DNA103464 (TAT263) | glioma tumor | normal glial tissue |
| DNA194776 (TAT264) | glioma tumor | normal glial tissue |
| DNA288204 (TAT265) | glioma tumor | normal glial tissue |
| DNA257354 (TAT266) | glioma tumor | normal glial tissue |
| DNA98566 (TAT267) | glioma tumor | normal glial tissue |
| DNA227212 (TAT268) | glioma tumor | normal glial tissue |
| DNA227461 (TAT269) | glioma tumor | normal glial tissue |
| DNA150762 (TAT270) | glioma tumor | normal glial tissue |
| DNA86382 (TAT271) | glioma tumor | normal glial tissue |
| DNA256608 (TAT272) | glioma tumor | normal glial tissue |
| DNA19902 (TAT273) | glioma tumor | normal glial tissue |
| DNA19902 (TAT273) | colorectal tumor | normal colorectal tissue |
| DNA182764 (TAT274) | glioma tumor | normal glial tissue |
| DNA119500 (TAT276) | glioma tumor | normal glial tissue |
| DNA19362 (TAT277) | glioma tumor | normal glial tissue |
| DNA226446 (TAT278) | glioma tumor | normal glial tissue |

EXAMPLE 3: Ouantitative Analysis of TAT mRNA Expression

In this assay, a 5' nuclease assay (for example, TaqMan®) and real-time quantitative PCR (for example, ABIPrizm 7700 Sequence Detection System® (Perkin Elmer, Applied Biosystems Division, Foster City, CA)), were used to find genes that are significantly overexpressed in a cancerous tumor or tumors as compared to other cancerous tumors or normal non-cancerous tissue. The 5' nuclease assay reaction is a fluorescent PCR-based technique which makes use of the 5' exonuclease activity of Taq DNA polymerase enzyme to monitor gene expression in real time. Two oligonucleotide primers (whose sequences are based upon the gene or EST sequence of interest) are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the PCR amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.
The 5' nuclease procedure is run on a real-time quantitative PCR device such as the ABI Prism 7700TM Sequence Detection. The system consists of a thermocycler, laser, charge-coupled device (CCD) camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

The starting material for the screen was mRNA isolated from a variety of different cancerous tissues. The mRNA is quantitated precisely, e.g., fluorometrically. As a negative control, RNA was isolated from various normal tissues of the same tissue type as the cancerous tissues being tested.
5' nuclease assay data are initially expressed as Ct, or the threshold cycle. This is defined as the cycle at which the reporter signal accumulates above the background level of fluorescence. The ΔCt values are used as quantitative measurement of the relative number of starting copies of a particular target sequence in a nucleic acid sample when comparing cancer mRNA results to normal human mRNA results. As one Ct unit corresponds to 1 PCR cycle or approximately a 2-fold relative increase relative to normal, two units corresponds to a 4-fold relative increase, 3 units corresponds to an 8-fold relative increase and so on, one can quantitatively measure the relative fold increase in mRNA expression between two or more different tissues. Using this technique, the molecules listed below have been identified as being significantly overexpressed in a particular tumor(s) as compared to their normal non-cancerous counterpart tissue(s) (from both the same and different tissue donors) and thus, represent excellent polypeptide targets for the diagnosis and therapy of cancer in mammals.

| Molecule | upregulation of expression in: | as compared to: |
|---|---|---|
| DNA227943 (TAT242) | breast tumor | matched normal breast tissue |
| DNA175959 (TAT251) | ovary tumor | matched normal ovary tissue |
| DNA59612 (TAT253) | ovary tumor | matched normal ovary tissue |
| DNA227465 (TAT241) | breast tumor | matched normal breast tissue |
| DNA82306 (TAT243) | kidney tumor | matched normal kidney tissue |
| DNA42551 (TAT246) | ovarian tumor | matched normal ovarian tissue |
| DNA68885 (TAT135) | ovarian tumor | matched normal ovarian tissue |
| DNA59619 (TAT249) | breast tumor | matched normal breast tissue |
| DNA288313 (TAT289) | ovarian tumor | matched normal ovarian tissue |
| DNA194838 (TAT280) | kidney tumor | matched normal kidney tissue |
| DNA194838 (TAT280) | colon tumor | matched normal colon tissue |
| DNA290924 (TAT290) | kidney tumor | matched normal kidney tissue |
| DNA290924 (TAT290) | colon tumor | matched normal colon tissue |
| DNA254340 (TAT287) | breast tumor | matched normal breast tissue |
| DNA299882 (TAT373) | breast tumor | matched normal breast tissue |
| DNA274297 (TAT257) | glioma tumor | normal brain tissue |
| DNA226027 (TAT259) | glioma tumor | normal brain tissue |
| DNA194776 (TAT264) | glioma tumor | normal brain tissue |
| DNA288204 (TAT265) | glioma tumor | normal brain tissue |
| DNA257354 (TAT266) | glioma tumor | normal brain tissue |
| DNA98566 (TAT267) | glioma tumor | normal brain tissue |
| DNA227212 (TAT268) | glioma tumor | normal brain tissue |
| DNA150762 (TAT270) | glioma tumor | normal brain tissue |
| DNA86382 (TAT271) | glioma tumor | normal brain tissue |
| DNA182764 (TAT274) | glioma tumor | normal brain tissue |
| DNA19362 (TAT277) | glioma tumor | normal brain tissue |

EXAMPLE 4: *In situ* Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis and aid in chromosome mapping.
*In situ* hybridization was performed following an optimized version of the protocol by Lu and Gillett, Cell Vision 1:169-176 (1994), using PCR generated $^{33}$P-labeled riboprobes. Briefly, formalin-fixed,paraffin-embedded human tissues were sectioned, deparaffinized, deproteinated in proteinase K (20 g/ml) for 15 minutes at 37°C, and further processed for *in situ* hybridization as described by Lu and Gillett, *supra*. A [$^{33}$-P] UTP-labeled antisense riboprobe was generated from a PCR product and hybridized at 55 °C overnight. The slides were dipped in Kodak NTB2 nuclear track emulsion and exposed for 4 weeks.

$^{33}$P-Riboprobe synthesis

6.0 μl (125 mCi) of $^{33}$P-UTP (Amersham BF 1002, SA<2000 Ci/mmol) were speed vac dried. To each tube containing dried $^{33}$P-UTP, the following ingredients were added:

2.0 μl 5x transcription buffer
1.0 μl DTT (100 mM)
2.0 μl NTP mix (2.5 mM : 10 μ; each of 10 mM GTP, CTP & ATP + 10 μl $H_2O$)
1.0 μl UTP (50 μM)
1.0 μl Rnasin
1.0 μl DNA template (1μg)
1.0 μl $H_2O$
1.0 μl RNA polymerase (for PCR products T3 = AS, T7 = S, usually)

The tubes were incubated at 37°C for one hour. 1.0 μl RQ1 DNase were added, followed by incubation at 37°C for 15 minutes. 90 μl TE (10 mM Tris pH 7.6/1mM EDTA pH 8.0) were added, and the mixture was pipetted onto DE81 paper. The remaining solution was loaded in a Microcon-50 ultrafiltration unit, and spun using program 10 (6 minutes). The filtration unit was inverted over a second tube and spun using program 2 (3 minutes). After the final recovery spin, 100 μl TE were added. 1 μl of the final product was pipetted on DE81 paper and counted in 6 ml ofBiofluor II.
The probe was run on a TBE/urea gel. 1-3 μl of the probe or 5 μl of RNA Mrk will were added to 3 ll of loading buffer. After heating on a 95°C heat block for three minutes, the probe was immediately placed on ice. The wells of gel were flushed, the sample loaded, and run at 180-250 volts for 45 minutes. The gel was wrapped in saran wrap and exposed to XAR film with an intensifying screen in -70°C freezer one hour to overnight $^{33}$P-Hybridization

A. Pretreatment of frozen sections

The slides were removed from the freezer, placed on aluminium trays and thawed at room temperature for 5 minutes. The trays were placed in 55°C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ $H_2O$). After deproteination in 0.5 μg/ml proteinase K for 10 minutes at 37°C (12.5 μl of 10 mg/ml stock in 250 ml prewarmed RNase-free RNAse buffer), the sections were washed in 0.5 x SSC for 10 minutes at room temperature. The sections were dehydrated in 70%, 95%, 100% ethanol, 2 minutes each.

B. Pretreatment of paraffin-embedded sections

The slides were deparaffinized, placed in SQ $H_2O$, and rinsed twice in 2 x SSC at room temperature, for 5 minutes each time. The sections were deproteinated in 20 μg/ml proteinase K (500 μl of 10 mg/ml in 250 ml RNase-free RNase buffer; 37 °C,15 minutes) - human embryo, or 8 x proteinase K (100 μl in 250 ml Rnase buffer, 37 °C, 30 minutes) - formalin tissues. Subsequent rinsing in 0.5 x SSC and dehydration were performed as described above.

C. Prehybridization

The slides were laid out in a plastic box lined with Box buffer (4 x SSC, 50% formamide) - saturated filter paper.

D. Hybridization

1.0 x $10^6$ cpm probe and 1.0 μl tRNA (50 mg/ml stock) per slide were heated at 95 °C for 3 minutes. The slides were cooled on ice, and 48 μl hybridization buffer were added per slide. After vortexing, 50 μl $^{33}$P mix were added to 50 μl prehybridization on slide. The slides were incubated overnight at 55 °C.

E. Washes

Washing was done 2 x 10 minutes with 2xSSC, EDTA at room temperature (400 ml 20 x SSC+ 16 ml 0.25M EDTA, $V_f$=4L), followed by RNaseA treatment at 37°C for 30 minutes (500 μl of 10 mg/ml in 250 ml Rnase buffer = 20 μg/ml), The slides were washed 2 x 10 minutes with 2 x SSC, EDTA at room temperature. The stringency wash conditions were as follows: 2 hours at 55°C, 0.1 x SSC, EDTA (20 ml 20 x SSC + 16 ml EDTA, $V_f$=4L).

F. Oligonucleotides

*In situ* analysis was performed on a variety of DNA sequences disclosed herein. The oligonucleotides employed for these analyses were obtained so as to be complementary to the nucleic acids (or the complements thereof) as shown in the accompanying figures.

G. Results

*In situ* analysis was performed on a variety of DNA sequences disclosed herein. The results from these analyses are as follows.

(1) DNA42551 (TAT246)

With regard to normal tissues, very weak expression is observed in the epithelial cells of submucosal bronchial glands, breast, gall bladder and prostate; the latter is inconsistent. No other normal tissues tested were positive for expression.
In one analysis, strong expression is observed in 12 of 15 ovarian carcinomas. In uterine adenocarcinomas, positive expression is observed in 4 of 8 samples.
In another analysis, weak to moderate expression is observed in 3 of 16 non-small cell lung carcinomas. Positive expression is also observed in 2 of 14 colorectal adenocarcinomas, 5 of 8 gastric adenocarcinomas, 3 of 4 esophageal carcinomas (2 adeno- and 1 squamous cell carcinoma) and 1 of 3 pancreatic ductal adenocarcinomas.

(2) DNA68885 (TAT135)

Expression of moderate intensity is seen in gastrointestinal mucosa. In colon and small intestine expression appears throughout the lining epithelium. In stomach expression appears concentrated in the foveolar epithelium, chief and parietal cells are negative. A weak to moderate signal is detected in two cores of kidney, it localizes to cells of the macula densa.
Expression is also observed in 11 of 15 ovarian carcinomas (surface epithelial and adenocarcinoma) and one case of Brenner tumor. Expression is also seen in 6 of 8 uterine adenocarcinomas, including one MIVIMT (Malignant Mixed Muellerian Tumor). Expression is also observed in normal bronchial mucosa, wherein the level of expression ranges from very weak to moderate. Strong expression is observed in 10 of 16 non-small cell lung carcinomas. Expression is also seen in the following malignant neoplasms: 19 of 19 colorectal adenocarcinomas, 8 of 9 gastric adenocarcinomas, 2 of 2 pancreatic adenocarcinomas, 2 of 4 esophageal carcinomas and 11 of 11 metastatic adenocarcinomas.

(3) DNA59619 (TAT249)

None of the normal tissues analyzed show a positive signal.
With regard to carcinoma samples, 22 of 86 cases of invasive ductal breast cancer are positive for expression.

(4) DNA288313 (TAT289)

A signal of moderate intensity is seen in 2 of 3 ovarian carcinomas, whereas no positive signal is observed in normal ovarian tissue.

(5) DNA194838 (TAT280)

Three of 4 renal cell carcinomas show a positive signal; wherein the three positive cases are classical clear cell carcinomas. There is no positive signal observed in any of the normal benign kidney tissue analyzed (cortex or medulla).

(6) DNA290924 (TAT290)

Three of 4 renal cell carcinomas show a positive signal; wherein the three positive cases are classical clear cell carcinomas. There is no positive signal observed in any of the normal benign kidney tissue analyzed (cortex or medulla).

EXAMPLE 5: Verification and Analysis of Differential TAT Polypeptide Expression by GEPIS

TAT polypeptides which may have been identified as a tumor antigen as described in one or more of the above Examples were analyzed and verified as follows. An expressed sequence tag (EST) DNA database (LIFESEQ®, Incyte Pharmaceuticals, Palo Alto, CA) was searched and interesting EST sequences were identified by GEPIS. Gene expression profiling *in silico* (GEPIS) is a bioinformatics tool developed at Genentech, Inc. that characterizes genes of interest for new cancer therapeutic targets. GEPIS takes advantage of large amounts of EST sequence and library information to determine gene expression profiles. GEPIS is capable of determining the expression profile of a gene based upon its proportional correlation with the number of its occurrences in EST databases, and it works by integrating the LIFESEQ® EST relational database and Genentech proprietary information in a stringent and statistically meaningful way. In this example, GEPIS is used to identify and cross-validate novel tumor antigens, although GEPIS can be configured to perform either very specific analyses or broad screening tasks. For the initial screen, GEPIS is used to identify EST sequences from the LIFESEQ® database that correlate to expression in a particular tissue or tissues of interest (often a tumor tissue of interest). The EST sequences identified in this initial screen (or consensus sequences obtained from aligning multiple related and overlapping EST sequences obtained from the initial screen) were then subjected to a screen intended to identify the presence of at least one transmembrane domain in the encoded protein. Finally, GEPIS was employed to generate a complete tissue expression profile for the various sequences of interest. Using this type of screening bioinformatics, various TAT polypeptides (and their encoding nucleic acid molecules) were identified as being significantly overexpressed in a particular type of cancer or certain cancers as compared to other cancers and/or normal non-cancerous tissues. The rating of GEPIS hits is based upon several criteria including, for example, tissue specificity, tumor specificity and expression level in normal essential and/or normal proliferating tissues. The following is a list of molecules whose tissue expression profile as determined by GEPIS evidences high tissue expression and significant upregulation of expression in a specific tumor or tumors as compared to other tumor(s) and/or normal tissues and optionally relatively low expression in normal essential and/or normal proliferating tissues. As such, the molecules listed below are excellent polypeptide targets for the diagnosis and therapy of cancer in mammals.

| Molecule | upregulation of expression in: | as compared to: |
|---|---|---|
| DNA172363 (TAT240) | bladder tumor | normal bladder tissue |
| DNA172363 (TAT240) | brain tumor | normal brain tissue |
| DNA172363 (TAT240) | breast tumor | normal breast tissue |
| DNA227943 (TAT242) | brain tumor | normal brain tissue |
| DNA227943 (TAT242) | breast tumor | normal breast tissue |
| DNA227943 (TAT242) | prostate tumor | normal prostate tissue |
| DNA227943 (TAT242) | kidney tumor | normal kidney tissue |
| DNA227943 (TAT242) | uterus tumor | normal uterus tissue |
| DNA227019 (TAT244) | lung tumor | normal lung tissue |
| DNA227019 (TAT244) | breast tumor | normal breast tissue |
| DNA227019 (TAT244) | prostate tumor | normal prostate tissue |
| DNA227019 (TAT244) | uterus tumor | normal uterus tissue |
| DNA96942 (TAT245) | brain tumor | normal brain tissue |
| DNA96942 (TAT245) | lung tumor | normal lung tissue |
| DNA96942 (TAT245) | uterus tumor | normal uterus tissue |
| DNA96942 (TAT245) | colon tumor | normal colon tissue |
| DNA96942 (TAT245) | breast tumor | normal breast tissue |
| DNA59619 (TAT249) | brain tumor | normal brain tissue |
| DNA59619 (TAT249) | breast tumor | normal breast tissue |
| DNA59619 (TAT249) | prostate tumor | normal prostate tissue |
| DNA227205 (TAT250) | colon tumor | normal colon tissue |
| DNA227205 (TAT250) | breast tumor | normal breast tissue |
| DNA227205 (TAT250) | uterus tumor | normal uterus tissue |
| DNA227205 (TAT250) | prostate tumor | normal prostate tissue |
| DNA227205 (TAT250) | lung tumor | normal lung tissue |
| DNA175959 (TAT251) | prostate tumor | normal prostate tissue |
| DNA175959 (TAT251) | uterus tumor | normal uterus tissue |

(continued)

| Molecule | upregulation of expression in: | as compared to: |
|---|---|---|
| DNA175959 (TAT251) | fallopian tube tumor | normal fallopian tube tissue |
| DNA175959 (TAT251) | colon tumor | normal colon tissue |
| DNA175959 (TAT251) | ovary tumor | normal ovary tissue |
| DNA48227 (TAT252) | colon tumor | normal colon tissue |
| DNA48227 (TAT252) | breast tumor | normal breast tissue |
| DNA48227 (TAT252) | lung tumor | normal lung tissue |
| DNA59612 (TAT253) | prostate tumor | normal prostate tissue |
| DNA59612 (TAT253) | lung tumor | normal lung tissue |
| DNA59612 (TAT253) | fallopian tube tumor | normal fallopian tube tissue |
| DNA59612 (TAT253) | uterus tumor | normal uterus tissue |
| DNA59612 (TAT253) | breast tumor | normal breast tissue |
| DNA226917 (TAT254) | ovary tumor | normal ovary tissue |
| DNA226917 (TAT254) | prostate tumor | normal prostate tissue |
| DNA226917 (TAT254) | colon tumor | normal colon tissue |
| DNA125219 (TAT255) | breast tumor | normal breast tissue |
| DNA125219 (TAT255) | colon tumor | normal colon tissue |
| DNA125219 (TAT255) | lung tumor | normal lung tissue |
| DNA151291 (TAT256) | breast tumor | normal breast tissue |
| DNA151291 (TAT256) | colon tumor | normal colon tissue |
| DNA151291 (TAT256) | lung tumor | normal lung tissue |
| DNA151291 (TAT256) | ovary tumor | normal ovary tissue |
| DNA227465 (TAT241) | lung tumor | normal lung tissue |
| DNA227465 (TAT241) | uterus tumor | normal uterus tissue |
| DNA82306 (TAT243) | kidney tumor | normal kidney tissue |
| DNA82306 (TAT243) | stomach tumor | normal stomach tissue |
| DNA82306 (TAT243) | breast tumor | normal breast tissue |
| DNA42551 (TAT246) | myeloid tumor | normal myeloid tissue |
| DNA42551 (TAT246) | prostate tumor | normal prostate tissue |
| DNA42551 (TAT246) | lung tumor | normal lung tissue |
| DNA42551 (TAT246) | colon tumor | normal colon tissue |
| DNA42551 (TAT246) | stomach tumor | normal stomach tissue |
| DNA68885 (TAT135) | ovarian tumor | normal ovarian tissue |
| DNA68885 (TAT135) | pancreatic tumor | normal pancreatic tissue |
| DNA68885 (TAT135) | kidney tumor | normal kidney tissue |
| DNA68885 (TAT135) | prostate tumor | normal prostate tissue |
| DNA68885 (TAT135) | uterine tumor | normal uterine tissue |
| DNA59619 (TAT249) | breast tumor | normal breast tissue |
| DNA59619 (TAT249) | ovarian tumor | normal ovarian tissue |
| DNA59619 (TAT249) | pancreatic tumor | normal pancreatic tissue |
| DNA290812 (TAT283) | colon tumor | normal colon tissue |
| DNA290812 (TAT283) | breast tumor | normal breast tissue |
| DNA292996 (TAT286) | lung tumor | normal lung tissue |
| DNA254932 (TAT288) | breast tumor | normal breast tissue |
| DNA254932 (TAT288) | colon tumor | normal colon tissue |
| DNA254932 (TAT288) | ovarian tumor | normal ovarian tissue |
| DNA288313 (TAT289) | colon tumor | normal colon tissue |
| DNA288313 (TAT289) | ovarian tumor | normal ovarian tissue |
| DNA227583 (TAT279) | colon tumor | normal colon tissue |
| DNA227583 (TAT279) | uterus tumor | normal uterus tissue |
| DNA227708 (TAT281) | breast tumor | normal breast tissue |

(continued)

| Molecule | upregulation of expression in: | as compared to: |
|---|---|---|
| DNA227708 (TAT281) | prostate tumor | normal prostate tissue |
| DNA226859 (TAT282) | colon tumor | normal colon tissue |
| DNA194838 (TAT280) | kidney tumor | normal kidney tissue |
| DNA194838 (TAT280) | stomach tumor | normal stomach tissue |
| DNA194838 (TAT280) | esophageal tumor | normal esophageal tissue |
| DNA290924 (TAT290) | kidney tumor | normal kidney tissue |
| DNA290924 (TAT290) | stomach tumor | normal stomach tissue |
| DNA290924 (TAT290) | esophageal tumor | normal esophageal tissue |
| DNA299882 (TAT373) | uterine tumor | normal uterine tissue |
| DNA299882 (TAT373) | ovarian tumor | normal ovarian tissue |
| DNA299882 (TAT373) | pancreas tumor | normal pancreas tissue |
| DNA299882 (TAT373) | bladder tumor | normal bladder tissue |
| DNA299882 (TAT373) | lung tumor | normal lung tissue |
| DNA299882 (TAT373) | kidney tumor | normal kidney tissue |
| DNA254340 (TAT287) | uterine tumor | normal uterine tissue |
| DNA254340 (TAT287) | ovarian tumor | normal ovarian tissue |
| DNA254340 (TAT287) | pancreas tumor | normal pancreas tissue |
| DNA254340 (TAT287) | bladder tumor | normal bladder tissue |
| DNA254340 (TAT287) | lung tumor | normal lung tissue |
| DNA254340 (TAT287) | kidney tumor | normal kidney tissue |
| DNA274297 (TAT257) | glioma tumor | normal glial tissue |
| DNA47369 (TAT258) | glioma tumor | normal glial tissue |
| DNA226027 (TAT259) | glioma tumor | normal glial tissue |
| DNA226713 (TAT260) | glioma tumor | normal glial tissue |
| DNA86517 (TAT261) | glioma tumor | normal glial tissue |
| DNA88126 (TAT262) | glioma tumor | normal glial tissue |
| DNA103464 (TAT263) | glioma tumor | normal glial tissue |
| DNA194776 (TAT264) | glioma tumor | normal glial tissue |
| DNA288204 (TAT265) | glioma tumor | normal glial tissue |
| DNA257354 (TAT266) | glioma tumor | normal glial tissue |
| DNA98566 (TAT267) | glioma tumor | normal glial tissue |
| DNA227212 (TAT268) | glioma tumor | normal glial tissue |
| DNA227461 (TAT269) | glioma tumor | normal glial tissue |
| DNA150762 (TAT270) | glioma tumor | normal glial tissue |
| DNA86382 (TAT271) | glioma tumor | normal glial tissue |
| DNA256608 (TAT272) | glioma tumor | normal glial tissue |
| DNA19902 (TAT273) | glioma tumor | normal glial tissue |
| DNA182764 (TAT274) | glioma tumor | normal glial tissue |
| DNA119500 (TAT276) | glioma tumor | normal glial tissue |
| DNA19362 (TAT277) | glioma tumor | normal glial tissue |
| DNA226446 (TAT278) | glioma tumor | normal glial tissue |
| (NOTE: gene is located on same amplicon as HER-2 gene which is also overexpressed in breast tumors) | | |

EXAMPLE 6: Use of TAT as a hybridization probe

The following method describes use of a nucleotide sequence encoding TAT as a hybridization probe for, i.e., diagnosis of the presence of a tumor in a mammal.
DNA comprising the coding sequence of full-length or mature TAT as disclosed herein can also be employed as a probe to screen for homologous DNAs (such as those encoding naturally-occurring variants of TAT) in human tissue cDNA libraries or human tissue genomic libraries.

Hybridization and washing of filters containing either library DNAs is performed under the following high stringency conditions. Hybridization of radiolabeled TAT-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence TAT can then be identified using standard techniques known in the art.

EXAMPLE 7: Expression of TAT in *E. coli*

This example illustrates preparation of an unglycosylated form of TAT by recombinant expression in *E. coli.* The DNA sequence encoding TAT is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli*; see Bolivar et al., Gene, 2: 95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the TAT coding region, lambda transcriptional terminator, and an argU gene.

The ligation mixture is then used to transform a selected *E. coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized TAT protein can then be purified using a metal chelating column under conditions that allow tight binding of the protein.

TAT may be expressed in *E. coli* in a poly-His tagged form, using the following procedure. The DNA encoding TAT is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) lon galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g $(NH_4)_2SO_4$, 0.71 g sodium citrate•2H2O, 1.07 g KCl, 5.36 g Difco yeast extract, 5.36 g Sheffield hycase SF in 500 mL water, as well as 110 mM MPOS, pH 7.3, 0.55% (w/v) glucose and 7 mM $MgSO_4$) and grown for approximately 20-30 hours at 30 °C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1% TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.
Fractions containing the desired folded TAT polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.
Certain of the TAT polypeptides disclosed herein have been successfully expressed and purified using this technique(s).

EXAMPLE 8: Expression of TAT in mammalian cells

This example illustrates preparation of a potentially glycosylated form of TAT by recombinant expression in mammalian cells.
The vector, pRK5 (see EP 307,247, published March 15, 1989), is employed as the expression vector. Optionally, the TAT DNA is ligated into pRK5 with selected restriction enzymes to allow insertion of the TAT DNA using ligation methods such as described in Sambrook et al., supra. The resulting vector is called pRK5-TAT.
In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 μg pRK5-TAT DNA is mixed with about 1 μg DNA encoding the VA RNA gene [Thimmappaya et al., Cell, 31:543 (1982)] and dissolved in 500 μl of 1 mM Tris-HCl, 0.1 mM EDTA, 0.227 M $CaCl_2$. To this mixture is added, dropwise, 500 μl of 50 mM HEPES (pH 7.35), 280 mM NaCl, 1.5 mM $NaPO_4$, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.
Approximately 24 hours after the transfections, the culture medium is removed and replaced with culture medium (alone) or culture medium containing 200 μCi/ml $^{35}$S-cysteine and 200 μCi/ml $^{35}$S-methonine. After a 12 hour incubation, the conditioned medium is collected, concentrated on a spin filter, and loaded onto a 15% SDS gel. The processed gel may be dried and exposed to film for a selected period of time to reveal the presence of TAT polypeptide. The cultures containing transfected cells may undergo further incubation (in serum free medium) and the medium is tested in selected bioassays.
In an alternative technique, TAT may be introduced into 293 cells transiently using the dextran sulfate method described by Somparyrac et al., Proc. Natl. Acad. Sci., 12:7575 (1981). 293 cells are grown to maximal density in a spinner flask and 700 μg pRK5-TAT DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and reintroduced into the spinner flask containing tissue culture medium, 5 μg/ml bovine insulin and 0.1 μg/ml bovine transferrin. After about four days, the conditioned media is centrifuged and filtered to remove cells and debris. The sample containing expressed TAT can then be concentrated and purified by any selected method, such as dialysis and/or column chromatography.
In another embodiment, TAT can be expressed in CHO cells. The pRK5-TAT can be transfected into CHO cells using known reagents such as $CaPO_4$ or DEAE-dextran. As described above, the cell cultures can be incubated, and the medium replaced with culture medium (alone) or medium containing a radiolabel such as $^{35}$S-methionine. After determining the presence of TAT polypeptide, the culture medium may be replaced with serum free medium. Preferably, the cultures are incubated for about 6 days, and then the conditioned medium is harvested. The medium containing the expressed TAT can then be concentrated and purified by any selected method.
Epitope-tagged TAT may also be expressed in host CHO cells. The TAT may be subcloned out of the pRK5 vector. The subclone insert can undergo PCR to fuse in frame with a selected epitope tag such as a poly-his tag into a Baculovirus expression vector. The poly-his tagged TAT insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged TAT can then be concentrated and purified by any selected method, such as by $Ni^{2+}$-chelate affinity chromatography.
TAT may also be expressed in CHO and/or COS cells by a transient expression procedure or in CHO cells by another stable expression procedure.
Stable expression in CHO cells is performed using the following procedure. The proteins are expressed as an IgG construct (immunoadhesin), in which the coding sequences for the soluble forms (e.g. extracellular domains) of the respective proteins are fused to an IgG1 constant region sequence containing the hinge, CH2 and CH2 domains and/or is a poly-His tagged form.
Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.
Twelve micrograms of the desired plasmid DNA is introduced into approximately 10 million CHO cells using commercially available transfection reagents Superfect® (Quiagen), Dosper® or Fugene® (Boehringer Mannheim). The cells are grown as described in Lucas et al., supra. Approximately 3 x $10^7$ cells are frozen in an ampule for further growth and production as described below.
The ampules containing the plasmid DNA are thawed by placement into water bath and mixed by vortexing. The contents are pipetted into a centrifuge tube containing 10 mLs of media and centrifuged at 1000 rpm for 5 minutes. The supernatant is aspirated and the cells are resuspended in 10 mL of selective media (0.2 μm filtered PS20 with 5% 0.2 μm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x $10^5$ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x $10^6$ cells/mL. On day 0, the cell number pH ie determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Coming 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 μm filter. The filtrate was either stored at 4°C or immediately loaded onto columns for purification.
For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.
Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 μL of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.
Certain of the TAT polypeptides disclosed herein have been successfully expressed and purified using this technique(s).

EXAMPLE 9: Expression of TAT in Yeast

The following method describes recombinant expression of TAT in yeast.
First, yeast expression vectors are constructed for intracellular production or secretion of TAT from the ADH2/GAPDH promoter. DNA encoding TAT and the promoter is inserted into suitable restriction enzyme sites in the selected plasmid to direct intracellular expression of TAT. For secretion, DNA encoding TAT can be cloned into the selected plasmid, together with DNA encoding the ADH2/GAPDH promoter, a native TAT signal peptide or other mammalian signal peptide, or, for example, a yeast alpha-factor or invertase secretory signal/leader sequence, and linker sequences (if needed) for expression of TAT.
Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.
Recombinant TAT can subsequently be isolated and purified by removing the yeast cells from the fermentation medium by centrifugation and then concentrating the medium using selected cartridge filters. The concentrate containing TAT may further be purified using selected column chromatography resins.

Certain of the TAT polypeptides disclosed herein have been successfully expressed and purified using this technique(s).

EXAMPLE 10: Expression of TAT in Baculovirus-Infected Insect Cells

The following method describes recombinant expression of TAT in Baculovirus-infected insect cells.
The sequence coding for TAT is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding TAT or the desired portion of the coding sequence of TAT such as the sequence encoding an extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.
Recombinant baculovirus is generated by co-transfecting the above plasmid and BaculoGold™ virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).
Expressed poly-his tagged TAT can then be purified, for example, by $Ni^{2+}$-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM $MgCl_2$; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 $\mu$m filter. A $Ni^{2+}$-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline $A_{280}$ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching $A_{280}$ baseline again, the column is developed with a 0 to 500 mM Imidazole gradient in the secondary wash buffer. One mL fractions are collected and analyzed by SDS-PAGE and silver staining or Western blot with $Ni^{2+}$-NTA-conjugated to alkaline phosphatase (Qiagen). Fractions containing the eluted $His_{10}$-tagged TAT are pooled and dialyzed against loading buffer.
Alternatively, purification of the IgG tagged (or Fc tagged) TAT can be performed using known chromatography techniques, including for instance, Protein A or protein G column chromatography.
Certain of the TAT polypeptides disclosed herein have been successfully expressed and purified using this technique(s).

EXAMPLE 11: Preparation of Antibodies that Bind TAT

This example illustrates preparation of monoclonal antibodies which can specifically bind TAT.
Techniques for producing the monoclonal antibodies are known in the art and are described, for instance, in Goding, supra. Immunogens that may be employed include purified TAT, fusion proteins containing TAT, and cells expressing recombinant TAT on the cell surface. Selection of the immunogen can be made by the skilled artisan without undue experimentation.
Mice, such as Balb/c, are immunized with the TAT immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-TAT antibodies.
After a suitable antibody titer has been detected, the animals "positive" for antibodies can be injected with a final intravenous injection of TAT. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.
The hybridoma cells will be screened in an ELISA for reactivity against TAT. Determination of "positive" hybridoma cells secreting the desired monoclonal antibodies against TAT is within the skill in the art.
The positive hybridoma cells can be injected intraperitoneally into syngeneic Balb/c mice to produce ascites containing the anti-TAT monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

Antibodies directed against certain of the TAT polypeptides disclosed herein have been successfully produced using this technique(s). More specifically, functional monoclonal antibodies that are capable of recognizing and binding to TAT protein (as measured by standard ELISA, FACS sorting analysis and/or immunohistochemistry analysis) have been successfully generated against the following TAT proteins as disclosed herein: TAT243 (DNA82306), TAT135 (DNA68885) and TAT246 (DNA42551).

In addition to the successful preparation of monoclonal antibodies directed against the TAT polypeptides as described herein, many of those monoclonal antibodies have been successfully conjugated to a cell toxin for use in directing the cellular toxin to a cell (or tissue) that expresses a TAT polypeptide of interested (both *in vitro* and *in vivo*). For example, toxin (e.g., DM1) derivitized monoclonal antibodies have been successfully generated to the following TAT polypeptides as described herein: TAT135 (DNA68885).

EXAMPLE 12: Purification of TAT Polypeptides Using Specific Antibodies

Native or recombinant TAT polypeptides may be purified by a variety of standard techniques in the art of protein purification. For example, pro-TAT polypeptide, mature TAT polypeptide, or pre-TAT polypeptide is purified by immunoaffinity chromatography using antibodies specific for the TAT polypeptide of interest. In general, an immunoaffinity column is constructed by covalently coupling the anti-TAT polypeptide antibody to an activated chromatographic resin. Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE™ (Pharmacia LKB Biotechnology). The antibody is coupled to the resin, the resin is blocked, and the derivative resin is washed according to the manufacturer's instructions.

Such an immunoaffinity column is utilized in the purification of TAT polypeptide by preparing a fraction from cells containing TAT polypeptide in a soluble form. This preparation is derived by solubilization of the whole cell or of a subcellular fraction obtained via differential centrifugation by the addition of detergent or by other methods well known in the art. Alternatively, soluble TAT polypeptide containing a signal sequence may be secreted in useful quantity into the medium in which the cells are grown.

A soluble TAT polypeptide-containing preparation is passed over the immunoaffinity column, and the column is washed under conditions that allow the preferential absorbance of TAT polypeptide (*e.g.*, high ionic strength buffers in the presence of detergent). Then, the column is eluted under conditions that disrupt antibody/TAT polypeptide binding (*e.g.*, a low pH buffer such as approximately pH 2-3, or a high concentration of a chaotrope such as urea or thiocyanate ion), and TAT polypeptide is collected.

EXAMPLE 13: *In Vitro* Tumor Cell Killing Assay

Mammalian cells expressing the TAT polypeptide of interest may be obtained using standard expression vector and cloning techniques. Alternatively, many tumor cell lines expressing TAT polypeptides of interest are publicly available, for example, through the ATCC and can be routinely identified using standard ELISA or FACS analysis. Anti-TAT polypeptide monoclonal antibodies (and toxin conjugated derivatives thereof) may then be employed in assays to determine the ability of the antibody to kill TAT polypeptide expressing cells *in vitro.*

For example, cells expressing the TAT polypeptide of interest are obtained as described above and plated into 96 well dishes. In one analysis, the antibody/toxin conjugate (or naked antibody) is included throughout the cell incubation for a period of 4 days. In a second independent analysis, the cells are incubated for 1 hour with the antibody/toxin conjugate (or naked antibody) and then washed and incubated in the absence of antibody/toxin conjugate for a period of 4 days. Cell viability is then measured using the CellTiter-Glo Luminescent Cell Viability Assay from Promega (Cat# G7571). Untreated cells serve as a negative control.

EXAMPLE 14: *In Vivo* Tumor Cell Killing Assay

To test the efficacy of conjugated or unconjugated anti-TAT polypeptide monoclonal antibodies, anti-TAT antibody is injected intraperitoneally into nude mice 24 hours prior to receiving tumor promoting cells subcutaneously in the flank. Antibody injections continue twice per week for the remainder of the study. Tumor volume is then measured twice per week.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

## Claims

1. A method of binding an antibody to a glioma tumor cell in vitro that expresses a protein comprising an amino acid sequence having at least 90% amino acid sequence identity to:

   (a) the amino acid sequence shown as SEQ ID NO:37;
   (b) the amino acid sequence shown as SEQ ID NO:37, lacking its associated signal peptide; or
   (c) an amino acid sequence encoded a nucleic acid comprising the nucleotide sequence shown in SEQ ID NO:18, said method comprising contacting said glioma tumor cell with an antibody that binds to said protein and allowing the binding of said antibody to said protein to occur, thereby binding said antibody to said glioma tumor cell.

2. An antibody that binds to the polypeptide of SEQ ID NO:37 for use in treating a glioma tumor comprising cells that express a protein comprising an amino acid sequence having at least 90% amino acid sequence identity to:

   (a) the amino acid sequence shown in SEQ ID NO:37;
   (b) the amino acid sequence shown in SEQ ID NO:37, lacking its associated signal peptide; or
   (c) an amino acid sequence encoded by a nucleic acid comprising the nucleotide sequence shown in SEQ ID NO:18.

3. The method of claim 1 or the antibody of claim 2, wherein said antibody is a monoclonal antibody.

4. The method of claim 1 or the antibody of claim 2, wherein said antibody is an antibody fragment,a chimeric or humanized antibody.

5. The method of claim 1 or the antibody of claim 2, wherein said antibody is a chimeric or humanized antibody.

6. The method of claim 1 or the antibody of claim 2, wherein said antibody is conjugated to a growth inhibitory agent or a cytotoxic agent.

7. The method or antibody of claim 6, wherein said cytotoxic agent is selected from the group consisting of a toxin, an antibiotic, a radioactive isotope, and a nucleolytic enzyme.

8. The method or antibody of claim 7, wherein said cytotoxic agent is a maytansinoid.

9. A method of diagnosing the presence of a glioma tumor in a mammal, said method comprising detecting the level of expression of a gene encoding a polypeptide comprising an amino acid sequence having at least 90% amino acid sequence identity to:

   (a) the amino acid sequence shown in SEQ ID NO:37;
   (b) the amino acid sequence shown in SEQ ID NO:37, lacking its associated signal peptide; or
   (c) an amino acid sequence encoded by a nucleic acid comprising the nucleotide sequence shown in SEQ ID NO:18,

in a test sample of tissue cells obtained from said mammal, wherein a higher level of expression of the gene encoding said polypeptide in the test sample, as compared to a control sample, is indicative of the presence of a glioma tumor in the mammal from which the test sample was obtained.

**10.** The method of Claim 9, wherein detecting the level of expression of a gene encoding said polypeptide comprises employing an oligonucleotide in an *in situ* hybridization or RT-PCR analysis.

**11.** The method of Claim 9, wherein detecting the level of expression of a gene encoding said polypeptide comprises employing an antibody in an immunohistochemistry analysis.

**12.** The method of claim 1 or claim 9, or the antibody of claim 2, wherein the level of amino acid sequence identity is 95%.

**13.** The method of claim 1 or claim 9, or the antibody of claim 2, wherein the level of amino acid sequence identity is 100%.

**14.** A method of diagnosing the presence of a glioma tumor in a mammal, said method comprising contacting a test sample of tissue cells obtained from said mammal with an antibody that specifically binds to the polypeptide shown in SEQ ID NO:37, and detecting the formation of a complex between said antibody and a polypeptide in the test sample, wherein the formation of a complex is indicative of the presence of a glioma tumor in said mammal.

**15.** The method of Claim 14, wherein said antibody is detectably labeled.

# FIGURE 1

AGAAACTCAAGATTGACTCATGAGGACCTGAAGGGTGACATCCCAGGAGGGGCCTCTGAAATTTCCCACACCCC

AGCGCCTGTGCTGAGGACTCCCTCCATGTGGCCCCAGGTGCCACCAATAAAAATCCTACAGAAAATTC

# FIGURE 2

CTGGAAGCCGGCGGGTGCCGCTGTGTAGGAAAGAAGCTAAAGCACTTCCAGAGCCTGTCCGGAGCTCAGAGGTT
CGGAAGACTTATCGACC<u>ATG</u>GAGCGCGCGTCCTGCTTGTTGCTGCTGCTGCTGCCGCTGGTGCACGTCTCTGCG
ACCACGCCAGAACCTTGTGAGCTGGACGATGAAGATTTCCGCTGCGTCTGCAACTTCTCCGAACCTCAGCCCGA
CTGGTCCGAAGCCTTCCAGTGTGTGTCTGCAGTAGAGGTGGAGATCCATGCCGGCGGTCTCAACCTAGAGCCGT
TTCTAAAGCGCGTCGATGCGGACGCCGACCCGCGGCAGTATGCTGACACGGTCAAGGCTCTCCGCGTGCGGCGG
CTCACAGTGGGAGCCGCACAGGTTCCTGCTCAGCTACTGGTAGGCGCCCTGCGTGTGCTAGCGTACTCCCGCCT
CAAGGAACTGACGCTCGAGGACCTAAAGATAACCGGCACCATGCCTCCGCTGCCTCTGGAAGCCACAGGACTTG
CACTTTCCAGCTTGCGCCTACGCAACGTGTCGTGGGCGACAGGGCGTTCTTGGCTCGCCGAGCTGCAGCAGTGG
CTCAAGCCAGGCCTCAAGGTACTGAGCATTGCCCAAGCACACTCGCCTGCCTTTTCCTGCGAACAGGTTCGCGC
CTTCCCGGCCCTTACCAGCCTAGACCTGTCTGACAATCCTGGACTGGGCGAACGCGGACTGATGGCGGCTCTCT
GTCCCCACAAGTTCCCGGCCATCCAGAATCTAGCGCTGCGCAACACAGGAATGGAGACGCCCACAGGCGTGTGC
GCCGCACTGGCGGCGGCAGGTGTGCAGCCCCACAGCCTAGACCTCAGCCACAACTCGCTGCGCGCCACCGTAAA
CCCTAGCGCTCCGAGATGCATGTGGTCCAGCGCCCTGAACTCCCTCAATCTGTCGTTCGCTGGGCTGGAACAGG
TGCCTAAAGGACTGCCAGCCAAGCTCAGAGTGCTCGATCTCAGCTGCAACAGACTGAACAGGGCGCCGCAGCCT
GACGAGCTGCCCGAGGTGGATAACCTGACACTGGACGGGAATCCCTTCCTGGTCCCTGGAACTGCCCTCCCCCA
CGAGGGCTCAATGAACTCCGGCGTGGTCCCAGCCTGTGCACGTTCGACCCTGTCGGTGGGGGTGTCGGGAACCC
TGGTGCTCCTCCAAGGGGCCCGGGGCTTTGCC<u>TAA</u>GATCCAAGACAGAATAATGAATGGACTCAAACTGCCTTG
GCTTCAGGGGAGTCCCGTCAGGACGTTGAGGACTTTTCGACCAATTCAACCCTTTGCCCCACCTTTATTAAAAT
CTTAAACAACAAAA

# FIGURE 3

GGGCGGGCCTCACCCGCTTCGAGTCCTCGGGCTTCCCCCACCCGGCCCGTGGGGAGTATCTGTCCTGCCGCCT
TCGCCCACGCCCTGCACTCCGGGACCGTCCCTGCGCGCTCTGGGCGACC<u>ATG</u>GCCCGCGGGGCTGCGCTGGCGC
TGCTGCTCTTCGGCCTGCTGGGTGTTCTGGTCGCCGCCCCGGATGGTGGTTTCGATTTATCTGATGCCCTTCCT
GACAATGAAAACAAGAAACCCACTGCAATCCCCAAGAAACCCAGTGCTGGGGATGACTTTGACTTAGGAGATGC
TGTTGTTGATGGAGAAAATGACGACCCACGACCACCGAACCCACCCAAACCGATGCCAAATCCAAACCCCAACC
ACCCTAGTTCCTCCGGTAGCTTTTCAGATGCTGACCTTGCGGATGGCGTTTCAGGTGGAGAAGGAAAAGGAGGC
AGTGATGGTGGAGGCAGCCACAGGAAAGAAGGGGAAGAGGCCGACGCCCCAGGCGTGATCCCCGGGATTGTGGG
GGCTGTCGTGGTCGCCGTGGCTGGAGCCATCTCTAGCTTCATTGCTTACCAGAAAAAGAAGCTATGCTTCAAAG
AAAATGCAGAACAAGGGGAGGTGGACATGGAGAGCCACCGGAATGCCAACGCAGAGCCAGCTGTTCAGCGTACT
CTTTTAGAGAAA<u>TAG</u>AAGATTGTCGGCAGAAACAGCCCAGGCGTTGGCAGCAGGGTTAGAACAGCTGCCTGAGG
CTCCTCCCTGAAGGACACCTGCCTGAGAGCAGAGATGGAGGCCTTCTGTTCACGGCGGATTCTTTGTTTTAATC
TTGCGATGTGCTTTGCTTGTTGCTGGGCGGATGATGTTTACTAACGATGAATTTTACATCCAAAGGGGGATAGG
CACTTGGACCCCCATTCTCCAAGGCCCGGGGGGGCGGTTTCCCATGGGATGTGAAAGGCTGGCCATTATTAAGT
CCCTGTAACTCAAATGTCAACCCCACCGAGGCACCCCCCCGTCCCCCAGAATCTTGGCTGTTTACAAATCACGT
GTCCATCGAGCACGTCTGAAACCCCTGGTAGCCCCGACTTCTTTTTAATTAAAATAAGGTAAGCCCTTCAATTT

# FIGURE 4

CCACGCGTCCGCGCTGCGCCACATCCCACCGGCCCTTACACTGTGGTGTCCAGCAGCATCCGGCTTCATGGGGG
GACTTGAACCCTGCAGCAGGCTCCTGCTCCTGCCTCTCCTGCTGGCTGTAAGTGGTCTCCGTCCTGTCCAGGCC
CAGGCCCAGAGCGATTGCAGTTGCTCTACGGTGAGCCCGGGCGTGCTGGCAGGGATCGTGATGGGAGACCTGGT
GCTGACAGTGCTCATTGCCCTGGCCGTGTACTTCCTGGGCCGGCTGGTCCCTCGGGGGCGAGGGGCTGCGGAGG
CAGCGACCCGGAAACAGCGTATCACTGAGACCGAGTCGCCTTATCAGGAGCTCCAGGGTCAGAGGTCGGATGTC
TACAGCGACCTCAACACACAGAGGCCGTATTACAAATGAGCCCGAATCATGACAGTCAGCAACATGATACCTGG
ATCCAGCCATTCCTGAAGCCCACCCTGCACCTCATTCCAACTCCTACCGCGATACAGACCCACAGAGTGCCATC
CCTGAGAGACCAGACCGCTCCCCAATACTCTCCTAAAATAAACATGAAGCACAAAAAAAAAAAAAAAAAAAAAA
AAAAAAAAAAAA

# FIGURE 5

TTCTTTTGGAAAACCAAACATGCTTTATTTCATTTTTTTCACAATTTATTTAAACATCTCACATATACAAAATA
GGTACAATTTAATTTTTCTGCTTGCCCAAGAAACAAAGCTTCTGTGGAACCATGGAAGAAGATGAAAATGAGAC
TGGCAAAGAACAAATGCTGAATCTGAAGAAGAGGACAACTTTGGGCAAATAATCTGCATACTTTTAATTGGGAA
TAAGATGGAAAATATGAATGCTAAATCAAATTTTTTAAAAAATACACCACACGATACAACTCAATACAGGAGTA
TTTCTTCTCAAATTCTTCTAGCACCATCAACATTCTTCAAGTATCTGAAATACTATTAATTAGCACCTTTGTAT
TATGAACAAAACAAAACAAGGACCTCAGTTCATCTCTGTCTAGGTCAGCACCTAACAATGTGGATCACACTCAT
GGGAAAGTGTTTTGAGGTAGTTTAAACCTTTGGAAGTTTGGGTTTTAAACTTCCCTCTGTGGAAGATATTCAAA
AGCCACAAGTGGTGCAAATGTTTATGGTTTTTATTTTTCAATTTTTATTTTGGTTTTCTTACAAAGGTTGACAT
TTTTCATAACAGGTGTAAGAGTGTTGAAAAAAAAATTTCAATTTTTGGXGGGAACGGGGGAAGGAGTTAATGAAACT

# FIGURE 6

GGACAAAAGGGTGAAAGAGGCCTCCCGGGGTTACAAGGTGTCATTGGGTTTCCTGGAATGCAAGGACCTGAGGG
GCCACAGGGACCACCAGGACAAAAGGGTGATACTGGAGAACCAGGACTACCTGGAACAAAAGGGACAAGAGGAC
CTCCGGGAGCATCTGGCTACCCTGGAAACCCAGGACTTCCCGGAATTCCTGGCCAAGACGGCCCGCCAGGCCCC
CCAGGTATTCCAGGATGCAATGGCACAAAGGGGGAGAGAGGGCCGCTCGGGCCTCCTGGCTTGCCTGGTTTCGC
AGGAAACCCCGGACCACCAGGCTTACCAGGGATGAAGGGTGATCCAGGTGAGATACTTGGCCATGTGCCCGGGA
TGCTGTTGAAAGGTGAAAGAGGATTTCCCGGAATCCCAGGGACTCCAGGCCCACCAGGACTGCCAGGGCTTCAA
GGTCCTGTTGGGCCTCCAGGATTTACCGGACCACCAGGTCCCCCAGGCCCTCCCGGCCCTCCAGGTGAAAAGGG
ACAAATGGGCTTAAGTTTTCAAGGACCAAAAGGTGACAAGGGTGACCAAGGGGTCAGTGGGCCTCCAGGAGTAC
CAGGACAAGCTCAAGTTCAAGAAAAAGGAGACTTCGCCACCAAGGGAGAAAAGGGCCAAAAAGGTGAACCTGGA
TTTCAGGGGATGCCAGGGGTCGGAGAGAAAGGTGAACCCGGAAAACCAGGACCCAGAGGCAAACCCGGAAAAGA
TGGTGACAAAGGGGAAAAAGGGAGTCCCGGTTTTCCTGGTGAACCCGGGTACCCAGGACTCATAGGCCGCCAGG
GCCCGCAGGGAGAAAAGGGTGAAGCAGGTCCTCCTGGCCCACCTGGAATTGTTATAGGCACAGGACCTTTGGGA
GAAAAAGGAGAGAGGGGCTACCCTGGAACTCCGGGGCCAAGAGGAGAGCCAGGCCCAAAAGGTTTCCCAGGACT
ACCAGGCCAACCCGGACCTCCAGGCCTCCCTGTACCTGGGCAGGCTGGTGCCCCTGGCTTCCCTGGTGAAAGAG
GAGAAAAAGGTGACCGAGGATTTCCTGGTACATCTCTGCCAGGACCAAGTGGAAGAGATGGGCTCCCGGGTCCT
CCTGGTTCCCCTGGCCCCCTGGGCAGCCTGGCTACACAAATGGAATTGTGGAATGTCAGCCCGGACCTCCAGG
TGACCAGGGTCCTCCTGGAATTCCAGGGCAGCCAGGATTTATAGGCGAAATTGGAGAGAAAGGTCAAAAAGGAG
AGAGTTGCCTCATCTGTGATATAGACGGATATCGGGGGCCTCCCGGGCCACAGGGACCCCCGGGAGAAATAGGT
TTCCCAGGGCAGCCAGGGGCCAAGGGCGACAGAGGTTTGCCTGGCAGAGATGGTGTTGCAGGAGTGCCAGGCCC
TCAAGGTACACCAGGGCTGATAGGCCAGCCAGGAGCCAAGGGGGAGCCTGGTGAGTTTTATTTCGACTTGCGGC
TCAAAGGTGACAAAGGAGACCCAGGCTTTCCAGGACAGCCCGGCATGCCAGGGAGAGCGGGTTCTCCTGGAAGA
GATGGCCATCCGGGTCTTCCTGGCCCCAAGGGCTCGCCGGGTTCTGTAGGATTGAAAGGAGAGCGTGGCCCCCC
TGGAGGAGTTGGATTCCCAGGCAGTCGTGGTGACACCGGCCCCCCTGGGCCTCCAGGATATGGTCCTGCTGGTC
CCATTGGTGACAAAGGACAAGCAGGCTTTCCTGGAGGCCCTGGATCCCCAGGCCTGCCAGGTCCAAAGGGTGAA
CCAGGAAAAATTGTTCCTTTACCAGGCCCCCCTGGAGCAGAAGGACTGCCGGGGTCCCCAGGCTTCCCAGGTCC
CCAAGGAGACCGAGGCTTTCCCGGAACCCCAGGAAGGCCAGGCCTGCCAGGAGAGAAGGGCGCTGTGGGCCAGC
CAGGCATTGGATTTCCAGGGCCCCCCGGCCCCAAAGGTGTTGACGGCTTACCTGGAGACATGGGGCCACCGGGG
ACTCCAGGTCGCCCGGGATTTAATGGCTTACCTGGGAACCCAGGTGTGCAGGGCCAGAAGGGAGAGCCTGGAGT
TGGTCTACCGGGACTCAAAGGTTTGCCAGGTCTTCCCGGCATTCCTGGCACACCCGGGGAGAAGGGGAGCATTG
GGGTACCAGGCGTTCCTGGAGAACATGGAGCGATCGGACCCCCTGGGCTTCAGGGGATCAGAGGTGAACCGGGA
CCTCCTGGATTGCCAGGCTCCGTGGGGTCTCCAGGAGTTCCAGGAATAGGCCCCCCTGGAGCTAGGGGTCCCCC
TGGAGGACAGGGACCACCGGGGTTGTCAGGCCCTCCTGGAATAAAAGGAGAGAAGGGTTTCCCCGGATTCCCTG
GACTGGACATGCCGGGCCCTAAAGGAGATAAAGGGGCTCAAGGACTCCCTGGCATAACGGGACAGTCGGGGCTC
CCTGGCCTTCCTGGACAGCAGGGGGCTCCTGGGATTCCTGGGTTTCCAGGTTCCAAGGGAGAAATGGGCGTCAT
GGGGACCCCCGGGCAGCCGGGCTCACCAGGACCATGGGGTGCTCCTGGATTACCGGGTGAAAAAGGGGACCATG
GCTTTCCGGGCTCCTCAGGACCCAGGGGAGACCCTGGCTTGAAAGGTGATAAGGGGGATGTCGGTCTCCCTGGC
AAGCCTGGCTCCATGGATAAGGTGGACATGGGCAGCATGAAGGGCCAGAAAGGAGACCAAGGAGAGAAAGGACA
AATTGGACCAATTGGTGAGAAGGGATCCCGAGGAGACCCTGGGACCCCAGGAGTGCCTGGAAAGGACGGGCAGG
CAGGACAGCCTGGGCAGCCAGGACCTAAAGGTGATCCAGGTATAAGTGGAACCCCAGGTGCTCCAGGACTTCCG
GGACCAAAAGGATCTGTTGGTGGAATGGGCTTGCCAGGAACACCTGGAGAGAAAGGTGTGCCTGGCATCCCTGG
CCCACAAGGTTCACCTGGCTTACCTGGAGACAAAGGTGCAAAAGGAGAGAAAGGGCAGGCAGGCCCACCTGGCA
TAGGCATCCCAGGGCTGCGAGGTGAAAAGGGAGATCAAGGGATAGCGGGTTTCCCAGGAAGCCCTGGAGAGAAG
GGAGAAAAAGGAAGCATTGGGATCCCAGGAATGCCAGGGTCCCCAGGCCTTAAAGGGTCTCCCGGGAGTGTTGG
CTATCCAGGAAGTCCTGGGCTACCTGGAGAAAAAGGTGACAAAGGCCTCCCAGGATTGGATGGCATCCCTGGTG
TCAAAGGAGAAGCAGGTCTTCCTGGGACTCCTGGCCCCACAGGCCCAGCTGGCCAGAAAGGGGAGCCAGGCAGT
GATGGAATCCCGGGGTCAGCAGGAGAGAAGGGTGAACCAGGTCTACCAGGAAGAGGATTCCCAGGGTTTCCAGG
GGCCAAAGGAGACAAAGGTTCAAAGGGTGAGGTGGGTTTCCCAGGATTAGCCGGGAGCCCAGGAATTCCTGGAT
CCAAAGGAGAGCAAGGATTCATGGGTCCTCCGGGGCCCCAGGGACAGCCGGGGTTACCGGGATCCCCAGGCCAT
GCAACGGAGGGGCCCAAAGGAGACCGCGGACCTCAGGGCCAGCCTGGCCTGCCAGGACTTCCGGGACCCATGGG
GCCTCCAGGG

# FIGURE 7

GGGGAACGAGGCCCACCTGGGAGCCCAGGACTTCAGGGGTTCCCAGGCATCACACCCCCTTCCAACATCTCTGG
GGCACCTGGTGACAAAGGGGCGCCAGGGATATTTGGCCTGAAAGGTTATCGGGGCCCACCAGGGCCACCAGGTT
CTGCTGCTCTTCCTGGAAGCAAAGGTGACACAGGGAACCCAGGAGCTCCAGGAACCCCAGGGACCAAAGGATGG
GCCGGGGACTCCGGGCCCCAGGGCAGGCCTGGTGTGTTTGGTCTCCCAGGAGAAAAAGGGCCCAGGGGTGAACA
AGGCTTCATGGGGAACACTGGACCCACCGGGGCGGTGGGCGACAGAGGCCCCAAGGGACCCAAGGGAGACCCAG
GATTCCCTGGTGCCCCCGGGACTGTGGGAGCCCCCGGGATTGCAGGAATCCCCCAGAAGATTGCCATCCAACCA
GGGACAGTGGGTCCCCAGGGGAGGCGAGGCCCCCCTGGGGCACCGGGGGAGATCGGGCCCCAGGGCCCCCCCGG
AGAACCAGGTTTTCGTGGGGCTCCAGGGAAAGCTGGGCCCCAAGGAAGAGGTGGTGTGTCTGCTGTTCCCGGCT
TCCGGGGAGATGAAGGACCCATAGGCCACCAGGGGCCGATTGGCCAAGAAGGTGCACCAGGCCGTCCAGGGAGC
CCGGGCCTGCCGGGTATGCCAGGCCGCAGCGTCAGCATCGGCTACCTCCTGGTGAAGCACAGCCAGACGGACCA
GGAGCCCATGTGCCCGGTGGGCATGAACAAACTCTGGAGTGGATACAGCCTGCTGTACTTCGAGGGCCAGGAGA
AGGCGCACAACCAGGACCTGGGGCTGGCGGGCTCCTGCCTGGCGCGGTTCAGCACCATGCCCTTCCTGTACTGC
AACCCTGGTGATGTCTGCTACTATGCCAGCCGGAACGACAAGTCCTACTGGCTCTCTACCACTGCGCCGCTGCC
CATGATGCCCGTGGCCGAGGACGAGATCAAGCCCTACATCAGCCGCTGTTCTGTGTGTGAGGCCCCGGCCATCG
CCATCGCGGTCCACAGTCAGGATGTCTCCATCCCACACTGCCCAGCTGGGTGGCGGAGTTTGTGGATCGGATAT
TCCTTCCTCATGCACACGGCGGCGGGAGACGAAGGCGGTGGCCAATCACTGGTGTCACCGGGCAGCTGTCTAGA
GGACTTCCGCGCCACACCATTCATCGAATGCAATGGAGGCCGCGGCACCTGCCACTACTACGCCAACAAGTACA
GCTTCTGGCTGACCACCATTCCCGAGCAGAGCTTCCAGGGCTCGCCCTCCGCCGACACGCTCAAGGCCGGCCTC
ATCCGCACACACATCAGCCGCTGCCAGGTGTGCATGAAGAACCTG**TGA**GCCGGCGCGTGCCAGGAAGGGCCATT
TTGGTGCTTATTCTTAACTTATTACCTCAGGTGCCAACCAAAAATTGGTTTTATTTTTTTCTTAAAAAAAAAAA
AAAGTCTACCAAAGGAATTTGCATCCAGCAGCAGCACTTAGACCTGCCAGCCACTGTCACCGAGCGGGTGCAAG
CACTCGGGGTCCCTGGAGGCAAGCCCTGCCCACAGAAAGCCAGGAGCAGCCCTGGCCCCCATCAGCCCTGCTAC
GACGCACCGCCTGAAGGCACAGCTAACCACTTCGCACACACCCATGTAACCACTGCACTTTCCAATGCCACAGA
CAACTCACATTGTTCAACTCCTTCTCGGGGTGGGACAGACGAGACAACAGCACACAGGCAGCCAGCCGTGGCCA
GAGGCTCGAGGGGCTCAGGGGCTCAGGCACCCGTCCCCACACGAGGGCCCCGTGGGTGGCCTGGCCCTGCTTTC
TACGCCAATGTTATGCCAGCTCCATGTTCTCCCAAATACCGTTGATGTGAATTATTTTAAAGGCAAAACTGTGC
TCTTTATTTTAAAAAACACTGATAATCACACTGCGGTAGGTCATTCTTTTGCCACATCCCTATAGACCACTGGG
TTTGGCAAAACTCAGGCAGAAGTGGAGACCTTTCTAGACATCATTGTCAGCCTTGCTACTTGAAGGTACACCCC
ATAGGGTCGGAGGTGCTGTCCCCACTGCCCCACCTTGTCCCTGAGATTTAACCCCTCCACTGCTGGGGGTGAGC
TGTACTCTTCTGACTGCCCCCTCCTGTGTAACGACTACAAAATAAAACTTGGTTCTGAATATTTTT

# FIGURE 8A

AGCCGGCCGTGGTGGCTCCGTGCGTCCGAGCGTCCGTCCGCGCCGTCGGCCATGGCCAAGCGCTCCAGGGGCCC
CGGGCGCCGCTGCCTGTTGGCGCTCGTGCTGTTCTGCGCCTGGGGGACGCTGGCCGTGGTGGCCCAGAAGCCGG
GCGCAGGGTGTCCGAGCCGCTGCCTGTGCTTCCGCACCACCGTGCGCTGCATGCATCTGCTGCTGGAGGCCGTG
CCCGCCGTGGCGCCGCAGACCTCCATCCTAGATCTTCGCTTTAACAGAATCAGAGAGATCCAACCTGGGGCATT
CAGGCGGCTGAGGAACTTGAACACATTGCTTCTCAATAATAATCAGATCAAGAGGATACCTAGTGGAGCATTTG
AAGACTTGGAAAATTTAAAATATCTCTATCTGTACAAGAATGAGATCCAGTCAATTGACAGGCAAGCATTTAAG
GGACTTGCCTCTCTAGAGCAACTATACCTGCACTTTAATCAGATAGAAACTTTGGACCCAGATTCGTTCCAGCA
TCTCCCGAAGCTCGAGAGGCTATTTTTGCATAACAACCGGATTACACATTTAGTTCCAGGGACATTTAATCACT
TGGAATCTATGAAGAGATTGCGACTGGACTCAAACACACTTCACTGCGACTGTGAAATCCTGTGGTTGGCGGAT
TTGCTGAAAACCTACGCGGAGTCGGGGAACGCGCAGGCAGCGGCCATCTGTGAATATCCCAGACGCATCCAGGG
ACGCTCAGTGGCAACCATCACCCCGGAAGAGCTGAACTGTGAAAGGCCCCGGATCACCTCCGAGCCCCAGGACG
CAGATGTGACCTCGGGGAACACCGTGTACTTCACCTGCAGAGCCGAAGGCAACCCCAAGCCTGAGATCATCTGG
CTGCGAAACAATAATGAGCTGAGCATGAAGACAGATTCCCGCCTAAACTTGCTGGACGATGGGACCCTGATGAT
CCAGAACACACAGGAGACAGACCAGGGTATCTACCAGTGCATGGCAAAGAACGTGGCCGGAGAGGTGAAGACGC
AAGAGGTGACCCTCAGGTACTTCGGGTCTCCAGCTCGACCCACTTTTGTAATCCAGCCACAGAATACAGAGGTG
CTGGTTGGGGAGAGCGTCACGCTGGAGTGCAGCGCCACAGGCCACCCCCCGCCGCGGATCTCCTGGACGAGAGG
TGACCGCACACCCTTGCCAGTTGACCCGCGGGTGAACATCACGCCTTCTGGCGGGCTTTACATACAGAACGTCG
TACAGGGGGACAGCGGAGAGTATGCGTGCTCTGCGACCAACAACATTGACAGCGTCCATGCCACCGCTTTCATC
ATCGTCCAGGCTCTTCCTCAGTTCACTGTGACGCCTCAGGACAGAGTCGTTATTGAGGGCCAGACCGTGGATTT
CCAGTGTGAAGCCAAGGGCAACCCGCCGCCCGTCATCGCCTGGACCAAGGGAGGGAGCCAGCTCTCCGTGGACC
GGCGGCACCTGGTCCTGTCATCGGGAACACTTAGAATCTCTGGTGTTGCCCTCCACGACCAGGGCCAGTACGAA
TGCCAGGCTGTCAACATCATCGGCTCCCAGAAGGTCGTGGCCCACCTGACTGTGCAGCCCAGAGTCACCCCAGT
GTTTGCCAGCATTCCCAGCGACACAACAGTGGAGGTGGGCGCCAATGTGCAGCTCCCGTGCAGCTCCCAGGGCG
AGCCCGAGCCAGCCATCACCTGGAACAAGGATGGGGTTCAGGTGACAGAAAGTGGAAAATTTCACATCAGCCCT
GAAGGATTCTTGACCATCAATGACGTTGGCCCTGCAGACGCAGGTCGCTATGAGTGTGTGGCCCGGAACACCAT
TGGGTCGGCCTCGGTGAGCATGGTGCTCAGTGTGAACGTTCCTGACGTCAGTCGAAATGGAGATCCGTTTGTAG
CTACCTCCATCGTGGAAGCGATTGCGACTGTTGACAGAGCTATAAACTCAACCCGAACACATTTGTTTGACAGC
CGTCCTCGTTCTCCAAATGATTTGCTGGCCTTGTTCCGGTATCCGAGGGATCCTTACACAGTTGAACAGGCACG
GGCGGGAGAAATCTTTGAACGGACATTGCAGCTCATTCAGGAGCATGTACAGCATGGCTTGATGGTCGACCTCA
ACGGAACAAGTTACCACTACAACGACCTGGTGTCTCCACAGTACCTGAACCTCATCGCAAACCTGTCGGGCTGT
ACCGCCCACCGGCGCGTGAACAACTGCTCGGACATGTGCTTCCACCAGAAGTACCGGACGCACGACGGCACCTG
TAACAACCTGCAGCACCCCATGTGGGGCGCCTCGCTGACCGCCTTCGAGCGCCTGCTGAAATCCGTGTACGAGA
ATGGCTTCAACACCCCTCGGGGCATCAACCCCCACCGACTGTACAACGGGCACGCCCTTCCCATGCCGCGCCTG
GTGTCCACCACCCTGATCGGGACGGAGACCGTCACACCCGACGAGCAGTTCACCCACATGCTGATGCAGTGGGG
CCAGTTCCTGGACCACGACCTCGACTCCACGGTGGTGGCCCTGAGCCAGGCACGCTTCTCCGACGGACAGCACT
GCAGCAACGTGTGCAGCAACGACCCCCCCTGCTTCTCTGTCATGATCCCCCCCAATGACTCCCGGGCCAGGAGC
GGGGCCCGCTGCATGTTCTTCGTGCGCTCCAGCCCTGTGTGCGGCAGCGGCATGACTTCGCTGCTCATGAACTC
CGTGTACCCGCGGGAGCAGATCAACCAGCTCACCTCCTACATCGACGCATCCAACGTGTACGGGAGCACGGAGC
ATGAGGCCCGCAGCATCCGCGACCTGGCCAGCCACCGCGGCCTGCTGCGGCAGGGCATCGTGCAGCGGTCCGGG
AAGCCGCTGCTCCCCTTCGCCACCGGGCCGCCCACGGAGTGCATGCGGGACGAGAACGAGAGCCCCATCCCCTG
CTTCCTGGCCGGGGACCACCGCGCCAACGAGCAGCTGGGCCTGACCAGCATGCACACGCTGTGGTTCCGCGAGC
ACAACCGCATTGCCACGGAGCTGCTCAAGCTGAACCCGCACTGGGACGGCGACACCATCTACTATGAGACCAGG
AAGATCGTGGGTGCGGAGATCCAGCACATCACCTACCAGCACTGGCTCCCGAAGATCCTGGGGGAGGTGGGCAT
GAGGACGCTGGGAGAGTACCACGGCTACGACCCCGGCATCAATGCTGGCATCTTCAACGCCTTCGCCACCGCGG
CCTTCAGGTTTGGCCACACGCTTGTCAACCCACTGCTTTACCGGCTGGACGAGAACTTCCAGCCCATTGCACAA
GATCACCTCCCCCTTCACAAAGCTTTCTTCTCTCCCTTCCGGATTGTGAATGAGGGCGGCATCGATCCGCTTCT
CAGGGGGCTGTTCGGGGTGGCGGGGAAAATGCGTGTGCCCTCGCAGCTGCTGAACACGGAGCTCACGGAGCGGC
TGTTCTCCATGGCACACACGGTGGCTCTGGACCTGGCGGCCATCAACATCCAGCGGGGCCGGGACCACGGGATC
CCACCCTACCACGACTACAGGGTCTACTGCAATCTATCGGCGGCACACACGTTCGAGGACCTGAAAAATGAGAT
TAAAAACCCTGAGATCCGGGAGAAACTGAAAAGGTTGTATGGCTCGACACTCAACATCGACCTGTTTCCGGCGC
TCGTGGTGGAGGACCTGGTGCCTGGCAGCCGGCTGGGCCCCACCCTGATGTGTCTTCTCAGCACACAGTTCAAG
CGCCTGCGAGATGGGGACAGGTTGTGGTATGAGAACCCTGGGGTGTTCTCCCCGGCCCAGCTGACTCAGATCAA
GCAGACGTCGCTGGCCAGGATCCTATGCGACAACGCGGACAACATCACCCGGGTGCAGAGCGACGTGTTCAGGG
TGGCGGAGTTCCCTCACGGCTACGGCAGCTGTGACGAGATCCCCAGGGTGGACCTCCGGGTGTGGCAGGACTGC

# FIGURE 8B

TGTGAAGACTGTAGGACCAGGGGGCAGTTCAATGCCTTTTCCTATCATTTCCGAGGCAGACGGTCTCTTGAGTT
CAGCTACCAGGAGGACAAGCCGACCAAGAAAACAAGACCACGGAAAATACCCAGTGTTGGGAGACAGGGGGAAC
ATCTCAGCAACAGCACCTCAGCCTTCAGCACACGCTCAGATGCATCTGGGACAAATGACTTCAGAGAGTTTGTT
CTGGAAATGCAGAAGACCATCACAGACCTCAGAACACAGATAAAGAAACTTGAATCACGGCTCAGTACCACAGA
GTGCGTGGATGCCGGGGGCGAATCTCACGCCAACAACACCAAGTGGAAAAAAGATGCATGCACCATTTGTGAAT
GCAAAGACGGGCAGGTCACCTGCTTCGTGGAAGCTTGCCCCCCTGCCACCTGTGCTGTCCCCGTGAACATCCCA
GGGGCCTGCTGTCCAGTCTGCTTACAGAAGAGGGCGGAGGAAAAGCCC<u>TAG</u>GCTCCTGGGAGGCTCCTCAGAGT
TTGTCTGCTGTGCCATCGTGAGATCGGGTGGCCGATGGCAGGGAGCTGCGGACTGCAGACCAGGAAACACCCAG
AACTCGTGACATTTCATGACAACGTCCAGCTGGTGCTGTTACAGAAGGCAGTGCAGGAGGCTTCCAACCAGAGC
ATCTGCGGAGAAGGAGGCACAGCAGGTGCCTGAAGGGAAGCAGGCAGGAGTCCTAGCTTCACGTTAGACTTCTC
AGGTTTTTATTTAATTCTTTTAAAATGAAAAATTGGTGCTACTATTAAATTGCACAGTTGAATCATTTAGGCGC
CTAAATTGGTTTTGCCTCCCAACACCATTTCTTTTTAAATAAAGCAGGATACCTCTATATGTCAGCCTTGCCTT
GTTCAGATGCCAGGAGCCGGCAGACCTGTCACCCGCAGGTGGGGTGAGTCTCGGAGCTGCCAGAGGGGCTCACC
GAAATCGGGGTTCCATCACAAGCTATGTTTAAAAAGAAAATTGGTGTTTGGCAAACGGAACAGAACCTTTGATG
AGAGCGTTCACAGGGACACTGTCTGGGGGTGCAGTGCAAGCCCCCGGCCTCTTCCCTGGGAACCTCTGAACTCC
TCCTTCCTCTGGGCTCTCTGTAACATTTCACCACACGTCAGCATCTAATCCCAAGACAAACATTCCCGCTGCTC
GAAGCAGCTGTATAGCCTGTGACTCTCCGTGTGTCAGCTCCTTCCACACCTGATTAGAACATTCATAAGCCACA
TTTAGAAACAGATTTGCTTTCAGCTGTCACTTGCACACATACTGCCTAGTTGTGAACCAAATGTGAAAAAACCT
CCTTCATCCCATTGTGTATCTGATACCTGCCGAGGGCCAAGGGTGTGTGTTGACAACGCCGCTCCCAGCCGGCC
CTGGTTGCGTCCACGTCCTGAACAAGAGCCGCTTCCGGATGGCTCTTCCCAAGGGAGGAGGAGCTCAAGTGTCG
GGAACTGTCTAACTTCAGGTTGTGTGAGTGCGTT

# FIGURE 9A

CAAACATGTCAGCTGTTACTGGAAGTGGCCTGGCCTCTATTTATCTTCCTGATCCTGATCTCTGTTCGGCTGAG
CTACCCACCCTATGAACAACATGAATGCCATTTTCCAAATAAAGCCATGCCCTCTGCAGGAACACTTCCTTGGG
TTCAGGGGATTATCTGTAATGCCAACAACCCCTGTTTCCGTTACCCGACTCCTGGGGAGGCTCCCGGAGTTGTT
GGAAACTTTAACAAATCCATTGTGGCTCGCCTGTTCTCAGATGCTCGGAGGCTTCTTTTATACAGCCAGAAAGA
CACCAGCATGAAGGACATGCGCAAAGTTCTGAGAACATTACAGCAGATCAAGAAATCCAGCTCAAACTTGAAGC
TTCAAGATTTCCTGGTGGACAATGAAACCTTCTCTGGGTTCCTGTATCACAACCTCTCTCTCCCAAAGTCTACT
GTGGACAAGATGCTGAGGGCTGATGTCATTCTCCACAAGGTATTTTTGCAAGGCTACCAGTTACATTTGACAAG
TCTGTGCAATGGATCAAAATCAGAAGAGATGATTCAACTTGGTGACCAAGAAGTTTCTGAGCTTTGTGGCCTAC
CAAGGGAGAAACTGGCTGCAGCAGAGCGAGTACTTCGTTCCAACATGGACATCCTGAAGCCAATCCTGAGAACA
CTAAACTCTACATCTCCCTTCCCGAGCAAGGAGCTGGCCGAAGCCACAAAAACATTGCTGCATAGTCTTGGGAC
TCTGGCCCAGGAGCTGTTCAGCATGAGAAGCTGGAGTGACATGCGACAGGAGGTGATGTTTCTGACCAATGTGA
ACAGCTCCAGCTCCTCCACCCAAATCTACCAGGCTGTGTCTCGTATTGTCTGCGGGCATCCCGAGGGAGGGGGG
CTGAAGATCAAGTCTCTCAACTGGTATGAGGACAACAACTACAAAGCCCTCTTTGGAGGCAATGGCACTGAGGA
AGATGCTGAAACCTTCTATGACAACTCTACAACTCCTTACTGCAATGATTTGATGAAGAATTTGGAGTCTAGTC
CTCTTTCCCGCATTATCTGGAAAGCTCTGAAGCCGCTGCTCGTTGGGAAGATCCTGTATACACCTGACACTCCA
GCCACAAGGCAGGTCATGGCTGAGGTGAACAAGACCTTCCAGGAACTGGCTGTGTTCCATGATCTGGAAGGCAT
GTGGGAGGAACTCAGCCCCAAGATCTGGACCTTCATGGAGAACAGCCAAGAAATGGACCTTGTCCGGATGCTGT
TGGACAGCAGGGACAATGACCACTTTTGGGAACAGCAGTTGGATGGCTTAGATTGGACAGCCCAAGACATCGTG
GCGTTTTTGGCCAAGCACCCAGAGGATGTCCAGTCCAGTAATGGTTCTGTGTACACCTGGAGAGAAGCTTTCAA
CGAGACTAACCAGGCAATCCGGACCATATCTCGCTTCATGGAGTGTGTCAACCTGAACAAGCTAGAACCCATAG
CAACAGAAGTCTGGCTCATCAACAAGTCCATGGAGCTGCTGGATGAGAGGAAGTTCTGGGCTGGTATTGTGTTC
ACTGGAATTACTCCAGGCAGCATTGAGCTGCCCCATCATGTCAAGTACAAGATCCGAATGGACATTGACAATGT
GGAGAGGACAAATAAAATCAAGGATGGGTACTGGGACCCTGGTCCTCGAGCTGACCCCTTTGAGGACATGCGGT
ACGTCTGGGGGGGCTTCGCCTACTTGCAGGATGTGGTGGAGCAGGCAATCATCAGGGTGCTGACGGGCACCGAG
AAGAAAACTGGTGTCTATATGCAACAGATGCCCTATCCCTGTTACGTTGATGACATCTTTCTGCGGGTGATGAG
CCGGTCAATGCCCCTCTTCATGACGCTGGCCTGGATTTACTCAGTGGCTGTGATCATCAAGGGCATCGTGTATG
AGAAGGAGGCACGGCTGAAAGAGACCATGCGGATCATGGGCCTGGACAACAGCATCCTCTGGTTTAGCTGGTTC
ATTAGTAGCCTCATTCCTCTTCTTGTGAGCGCTGGCCTGCTAGTGGTCATCCTGAAGTTAGGAAACCTGCTGCC
CTACAGTGATCCCAGCGTGGTGTTTGTCTTCCTGTCCGTGTTTGCTGTGGTGACAATCCTGCAGTGCTTCCTGA
TTAGCACACTCTTCTCCAGAGCCAACCTGGCAGCAGCCTGTGGGGGCATCATCTACTTCACGCTGTACCTGCCC
TACGTCCTGTGTGTGGCATGGCAGGACTACGTGGGCTTCACACTCAAGATCTTCGCTAGCCTGCTGTCTCCTGT
GGCTTTTGGGTTTGGCTGTGAGTACTTTGCCCTTTTTGAGGAGCAGGGCATTGGAGTGCAGTGGGACAACCTGT
TTGAGAGTCCTGTGGAGGAAGATGGCTTCAATCTCACCACTTCGGTCTCCATGATGCTGTTTGACACCTTCCTC
TATGGGGTGATGACCTGGTACATTGAGGCTGTCTTTCCAGGCCAGTACGGAATTCCCAGGCCCTGGTATTTTCC
TTGCACCAAGTCCTACTGGTTTGGCGAGGAAAGTGATGAGAAGAGCCACCCTGGTTCCAACCAGAAGAGAATAT
CAGAAATCTGCATGGAGGAGGAACCCACCCACTTGAAGCTGGGCGTGTCCATTCAGAACCTGGTAAAAGTCTAC
CGAGATGGGATGAAGGTGGCTGTCGATGGCCTGGCACTGAATTTTTATGAGGGCCAGATCACCTCCTTCCTGGG
CCACAATGGAGCGGGGAAGACGACCACCATGTCAATCCTGACCGGGTTGTTCCCCCCGACCTCGGGCACCGCCT
ACATCCTGGGAAAAGACATTCGCTCTGAGATGAGCACCATCCGGCAGAACCTGGGGGTCTGTCCCCAGCATAAC
GTGCTGTTTGACATGCTGACTGTCGAAGAACACATCTGGTTCTATGCCCGCTTGAAAGGGCTCTCTGAGAAGCA
CGTGAAGGCGGAGATGGAGCAGATGGCCCTGGATGTTGGTTTGCCATCAAGCAAGCTGAAAAGCAAAACAAGCC
AGCTGTCAGGTGGAATGCAGAGAAAGCTATCTGTGGCCTTGGCCTTTGTCGGGGGATCTAAGGTTGTCATTCTG
GATGAACCCACAGCTGGTGTGGACCCTTACTCCCGCAGGGGAATATGGGAGCTGCTGCTGAAATACCGACAAGG
CCGCACCATTATTCTCTCTACACACCACATGGATGAAGCGGACGTCCTGGGGGACAGGATTGCCATCATCTCCC
ATGGGAAGCTGTGCTGTGTGGGCTCCTCCCTGTTTCTGAAGAACCAGCTGGGAACAGGCTACTACCTGACCTTG
GTCAAGAAAGATGTGGAATCCTCCCTCAGTTCCTGCAGAAACAGTAGTAGCACTGTGTCATACCTGAAAAAGGA
GGACAGTGTTTCTCAGAGCAGTTCTGATGCTGGCCTGGGCAGCGACCATGAGAGTGACACGCTGACCATCGATG
TCTCTGCTATCTCCAACCTCATCAGGAAGCATGTGTCTGAAGCCCGGCTGGTGGAAGACATAGGGCATGAGCTG
ACCTATGTGCTGCCATATGAAGCTGCTAAGGAGGGAGCCTTTGTGGAACTCTTTCATGAGATTGATGACCGGCT
CTCAGACCTGGGCATTTCTAGTTATGGCATCTCAGAGACGACCCTGGAAGAAATATTCCTCAAGGTGGCCGAAG
AGAGTGGGGTGGATGCTGAGACCTCAGATGGTACCTTGCCAGCAAGACGAAACAGGCGGGCCTTCGGGGACAAG
CAGAGCTGTCTTCGCCCGTTCACTGAAGATGATGCTGCTGATCCAAATGATTCTGACATAGACCCAGAATCCAG
AGAGACAGACTTGCTCAGTGGGATGGATGGCAAAGGGTCCTACCAGGTGAAAGGCTGGAAACTTACACAGCAAC
AGTTTGTGGCCCTTTTGTGGAAGAGACTGCTAATTGCCAGACGGAGTCGGAAAGGATTTTTGCTCAGATTGTC

# FIGURE 9B

TTGCCAGCTGTGTTTGTCTGCATTGCCCTTGTGTTCAGCCTGATCGTGCCACCCTTTGGCAAGTACCCCAGCCT
GGAACTTCAGCCCTGGATGTACAACGAACAGTACACATTTGTCAGCAATGATGCTCCTGAGGACACGGGAACCC
TGGAACTCTTAAACGCCCTCACCAAAGACCCTGGCTTCGGGACCCGCTGTATGGAAGGAAACCCAATCCCAGAC
ACGCCCTGCCAGGCAGGGGAGGAAGAGTGGACCACTGCCCCAGTTCCCCAGACCATCATGGACCTCTTCCAGAA
TGGGAACTGGACAATGCAGAACCCTTCACCTGCATGCCAGTGTAGCAGCGACAAAATCAAGAAGATGCTGCCTG
TGTGTCCCCCAGGGGCAGGGGGGCTGCCTCCTCCACAAAGAAAACAAAACACTGCAGATATCCTTCAGGACCTG
ACAGGAAGAAACATTTCGGATTATCTGGTGAAGACGTATGTGCAGATCATAGCCAAAAGCTTAAAGAACAAGAT
CTGGGTGAATGAGTTTAGGTATGGCGGCTTTTCCCTGGGTGTCAGTAATACTCAAGCACTTCCTCCGAGTCAAG
AAGTTAATGATGCCACCAAACAAATGAAGAAACACCTAAAGCTGGCCAAGGACAGTTCTGCAGATCGATTTCTC
AACAGCTTGGGAAGATTTATGACAGGACTGGACACCAGAAATAATGTCAAGGTGTGGTTCAATAACAAGGGCTG
GCATGCAATCAGCTCTTTCCTGAATGTCATCAACAATGCCATTCTCCGGGCCAACCTGCAAAAGGGAGAGAACC
CTAGCCATTATGGAATTACTGCTTTCAATCATCCCCTGAATCTCACCAAGCAGCAGCTCTCAGAGGTGGCTCCG
ATGACCACATCAGTGGATGTCCTTGTGTCCATCTGTGTCATCTTTGCAATGTCCTTCGTCCCAGCCAGCTTTGT
CGTATTCCTGATCCAGGAGCGGGTCAGCAAAGCAAAACACCTGCAGTTCATCAGTGGAGTGAAGCCTGTCATCT
ACTGGCTCTCTAATTTTGTCTGGGATATGTGCAATTACGTTGTCCCTGCCACACTGGTCATTATCATCTTCATC
TGCTTCCAGCAGAAGTCCTATGTGTCCTCCACCAATCTGCCTGTGCTAGCCCTTCTACTTTTGCTGTATGGGTG
GTCAATCACACCTCTCATGTACCCAGCCTCCTTTGTGTTCAAGATCCCCAGCACAGCCTATGTGGTGCTCACCA
GCGTGAACCTCTTCATTGGCATTAATGGCAGCGTGGCCACCTTTGTGCTGGAGCTGTTCACCGACAATAAGCTG
AATAATATCAATGATATCCTGAAGTCCGTGTTCTTGATCTTCCCACATTTTTGCCTGGGACGAGGGCTCATCGA
CATGGTGAAAAACCAGGCAATGGCTGATGCCCTGGAAAGGTTTGGGGAGAATCGCTTTGTGTCACCATTATCTT
GGGACTTGGTGGGACGAAACCTCTTCGCCATGGCCGTGGAAGGGGTGGTGTTCTTCCTCATTACTGTTCTGATC
CAGTACAGATTCTTCATCAGGCCCAGACCTGTAAATGCAAAGCTATCTCCTCTGAATGATGAAGATGAAGATGT
GAGGCGGGAAAGACAGAGAATTCTTGATGGTGGAGGCCAGAATGACATCTTAGAAATCAAGGAGTTGACGAAGA
TATATAGAAGGAAGCGGAAGCCTGCTGTTGACAGGATTTGCGTGGGCATTCCTCCTGGTGAGTGCTTTGGGCTC
CTGGGAGTTAATGGGGCTGGAAAATCATCAACTTTCAAGATGTTAACAGGAGATACCACTGTTACCAGAGGAGA
TGCTTTCCTTAACAGAAATAGTATCTTATCAAACATCCATGAAGTACATCAGAACATGGGCTACTGCCCTCAGT
TTGATGCCATCACAGAGCTGTTGACTGGGAGAGAACACGTGGAGTTCTTTGCCCTTTTGAGAGGAGTCCCAGAG
AAAGAAGTTGGCAAGGTTGGTGAGTGGGCGATTCGGAAACTGGGCCTCGTGAAGTATGGAGAAAAATATGCTGG
TAACTATAGTGGAGGCAACAAACGCAAGCTCTCTACAGCCATGGCTTTGATCGGCGGGCCTCCTGTGGTGTTTC
TGGATGAACCCACCACAGGCATGGATCCCAAAGCCCGGCGGTTCTTGTGGAATTGTGCCCTAAGTGTTGTCAAG
GAGGGGAGATCAGTAGTGCTTACATCTCATAGTATGGAAGAATGTGAAGCTCTTTGCACTAGGATGGCAATCAT
GGTCAATGGAAGGTTCAGGTGCCTTGGCAGTGTCCAGCATCTAAAAAATAGGTTTGGAGATGGTTATACAATAG
TTGTACGAATAGCAGGGTCCAACCCGGACCTGAAGCCTGTCCAGGATTTCTTTGGACTTGCATTTCCTGGAAGT
GTTCCAAAAGAGAAACACCGGAACATGCTACAATACCAGCTTCCATCTTCATTATCTTCTCTGGCCAGGATATT
CAGCATCCTCTCCCAGAGCAAAAAGCGACTCCACATAGAAGACTACTCTGTTTCTCAGACAACACTTGACCAAG
TATTTGTGAACTTTGCCAAGGACCAAAGTGATGATGACCACTTAAAAGACCTCTCATTACACAAAAACCAGACA
GTAGTGGACGTTGCAGTTCTCACATCTTTTCTACAGGATGAGAAAGTGAAAGAAAGCTATGTATGAAGAATCCT
GTTCATACGGGGTGGCTGAAAGTAAAGAGGXACTAGACTTTCCTTTGCACCATGTGAAGTGTTGTGGAGAAAAG
AGCCAGAAGTTGATGTGGGAAGAAGTAAACTGGATACTGTACTGATACTATTCAATGCAATGCAATTCAATGCA
ATGAAAACAAAATTCCATTACAGGGGCAGTGCCTTTGTAGCCTATGTCTTGTATGGCTCTCAAGTGAAAGACTT
GAATTTAGTTTTTTACCTATACCTATGTGAAACTCTATTATGGAACCCAATGGACATATGGGTTTGAACTCACA
CTTTTTTTTTTTTTTTGTTCCTGTGTATTCTCATTGGGGTTGCAACAATAATTCATCAAGTAATCATGGCCAGC
GATTATTGATCAAAATCAAAAGGTAATGCACATCCTCATTCACTAAGCCATGCCATGCCCAGGAGACTGGTTTC
CCGGTGACACATCCATTGCTGGCAATGAGTGTGCCAGAGTTATTAGTGCCAAGTTTTTCAGAAAGTTTGAAGCA
CCATGGTGTGTCATGCTCACTTTTGTGAAAGCTGCTCTGCTCAGAGTCTATCAACATTGAATATCAGTTGACAG
AATGGTGCCATGCGTGGCTAACATCCTGCTTTGATTCCCTCTGATAAGCTGTTCTGGTGGCAGTAACATGCAAC
AAAAATGTGGGTGTCTCTAGGCACGGGAAACTTGGTTCCATTGTTATATTGTCCTATGCTTCGAGCCATGGGTC
TACAGGGTCATCCTTATGAGACTCTTAAATATACTTAGATCCTGGTAAGAGGCAAAGAATCAACAGCCAAACTG
CTGGGGCTGCAAGCTGCTGAAGCCAGGGCATGGGATTAAAGAGATTGTGCGTTCAAACCTAGGGAAGCCTGTGC
CCATTTGTCCTGACTGTCTGCTAACATGGTACACTGCATCTCAAGATGTTTATCTGACACAAGTGTATTATTTC
TGGCTTTTTGAATTAATCTAGAAAATGAAAAGATGGAGTTGTATTTTGACAAAAATGTTTGTACTTTTTAATGT
TATTTGGAATTTTAAGTTCTATCAGTGACTTCTGAATCCTTAGAATGGCCTCTTTGTAGAACCCTGTGGTATAG
AGGAGTATGGCCACTGCCCCACTATTTTTATTTTCTTATGTAAGTTTGCATATCAGTCATGACTAGTGCCTAGA
AAGCAATGTGATGGTCAGGATCTCATGACATTATATTTGAGTTTCTTTCAGATCATTTAGGATACTCTTAATCT
CACTTCATCAATCAAATATTTTTTGAGTGTATGCTGTAGCTGAAAGAGTATGTACGTACGTATAAGACTAGAGA

# FIGURE 9C

GATATTAAGTCTCAGTACACTTCCTGTGCCATGTTATTCAGCTCACTGGTTTACAAATATAGGTTGTCTTGTGG
TTGTAGGAGCCCACTGTAACAATACTGGGCAGCCTTTTTTTTTTTTTTTAATTGCAACAATGCAAAAGCCAAGA
AAGTATAAGGGTCACAAGTCTAAACAATGAATTCTTCAACAGGGAAAACAGCTAGCTTGAAAACTTGCTGAAAA
ACACAACTTGTGTTTATGGCATTTAGTACCTTCAAATAATTGGCTTTGCAGATATTGGATACCCCATTAAATCT
GACAGTCTCAAATTTTTCATCTCTTCAATCACTAGTCAAGAAAAATATAAAAACAACAAATACTTCCATATGGA
GCATTTTTCAGAGTTTTCTAACCCAGTCTTATTTTTCTAGTCAGTAAACATTTGTAAAAATACTGTTTCACTAA
TACTTACTGTTAACTGTCTTGAGAGAAAAGAAAAATATGAGAGAACTATTGTTTGGGGAAGTTCAAGTGATCTT
TCAATATCATTACTAACTTCTTCCACTTTTTCCAAAATTTGAATATTAACGCTAAAGGTGTAAGACTTCAGATT
TCAAATTAATCTTTCTATATTTTTTAAATTTACAGAATATTATATAACCCACTGCTGAAAAAGAAAAAAATGAT
TGTTTTAGAAGTTAAAGTCAATATTGATTTTAAATATAAGTAATGAAGGCATATTTCCAATAACTAGTGATATG
GCATCGTTGCATTTTACAGTATCTTCAAAAATACAGAATTTATAGAATAATTTCTCCTCATTTAATATTTTTCA
AAATCAAAGTTATGGTTTCCTCATTTTACTAAAATCGTATTCTAATTCTTCATTATAGTAAATCTATGAGCAAC
TCCTTACTTCGGTTCCTCTGATTTCAAGGCCATATTTTAAAAAATCAAAAGGCACTGTGAACTATTTTGAAGAA
AACACAACATTTTAATACAGATTGAAAGGACCTCTTCTGAAGCTAGAAACAATCTATAGTTATACATCTTCATT
AATACTGTGTTACCTTTTAAAATAGTAATTTTTTACATTTTCCTGTGTAAACCTAATTGTGGTAGAAATTTTTA
CCAACTCTATACTCAATCAAGCAAAATTTCTGTATATTCCCTGTGGAATGTACCTATGTGAGTTTCAGAAATTC
TCAAAATACGTGTTCAAAAATTTCTGCTTTTGCATCTTTGGGACACCTCAGAAAACTTATTAACAACTGTGAAT
ATGAGAAATACAGAAGAAAATAATAAGCCCTCTATACATAAATGCCCAGCACAATTCATTGTTAAAAAACAACC
AAACCTCACACTACTGTATTTCATTATCTGTACTGAAAGCAAATGCTTTGTGACTATTAAATGTTGCACATCAT
TCATTCACTGTATAGTAATCATTGACTAAAGCCATTTGTCTGTGTTTTCTTCTTGTGGTTGTATATATCAGGTA
AAATATTTTCCAAAGAGCCATGTGTCATGTAATACTGAACCACTTTGATATTGAGACATTAATTTGTACCCTTG
TTATTATCTACTAGTAATAATGTAATACTGTAGAAATATTGCTCTAATTCTTTTCAAAATTGTTGCATCCCCCT
TAGAATGTTTCTATTTCCATAAGGATTTAGGTATGCTATTATCCCTTCTTATACCCTAAGATGAAGCTGTTTTT
GTGCTCTTTGTTCATCATTGGCCCTCATTCCAAGCACTTTACGCTGTCTGTAATGGGATCTATTTTTGCACTGG
AATATCTGAGAATTGCAAAACTAGACAAAAGTTTCACAACAGATTTCTAAGTTAAATCATTTTCATTAAAAGGA
AAAAAGAAAAAAAATTTTGTATGTCAATAACTTTATATGAAGTATTAAAATGCATATTTCTATGTTGTAATATA
ATGAGTCACAAAATAAAGCTGTGACAGTTCTGTTGGTCTACAGAAATTTACTTTTGTGCATTTGTGGCACCACC
TACTGTTGAAGGGTTATAAAGCCATTAGAAAAGTAGAGGGGAAGTGATTTGGATCAAAAGGAAAAACTTTAGAA
AAGATTCAGATGTTCCCTTAATCATAAAAGAGAACTGAGGGGACTACTTGAAAATAAAAGGTTGTTTTGTATTT
TCATGTTGGTTAAGATACTGAGTAACTGGTATTAAGTGTTAGAGGTTTTTAGATAAATATTCTGCTTAATGATT
ATGAAGCTGCACTGAGATTTCTGAAAATGCTCTGTAGCTGAGCTTATTTAATAAATGTTCACTTGGTATAGGGG
AAGCTACAAAGGCAGCCTTCAGTGTCCTTTTGTTTATTCAACCAAAAATATAAGGACACAATGTAGCAGTTATA
CTGGGAAGGTGCTGGGGGTGGTGGCAATGGTGAGCAGGAAGGCG

# FIGURE 10

CAGACCCCGACCCCGACCCGGACCCCGAGCCTGCCGGCGGCTCCCGTCCCGGCCCCGCGGTCCCCGGGCTCCGC
GCCCTGCTGCCGGCGCGGGCTTTCCTCTGCTCTCTCAAAGGCCGCCTCCTGCTGGCCGAGTCGGGTCTCTCATT
CATCACTTTTATCTGCTATGTGGCGTCCTCAGCATCTGCCTTCCTCACAGCGCCTCTGCTGGAGTTCCTGCTGG
CCTTGTACTTCCTCTTTGCTGATGCCATGCAGCTGAATGACAAGTGGCAGGGCTTGTGCTGGCCCATGATGGAC
TTCCTGCGCTGTGTCACCGCGGCCCTCATCTACTTTGCTATCTCCATCACGGCCATCGCCAAGTACTCGGATGG
GGCTTCCAAAGCCGCTGGGGTGTTTGGCTTCTTTGCTACCATCGTGTTTGCAACTGATTTCTACCTGATCTTTA
ACGACGTGGCCAAATTCCTCAAACAAGGGGACTCTGCAGATGAGACCACAGCCCACAAGACAGAAGAAGAGAAT
TCCGACTCGGACTCTGAC<u>TGA</u>AGGCCTGGCGGGTGCCTTGGCAACCTGAGCCACACAGGCCTCCACCCCTGTGC
CTCACAGGGGTCGCTGGCGTTGGAGCGGAGGCCTGGACTTCTGAGTTGCAGAGGGGGCTGCGGACACAGCAGGC
CCCCTACAGCCTCAGGTTCTGCCTGAGCCCAGCCTACCAGGCTTGCCCCTCAGCTCAGCACTGTTGACCACGCT
GCGTATGAGGGCATCTTGGGTATCCCACTCCTTCTCCCCATTTCTGTCCCACAGGCCTTCAGCCCTTTAACGTC
TCTGCCAAAAACCAGCACAAGGAGACAAAGCAGAGCCTTGTCTGTATCTGGGCAGCAGGTGTTCCATGCTGCTA
GGTGGCGGGGGTCGGGGGTCTTCTGTTTCACTAACAGGAACAAAGACAGAAACCATGACAGGGCTGCCCCGCCA
GGCCCCGGTGGGTTTGTCTGCACTTGGTGCTCCTGCCCACACCAGCCACTTTGGTGACAATGACCCTTCCAAGA
ATCTTTGGTTCAAGGAGCACCAGTTCCCTCTTCATTCTTGAAGCAGGGAGAAATTGACCTTTGCCTTGTCGCCC
AGGAAGTGGGGCTCGGCACCCATAACTAACACCTCCCACCCTTGGAAACCATGTCTTCTGGGGGTGAGATGACC
ATTCTGGGTCTAAGACTGTTTCAAAGAAGAGCTCATAGACTGACTGGTCCAGAAGACAGAGGGTACAACAGTGG
CATCACAGTGACAGTGTCATGGGGAGCTGGGCGGGCCCAGCCAAACCCTCCTTCTTCCTAGAGCCCAGCCAGCA
GGCAGGAGTTCCTGGACCCTCAGGACAGTGAACTTCCAGACCTCAGGGCAGGTCTATGGGCCACTGCAGGAGAT
GAGACCAGCCTTCTGTGTTCACCTAACGATTTATACTGTGTATCTGTCTTTGATGGAATTTTGTAACTTTTTAT
ATTTTTTTATGCAAAAGCAGCTTCTTAACAGATGGCATTTTCTGTGACTCTAGGCCTCACAAAAGAGCCAGAGT
TCTGGACCCATGTTTGGAGCATTTGTAGCCTTATTCTCTTGCGTGTGAATCTCTTACCCTGAAAAAAAGCCATA
ATGAATTAAGCCAGACTGACCACTTGCTTGGAGTGTGTGCTTGAAAAAACCAGAGCAATACTGTTGGGTATTGT
ATCAGGCTTCAGTACAAACTGGTAACACCAATGTGGATCCTGACAGCTTTCAGTTTTAGCAAAAATACACGTGA
AATCTGAAAAAAAAAAAA

# FIGURE 11

TCGGCCGAGATGTCTCGCTCCGTGGCCTTAGCTGTGCTCGCGCTACTCTCTCTTTCTGGCCTGGAGGCTATCCA
GCGTACTCCAAAGATTCAGGTTTACTCACGTCATCCAGCAGAGAATGGAAAGTCAAATTTCCTGAATTGCTATG
TGTCTGGGTTTCATCCATCCGACATTGAAGTTGACTTACTGAAGAATGGAGAGAGAATTGAAAAAGTGGAGCAT
TCAGACTTGTCTTTCAGCAAGGACTGGTCTTTCTATCTCTTGTACTACACTGAATTCACCCCCACTGAAAAAGA
TGAGTATGCCTGCCGTGTGAACCATGTGACTTTGTCACAGCCCAAGATAGTTAAGTGGGATCGAGACATGTAAG
CAGCATCATGGAGGTTTGAAGATGCCGCATTTGGATTGGATGAATTCCAAATTCTGCTTGCTTGCTTTTTAATA
TTGATATGCTTATACACTTACACTTTATGCACAAAATGTAGGGTTATAATAATGTTAACATGGACATGATCTTC
TTTATAATTCTACTTTGAGTGCTGTCTCCATGTTTGATGTATCTGAGCAGGTTGCTCCACAGGTAGCTCTAGGA
GGGCTGGCAACTTAGAGGTGGGGAGCAGAGAATTCTCTTATCCAACATCAACATCTTGGTCAGATTTGAACTCT
TCAATCTCTTGCACTCAAAGCTTGTTAAGATAGTTAAGCGTGCATAAGTTAACTTCCAATTTACATACTCTGCT
TAGAATTTGGGGGAAAATTTAGAAATATAATTGACAGGATTATTGGAAATTTGTTATAATGAATGAAACATTTT
GTCATATAAGATTCATATTTACTTCTTATACATTTGATAAAGTAAGGCATGGTTGTGGTTAATCTGGTTTATTT
TTGTTCCACAAGTTAAATAAATCATAAAACTTGAAAAAAAAAAAAAAAAA

# FIGURE 12

AGCTGGCTCAGGGCGTCCGCTAGGCTCGGACGACCTGCTGAGCCTCCCAAACCGCTTCCATAAGGCTTTGCTTT
CCAACTTCAGCTACAGTGTTAGCTAAGTTTGGAAAGAAGGAAAAAAGAAAATCCCTGGGCCCCTTTTCTTTTGT
TCTTTGCCAAAGTCGTCGTTGTAGTCTTTTTGCCCAAGGCTGTTGTGTTTTTAGAGGTGCTATCTCCAGTTCCT
TGCACTCCTGTTAACAAGCACCTCAGCGAGAGCAGCAGCAGCGATAGCAGCCGCAGAAGAGCCAGCGGGGTCGC
CTAGTGTCATGACCAGGGCGGGAGATCACAACCGCCAGAGAGGATGCTGTGGATCCTTGGCCGACTACCTGACC
TCTGCAAAATTCCTTCTCTACCTTGGTCATTCTCTCTCTACTTGGGGAGATCGGATGTGGCACTTTGCGGTGTC
TGTGTTTCTGGTAGAGCTCTATGGAAACAGCCTCCTTTTGACAGCAGTCTACGGGCTGGTGGTGGCAGGGTCTG
TTCTGGTCCTGGGAGCCATCATCGGTGACTGGGTGGACAAGAATGCTAGACTTAAAGTGGCCCAGACCTCGCTG
GTGGTACAGAATGTTTCAGTCATCCTGTGTGGAATCATCCTGATGATGGTTTTCTTACATAAACATGARCTTCT
GACCATGTACCATGGATGGGTTCTCACTTCCTGCTATATCCTGATCATCACTATTGCAAATATTGCAAATTTGG
CCAGTACTGCTACTGCAATCACAATCCAAAGGGATTGGATTGTTGTTGTTGCAGGAGAAGACAGAAGCAAACTA
GCAAATATGAATGCCACAATACGAAGGATTGACCAGTTAACCAACATCTTAGCCCCCATGGCTGTTGGCCAGAT
TATGACATTTGGCTCCCCAGTCATCGGCTGTGGCTTTATTTCGGGATGGAACTTGGTATCCATGTGCGTGGAGT
ACGTCCTGCTCTGGAAGGTTTACCAGAAAACCCCAGCTCTAGCTGTGAAAGCTGGTCTTAAAGAAGAGGAAACT
GAATTGAAACAGCTGAATTTACACAAAGATACTGAGCCAAAACCCCTGGAGGGAACTCATCTAATGGGTGTGAA
GGACTCTAACATCCATGAGCTTGAACATGAGCAAGAGCCTACTTGTGCCTCCCAGATGGCTGAGCCCTTCCGTA
CCTTCCGAGATGGATGGGTCTCCTACTACAACCAGCCTGTGTTTCTGGCTGGCATGGGTCTTGCTTTCCTTTAT
ATGACTGTCCTGGGCTTTGACTGCATCACCACAGGGTACGCCTACACTCAGGGACTGAGTGGTTCCATCCTCAG
TATTTTGATGGGAGCATCAGCTATAACTGGAATAATGGGAACTGTAGCTTTTACTTGGCTACGTCGAAAATGTG
GTTTGGTTCGGACAGGTCTGATCTCAGGATTGGCACAGCTTTCCTGTTTGATCTTGTGTGTGATCTCTGTATTC
ATGCCTGGAAGCCCCCTGGACTTGTCCGTTTCTCCTTTTGAAGATATCCGATCAAGGTTCATTCAAGGAGAGTC
AATTACACCTACCAAGATACCTGAAATTACAACTGAAATATACATGTCTAATGGGTCTAATTCTGCTAATATTG
TCCCGGAGACAAGTCCTGAATCTGTGCCCATAATCTCTGTCAGTCTGCTGTTTGCAGGCGTCATTGCTGCTAGA
ATCGGTCTTTGGTCCTTTGATTTAACTGTGACACAGTTGCTGCAAGAAAATGTAATTGAATCTGAAAGAGGCAT
TATAAATGGTGTACAGAACTCCATGAACTATCTTCTTGATCTTCTGCATTTCATCATGGTCATCCTGGCTCCAA
ATCCTGAAGCTTTTGGCTTGCTCGTATTGATTTCAGTCTCCTTTGTGGCAATGGGCCACATTATGTATTTCCGA
TTTGCCCAAAATACTCTGGGAAACAAGCTCTTTGCTTGCGGTCCTGATGCAAAAGAAGTTAGGAAGGAAAATCA
AGCAAATACATCTGTTGTTTGAGACAGTTTAACTGTTGCTATCCTGTTACTAGATTATATAGAGCACATGTGCT
TATTTTGTACTGCAGAATTCCAATAAATGGCTGGGTGTTTTGCTCTGTTTTTACCACAGCTGTGCCTTGAGAAC
TAAAAGCTGTTTAGGAAACCTAAGTCAGCAGAAATTAACTGATTAATTTCCCTTATGTTGAGGCATGGRAAAAA
AATTGGRAAAAGGAAAAACTCAGTTTTAAATACGGGAGACTATAATGGATAACACTGRATTCCCCTATTTCTCA
TGAGTAGATACAATCTTACGTAAAAGAGTGGTTAGTCACGTGAATTCAGTTATCATTTGACAGATTCTTATCTG
TACTAGAATTCAGATATGTCAGTTTTCTGCAAAACTCACTCTTGTTCAAGACTAGCTAATTTATTTTTTTGCATC

# FIGURE 13

CTTCCCCTTCTCTGCCCTGCTCCAGGCACCAGGCTCTTTCCCCTTCAGTGTCTCAGAGGAGGGGACGGCAGCAC
CATGGACCCCCGCTTGTCCACTGTCCGCCAGACCTGCTGCTGCTTCAATGTCCGCATCGCAACCACCGCCCTGG
CCATCTACCATGTGATCATGAGCGTCTTGTTGTTCATCGAGCACTCAGTAGAGGTGGCCCATGGCAAGGCGTCC
TGCAAGCTCTCCCAGATGGGCTACCTCAGGATCGCTGACCTGATCTCCAGCTTCCTGCTCATCACCATGCTCTT
CATCATCAGCCTGAGCCTACTGATCGGCGTAGTCAAGAACCGGGAGAAGTACCTGCTGCCCTTCCTGTCCCTGC
AAATCATGGACTATCTCCTGTGCCTGCTCACCCTGCTGGGCTCCTACATTGAGCTGCCCGCCTACCTCAAGTTG
GCCTCCCGGAGCCGTGCTAGCTCCTCCAAGTTCCCCCTGATGACGCTGCAGCTGCTGGACTTCTGCCTGAGCAT
CCTGACCCTCTGCAGCTCCTACATGGAAGTGCCCACCTATCTCAACTTCAAGTCCATGAACCACATGAATTACC
TCCCCAGCCAGGAGGATATGCCTCATAACCAGTTCATCAAGATGATGATCATCTTTTCCATCGCCTTCATCACT
GTCCTTATCTTCAAGGTCTACATGTTCAAGTGCGTGTGGCGGTGCTACAGATTGATCAAGTGCATGAACTCGGT
GGAGGAGAAGAGAAACTCCAAGATGCTCCAGAAGGTGGTCCTGCCGTCCTACGAGGAAGCCCTGTCTTTGCCAT
CGAAGACCCCAGAGGGGGGCCCAGCACCACCCCCATACTCAGAGGTGTGACCCTCGCCAGGCCCCAGCCCCAGT
GCTGGGAGGGGTGGAGCTGCCTCATAATCTGCTTTTTTGCTTTGGTGGCCCCTGTGGCCTGGGTGGGCCCTCCC
GCCCCTCCCTGGCAGGACAATCTGCTTGTGTCTCCCTCGCTGGCCTGCTCCTCCTGCAGGGCCTGTGAGCTGCT
CACAACTGGGTCAACGCTTTAGGCTGAGTCACTCCTCGGGTCTCTCCATAATTCAGCCCAACAATGCTTGGTTT
ATTTCAATCAGCTCTGACACTTGTTTAGACGATTGGCCATTCTAAAGTTGGTGAGTTTGTCAAGCAACTATCGA
CTTGATCAGTTCAGCCAAGCAACTGACAAATCAAAAACCCACTTGTCAGTTCAGTAAAATAATTTGGTCAAACA
ACAGTCTATTGCATTGATTTATAAATAGTTGTCAGTTCACATAGCAATTTAATCAAGTAATCATTAATTAGTTA
CCCCCTATATATAAATATATGTAATCAATTTCTTCAAATAGCTTGCTTACATGATAATCAATTAGCCAACCATG
AGTCATTTAGAATAGTGATAAATAGAATACACAGAATAGTGATGAAATTCAATTTAAAAAATCACGTTAGCCTC
CAAACCATTTAATTCAAATGAACCCATCAACTGGATGCCAACTCTGGCGAATGTAGGACCTCTGAGTGGCTGTA
TAATTGTTAATTCAAATGAAATTCATTTAAACAGTTGACAAACTGTCATTCAACAATTAGCTCCAGGAAATAAC
AGTTATTTCATCATAAAACAGTCCCTTCAAACACACAATTGTTCTGCTGAAGAGTTGTCATCAACAATCCAATG
CTCACCTATTCAGTTGCTCTGTGGTCAGTGTGGCTGCATAGCAGTGGATTCCATGAAAGGAGTCATTTTAGTGA
TGAGCTGCCAGTCCATTCCCAGGCCAGGCTGTCGCTGGCCATCCATTCAGTCGATTCAGTCATAGGCGAATCTG
TTCTGCCCGAGGCTTGTGGTCAAGCAAAAATTCAGCCCTGAAATCAGGCACATCTGTTCGTTGGACTAAACCCA
CAGGTTAGTTCAGTCAAAGCAGGCAACCCCCCTTGTGGGCACTGACCCTGCCACTGGGGTCATGGCGGTTGTGGC
AGCTGGGGAGGTTTGGCCCCAACAGCCCTCCTGTGCCTGCTTCCCTGTGTGTCGGGGTCCTCCAGGGAGCTGAC
CCAGAGGTGGAGGCCACGGAGGCAGGGTCTCTGGGGACTGTCGGGGGGTACAGAGGGAGAAGGCTCTGCAAGAG
CTCCCTGGCAATACCCCCTTGTGTAATTGCTTTGTGTGCGACAGGGAGGAAGTTTCAATAAAGCAACAACAAGC
TTCAAGGAATTC

# FIGURE 14A

GGGAAAGCGAGGAGCCGCGGCGGCGTGGAGCCGGCGGGCCCGGGCGGGGGCTCCCCGGAGCCCTACCACCCCAC
CCTGGGCATCTACGCCCGCTGCATCCGGAACCCAGGGGTGCAGCACTTCCAGCGGGACACGCTGTGCGGGCCCT
ACGCCGAGAGCTTCGGCGAGATCGCCAGCGGCTTCTGGCAGGCCACAGCTATTTTCCTGGCTGTGGGAATCTTT
ATTCTCTGCATGGTGGCCTTGGTGTCCGTCTTCACCATGTGTGTACAGAGCATCATGAAGAAAAGCATCTTCAA
TGTCTGTGGGCTGTTGCAAGGAATTGCAGGTCTATTCCTTATCCTCGGTTTGATACTCTACCCTGCTGGCTGGG
GTTGCCAGAAGGCCATAGACTACTGTGGACATTATGCATCTGCCTACAAACCTGGAGACTGCTCCTTGGGCTGG
GCCTTTTATACCGCCATTGGGGGCACAGTCCTCACTTTCATCTGTGCTGTCTTCTCTGCACAAGCAGAAATTGC
AACCTCTAGTGACAAAGTACAGGAAGAAATTGAAGAGGGGAAAAACCTGATCTGCCTCCTTTAGTTTGGAAGAG
ACAATGCCATTTTCTCCCTTGAGTAATCTTGTGAAACAGTCCACAGTTTCATCATTTGAGTCAAGTGGAGAACT
AACCTTTACCTACCAAAGCCACGTTCCACGGCCCGAGGCTTAAACAGGACCAATGAGAGGCCACATCCAGCTAC
GCAAAGTTACTGGACATGCGGTCTGCAGTGCACATTATAAGGAATGGAACATGAAAATAGTATATAATCCTAGA
CCTGGAGTTGCCAAGTTCTGTCAGACTCCATCTCCCCCAGGTTCAATGATGGATGATAATCTAAATCATTAGGG
CAGCAGTTTCTCTGGTAACGGAAGAGACCGTCCGCCAGATCTGCAGGCTGTTTCTGCTCCAACACTGCTTGCTT
GTGAGCATCTCTGCCTCAGAATGGGGTTTTGGGTTGGAGTTCTTGTTTTCCTCTGTTCTTTCAAGTTGTCTCCA
ACGAACAGAAAACTATAAACTTACTGGGGACAGGATGTGTGCTAAAGGGCACAGCAAGACACTGTCTTTTGCTT
AGCTGACCAAAGGGGTCAGCAGGGATGGCGTGGAGTCATGCTGTGGAACTTATTCTAGGCTGAATCCTAGGGTA
AGGTGGATCAACTGAACTGTCACTCCAGAGATTTTAGAAATTTGAGTAAAGAAACAATAAGGACCTATACAATC
ATATGAGAACAAAAATATGAAATCTTGCTAGTGAAGACGTATTTTTTCTTCTTCCCAGCAGCCAGGCTAGCACC
AGTTCTGGCCCAGTCTCCTCTTCTTCTGGAGATCACATGTTTTTCTTCTAAGGTTAGGATTGTGCTTTGACTGC
GAAAGGAAACCTCACTGTTTCCTCCTTCCAGGGACTGAGGTCTCCAAGCTAGCTGTGGCTTATGCAGATGTTCA
CTGGGAGGACCTGCCAGAATCTCGGCACTTGGGGGGAGACCTTTACTCCCAGTTTGGTGACCATGCTGTAGTCA
GCTCTATTTCCAATCCCGACAGTAGCAGAATGGCATTCTACAACAAAAAGAAGCTAGTTATGGGAGTTAAGTTT
TTGTAGTTACTGGTGTTGATCCTGAAAGCAGACTGAGATAACATTAAATTGCTGCAACTGAAGAACTGCAGCCA
AGACCTTAATTCCAGGAAAGCACAGAGGACAAAGTTAATTCAAAAAGAGGCGCTAGATCAAGGTCACAGCACTG
CCTACACCTGTTTACAAAAAGAATCAAATACCACTATGAATAAGGATTCAGGGGTTTTTAATCTACTTTCCATA
AATTACCAATATCACTGATTCAGGAAGATAGTATCTCAGAATGACCAGAGCAGCACAGAAACAAGCTACTCTGA
CATTATGGGAGCTTCAAAATTGTATCATGATACAGAAACACTCCTTAGCACTTTAAGAAAGTGAGATGGAACTG
CCAGATTTCTGGAAGGAGAAAAAGTGTAGGTATTTGGGTTCATTAATCTGCTCACTTGAGGACTTTGTTTTGAA
AAAGTACCTTCTGTGGACAAGGTATTGTGCTACCAGCTATACAACCCTGACTTCAGAGTTTGCAACCTTGCCCT
GAGTGAATCATGTTAAAGCTGTCTGAGTCTAAAGCACCGTATCTTGGTGCAGAACAGATAATTATACAGAGATG
GAATGGGACAACCGCAGTTTTACTACATTCTGGTGTTTGGCCTATATGAGAAACCATCTTCTCACAGATTAAGG
GCTAAGGGCAAAAGGGGTGGGAGGTGTGGAACTAGCCTTAATGAGTTTCCCATTCCTGAACCAAAATTCAAAGT
GAGTGAGATGTAAATCCTGTGATTTTGGTGAAGAAAAAAACGGGTATCTTCATAGCAGCCTAGGAAACCTTAAC
CATATCTCTAACACCACACAGAAAGAGGCTGGAGGAGCCACTGGACAAAGCTTCTGTCTCTGTGTGTACATTTA
TAATGTTCTAACCAAGTCTCAAACCTTGATGAAAAACACAAAATTTTTCCATAAACTTATCAGAAGACTCACTT
TTCTTTCTTTCTTGGATAGAGAAACCATTTCTGACACTAGGTTTACAATCTCAGTGTCCTTACAAGTTAAGTC
CTAAGCTCACAGGATCCTCCGAGCATGTCCATCACCTGCTCTTTGGCTAAGGTGGCAGTGTACCTCTAGATCAA
CCTGGGAACAGTCACAAGGGAGTGTGACTTCTTGGCCATAATAAACTCACTCGATAGTGTTTATGTTATTAATC
TGAATGCAACAGAAGACAAAAGCACAGGCATGCACACACACAGAACCCCAAACCACTAAAAACTACCTAAACAC
TGACTTAGTAAATAGTAAAAAGGTAATGTTGGGACTTTTAAACCTTGAATCCATTAGCCAGGCTTGGGATGAAA
GGACCATCTAAAATCATGCTAGTCTAAACCATGCTCTTCCACACAGCTGTTTAAAAACCACTGGGTATGAGGAA
TATGCTAGAAAGAAATGTTAAAAATAGATTGTTGGCTCACACTTATTTTTCTAATAAATAGGACCATTATTACT
ACCAGGAAAGTCTTATTTATTTTGCCTGAAATTGGCTTAAAGAAAGTCTCATGACGGGATGGGATGGGCTGCGC
TTCTCAATGAACTCTGAGGCAGAAATATTTGCCTTGGATTCTGTGGATTCTTTAAACCTGTGTGCTAATAATTC
AAACAATGTTGCATTAATTGTATAAGGGTTTTTGTATAGTTTCAAACATCTGTGGTGTAATGATCTTTGTTAA
ACATATATTCTGTAAAGTGCCATAGTCTTTTTTTATGTGTAGCATATTTAAAAATATATATGTATATTATACAT
ACACAAGTTTGTGTGAAAGATGTGCAATAACAAAGGTGTATGTATGTTTTGTTGTTTTGTTTTGGAAACTGGAC
AGGAGTCAAAACAGGGATGTTTGTTTCTGTTTTGGCAAAGGAGAGTTCCACATTTTTGCCTTCATGGCTTATTC
AGTAACCCATAATTTTAATGCTACACAAATCTTATGTGAAGAAAAGACTGGTATGAAATCATTTTTTCCTGGGT
CTAAAATAATCGCTAGTGTTATGTCAAAGTTAAGCCCGCACGCCAGGCCCAGTTAATGCTAGTCTTTCATGTGA
AATGTGAAGCTGCCATGTTGCCTTTTCTCTTAGTAGGATAACTAGTAGCTGGTACATAATCACTGAGGAGCTAT
TTCTTAACATGCTTTTATAGACCATGCTAATGCTAGACCAGTATTTAAGGGCTAATCTCACACCTCCTTAGCTG
TAAGAGTCTGGCTTAGAACAGACCTCTCTGTGCAATAACTTGTGGCCACTGGAAATCCCTGGGCCGGCATTTGT
ATTGGGGTTGCAATGACTCCCAAGGGCCAAAAGAGTTAAAGGCACGACTGGGATTTCTTCTGAGACTGTGGTGA

# FIGURE 14B

AACTCCTTCCAAGGCTGAGGGGGTCAGTAGGTGCTCTGGAGGGACTCGGCACCACTTGATATTCAACAGCCACT
TGAGCCAAATATAAAATTGTATTTACAGCTGATGGACTCAATTTGAGCCTTCAAACTTGTAGTTATCCTATTAT
ATTGTAAACTAATACATTGTCTAGCATTGATTTGGTTCCTGTGCATATGTATTTTCACTATGTGCTCCCCTCCC
CAGATCTTAATTAAACCAGATTTTGCAATTC

# FIGURE 15

TTCAGAAGTGTAATTACTTTAAAATACACTACTTCCACTTTTGTAAGTATTTTACATTTATGTATATATTCTAT
AGTGGAAGCAGAAATTCTCTC

# FIGURE 16

CATTTGCTATGAATATTCTCTATAACAAAGCAAGACAAATTTAGCAGCACTTCATTGCATCTGGATGGGGGAGA
GAGCTGGACAATTTCTTGCTAACAAGAG**ATG**GTTAACTGCCCTCACCTCAGCCGTGAATTCTGCACACCTCGCA
TCCGGGGCAACACCTGCTTCTGCTGTGACCTCTACAACTGTGGCAACCGGGTGGAGATCACTGGTGGGTACTAC
GAATACATCGATGTCAGCAGTTGCCAAGATATCATCCACCTCTACCACCTGCTCTGGTCTGCCACCATCCTCAA
CATTGTTGGCCTGTTCCTGGGCATCATCACTGCCGCTGTCCTTGGAGGCTTTAAGGACATGAACCCAACTCTCC
CAGCACTGAACTGTTCTGTTGAAAATACCCATCCAACAGTTTCTTACTATGCTCATCCCCAAGTGGCATCCTAC
AATACCTACTACCATAGCCCTCCTCACCTGCCACCATATTCTGCTTATGACTTTCAGCATTCCGGTGTCTTTCC
ATCCTCCCCTCCCTCTGGACTTTCTGATGAGCCCCAGTCTGCCTCTCCCTCACCCAGCTACATGTGGTCCTCAA
GTGCACCGCCCCGTTACTCTCCACCCTACTATCCACCTTTTGAAAAGCCACCACCTTACAGTCCC**TAA**AGAGGA
ATGCCTGCTGGCTATTGAGATTATTGTGGCTTTTGTATTTCTGCTTCAGTGGAAGTGTGTAGGGTACAAAATTT
AAAGTGTGACTCTTATGCATAAAGTTTTACAATGGCCTGCCAGGCTAGGGAAAGATAGGGACGAAGCTTATTCA
TTATTAGTGCAGAGCAGGGGTGGTCAGGCTGAACGCAGCACAGAAGGGCAGCTCACATTCTCTAAGCAAGACTG
GGGAGCCAGCCCAGCAAGAAGCTTGTTTGGACTTGCATTACCCTATGCTCCACCTCTGTATTCAGCAGAAGTGT
GGTTGCCATCTTTTTCACTTTATGTAAAGGAGTGTTGCCCTCGGGCCCTTGGCAGATTGCCACCCCAGCACCTA
GGTTGAAGCACCTGGTTTATAGGCCCTATCTTTCCCTACCCCTAAAGTCAGTCCCTAAGGACAATTTCCCAGCT
GATGGGGCTACACAGTAGTTCCAATACAGAGAGTTCTGGCTAAGATTTTGTTTGCTTGTGTCTGGATGTTGAAA
AAGACTGCCCGTATCTCTTACTCCTTCCTTCTCTGTGAGTATTGTAAAAATGGCTGTTGTGATCACTCAGCTCA
GCTTTTGTTATTGGTACCTCCTAAAGGGAAAAGTGCAATATTCTTGCATCTTCAGTAGTGGGGAACAGGATGTA
TTGTTCCGGAAACACTGAAATACACAGCAACATGTGAGATGTTTTAAGTAGATCACTTAGGAGACAGTGGTTCT
ACTACATGTTGCATTATTACAAAATACATTTGCTACAGGAGATATAAATCTTATGGTTGTAATTCAGAGTTTAA
AAATGTTATAAATTAGGTTCTTGGGTCGTGATATGAATTGTTACTAATCTTTGTGACTATTTAATCTTCAAATA
TTGTGCTTAACCCCAGCAATCCGCACGTATCCTGCACCCCACCCCAAAAGAGTCATCTGTATTTTAATGCCACT
GGTCTTATCGGTCCTTTTGTCTGTTGAGACCAGTCATGACAGCATTCAAGATTATGAAAGTGTTACAATGCCGC
TTCAAGTCTGCAAAACCTCAAACGTAGCCAACTTGACAAATATTTAAGTGTTACGGCAGATTTAAAATCCATCT
GGCACACCGTGGTAGGTATTTGTACAGTTCTTTTAATTACACATAGCTTTAAACCATCAACCTGATGAGTTTAA
AGCTTTTGCACCCATGCCTTCACTTCAGAATGAACACCTTCATTGTGATCTTATGTTAACCTGAGAATTGATTT
AAAGGAAGATTGATAATCCTATACTTTATAACGTAAAAATACAGGGGCTACAGGAGGGTACCTAATTAGACAGT
TCTCCAAACACAGAACACACACTGGAAAATTTTCCGGCCAATTTTGCTACCTCCCAACTTGATGGATTAGAGGT
AGCGCATATGCTGGTGCTCCCATCTACCTTGTAGACACTTAGCCATCAAGAATCAAGGCACAAGAAGTGCACTC
TCTCATTAACAGTAAATGTTTGCAAGATATTCAGTTTAACTTTCAGCATCATGAATGTTCTTATCCAGATTTTG
AATCCGAAAAACTATAATCCTTTTATGTTATACAAAATTACTATGATTTTTTACAGTTCTGAGCATATTAAAAT
TCTACTGGATTTCAAAAAGAGACTAATACCCAACTGACTAACTAAACAAATATCAACTTGTAATACTCAATGAA
TTTTTTTGCCATTTACATTTGACCGTTGGCTTTAGTGAATGTCCATATTTAATTTTTTAAGGCACCATTACACA
GTTTATCCTACATTTATCACATTTCTTAAAGTGTTAAGATTCTATGGCTCATTTCTATGTATTTTTCTTACTTT
ACAAAATAACCTGAAACAGTATAGATTTTGTAACACTTAATTTGAGCAGCTTTTTTATTACATTGAATTATATA
AAGTGCATGTTACCTTAGAAAAATTAGTATTTGCTGCTTTACTCTTTTGCAAAACATTTGCTGTAATGAATGGA
TTTGTATTTCCAATATGTATCTTGACTGCATTTTGTAATATTTACTGCTTTATTCCTAATTCTGCTTTAAAGTA
CTGAACTGGGCATGAAACATTAAAATATTAATCCAGAAACTGTATAAACTGGATGTTGCTTAAAATCTGTATCA
CTGCCATGTTGAAAATTCAGACTGCTTTTGTGATGTTTCAAATGAATAAAACTATCCTCCCCTCGTT

# FIGURE 17

CCAATCGCCCGGTGCGGTGGTGCAGGGTCTCGGGCTAGTC**ATG**GCGTCCCCGTCTCGGAGACTGCAGACTAAAC
CAGTCATTACTTGTTTCAAGAGCGTTCTGCTAATCTACACTTTTATTTTCTGGATCACTGGCGTTATCCTTCTT
GCAGTTGGCATTTGGGGCAAGGTGAGCCTGGAGAATTACTTTTCTCTTTTAAATGAGAAGGCCACCAATGTCCC
CTTCGTGCTCATTGCTACTGGTACCGTCATTATTCTTTTGGGCACCTTTGGTTGTTTTGCTACCTGCCGAGCTT
CTGCATGGATGCTAAAACTGTATGCAATGTTTCTGACTCTCGTTTTTTTGGTCGAACTGGTCGCTGCCATCGTA
GGATTTGTTTTCAGACATGAGATTAAGAACAGCTTTAAGAATAATTATGAGAAGGCTTTGAAGCAGTATAACTC
TACAGGAGATTATAGAAGCCATGCAGTAGACAAGATCCAAAATACGTTGCATTGTTGTGGTGTCACCGATTATA
GAGATTGGACAGATACTAATTATTACTCAGAAAAAGGATTTCCTAAGAGTTGCTGTAAACTTGAAGATTGTACT
CCACAGAGAGATGCAGACAAAGTAAACAATGAAGGTTGTTTTATAAAGGTGATGACCATTATAGAGTCAGAAAT
GGGAGTCGTTGCAGGAATTTCCTTTGGAGTTGCTTGCTTCCAACTGATTGGAATCTTTCTCGCCTACTGCCWCT
CTCGTGCCATAACAAATAACCAGTATGAGATAGTG**TAA**CCCAATGTATCTGTGGGCCTATTCCTCTCTACCTTT
AAGGACATTTAGGGTCCCCCCTGTGAATTAGAAAGTTGCTTGGCTGGAGAACTGACAACACTACTTACTGATAG
ACCAAAAAACTACACCAGTAGGTTGATTCAATCAAGATGTATGTAGACCTAAAACTACACCAATAGGCTGATTC
AATCAAGATCCGTGCTCGCAGTGGGCTGATTCAATCAAGATGTATGTTTGCTATGTTCTAAGTCCACCTTCTAT
CCCATTCATGTTAGATCGTTGAAACCCTGTATCCCTCTGAAACACTGGAAGAGCTAGTAAATTGTAAATGAAGT

# FIGURE 18

GTGCACTATGGCTCGGGGCTCGCTGCGCCGGTTGCTGCGGCTCCTCGTGCTGGGGCTCTGGCTGGCGTTGCTGC
GCTCCGTGGCCGGGGAGCAAGCGCCAGGCACCGCCCCCTGCTCCCGCGGCAGCTCCTGGAGCGCGGACCTGGAC
AAGTGCATGGACTGCGCGTCTTGCAGGGCGCGACCGCACAGCGACTTCTGCCTGGGCTGCGCTGCAGCACCTCC
TGCCCCCTTCCGGCTGCTTTGGCCCATCCTTGGGGGCGCTCTGAGCCTGACCTTCGTGCTGGGGCTGCTTTCTG
GCTTTTTGGTCTGGAGACGATGCCGCAGGAGAGAGAAGTTCACCACCCCCATAGAGGAGACCGGCGGAGAGGGC
TGCCCAGCTGTGGCGCTGATCCAGTGACAATGTGCCCCCTGCCAGCCGGGGCTCGCCCACTCATCATTCATTCA
TCCATTCTAGAGCCAGTCTCTGCCTCCCAGACGCGGCGGGAGCCAAGCTCCTCCAACCACAAGGGGGGTGGGGG
GCGGTGAATCACCTCTGAGGCCTGGGCCCAGGGTTCAGGGGAACCTTCCAAGGTGTCTGGTTGCCCTGCCTCTG
GCTCCAGAACAGAAAGGGAGCCTCACGCTGGCTCACACAAAACAGCTGACACTGACTAAGGAACTGCAGCATTT
GCACAGGGGAGGGGGGTGCCCTCCTTCCTAGAGGCCCTGGGGGCCAGGCTGACTTGGGGGGCAGACTTGACACT
AGGCCCCACTCACTCAGATGTCCTGAAATTCCACCACGGGGGTCACCCTGGGGGGTTAGGGACCTATTTTTAAC
ACTAGGGGGCTGGCCCACTAGGAGGGCTGGCCCTAAGATACAGACCCCCCCAACTCCCCAAAGCGGGGAGGAGA
TATTTATTTTGGGGAGAGTTTGGAGGGGAGGGAGAATTTATTAATAAAAGAATCTTTAACTTT

# FIGURE 19A

GCTACAATCCATCTGGTCTCCTCCAGCTCCTTCTTTCTGCAACATGGGGAAGAACAAACTCCTTCATCCAAGTC
TGGTTCTTCTCCTCTTGGTCCTCCTGCCCACAGACGCCTCAGTCTCTGGAAAACCGCAGTATATGGTTCTGGTC
CCCTCCCTGCTCCACACTGAGACCACTGAGAAGGGCTGTGTCCTTCTGAGCTACCTGAATGAGACAGTGACTGT
AAGTGCTTCCTTGGAGTCTGTCAGGGGAAACAGGAGCCTCTTCACTGACCTGGAGGCGGAGAATGACGTACTCC
ACTGTGTCGCCTTCGCTGTCCCAAAGTCTTCATCCAATGAGGAGGTAATGTTCCTCACTGTCCAAGTGAAAGGA
CCAACCCAAGAATTTAAGAAGCGGACCACAGTGATGGTTAAGAACGAGGACAGTCTGGTCTTTGTCCAGACAGA
CAAATCAATCTACAAACCAGGGCAGACAGTGAAATTTCGTGTTGTCTCCATGGATGAAAACTTTCACCCCCTGA
ATGAGTTGATTCCACTAGTATACATTCAGGATCCCAAAGGAAATCGCATCGCACAATGGCAGAGTTTCCAGTTA
GAGGGTGGCCTCAAGCAATTTTCTTTTCCCCTCTCATCAGAGCCCTTCCAGGGCTCCTACAAGGTGGTGGTACA
GAAGAAATCAGGTGGAAGGACAGAGCACCCTTTCACCGTGGAGGAATTTGTTCTTCCCAAGTTTGAAGTACAAG
TAACAGTGCCAAAGATAATCACCATCTTGGAAGAAGAGATGAATGTATCAGTGTGTGGCCTATACACATATGGG
AAGCCTGTCCCTGGACATGTGACTGTGAGCATTTGCAGAAAGTATAGTGACGCTTCCGACTGCCACGGTGAAGA
TTCACAGGCTTTCTGTGAGAAATTCAGTGGACAGCTAAACAGCCATGGCTGCTTCTATCAGCAAGTAAAAACCA
AGGTCTTCCAGCTGAAGAGGAAGGAGTATGAAATGAAACTTCACACTGAGGCCCAGATCCAAGAAGAAGGAACA
GTGGTGGAATTGACTGGAAGGCAGTCCAGTGAAATCACAAGAACCATAACCAAACTCTCATTTGTGAAAGTGGA
CTCACACTTTCGACAGGGAATTCCCTTCTTTGGGCAGGTGCGCCTAGTAGATGGGAAAGGCGTCCCTATACCAA
ATAAAGTCATATTCATCAGAGGAAATGAAGCAAACTATTACTCCAATGCTACCACGGATGAGCATGGCCTTGTA
CAGTTCTCTATCAACACCACCAACGTTATGGGTACCTCTCTTACTGTTAGGGTCAATTACAAGGATCGTAGTCC
CTGTTACGGCTACCAGTGGGTGTCAGAAGAACACGAAGAGGCACATCACACTGCTTATCTTGTGTTCTCCCCAA
GCAAGAGCTTTGTCCACCTTGAGCCCATGTCTCATGAACTACCCTGTGGCCATACTCAGACAGTCCAGGCACAT
TATATTCTGAATGGAGGCACCCTGCTGGGGCTGAAGAAGCTCTCCTTTTATTATCTGATAATGGCAAAGGGAGG
CATTGTCCGAACTGGGACTCATGGACTGCTTGTGAAGCAGGAAGACATGAAGGGCCATTTTTCCATCTCAATCC
CTGTGAAGTCAGACATTGCTCCTGTCGCTCGGTTGCTCATCTATGCTGTTTTACCTACCGGGGACGTGATTGGG
GATTCTGCAAAATATGATGTTGAAAATTGTCTGGCCAACAAGGTGGATTTGAGCTTCAGCCCATCACAAAGTCT
CCCAGCCTCACACGCCCACCTGCGAGTCACAGCGGCTCCTCAGTCCGTCTGCGCCCTCCGTGCTGTGGACCAAA
GCGTGCTGCTCATGAAGCCTGATGCTGAGCTCTCGGCGTCCTCGGTTTACAACCTGCTACCAGAAAAGGACCTC
ACTGGCTTCCCTGGGCCTTTGAATGACCAGGACGATGAAGACTGCATCAATCGTCATAATGTCTATATTAATGG
AATCACATATACTCCAGTATCAAGTACAAATGAAAAGGATATGTACAGCTTCCTAGAGGACATGGGCTTAAAGG
CATTCACCAACTCAAAGATTCGTAAACCCAAAATGTGTCCACAGCTTCAACAGTATGAAATGCATGGACCTGAA
GGTCTACGTGTAGGTTTTTATGAGTCAGATGTAATGGGAAGAGGCCATGCACGCCTGGTGCATGTTGAAGAGCC
TCACACGGAGACCGTACGAAAGTACTTCCCTGAGACATGGATCTGGGATTTGGTGGTGGTAAACTCAGCAGGGG
TGGCTGAGGTAGGAGTAACAGTCCCTGACACCATCACCGAGTGGAAGGCAGGGGCCTTCTGCCTGTCTGAAGAT
GCTGGACTTGGTATCTCTTCCACTGCCTCTCTCCGAGCCTTCCAGCCCTTCTTTGTGGAGCTTACAATGCCTTA
CTCTGTGATTCGTGGAGAGGCCTTCACACTCAAGGCCACGGTCCTAAACTACCTTCCCAAATGCATCCGGGTCA
GTGTGCAGCTGGAAGCCTCTCCCGCCTTCCTTGCTGTCCCAGTGGAGAAGGAACAAGCGCCTCACTGCATCTGT
GCAAACGGGCGGCAAACTGTGTCCTGGGCAGTAACCCCAAAGTCATTAGGAAATGTGAATTTCACTGTGAGCGC
AGAGGCACTAGAGTCTCAAGAGCTGTGTGGGACTGAGGTGCCTTCAGTTCCTGAACACGGAAGGAAAGACACAG
TCATCAAGCCTCTGTTGGTTGAACCTGAAGGACTAGAGAAGGAAACAACATTCAACTCCCTACTTTGTCCATCA
GGTGGTGAGGTTTCTGAAGAATTATCCCTGAAACTGCCACCAAATGTGGTAGAAGAATCTGCCCGAGCTTCTGT
CTCAGTTTTGGGAGACATATTAGGCTCTGCCATGCAAAACACACAAAATCTTCTCCAGATGCCCTATGGCTGTG
GAGAGCAGAATATGGTCCTCTTTGCTCCTAACATCTATGTACTGGATTATCTAAATGAAACACAGCAGCTTACT
CCAGAGGTCAAGTCCAAGGCCATTGGCTATCTCAACACTGGTTACCAGAGACAGTTGAACTACAAACACTATGA
TGGCTCCTACAGCACCTTTGGGGAGCGATATGGCAGGAACCAGGGCAACACCTGGCTCACAGCCTTTGTTCTGA
AGACTTTTGCCCAAGCTCGAGCCTACATCTTCATCGATGAAGCACACATTACCCAAGCCCTCATATGGCTCTCC
CAGAGGCAGAAGGACAATGGCTGTTTCAGGAGCTCTGGGTCACTGCTCAACAATGCCATAAAGGGAGGAGTAGA
AGATGAAGTGACCCTCTCCGCCTATATCACCATCGCCCTTCTGGAGATTCCTCTCACAGTCACTCACCCTGTTG
TCCGCAATGCCCTGTTTTGCCTGGAGTCAGCCTGGAAGACAGCACAAGAAGGGGACCATGGCAGCCATGTATAT
ACCAAAGCACTGCTGGCCTATGCTTTTGCCCTGGCAGGTAACCAGGACAAGAGGAAGGAAGTACTCAAGTCACT
TAATGAGGAAGCTGTGAAGAAAGACAACTCTGTCCATTGGGAGCGCCCTCAGAAACCCAAGGCACCAGTGGGGC
ATTTTTACGAACCCCAGGCTCCCTCTGCTGAGGTGGAGATGACATCCTATGTGCTCCTCGCTTATCTCACGGCC
CAGCCAGCCCCAACCTCGGAGGACCTGACCTCTGCAACCAACATCGTGAAGTGGATCACGAAGCAGCAGAATGC
CCAGGGCGGTTTCTCCTCCACCCAGGACACAGTGGTGGCTCTCCATGCTCTGTCCAAATATGGAGCCGCCACAT
TTACCAGGACTGGGAAGGCTGCACAGGTGACTATCCAGTCTTCAGGGACATTTTCCAGCAAATTCCAAGTGGAC
AACAACAATCGCCTGTTACTGCAGCAGGTCTCATTGCCAGAGCTGCCTGGGGAATACAGCATGAAAGTGACAGG

# FIGURE 19B

AGAAGGATGTGTCTACCTCCAGACCTCCTTGAAATACAATATTCTCCCAGAAAAGGAAGAGTTCCCCTTTGCTT
TAGGAGTGCAGACTCTGCCTCAAACTTGTGATGAACCCAAAGCCCACACCAGCTTCCAAATCTCCCTAAGTGTC
AGTTACACAGGGAGCCGCTCTGCCTCCAACATGGCGATCGTTGATGTGAAGATGGTCTCTGGCTTCATTCCCCT
GAAGCCAACAGTGAAAATGCTTGAAAGATCTAACCATGTGAGCCGGACAGAAGTCAGCAGCAACCATGTCTTGA
TTTACCTTGATAAGGTGTCAAATCAGACACTGAGCTTGTTCTTCACGGTTCTGCAAGATGTCCCAGTAAGAGAT
CTCAAACCAGCCATAGTGAAAGTCTATGATTACTACGAGACGGATGAGTTTGCAATCGCTGAGTACAATGCTCC
TTGCAGCAAAGATCTTGGAAATGCT<u>**TGA**</u>AGACCACAAGGCTGAAAAGTGCTTTGCTGGAGTCCTGTTCTCTGAG
CTCCACAGAAGACACGTGTTTTTGTATCTTTAAAGACTTGATGAATAAACACTTTTTCTGGTC

# FIGURE 20

```
CGAAAGATGGCGGCGGAAACGCTGCTGTCCAGTTTGTTAGGACTGCTGCTTCTGGGACTCCTGTTACCCGCAAG
TCTGACCGGCGGTGTCGGGAGCCTGAACCTGGAGGAGCTGAGTGAGATGCGTTATGGGATCGAGATCCTGCCGT
TGCCTGTCATGGGAGGGCAGAGCCAATCTTCGGACGTGGTGATTGTCTCCTCTAAGTACAAACAGCGCTATGAG
TGTCGCCTGCCAGCTGGAGCTATTCACTTCCAGCGTGAAAGGGAGGAGGAAACACCTGCTTACCAAGGGCCTGG
GATCCCTGAGTTGTTGAGCCCAATGAGAGATGCTCCCTGCTTGCTGAAGACAAAGGACTGGTGGACATATGAAT
TCTGTTATGGACGCCACATCCAGCAATACCACATGGAAGATTCAGAGATCAAAGGTGAAGTCCTCTATCTCGGC
TACTACCAATCAGCCTTCGACTGGGATGATGAAACAGCCAAGGCCTCCAAGCAGCATCGTCTTAAACGCTACCA
CAGCCAGACCTATGGCAATGGGTCCAAGTGCGACCTTAATGGGAGGCCCCGGGAGGCCGAGGTTCGGTTCCTCT
GTGACGAGGGTGCAGGTATCTCTGGGGACTACATCGATCGCGTGGACGAGCCCTTGTCCTGCTCTTATGTGCTG
ACCATTCGCACTCCTCGGCTCTGCCCCCACCCTCTCCTCCGGCCCCCACCCAGTGCTGCACCGCAGGCCATCCT
CTGTCACCCTTCCCTACAGCCTGAGGAGTACATGGCCTACGTTCAGAGGCAAGCCGACTCAAAGCAGTATGGAG
ATAAAATCATAGAGGAGCTGCAAGATCTAGGCCCCCAAGTGTGGAGTGAGACCAAGTCTGGGGTGGCACCCCAA
AAGATGGCAGGTGCGAGCCCGACCAAGGATGACAGTAAGGACTCAGATTTCTGGAAGATGCTTAATGAGCCAGA
GGACCAGGCCCCAGGAGGGGAGGAGGTGCCGGCTGAGGAGCAGGACCCAAGCCCTGAGGCAGCAGATTCAGCTT
CTGGTGCTCCCAATGATTTTCAGAACAACGTGCAGGTCAAAGTCATTCGAAGCCCTGCGGATTTGATTCGATTC
ATAGAGGAGCTGAAAGGTGGAACAAAAAAGGGGAAGCCAAATATAGGCCAAGAGCAGCCTGTGGATGATGCTGC
AGAAGTCCCTCAGAGGGAACCAGAGAAGGAAAGGGGTGATCCAGAACGGCAGAGAGAGATGGAAGAAGAGGAGG
ATGAGGATGAGGATGAGGATGAAGATGAGGATGAACGGCAGTTACTGGGAGAATTTGAGAAGGAACTGGAAGGG
ATCCTGCTTCCGTCAGACCGAGACCGGCTCCGTTCGGAGACAGAGAAAGAGCTGGACCCAGATGGGCTGAAGAA
GGAGTCAGAGCGGGATCGGGCAATGCTGGCTCTCACATCCACTCTCAACAAACTCATCAAAAGACTGGAGGAAA
AACAGAGTCCAGAGCTGGTGAAGAAGCACAAGAAAAAGAGGGTTGTCCCCAAAAAGCCTCCCCCATCACCCCAA
CCTACAGGGAAAATTGAGATCAAAATTGTCCGCCCATGGGCTGAAGGGACTGAAGAGGGTGCACGTTGGCTGAC
TGATGAGGACACGAGAAACCTCAAGGAGATCTTCTTCAATATCTTGGTGCCGGGAGCTGAAGAGGCCCAGAAGG
AACGCCAGCGGCAGAAAGAGCTGGAGAGCAATTACCGCCGGGTGTGGGGCTCTCCAGGTGGGGAGGGCACAGGG
GACCTGGACGAATTTGACTTCTGAGACCAACACTACACTTGACCCTTCACGGAATCCAGACTCTTCCTGGACTG
GCTTGCCTCCTCCCCACCTCCCCACCCTGGAACCCCTGAGGGCCAAACAGCAGAGTGGAGCTGAGCTGTGGACC
TCTCGGGCAACTCTGTGGGTGTGGGGGCCCTGGGTGAATGCTGCTGCCCCTGCTGGCAGCCACCTTGAGACCTC
ACCGGGCCTGTGATATTTGCTCTCCTGAACTCTCACTCAATCCTCTTCCTCTCCTCTGTGGCTTTTCCTGTTAT
TGTCCCCTAATGATAGGATATTCCCTGCTGCCTACCTGGAGATTCAGTAGGATCTTTTGAGTGGAGGTGGGTAG
AGAGAGCAAGGAGGGCAGGACACTTAGCAGGCACTGAGCAAGCAGGCCCCCACCTGCCCTTAGTGATGTTTGGA
GTCGTTTTACCCTCTTCTATTGAATTGCCTTGGGATTTCCTTCTCCCTTTCCCTGCCCACCCTGTCCCCTACAA
TTTGTGCTTCTGAGTTGAGGAGCCTTCACCTCTGTTGCTGAGGAAATGGTAGAATGCTGCCTATCACCTCCAGC
ACAATCCCAGTGAAAAAGGTGTGAAGCACCCACCATGTTCTTGAACAATCAGGTTTCTAAATAAACAACTGGAC
CATCAAAAAAAAAAAAAAA
```

# FIGURE 21

GCGGCGGGAGAGGAACGCGCAGCCAGCCTTGGGAAGCCCAGGCCCGGCAGCC<u>ATG</u>GCGGTGGAAGGAGGAATGA
AATGTGTGAAGTTCTTGCTCTACGTCCTCCTGCTGGCCTTTTGCGCCTGTGCAGTGGGACTGATTGCCGTGGGT
GTCGGGGCACAGCTTGTCCTGAGTCAGACCATAATCCAGGGGGCTACCCCTGGCTCTCTGTTGCCAGTGGTCAT
CATCGCAGTGGGTGTCTTCCTCTTCCTGGTGGCTTTTGTGGGCTGCTGCGGGGCCTGCAAGGAGAACTATTGTC
TTATGATCACGTTTGCCATCTTTCTGTCTCTTATCATGTTGGTGGAGGTGGCCGCAGCCATTGCTGGCTATGTG
TTTAGAGATAAGGTGATGTCAGAGTTTAATAACAACTTCCGGCAGCAGATGGAGAATTACCCGAAAAACAACCA
CACTGCTTCGATCCTGGACAGGATGCAGGCAGATTTTAAGTGCTGTGGGGCTGCTAACTACACAGATTGGGAGA
AAATCCCTTCCATGTCGAAGAACCGAGTCCCCGACTCCTGCTGCATTAATGTTACTGTGGGCTGTGGGATTAAT
TTCAACGAGAAGGCGATCCATAAGGAGGGCTGTGTGGAGAAGATTGGGGGCTGGCTGAGGAAAAATGTGCTGGT
GGTAGCTGCAGCAGCCCTTGGAATTGCTTTTGTCGAGGTTTTGGGAATTGTCTTTGCCTGCTGCCTCGTGAAGA
GTATCAGAAGTGGCTACGAGGTGATG<u>TAG</u>GGGTCTGGTCTCCTCAGCCTCCTCATCTGGGGGAGTGGAATAGTA
TCCTCCAGGTTTTTCAATTAAACGGATTATTTTTTCAGACCGAAAAAAAAAAAAAAAAAAAAAAAAAAAAAAAA
AAAAAAAAAAAA

# FIGURE 22

CGCGCCCCCCAGTCCCGCACCCGTTCGGCCCAGGCTAAGTTAGCCCTCACC**ATG**CCGGTCAAAGGAGGCACCAA
GTGCATCAAATACCTGCTGTTCGGATTTAACTTCATCTTCTGGCTTGCCGGGATTGCTGTCCTTGCCATTGGAC
TATGGCTCCGATTCGACTCTCAGACCAAGAGCATCTTCGAGCAAGAAACTAATAATAATAATTCCAGCTTCTAC
ACAGGAGTCTATATTCTGATCGGAGCCGGCGCCCTCATGATGCTGGTGGGCTTCCTGGGCTGCTGCGGGGCTGT
GCAGGAGTCCCAGTGCATGCTGGGACTGTTCTTCGGCTTCCTCTTGGTGATATTCGCCATTGAAATAGCTGCGG
CCATCTGGGGATATTCCCACAAGGATGAGGTGATTAAGGAAGTCCAGGAGTTTTACAAGGACACCTACAACAAG
CTGAAAACCAAGGATGAGCCCCAGCGGGAAACGCTGAAAGCCATCCACTATGCGTTGAACTGCTGTGGTTTGGC
TGGGGGCGTGGAACAGTTTATCTCAGACATCTGCCCCAAGAAGGACGTACTCGAAACCTTCACCGTGAAGTCCT
GTCCTGATGCCATCAAAGAGGTCTTCGACAATAAATTCCACATCATCGGCGCAGTGGGCATCGGCATTGCCGTG
GTCATGATATTTGGCATGATCTTCAGTATGATCTTGTGCTGTGCTATCCGCAGGAACCGCGAGATGGTC**TAG**AG
TCAGCTTACATCCCTGAGCAGGAAAGTTTACCCATGAAGATTGGTGGGATTTTTTGTTTGTTTGTTTTGTTTTG
TTTGTTGTTTGTTGTTTGTTTTTTTGCCACTAATTTTAGTATTCATTCTGCATTGCTAGATAAAAGCTGAAGTT
ACTTTATGTTTGTCTTTTAATGCTTCATTCAATATTGACATTTGTAGTTGAGCGGGGGGTTTGGTTTGCTTGGT
TTATATTTTTCAGTTGTTTGTTTTTGCTTGTTATATTAAGCAGAAATCCTGCAATGAAAGGTACTATATTTGCT
AGACTCTAGACAAGATATTGTACATAAAAGAATTTTTTTGTCTTTAAATAGATACAAATGTCTATCAACTTTAA
TCAAGTTGTAACTTATATTGAAGACAATTTGATACATAATAAAAAATTATGACAATGAAAAAAAAAAAAAAAAA
AAAAAAGG

# FIGURE 23

MERASCLLLLLLPLVHVSATTPEPCELDDEDFRCVCNFSEPQPDWSEAFQCVSAVEVEIHAGGLNLEPFLKRVD
ADADPRQYADTVKALRVRRLTVGAAQVPAQLLVGALRVLAYSRLKELTLEDLKITGTMPPLPLEATGLALSSLR
LRNVSWATGRSWLAELQQWLKPGLKVLSIAQAHSPAFSCEQVRAFPALTSLDLSDNPGLGERGLMAALCPHKFP
AIQNLALRNTGMETPTGVCAALAAAGVQPHSLDLSHNSLRATVNPSAPRCMWSSALNSLNLSFAGLEQVPKGLP
AKLRVLDLSCNRLNRAPQPDELPEVDNLTLDGNPFLVPGTALPHEGSMNSGVVPACARSTLSVGVSGTLVLLQG
ARGFA

Signal sequence.
amino acids 1-20

Transmembrane domain.
amino acids 352-372

N-glycosylation sites.
amino acids 37-40, 151-154, 282-285, 323-326

cAMP- and cGMP-dependent protein kinase phosphorylation site.
amino acids 92-95

N-myristoylation sites.
amino acids 141-146, 211-216, 239-244, 342-347, 347-352, 360-365

Leucine zipper pattern.
amino acids 281-302, 288-309

# FIGURE 24

MARGAALALLLFGLLGVLVAAPDGGFDLSDALPDNENKKPTAIPKKPSAGDDFDLGDAVVDGENDDPRPPNPPK
PMPNPNPNHPSSSGSFSDADLADGVSGGEGKGGSDGGGSHRKEGEEADAPGVIPGIVGAVVVAVAGAISSFIAY
QKKKLCFKENAEQGEVDMESHRNANAEPAVQRTLLEK

Signal sequence.
amino acids 1-20

Transmembrane domain.
amino acids 123-143

cAMP- and cGMP-dependent protein kinase phosphorylation sites.
amino acids 38-41, 45-48

N-myristoylation sites.
amino acids 4-9, 16-21, 98-103, 106-111, 107-112, 125-130, 129-134, 140-145

# FIGURE 25

MGGLEPCSRLLLLPLLLAVSGLRPVQAQAQSDCSCSTVSPGVLAGIVMGDLVLTVLIALAVYFLGRLVPRGRGA
AEAATRKQRITETESPYQELQGQRSDVYSDLNTQRPYYK

Signal sequence.
amino acids 1-21

Transmembrane domain.
amino acids 43-63

N-myristoylation sites.
amino acids 3-8, 41-46, 45-51, 73-78

# FIGURE 26

GQKGERGLPGLQGVIGFPGMQGPEGPQGPPGQKGDTGEPGLPGTKGTRGPPGASGYPGNPGLPGIPGQDGPPGP
PGIPGCNGTKGERGPLGPPGLPGFAGNPGPPGLPGMKGDPGEILGHVPGMLLKGERGFPGIPGTPGPPGLPGLQ
GPVGPPGFTGPPGPPGPPGPPGEKGQMGLSFQGPKGDKGDQGVSGPPGVPGQAQVQEKGDFATKGEKGQKGEPG
FQGMPGVGEKGEPGKPGPRGKPGKDGDKGEKGSPGFPGEPGYPGLIGRQGPQGEKGEAGPPGPPGIVIGTGPLG
EKGERGYPGTPGPRGEPGPKGFPGLPGQPGPPGLPVPGQAGAPGFPGERGEKGDRGFPGTSLPGPSGRDGLPGP
PGSPGPPGQPGYTNGIVECQPGPPGDQGPPGIPGQPGFIGEIGEKGQKGESCLICDIDGYRGPPGPQGPPGEIG
FPGQPGAKGDRGLPGRDGVAGVPGPQGTPGLIGQPGAKGEPGEFYFDLRLKGDKGDPGFPGQPGMPGRAGSPGR
DGHPGLPGPKGSPGSVGLKGERGPPGGVGFPGSRGDTGPPGPPGYGPAGPIGDKGQAGFPGGPGSPGLPGPKGE
PGKIVPLPGPPGAEGLPGSPGFPGPQGDRGFPGTPGRPGLPGEKGAVGQPGIGFPGPPGPKGVDGLPGDMGPPG
TPGRPGFNGLPGNPGVQGQKGEPGVGLPGLKGLPGLPGIPGTPGEKGSIGVPGVPGEHGAIGPPGLQGIRGEPG
PPGLPGSVGSPGVPGIGPPGARGPPGGQGPPGLSGPPGIKGEKGFPGFPGLDMPGPKGDKGAQGLPGITGQSGL
PGLPGQQGAPGIPGFPGSKGEMGVMGTPGQPGSPGPWGAPGLPGEKGDHGFPGSSGPRGDPGLKGDKGDVGLPG
KPGSMDKVDMGSMKGQKGDQGEKGQIGPIGEKGSRGDPGTPGVPGKDGQAGQPGQPGPKGDPGISGTPGAPGLP
GPKGSVGGMGLPGTPGEKGVPGIPGPQGSPGLPGDKGAKGEKGQAGPPGIGIPGLRGEKGDQGIAGFPGSPGEK
GEKGSIGIPGMPGSPGLKGSPGSVGYPGSPGLPGEKGDKGLPGLDGIPGVKGEAGLPGTPGPTGPAGQKGEPGS
DGIPGSAGEKGEPGLPGRGFPGFPGAKGDKGSKGEVGFPGLAGSPGIPGSKGEQGFMGPPGPQGQPGLPGSPGH
ATEGPKGDRGPQGQPGLPGLPGPMGPPG

N-glycosylation site.
amino acids 81-84

N-myristoylation sites.
amino acids 40-45, 43-48, 76-81, 79-84, 287-292, 385-390, 447-452, 477-482, 529-534, 743-748, 899-904, 966-971, 1055-1060, 1112-1117, 1156-1161

Cell attachment sequence.
amino acids 552-554, 872-874, 923-925

Collagen triple helix repeat (20 copies).
amino acids 1-60, 61-120, 143-201, 233-292, 293-351, 363-421, 427-485, 502-561, 564-624, 628-686, 695-754, 769-827, 831-890, 898-956, 957-1016, 1019-1078, 1088-1146, 1153-1211

# FIGURE 27

GERGPPGSPGLQGFPGITPPSNISGAPGDKGAPGIFGLKGYRGPPGPPGSAALPGSKGDTGNPGAPGTPGTKGW
AGDSGPQGRPGVFGLPGEKGPRGEQGFMGNTGPTGAVGDRGPKGPKGDPGFPGAPGTVGAPGIAGIPQKIAIQP
GTVGPQGRRGPPGAPGEIGPQGPPGEPGFRGAPGKAGPQGRGGVSAVPGFRGDEGPIGHQGPIGQEGAPGRPGS
PGLPGMPGRSVSIGYLLVKHSQTDQEPMCPVGMNKLWSGYSLLYFEGQEKAHNQDLGLAGSCLARFSTMPFLYC
NPGDVCYYASRNDKSYWLSTTAPLPMMPVAEDEIKPYISRCSVCEAPAIAIAVHSQDVSIPHCPAGWRSLWIGY
SFLMHTAAGDEGGGQSLVSPGSCLEDFRATPFIECNGGRGTCHYYANKYSFWLTTIPEQSFQGSPSADTLKAGL
IRTHISRCQVCMKNL

N-glycosylation site.
amino acids 22-25

N-myristoylation sites.
amino acids 67-72, 127-132, 190-195, 269-274, 279-284, 282-287, 382-387, 407-412, 433-438, 443-448

Amidation site.
amino acids 154-157

Cell attachment sequence.
amino acids 199-201

C-terminal tandem repeated domain in type 4.
amino acids 236-343, 344-458

Collagen Collagen triple helix repeat (20 copies).
amino acids 25-84, 88-147, 152-211

# FIGURE 28A

SRPWWLRASERPSAPSAMAKRSRGPGRRCLLALVLFCAWGTLAVVAQKPGAGCPSRCLCFRTTVRCMHLLLEAV
PAVAPQTSILDLRFNRIREIQPGAFRRLRNLNTLLLNNNQIKRIPSGAFEDLENLKYLYLYKNEIQSIDRQAFK
GLASLEQLYLHFNQIETLDPDSFQHLPKLERLFLHNNRITHLVPGTFNHLESMKRLRLDSNTLHCDCEILWLAD
LLKTYAESGNAQAAAICEYPRRIQGRSVATITPEELNCERPRITSEPQDADVTSGNTVYFTCRAEGNPKPEIIW
LRNNNELSMKTDSRLNLLDDGTLMIQNTQETDQGIYQCMAKNVAGEVKTQEVTLRYFGSPARPTFVIQPQNTEV
LVGESVTLECSATGHPPPRISWTRGDRTPLPVDPRVNITPSGGLYIQNVVQGDSGEYACSATNNIDSVHATAFI
IVQALPQFTVTPQDRVVIEGQTVDFQCEAKGNPPPVIAWTKGGSQLSVDRRHLVLSSGTLRISGVALHDQGQYE
CQAVNIIGSQKVVAHLTVQPRVTPVFASIPSDTTVEVGANVQLPCSSQGEPEPAITWNKDGVQVTESGKFHISP
EGFLTINDVGPADAGRYECVARNTIGSASVSMVLSVNVPDVSRNGDPFVATSIVEAIATVDRAINSTRTHLFDS
RPRSPNDLLALFRYPRDPYTVEQARAGEIFERTLQLIQEHVQHGLMVDLNGTSYHYNDLVSPQYLNLIANLSGC
TAHRRVNNCSDMCFHQKYRTHDGTCNNLQHPMWGASLTAFERLLKSVYENGFNTPRGINPHRLYNGHALPMPRL
VSTTLIGTETVTPDEQFTHMLMQWGQFLDHDLDSTVVALSQARFSDGQHCSNVCSNDPPCFSVMIPPNDSRARS
GARCMFFVRSSPVCGSGMTSLLMNSVYPREQINQLTSYIDASNVYGSTEHEARSIRDLASHRGLLRQGIVQRSG
KPLLPFATGPPTECMRDENESPIPCFLAGDHRANEQLGLTSMHTLWFREHNRIATELLKLNPHWDGDTIYYETR
KIVGAEIQHITYQHWLPKILGEVGMRTLGEYHGYDPGINAGIFNAFATAAFRFGHTLVNPLLYRLDENFQPIAQ
DHLPLHKAFFSPFRIVNEGGIDPLLRGLFGVAGKMRVPSQLLNTELTERLFSMAHTVALDLAAINIQRGRDHGI
PPYHDYRVYCNLSAAHTFEDLKNEIKNPEIREKLKRLYGSTLNIDLFPALVVEDLVPGSRLGPTLMCLLSTQFK
RLRDGDRLWYENPGVFSPAQLTQIKQTSLARILCDNADNITRVQSDVFRVAEFPHGYGSCDEIPRVDLRVWQDC
CEDCRTRGQFNAFSYHFRGRRSLEFSYQEDKPTKKTRPRKIPSVGRQGEHLSNSTSAFSTRSDASGTNDFREFV
LEMQKTITDLRTQIKKLESRLSTTECVDAGGESHANNTKWKKDACTICECKDGQVTCFVEACPPATCAVPVNIP
GACCPVCLQKRAEEKP

Transmembrane domains.

amino acids 26-46, 62-82

N-glycosylation sites.

amino acids 657-660, 716-719, 736-739, 748-751, 882-887, 1195-1198, 1297-1302, 1385-1388, 1442-1445

Tyrosine kinase phosphorylation sites.

amino acids 1062-1070, 1261-1268, 1352-1359

N-myristoylation sites.

amino acids 231-236, 330-335, 487-492, 515-520, 579-584, 763-768, 774-779, 861-866, 903-908, 1073-1078, 1077-1082, 1223-1228, 1340-1345, 1459-1464

Amidation sites.

amino acids 25-28, 1350-1353

Cell attachment sequence.

amino acids 394-396

Leucine zipper pattern.

amino acids 490-511

Animal haem peroxidase.

amino acids 757-1299

Immunoglobulin domains.

amino acids 277-336, 373-431, 464-521, 556-613

# FIGURE 28B

Leucine Rich Repeats.

amino acids 80-103, 104-127, 128-151, 152-175, 176-199

Leucine rich repeat C-terminal domain.

amino acids 209-261

von Willebrand factor type C domain.

amino acids 1432-1487

# FIGURE 29

MPSAGTLPWVQGIICNANNPCFRYPTPGEAPGVVGNFNKSIVARLFSDARRLLLYSQKDTSMKDMRKVLRTLQQ
IKKSSSNLKLQDFLVDNETFSGFLYHNLSLPKSTVDKMLRADVILHKVFLQGYQLHLTSLCNGSKSEEMIQLGD
QEVSELCGLPREKLAAAERVLRSNMDILKPILRTLNSTSPFPSKELAEATKTLLHSLGTLAQELFSMRSWSDMR
QEVMFLTNVNSSSSSTQIYQAVSRIVCGHPEGGGLKIKSLNWYEDNNYKALFGGNGTEEDAETFYDNSTTPYCN
DLMKNLESSPLSRIIWKALKPLLVGKILYTPDTPATRQVMAEVNKTFQELAVFHDLEGMWEELSPKIWTFMENS
QEMDLVRMLLDSRDNDHFWEQQLDGLDWTAQDIVAFLAKHPEDVQSSNGSVYTWREAFNETNQAIRTISRFMEC
VNLNKLEPIATEVWLINKSMELLDERKFWAGIVFTGITPGSIELPHHVKYKIRMDIDNVERTNKIKDGYWDPGP
RADPFEDMRYVWGGFAYLQDVVEQAIIRVLTGTEKKTGVYMQQMPYPCYVDDIFLRVMSRSMPLFMTLAWIYSV
AVIIKGIVYEKEARLKETMRIMGLDNSILWFSWFISSLIPLLVSAGLLVVILKLGNLLPYSDPSVVFVFLSVFA
VVTILQCFLISTLFSRANLAAACGGIIYFTLYLPYVLCVAWQDYVGFTLKIFASLLSPVAFGFGCEYFALFEEQ
GIGVQWDNLFESPVEEDGFNLTTSVSMMLFDTFLYGVMTWYIEAVFPGQYGIPRPWYFPCTKSYWFGEESDEKS
HPGSNQKRISEICMEEEPTHLKLGVSIQNLVKVYRDGMKVAVDGLALNFYEGQITSFLGHNGAGKTTTMSILTG
LFPPTSGTAYILGKDIRSEMSTIRQNLGVCPQHNVLFDMLTVEEHIWFYARLKGLSEKHVKAEMEQMALDVGLP
SSKLKSKTSQLSGGMQRKLSVALAFVGGSKVVILDEPTAGVDPYSRRGIWELLLKYRQGRTIILSTHHMDEADV
LGDRIAIISHGKLCCVGSSLFLKNQLGTGYYLTLVKKDVESSLSSCRNSSSTVSYLKKEDSVSQSSSDAGLGSD
HESDTLTIDVSAISNLIRKHVSEARLVEDIGHELTYVLPYEAAKEGAFVELFHEIDDRLSDLGISSYGISETTL
EEIFLKVAEESGVDAETSDGTLPARRNRRAFGDKQSCLRPFTEDDAADPNDSDIDPESRETDLLSGMDGKGSYQ
VKGWKLTQQQFVALLWKRLLIARRSRKGFFAQIVLPAVFVCIALVFSLIVPPFGKYPSLELQPWMYNEQYTFVS
NDAPEDTGTLELLNALTKDPGFGTRCMEGNPIPDTPCQAGEEEWTTAPVPQTIMDLFQNGNWTMQNPSPACQCS
SDKIKKMLPVCPPGAGGLPPPQRKQNTADILQDLTGRNISDYLVKTYVQIIAKSLKNKIWVNEFRYGGFSLGVS
NTQALPPSQEVNDATKQMKKHLKLAKDSSADRFLNSLGRFMTGLDTRNNVKVWFNNKGWHAISSFLNVINNAIL
RANLQKGENPSHYGITAFNHPLNLTKQQLSEVAPMTTSVDVLVSICVIFAMSFVPASFVVFLIQERVSKAKHLQ
FISGVKPVIYWLSNFVWDMCNYVVPATLVIIIFICFQQKSYVSSTNLPVLALLLLLYGWSITPLMYPASFVFKI
PSTAYVVLTSVNLFIGINGSVATFVLELFTDNKLNNINDILKSVFLIFPHFCLGRGLIDMVKNQAMADALERFG
ENRFVSPLSWDLVGRNLFAMAVEGVVFFLITVLIQYRFFIRPRPVNAKLSPLNDEDEDVRRERQRILDGGGQND
ILEIKELTKIYRRKRKPAVDRICVGIPPGECFGLLGVNGAGKSSTFKMLTGDTTVTRGDAFLNRNSILSNIHEV
HQNMGYCPQFDAITELLTGREHVEFFALLRGVPEKEVGKVGEWAIRKLGLVKYGEKYAGNYSGGNKRKLSTAMA
LIGGPPVVFLDEPTTGMDPKARRFLWNCALSVVKEGRSVVLTSHSMEECEALCTRMAIMVNGRFRCLGSVQHLK
NRFGDGYTIVVRIAGSNPDLKPVQDFFGLAFPGSVPKEKHRNMLQYQLPSSLSSLARIFSILSQSKKRLHIEDY
SVSQTTLDQVFVNFAKDQSDDDHLKDLSLHKNQTVVDVAVLTSFLQDEKVKESYV

Transmembrane domains.
amino acids 576-596, 623-643, 649-669, 665-685, 692-712, 709-729, 761-781, 1288-1308, 1593-1613, 1637-1657, 1671-1691, 1706-1726, 1741-1761, 1789-1809
N-glycosylation sites.
amino acids 38-41, 91-94, 101-104, 136-139, 184-187, 232-235, 277-280, 289-292, 340-343, 418-421, 429-432, 461-464, 760-763, 1084-1087, 1234-1237, 1393-1396, 1444-1447, 1577-1580, 1720-1723, 1984-1987, 2178-2181
cAMP- and cGMP-dependent protein kinase phosphorylation sites.
amino acids 76-79, 821-824, 979-982, 1991-1994
N-myristoylation sites.
amino acids 12-17, 32-37, 275-280, 395-400, 419-424, 475-480, 480-485, 615-620, 851-856, 858-863, 866-871, 876-881, 960-965, 1178-1183, 1204-1209, 1478-1483, 1718-1723, 1721-1726, 1845-1850, 1875-1880, 1886-1891, 1889-1894, 1983-1988
Cell attachment sequence.
amino acids 1907-1909
ATP/GTP-binding site motif A (P-loop).
amino acids 873-880, 1886-1893
Leucine zipper pattern.
amino acids 162-183, 2120-2141
ABC transporter.
amino acids 866-1047, 1879-2060

# FIGURE 30

DPDPDPDPEPAGGSRPGPAVPGLRALLPARAFLCSLKGRLLLAESGLSFITFICYVASSASAFLTAPLLEFLLA
LYFLFADAMQLNDKWQGLCWPMMDFLRCVTAALIYFAISITAIAKYSDGASKAAGVFGFFATIVFATDFYLIFN
DVAKFLKQGDSADETTAHKTEEENSDSDSD

Signal sequence.
amino acids 1-31

Transmembrane domains.
amino acids 60-80, 95-115, 128-148

N-myristoylation site.
amino acids 123-128

Leucine zipper pattern.
amino acids 26-47

# FIGURE 31

MSRSVALAVLALLSLSGLEAIQRTPKIQVYSRHPAENGKSNFLNCYVSGFHPSDIEVDLLKNGERIEKVEHSDL
SFSKDWSFYLLYYTEFTPTEKDEYACRVNHVTLSQPKIVKWDRDM

Signal sequence.
amino acids 1-20

Immunoglobulins and major histocompatibility complex proteins signature.
amino acids 98-104

Immunoglobulin domain.
amino acids 38-102

# FIGURE 32

MTRAGDHNRQRGCCGSLADYLTSAKFLLYLGHSLSTWGDRMWHFAVSVFLVELYGNSLLLTAVYGLVVAGSVLV
LGAIIGDWVDKNARLKVAQTSLVVQNVSVILCGIILMMVFLHKHELLTMYHGWVLTSCYILIITIANIANLAST
ATAITIQRDWIVVVAGEDRSKLANMNATIRRIDQLTNILAPMAVGQIMTFGSPVIGCGFISGWNLVSMCVEYVL
LWKVYQKTPALAVKAGLKEEETELKQLNLHKDTEPKPLEGTHLMGVKDSNIHELEHEQEPTCASQMAEPFRTFR
DGWVSYYNQPVFLAGMGLAFLYMTVLGFDCITTGYAYTQGLSGSILSILMGASAITGIMGTVAFTWLRRKCGLV
RTGLISGLAQLSCLILCVISVFMPGSPLDLSVSPFEDIRSRFIQGESITPTKIPEITTEIYMSNGSNSANIVPE
TSPESVPIISVSLLFAGVIAARIGLWSFDLTVTQLLQENVIESERGIINGVQNSMNYLLDLLHFIMVILAPNPE
AFGLLVLISVSFVAMGHIMYFRFAQNTLGNKLFACGPDAKEVRKENQANTSVV

Signal sequence.
amino acids 1-38

Transmembrane domains.
amino acids 52-72, 94-114, 130-150, 303-323, 338-358, 372-392, 450-470, 494-514, 510-530

N-glycosylation sites.
amino acids 100-103, 174-177, 434-437, 567-570

N-myristoylation sites.
amino acids 12-17, 65-70, 76-81, 193-198, 267-272, 311-316, 336-341, 339-344, 353-358, 368-373, 373-378, 435-440, 461-466, 490-495, 494-499

Mitochondrial energy transfer proteins signature.
amino acids 404-412

# FIGURE 33

MDPRLSTVRQTCCCFNVRIATTALAIYHVIMSVLLFIEHSVEVAHGKASCKLSQMGYLRIADLISSFLLITMLF
IISLSLLIGVVKNREKYLLPFLSLQIMDYLLCLLTLLGSYIELPAYLKLASRSRASSSKFPLMTLQLLDFCLSI
LTLCSSYMEVPTYLNFKSMNHMNYLPSQEDMPHNQFIKMMIIFSIAFITVLIFKVYMFKCVWRCYRLIKCMNSV
EEKRNSKMLQKVVLPSYEEALSLPSKTPEGGPAPPPYSEV

Transmembrane domains.
amino acids 15-35, 60-80, 97-117, 136-156, 183-203

cAMP- and cGMP-dependent protein kinase phosphorylation site.
amino acids 225-228

N-myristoylation site.
amino acids 83-88

# FIGURE 34

GKARSRGGVEPAGPGGGSPEPYHPTLGIYARCIRNPGVQHFQRDTLCGPYAESFGEIASGFWQATAIFLAVGIF
ILCMVALVSVFTMCVQSIMKKSIFNVCGLLQGIAGLFLILGLILYPAGWGCQKAIDYCGHYASAYKPGDCSLGW
AFYTAIGGTVLTFICAVFSAQAEIATSSDKVQEEIEEGKNLICLL

Transmembrane domains.
amino acids 64-84, 101-121, 146-166

N-myristoylation sites.
amino acids 8-13, 102-107, 124-129, 156-161

# FIGURE 35

MVNCPHLSREFCTPRIRGNTCFCCDLYNCGNRVEITGGYYEYIDVSSCQDIIHLYHLLWSATILNIVGLFLGII

TAAVLGGFKDMNPTLPALNCSVENTHPTVSYYAHPQVASYNTYYHSPPHLPPYSAYDFQHSGVFPSSPPSGLSD

EPQSASPSPSYMWSSSAPPRYSPPYYPPFEKPPPYSP

Transmembrane domain.
amino acids 56-76

N-glycosylation site.
amino acids 93-96

N-myristoylation sites.
amino acids 68-73, 72-77, 136-141

# FIGURE 36

MASPSRRLQTKPVITCFKSVLLIYTFIFWITGVILLAVGIWGKVSLENYFSLLNEKATNVPFVLIATGTVIILL
GTFGCFATCRASAWMLKLYAMFLTLVFLVELVAAIVGFVFRHEIKNSFKNNYEKALKQYNSTGDYRSHAVDKIQ
NTLHCCGVTDYRDWTDTNYYSEKGFPKSCCKLEDCTPQRDADKVNNEGCFIKVMTIIESEMGVVAGISFGVACF
QLIGIFLAYCXSRAITNNQYEIV

Signal sequence.
amino acids 1-42

N-glycosylation site.
amino acids 134-137

Tyrosine kinase phosphorylation site.
amino acids 160-168

N-myristoylation sites.
amino acids 75-80, 78-83, 210-215, 214-219, 226-231

Tetraspanin family.
amino acids 18-237

# FIGURE 37

MARGSLRRLLRLLVLGLWLALLRSVAGEQAPGTAPCSRGSSWSADLDKCMDCASCRARPHSDFCLGCAAAPPAP
FRLLWPILGGALSLTFVLGLLSGFLVWRRCRRREKFTTPIEETGGEGCPAVALIQ

Signal sequence.
amino acids 1-24

Transmembrane domain.
amino acids 77-97

N-myristoylation sites.
amino acids 16-21, 32-37, 39-44, 83-88, 93-98

# FIGURE 38

MGKNKLLHPSLVLLLLVLLPTDASVSGKPQYMVLVPSLLHTETTEKGCVLLSYLNETVTVSASLESVRGNRSLF
TDLEAENDVLHCVAFAVPKSSSNEEVMFLTVQVKGPTQEFKKRTTVMVKNEDSLVFVQTDKSIYKPGQTVKFRV
VSMDENFHPLNELIPLVYIQDPKGNRIAQWQSFQLEGGLKQFSFPLSSEPFQGSYKVVVQKKSGGRTEHPFTVE
EFVLPKFEVQVTVPKIITILEEEMNVSVCGLYTYGKPVPGHVTVSICRKYSDASDCHGEDSQAFCEKFSGQLNS
HGCFYQQVKTKVFQLKRKEYEMKLHTEAQIQEEGTVVELTGRQSSEITRTITKLSFVKVDSHFRQGIPFFGQVR
LVDGKGVPIPNKVIFIRGNEANYYSNATTDEHGLVQFSINTTNVMGTSLTVRVNYKDRSPCYGYQWVSEEHEEA
HHTAYLVFSPSKSFVHLEPMSHELPCGHTQTVQAHYILNGGTLLGLKKLSFYYLIMAKGGIVRTGTHGLLVKQE
DMKGHFSISIPVKSDIAPVARLLIYAVLPTGDVIGDSAKYDVENCLANKVDLSFSPSQSLPASHAHLRVTAAPQ
SVCALRAVDQSVLLMKPDAELSASSVYNLLPEKDLTGFPGPLNDQDDEDCINRHNVYINGITYTPVSSTNEKDM
YSFLEDMGLKAFTNSKIRKPKMCPQLQQYEMHGPEGLRVGFYESDVMGRGHARLVHVEEPHTETVRKYFPETWI
WDLVVVNSAGVAEVGVTVPDTITEWKAGAFCLSEDAGLGISSTASLRAFQPFFVELTMPYSVIRGEAFTLKATV
LNYLPKCIRVSVQLEASPAFLAVPVEKEQAPHCICANGRQTVSWAVTPKSLGNVNFTVSAEALESQELCGTEVP
SVPEHGRKDTVIKPLLVEPEGLEKETTFNSLLCPSGGEVSEELSLKLPPNVVEESARASVSVLGDILGSAMQNT
QNLLQMPYGCGEQNMVLFAPNIYVLDYLNETQQLTPEVKSKAIGYLNTGYQRQLNYKHYDGSYSTFGERYGRNQ
GNTWLTAFVLKTFAQARAYIFIDEAHITQALIWLSQRQKDNGCFRSSGSLLNNAIKGGVEDEVTLSAYITIALL
EIPLTVTHPVVRNALFCLESAWKTAQEGDHGSHVYTKALLAYAFALAGNQDKRKEVLKSLNEEAVKKDNSVHWE
RPQKPKAPVGHFYEPQAPSAEVEMTSYVLLAYLTAQPAPTSEDLTSATNIVKWITKQQNAQGGFSSTQDTVVAL
HALSKYGAATFTRTGKAAQVTIQSSGTFSSKFQVDNNNRLLLQQVSLPELPGEYSMKVTGEGCVYLQTSLKYNI
LPEKEEFPFALGVQTLPQTCDEPKAHTSFQISLSVSYTGSRSASNMAIVDVKMVSGFIPLKPTVKMLERSNHVS
RTEVSSNHVLIYLDKVSNQTLSLFFTVLQDVPVRDLKPAIVKVYDYYETDEFAIAEYNAPCSKDLGNA

Signal sequence.

amino acids 1-23

N-glycosylation sites.

amino acids 55-58, 70-73, 247-250, 396-399, 410-413, 869-872, 991-994, 1424-1427

cAMP- and cGMP-dependent protein kinase phosphorylation sites.

amino acids 115-118, 116-119, 270-273, 491-494, 895-898

Tyrosine kinase phosphorylation sites.

amino acids 387-394, 1019-1025, 1315-1323

N-myristoylation sites.

amino acids 172-177, 292-297, 388-393, 416-421, 485-490, 504-509, 702-707, 777-782, 779-784, 924-929, 1023-1028, 1078-1083, 1084-1089, 1246-1251, 1284-1289, 1371-1376

Amidation site.

amino acids 893-896

TonB-dependent receptor proteins signature 1.

amino acids 1-64

Alpha-2-macroglobulin family thiolester region signature.

amino acids 969-978

# FIGURE 39

MAAETLLSSLLGLLLLGLLLPASLTGGVGSLNLEELSEMRYGIEILPLPVMGGQSQSSDVVIVSSKYKQRYECR
LPAGAIHFQREREEETPAYQGPGIPELLSPMRDAPCLLKTKDWWTYEFCYGRHIQQYHMEDSEIKGEVLYLGYY
QSAFDWDDETAKASKQHRLKRYHSQTYGNGSKCDLNGRPREAEVRFLCDEGAGISGDYIDRVDEPLSCSYVLTI
RTPRLCPHPLLRPPPSAAPQAILCHPSLQPEEYMAYVQRQADSKQYGDKIIEELQDLGPQVWSETKSGVAPQKM
AGASPTKDDSKDSDFWKMLNEPEDQAPGGEEVPAEEQDPSPEAADSASGAPNDFQNNVQVKVIRSPADLIRFIE
ELKGGTKKGKPNIGQEQPVDDAAEVPQREPEKERGDPERQREMEEEEDEDEDEDEDERQLLGEFEKELEGIL
LPSDRDRLRSETEKELDPDGLKKESERDRAMLALTSTLNKLIKRLEEKQSPELVKKHKKKRVVPKKPPPSPQPT
GKIEIKIVRPWAEGTEEGARWLTDEDTRNLKEIFFNILVPGAEEAQKERQRQKELESNYRRVWGSPGGEGTGDL
DEFDF

Signal sequence.
amino acids 1-25

N-glycosylation site.
amino acids 177-180

cAMP- and cGMP-dependent protein kinase phosphorylation site.
amino acids 466-469

Tyrosine kinase phosphorylation sites.
amino acids 86-93, 113-120, 261-268, 571-577

N-myristoylation sites.
amino acids 26-31, 53-58, 199-204, 298-303, 375-380, 532-537, 559-564, 582-587, 585-590

Cell attachment sequence.
amino acids 404-406

# FIGURE 40

MAVEGGMKCVKFLLYVLLLAFCACAVGLIAVGVGAQLVLSQTIIQGATPGSLLPVVIIAVGVFLFLVAFVGCCG
ACKENYCLMITFAIFLSLIMLVEVAAAIAGYVFRDKVMSEFNNNFRQQMENYPKNNHTASILDRMQADFKCCGA
ANYTDWEKIPSMSKNRVPDSCCINVTVGCGINFNEKAIHKEGCVEKIGGWLRKNVLVVAAAALGIAFVEVLGIV
FACCLVKSIRSGYEVM

Signal sequence.
amino acids 1-25

Transmembrane domains.
amino acids 49-69, 84-104, 202-222

N-glycosylation sites.
amino acids 130-133, 150-153, 172-175

N-myristoylation sites.
amino acids 5-10, 46-51, 71-76, 176-181, 178-183, 220-225

Serum albumin family signature.
amino acids 65-90

Tetraspanin family.
amino acids 11-231

# FIGURE 41

MPVKGGTKCIKYLLFGFNFIFWLAGIAVLAIGLWLRFDSQTKSIFEQETNNNNSSFYTGVYILIGAGALMMLVG
FLGCCGAVQESQCMLGLFFGFLLVIFAIEIAAAIWGYSHKDEVIKEVQEFYKDTYNKLKTKDEPQRETLKAIHY
ALNCCGLAGGVEQFISDICPKKDVLETFTVKSCPDAIKEVFDNKFHIIGAVGIGIAVVMIFGMIFSMILCCAIR
RNREMV

Transmembrane domains.
amino acids 14-34, 54-74, 88-108, 193-213

N-glycosylation sites.
amino acids 52-55, 53-56

N-myristoylation sites.
amino acids 5-10, 77-82, 90-95, 154-159, 200-205, 210-215

Tetraspanin family.
amino acids 11-221

# FIGURE 42

AGCCTTCCTGCTCCGAGTCTCTGCACCTCCCTCAGGAGCCTGTCAGCCTGGCCCTCGTGAGAGGGGCGCCAGCC
CAGCAGCCTGCTCTGGGGCACCCTCCCCTACCTGAAGGGCACAGGGTTTCGGGAGTTTTCCACCATGACTATTG
CCCTGCTGGGTTTTGCCATATTCTTGCTCCATTGTGCGACCTGTGAGAAGCCTCTAGAAGGGATTCTCTCCTCC
TCTGCTTGGCACTTCACACACTCCCATTACAATGCCACCATCTATGAAAATTCTTCTCCCAAGACCTATGTGGA
GAGCTTCGAGAAAATGGGCATCTACCTCGCGGAGCCACAGTGGGCAGTGAGGTACCGGATCATCTCTGGGGATG
TGGCCAATGTATTTAAAACTGAGGAGTATGTGGTGGGCAACTTCTGCTTCCTAAGAATAAGGACAAAGAGCAGC
AACACAGCTCTTCTGAACAGAGAGGTGCGAGACAGCTACACCCTCATCATCCAAGCCACAGAGAAGACCTTGGA
GTTGGAAGCTTTGACCCGTGTGGTGGTCCACATXXXXXXXXXXXXXXXXXXXXXXXXXXXGCTGATCTAGGCC
AGAATGCTGAGTTCTATTATGCCTTTAACACAAGGTCAGAGATGTTTGCCATCCATCCCACCAGCGGTGTGGTC
ACTGTGGCTGGGAAGCTTAACGTCACCTGGCGAGGAAAGCATGAGCTCCAGGTGCTAGCTGTGGACCGCATGCG
GAAAATCTCTGAGGGCAATGGGTTTGGCAGCCTGGCTGCACTTGTGGTTCATGTGGAGCCTGCCCTCAGGAAGC
CCCCAGCCATTGCTTCAGTGGTGGTGACTCCACCAGACAGCAATGATGGTACCACCTATGCCACTGTACTGGTC
GATGCAAATAGCTCAGGAGCTGAAGTGGAGTCAGTGGAAGTTGTTGGTGGTGACCCTGGAAAGCACTTCAAAGC
CATCAAGTCTTATGCCCGGAGCAATGAGTTCAGTTTGGTGTCTGTCAAAGACATCAACTGGATGGAGTACCTTC
ATGGGTTCAACCTCAGCCTCCAGGCCAG

# FIGURE 43A

TGACTGCATCACCTGGTCTGTGAATTTTCCATTAGAAGCTTGGTGTGCTGTTAGGTGAAAGACTTGCTCAGCTA
TGCGTCATTGGGTTTTATCAACATATAGGCGAAAAAAATCCTGGTCTCTGAGTGTACAGCTGAGATGAAAATTT
CTTTTATTGGAGGAAGTATTGAGTGTGTGCTCTCAAATGCGGCCTCAGTTGAGTAGTGCATTCCTGAGTTTTGG
AAGCAAATTTGCAAACAATTGAGAGTCGTACAGTGGGTGTTCTAACTGGATTCAGGTTTTTTCTAATGTAATTT
TTTCACACGTAAATTAAAAAGTTTAGAAATGTCACACATAACTTCATAACACTTTATGGAGAAATGGTTGTACT
TTTAATTTTTTTCTTTTTATTTATACTCCAACTGACTGAGCAGAGGTTGTACTTCTAAATAACTTTGTGGAAGT
TTTTAGTACCATAATTTTTATAATTTTCATTCCAGTCCTTTGATATTTATGACAGTACTTCTGAAGCGCTTACT
GAGTGCCGGACACTGTTGTAAGTGCTTTACGGAACTTGACTTTTTTTTTTTTTTGAGACGGACTCTCGCTCTGT
CGCCCAGGCTGGAGTGCAGTGGTGCAGTGGCTCGATCTCGGCTCACTGCCACCTCTCCCTCATGGTTTCAAACA
CTTCTCCTGCCTCAGCCTCCCAGGTAGCCAGGATTATAGCCGCCCGCCACCACTCCCGACTAATTTTATTTTGT
ATGTTCTTTTTTAGTAGAGACGGAGGAGTTTCACCATGTTGGCCAGGCTGGTATCGACCTCCTGACCTCAAGTG
ATGTGTCCATCTCGGCCTCCCAAGGTGCTGGAATTACAGGTGTGAGCCACTGTGCTCGGCCTACCTTTTTTTTT
TGTTTTTTGTTTTTTTGAAAAGGAGTTTCGCTCTTGTCCAGGCTGGAGTATAATGGTGCGATCTCAGCTCACCG
CAATCTCCGCCTCCCAGATTCAAGCGATTCTCCTGCCTCAGCCTCCTCAGGAGCTGGGATTACAGGCGCCCACC
GCCATGCCCGGCTAATTTTTGTATTTTTAGTAGAGACGGGGTTTCACTATATTGGCCAGGCTGGTCTCGAACTG
CTGACCTCAAGTAATCCGCCTGCCTCAGCCTCCCAAAGTGCTGGGATTACAGACGTGATCCACCAGGATCACAC
CAGGCCGCGCCTGGCCTGCTTTCATTTTAAAAGTCAAATTTGTCATCCGCCTCAGTGCTTGTAATCTTTTCTGA
GTGAGATACTGAAATTTGCAGTTTCGTTTTGCTTGCACTTGTTCACTGGACCAGTAGTCACTGTTAAATGTAAA
AGTATCTACTTCCTCTGAAAGTTTTTTATTCCTTTATTTCCTGCCTGGGCTTGTCCTCCACCCTACATGTATGC
GTAGTAGATTTAGTGTTTGTTATCCTAACCTTTAGGTTTAGGGATTGACTGGGTTTCTGACTTTTTATTTGGCC
AATGAGGACGATACAGAAAATGAAGCATTGGTCATTATCACATTTTAACGCTGAAAAAGTAAGAAGGACAACCC
CGGAATAAAATGATATCAGTATCAAGATAAAAGTTTGGAATGGGAGAAAAATTCTCAAAGCCTGAAAGAAAATC
TGTAGTTACTTTTGGTGACGCTGTCCAGTTCCCACAATGTATCATTCCTTATCTGAAACTAGACATCCTCTGCA
GCCAGAAGAACAAGAAGTAGGCATTGACCCCTTGTCCAGTTACTCTAACAAGTCTGGAGGAGATTCAAATAAAA
ATGGAAGAAGAACAAGTTCTACTTTAGACTCTGAAGGGACTTTTAATTCCTATAGGAAAGAATGGAAGAACTA
TTTGTAAACAACAATTACTTGGCAACAATAAGGCAGAAGGGGATTAATGGGCAGCTGAGAAGCAGCAGGTTCCG
CAGCATTTGCTGGAAGCTATTTCTTTGTGTTCTTCCTCAAGACAAAAGTCAATGGATAAGTAGAATTGAAGAAT
TAAGAGCATGGTATAGCAACATTAAAGAAATACATATTACCAACCCGAGGAAGGTTGTTGGCCAACAAGATTTG
ATGATCAATAATCCTCTTTCACAGGATGAAGGGAGTCTTTGGAACAAATTCTTCCAAGATAAAGAACTTCGATC
AATGATTGAACAAGATGTCAAAAGAACGTTTCCTGAAATGCAGTTTTTCCAGCAAGAAAATGTGAGAAAAATTC
TTACAGATGTTCTTTTCTGTTATGCCAGAGAAAACGAGCAGTTGCTTTATAAACAGGGCATGCACGAACTGTTA
GCACCTATAGTCTTTGTCCTTCACTGTGACCACCAAGCTTTTCTACATGCCAGTGAGTCTGCACAGCCCAGTGA
GGAAATGAAAACTGTCTTGAACCCTGAGTATCTGGAACATGATGCCTATGCAGTGTTCTCACAACTTATGGAAA
CTGCTGAACCTTGGTTTTCAACTTTTGAGCATGATGGTCAGAAGGGGAAAGAAACACTGATGACTCCCATTCCC
TTTGCTAGACCACAAGATTTAGGGCCAACAATTGCTATTGTTACTAAAGTCAACCAGATCCAGGATCATCTACT
GAAGAAGCATGATATTGAGCTTTACATGCACTTGAACAGACTAGAAATTGCACCACAGATATATGGGTTAAGGT
GGGTGCGGCTGCTATTTGGACGAGAGTTCCCCCTGCAGGACCTTCTGGTGGTCTGGGATGCCTTGTTTGCAGAC
GGCCTCAGCCTGGGTTTAGTAGATTATATCTTCGTAGCCATGTTACTTTACATCCGAGATGCTTTGATCTCTAG
TAACTACCAGACCTGTCTCGGCCTTCTGATGCATTACCCATTCATCGGGGATGTACACTCACTGATTCTTAAGG
CTCTGTTCCTTAGAGATCCAAAGAGAAATCCAAGACCAGTGACTTATCAATTCCATCCAAATTTAGATTATTAC
AAAGCACGAGGAGCAGACCTCATGAATAAAAGCCGGACCAATGCCAAAGGTGCTCCCCTGAATATAAATAAGGT
CTCTAATAGCCTGATTAATTTTGGAAGAAAGTTGATTTCCCCAGCAATGGCTCCAGGCAGTGCAGGTGGCCCTG
TACCTGGAGGCAACAGCAGTAGCTCCTCCTCTGTTGTAATTCCTACCAGGACCTCAGCAGAGGCCCCAAGCCAT
CACTTGCAACAGCAACAGCAGCAGCAGAGGCTGATGAAATCAGAAAGCATGCCTGTGCAATTGAACAAAGGGCT
AAGTTCTAAAAACATCAGTTCATCTCCAAGCGTTGAGAGTTTGCCTGGAGGAAGAGAATTCACTGGCTCTCCAC
CTTCATCTGCTACTAAAAAAGATTCCTTTTTTAGCAACATCTCACGTTCTCGCTCACACAGCAAAACTATGGGC
AGAAAAGAATCTGAAGAAGAATTAGAAGCCCAAATTTCCTTCCTTCAAGGGCAGTTGAATGACCTGGATGCCAT
GTGCAAATACTGTGCAAAGGTGATGGACACTCATCTTGTAAATATTCAAGATGTGATATTACAAGAAAATTTGG
AAAAAGAAGATCAAATTCTGGTTTCCCTGGCAGGATTAAAACAGATCAAAGACATTCTAAAAGGTTCCCTGCGT
TTTAACCAGAGCCAGCTAGAGGCCGAAGAGAACGAACAGATCACCATTGCGGACAACCACTACTGCTCCAGCGG
CCAGGGCCAGGGCCGAGGCCAAGGCCAGAGCGTTCAAATGTCAGGGGCCATTAAACAGGCCTCTTCAGAAACGC
CAGGGTGCACTGATAGAGGGAATTCCGATGACTTCATCCTGATTTCCAAAGATGATGATGGGAGCAGTGCCAGG
GGCTCCTTCTCCGGCCAGGCCCAGCCTCTTCGCACCCTCAGAAGCACCTCTGGGAAAAGCCAGGCCCCAGTCTG
CTCCCCACTGGTGTTCTCAGATCCACTGATGGGCCCAGCCTCAGCTTCCTCCAGCAACCCCAGCTCCAGTCCTG

# FIGURE 43B

ATGACGACAGCAGCAAGGACTCTGGCTTCACCATTGTGAGTCCCCTGGACATCTGACCACAGTGCCCAGTCCTG
CCCCACAGGGATCTAGCCACCCTTCAGTGGCCCCAAGGCCAGACTGAGGCTCATCCAGTGGAGAACCTTCTTAA
ACCACTGCTTCCTTCCCGGCATGCATTTGGCATTGGTCCAGCCCTTTGAAACCCCTTAGAGAGAAGCATATATG
GCCACAAAGCACAGAGGCTTAGGTTTGCCACATGCAGACAGGGCTTTCTGGGCCCTTACCTAATCCCCACCCGA
CTCTTGCTCTGAGTTAGAGCTGAGTTACGTACCCAGTATCACACTCACAGTTAGAAAAGACCGAATCACAATTT
AGAATCACTTTTCCTCTGTCCCCTTCTCCCCAGCTAAGAATGTGTGGCACCTCCATCAGTTATACTTAGAAGGA
GCAGAAATAGTTATTTTCGTATCTTCTATCCCTCAAAGCATCAGACATGGGAAAATTGGTTTATACCAAGAAAG
CTTCCTCTGTGGAAATCTGTCTCAGCCTACTTTATTCCTGCATTGGGAAGCCATATCGCAGAGCTAAATGCAAT
AGAATGAACCAGAACTAGTGGATTCCAGGGCTGGGGAAAAAAAAAAAAGAAAAAACCTCATTACTGACCTCTC
AAAGTTATAAGGATCTCTGCAAACAGGATCTAAGCTTAGGAATAATATTTAGGTGTGATATAGTGTTAGATTTT
TTTGATGTATTAAAGAATGCATCTCCAATCCTTAGGCCATATCAACTTTGGCCATCAATATCTCTCCTTAAACA
ATTATATTTCACCTTTTAGAATCTTTCATAGCCAGAAAACAAGATTACTGTAAGCCAGTTTTAGCTGCACTGAT
TTCAAAAGATATAAGAATATTACTATCCTTCAAATGGAAAATGCGACCTTGACTTTATGGGATAAACATCTTTC
AGACAGTCAGTTTTCTAGTCAGGTTTCTCTGGTTTCAGAGCTGTATATACCTGTCAACTGAGGAATAAAGGGAA
AAACCCAAGTTCATTCCCACCCAAAGTCAGAATCCCTCATTGGCCTTAAGGTAGCAGTCATAAGACAGAGAATT
GGACCTAGAGTCCCTTCTGTGGGGAATAAGGATACCTAGAGAACATTCCACATGCCAAGAGGATGCAGGATTTC
TACACAACCCCTTCCCTTCTTGGAAGTCAAGTGTAGGTACTGCAGGGCCTGTGCTCAGCTGTGAACCCCGTATC
CTGGGCCCCACTGCCGGGACCGGGTCTGACATGCCAGTGCCTTCCTGGGCTGAGCACAGATTAGAGACTCTCCC
CCTTGTCAGTCAGCACCTTAGGAAACCATGATGGGCACAGAGCATCACATGAGCTGTTTCTCTCCTTAAAGAAG
ATCCCTGGAAAGGATGCTTTTCCTCTCCTTTGCCTGCGCAGGAATTCTAACAGGAGTGGGTGAGGATGGCAGAG
GGACACAGTGCCTGTCTCGCCTCCATCAGGGAGAGCAGCCATGCCAGGGATGACTAGCTCTTTGAGCCTGTCCT
CAGAGGATGGCGAGGCAGCCGGGCAGTGGAGGCCTTCATGGTAACAAATGAAAGCTCAGTATAGAGGAACAGAC
ACTGTTTACGTCCCTCCCACTGCTAACCTTATATATCTCTATAGACAAATGTGATAATGACATGATTTCCCACC
TGCCCTCCAAGAAAATGGTGACTCACTCTCAAGTCAGCTACTGTAGAGAGGGTTCTAATTGGTTCTGCAATTTG
CTCTTAAACTCTAGCAGGGAACTCTCCTCTTACCACATCAGCATGTAAGGTGAATAATAACTCTGGTTTTGCCA
GACAGCAGGTTGTCTGACCTTCAACCACTGGGCAATTGCCTGGCAGATGCACACAGTAGCTCCCTGGCTTCTGG
CTCTGAGTGTTCCTCTCAGCACCTCTGAGTAAGCTGCTGCCAAGCACATATCCCTATGACAACACTTTGTAAAA
GCCGCGGGGCCCCCATACAGCGAGTGACCTTGCAACTGTGCAGGGTTGCCATTGGTCACTTTCTCACCTTGGGA
AGGTGTCAGTGTTTTCAGTTCTAAGGTAAGAGGTGTAGAGCTGTTCCCACCAGGGCTCTGGGACAGACTGGAAA
GGACCACAGACCTGGCCATCCCTGGGCAGCAGGGCCAGTGTCACCTGCTGACCTCTAGTATTTCCTTTGCCCTA
GAGCTAGAGTCATGATAGCTGAGGGTCACTCGCCCTGCAAGAGTCACTAGGCACCCACCATGCCAATAAGGCTC
TCCGCTGGCTCCCTGCAGTTGGCTGGGTGTTTAATAGTCACTGAAAACTCCCAGCCCTGCTGCACACTAGAGGC
AGGTCCTCTCGGTCCTCTCCATCCTGTGCTTCTGTGGCCCCCAGCAAGCTCACCGCCTCCTTGGAGGAGAGAGA
CATACAAGGACAGTGGGTCATGGGTAGTACCAGCCTCAAATTCCCACAGGCTCATACTCAGACAATTGTATTAC
TGCCTTATGTTTTTTAAGTGTTTTTTTAAATTCTTCATAGTTGAGTATTATTTGCAATTTTATTAGTTACAGTG
CTATTAAAGAATATGTGCTCCTTTT

# FIGURE 44

TAGAGAAGGCAGACGCATCCCGAACTCGCTGGAGGACAAGGCTCAGCTCTTGCCAGGCCAAATTGAGACATGTC
TGACACAAGCGAGAGTGGTGCAGGTCTAACTCGCTTCCAGGCTGAAGCCTCAGAAAAGGACAGTAGCTCGATGA
TGCAGACTCTGTTGACAGTGACCCAGAATGTGGAGGTCCCAGAGACACCGAAGGCCTCAAAGGCACTGGAGGTC
TCAGAGGATGTGAAGGTCTCAAAAGCCTCTGGGGTCTCAAAGGCCACAGAGGTCTCAAAGACCCCAGAGGCTCG
GGAGGCACCTGCCACCCAGGCCTCATCTACTACTCAGCTGACTGATACCCAGGTTCTGGCAGCTGAAAACAAGA
GTCTAGCAGCTGACACCAAGAAACAGAATGCTGACCCGCAGGCTGTGACAATGCCTGCCACTGAGACCAAAAAG
GTCAGCCATGTGGCTGATACAAAGGTCAATACAAAGGCTCAGGAGACTGAGGCTGCACCCTCTCAGGCCCCAGC
AGATGAACCTGAGCCTGAGAGTGCAGCTGCCCAGTCTCAGGAGAATCAGGATACTCGGCCCAAGGTCAAAGCCA
AGAAAGCCCGAAAGGTGAAGCATCTGGATGGGGAAGAGGATGGCAGCAGTGATCAGAGTCAGGCTTCTGGAACC
ACAGGTGGCCGAAGGGTCTCAAAGGCCCTAATGGCCTCAATGGCCCGCAGGGCTTCAAGGGGTCCCATAGCCTT
TTGGGCCCGCAGGGCATCAAGGACTCGGTTGGCTGCTTGGGCCCGGAGAGCCTTGCTCTCCCTGAGATCACCTA
AAGCCCGTAGGGGCAAGGCTCGCCGTAGAGCTGCCAAGCTCCAGTCATCCCAAGAGCCTGAAGCACCACCACCT
CGGGATGTGGCCCTTTTGCAAGGGAGGGCAAATGATTTGGTGAAGTACCTTTTGGCTAAAGACCAGACGAAGAT
TCCCATCAAGCGCTCGGACATGCTGAAGGACATCATCAAAGAATACACTGATGTGTACCCCGAAATCATTGAAC
GAGCAGGCTATTCCTTGGAGAAGGTATTTGGGATTCAATTGAAGGAAATTGATAAGAATGACCACTTGTACATT
CTTCTCAGCACCTTAGAGCCCACTGATGCAGGCATACTGGGAACGACTAAGGACTCACCCAAGCTGGGTCTGCT
CATGGTGCTTCTTAGCATCATCTTCATGAATGGAAATCGGTCCAGTGAGGCTGTCATCTGGGAGGTGCTGCGCA
AGTTGGGGCTGCGCCCTGGGATACATCATTCACTCTTTGGGGACGTGAAGAAGCTCATCACTGATGAGTTTGTG
AAGCAGAAGTACCTGGACTATGCCAGAGTCCCCAATAGCAATCCCCCTGAATATGAGTTCTTCTGGGGCCTGCG
CTCTTACTATGAGACCAGCAAGATGAAAGTCCTCAAGTTTGCCTGCAAGGTACAAAAGAAGGATCCCAAGGAAT
GGGCAGCTCAGTACCGAGAGGCGATGGAAGCGGATTTGAAGGCTGCAGCTGAGGCTGCAGCTGAAGCCAAGGCT
AGGGCCGAGATTAGAGCTCGAATGGGCATTGGGCTCGGCTCGGAGAATGCTGCCGGGCCCTGCAACTGGGACGA
AGCTGATATCGGACCCTGGGCCAAAGCCCGGATCCAGGCGGGAGCAGAAGCTAAAGCCAAAGCCCAAGAGAGTG
GCAGTGCCAGCACTGGTGCCAGTACCAGTACCAATAACAGTGCCAGTGCCAGTGCCAGCACCAGTGGTGGCTTC
AGTGCTGGTGCCAGCCTGACCGCCACTCTCACATTTGGGCTCTTCGCTGGCCTTGGTGGAGCTGGTGCCAGCAC
CAGTGGCAGCTCTGGTGCCTGTGGTTTCTCCTACAAGTGAGATTTTAGATATTGTTAATCCTGCCAGTCTTTCT
CTTCAAGCCAGGGTGCATCCTCAGAAACCTATCCAACACAGCACTCTAGGCAGCCACT

# FIGURE 45

CGCCGCGGCG<u>ATG</u>CCGGAGGAGGGTTCGGGCTGCTCGGTGCGGCGCAGGCCCTATGGGTGCGTCCTGCGGGCTG
CTTTGGTCCCATTGGTCGCGGGCTTGGTGATCTGCCTCGTGGTGTGCATCCAGCGCTTCGCACAGGCTCAGCAG
CAGCTGCCGCTCGAGTCACTTGGGTGGGACGTAGCTGAGCTGCAGCTGAATCACACAGGACCTCAGCAGGACCC
CAGGCTATACTGGCAGGGGGGCCCAGCACTGGGCCGCTCCTTCCTGCATGGACCAGAGCTGGACAAGGGGCAGC
TACGTATCCATCGTGATGGCATCTACATGGTACACATCCAGGTGACGCTGGCCATCTGCTCCTCCACGACGGCC
TCCAGGCACCACCCCACCACCCTGGCCGTGGGAATCTGCTCTCCCGCCTCCCGTAGCATCAGCCTGCTGCGTCT
CAGCTTCCACCAAGGTTGTACCATTGCCTCCCAGCGCCTGACGCCCCTGGCCCGAGGGGACACACTCTGCACCA
ACCTCACTGGGACACTTTTGCCTTCCCGAAACACTGATGAGACCTTCTTTGGAGTGCAGTGGGTGCGCCCC<u>TGA</u>
CCACTGCTGCTGATTAGGGTTTTTTAAATTTTATTTTATTTTATTTAAGTTCAAGAGAAAAAGTGTACACACAG
GGGCCACCCGGGGTTGGGGTGGGAGTGTGGTGGGGGGTAGTGGTGGCAGGACAAGAGAAGGCATTGAGCTTTTT
CTTTCATTTTCCTATTAAAAAATACAAAAATCCAAAAAAAAAAAAAAAAAAAAAAAAAAA

# FIGURE 46

CAGCACATCCCGCTCTGGGCTTTAAACGTGACCCCTCGCCTCGACTCGCCCTGCCCTGTGAAA<u>ATG</u>TTGGTGCT
TCTTGCTTTCATCATCGCCTTCCACATCACCTCTGCAGCCTTGCTGTTCATTGCCACCGTCGACAATGCCTGGT
GGGTAGGAGATGAGTTTTTTGCAGATGTCTGGAGAATATGTACCAACAACACGAATTGCACAGTCATCAATGAC
AGCTTTCAAGAGTACTCCACGCTGCAGGCGGTCCAGGCCACCATGATCCTCTCCACCATTCTCTGCTGCATCGC
CTTCTTCATCTTCGTGCTCCAGCTCTTCCGCCTGAAGCAGGGAGAGAGGTTTGTCCTAACCTCCATCATCCAGC
TAATGTCATGTCTGTGTGTCATGATTGCGGCCTCCATTTATACAGACAGGCGTGAAGACATTCACGACAAAAAC
GCGAAATTCTATCCCGTGACCAGAGAAGGCAGCTACGGCTACTCCTACATCCTGGCGTGGGTGGCCTTCGCCTG
CACCTTCATCAGCGGCATGATGTACCTGATACTGAGGAAGCGCAAA<u>TAG</u>AGTTCCGGAGCTGGGTTGCTTCTGC
TGCAGTACAGAATCCACATTCAGATAACCATTTTGTATATAATCATTATTTTTTGAGGTTTTTCTAGCAAACCG
TATTGTTTCCTTTAAAAGCCAAAA

# FIGURE 47

GCGCGGAGCTGGGAGTGGCTTCGCCATGGCTGTGAGAAGGGACTCCGTGTGGAAGTACTGCTGGGGTGTTTTGA
TGGTTTTATGCAGAACTGCGATTTCCAAATCGATAGTTTTAGAGCCTATCTATTGGAATTCCTCGAACTCCAAA
TTTCTACCTGGACAAGGACTGGTACTATACCCACAGATAGGAGACAAATTGGATATTATTTGCCCCAAAGTGGA
CTCTAAAACTGTTGGCCAGTATGAATATTATAAAGTTTATATGGTTGATAAAGACCAAGCAGACAGATGCACTA
TTAAGAAGGAAAATACCCCTCTCCTCAACTGTGCCAAACCAGACCAAGATATCAAATTCACCATCAAGTTTCAA
GAATTCAGCCCTAACCTCTGGGGTCTAGAATTTCAGAAGAACAAAGATTATTACATTATATCTACATCAAATGG
GTCTTTGGAGGGCCTGGATAACCAGGAGGGAGGGGTGTGCCAGACAAGAGCCATGAAGATCCTCATGAAAGTTG
GACAAGATGCAAGTTCTGCTGGATCAACCAGGAATAAAGATCCAACAAGACGTCCAGAACTAGAAGCTGGTACA
AATGGAAGAAGTTCGACAACAAGTCCCTTTGTAAAACCAAATCCAGGTTCTAGCACAGACGGCAACAGCGCCGG
ACATTCGGGGAACAACATCCTCGGTTCCGAAGTGGCCTTATTTGCAGGGATTGCTTCAGGATGCATCATCTTCA
TCGTCATCATCATCACGCTGGTGGTCCTCTTGCTGAAGTACCGGAGGAGACACAGGAAGCACTCGCCGCAGCAC
ACGACCACGCTGTCGCTCAGCACACTGGCCACACCCAAGCGCAGCGGCAACAACAACGGCTCAGAGCCCAGTGA
CATTATCATCCCGCTAAGGACTGCGGACAGCGTCTTCTGCCCTCACTACGAGAAGGTCAGCGGGGACTACGGGC
ACCCGGTGTACATCGTCCAGGAGATGCCCCCGCAGAGCCCGGCGAACATTTACTACAAGGTCTGAGAGGGACCC
TGGTGGTACCTGTGCTTTCCCAGAGGACACCTAATGTCCCGATGCCTCCCTTGAGGGTTTGAGAGCCCGCGTGC
TGGAGAATTGACTGAAGCACAGCACCGGGGGAGAGGGACACTCCTCCTCGGAAGAGCCCGTCGCGCTGGACAGC
TTACCTAGTCTTGTAGCATTCGGCCTTGGTGAACACACACGCTCCCTGGAAGCTGGAAGACTGTGCAGAAGACG
CCCATTCGGACTGCTGTGCCGCGTCCCACGTCTCCTCCTCGAAGCCATGTGCTGCGGTCACTCAGGCCTCTGCA
GAAGCCAAGGGAAGACAGTGGTTTGTGGACGAGAGGGCTGTGAGCATCCTGGCAGGTGCCCCAGGATGCCACGC
CTGGAAGGGCCGGCTTCTGCCTGGGGTGCATTTCCCCCGCAGTGCATACCGGACTTGTCACACGGACCTCGGGC
TAGTTAAGGTGTGCAAAGATCTCTAGAGTTTAGTCCTTACTGTCTCACTCGTTCTGTTACCCAGGGCTCTGCAG
CACCTCACCTGAGACCTCCACTCCACATCTGCATCACTCATGGAACACTCATGTCTGGAGTCCCCTCCTCCAGC
CGCTGGCAACAACAGCTTCAGTCCATGGGTAATCCGTTCATAGAAATTGTGTTTGCTAACAAGGTGCCCTTTAG
CCAGATGCTAGGCTGTCTGCGAAGAAGGCTAGGAGTTCATAGAAGGGAGTGGGGCTGGGGAAAGGGCTGGCTGC
AATTGCAGCTCACTGCTGCTGCCTCTGAAACAGAAAGTTGGAAAGGA

# FIGURE 48

GGCCGCGGGAGAGGAGGCC**ATG**GGCGCGCGCGGGGCGCTGCTGCTGGCGCTGCTGCTGGCTCGGGCTGGACTCA
GGAAGCCGGAGTCGCAGGAGGCGGCGCCGTTATCAGGACCATGCGGCCGACGGGTCATCACGTCGCGCATCGTG
GGTGGAGAGGACGCCGAACTCGGGCGTTGGCCGTGGCAGGGGAGCCTGCGCCTGTGGGATTCCCACGTATGCGG
AGTGAGCCTGCTCAGCCACCGCTGGGCACTCACGGCGGCGCACTGCTTTGAAACCTATAGTGACCTTAGTGATC
CCTCCGGGTGGATGGTCCAGTTTGGCCAGCTGACTTCCATGCCATCCTTCTGGAGCCTGCAGGCCTACTACACC
CGTTACTTCGTATCGAATATCTATCTGAGCCCTCGCTACCTGGGGAATTCACCCTATGACATTGCCTTGGTGAA
GCTGTCTGCACCTGTCACCTACACTAAACACATCCAGCCCATCTGTCTCCAGGCCTCCACATTTGAGTTTGAGA
ACCGGACAGACTGCTGGGTGACTGGCTGGGGGTACATCAAAGAGGATGAGGCACTGCCATCTCCCCACACCCTC
CAGGAAGTTCAGGTCGCCATCATAAACAACTCTATGTGCAACCACCTCTTCCTCAAGTACAGTTTCCGCAAGGA
CATCTTTGGAGACATGGTTTGTGCTGGCAACGCCCAAGGCGGGAAGGATGCCTGCTTCGGTGACTCAGGTGGAC
CCTTGGCCTGTAACAAGAATGGACTGTGGTATCAGATTGGAGTCGTGAGCTGGGGAGTGGGCTGTGGTCGGCCC
AATCGGCCCGGTGTCTACACCAATATCAGCCACCACTTTGAGTGGATCCAGAAGCTGATGGCCCAGAGTGGCAT
GTCCCAGCCAGACCCCTCCTGGCCACTACTCTTTTTCCCTCTTCTCTGGGCTCTCCCACTCCTGGGGCCGGTC**T**
**GA**GCCTACCTGAGCCCATGCAGCCTGGGGCCACTGCCAAGTCAGGCCCTGGTTCTCTTCTGTCTTGTTTGGTAA
TAAACACATTCCAGTTGATGCCTTGCAGGGCATTCTTCAAAAAAAAAAAAAAAAAAAAAAAAA

# FIGURE 49

GAGAGAGGCAGCAGCTTGCTCAGCGGACAAGGATGCTGGGCGTGAGGGACCAAGGCCTGCCCTGCACTCGGGCC
TCCTCCAGCCAGTGCTGACCAGGGACTTCTGACCTGCTGGCCAGCCAGGACCTGTGTGGGGAGGCCCTCCTGCT
GCCTTGGGGTGACAATCTCAGCTCCAGGCTACAGGGAGACCGGGAGGATCACAGAGCCAGC**ATG**TTACAGGATC
CTGACAGTGATCAACCTCTGAACAGCCTCGATGTCAAACCCCTGCGCAAACCCCGTATCCCCATGGAGACCTTC
AGAAAGGTGGGGATCCCCATCATCATAGCACTACTGAGCCTGGCGAGTATCATCATTGTGGTTGTCCTCATCAA
GGTGATTCTGGATAAATACTACTTCCTCTGCGGGCAGCCTCTCCACTTCATCCCGAGGAAGCAGCTGTGTGACG
GAGAGCTGGACTGTCCCTTGGGGGAGGACGAGGAGCACTGTGTCAAGAGCTTCCCCGAAGGGCCTGCAGTGGCA
GTCCGCCTCTCCAAGGACCGATCCACACTGCAGGTGCTGGACTCGGCCACAGGGAACTGGTTCTCTGCCTGTTT
CGACAACTTCACAGAAGCTCTCGCTGAGACAGCCTGTAGGCAGATGGGCTACAGCAGAGCTGTGGAGATTGGCC
CAGACCAGGATCTGGATGTTGTTGAAATCACAGAAAACAGCCAGGAGCTTCGCATGCGGAACTCAAGTGGGCCC
TGTCTCTCAGGCTCCCTGGTCTCCCTGCACTGTCTTGCCTGTGGGAAGAGCCTGAAGACCCCCCGTGTGGTGGG
TGGGGAGGAGGCCTCTGTGGATTCTTGGCCTTGGCAGGTCAGCATCCAGTACGACAAACAGCACGTCTGTGGAG
GGAGCATCCTGGACCCCCACTGGGTCCTCACGGCAGCCCACTGCTTCAGGAAACATACCGATGTGTTCAACTGG
AAGGTGCGGGCAGGCTCAGACAAACTGGGCAGCTTCCCATCCCTGGCTGTGGCCAAGATCATCATCATTGAATT
CAACCCCATGTACCCCAAAGACAATGACATCGCCCTCATGAAGCTGCAGTTCCCACTCACTTTCTCAGGCACAG
TCAGGCCCATCTGTCTGCCCTTCTTTGATGAGGAGCTCACTCCAGCCACCCCACTCTGGATCATTGGATGGGGC
TTTACGAAGCAGAATGGAGGGAAGATGTCTGACATACTGCTGCAGGCGTCAGTCCAGGTCATTGACAGCACACG
GTGCAATGCAGACGATGCGTACCAGGGGGAAGTCACCGAGAAGATGATGTGTGCAGGCATCCCGGAAGGGGGTG
TGGACACCTGCCAGGGTGACAGTGGTGGGCCCCTGATGTACCAATCTGACCAGTGGCATGTGGTGGGCATCGTT
AGCTGGGGCTATGGCTGCGGGGGCCCGAGCACCCCAGGAGTATACACCAAGGTCTCAGCCTATCTCAACTGGAT
CTACAATGTCTGGAAGGCTGAGCTG**TAA**TGCTGCTGCCCCTTTGCAGTGCTGGGAGCCGCTTCCTTCCTGCCCT
GCCCACCTGGGGATCCCCCAAAGTCAGACACAGAGCAAGAGTCCCCTTGGGTACACCCCTCTGCCCACAGCCTC
AGCATTTCTTGGAGCAGCAAAGGGCCTCAATTCCTGTAAGAGACCCTCGCAGCCCAGAGGCGCCCAGAGGAAGT
CAGCAGCCCTAGCTCGGCCACACTTGGTGCTCCCAGCATCCCAGGGAGAGACACAGCCCACTGAACAAGGTCTC
AGGGGTATTGCTAAGCCAAGAAGGAACTTTCCCACACTACTGAATGGAAGCAGGCTGTCTTGTAAAAGCCCAGA
TCACTGTGGGCTGGAGAGGAGAAGGAAAGGGTCTGCGCCAGCCCTGTCCGTCTTCACCCATCCCCAAGCCTACT
AGAGCAAGAAACCAGTTGTAATATAAAATGCACTGCCCTACTGTTGGTATGACTACCGTTACCTACTGTTGTCA
TTGTTATTACAGCTATGGCCACTATTATTAAAGAGCTGTGTAACATCTCTGGCAAAAAAAAAAAA

# FIGURE 50

CCCGGGTCGACCCACGCGTCCGGGGAGAAAGGATGGCCGGCCTGGCGGCGCGGTTGGTCCTGCTAGCTGGGGCA
GCGGCGCTGGCGAGCGGCTCCCAGGGCGACCGTGAGCCGGTGTACCGCGACTGCGTACTGCAGTGCGAAGAGCA
GAACTGCTCTGGGGGCGCTCTGAATCACTTCCGCTCCCGCCAGCCAATCTACATGAGTCTAGCAGGCTGGACCT
GTCGGGACGACTGTAAGTATGAGTGTATGTGGGTCACCGTTGGGCTCTACCTCCAGGAAGGTCACAAAGTGCCT
CAGTTCCATGGCAAGTGGCCCTTCTCCCGGTTCCTGTTCTTTCAAGAGCCGGCATCGGCCGTGGCCTCGTTTCT
CAATGGCCTGGCCAGCCTGGTGATGCTCTGCCGCTACCGCACCTTCGTGCCAGCCTCCTCCCCCATGTACCACA
CCTGTGTGGCCTTCGCCTGGGTGTCCCTCAATGCATGGTTCTGGTCCACAGTCTTCCACACCAGGGACACTGAC
CTCACAGAGAAAATGGACTACTTCTGTGCCTCCACTGTCATCCTACACTCAATCTACCTGTGCTGCGTCAGGAC
CGTGGGGCTGCAGCACCCAGCTGTGGTCAGTGCCTTCCGGGCTCTCCTGCTGCTCATGCTGACCGTGCACGTCT
CCTACCTGAGCCTCATCCGCTTCGACTATGGCTACAACCTGGTGGCCAACGTGGCTATTGGCCTGGTCAACGTG
GTGTGGTGGCTGGCCTGGTGCCTGTGGAACCAGCGGCGGCTGCCTCACGTGCGCAAGTGCGTGGTGGTGGTCTT
GCTGCTGCAGGGGCTGTCCCTGCTCGAGCTGCTTGACTTCCCACCGCTCTTCTGGGTCCTGGATGCCCATGCCA
TCTGGCACATCAGCACCATCCCTGTCCACGTCCTCTTTTTCAGCTTTCTGGAAGATGACAGCCTGTACCTGCTG
AAGGAATCAGAGGACAAGTTCAAGCTGGACTGAAGACCTTGGAGCGAGTCTGCCCCAGTGGGGATCCTGCCCCC
GCCCTGCTGGCCTCCCTTCTCCCCTCAACCCTTGAGATGATTTTCTCTTTTCAACTTCTTGAACTTGGACATGA
AGGATGTGGGCCCAGAATCATGTGGCCAGCCCACCCCCTGTTGGCCCTCACCAGCCTTGGAGTCTGTTCTAGGG
AAGGCCTCCCAGCATCTGGGACTCGAGAGTGGGCAGCCCCTCTACCTCCTGGAGCTGAACTGGGGTGGAACTGA
GTGTGTTCTTAGCTCTACCGGGAGGACAGCTGCCTGTTTCCTCCCCACCAGCCTCCTCCCCACATCCCCAGCTG
CCTGGCTGGGTCCTGAAGCCCTCTGTCTACCTGGGAGACCAGGGACCACAGGCCTTAGGGATACAGGGGGTCCC
CTTCTGTTACCACCCCCCACCCTCCTCCAGGACACCACTAGGTGGTGCTGGATGCTTGTTCTTTGGCCAGCCAA
GGTTCACGGCGATTCTCCCCATGGGATCTTGAGGGACCAAGCTGCTGGGATTGGGAAGGAGTTTCACCCTGACC
GTTGCCCTAGCCAGGTTCCCAGGAGGCCTCACCATACTCCCTTTCAGGGCCAGGGCTCCAGCAAGCCCAGGGCA
AGGATCCTGTGCTGCTGTCTGGTTGAGAGCCTGCCACCGTGTGTCGGGAGTGTGGGCCAGGCTGAGTGCATAGG
TGACAGGGCCGTGAGCATGGGCCTGGGTGTGTGTGAGCTCAGGCCTAGGTGCGCAGTGTGGAGACGGGTGTTGT
CGGGGAAGAGGTGTGGCTTCAAAGTGTGTGTGTGCAGGGGGTGGGTGTGTTAGCGTGGGTTAGGGGAACGTGTG
TGCGCGTGCTGGTGGGCATGTGAGATGAGTGACTGCCGGTGAATGTGTCCACAGTTGAGAGGTTGGAGCAGGAT
GAGGGAATCCTGTCACCATCAATAATCACTTGTGGAGCGCCAGCTCTGCCCAAGACGCCACCTGGGCGGACAGC
CAGGAGCTCTCCATGGCCAGGCTGCCTGTGTGCATGTTCCCTGTCTGGTGCCCCTTTGCCCGCCTCCTGCAAAC
CTCACAGGGTCCCCACACAACAGTGCCCTCCAGAAGCAGCCCCTCGGAGGCAGAGGAAGGAAAATGGGGATGGC
TGGGGCTCTCTCCATCCTCCTTTTCTCCTTGCCTTCGCATGGCTGGCCTTCCCCTCCAAAACCTCCATTCCCCT
GCTGCCAGCCCCTTTGCCATAGCCTGATTTTGGGGAGGAGGAAGGGGCGATTTGAGGGAGAAGGGGAGAAAGCT
TATGGCTGGGTCTGGTTTCTTCCCTTCCCAGAGGGTCTTACTGTTCCAGGGTGGCCCCAGGGCAGGCAGGGGCC
ACACTATGCCTGTGCCCTGGTAAAGGTGACCCCTGCCATTTACCAGCAGCCCTGGCATGTTCCTGCCCCACAGG
AATAGAATGGAGGGAGCTCCAGAAACTTTCCATCCCAAAGGCAGTCTCCGTGGTTGAAGCAGACTGGATTTTTG
CTCTGCCCCTGACCCCTTGTCCCTCTTTGAGGGAGGGGAGCTATGCTAGGACTCCAACCTCAGGGACTCGGGTG
GCCTGCGCTAGCTTCTTTTGATACTGAAAACTTTTAAGGTGGAGGGTGGCAAGGGATGTGCTTAATAAATCAA
TTCCAAGCCTCAAAAAAAAAAAAAAAAA

# FIGURE 51

CGGCACGAGGGTCCCGCGCGCTCCTCCGACCCGCTCCGCTCCGCTCCGCTCGGCCCCGCGCCGCCCGTCAACAT
GATCCGCTGCGGCCTGGCCTGCGAGCGCTGCCGCTGGATCCTGCCCCTGCTCCTACTCAGCGCCATCGCCTTCG
ACATCATCGCGCTGGCCGGCCGCGGCTGGTTGCAGTCTAGCGACCACGGCCAGACGTCCTCGCTGTGGTGGAAA
TGCTCCCAAGAGGGCGGCGGCAGCGGGTCCTACGAGGAGGGCTGTCAGAGCCTCATGGAGTACGCGTGGGGTAG
AGCAGCGGCTGCCATGCTCTTCTGTGGCTTCATCATCCTGGTGATCTGTTTCATCCTCTCCTTCTTCGCCCTCT
GTGGACCCCAGATGCTTGTCTTCCTGAGAGTGATTGGAGGTCTCCTTGCCTTGGCTGCTGTGTTCCAGATCATC
TCCCTGGTAATTTACCCCGTGAAGTACACCCAGACCTTCACCCTTCATGCCAACCCTGCTGTCACTTACATCTA
TAACTGGGCCTACGGCTTTGGGTGGGCAGCCACGATTATCCTGATTGGCTGTGCCTTCTTCTTCTGCTGCCTCC
TCAACTACGAAGATGACCTTCTGGGCAATGCCAAGCCCAGGTACTTCTACACATCTGCCTAACTTGGGAATGAA
TGTGGGAGAAAATCGCTGCTGCTGAGATGGACTCCAGAAGAAGAAACTGTTTCTCCAGGCGACTTTGAACCCAT
TTTTTGGCAGTGTTCATATTATTAAACTAGTCAAAAATGCTAAAATAATTTGGGAGAAAATATTTTTTAAGTAG
TGTTATAGTTTCATGTTTATCTTTTATTATGTTTTGTGAAGTTGTGTCTTTTCACTAATTACCTATACTATGCC
AATATTTCCTTATATCTATCCATAACATTTATACTACATTTGTAAGAGAATATGCACGTGAAACTTAACACTTT
ATAAGGTAAAAATGAGGTTTCCAAGATTTAATAATCTGATCAAGTTCTTGTTATTTCCAAATAGAATGGACTCG
GTCTGTTAAGGGCTAAGGAGAAGAGGAAGATAAGGTTAAAAGTTGTTAATGACCAAACATTC

# FIGURE 52

GAACGCTTGTGTCTAACTGATGCTCCTAATGCGGAAGCCCCTGAAAGGCGGTTGTGGTGCAAAGGAAAACCCAC
AGGCCAAGGAATGGGAAGACCAAGGTTGACACTTGTTTGTCAAGTGTCAATAATCATCTCTGCCCGGGACCTCA
GCATGAACAACCTCACAGAGCTTCAGCCTGGCCTCTTCCACCACCTGCGCTTCTTGGAGGAGCTGCGTCTCTCT
GGGAACCATCTCTCACACATCCCAGGACAAGCATTCTCTGGTCTCTACAGCCTGAAAATCCTGATGCTGCAGAA
CAATCAGCTGGGAGGAATCCCCGCAGAGGCGCTGTGGGAGCTGCCGAGCCTGCAGTCGCTGCGCCTAGATGCCA
ACCTCATCTCCCTGGTCCCGGAGAGGAGCTTTGAGGGGCTGTCCTCCCTCCGCCACCTCTGGCTGGACGACAAT
GCACTCACGGAGATCCCTGTCAGGGCCCTCAACAACCTCCCTGCCCTGCAGGCCATGACCCTGGCCCTCAACCG
CATCAGCCACATCCCCGACTACGCGTTCCAGAATCTCACCAGCCTTGTGGTGCTGCATTTGCATAACAACCGCA
TCCAGCATCTGGGGACCCACAGCTTCGAGGGGCTGCACAATCTGGAGACACTAGACCTGAATTATAACAAGCTG
CAGGAGTTCCCTGTGGCCATCCGGACCCTGGGCAGACTGCAGGAACTGGGGTTCCATAACAACAACATCAAGGC
CATCCCAGAAAAGGCCTTCATGGGGAACCCTCTGCTACAGACGATACACTTTTATGATAACCCAATCCAGTTTG
TGGGAAGATCGGCATTCCAGTACCTGCCTAAACTCCACACACTATCTCTGAATGGTGCCATGGACATCCAGGAG
TTTCCAGATCTCAAAGGCACCACCAGCCTGGAGATCCTGACCCTGACCCGCGCAGGCATCCGGCTGCTCCCATC
GGGGATGTGCCAACAGCTGCCCAGGCTCCGAGTCCTGGAACTGTCTCACAATCAAATTGAGGAGCTGCCCAGCC
TGCACAGGTGTCAGAAATTGGAGGAAATCGGCCTCCAACACAACCGCATCTGGGAAATTGGAGCTGACACCTTC
AGCCAGCTGAGCTCCCTGCAAGCCCTGGATCTTAGCTGGAACGCCATCCGGTCCATCCACCCTGAGGCCTTCTC
CACCCTGCACTCCCTGGTCAAGCTGGACCTGACAGACAACCAGCTGACCACACTGCCCCTGGCTGGACTTGGGG
GCTTGATGCATCTGAAGCTCAAAGGGAACCTTGCTCTCTCCCAGGCCTTCTCCAAGGACAGTTTCCCAAAACTG
AGGATCCTGGAGGTGCCTTATGCCTACCAGTGCTGTCCCTATGGGATGTGTGCCAGCTTCTTCAAGGCCTCTGG
GCAGTGGGAGGCTGAAGACCTTCACCTTGATGATGAGGAGTCTTCAAAAAGGCCCCTGGGCCTCCTTGCCAGAC
AAGCAGAGAACCACTATGACCAGGACCTGGATGAGCTCCAGCTGGAGATGGAGGACTCAAAGCCACACCCCAGT
GTCCAGTGTAGCCCTACTCCAGGCCCCTTCAAGCCCTGTGAGTACCTCTTTGAAAGCTGGGGCATCCGCCTGGC
CGTGTGGGCCATCGTGTTGCTCTCCGTGCTCTGCAATGGACTGGTGCTGCTGACCGTGTTCGCTGGCGGGCCTG
CCCCCCTGCCCCCGGTCAAGTTTGTGGTAGGTGCGATTGCAGGCGCCAACACCTTGACTGGCATTTCCTGTGGC
CTTCTAGCCTCAGTCGATGCCCTGACCTTTGGTCAGTTCTCTGAGTACGGAGCCCGCTGGGAGACGGGGCTAGG
CTGCCGGGCCACTGGCTTCCTGGCAGTACTTGGGTCGGAGGCATCGGTGCTGCTGCTCACTCTGGCCGCAGTGC
AGTGCAGCGTCTCCGTCTCCTGTGTCCGGGCCTATGGGAAGTCCCCCTCCCTGGGCAGCGTTCGAGCAGGGGTC
CTAGGCTGCCTGGCACTGGCAGGGCTGGCCGCCGCACTGCCCCTGGCCTCAGTGGGAGAATACGGGGCCTCCCC
ACTCTGCCTGCCCTACGCGCCACCTGAGGGTCAGCCAGCAGCCCTGGGCTTCACCGTGGCCCTGGTGATGATGA
ACTCCTTCTGTTTCCTGGTCGTGGCCGGTGCCTACATCAAACTGTACTGTGACCTGCCGCGGGGCGACTTTGAG
GCCGTGTGGGACTGCGCCATGGTGAGGCACGTGGCCTGGCTCATCTTCGCAGACGGGCTCCTCTACTGTCCCGT
GGCCTTCCTCAGCTTCGCCTCCATGCTGGGCCTCTTCCCTGTCACGCCCGAGGCCGTCAAGTCTGTCCTGCTGG
TGGTGCTGCCCCTGCCTGCCTGCCTCAACCCACTGCTGTACCTGCTCTTCAACCCCCACTTCCGGGATGACCTT
CGGCGGCTTCGGCCCCGCGCAGGGGACTCAGGGCCCCTAGCCTATGCTGCGGCCGGGGAGCTGGAGAAGAGCTC
CTGTGATTCTACCCAGGCCCTGGTAGCCTTCTCTGATGTGGATCTCATTCTGGAAGCTTCTGAAGCTGGGCGGC
CCCCTGGGCTGGAGACCTATGGCTTCCCCTCAGTGACCCTCATCTCCTGTCAGCAGCCAGGGGCCCCCAGGCTG
GAGGGCAGCCATTGTGTAGAGCCAGAGGGGAACCACTTTGGGAACCCCCAACCCTCCATGGATGGAGAACTGCT
GCTGAGGGCAGAGGGATCTACGCCAGCAGGTGGAGGCTTGTCAGGGGGTGGCGGCTTTCAGCCCTCTGGCTTGG
CCTTTGCTTCACACGTGTAAATATCCCTCCCCATTCTTCTCTTCCCCTCTCTTCCCTTTCCTCTCTCCCCCTCG
GTGAATGATGGCTGCTTCTAAAACAAATACAACCAAAACTCAGCAGTGTGATCTATAGCAGGATGGCCCAGTAC
CTGGCTCCACTGATCACCTCTCTCCTGTGACCATCACCAACGGGTGCCTCTTGGCCTGGCTTTCCCTTGGCCTT
CCTCAGCTTCACCTTGATACTGGGCCTCTTCCTTGTCATGTCTGAAGCTGTGGACCAGAGACCTGGACTTTTGT
CTGCTTAAGGGAAATGAGGGAAGTAAAGACAGTGAAGGGGTGGAGGGTTGATCAGGGCACAGTGGACAGGGAGA
CCTCACAGAGAAAGGCCTGGAAGGTGATTTCCCGTGTGACTCATGGATAGGATACAAAATGTGTTCCATGTACC
ATTAATCTTGACATATGCCATGCATAAAGACTTCCTATTAAAATAAGCTTTGGAAGAGAAAAAAAAAAA

# FIGURE 53

CGCCTCCGCCTTCGGAGGCTGACGCGCCCGGGCGCCGTTCCAGGCCTGTGCAGGGCGGATCGGCAGCCGCCTGG
CGGCGATCCAGGGCGGTGCGGGGCCTGGGCGGGAGCCGGGAGGCGCGGCCGGC**ATG**GAGGCGCTGCTGCTGGGC
GCGGGGTTGCTGCTGGGCGCTTACGTGCTTGTCTACTACAACCTGGTGAAGGCCCCGCCGTGCGGCGGCATGGG
CAACCTGCGGGGCCGCACGGCCGTGGTCACGGGCGCCAACAGCGGCATCGGAAAGATGACGGCGCTGGAGCTGG
CGCGCCGGGGAGCGCGCGTGGTGCTGGCCTGCCGCAGCCAGGAGCGCGGGGAGGCGGCTGCCTTCGACCTCCGC
CAGGAGAGTGGGAACAATGAGGTCATCTTCATGGCCTTGGACTTGGCCAGTCTGGCCTCGGTGCGGGCCTTTGC
CACTGCCTTTCTGAGCTCTGAGCCACGGTTGGACATCCTCATCCACAATGCCGGTATCAGTTCCTGTGGCCGGA
CCCGTGAGGCGTTTAACCTGCTGCTTCGGGTGAACCATATCGGTCCCTTTCTGCTGACACATCTGCTGCTGCCT
TGCCTGAAGGCATGTGCCCCTAGCCGCGTGGTGGTAGCCTCAGCTGCCCACTGTCGGGGACGTCTTGACTT
CAAACGCCTGGACCGCCCAGTGGTGGGCTGGCGGCAGGAGCTGCGGGCATATGCTGACACTAAGCTGGCTAATG
TACTGTTTGCCCGGGAGCTCGCCAACCAGCTTGAGGCCACTGGCGTCACCTGCTATGCAGCCCACCCAGGGCCT
GTGAACTCGGAGCTGTTCCTGCGCCATGTTCCTGGATGGCTGCGCCCACTTTTGCGCCCATTGGCTTGGCTGGT
GCTCCGGGCACCAAGAGGGGGTGCCCAGACACCCCTGTATTGTGCTCTACAAGAGGGCATCGAGCCCCTCAGTG
GGAGATATTTTGCCAACTGCCATGTGGAAGAGGTGCCTCCAGCTGCCCGAGACGACCGGGCAGCCCATCGGCTA
TGGGAGGCCAGCAAGAGGCTGGCAGGGCTTGGGCCTGGGGAGGATGCTGAACCCGATGAAGACCCCCAGTCTGA
GGACTCAGAGGCCCCATCTTCTCTAAGCACCCCCCACCCTGAGGAGCCCACAGTTTCTCAACCTTACCCCAGCC
CTCAGAGCTCACCAGATTTGTCTAAGATGACGCACCGAATTCAGGCTAAAGTTGAGCCTGAGATCCAGCTCTCC
**TAA**CCCTCAGGCCAGGATGCTTGCCATGGCACTTCATGGTCCTTGAAAACCTCGGATGTGTGTGAGGCCATGCC
CTGGACACTGACGGGTTTGTGATCTTGACCTCCGTGGTTACTTTCTGGGGCCCCAAGCTGTGCCCTGGACATCT
CTTTTCCTGGTTGAAGGAATAATGGGTGATTATTTCTTCCTGAGAGTGACAGTAACCCCAGATGGAGAGATAGG
GGTATGCTAGACACTGTGCTTCTCGGAAATTTGGATGTAGTATTTTCAGGCCCCACCCTTATTGATTCTGATCA
GCTCTGGAGCAGAGGCAGGGAGTTTGCAATGTGATGCACTGCCAACATTGAGAATTAGTGAACTGATCCCTTTG
CAACCGTCTAGCTAGGTAGTTAAATTACCCCCATGTTAATGAAGCGGAATTAGGCTCCCGAGCTAAGGGACTCG
CCTAGGGTCTCACAGTGAGTAGGAGGAGGGCCTGGGATCTGAACCCAAGGGTCTGAGGCCAGGGCCGACTGCCG
TAAGATGGGTGCTGAGAAGTGAGTCAGGGCAGGGCAGCTGGTATCGAGGTGCCCCATGGGAGTAAGGGGACGCC
TTCCGGGCGGATGCAGGGCTGGGGTCATCTGTATCTGAAGCCCCTCGGAATAAAGCGCGTTGACCGCCAAAAAA
AAAAAAAAAAAAAA

# FIGURE 54

GAATTTGTAGAAGACAGCGGCGTTGCCATGGCGGCGTCTCTGGGGCAGGTGTTGGCTCTGGTGCTGGTGGCCGC
TCTGTGGGGTGGCACGCAGCCGCTGCTGAAGCGGGCCTCCGCCGGCCTGCAGCGGGTTCATGAGCCGACCTGGG
CCCAGCAGTTGCTACAGGAGATGAAGACCCTCTTCTTGAATACTGAGTACCTGATGCCCTTTCTCCTCAACCAG
TGTGGATCCCTTCTCTATTACCTCACCTTGGCATCGACAGATCTGACCCTGGCTGTGCCCATCTGTAACTCTCT
GGCTATCATCTTCACACTGATTGTTGGGAAGGCCCTTGGAGAAGATATTGGTGGAAAACGTAAGTTAGACTACT
GCGAGTGCGGGACGCAGCTCTGTGGATCTCGACATACCTGTGTTAGTTCCTTCCCAGAACCCATCTCCCCAGAG
TGGGTGAGGACACGGCCTTTTCCCATCCTGCCCTTTCCTCTGCAGCTGTTTTGCTTCCTTGTGGCCATCAGAGT
TCCCTTCCCCTGGACAGTCTGGAGAAAGACAGAGGCTGGGGTTTGGGATTGAAGACCAGACCCCATCTGAGCCC
TTCCTCCAGCCCTGTACCAGCTCCTACTGGCATGGCTGAGCTCAGACCCTCCTGATTTCTGCCTATTATCCCAG
GAGCAGTTGCTGGCATGGTGCTCACCGTGATAGGAATTTCACTCTGCATCACAAGCTCAGTGAGTAAGACCCAG
GGGCAACAGTCTACCCTTTGAGTGGGCCGAACCCACTTCCAGCTCTGCTGCCTCCAGGAAGCCCCTGGGCCATG
AAGTGCTGGCAGTGAGCGGATGGACCTAGCACTTCCCCTCTCTGGCCTTAGCTTCCTCCTCTCTTATGGGGATA
ACAGCTACCTCATGGATCACAATAAGAGAACAAGAGTGAAAGAGTTTTGTAACCTTCAAGTGCTGTTCAGCTGC
GGGGATTTAGCACAGGAGACTCTACGCTCACCCTCAGCAACCTTTCTGCCCCAGCAGCTCTCTTCCTGCTAACA
TCTCAGGCTCCCAGCCCAGCCACCATTACTGTGGCCTGATCTGGACTATCATGGTGGCAGGTTCCATGGACTGC
AGAACTCCAGCTGCATGGAAAGGGCCAGCTGCAGACTTTGAGCCAGAAATGCAAACGGGAGGCCTCTGGGACTC
AGTCAGAGCGCTTTGGCTGAATGAGGGGTGGAACCGAGGGAAGAAGGTGCGTCGGAGTGGCAGATGCAGGAAAT
GAGCTGTCTATTAGCCTTGCCTGCCCCACCCATGAGGTAGGCAGAAATCCTCACTGCCAGCCCCTCTTAAACAG
GTAGAGAGCTGTGAGCCCCAGCCCCACCTGACTCCAGCACACCTGGCGAGTAGTAGCTGTCAATAAATCTATGT
AAACAGACAAAAAAAAAAAAAAAAAAAAAAAAAAAAAAAAAAAAAAAAAAAAAAAAAAAAAAAAAAAAAAAAAAA
AAAA

# FIGURE 55A

CGGCGAACAGACGTTCTTTCTCCTCCATGCAGTTACACAAAAGGAGGGCTACGGAAACTAAAAGTTTCGGGGCC
TCTGGCTCGGTGTGTGGAGAAAAGAGAAAACCTGGAGACGGGATATGAAGATCAATGATGCAGACTGATGGTCT
TGATGAAGCTGGGCATTTATAACTAGATTCATTAAGGAATACAAAGAAAATACTTAAAGGGATCAATAATGGTG
TCTTCTGGTTGCAGAATGCGAAGTCTGTGGTTTATCATTGTAATCAGCTTCTTACCAAATACAGAAGGTTTCAG
CAGAGCAGCTTTACCATTTGGGCTGGTGAGGCGAGAATTATCCTGTGAAGGTTATTCTATAGATCTGCGATGCC
CGGGCAGTGATGTCATCATGATTGAGAGCGCTAACTATGGTCGGACGGATGACAAGATTTGTGATGCTGACCCA
TTTCAGATGGAGAATACAGACTGCTACCTCCCCGATGCCTTCAAAATTATGACTCAAAGGTGCAACAATCGAAC
ACAGTGTATAGTAGTTACTGGGTCAGATGTGTTTCCTGATCCATGTCCTGGAACATACAAATACCTTGAAGTCC
AATATGAATGTGTCCCTTACATTTTTGTGTGTCCTGGGACCTTGAAAGCAATTGTGGACTCACCATGTATATAT
GAAGCTGAACAAAAGGCGGGTGCTTGGTGCAAGGACCCTCTTCAGGCTGCAGATAAAATTTATTTCATGCCCTG
GACTCCCTATCGTACCGATACTTTAATAGAATATGCTTCTTTAGAAGATTTCCAAAATAGTCGCCAAACAACAA
CATATAAACTTCCAAATCGAGTAGATGGTACTGGATTTGTGGTGTATGATGGTGCTGTCTTCTTTAACAAAGAA
AGAACGAGGAATATTGTGAAATTTGACTTGAGGACTAGAATTAAGAGTGGCGAGGCCATAATTAACTATGCCAA
CTACCATGATACCTCACCATACAGATGGGGAGGAAAGACTGATATCGACCTAGCAGTTGATGAAAATGGTTTAT
GGGTCATTTACGCCACTGAACAGAACAATGGAATGATAGTTATTAGCCAGCTGAATCCATACACTCTTCGATTT
GAAGCAACGTGGGAGACTGTATACGACAAACGTGCCGCATCAAATGCTTTTATGATATGCGGAGTCCTCTATGT
GGTTAGGTCAGTTTATCAAGACAATGAAAGTGAAACAGGCAAGAACTCAATTGATTACATTTATAATACCCGAT
TAAACCGAGGAGAATATGTAGACGTTCCCTTCCCCAACCAGTATCAGTATATTGCTGCAGTGGATTACAATCCA
AGAGATAACCAACTTTACGTGTGGAACAATAACTTCATTTTACGATATTCTCTGGAGTTTGGTCCACCTGATCC
TGCCCAAGTGCCTACCACAGCTGTGACAATAACTTCTTCAGCTGAGCTGTTCAAAACCATAATATCAACCACAA
GCACTACTTCACAGAAAGGCCCCATGAGCACAACTGTAGCTGGATCACAGGAAGGAAGCAAAGGGACAAAACCA
CCTCCAGCAGTTTCTACAACCAAAATTCCACCTATAACAAATATTTTTCCCCTGCCAGAGAGATTCTGTGAAGC
ATTAGACTCCAAGGGGATAAAGTGGCCTCAGACACAAAGGGGAATGATGGTTGAACGACCATGCCCTAAGGGAA
CAAGAGGAACTGCCTCATATCTCTGCATGATTTCCACTGGAACATGGAACCCTAAGGGCCCCGATCTTAGCAAC
TGTACCTCACACTGGGTGAATCAGCTGGCTCAGAAGATCAGAAGCGGAGAAAATGCTGCTAGTCTTGCCAATGA
ACTGGCTAAACATACCAAAGGGCCAGTGTTTGCTGGGGATGTAAGTTCTTCAGTGAGATTGATGGAGCAGTTGG
TGGACATCCTTGATGCACAGCTGCAGGAACTGAAACCTAGTGAAAAAGATTCAGCTGGACGGAGTTATAACAAG
GCAATTGTTGACACAGTGGACAACCTTCTGAGACCTGAAGCTTTGGAATCATGGAAACATATGAATTCTTCTGA
ACAAGCACATACTGCAACAATGTTACTCGATACATTGGAAGAAGGAGCTTTTGTCCTAGCTGACAATCTTTTAG
AACCAACAAGGGTCTCAATGCCCACAGAAAATATTGTCCTGGAAGTTGCCGTACTCAGTACAGAAGGACAGATC
CAAGACTTTAAATTTCCTCTGGGCATCAAAGGAGCAGGCAGCTCAATCCAACTGTCCGCAAATACCGTCAAACA
GAACAGCAGGAATGGGCTTGCAAAGTTGGTGTTCATCATTTACCGGAGCCTGGGACAGTTCCTTAGTACAGAAA
ATGCAACCATTAAACTGGGTGCTGATTTTATTGGTCGTAATAGCACCATTGCAGTGAACTCTCACGTCATTTCA
GTTTCAATCAATAAAGAGTCCAGCCGAGTATACCTGACTGATCCTGTGCTTTTTACCCTGCCACACATTGATCC
TGACAATTATTTCAATGCAAACTGCTCCTTCTGGAACTACTCAGAGAGAACTATGATGGGATATTGGTCTACCC
AGGGCTGCAAGCTGGTTGACACTAATAAAACTCGAACAACGTGTGCATGCAGCCACCTAACCAATTTTGCAATT
CTCATGGCCCACAGGGAAATTGCATATAAAGATGGCGTTCATGAATTACTTCTTACAGTCATCACCTGGGTGGG
AATTGTCATTTCCCTTGTTTGCCTGGCTATCTGCATCTTCACCTTCTGCTTTTTCCGTGGCCTACAGAGTGACC
GAAATACTATTCACAAGAACCTTTGTATCAACCTTTTCATTGCTGAATTTATTTTCCTAATAGGCATTGATAAG
ACAAAATATGCGATTGCATGCCCAATATTTGCAGGACTTCTACACTTTTTCTTTTTGGCAGCTTTTGCTTGGAT
GTGCCTAGAAGGTGTGCAGCTCTACCTAATGTTAGTTGAAGTTTTTGAAAGTGAATATTCAAGGAAAAAATATT
ACTATGTTGCTGGTTACTTGTTTCCTGCCACAGTGGTTGGAGTTTCAGCTGCTATTGACTATAAGAGCTATGGA
ACAGAAAAAGCTTGCTGGCTTCATGTTGATAACTACTTTATATGGAGCTTCATTGGACCTGTTACCTTCATTAT
TCTGCTAAATATTATCTTCTTGGTGATCACATTGTGCAAAATGGTGAAGCATTCAAACACTTTGAAACCAGATT
CTAGCAGGTTGGAAAACATTAAGTCTTGGGTGCTTGGCGCTTTCGCTCTTCTGTGTCTTCTTGGCCTCACCTGG
TCCTTTGGGTTGCTTTTTATTAATGAGGAGACTATTGTGATGGCATATCTCTTCACTATATTTAATGCTTTCCA
GGGAGTGTTCATTTTCATCTTTCACTGTGCTCTCCAAAAGAAAGTACGAAAAGAATATGGCAAGTGCTTCAGAC
ACTCATACTGCTGTGGAGGCCTCCCAACTGAGAGTCCCCACAGTTCAGTGAAGGCATCAACCACCAGAACCAGT
GCTCGCTATTCCTCTGGCACACAGAGTCGTATAAGAAGAATGTGGAATGATACTGTGAGAAAACAATCAGAATC
TTCTTTTATCTCAGGTGACATCAATAGCACTTCAACACTTAATCAAGGACATTCACTGAACAATGCCAGGGATA
CAAGTGCCATGGATACTCTACCGCTAAATGGTAATTTTAACAACAGCTACTCGCTGCACAAGGGTGACTATAAT
GACAGCGTGCAAGTTGTGGACTGTGGACTAAGTCTGAATGATACTGCTTTTGAGAAAATGATCATTTCAGAATT
AGTGCACAACAACTTACGGGGCAGCAGCAAGACTCACAACCTCGAGCTCACGCTACCAGTCAAACCTGTGATTG
GAGGTAGCAGCAGTGAAGATGATGCTATTGTGGCAGATGCTTCATCTTTAATGCACAGCGACAACCCAGGGCTG

# FIGURE 55B

GAGCTCCATCACAAAGAACTCGAGGCACCACTTATTCCTCAGCGGACTCACTCCCTTCTGTACCAACCCCAGAA
GAAAGTGAAGTCCGAGGGAACTGACAGCTATGTCTCCCAACTGACAGCAGAGGCTGAAGATCACCTACAGTCCC
CCAACAGAGACTCTCTTTATACAAGCATGCCCAATCTTAGAGACTCTCCCTATCCGGAGAGCAGCCCTGACATG
GAAGAAGACCTCTCTCCCTCCAGGAGGAGTGAGAATGAGGACATTTACTATAAAAGCATGCCAAATCTTGGAGC
TGGCCATCAGCTTCAGATGTGCTACCAGATCAGCAGGGGCAATAGTGATGGTTATATAATCCCCATTAACAAAG
AAGGGTGTATTCCAGAAGGAGATGTTAGAGAAGGACAAATGCAGCTGGTTACAAGTCTTTAATCATACAGCTAA
GGAATTCCAAGGGCCACATGCGAGTATTAATAAATAAAGACACCATTGGCCTGACGCAGCTCCCTCAAACTCTG
CTTGAAGAGATGACTCTTGACCTGTGGTTCTCTGGTGTAAAAAAGATGACTGAACCTTGCAGTTCTGTGAATTT
TTATAAAACATACAAAAACTTTGTATATACACAGAGTATACTAAAGTGAATTATTTGTTACAAAGAAAAGAGAT
GCCAGCCAGGTATTTTAAGATTCTGCTGCTGTTTAGAGAAATTGTGAAACAAGCAAAACAAAACTTTCCAGCCA
TTTTACTGCAGCAGTCTGTGAACTAAATTTGTAAATATGGCTGCACCATTTTTGTAGGCCTGCATTGTATTATA
TACAAGACGTAGGCTTTAAAATCCTGTGGGACAAATTTACTGTACCTTACTATTCCTGACAAGACTTGGAAAAG
CAGGAGAGATATTCTGCATCAGTTTGCAGTTCACTGCAAATCTTTTACATTAAGGCAAAGATTGAAAACATGCT
TAACCACTAGCAATCAAGCCACAGGCCTTATTTCATATGTTTCCTCAACTGTACAATGAACTATTCTCATGAAA
AATGGCTAAAGAAATTATATTTTGTTCTATTGCTAGGGTAAAATAAATACATTTGTGTCCAACTGAAATATAAT
TGTCATTAAAATAATTTTAAAGAGTGAAGAAAATATTGTGAAAAGCTCTTGGTTGCACATGTTATGAAATGTTT
TTTCTTACACTTTGTCATGGTAAGTTCTACTCATTTTCACTTCTTTTCCACTGTATACAGTGTTCTGCTTTGAC
AAAGTTAGTCTTTATTACTTACATTTAAATTTCTTATTGCCAAAAGAACGTGTTTTATGGGAGAAACAAACTC
TTTGAAGCCAGTTATGTCATGCCTTGCACAAAAGTGATGAAATCTAGAAAAGATTGTGTGTCACCCCTGTTTAT
TCTTGAACAGAGGGCAAAGAGGGCACTGGGCACTTCTCACAAACTTTCTAGTGAACAAAAGGTGCCTATTCTTTTT

# FIGURE 56

GCATAGATGAATGTATCAGTGGATGGATAGTTGGCTAGATGGGTGGGTTGGTGGATGAATGGCAGAGCTTGCAC
CTGCCAGTCCATCTGACATCAAAGCCAGTGTCTCTAATGGTGACACCACCCTCCTCTGCAGCAGGAGGCAGAGC
TGTGGGATGAATGAGGTTCGCCAGGTCTCCCTTACCTATCCTGGGTCCCCAGCTCCTTCTCACTCTCTTCCCTT
GCAGCCTCGAAGCGGAGGATCCCTGTGTCCCAGCCGGGC**ATG**GCCGACCCCCACCAGCTTTTCGATGACACAAG
TTCAGCCCAGAGCCGGGGCTATGGGGCCCAGCGGGCACCTGGTGGCCTGAGTTATCCTGCAGCCTCTCCCACGC
CCCATGCAGCCTTCCTGGCTGACCCGGTGTCCAACATGGCCATGGCCTATGGGAGCAGCCTGGCCGCGCAGGGC
AAGGAGCTGGTGGATAAGAACATCGACCGCTTCATCCCCATCACCAAGCTCAAGTATTACTTTGCTGTGGACAC
CATGTATGTGGGCAGAAAGCTGGGCCTGCTGTTCTTCCCCTACCTACACCAGGACTGGGAAGTGCAGTACCAAC
AGGACACCCCGGTGGCCCCCCGCTTTGACGTCAATGCCCCGGACCTCTACATTCCAGCAATGGCTTTCATCACC
TACGTTTTGGTGGCTGGTCTTGCGCTGGGGACCCAGGATAGGTTCTCCCCAGACCTCCTGGGGCTGCAAGCGAG
CTCAGCCCTGGCCTGGCTGACCCTGGAGGTGCTGGCCATCCTGCTCAGCCTCTATCTGGTCACTGTCAACACCG
ACCTCACCACCATCGACCTGGTGGCCTTCTTGGGCTACAAATATGTCGGGATGATTGGCGGGGTCCTCATGGGC
CTGCTCTTCGGGAAGATTGGCTACTACCTGGTGCTGGGCTGGTGCTGCGTAGCCATCTTTGTGTTCATGATCCG
GACGCTGCGGCTGAAGATCTTGGCAGACGCAGCAGCTGAGGGGGTCCCGGTGCGTGGGGCCCGGAACCAGCTGC
GCATGTACCTGACCATGGCGGTGGCGGCGGCGCAGCCTATGCTCATGTACTGGCTCACCTTCCACCTGGTGCGG
**TGA**GCGCGCCCGCTGAACCTCCCGCTGCTGCTGCTGCTGCTGGGGGCCACTGTGGCCGCCGAACTCATCTCCTG
CCTGCAGGCCCCAAGGTCCACCCTGTCTGGCCACAGGCACCGCCTCCATCCCATGTCCCGCCCAGCCCCGCCCC
CAACCCAAGGTGCTGAGAGATCTCCAGCTGCACAGGCCACCGCCCCAGGGCGTGGCCGCTGTTACAGAAACAAT
AAACCCTGATGGGCATGGCAAAAAAAAAAAAAAAAAAAAAAAAAAAAAAAAAAAAAAAAAAAAAAAAAAAAAAA
AAAAAAAAAAAAAAAAAAAAAAAAAAGA

# FIGURE 57

CTTCCACGCCCGAGGGCATCGCGCTGGCCTACGGCAGCCTCCTGCTC**ATG**GCGCTGCTGCCCATCTTCTTCGGC
GCCCTGCGCTCCGTACGCTGCGCCCGCGGCAAGAATGCTTCAGACATGCCTGAAACAATCACCAGCCGGGATGC
CGCCCGCTTCCCCATCATCGCCAGCTGCACACTCTTGGGGCTCTACCTCTTTTTCAAAATATTCTCCCAGGAGT
ACATCAACCTCCTGCTGTCCATGTATTTCTTCGTGCTGGGAATCCTGGCCCTGTCCCACACCATCAGCCCCTTC
ATGAATAAGTTTTTTCCAGCCAGCTTTCCAAATCGACAGTACCAGCTGCTCTTCACACAGGGTTCTGGGGAAAA
CAAGGAAGAGATCATCAATTATGAATTTGACACCAAGGACCTGGTGTGCCTGGGCCTGAGCAGCATCGTTGGCG
TCTGGTACCTGCTGAGGAAGCACTGGATTGCCAACAACCTTTTTGGCCTGGCCTTCTCCCTTAATGGAGTAGAG
CTCCTGCACCTCAACAATGTCAGCACTGGCTGCATCCTGCTGGGCGGACTCTTCATCTACGATGTCTTCTGGGT
ATTTGGCACCAATGTGATGGTGACAGTGGCCAAGTCCTTCGAGGCACCAATAAAATTGGTGTTTCCCCAGGATC
TGCTGCAGAAAGGCCTCGAAGCAAACAACTTTGCCATGCTGGGACTTGGAGATGTCGTCATTCCAGGGATCTTC
ATTGCCTTGCTGCTGCGCTTTGACATCAGCTTGAAGAAGAATACCCACACCTACTTCTACACCAGCTTTGCAGC
CTACATCTTCGGCCTGGGCCTTACCATCTTCATCATGCACATCTTCAAGCATGCTCAGCCTGCCCTCCTATACC
TGGTCCCCGCCTGCATCGGTTTTCCTGTCCTGGTGGCGCTGGCCAAGGGAGAAGTGACAGAGATGTTCAGTTAT
GAGGAGTCAAATCCTAAGGATCCAGCGGCAGTGACAGAATCCAAAGAGGGAACAGAGGCATCAGCATCGAAGGG
GCTGGAGAAGAAAGAGAAA**TGA**TGCAGCTGGTGCCCGAGCCTCTCAGGGCCAGACCAGACAGATGGGGGCTGGG
CCCACACAGGCGTGCACCGGTAGAGGGCACAGGAGGCCAAGGGCAGCTCCAGGACAGGGCAGGGGGCAGCAGGA
TACCTCCAGCCAGGCCTCTGTGGCCTCTGTTTCCTTCTCCCTTTCTTGGCCCTCCTCTGCTCCTCCCCACACCC
TGCAGGCAAAAGAAACCCCCAGCTTCCCCCCTCCCCGGGAGCCAGGTGGGAAAAGTGGGTGTGATTTTTAGATT
TTGTATTGTGGACTGATTTTGCCTCACATTAAAAACTCATCCCATGGCCAGGGCGGGCCACTGTAAAAAAAAAA
AAAAAAAA

# FIGURE 58

MTIALLGFAIFLLHCATCEKPLEGILSSSAWHFTHSHYNATIYENSSPKTYVESFEKMGIYLAEPQWAVRYRII
SGDVANVFKTEEYVVGNFCFLRIRTKSSNTALLNREVRDSYTLIIQATEKTLELEALTRVVVHXXXXXXXXXXA
DLGQNAEFYYAFNTRSEMFAIHPTSGVVTVAGKLNVTWRGKHELQVLAVDRMRKISEGNGFGSLAALVVHVEPA
LRKPPAIASVVVTPPDSNDGTTYATVLVDANSSGAEVESVEVVGGDPGKHFKAIKSYARSNEFSLVSVKDINWM
EYLHGFNLSLQA

Signal sequence.
amino acids 1-18

N-glycosylation sites.
amino acids 39-42, 183-186, 253-256, 303-306

cAMP- and cGMP-dependent protein kinase phosphorylation site.
amino acids 201-204

N-myristoylation sites.
amino acids 24-29, 59-64, 206-211, 210-215, 242-247, 266-271

# FIGURE 59

MYHSLSETRHPLQPEEQEVGIDPLSSYSNKSGGDSNKNGRRTSSTLDSEGTFNSYRKEWEELFVNNNYLATIRQ
KGINGQLRSSRFRSICWKLFLCVLPQDKSQWISRIEELRAWYSNIKEIHITNPRKVVGQQDLMINNPLSQDEGS
LWNKFFQDKELRSMIEQDVKRTFPEMQFFQQENVRKILTDVLFCYARENEQLLYKQGMHELLAPIVFVLHCDHQ
AFLHASESAQPSEEMKTVLNPEYLEHDAYAVFSQLMETAEPWFSTFEHDGQKGKETLMTPIPFARPQDLGPTIA
IVTKVNQIQDHLLKKHDIELYMHLNRLEIAPQIYGLRWVRLLFGREFPLQDLLVVWDALFADGLSLGLVDYIFV
AMLLYIRDALISSNYQTCLGLLMHYPFIGDVHSLILKALFLRDPKRNPRPVTYQFHPNLDYYKARGADLMNKSR
TNAKGAPLNINKVSNSLINFGRKLISPAMAPGSAGGPVPGGNSSSSSSVVIPTRTSAEAPSHHLQQQQQQRLM
KSESMPVQLNKGLSSKNISSSPSVESLPGGREFTGSPPSSATKKDSFFSNISRSRSHSKTMGRKESEEELEAQI
SFLQGQLNDLDAMCKYCAKVMDTHLVNIQDVILQENLEKEDQILVSLAGLKQIKDILKGSLRFNQSQLEAEENE
QITIADNHYCSSGQGQGRGQGQSVQMSGAIKQASSETPGCTDRGNSDDFILISKDDDGSSARGSFSGQAQPLRT
LRSTSGKSQAPVCSPLVFSDPLMGPASASSSNPSSSPDDDSSKDSGFTIVSPLDI

Transmembrane domains.
amino acids 352-372, 385-405

N-glycosylation sites.
amino acids 29-32, 441-444, 486-489, 535-538, 568-571, 656-659

Glycosaminoglycan attachment site.
amino acids 678-681

cAMP- and cGMP-dependent protein kinase phosphorylation sites.
amino acids 40-43, 561-564, 581-584

Tyrosine kinase phosphorylation sites.
amino acids 195-202, 310-317

N-myristoylation sites.
amino acids 32-37, 50-55, 79-84, 147-152, 359-364, 449-454, 484-489, 485-490, 553-558, 679-684, 681-686, 685-690, 729-734

Amidation sites.
amino acids 38-41, 464-467, 579-582

TBC domain.
amino acids 78-383

# FIGURE 60

MSDTSESGAGLTRFQAEASEKDSSSMMQTLLTVTQNVEVPETPKASKALEVSEDVKVSKASGVSKATEVSKTPE
AREAPATQASSTTQLTDTQVLAAENKSLAADTKKQNADPQAVTMPATETKKVSHVADTKVNTKAQETEAAPSQA
PADEPEPESAAAQSQENQDTRPKVKAKKARKVKHLDGEEDGSSDQSQASGTTGGRRVSKALMASMARRASRGPI
AFWARRASRTRLAAWARRALLSLRSPKARRGKARRRAAKLQSSQEPEAPPPRDVALLQGRANDLVKYLLAKDQT
KIPIKRSDMLKDIIKEYTDVYPEIIERAGYSLEKVFGIQLKEIDKNDHLYILLSTLEPTDAGILGTTKDSPKLG
LLMVLLSIIFMNGNRSSEAVIWEVLRKLGLRPGIHHSLFGDVKKLITDEFVKQKYLDYARVPNSNPPEYEFFWG
LRSYYETSKMKVLKFACKVQKKDPKEWAAQYREAMEADLKAAAEAAAEAKARAEIRARMGIGLGSENAAGPCNW
DEADIGPWAKARIQAGAEAKAKAQESGSASTGASTSTNNSASASASTSGGFSAGASLTATLTFGLFAGLGGAGA
STSGSSGACGFSYK

Transmembrane domains.
amino acids 367-387, 571-591

N-glycosylation sites.
amino acids 99-102, 384-387, 556-559

Glycosaminoglycan attachment site.
amino acids 7-10

cAMP- and cGMP-dependent protein kinase phosphorylation sites.
amino acids 124-127, 203-206, 215-218, 227-230

Tyrosine kinase phosphorylation site.
amino acids 282-289

N-myristoylation sites.
amino acids 8-13, 62-67, 198-203, 358-363, 383-388, 399-404, 403-408, 504-509, 508-513, 545-550, 550-555, 567-572, 572-577, 582-587, 586-591, 588-593, 589-594, 591-596, 596-601

Amidation site.
amino acids 201-204

MAGE family.
amino acids 68-401

# FIGURE 61

MPEEGSGCSVRRRPYGCVLRAALVPLVAGLVICLVVCIQRFAQAQQQLPLESLGWDVAELQLNHTGPQQDPRLY
WQGGPALGRSFLHGPELDKGQLRIHRDGIYMVHIQVTLAICSSTTASRHHPTTLAVGICSPASRSISLLRLSFH
QGCTIASQRLTPLARGDTLCTNLTGTLLPSRNTDETFFGVQWVRP

Transmembrane domain.
amino acids 21-40

N-glycosylation site.
amino acids 63-66, 170-173

Tyrosine kinase phosphorylation site.
amino acids 97-104

N-myristoylation sites.
amino acids 5-10, 29-34, 150-155

Cell attachment sequence.
amino acids 163-166

TNF(Tumor Necrosis Factor) family.
amino acids 66-191

TNF family proteins.
amino acids 83-117

# FIGURE 62

MLVLLAFIIAFHITSAALLFIATVDNAWWVGDEFFADVWRICTNNTNCTVINDSFQEYSTLQAVQATMILSTIL
CCIAFFIFVLQLFRLKQGERFVLTSIIQLMSCLCVMIAASIYTDRREDIHDKNAKFYPVTREGSYGYSYILAWV
AFACTFISGMMYLILRKRK

Transmembrane domains.
amino acids 1-17, 64-84, 94-114, 137-157

N-glycosylation sites.
amino acids 44-47, 47-50, 52-55

# FIGURE 63

MAVRRDSVWKYCWGVLMVLCRTAISKSIVLEPIYWNSSNSKFLPGQGLVLYPQIGDKLDIICPKVDSKTVGQYE
YYKVYMVDKDQADRCTIKKENTPLLNCAKPDQDIKFTIKFQEFSPNLWGLEFQKNKDYYIISTSNGSLEGLDNQ
EGGVCQTRAMKILMKVGQDASSAGSTRNKDPTRRPELEAGTNGRSSTTSPFVKPNPGSSTDGNSAGHSGNNILG
SEVALFAGIASGCIIFIVIIITLVVLLLKYRRRHRKHSPQHTTTLSLSTLATPKRSGNNNGSEPSDIIIPLRTA
DSVFCPHYEKVSGDYGHPVYIVQEMPPQSPANIYYKV

Signal sequence.
amino acids 1-25

Transmembrane domain.
amino acids 227-247

N-glycosylation sites.
amino acids 36-39, 139-142, 282-285

cAMP- and cGMP-dependent protein kinase phosphorylation site.
amino acids 4-7, 257-260

N-myristoylation sites.
amino acids 140-145, 151-156, 165-170, 172-177, 210-215, 222-227, 230-235, 279-284, 283-288

Ephrin.
amino acids 25-166

# FIGURE 64

MGARGALLLALLLARAGLRKPESQEAAPLSGPCGRRVITSRIVGGEDAELGRWPWQGSLRLWDSHVCGVSLLSH
RWALTAAHCFETYSDLSDPSGWMVQFGQLTSMPSFWSLQAYYTRYFVSNIYLSPRYLGNSPYDIALVKLSAPVT
YTKHIQPICLQASTFEFENRTDCWVTGWGYIKEDEALPSPHTLQEVQVAIINNSMCNHLFLKYSFRKDIFGDMV
CAGNAQGGKDACFGDSGGPLACNKNGLWYQIGVVSWGVGCGRPNRPGVYTNISHHFEWIQKLMAQSGMSQPDPS
WPLLFFPLLWALPLLGPV

Signal sequence.
amino acids 1-18

Transmembrane domain.
amino acids 295-313

N-glycosylation sites.
amino acids 167-170, 200-203, 273-276

N-myristoylation sites.
amino acids 2-7, 44-49, 101-106, 225-230, 229-234, 239-244, 259-264, 269-274

Amidation site.
amino acids 33-36

Serine proteases, trypsin family, histidine active site.
amino acids 78-83

Trypsin.
amino acids 42-281

Apple domain proteins.
amino acids 41-81, 38-78, 98-128, 150-180, 224-257, 260-287

Kringle domain proteins.
amino acids 67-84, 232-244

Serine proteases, trypsin.
amino acids 67-83, 234-244, 258-281

# FIGURE 65

MLQDPDSDQPLNSLDVKPLRKPRIPMETFRKVGIPIIIALLSLASIIIVVVLIKVILDKYYFLCGQPLHFIPRK
QLCDGELDCPLGEDEEHCVKSFPEGPAVAVRLSKDRSTLQVLDSATGNWFSACFDNFTEALAETACRQMGYSRA
VEIGPDQDLDVVEITENSQELRMRNSSGPCLSGSLVSLHCLACGKSLKTPRVVGGEEASVDSWPWQVSIQYDKQ
HVCGGSILDPHWVLTAAHCFRKHTDVFNWKVRAGSDKLGSFPSLAVAKIIIIEFNPMYPKDNDIALMKLQFPLT
FSGTVRPICLPFFDEELTPATPLWIIGWGFTKQNGGKMSDILLQASVQVIDSTRCNADDAYQGEVTEKMMCAGI
PEGGVDTCQGDSGGPLMYQSDQWHVVGIVSWGYGCGGPSTPGVYTKVSAYLNWIYNVWKAEL

Transmembrane domain.
amino acids 33-60

Trypsin.
amino acids 200-424

Kringle domain proteins.
amino acids 225-242, 376-388

Apple domain proteins.
amino acids 199-239, 196-236, 237-270, 298-328, 368-401, 367-400, 403-430

LDL-receptor class A.
amino acids 70-89

CUB domain proteins.
amino acids 397-406

# FIGURE 66

MAGLAARLVLLAGAAALASGSQGDREPVYRDCVLQCEEQNCSGGALNHFRSRQPIYMSLAGWTCRDDCKYECMW
VTVGLYLQEGHKVPQFHGKWPFSRFLFFQEPASAVASFLNGLASLVMLCRYRTFVPASSPMYHTCVAFAWVSLN
AWFWSTVFHTRDTDLTEKMDYFCASTVILHSIYLCCVRTVGLQHPAVVSAFRALLLLMLTVHVSYLSLIRFDYG
YNLVANVAIGLVNVVWWLAWCLWNQRRLPHVRKCVVVVLLLQGLSLLELLDFPPLFWVLDAHAIWHISTIPVHV
LFFSFLEDDSLYLLKESEDKFKLD

Signal peptide.
amino acids 1-20

Transmembrane domains.
amino acids 101-121, 192-212, 221-241, 254-274, 287-307

N-glycosylation site.
amino acids 40-44

N-myristoylation site.
amino acids 43-49

CUB domain proteins profile.
amino acids 285-302

Amiloride-sensitive sodium channels proteins.
amino acids 162-186

# FIGURE 67

MIRCGLACERCRWILPLLLLSAIAFDIIALAGRGWLQSSDHGQTSSLWWKCSQEGGGSGSYEEGCQSLMEYAWG
RAAAAMLFCGFIILVICFILSFFALCGPQMLVFLRVIGGLLALAAVFQIISLVIYPVKYTQTFTLHANPAVTYI
YNWAYGFGWAATIILIGCAFFFCCLLNYEDDLLGNAKPRYFYTSA

Signal sequence.
amino acids 1-24

Transmembrane domains.
amino acids 75-95, 105-125, 121-141, 141-161, 157-177

N-myristoylation sites.
amino acids 42-47, 55-60, 56-61, 112-117

# FIGURE 68

MGRPRLTLVCQVSIIISARDLSMNNLTELQPGLFHHLRFLEELRLSGNHLSHIPGQAFSGLYSLKILMLQNNQL
GGIPAEALWELPSLQSLRLDANLISLVPERSFEGLSSLRHLWLDDNALTEIPVRALNNLPALQAMTLALNRISH
IPDYAFQNLTSLVVLHLHNNRIQHLGTHSFEGLHNLETLDLNYNKLQEFPVAIRTLGRLQELGFHNNNIKAIPE
KAFMGNPLLQTIHFYDNPIQFVGRSAFQYLPKLHTLSLNGAMDIQEFPDLKGTTSLEILTLTRAGIRLLPSGMC
QQLPRLRVLELSHNQIEELPSLHRCQKLEEIGLQHNRIWEIGADTFSQLSSLQALDLSWNAIRSIHPEAFSTLH
SLVKLDLTDNQLTTLPLAGLGGLMHLKLKGNLALSQAFSKDSFPKLRILEVPYAYQCCPYGMCASFFKASGQWE
AEDLHLDDEESSKRPLGLLARQAENHYDQDLDELQLEMEDSKPHPSVQCSPTPGPFKPCEYLFESWGIRLAVWA
IVLLSVLCNGLVLLTVFAGGPAPLPPVKFVVGAIAGANTLTGISCGLLASVDALTFGQFSEYGARWETGLGCRA
TGFLAVLGSEASVLLLTLAAVQCSVSVSCVRAYGKSPSLGSVRAGVLGCLALAGLAAALPLASVGEYGASPLCL
PYAPPEGQPAALGFTVALVMMNSFCFLVVAGAYIKLYCDLPRGDFEAVWDCAMVRHVAWLIFADGLLYCPVAFL
SFASMLGLFPVTPEAVKSVLLVVLPLPACLNPLLYLLFNPHFRDDLRRLRPRAGDSGPLAYAAAGELEKSSCDS
TQALVAFSDVDLILEASEAGRPPGLETYGFPSVTLISCQQPGAPRLEGSHCVEPEGNHFGNPQPSMDGELLLRA
EGSTPAGGGLSGGGGFQPSGLAFASHV

Signal sequence.

amino acids 1-18

Transmembrane domains.

amino acids 512-532, 552-572, 594-614, 634-654, 678-698, 719-739, 757-777

N-glycosylation sites.

amino acids 25-28, 156-159

Glycosaminoglycan attachment site.

amino acids 899-902

Tyrosine kinase phosphorylation sites.

amino acids 417-423, 465-471

N-myristoylation sites.

amino acids 47-52, 55-60, 75-80, 328-333, 431-436, 441-446, 511-516, 550-555, 560-565, 564-569, 589-594, 600-605, 632-637, 637-642, 646-651, 673-678, 870-875, 890-895, 895-900, 896-901, 897-902, 908-913

Microbodies C-terminal targeting signal.

amino acids 913-916

Cell attachment sequence.

amino acids 708-710

Leucine Rich Repeats.

amino acids 20-38, 39-62, 63-86, 87-110, 111-134, 135-158, 159-182, 183-205, 206-229, 230-253, 254-276, 277-300, 301-322, 323-346, 347-370, 371-394

G-protein coupled receptor.

amino acids 617-647, 755-785, 527-557, 585-615, 583-623, 726-766, 723-763, 754-773, 767-783

# FIGURE 69

MEALLLGAGLLLGAYVLVYYNLVKAPPCGGMGNLRGRTAVVTGANSGIGKMTALELARRGARVVLACRSQERGE
AAAFDLRQESGNNEVIFMALDLASLASVRAFATAFLSSEPRLDILIHNAGISSCGRTREAFNLLLRVNHIGPFL
LTHLLLPCLKACAPSRVVVVASAAHCRGRLDFKRLDRPVVGWRQELRAYADTKLANVLFARELANQLEATGVTC
YAAHPGPVNSELFLRHVPGWLRPLLRPLAWLVLRAPRGGAQTPLYCALQEGIEPLSGRYFANCHVEEVPPAARD
DRAAHRLWEASKRLAGLGPGEDAEPDEDPQSEDSEAPSSLSTPHPEEPTVSQPYPSPQSSPDLSKMTHRIQAKV
EPEIQLS

Signal sequence.
amino acids 1-16

Transmembrane domain.
amino acids 139-159

Glycosaminoglycan attachment site.
amino acids 46-49

N-myristoylation sites.
amino acids 9-14, 29-34, 32-37, 43-48, 124-129, 312-317

Short chain dehydrogenase.
amino acids 35-288

Short-chain alcohol dehydrogenase.
amino acids 37-49, 114-124, 138-147, 219-228

# FIGURE 70

MAASLGQVLALVLVAALWGGTQPLLKRASAGLQRVHEPTWAQQLLQEMKTLFLNTEYLMPFLLNQCGSLLYYLT
LASTDLTLAVPICNSLAIIFTLIVGKALGEDIGGKRKLDYCECGTQLCGSRHTCVSSFPEPISPEWVRTRPFPI
LPFPLQLFCFLVAIRVPFPWTVWRKTEAGVWD

Signal sequence.
1-19

Transmembrane domains.
amino acids 81-101, 146-166

cAMP- and cGMP-dependent protein kinase phosphorylation site.
amino acids 26-29

N-myristoylation sites.
amino acids 6-11, 118-123, 123-128

Amidation site.
amino acids 107-110

# FIGURE 71

MVSSGCRMRSLWFIIVISFLPNTEGFSRAALPFGLVRRELSCEGYSIDLRCPGSDVIMIESANYGRTDDKICDA
DPFQMENTDCYLPDAFKIMTQRCNNRTQCIVVTGSDVFPDPCPGTYKYLEVQYECVPYIFVCPGTLKAIVDSPC
IYEAEQKAGAWCKDPLQAADKIYFMPWTPYRTDTLIEYASLEDFQNSRQTTTYKLPNRVDGTGFVVYDGAVFFN
KERTRNIVKFDLRTRIKSGEAIINYANYHDTSPYRWGGKTDIDLAVDENGLWVIYATEQNNGMIVISQLNPYTL
RFEATWETVYDKRAASNAFMICGVLYVVRSVYQDNESETGKNSIDYIYNTRLNRGEYVDVPFPNQYQYIAAVDY
NPRDNQLYVWNNNFILRYSLEFGPPDPAQVPTTAVTITSSAELFKTIISTTSTTSQKGPMSTTVAGSQEGSKGT
KPPPAVSTTKIPPITNIFPLPERFCEALDSKGIKWPQTQRGMMVERPCPKGTRGTASYLCMISTGTWNPKGPDL
SNCTSHWVNQLAQKIRSGENAASLANELAKHTKGPVFAGDVSSSVRLMEQLVDILDAQLQELKPSEKDSAGRSY
NKAIVDTVDNLLRPEALESWKHMNSSEQAHTATMLLDTLEEGAFVLADNLLEPTRVSMPTENIVLEVAVLSTEG
QIQDFKFPLGIKGAGSSIQLSANTVKQNSRNGLAKLVFIIYRSLGQFLSTENATIKLGADFIGRNSTIAVNSHV
ISVSINKESSRVYLTDPVLFTLPHIDPDNYFNANCSFWNYSERTMMGYWSTQGCKLVDTNKTRTTCACSHLTNF
AILMAHREIAYKDGVHELLLTVITWVGIVISLVCLAICIFTFCFFRGLQSDRNTIHKNLCINLFIAEFIFLIGI
DKTKYAIACPIFAGLLHFFFLAAFAWMCLEGVQLYLMLVEVFESEYSRKKYYYVAGYLFPATVVGVSAAIDYKS
YGTEKACWLHVDNYFIWSFIGPVTFIILLNIIFLVITLCKMVKHSNTLKPDSSRLENIKSWVLGAFALLCLLGL
TWSFGLLFINEETIVMAYLFTIFNAFQGVFIFIFHCALQKKVRKEYGKCFRHSYCCGGLPTESPHSSVKASTTR
TSARYSSGTQSRIRRMWNDTVRKQSESSFISGDINSTSTLNQGHSLNNARDTSAMDTLPLNGNFNNSYSLHKGD
YNDSVQVVDCGLSLNDTAFEKMIISELVHNNLRGSSKTHNLELTLPVKPVIGGSSSEDDAIVADASSLMHSDNP
GLELHHKELEAPLIPQRTHSLLYQPQKKVKSEGTDSYVSQLTAEAEDHLQSPNRDSLYTSMPNLRDSPYPESSP
DMEEDLSPSRRSENEDIYYKSMPNLGAGHQLQMCYQISRGNSDGYIIPINKEGCIPEGDVREGQMQLVTSL

Signal sequence.
amino acids 1-25
Transmembrane domains.
amino acids 833-853, 871-891, 905-925, 939-959, 980-1000, 1020-1040, 1052-1072
N-glycosylation sites.
amino acids 99-102, 331-334, 520-523, 616-619, 718-721, 731-734, 774-777, 779-782, 800-803, 1128-1131, 1145-1148, 1175-1178, 1186-1189, 1199-1202
cAMP- and cGMP-dependent protein kinase phosphorylation site.
amino acids 1132-1135
Tyrosine kinase phosphorylation sites.
amino acids 121-127, 337-344, 1343-1351
N-myristoylation sites.
amino acids 138-143, 436-441, 440-445, 495-500, 676-681, 679-684, 711-716, 841-846, 887-892, 953-958, 964-969, 1035-1040, 1093-1098, 1172-1177, 1195-1200, 1218-1223, 1236-1241, 1372-1377
Protein prenyltransferases alpha subunit repeat signature.
amino acids 973-982
Latrophilin Cytoplasmic C-terminal region.
amino acids 1080-1403
7 transmembrane receptor (Secretin family).
amino acids 831-1078
Galactose binding lectin domain.
amino acids 49-129
Latrophilin/CL-1-like GPS domain.
amino acids 771-823
Hormone receptor domain.
amino acids 466-526

# FIGURE 72

MADPHQLFDDTSSAQSRGYGAQRAPGGLSYPAASPTPHAAFLADPVSNMAMAYGSSLAAQGKELVDKNIDRFIP
ITKLKYYFAVDTMYVGRKLGLLFFPYLHQDWEVQYQQDTPVAPRFDVNAPDLYIPAMAFITYVLVAGLALGTQD
RFSPDLLGLQASSALAWLTLEVLAILLSLYLVTVNTDLTTIDLVAFLGYKYVGMIGGVLMGLLFGKIGYYLVLG
WCCVAIFVFMIRTLRLKILADAAAEGVPVRGARNQLRMYLTMAVAAAQPMLMYWLTFHLVR

Transmembrane domains.
amino acids 123-143, 162-182, 192-212, 208-228, 224-244, 259-279

N-myristoylation sites.
amino acids 54-59, 141-146, 156-161, 201-206, 205-210, 209-214

Amidation site.
amino acids 89-92

# FIGURE 73

MALLPIFFGALRSVRCARGKNASDMPETITSRDAARFPIIASCTLLGLYLFFKIFSQEYINLLLSMYFFVLGIL
ALSHTISPFMNKFFPASFPNRQYQLLFTQGSGENKEEIINYEFDTKDLVCLGLSSIVGVWYLLRKHWIANNLFG
LAFSLNGVELLHLNNVSTGCILLGGLFIYDVFWVFGTNVMVTVAKSFEAPIKLVFPQDLLQKGLEANNFAMLGL
GDVVIPGIFIALLLRFDISLKKNTHTYFYTSFAAYIFGLGLTIFIMHIFKHAQPALLYLVPACIGFPVLVALAK
GEVTEMFSYEESNPKDPAAVTESKEGTEASASKGLEKKEK

Signal sequence.
amino acids 1-16

Transmembrane domains.
amino acids 32-52, 49-69, 65-85, 116-136, 157-177, 173-193, 217-237, 252-272, 273-293

N-glycosylation sites.
amino acids 21-24, 163-166

cAMP- and cGMP-dependent protein kinase phosphorylation site.
amino acids 243-246

N-myristoylation sites.
amino acids 9-14, 104-109, 148-153, 211-216, 229-234, 260-265, 322-327

# FIGURE 74A

GGGCGCAGAGGAGGAAAGGGAGCAGGCGCAGGGGGACTGGAAAGGCAGCATGCGCTCGCCAGGAGCAACCTCGG
CGCCCAGGGTCTGAGGCTGCAGCCCCAGTTCGCCATTGTGAGCCGCCGCCGGGGGAGTCCGCTAGCGCAGCCGT
GCCCCCGAGTCCCCGTCCGCGCAGCG**ATG**GGGCACCTGCCCACGGGGATACACGGCGCCCGCCGCCTCCTGCCT
CTGCTCTGGCTCTTTGTGCTGTTCAAGAATGCTACAGCTTTCCATGTAACTGTCCAAGATGATAATAACATCGT
TGTCTCATTAGAAGCTTCAGACGTCATCAGTCCAGCATCTGTGTATGTTGTGAAGATAACTGGTGAATCCAAAA
ATTATTTCTTCGAATTTGAGGAATTCAACAGCACTTTGCCTCCTCCTGTTATTTTCAAGGCCAGTTATCATGGC
CTTTATTATATAATCACTCTGGTAGTGGTAAATGGAAATGTGGTGACCAAGCCATCCAGATCAATCACTGTGTT
AACAAAACCTCTACCTGTAACCAGTGTTTCCATATATGACTATAAACCTTCTCCTGAAACAGGAGTCCTGTTTG
AAATACATTATCCAGAAAAATATAACGTTTTCACAAGAGTGAACATTAGCTACTGGGAAGGTAAAGACTTCCGG
ACAATGCTATATAAAGATTTCTTTAAGGGAAAAACAGTATTTAATCACTGGCTGCCAGGAATGTGTTATAGTAA
TATCACCTTTCAGCTGGTATCTGAGGCAACTTTTAATAAAAGTACCCTTGTTGAGTACAGTGGTGTCAGTCACG
AACCCAAACAGCACAGAACTGCCCCTTATCCACCTCAAAATATTTCCGTTCGTATCGTAAACTTGAACAAAAAC
AACTGGGAAGAACAGAGTGGCAATTTCCCAGAAGAATCCTTCATGAGATCACAAGATACAATAGGAAAAGAAAA
ACTCTTCCATTTTACAGAAGAAACCCCTGAAATTCCCTCGGGCAACATTTCTTCCGGTTGGCCTGATTTTAATA
GCAGTGACTATGAAACTACGTCTCAGCCATATTGGTGGGACAGTGCATCTGCAGCTCCTGAAAGTGAAGATGAA
TTTGTCAGCGTACTTCCCATGGAATACGAAAATAACAGTACACTCAGTGAGACAGAGAAGTCAACATCAGGCTC
TTTCTCCTTTTTCCCTGTGCAAATGATATTGACCTGGTTACCACCCAAACCACCCACTGCTTTTGATGGGTTCC
ATATCCATATTGAACGAGAAGAGAACTTTACTGAATATTTGATGGTGGATGAAGAAGCACATGAATTTGTTGCA
GAACTGAAGGAACCTGGGAAATATAAGTTATCTGTGACAACCTTTAGTTCCTCAGGATCTTGTGAAACTCGAAA
AAGTCAGTCAGCAAAATCACTCAGCTTTTATATCAGTCCTTCAGGAGAGTGGATTGAAGAACTGACCGAGAAGC
CGCAGCACGTGAGTGTCCACGTTTTAAGCTCAACCACTGCCTTGATGTCCTGGACATCTTCCCAAGAGAACTAC
AACAGCACCATTGTGTCTGTGGTGTCGCTGACCTGCCAGAAACAAAAGGAGAGCCAGAGGCTTGAAAAGCAGTA
CTGCACTCAGGTGAACTCAAGCAAACCTATTATTGAAAATCTGGTTCCTGGTGCCCAGTACCAGGTTGTAATAT
ACCTAAGGAAAGGCCCTTTGATTGGACCACCTTCAGATCCTGTGACATTTGCTATTGTTCCCACAGGAATAAAG
GATTTAATGCTCTATCCTTTGGGTCCTACGGCCGTGGTTCTGAGCTGGACCAGACCTTATTTAGGCGTGTTCAG
AAAATACGTGGTTGAAATGTTTTATTTCAACCCTGCTACAATGACATCAGAGTGGACCACCTACTATGAAATAG
CAGCAACTGTTTCCTTAACTGCATCCGTGAGAATAGCTAATCTGCTGCCAGCATGGTACTACAACTTCCGGGTT
ACCATGGTGACGTGGGGAGATCCAGAATTGAGCTGCTGTGACAGCTCTACCATCAGCTTCATAACAGCCCCAGT
GGCTCCGGAAATCACTTCTGTGGAATATTTCAACAGTCTGTTATATATCAGTTGGACATATGGGGATGATACAA
CGGACTTGTCCCATTCTAGAATGCTTCACTGGATGGTGGTTGCAGAAGGAAAAAAGAAAATTAAAAAGAGTGTA
ACACGCAATGTCATGACTGCAATTCTCAGCTTGCCTCCAGGCGACATCTATAACCTCTCAGTAACTGCTTGTAC
TGAAAGAGGAAGTAATACCTCCATGCTCCGCCTTGTCAAGCTAGAACCAGCTCCACCCAAATCACTCTTCGCAG
TGAACAAAACCCAGACTTCAGTGACTTTGCTGTGGGTGGAAGAGGGAGTAGCTGATTTCTTTGAAGTTTTCTGT
CAACAAGTTGGCTCCAGTCAGAAAACCAAACTTCAGGAACCAGTTGCTGTTTCTTCCCATGTCGTGACCATCTC
CAGCCTTCTTCCTGCCACTGCCTACAATTGTAGTGTCACCAGCTTTAGCCATGACAGCCCCAGTGTCCCTACGT
TCATAGCCGTCTCAACAATGGTTACAGAGATGAATCCCAATGTGGTAGTGATCTCCGTGCTGGCCATCCTTAGC
ACACTTTTAATTGGACTGTTGCTTGTTACCCTCATTATTCTTAGGAAAAAGCATCTGCAGATGGCTAGGGAGTG
TGGAGCTGGTACATTTGTCAATTTTGCATCCTTAGAGAGGGATGGAAAGCTTCCATACAACTGGAGTAAAAATG
GTTTAAAGAAGAGGAAACTGACAAACCCGGTTCAACTGGATGACTTTGATGCCTATATTAAGGATATGGCCAAA
GACTCTGACTATAAATTTTCTCTTCAGTTTGAGGAGTTGAAATTGATTGGACTGGATATCCCACACTTTGCTGC
AGATCTTCCACTGAATCGATGTAAAAACCGTTACACAAACATCCTACCATATGACTTCAGCCGTGTGAGATTAG
TCTCCATGAATGAAGAGGAAGGTGCAGACTACATCAATGCCAACTATATTCCTGGATACAACTCACCCCAGGAG
TATATTGCCACCCAGGGGCCACTGCCTGAAACCAGAAATGACTTCTGGAAGATGGTCCTGCAACAAAAGTCTCA
GATTATTGTCATGCTCACTCAGTGTAATGAGAAAAGGAGGGTGAAATGTGACCATTACTGGCCATTCACGGAAG
AACCTATAGCCTATGGAGACATCACTGTGGAGATGATTTCAGAGGAAGAGCAGGACGACTGGGCCTGTAGACAC
TTCCGGATCAACTATGCTGACGAGATGCAGGATGTGATGCATTTTAACTACACTGCATGGCCTGATCATGGTGT
GCCCACAGCAAATGCTGCAGAAAGTATCCTGCAGTTTGTACACATGGTCCGACAGCAAGCTACCAAGAGCAAAG
GTCCCATGATCATTCACTGCAGTGCTGGCGTGGGACGGACAGGAACATTCATTGCCCTGGACAGGCTCTTGCAG
CACATTCGGGATCATGAGTTTGTTGACATCTTAGGGCTGGTGTCAGAAATGAGGTCATACCGGATGTCTATGGT
ACAGACAGAGGAGCAGTACATTTTTATCCATCAGTGTGTGCAACTGATGTGGATGAAGAAGAAGCAGCAGTTCT
GCATCAGTGATGTCATATACGAGAATGTTAGCAAGTCC**TAG**TTCAGAATCCGGAGCAGAGAGGACATGATGTGC
GCCCATCCTCCCTTGCTTCCAGATTGTTTTAGTGGGCCCTGATGGTCATTTTTCTAAACAGAGGCCCTGCTTTG
TAATATGTGGCCAAGGAGATAATTTATCTCACAGAAGCACCGGGAAGACTTAGCCTTAAAGAGCCTACAGTGTC
CTTTTGGACTCTTTCACTTCGGGACATTTAATAATGGACCAAATTCAACAGAACACCAGGAAGGTCAAGACGCT

# FIGURE 74B

CTCCAAAGGGCAGGAAGTACAGCACTTCCGAAGAGTTTAGTTGGCCCTTTGCTGGTTGGGCTGAGTTTTTTATT
TTTAAGTGTTTGTTTTTCAGTGCAATAATTTTTGTGTGTGTGTGATTCTTATCAGAAAGTTGAATTGTTTTCTG
CCTACACCGTTCATCAGCCCCATAACCCAGGAAGGAACAGGCATTGTTAGCATCAGATTATACCTCATTATTAA
AAGGAGGCATGGCCACACATGAAGAAATGGTCATTCTACTTCAAAGAAATTGAGCCAGCACTATCTGTACTCCA
ACATTACCGGATCTGGATTGGGGAGGTTGGTCAGGGAAGAGAGGGGTTCTACCCACAGATCAACTGTGTAATCT
TTTACTATTCAAGCTATAATTCAGCTTCAAAGTAGAGTAGAAAAAAAATTGTCTTAACTGTTCTAGTTCTTGAT
GGTTTTCTTCCTTATTAACAGTTGGTGTTTCTTCCTTGGCCCTTTTGGACTAATGTTACTGTCCAAGTTCTTTC
TCAAGAAACCACATCTGGTTCAGAAGAGTGTCAAGTTGGACTCTTTGAACTCTGTTGCTGTCTGAGCAATCGTG
GTGCCTAGACTTTGCATTCCTTGTTCTGTTGACCTGCATACATGTGAGAGCTATTTCTTTAAGAACTATATAGG
CTGTGAAAACGCACTTTCTTTCCCCCAAAGAGCTGGGAATTTATGAAGTTATGGCAATGAACTGCAGCATGCTG
GGACAATTATTTGACTACTTTTTTTTGTAATATTGTCAAATGTCTCTATGGATTCTGACAGAGATTTCTTTTTG
TTTTGTTATTCTTTTGGTTGTCAGTTTCATTTTAACGAGTGTAACTAGTAACATTTTATTCTTTGGATTTTGTA
TAATTACAGTACATGATTGTGTATTGTGACATGAATGCTGTCAAAATGACATTGATGGCATTGTGAAGCCTGTT
ACTTTGTGTCACTTCCTGATAAATAAGAGGTGATGACATGGATATACAACAGAAAACACTTTGAGTTGAAAGTA
AACACAAGCTGGCTGCTTCCCTGTGGCAACTGTGGCT

# FIGURE 75

GCAAAGGTGACTGGCTTCAGTGAAGGTGTGGTGGATAGTGTCAAAGGTGGGTTTTCCAGCTTCTCCCAGGCCAC
CCATTCAGCAGCAGGCGCTGTAGTCTCAAAGCCCAGAGAGATTGCCTCACTCATTCGGAACAAATTTGGCAGTG
CAGACAACATCCCCAACCTGAAGGACTCTTTAGAGGAAGGGCAAGTGGATGATGCGGGGAAGGCTTTGGGAGTG
ATTTCAAACTTTCAGTCTAGCCCAAAATATGGTAGTGAAGAAGATTGTTCTAGTGCCACTTCAGGCTCAGTGGG
AGCCAACAGCACCACAGGGGGCATCGCTGTAGGAGCATCCAGCTCCAAAACAAACACCCTGGACATGCAGAGCT
CAGGATTTGATGCACTACTACATGAGATCCAGGAGATCCGGGAAACCCAGGCCAGACTAGAGGAATCCTTTGAG
ACTCTCAAGGAACATTATCAGAGGGACTATTCCTTAATAATGCAGACCTTACAGGAGGAGCGATATAGATGTGA
ACGATTGGAAGAACAGCTAAATGACCTAACAGAGCTCCACCAGAATGAAATCTTGAACTTGAAGCAGGAACTGG
CAAGCATGGAAGAAAAAATCGCGTATCAGTCCTATGAACGGGCCCGGGACATCCAGGAGGCCCTGGAGGCATGC
CAGACGCGCATCTCCAAGATGGAGCTGCAGCAGCAGCAGCAGCAGGTGGTGCAGCTAGAAGGGCTGGAGAATGC
CACTGCCCGGAACCTTCTGGGCAAACTCATCAACATCCTCCTGGCTGTCATGGCAGTCCTTTTGGTCTTTGTCT
CCACTGTAGCCAACTGTGTGGTCCCCCTCATGAAGACTCGCAACAGGACGTTCAGCACTTTATTCCTTGTGGTT
TTTATTGCCTTTCTCTGGAAGCACTGGGACGCCCTCTTCAGCTATGTGGAACGGTTCTTTTCATCCCCTAGATG
ATGCTGGCACAGAAGGCATTGTTCCCTACCCTCTGGCGAGTGCATGCAGCAGAGAGTTAGACAGCAACTTACCT
ACTCTGAAGTTTTCTACAACAAAAAAAGAGTTGAGTGAATCTGTTTACATTTAGAATAATGTTTTTTTCTTCAA
GAGACGCAATTGCAATAGTATTTTTTAGATTTTATCCAAGAAGTTTTTTGGGCGAAAATCTTGGATCATTTTTA
TGTAGCATGATTTTCCTTGGGATGCAAATCTTAAAACAGTCCTTTAATATGAACCAACAATCTGGAGCACACCG
AAGGGCAATCTAAATTGTGGCTTGAAGGACTGCACTAAAACCCACTAAAAAGATGCGAAAACCTGATGAGGGCA
AACCAGTTAAACCTAACACCCTGCCTTGTCTGGGCTCATCACCTCTCCCTATCCCAGACTAACTTTACTGTGAA
ATCCTACCACATTCCATGTCTGAATTTTTGGATTCGGGGTGGATTTTCGTTGTCCGTGGAAGAACACATGGATC
TCTCTGGCTTTCTCACCCAAGTTGGCCACTTACGCTAATCCTGGAAGTATGATCACTTTTGAACCTGCCCCTTA
ACCTTGACGAGGATACAAAAGTGAAAGCATCATCCCCCAAAGGATCACTGCACAGTCCTACTACAGTATTTTTA
AGTAGCCCTCTAAATACTTAATTTTAAGCAAAATCCCTTGGCCGCACTTTTAAGGTTTTTTTATATGTGTATAG
TTACCAACCTAAAAATAAAAAATCCGAACAGCATACTTGAAGAATGTAATACTCAAACTCTCAGTGCTTCCTTA
TGGTTTCTAATAGGATTTTTTATTATTGTTATTATTATTATTGGGTTTTTTTGGACAGGGTTGGAGGGTCTTT
TATTTTTCCTTTGAAATAAAGAAGTGATGTTTTTAAATGAAGAAATGTGTGGATATTTAAGTGTGCTGCTCCCT
CTTGTCTTGAAACAGTTTGAGTAAGAAAGTCTTGCTGTAAATGCTGCCCTCTGCCGCCTTTGTTTTGAGATGCA
GTTTAAACTCCCTCTGGCTGCTGCTGCTGCTTTTTGGTGTCCCGACATACCTACGCCCCCGTTTTATGGGTTTG
GCTTAGTTGAAGAGGAAAGGGTTGTGCAAGGAGAGCAGGAGGCTGTTTCCAAAAACCAGTGTAGTAGGATAGGG
ATTTTTTTTTTTTTTTTTGCCCCAAGAAAACGTTCACCCAGTGATCTTGGGCTGGGGTTGTCTTTAGGAAAAGT
TGAGACTATAAGAGTCATAAATAAGTCCTTGTGTTTCCTTAATTTATTTTGTTAACACCCCTAATTACAACCAA
AGTGATGATGTGGAGTCTTCTGTCTTCATTTTGGCCCCAGCATTCTTAATTTCAAAGCTTTATTCTGTCTGCCT
AAGAGAATCAACCAAAGGTGATTCTCCTAAAGAGCAGTGAAGGAAATGTCAGGTTAGCAGGACCCAAGTTTTGG
GTGTGAAATGTTGCCAGCTTCCTATAATGTAAACGGACTTGTTAACCTAACCTAATTATGCTCAGTGGACTTCT
ATAGATGGTTTTGAAAAATGAACTGAGCTGCCTTCCCGCATCGCATAACCAGTTCCATCATCCTGGTGGAACTT
GAACATTTAGAGTTTATCTAGAGAGCTTGGTTAATCTTTCCATATTATTTGTAGTATTGGTCACAAATGCTGTT
CCCTCTTAGCCTCATTCTGTGCAACCAAGTGCATATAAGATGCCCTGAAAAGAGTAACAAAGTATGCTTTGCCT
GTTTCCACTTACCAGGAAATTCCTTCAGAACTAGATTAGCATTGCCCTGCCTGTCTGAAAGGACAGTTTACCTA
ATGGTGCCAGCCTCCTTTTGCTTTGGCAAGCTGGATTTCTCAGAGCCAGCATGTTGTTTCCATAACTACTTTGA
TATTTTAACTCAGGTACTCCAGTCTTCACCCCAACCTCAGCTGATTGTAGTACACCTGCTAGCTCTGTTGCCCC
CTCAAAACTGCACCCAGAGCAGGGCCACAAGGGTGCTTTTTTTTCTTTAAAAAAAAAAAAAATTAGAACCAATTC
ATGTTCATGCCAAAAACAAATTGTCCCCAAGCCTATATGTATTAAAATGTTAACTTTGCCTAAAAATATTGCAG
TGACTTTTTAGGCAGGAGTGCCAAAGGACACTATGAACTTTTTGAACTGACAGTTTCTCCTAACTTTCTGCTTT
AGCGTAATTGCTCAGAGTAGAGAGCCCCCACAAAGTTATTTAAAAGATGCCCTAGCAGCAATCCACCAGTTTTT
CTAAGCTAGAACCTTTGAGTCCCCCAAACTGCCTGAAGACTTAAGTTTTGTGGGCACTGGAAGTCACTTTGATA
GATGGATTGAAACTGTTCCTATTTGCCCTGGGACGGTTTCTATCTATCAAAGGAAGGTTTTCACCTGTAGAAAG
CCCCCTGCCTCCAGCCAAATAGTCCCATGCTGACTTTCTATCTTCCTTTCTCAAACTGTCTTAGGAAGGACCTT
CAGTGCAGATCAGGTGCAGTAATGGCTTTCTTGTCCCTTAATTATTCACCAGACCCAGAAGTTGTACGCATTTA
ATGCTGTTTGTAACCATGCATCTGTTTTCATTCTTTGCTGTACCTTTTGCTGCCCATCCTGTTACTTTTGAGTT
TCTTTCATTGTGGTTGTTCTTGGGTTCTTTTGTCTTGTCAGAGCTCTTCTATAACCTCGCTCTAATGGCTTAAC
AGTTGTTCTGGGTGGAAACGTCCCCTCATTTGAATGCTCCTCT

# FIGURE 76A

AGTGGAAGGAGCAGGCGCTTGAGCTCGAGCGACGGCGCTGGCGGAGACGCCGGCTGCTCCTCCCCTCCCCGCCG
GTATTAATCTCTGGAGAAGACACATCCACAGTTAGCACTTTCTTCAGATGCTGACGCTCGGTGAACAGTTGCCT
TTGGTCACAAGATTTAGAAGACACAGTGTCCATCCTCCCAGATTGGATCTCTTTTTCATATGGATCTTCTGTTT
CTATGTCTTTTTAAAAAATAACTTTTTGGGAAACCTTTTGGATTACAACTGTTCATCCTCACCTATGCAAAGAA
AGGGAAGCTATTGCTGGGATTTTGAGGAGCTTTTCCTAAAAGGATTGTACACCTTAGAAGTGCTTAAGGAAGAG
TGATGAAGATAGGCATGAAGCCTTCGTCTCACAGCTGCATGCGTAGTCACTGTTGAAGCAAATGCCTACCTAAT
TTGACACTCTTGGTGTGTTTAAAAAATTTTTTTGAGTTTGCAAATAAGCATATTAAGTCTACTG**ATG**GAGCCTT
CGGGCAGTGAACAGTTATTTGAGGACCCTGATCCTGGAGGCAAATCCCAAGATGCAGAGGCCAGAAAGCAGACA
GAATCAGAACAAAAATTGTCTAAAATGACCCACAATGCTTTGGAGAACATTAACGTGATTGGCCAAGGCTTGAA
GCATCTCTTCCAGCACCAGCGCAGGAGGTCATCAGTGTCTCCACATGATGTGCAGCAAATTCAGGCAGATCCAG
AACCTGAAATGGATCTGGAAAGCCAGAACGCATGTGCTGAGATTGATGGTGTCCCCACCCACCCCACAGCTCTG
AATCGTGTCCTGCAGCAGATTCGAGTGCCACCCXAGATGAAGAGAGGGACAAGCTTGCATAGTAGGCGGGGCAA
GCCAGAGGCCCCAAAGGGAAGTCCCCAAATCAACAGGAAGTCTGGTCAGGAGATGACAGCTGTTATGCAGTCAG
GCCGACCCATGTCTTCATCCACAACTGATGCACCTACCGGCTCTGCTATGATGGAAATAGCTTGTGCTGCTGCT
GCTGCTGCTGCTGCATGTCTACCXGGAGAGGAGGGAACTGCGGAGCGGATCGAACGGTTGGAAGTAAGCAGCCT
TGCCCAAACATCCAGTGCAGTGGCCTCCAGTACCGATGGCAGCATCCACACAGACTCTGTGGATGGAACACCAG
ACCCTCAGCGCACAAAGGCTGCCATTGCTCACCTGCAGCAGAAGATCCTGAAGCTCACAGAACXAATCAAGATT
GCACAAACAGCCCGGGACGACAACGTTGCTGAATACTTGAAGCTTGCCAACAGTGCAGACAAACAGCAGGCTGC
CCGCATCAAGCAAGTCTTTGAGAAGAAGAACCAGAAATCTGCCCXAACTATCCTCCAGCTGCAAAAGAAACTTG
AGCACTACCACAGGAAGCTCAGAGAGGTAGAGCAGAATGGGATCCCCCGGCAGCCAAAGGATGTCTTCAGGGAC
ATGCACCAGGGTCTGAAGGATGTAGGAGCAAAGGTGACTGGCTTCAGTGAAGGTGTGGTGGATAGTGTCAAAGG
TGGGTTTTCCAGCTTCTCCCAGGCCACCCATTCAGCAGCAGGCGCTGTAGTCTCAAAGCCCAGAGAGATTGCCT
CACTCATTCGGAACAAATTTGGCAGTGCAGACAACATCCCCAACCTGAAGGACTCTTTAGAGGAAGGGCAAGTG
GATGATGCGGGGAAGGCTTTGGGAGTGATTTCAAACTTTCAGTCTAGCCCAAAATATGGTAGTGAAGAAGATTG
TTCTAGTGCCACTTCAGGCTCAGTGGGAGCCAACAGCACCACAGGGGGCATCGCTGTAGGAGCATCCAGCTCCA
AAACAAACACCCTGGACATGCAGAGCTCAGGATTTGATGCACTACTACATGAGATCCAGGAGATCCGGGAAACC
CAGGCCAGACTAGAGGAATCCTTTGAGACTCTCAAGGAACATTATCAGAGGGACTATTCCTTAATAATGCAGAC
CTTACAGGAGGAGCGATATAGATGTGAACGATTGGAAGAACAGCTAAATGACCTAACAGAGCTCCACCAGAATG
AAATCTTGAACTTGAAGCAGGAACTGGCAAGCATGGAAGAAAAAATCGCGTATCAGTCCTATGAACGGGCCCGG
GACATCCAGGAGGCCCTGGAGGCATGCCAGACGCGCATCTCCAAGATGGAGCTGCAGCAGCAGCAGCAGCAGGT
GGTGCAGCTAGAAGGGCTGGAGAATGCCACTGCCCGGAACCTTCTGGGCAAACTCATCAACATCCTCCTGGCTG
TCATGGCAGTCCTTTXGGTCTTTGXCTCCACTGTAGCCAACTGTGTGGTCCCCCTCATGAAGACTCGCAACAGG
ACGTTCAGCACTTTATTCCTTGTGGTTTTTATTGCCTTTCTCTGGAAGCACTGGGACGCCCTCTTCAGCTATGT
GGAACGGTTCTTTTCATCCCCTAGA**TGA**TGCTGGCACAGAAGGCATTGTTCCCTACCCTCTGGCGAGTGCATGC
AGCAGAGAGTTAGACAGCAACTTACCTACTCTGAAGTTTTCTACAACAAAAAAAGAGTTGAGTGAATCTGTTTA
CATTTAGAATAATGTTTTTTTCTTCAAGAGACGCAATTGCAATAGTATTTTTTAGATTTTATCCAAGAAGTTTT
TTGGGCGAAAATCTTGGATCATTTTTATGTAGCATGATTTTCCTTGGGATGCAAATCTTAAAACAGTCCTTTAA
TATGAACCAACAATCTGGAGCACACCGAAGGGCAATCTAAATTGTGGCTTGAAGGACTGCACTAAAACCCACTA
AAAAGATGCGAAAACCTGATGAGGGCXAACCAGTTAAACCTAACACCCTGCCTTGTCTGGGCTCATCACCTCTC
CCTATCCCAGACTAACTTTACTGTGAAATCCTACACATTCCATGTCTGAATTTTTGGATTCGGGGTGGATTTTC
GTTGTCCGTGGAAGAACACATGGATCTCTCTGGCTTTCTCACCCAAGTTGGCCACTTACGCTAATCCTGGAAGT
ATGATCACTTTTGAACCTGCCCCTTAACCTTGACGAGGATACAAAAGTGAAAGCATCATCCCCCAAAGGATCAC
TGCACAGTCCTACTACAGTATTTTTAAGTAGCCCTCTAAATACTTAATTTTAAGCAAAATCCCTTGGCCGCACT
TTTAAGGTTTTTTTATATGTGTATAGTTACCAACCTAAAAATAAAAAATCCGAACAGCATACTTGAAGAATGTA
ATACTCAAACTCTCAGTGCTTCCTTATGGTTTCTAATAGGATTTTTTATTATTGTTATTATTATTATTGGGTTT
TTTTGGACAGGGTTGGGAGGGTCTTTTATTTTTCCTTTGAAATAAAGAAGTGATGTTTTTAAATGAAGAAATGT
GTGGATATTTAAGTGTGCTGCTCCCTCTTGTCTTGAAACAGTTTGAGTAAGAAAGTCTTGCTGTAAATGCTGCC
CTCTGCCGCCTTTGTTTTGAGATGCAGTTTAAACTCCCTCTGGCTGCTGCTGCTGCTTTTTGGTGTCCCGACAT
ACCTACGCCCCCGTTTTATGGGTTTGGCTTAGTTGAAGAGGAAAGGGTTGTGCAAGGAGAGCAGGAGGCTGTTT
CCAAAAACCAGTGTAGTAGGATAGGGATTTTTTTTTTTTTTTGCCCCAAGAAAACGTTCACCCAGTGATCTTG
GGCTGGGGTTGTCTTTAGGAAAAGTTGAGACTATAAGAGTCATAAATAAGTCCTTGTGTTTCCTTAATTTATTT
TGTTAACACCCCTAATTACAACCAAAGTGATGATGTGGAGTCTTCTGTCTTCATTTTGGCCCCAGCATTCTTAA
TTTCAAAGCTTTATTCTGTCTGCCTAAGAGAATCAACCAAAGGTGATTCTCCTAAAGAGCAGTGAAGGAAATGT
CAGGTTAGCAGGACCCAAGTTTTGGGTGTGAAATGTTGCCAGCTTCCTATAATGTAAACGGACTTGTTAACCTA

# FIGURE 76B

ACCTAATTATGCTCAGTGGACTTCTATAGATGGTTTTGAAAAATGAACTGAGCTGCCTTCCCGCATCGCATAAC
CAGTTCCATCATCCTGGTGGAACTTGAACATTTAGAGTTTATCTAGAGAGCTTGGTTAATCTTTCCATATTATT
TGTAGTATTGGTCACAAATGCTGTTCCCTCTTAGCCTCATTCTGTGCAACCAAGTGCATATAAGATGCCCTGAA
AAGAGTAACAAAGTATGCTTTGCCTGTTTCCACTTACCAGGAAATTCCTTCAGAACTAGATTAGCATTGCCCTG
CCTGTCTGAAAGGACAGTTTACCTAATGGTGCCAGCCTCCTTTTGCTTTGGCAAGCTGGATTTCTCAGAGCCAG
CATGTTGTTTCCATAACTACTTTGATATTTTAACTCAGGTACTCCAGTCTTCACCCCAACCTCAGCTGATTGTA
GTACACCTGCTAGCTCTGTTGCCCCCTCAAAACTGCACCCAGAGCAGGGCCACAAGGGTGCTTTTTTTCTTTAA
AAAAAAAAAAATTAGAACCAATTCATGTTCATGCCAAAAACAAATTGTCCCCAAGCCTATATGTATTAAAATGT
TAACTTTGCCTAAAAATATTGCAGTGACTTTTTAGGCAGGAGTGCCAAAGGACACTATGAACTTTTTGAACTGA
CAGTTTCTCCTAACTTTCTGCTTTAGCGTAATTGCTCAGAGTAGAGAGCCCCCACAAAGTTATTTAAAAGATGC
CCTAGCAGCAATCCACCAGTTTTTCTAAGCTAGAACCTTTGAGTCCCCCAAACTGCCTGAAGACTTAAGTTTTG
TGGGCACTGGAAGTCACTTTGATAGATGGATTGAAACTGTTCCTATTTGCCCTGGGACGGTTTCTATCTATCAA
AGGAAGGTTTTCACCTGTAGAAAGCCCCCTGCCTCCAGCCAAATAGTCCCATGCTGACTTTCTATCTTCCTTTC
TCAAACTGTCTTAGGAAGGACCTTCAGTGCAGATCAGGTGCAGTAATGGCTTTCTTGTCCCTTAATTATTCACC
AGACCCAGAAGTTGTACGCATTTAATGCTGTTTGTAACCATGCATCTGTTTTCATTCTTTGCTGTACCTTTTGC
TGCCCATCCTGTTACTTTTGAGTTTCTTTCATTGTGGTTGTTCTTGGGTTCTTTTGTCTTGTCAGAGCTCTTCT
ATAACCTCGCTCTAATGGCTTAACAGTTGTTCTGGGTGGAAACGTCCCCTCATTTGAATGCTCCTCTAAAAAAA
AAAAAAAAA

# FIGURE 77A

TATTAGCCAAGCTAAGTTACTCTTTTGCCTCCTGTTGTTACTCAAGTCTTTTCTCTTCTGTCCTTCTGCCAGCC
TTACCCCACTCCTTAATCCTCTGAACCAGCAAACCATTGCCAAGTTCTGATGCAAAGTGGTTTATAGGCCTGAC
TGGACCAGACTAAAAGTGTTCAAAATAGCAAGCAACAAGGAGCAGAAATCCATATTAGAATGGGATATGGACTA
TATTTATATTGGTACAGAATGCCTTCAATAAAGAGTTGTGAGTTGTGTAGGTGAGTTGCCATGGAGCTACAAAT
ATGAGTTGATATTCTGAAATCCTAGACAGCCATCTCCAAGGTTAAGAAAAATCCTTATGCACTCACTTGCAAAG
ATATCCACAGCATGCTCTTGGAGCGCCGCCGGCCGGGAGGCGAAGGATGCAGGCGGCTCCGCGCGCCGGCTGCG
GGGCAGCGCTCCTGCTGTGGATTGTCAGCAGCTGCCTCTGCAGAGCCTGGACGGCTCCCTCCACGTCCCAAAAA
TGTGATGAGCCACTTGTCTCTGGACTCCCCCATGTGGCTTTCAGCAGCTCCTCCTCCATCTCTGGTAGCTATTC
TCCCGGCTATGCCAAGATAAACAAGAGAGGAGGTGCTGGGGGATGGTCTCCATCAGACAGCGACCATTATCAAT
GGCTTCAGGTTGACTTTGGCAATCGGAAGCAGATCAGTGCCATTGCAACCCAAGGAAGGTATAGCAGCTCAGAT
TGGGTGACCCAATACCGGATGCTCTACAGCGACACAGGGAGAAACTGGAAACCCTATCATCAAGATGGGAATAT
CTGGGCATTTCCCGGAAACATTAACTCTGACGGTGTGGTCCGGCACGAATTACAGCATCCGATTATTGCCCGCT
ATGTGCGCATAGTGCCTCTGGATTGGAATGGAGAAGGTCGCATTGGACTCAGAATTGAAGTTTATGGCTGTTCT
TACTGGGCTGATGTTATCAACTTTGATGGCCATGTTGTATTACCATATAGATTCAGAAACAAGAAGATGAAAAC
ACTGAAAGATGTCATTGCCTTGAACTTTAAGACGTCTGAAAGTGAAGGAGTAATCCTGCACGGAGAAGGACAGC
AAGGAGATTACATTACCTTGGAACTGAAAAAAGCCAAGCTGGTCCTCAGTTTAAACTTAGGAAGCAACCAGCTT
GGCCCCATATATGGCCACACATCAGTGATGACAGGAAGTTTGCTGGATGACCACCACTGGCACTCTGTGGTCAT
TGAGCGCCAGGGGCGGAGCATTAACCTCACTCTGGACAGGAGCATGCAGCACTTCCGTACCAATGGAGAGTTTG
ACTACCTGGACTTGGACTATGAGATAACCTTTGGAGGCATCCCTTTCTCTGGCAAGCCCAGCTCCAGCAGTAGA
AAGAATTTCAAAGGCTGCATGGAAAGCATCAACTACAATGGCGTCAACATTACTGATCTTGCCAGAAGGAAGAA
ATTAGAGCCCTCAAATGTGGGAAATTTGAGCTTTTCTTGTGTGGAACCCTATACGGTGCCTGTCTTTTTCAACG
CTACAAGTTACCTGGAGGTGCCCGGACGGCTTAACCAGGACCTGTTCTCAGTCAGTTTCCAGTTTAGGACATGG
AACCCCAATGGTCTCCTGGTCTTCAGTCACTTTGCGGATAATTTGGGCAATGTGGAGATTGACCTCACTGAAAG
CAAAGTGGGTGTTCACATCAACATCACACAGACCAAGATGAGCCAAATCGATATTTCCTCAGGTTCTGGGTTGA
ATGATGGACAGTGGCACGAGGTTCGCTTCCTAGCCAAGGAAAATTTTGCTATTCTCACCATCGATGGAGATGAA
GCATCAGCAGTTCGAACTAATAGTCCCCTTCAAGTTAAAACTGGCGAGAAGTACTTTTTTGGAGGTTTTCTGAA
CCAGATGAATAACTCAAGTCACTCTGTCCTTCAGCCTTCATTCCAAGGATGCATGCAGCTCATTCAAGTGGACG
ATCAACTTGTAAATTTATACGAAGTGGCACAAAGGAAGCCGGGAAGTTTCGCGAATGTCAGCATTGACATGTGT
GCGATCATAGACAGATGTGTGCCCAATCACTGTGAGCATGGTGGAAAGTGCTCGCAAACATGGGACAGCTTCAA
ATGCACTTGTGATGAGACAGGATACAGTGGGGCCACCTGCCACAACTCTATCTACGAGCCTTCCTGTGAAGCCT
ACAAACACCTAGGACAGACATCAAATTATTACTGGATAGATCCTGATGGCAGCGGACCTCTGGGGCCTCTGAAA
GTTTACTGCAACATGACAGAGGACAAAGTGTGGACCATAGTGTCTCATGACTTGCAGATGCAGACGCCTGTGGT
CGGCTACAACCCAGAAAAATACTCAGTGACACAGCTCGTTTACAGCGCCTCCATGGACCAGATAAGTGCCATCA
CTGACAGTGCCGAGTACTGCGAGCAGTATGTCTCCTATTTCTGCAAGATGTCAAGATTGTTGAACACCCCAGAT
GGAAGCCCTTACACTTGGTGGGTTGGCAAAGCCAACGAGAAGCACTACTACTGGGGAGGCTCTGGGCCTGGAAT
CCAGAAATGTGCCTGCGGCATCGAACGCAACTGCACAGATCCCAAGTACTACTGTAACTGCGACGCGGACTACA
AGCAATGGAGGAAGGATGCTGGTTTCTTATCATACAAAGATCACCTGCCAGTGAGCCAAGTGGTGGTTGGAGAT
ACTGACCGTCAAGGCTCAGAAGCCAAATTGAGCGTAGGTCCTCTGCGCTGCCAAGGAGACAGGAATTATTGGAA
TGCCGCCTCTTTCCCAAACCCATCCTCCTACCTGCACTTCTCTACTTTCCAAGGGGAAACTAGCGCTGACATTT
CTTTCTACTTCAAAACATTAACCCCCTGGGGAGTGTTTCTTGAAAATATGGGAAAGGAAGATTTCATCAAGCTG
GAGCTGAAGTCTGCCACAGAAGTGTCCTTTTCATTTGATGTGGGAAATGGGCCAGTAGAGATTGTAGTGAGGTC
ACCAACCCCTCTCAACGATGACCAGTGGCACCGGGTCACTGCAGAGAGGAATGTCAAGCAGGCCAGCCTACAGG
TGGACCGGCTACCGCAGCAGATCCGCAAGGCCCCAACAGAAGGCCACACCCGCCTGGAGCTCTACAGCCAGTTA
TTTGTGGGTGGTGCTGGGGGCCAGCAGGGCTTCCTGGGCTGCATCCGCTCCTTGAGGATGAATGGGGTGACACT
TGACCTGGAGGAAAGAGCAAAGGTCACATCTGGGTTCATATCCGGATGCTCGGGCCATTGCACCAGCTATGGAA
CAAACTGTGAAAATGGAGGCAAATGCCTAGAGAGATACCACGGTTACTCCTGCGATTGCTCTAATACTGCATAT
GATGGAACATTTTGCAACAAAGATGTTGGTGCATTTTTTGAAGAAGGGATGTGGCTACGATATAACTTTCAGGC
ACCAGCAACAAATGCCAGAGACTCCAGCAGCAGAGTAGACAACGCTCCCGACCAGCAGAACTCCCACCCGGACC
TGGCACAGGAGGAGATCCGCTTCAGCTTCAGCACCACCAAGGCGCCCTGCATTCTCCTCTACATCAGCTCCTTC
ACCACAGACTTCTTGGCAGTCCTCGTCAAACCCACTGGAAGCTTACAGATTCGATACAACCTGGGTGGCACCCG
AGAGCCATACAATATTGACGTAGACCACAGGAACATGGCCAATGGACAGCCCCACAGTGTCAACATCACCCGCC
ACGAGAAGACCATCTTTCTCAAGCTCGATCATTATCCTTCTGTGAGTTACCATCTGCCAAGTTCATCCGACACC
CTCTTCAATTCTCCCAAGTCGCTCTTTCTGGGAAAAGTTATAGAAACAGGGAAAATTGACCAAGAGATTCACAA
ATACAACACCCCAGGATTCACTGGTTGCCTCTCCAGAGTCCAGTTCAACCAGATCGCCCCTCTCAAGGCCGCCT

# FIGURE 77B

TGAGGCAGACAAACGCCTCGGCTCACGTCCACATCCAGGGCGAGCTGGTGGAGTCCAACTGCGGGGCCTCGCCG
CTGACCCTCTCCCCCATGTCGTCCGCCACCGACCCCTGGCACCTGGATCACCTGGATTCAGCCAGTGCAGATTT
TCCATATAATCCAGGACAAGGCCAAGCTATAAGAAATGGAGTCAACAGAAACTCGGCTATCATTGGAGGCGTCA
TTGCTGTGGTGATTTTCACCATCCTGTGCACCCTGGTCTTCCTGATCCGGTACATGTTCCGCCACAAGGGCACC
TACCATACCAACGAAGCAAAGGGGGCGGAGTCGGCAGAGAGCGCGGACGCCGCCATCATGAACAACGACCCCAA
CTTCACAGAGACCATTGATGAAAGCAAAAAGGAATGGCTCATT<u>TGA</u>GGGGTGGCTACTTGGCTATGGATAGGG
AGGAGGGAATTACTAGGGAGGAGAGAAAGGACAAAAGCACCCTGCTTCATACTCTTGAGCACATCCTTAAAAT
ATCAGCACAAGTTGGGGGAGGCAGGCAATGGAATATAATGGAATATTCTTGAGACTGATCACAAAAAAAAAAAA
AACCTTTTTAATATTTCTTTATAGCTGAGTTTTCCCTTCTGTATCAAAACAAAATAATACAAAAAATGCTTTTA
GAGTTTAAGCAATGGTTGAAATTTGTAGGTACTATCTGTCTTATTTTGTGTGTGTTTAGAGGTGTTCTAAAGAC
CCGTGGTAACAGGGCAAGTTTTCTACGTTTTTAAGAGCCCTTAGAACGTGGGTATTTTTTTTCTTGAGAAAAGC
TAATGCACCTACAGATGGCCCCCAACATTCTCTTCCTTTTGCTTCTAGTCAACCTTAATGGGCTGTTACAGAAA
CTAGTTCGTGTTTATATACTATTTCCTTTGATGTCCTATAAGTCGGAAAAGAAAGGGGCAAAGAGAACCTATTA
TTTGCCAGTTTTTAAGCAGAGCTCAATCTATGCCAGCTCTCTGGCATCTGGGGTTCCTGACTGATACCAGCAGT
TGAAGGAAGAGAGTGCATGGCACCTGGTGTGTAACGACACAATCAGCACAACTGGAGAGAGGCATTAAAGAACC
AGGGAAGGTAGTTTGATTTTTCATTG

# FIGURE 78A

TCACTTGCCTGATATTTCCAGTGTCAGAGGGACACAGCCAACGTGGGGTCCCTTCTAGGCTGACAGCCGCTCTC
CAGCCACTGCCGCGAGCCCGTCTGCTCCCGCCCTGCCCGTGCACTCTCCGCAGCCGCCCTCCGCCAAGCCCCAG
CGCCCGCTCCCATCGCCGATGACCGCGGGGAGGAGGATGGAGATGCTCTGTGCCGGCAGGGTCCCTGCGCTGCT
GCTCTGCCTGGGTTTCCATCTTCTACAGGCAGTCCTCAGTACAACTGTGATTCCATCATGTATCCCAGGAGAGT
CCAGTGATAACTGCACAGCTTTAGTTCAGACAGAAGACAATCCACGTGTGGCTCAAGTGTCAATAACAAAGTGT
AGCTCTGACATGAATGGCTATTGTTTGCATGGACAGTGCATCTATCTGGTGGACATGAGTCAAAACTACTGCAG
GTGTGAAGTGGGTTATACTGGTGTCCGATGTGAACACTTCTTTTTAACCGTCCACCAACCTTTAAGCAAAGAGT
ATGTGGCTTTGACCGTGATTCTTATTATTTTGTTTCTTATCACAGTCGTCGGTTCCACATATTATTTCTGCAGA
TGGTACAGAAATCGAAAAAGTAAAGAACCAAAGAAGGAATATGAGAGAGTTACCTCAGGGGATCCAGAGTTGCC
GCAAGTCTGAATGGCGCCATCAAACTTATGGGCAGGGATAACAGTGTGCCTGGTTAATATTAATATTCCATTTT
ATTAATAATATTTATGTTGGGTCAAGTGTTAGGTCAATAACACTGTATTTTAATGTACTTGAAAAATGTTTTTA
TTTTTGTTTTATTTTTGACAGACTATTTGCTAATGTATAATGTGCAGAAAATATTTAATATCAAAAGAAAATTG
ATATTTTTATACAAGTAATTTCCTGAGCTAAATGCTTCATTGAAAGCTTCAAAGTTTATATGCCTGGTGCACAG
TGCTTAGAAGTAAGCAATTCCCAGGTCATAGCTCAAGAATTGTTAGCAAATGACAGATTTCTGTAAGCCTATAT
ATATAGTCAAATCGATTTAGTAAGTATGTTTTTTATGTTCCTCAAATCAGTGATAATTGGTTTGACTGTACCAT
GGTTTGATATGTAGTTGGCACCATGGTATCATATATTAAAACAATAATGCAATTAGAATTTGGGAGAAGCAAAT
ATAGGTCCTGTGTTAAACACTACACATTTGAAACAAGCTAACCCTGGGGAGTCTATGGTCTCTTCACTCAGGTC
TCAGCTATAATTCTGTTATATGAGGGGCAGTGGACAGTTCCCTATGCCAACTCACGACTCCTACAGGTACTAGT
CACTCATCTACCAGATTCTGCCTATGTAAAATGAATTGAAAAACAATTTTCTGTAATCTTTTATTTAAGTAGTG
GGCATTTCATAGCTTCACAATGTTCCTTTTTTGTATATTACAACATTTATGTGAGGTAATTATTGCTCAACAGA
CAATTAGAAAAAAGTCCACACTTGAAGCCTAAATTTGTGCTTTTTAAGAATATTTTTAGACTATTTCTTTTTAT
AGGGGCTTTGCTGAATTCTAACATTAAATCACAGCCCAAAATTTGATGGACTAATTATTATTTTAAAATATATG
AAGACAATAATTCTACATGTTGTCTTAAGATGGAAATACAGTTATTTCATCTTTTATTCAAGGAAGTTTTAACT
TTAATACAGCTCAGTAAATGGCTTCTTCTAGAATGTAAAGTTATGTATTTAAAGTTGTATCTTGACACAGGAAA
TGGGAAAAAACTTAAAAATTAATATGGTGTATTTTTCCAAATGAAAAATCTCAATTGAAAGCTTTTAAAATGTA
GAAACTTAAACACACCTTCCTGTGGAGGCTGAGATGAAAACTAGGGCTCATTTTCCTGACATTTGTTTATTTTT
TGGAAGAGACAAAGATTTCTTCTGCACTCTGAGCCCATAGGTCTCAGAGAGTTAATAGGAGTATTTTTGGGCTA
TTGCATAAGGAGCCACTGCTGCCACCACTTTTGGATTTTATGGGAGGCTCCTTCATCGAATGCTAAACCTTTGA
GTAGAGTCTCCCTGGATCACATACCAGGTCAGGGAGGATCTGTTCTTCCTCTACGTTTATCCTGGCATGTGCTA
GGGTAAACGAAGGCATAATAAGCCATGGCTGACCTCTGGAGCACCAGGTGCCAGGACTTGTCTCCATGTGTATC
CATGCATTATATACCCTGGTGCAATCACACGACTGTCATCTAAAGTCCTGGCCCTGGCCCTTACTATTAGGAAA
ATAAACAGACAAAAACAAGTAAATATATATGGTCCTATACATATTGTATATATATTCATATACAAACATGTATG
TATACATGACCTTAATGGATCATAGAATTGCAGTCATTTGGTGCTCTGCTAACCATTTATATAAAACTTAAAAA
CAAGAGAAAAGAAAAATCAATTAGATCTAAACAGTTATTTCTGTTTCCTATTTAATATAGCTGAAGTCAAAATA
TGTAAGAACACATTTTAAATACTCTACTTACAGTTGGCCCTCTGTGGTTAGTTCCACATCTGTGGATTCAACCA
ACCAAGGACGGAAAATGCTTAAAAAATAATACAACAACAACAAAAAAATACATTATAACAACTATTTACTTTTTT
TTTTTTCTTTTTGAGATGGAGTCTCGCTCTGTTGCCCAGGTTGGAGTGCAGTGGCACGATCTCGGCTCACTGCA
ACCTCACCTCCCGGGTTCAAGAGATCCTCCTGCCTCAGCCTCCTGAGCAGCTGGGACTACAGGCGCATGCCACC
ATGCCCAGCTAATTTTTGTATTTTTAGTAGAGGCGGGGTTTCACCATGTTGGCCAGGATGGTCTCAATCTCCTA
ACCTTGAGATCCACCCTCCACAGCCTCCCAAACTGCTGGGATTACAGGCGTGAGCCACCGCACGTAGCATTTAC
ATTAGGTATTACAAGTAATGTAAAGATGATTTAAGTATACAGGAGGATGTGAATAGGTTATATGCAAGCACTAT
GCCCTTTTATATAAGTGACTTGAACATCTGTGCCCGATTTTAGTATGTGCAGGGGGGGCGATCTGGGAATCAGTC
CCCTGTGGATACCAAGGTACAACTGTATTTATTAACGCTTACTAGATGTGAGGAGAGTCTGAATATTTTCAGTG
ATCTTGGCTGTTTCAAAAAAAATCTATTGACTTTTCAATAAATCAGCTGCAATCCATTTATTTCATTTACAAAAG
ATTTATTGTAAGCCTCTCAATCTTGGTTTTTCAGTTGATCTTAAGCATGTCAATTCATAAAAACAAGTCATTTT
TGTATTTTTCATCTTTAAGAATGCTTAAAAAAGCTAATCCCTAAAATAGTTAGATCTTTGTAAATGCATATTAA
ATAATAAAGTATGACCCACATTACTTTTTATGGGTGAAAATAAGACAAAAATAATAGTTTAGTGAGGATGGTG
CTGAGTAAACATAAAAACTGATTTGCTCTCAGCTGATGTGTCCTGTACACAGTGGGAAGATTTTAGTTCACACT
TAGTCTAACTCCCCCATTTTACAGATTTCTCACTATATATATTTCTAGAAGGGGCTATGCATATTCAATGTATT
GAGAACCAAAGCAACCACAAATGCATAAATGCATAATTTATGGTCTTCAACCAAGGCCACATAATAACCCAGTT
AACTTACTCTTTAACCAGGAATATTAAGTTCTATAACTAGTACTCAAGGTTTAACCTTAAAATTAAGATTTCCT
TAACCTTAACCTTAAAATTGATATTATATTAAACATACATAATACAATGTAACTCCACTGTTCTCCTGAATATT
TTTTGCTCTAATCTCTCTGCCGAAAGTCAAAGTGATGGGAGAATTGGTATACTGGTATGACTACGTCTTAAGTC
AGATTTTTATTTATGAGTCTTTGAGACTAAATTCAATCACCACCAGGTATCAAATCAACTTTTATGCAGCAAAT
ATATGATTCTAGTGTCTGACTTTTGTTAAATTCAGTAATGCAGTTTTTAAAAACCTGTATCTGACCCACTTTGT

# FIGURE 78B

AATTTTTGCTCCAATATCCATTCTGTAGACTTTTGAAAAAAAAGTTTTTAATTTGATGCCCAATATATTCTGAC
CGTTAAAAAATTCTTGTTCATATGGGAGAAGGGGGAGTAATGACTTGTACAAACAGTATTTCTGGTGTATATTT
TAATGTTTTTAAAAAGAGTAATTTCATTTAAATATCTGTTATTCAAATTTGATGATGTTAAATGTAATATAATG
TATTTTCTTTTTATTTTGCACTCTGTAATTGCACTTTTTAAGTTTGAAGAGCCATTTTGGTAAACGGTTTTTAT
TAAAGATGCTATGGAACATAAAGTTGTATTGCATGCAATTTAAAGTAACTTATTTGACTATGAATATTATCGGA
TTACTGAATTGTATCAATTTGTTTGTGTTCAATATCAGCTTTGATAATTGTGTACCTTAAGATATTGAAGGAGA
AAATAGATAATTTACAAGATATTATTAATTTTTATTTATTTTTCTTGGGAATTGAAAAAAATTGAAATAAATAA
AAATGCATTGAACATCTTGCATTCAAAATCTTCACTGAC

# FIGURE 79

MGHLPTGIHGARRLLPLLWLFVLFKNATAFHVTVQDDNNIVVSLEASDVISPASVYVVKITGESKNYFFEFEEF
NSTLPPPVIFKASYHGLYYIITLVVVNGNVVTKPSRSITVLTKPLPVTSVSIYDYKPSPETGVLFEIHYPEKYN
VFTRVNISYWEGKDFRTMLYKDFFKGKTVFNHWLPGMCYSNITFQLVSEATFNKSTLVEYSGVSHEPKQHRTAP
YPPQNISVRIVNLNKNNWEEQSGNFPEESFMRSQDTIGKEKLFHFTEETPEIPSGNISSGWPDFNSSDYETTSQ
PYWWDSASAAPESEDEFVSVLPMEYENNSTLSETEKSTSGSFSFFPVQMILTWLPPKPPTAFDGFHIHIEREEN
FTEYLMVDEEAHEFVAELKEPGKYKLSVTTFSSSGSCETRKSQSAKSLSFYISPSGEWIEELTEKPQHVSVHVL
SSTTALMSWTSSQENYNSTIVSVVSLTCQKQKESQRLEKQYCTQVNSSKPIIENLVPGAQYQVVIYLRKGPLIG
PPSDPVTFAIVPTGIKDLMLYPLGPTAVVLSWTRPYLGVFRKYVVEMFYFNPATMTSEWTTYYEIAATVSLTAS
VRIANLLPAWYYNFRVTMVTWGDPELSCCDSSTISFITAPVAPEITSVEYFNSLLYISWTYGDDTTDLSHSRML
HWMVVAEGKKKIKKSVTRNVMTAILSLPPGDIYNLSVTACTERGSNTSMLRLVKLEPAPPKSLFAVNKTQTSVT
LLWVEEGVADFFEVFCQQVGSSQKTKLQEPVAVSSHVVTISSLLPATAYNCSVTSFSHDSPSVPTFIAVSTMVT
EMNPNVVVISVLAILSTLLIGLLLVTLIILRKKHLQMARECGAGTFVNFASLERDGKLPYNWSKNGLKKRKLTN
PVQLDDFDAYIKDMAKDSDYKFSLQFEELKLIGLDIPHFAADLPLNRCKNRYTNILPYDFSRVRLVSMNEEEGA
DYINANYIPGYNSPQEYIATQGPLPETRNDFWKMVLQQKSQIIVMLTQCNEKRRVKCDHYWPFTEEPIAYGDIT
VEMISEEEQDDWACRHFRINYADEMQDVMHFNYTAWPDHGVPTANAAESILQFVHMVRQQATKSKGPMIIHCSA
GVGRTGTFIALDRLLQHIRDHEFVDILGLVSEMRSYRMSMVQTEEQYIFIHQCVQLMWMKKKQQFCISDVIYEN
VSKS

Signal sequence.

amino acids 1-29

Transmembrane domain.

amino acids 821-841

N-glycosylation sites.

amino acids 26-29, 75-78, 154-157, 189-192, 201-204, 227-230, 278-281, 287-290, 323-326, 324-327, 370-373, 461-464, 490-493, 700-703, 712-715, 733-758, 790-793, 875-878, 1068-1071, 1184-1187

cAMP- and cGMP-dependent protein kinase phosphorylation site.

amino acids 884-887

Tyrosine kinase phosphorylation sites.

amino acids 59-67, 560-567

N-myristoylation sites.

amino acids 7-12, 102-107, 184-189, 277-232, 405-410, 710-715, 858-863, 1076-1081, 1111-1116, 1116-1121

Amidation site.

amino acids 673-676

Tyrosine specific protein phosphatases active site.

amino acids 1106-1118

Protein-tyrosine phosphatase.

amino acids 934-1166

Fibronectin type III domains.

amino acids 433-521, 530-617, 723-802

# FIGURE 80

AKVTGFSEGVVDSVKGGFSSFSQATHSAAGAVVSKPREIASLIRNKFGSADNIPNLKDSLEEGQVDDAGKALGV
ISNFQSSPKYGSEEDCSSATSGSVGANSTTGGIAVGASSSKTNTLDMQSSGFDALLHEIQEIRETQARLEESFE
TLKEHYQRDYSLIMQTLQEERYRCERLEEQLNDLTELHQNEILNLKQELASMEEKIAYQSYERARDIQEALEAC
QTRISKMELQQQQQVVQLEGLENATARNLLGKLINILLAVMAVLLVFVSTVANCVVPLMKTRNRTFSTLFLVV
FIAFLWKHWDALFSYVERFFSSPR

Transmembrane domains.
amino acids 253-273, 288-308

N-glycosylation sites.
amino acids 101-104, 246-249, 286-289

N-myristoylation sites.
amino acids 9-14, 16-21, 30-35, 48-53, 73-78, 96-101, 99-104, 106-111, 110-115, 243-248

# FIGURE 81

MEPSGSEQLFEDPDPGGKSQDAEARKQTESEQKLSKMTHNALENINVIGQGLKHLFQHQRRRSSVSPHDVQQIQ
ADPEPEMDLESQNACAEIDGVPTHPTALNRVLQQIRVPPXMKRGTSLHSRRGKPEAPKGSPQINRKSGQEMTAV
MQSGRPMSSSTTDAPTGSAMMEIACAAAAAAAACLPGEEGTAERIERLEVSSLAQTSSAVASSTDGSIHTDSVD
GTPDPQRTKAAIAHLQQKILKLTEXIKIAQTARDDNVAEYLKLANSADKQQAARIKQVFEKKNQKSAXTILQLQ
KKLEHYHRKLREVEQNGIPRQPKDVFRDMHQGLKDVGAKVTGFSEGVVDSVKGGFSSFSQATHSAAGAVVSKPR
EIASLIRNKFGSADNIPNLKDSLEEGQVDDAGKALGVISNFQSSPKYGSEEDCSSATSGSVGANSTTGGIAVGA
SSSKTNTLDMQSSGFDALLHEIQEIRETQARLEESFETLKEHYQRDYSLIMQTLQEERYRCERLEEQLNDLTEL
HQNEILNLKQELASMEEKIAYQSYERARDIQEALEACQTRISKMELQQQQQVVQLEGLENATARNLLGKLINI
LLAVMAVLXVFXSTVANCVVPLMKTRNRTFSTLFLVVFIAFLWKHWDALFSYVERFFSSPR

Transmembrane domains.
amino acids 164-184, 585-605, 619-639

N-glycosylation sites.
amino acids 434-437, 579-582, 619-622

N-myristoylation sites.
amino acids 142-147, 214-219, 333-338, 342-347, 349-354, 363-368, 381-386, 406-411, 429-434, 432-437, 439-444, 443-448, 576-581

# FIGURE 82

MQAAPRAGCGAALLLWIVSSCLCRAWTAPSTSQKCDEPLVSGLPHVAFSSSSSISGSYSPGYAKINKRGGAGGW
SPSDSDHYQWLQVDFGNRKQISAIATQGRYSSSDWVTQYRMLYSDTGRNWKPYHQDGNIWAFPGNINSDGVVRH
ELQHPIIARYVRIVPLDWNGEGRIGLRIEVYGCSYWADVINFDGHVVLPYRFRNKKMKTLKDVIALNFKTSESE
GVILHGEGQQGDYITLELKKAKLVLSLNLGSNQLGPIYGHTSVMTGSLLDDHHWHSVVIERQGRSINLTLDRSM
QHFRTNGEFDYLDLDYEITFGGIPFSGKPSSSSRKNFKGCMESINYNGVNITDLARRKKLEPSNVGNLSFSCVE
PYTVPVFFNATSYLEVPGRLNQDLFSVSFQFRTWNPNGLLVFSHFADNLGNVEIDLTESKVGVHINITQTKMSQ
IDISSGSGLNDGQWHEVRFLAKENFAILTIDGDEASAVRTNSPLQVKTGEKYFFGGFLNQMNNSSHSVLQPSFQ
GCMQLIQVDDQLVNLYEVAQRKPGSFANVSIDMCAIIDRCVPNHCEHGGKCSQTWDSFKCTCDETGYSGATCHN
SIYEPSCEAYKHLGQTSNYYWIDPDGSGPLGPLKVYCNMTEDKVWTIVSHDLQMQTPVVGYNPEKYSVTQLVYS
ASMDQISAITDSAEYCEQYVSYFCKMSRLLNTPDGSPYTWWVGKANEKHYYWGGSGPGIQKCACGIERNCTDPK
YYCNCDADYKQWRKDAGFLSYKDHLPVSQVVVGDTDRQGSEAKLSVGPLRCQGDRNYWNAASFPNPSSYLHFST
FQGETSADISFYFKTLTPWGVFLENMGKEDFIKLELKSATEVSFSFDVGNGPVEIVVRSPTPLNDDQWHRVTAE
RNVKQASLQVDRLPQQIRKAPTEGHTRLELYSQLFVGGAGGQQGFLGCIRSLRMNGVTLDLEERAKVTSGFISG
CSGHCTSYGTNCENGGKCLERYHGYSCDCSNTAYDGTFCNKDVGAFFEEGMWLRYNFQAPATNARDSSSRVDNA
PDQQNSHPDLAQEEIRFSFSTTKAPCILLYISSFTTDFLAVLVKPTGSLQIRYNLGGTREPYNIDVDHRNMANG
QPHSVNITRHEKTIFLKLDHYPSVSYHLPSSSDTLFNSPKSLFLGKVIETGKIDQEIHKYNTPGFTGCLSRVQF
NQIAPLKAALRQTNASAHVHIQGELVESNCGASPLTLSPMSSATDPWHLDHLDSASADFPYNPGQGQAIRNGVN
RNSAIIGGVIAVVIFTILCTLVFLIRYMFRHKGTYHTNEAKGAESAESADAAIMNNDPNFTETIDESKKEWLI

Signal sequence.

amino acids 1-25

Transmembrane domain.

amino acids 1262-1282

N-glycosylation sites.

amino acids 289-292, 346-349, 363-366, 379-382, 436-439, 506-509, 507-510, 546-549, 630-633, 735-738, 1116-1119, 1198-1201, 1317-1320

Glycosaminoglycan attachment sites.

amino acids 449-452, 721-724

Tyrosine kinase phosphorylation sites.

amino acids 300-307, 334-342, 577-585, 710-717, 734-742, 790-797, 1162-1170

N-myristoylation sites.

amino acids 8-13, 69-74, 131-136, 138-143, 318-323, 335-340, 344-349, 432-437, 450-455, 452-457, 499-504, 542-547, 566-571, 606-611, 701-706, 720-725, 724-729, 731-736, 925-930, 928-933, 935-940, 998-1103, 1110-1115, 1248-1253, 1256-1261, 1265-1270, 1291-1296, 1300-1305

Neutral zinc metallopeptidases, zinc-binding region signature.

amino acids 145-154

Glycoprotein hormones beta chain signature 1.

amino acids 963-969

# FIGURE 83

MTAGRRMEMLCAGRVPALLLCLGFHLLQAVLSTTVIPSCIPGESSDNCTALVQTEDNPRVAQVSITKCSSDMNG
YCLHGQCIYLVDMSQNYCRCEVGYTGVRCEHFFLTVHQPLSKEYVALTVILIIILFLITVVGSTYYFCRWYRNRK
SKEPKKEYERVTSGDPELPQV

Signal sequence.
amino acids 1-29

Transmembrane domain.
amino acids 118-138

N-glycosylation site.
amino acids 47-50

Tyrosine kinase phosphorylation site.
amino acids 67-75

Amidation site.
amino acids 3-6

EGF-like domain cysteine pattern signature.
amino acids 92-103

EGF domain.
amino acids 68-103

# FIGURE 84

TCGGCTCGCGGCTTTCTGATTATGCAGAACTTAAATCTATGCCTCAGTGACCCATACAGCATTCCAGTTCCTAT
CACCTACTGTCTTGTCCCTATACTTGCAGCAGTTGTCCAGGGTTATTCTTTGTCTGTATTAGAATTTTTTTTCA
GGTTGCTTAAGGAATCTTGCAGATACTTGTGACAAAGAATCATAAATGCTGTTGTTAAACTGAATAATGAATTG
AGTCCCAAATGTTCGTGCTAATTAATGCTTTTTGAGTTGGAGATGAAATGAGAGTAATATCATCAAGCTGTGGA
TTAAAGTTATCCTCAAAGCCCCATCATCTACAAAAAGAATAGGACAGGAACTGCCTTTGTGCAGGTGCAAGACC
ATGTTACTTTTGAGCAGTGAGCTTGAGATGTCTGGGATACAAATTGGGTTCCCTATTAACTACTAATCATTCCT
TTTTTTTCTTTCACCTTCAGCCACTCACAACTGACCTTCACTACTATTACATCCTGGAGCTGTCGTTTTATTGG
TCTTTG**ATG**TTTTCTCAGTTCACTGATATCAAAAGAAAGGACTTTGGCATTATGTTCCTGCACCACCTTGTATC
TATTTTCTTGATTACCTTTTCATATGTCAACAATATGGCCCGAGTAGGAACGCTGGTCCTTTGTCTTCATGATT
CAGCTGATGCTCTTCTGGAGGCTGCCAAAATGGCAAATTATGCCAAGTTTCAGAAAATGTGTGATCTCCTGTTT
GTTATGTTTGCCGTGGTTTTTATCACCACACGACTGGGTATATTTCCTCTCTGGGTGTTAAATACCACATTATT
TGAAAGCTGGGAGATCGTTGGACCTTACCCTTCCTGGTGGGTTTTTAACCTACTGCTATTGCTAGTACAAGGGT
TGAACTGCTTCTGGTCTTACTTGATTGTGAAAATAGCTTGCAAAGCTGTTTCAAGAGGCAAGGTGTCCAAGGAT
GATCGAAGTGATATTGAGTCTAGCTCAGATGAGGAGGACTCAGAACCTCCGGGAAAGAATCCCCACACTGCGAC
AACCACCAATGGGACCAGTGGTACCAACGGGTATCTCCTGACTGGCTCCTGCTCCATGGATGAT**TAA**TTACTCA
AAACTACAAGTCCCAAGCAAAGTGAACTATTTGTTCCTGGAAGTATTTAATAAGTTGCAAATGCAGTTCCTTTC
ATAATATCTCAGCACCAGAAACAAAAATTAAGATTATCAAAGCATTTTGAATAGTGCACTGCCATGTGTCCTGT
CTGTGAATGAAGAAGAATTACCATTCTCTCTTTGTAGGCATGCTGTATGTAATTGACACAAGGGAACAGTATTT
GCATTTGTACTGTCTTAGAATATTATTTATTTTTTTGTATTTGTAAATCTGTGGACAAAAGAGGGTTTCCTCAC
TCCTTTTACTCACTGGGCTCATGACAGTGAAGGAGATGCTCCATCTGCTTCTCCCCCTTTCTCTTGCTGTAGTC
CAATGTGCTATGAGCATCAGCTTACTTTGTCACTTAGAGCAAGCAAAACCCAGTGCAAGAGTCTCGTTCAGCTC
TAAATAGGTTTGCTTTCTTTTAGTTACAGTGCCCATTTTGAAATTGCCTATACAGTCTTAGTGACCATTTAAAC
CGGACGAACTAGGTGTTTAATTTTCACTCTTCATGTTCAATTAGCAGTTCAAATTAAAGAAGATGGTTATTGGA
GAACTTTTTTGAATGGTTTTGTATTAAATTGCTTTGAAATAGATTTCATTTCTTGTGCACACAGCCAAGATTTC
TTCAATGGGTGTGAGCTAGTTGAGGGTTAACCTTGTAGGTTGCAGANNNNNNNNNNNNNNNNNNNNNNNNNNNN
NNNNGATGAGGTCAGTGCTCTGATTTTGAAGGAGGATATTCACTGAAGCTCATAGTTATAAACAAGGAAATCAC
TGTTAAGAATGGGAATTTGTCCTGTGTTCTGGGAATAACATAAAGAGAGCAACTGATTTCAGCCAGGTTTTGCC
ACTACCCTATAATTAGTGCAGTCTTATGTTATAAAAGAAAGAAGTTAACTATATTTGGGGACAAAAAAATATTT
CAAGAGTTGATAAAGATTACCTGTGCAGTGCAGAGCACTTTAATGCAACCAGCTTTCAAGAAAAAGCCCTATCT
AGTACTTGATGTTGATGTTTTTATTTTGCTGAGCAAAATAAAGCCAATGGGAGAAGGACAA

# FIGURE 85

MFSQFTDIKRKDFGIMFLHHLVSIFLITFSYVNNMARVGTLVLCLHDSADALLEAAKMANYAKFQKMCDLLFVM
FAVVFITTRLGIFPLWVLNTTLFESWEIVGPYPSWWVFNLLLLLVQGLNCFWSYLIVKIACKAVSRGKVSKDDR
SDIESSSDEEDSEPPGKNPHTATTTNGTSGTNGYLLTGSCSMDD

Transmembrane domains.
amino acids 17-37, 70-90, 107-127

N-glycosylation sites.
amino acids 93-96, 174-177

N-myristoylation site.
amino acids 175-180

# FIGURE 86

**ATG**TCTCTTGAGCAGAAGAGTCAGCACTGCAAGCCTGAGGAAGGCCTTGACACCCAAGAAGAGGCCCTGGGCCT
GGTGGGTGTGCAGGCTGCCACTACTGAGGAGCAGGAGGCTGTGTCCTCCTCCTCTCCTCTGGTCCCAGGCACCC
TGGGGGAGGTGCCTGCTGCTGGGTCACCAGGTCCTCTCAAGAGTCCTCAGGGAGCCTCCGCCATCCCCACTGCC
ATCGATTTCACTCTATGGAGGCAATCCATTAAGGGCTCCAGCAACCAAGAAGAGGAGGGGCCAAGCACCTCCCC
TGACCCAGAGTCTGTGTTCCGAGCAGCACTCAGTAAGAAGGTGGCTGACTTGATTCATTTTCTGCTCCTCAAGT
AT**TAA**

# FIGURE 87A

GAGGCGGCCAAGGACCTGGCCGACATCGCGGCCTTCTTCCGATCCGGGTTTCGAAAAAACGATGAAATGAAAGC
TATGGATGTTTTACCAATTTTGAAGGAAAAAGTTGCATACCTTTCAGGTGGGAGAGATAAACGTGGAGGTCCCA
TTTTAACGTTTCCGGCCCGCAGCAATCATGACAGAATACGACAGGAGGATCTCAGGAGACTCATTTCCTATCTA
GCCTGTATTCCCAGCGAGGAGGTCTGCAAGCGTGGCTTCACGGTGATCGTGGACATGCGTGGGTCCAAGTGGGA
CTCCATCAAGCCCCTTCTGAAGATCCTGCAGGAGTCCTTCCCCTGCTGCATCCATGTGGCCCTGATCATCAAGC
CAGACAACTTCTGGCAGAAACAGAGGACTAATTTTGGCAGTTCTAAATTTGAATTTGAGACAAATATGGTCTCT
TTAGAAGGCCTTACCAAAGTAGTTGATCCTTCTCAGCTAACTCCTGAGTTTGATGGCTGCCTGGAATACAACCA
CGAAGAATGGATTGAAATCAGAGTTGCTTTTGAAGACTACATTAGCAATGCCACCCACATGCTGTCTCGGCTGG
AGGAACTTCAGGACATCCTAGCTAAGAAGGAGCTGCCTCAGGATTTAGAGGGGGCTCGGAATATGATCGAGGAA
CATTCTCAGCTGAAGAAGAAGGTGATTAAGGCCCCCATCGAGGACCTGGATTTGGAGGGACAGAAGCTGCTTCA
GAGGATACAGAGCAGTGAAAGCTTTCCCAAAAAGAACTCAGGCTCAGGCAATGCGGACCTGCAGAACCTCTTGC
CCAAGGTGTCCACCATGCTGGACCGGCTGCACTCGACACGGCAGCATCTGCACCAGATGTGGCATGTGAGGAAG
CTGAAGCTGGACCAGTGCTTCCAGCTGAGGCTGTTTGAACAGGATGCTGAGAAGATGTTTGACTGGATCACACA
CAACAAAGGCCTGTTTCTAAACAGCTACACAGAGATTGGGACCAGCCACCCTCATGCCATGGAGCTTCAGACGC
AGCACAATCACTTTGCCATGAACTGTATGAACGTGTATGTAAATATAAACCGCATCATGTCGGTGGCCAATCGT
CTGGTGGAGTCTGGCCACTATGCCTCGCAGCAGATCAGGCAGATCGCGAGTCAGCTGGAGCAGGAGTGGAAGGC
GTTTGCGGCAGCCCTGGATGAGCGGAGCACCTTGCTGGACATGTCCTCCATTTTCCACCAGAAGGCCGAAAAGT
ATATGAGCAACGTGGATTCATGGTGTAAAGCTTGCGGTGAGGTAGACCTTCCCTCAGAGCTGCAGGACCTAGAA
GATGCCATTCATCACCACCAGGGAATATATGAACATATCACTCTTGCTTATTCTGAGGTCAGCCAAGATGGGAA
GTCGCTCCTTGACAAGCTCCAGCGGCCCTTGACTCCCGGCAGCTCCGATTCCCTGACAGCCTCTGCCAACTACT
CCAAGGCCGTGCACCATGTCCTGGATGTCATCCACGAGGTGCTGCACCACCAGCGGCACGTGAGAACAATCTGG
CAACACCGCAAGGTCCGGCTGCATCAGAGGCTGCAGCTGTGTGTTTTCCAGCAGGAAGTTCAGCAGGTGCTAGA
CTGGATCGAGAACCACGGAGAAGCATTTCTGAGCAAACATACAGGTGTGGGGAAATCTCTTCATCGGGCCAGAG
CATTGCAGAAACGTCATGAAGATTTTGAAGAAGTGGCACAGAACACATACACCAATGCGGATAAATTACTGGAA
GCAGCAGAACAGCTGGCTCAGACTGGGGAATGTGACCCCGAAGAGATTTATCAGGCTGCCCATCAGCTGGAAGA
CCGGATTCAAGATTTCGTTCGGCGTGTTGAGCAGCGAAAGATCCTACTGGACATGTCAGTGTCCTTTCACACCC
ATGTGAAAGAGCTGTGGACGTGGCTGGAGGAGCTGCAGAAGGAGCTGCTGGACGACGTGTATGCCGAGTCGGTG
GAGGCCGTGCAGGACCTCATCAAGCGCTTTGGCCAGCAGCAGCAGACCACCCTGCAGGTGACTGTCAACGTGAT
CAAGGAAGGGGAGGACCTCATCCAGCAGCTCAGGGACTCTGCCATCTCCAGTAACAAGACCCCCCACAACAGCT
CCATCAACCACATTGAGACGGTGCTGCAGCAGCTGGACGAGGCGCAGTCGCAGATGGAGGAGCTCTTCCAGGAG
CGCAAGATCAAGCTGGAGCTCTTCCTGCACGTGCGCATCTTCGAGAGGGACGCCATCGACATTATCTCAGACCT
CGAGTCTTGGAATGATGAGCTTTCTCAGCAAATGAATGACTTCGACACAGAAGATCTCACGATTGCAGAGCAGC
GCCTCCAGCACCATGCAGACAAAGCCTTGACCATGAACAACTTGACTTTTGACGTCATCCACCAAGGGCAAGAT
CTTCTGCAGTATGTCAATGAGGTCCAGGCCTCTGGTGTGGAGCTGCTGTGTGATAGAGATGTAGACATGGCAAC
TCGGGTCCAGGACCTGCTGGAGTTTCTTCATGAAAAACAGCAGGAATTGGATTTAGCCGCAGAGCAGCATCGGA
AACACCTGGAGCAGTGCGTGCAGCTGCGCCACCTGCAGGCAGAAGTGAAACAGGTGCTGGGTTGGATCCGCAAC
GGAGAGTCCATGTTAAATGCCGGACTTATCACAGCCAGCTCGTTACAAGAGGCAGAGCAGCTCCAGCGAGAGCA
CGAGCAGTTCCAGCATGCCATTGAGAAAACACATCAGAGCGCGCTGCAGGTGCAGCAGAAGGCAGAAGCCATGC
TACAGGCCAACCACTACGACATGGACATGATCCGGGACTGCGCCGAGAAGGTGGCGTCTCACTGGCAACAGCTC
ATGCTCAAGATGGAAGATCGCCTCAAGCTCGTCAACGCCTCTGTCGCTTTCTACAAAACCTCAGAGCAGGTCTG
CAGCGTCCTCGAGAGCCTGGAACAGGAGTACAAGAGAGAAGAAGACTGGTGTGGCGGGGCGGATAAGCTGGGCC
CAAACTCTGAGACGGACCACGTGACGCCCATGATCAGCAAGCACCTGGAGCAGAAGGAGGCATTCCTGAAGGCT
TGCACCCTTGCTCGGAGGAATGCAGACGTCTTCCTGAAATACCTGCACAGGAACAGCGTGAACATGCCAGGAAT
GGTGACGCACATCAAAGCTCCTGAACAGCAAGTGAAAAATATCTTGAATGAACTCTTCCAACGGGAGAACAGGG
TATTGCATTACTGGACCATGAGGAAGAGACGGCTGGACCAGTGTCAGCAGTACGTGGTCTTTGAGAGGAGTGCC
AAGCAGGCTTTGGAATGGATCCATGACAATGGCGAGTTCTACCTTTCCACACACACCTCCACGGGCTCCAGTAT
ACAGCACACCCAGGAGCTCCTGAAAGAGCACGAGGAGTTCCAGATAACTGCAAAGCAAACCAAAGAGAGAGTGA
AGCTATTGATACAGCTGGCTGATGGCTTTTGTGAAAAAGGGCATGCCCATGCGGCAGAGATAAAAAAATGTGTT
ACTGCTGTGGATAAGAGGTACAGAGATTTCTCTCTGCGGATGGAGAAGTACAGGACCTCTTTGGAAAAAGCCCT
GGGGATTTCTTCAGATTCCAACAAATCGAGTAAAAGTCTCCAGCTAGATATCATTCCAGCCAGTATCCCTGGCT
CAGAGGTGAAACTTCGAGATGCTGCTCATGAACTTAATGAAGAGAAGCGGAAATCTGCCCGCAGGAAAGAGTTC
ATAATGGCTGAGCTCATTCAAACTGAAAAGGCTTATGTAAGAGACCTCCGGGAATGTATGGATACGTACCTGTG
GGAAATGACCAGTGGCGTGGAAGAGATTCCACCTGGCATTGTAAACAAAGAACTCATCATCTTCGGAAACATGC
AAGAAATCTACGAATTTCATAATAACATATTCCTAAAGGAGCTGGAAAAATATGAACAGTTGCCAGAGGATGTT

# FIGURE 87B

GGACATTGTTTTGTTACTTGGGCAGACAAGTTTCAGATGTATGTCACATATTGCAAAAATAAGCCTGATTCTAC
TCAGCTGATATTGGAACATGCAGGGTCCTATTTTGACGAGATACAGCAGCGACATGGATTAGCCAATTCCATTT
CTTCCTACCTTATTAAACCAGTTCAGCGAATAACGAAATATCAGCTCCTTTTAAAAGAGCTGCTGACGTGCTGT
GAGGAAGGAAAGGGAGAGATTAAAGATGGCCTGGAGGTGATGCTCAGCGTGCCGAAGCGAGCCAATGACGCCAT
GCACCTCAGCATGCTGGAAGGGTTTGATGAAAACATTGAGTCTCAGGGAGAACTCATCCTACAGGAATCCTTCC
AAGTGTGGGACCCAAAAACCTTAATTCGAAAGGGTCGAGAACGGCATCTCTTCCTTTTTGAAATGTCCTTAGTA
TTTAGTAAAGAAGTGAAAGATTCCAGTGGGAGAAGCAAGTACCTTTATAAAAGCAAATTGTTTACCTCAGAGTT
GGGTGTCACAGAACATGTTGAAGGAGACCCTTGCAAATTTGCACTGTGGGTGGGGAGAACACCAACTTCAGATA
ATAAAATTGTCCTTAAGGCTTCCAGCATAGAGAACAAGCAGGACTGGATAAAGCATATCCGCGAAGTCATCCAG
GAGCGGACGATCCACCTGAAGGGAGCCCTGAAGGAGCCCATTCACATCCCTAAGACCGCTCCCGCCACAAGACA
GAAGGGAAGGAGGGATGGAGAGGATCTGGACAGCCAAGGAGACGGCAGCAGCCAGCCTGATACGATTTCCATCG
CCTCACGGACGTCTCAGAACACGCTGGACAGCGATAAGCTCTCTGGTGGCTGTGAGCTGACAGTGGTGATCCAT
GACTTCACCGCTTGCAACAGCAACGAGCTGACCATCCGACGGGGCCAGACCGTGGAAGTTCTGGAGCGGCCGCA
TGACAAGCCTGACTGGTGTCTGGTGCGGACCACTGACCGCTCCCCAGCGGCAGAAGGCCTGGTCCCCTGTGGTT
CACTGTGCATCGCCCACTCCAGAAGTAGCATGGAAATGGAGGGCATCTTCAACCACAAAGACTCGCTCTCCGTC
TCCAGCAATGACGCCAGTCCACCCGCATCCGTGGCTTCCCTCCAGCCCCACATGATCGGGGCCCAGAGCTCGCC
GGGCCCCAAGCGGCCGGGCAACACCCTGCGCAAGTGGCTCACCAGCCCCGTGCGGCGGCTCAGCAGCGGCAAGG
CCGACGGGCACGTGAAGAAGCTGGCGCACAAGCACAAGAAGAGCCGCGAGGTCCGCAAGAGCGCCGACGCCGGC
TCGCAGAAGGACTCCGACGACAGTGCGGCCACCCCGCAGGACGAGACGGTCGAGGAGAGAGGCCGGAACGAGGG
CCTGAGCAGCGGTACTCTCTCCAAATCCTCCTCCTCGGGGATGCAGAGCTGTGGAGAAGAGGAAGGCGAGGAGG
GGGCCGACGCCGTGCCCCTGCCGCCACCCATGGCCATCCAGCAGCACAGCCTCCTCCAGCCAGACTCACAGGAT
GACAAGGCCTCTTCTCGGTTATTAGTCCGCCCCACCAGCTCCGAAACACCGAGTGCAGCCGAGCTCGTCAGTGC
AATTGAGGAACTCGTGAAAAGCAAGATGGCACTGGAGGATCGCCCCAGCTCACTCCTTGTTGACCAGGGAGATA
GTAGCAGCCCTTCCTTCAACCCTTCGGATAATTCCCTTCTCTCTTCCTCCTCGCCCATTGATGAGATGGAAGAA
AGGAAATCCAGCTCTTTAAAGAGAAGACACTACGTTTTGCAAGAACTAGTGGAGACAGAGCGTGACTATGTGCG
GGACCTTGGCTATGTGGTTGAGGGCTACATGGCACTTATGAAAGAAGATGGTGTTCCTGATGACATGAAAGGAA
AAGACAAAATTGTGTTCGGCAACATCCATCAGATTTACGACTGGCACAGAGACTTTTTTTTAGGAGAGTTAGAG
AAGTGCCTTGAAGATCCAGAAAAACTAGGATCCCTTTTTGTTAAACACGAGAGAAGGTTGCACATGTACATAGC
TTATTGTCAAAATAAACCAAAGTCTGAGCACATTGTCTCAGAATACATTGATACCTTTTTTGAGGACTTAAAGC
AGCGTCTTGGCCACAGGTTACAGCTCACAGATCTGTTGATCAAACCAGTGCAGAGAATCATGAAGTATCAGCTG
TTACTGAAGGACTTCCTCAAGTATTCCAAAAAGGCCAGCCTGGATACATCAGAATTAGAGAGAGCTGTGGAAGT
CATGTGCATAGTACCCAGGCGGTGCAACGACATGATGAACGTGGGGCGGCTGCAAGGATTCGACGGGAAAATCG
TTGCCCAGGGTAAACTGCTCTTGCAGGACACATTCTTGGTCACAGACCAAGATGCAGGACTTCTGCCTCGCTGC
AGAGAGAGGCGCATCTTCCTCTTTGAGCAGATCGTCATATTCAGCGAACCACTTGATAAAAAGAAGGGCTTCTC
CATGCCGGGATTCCTGTTTAAGAACAGTATCAAGGTGAGTTGCCTTTGCCTGGAGGAAAATGTGGAAAATGATC
CCTGTAAATTTGCTCTGACATCGAGGACGGGTGACGTGGTAGAGACCTTCATTTTGCATTCATCTAGTCCAAGT
GTCCGGCAAACTTGGATCCATGAAATCAACCAAATTTTAGAAAACCAGCGCAATTTTTTAAATGCCTTGACATC
GCCAATCGAGTACCAGAGGAACCACAGCGGGGGCGGCGGCGGCGGCGGCAGCGGGGCAGCGGCGGGGGTGGGGG
CAGCGGCGGCGGCGGGGCCCCCAGTGGCGGCAGCGGCCACAGTGGCGGCCCCAGCAGCTGCGGCGGCGCCCCCA
GCACGAGCAGGAGCCGGCCCTCCCGGATCCCCCAGCCTGTCCGACACCACCCCCCCGTGCTGGTCTCCTCTGCA
GCCTCGAGCCAGGCAGAGGCAGACAAGATGTCAGAGTGAAAGCAGCAGCAGTAGCAACATCTCCACCATGTTGG
TGACACACGATTACACGGCAGTGAAGGAGGATGAGATCAACGTCTACCAAGGAGAGGTCGTTCAAATTCTGGCC
AGCAACCAGCAGAACATGTTTCTGGTGTTCCGAGCCGCCACTGACCAGTGCCCCGCAGCTGAGGGCTGGATTCC
AGGCTTTGTCCTGGGCCACACCAGTGCAGTCATCGTGGAGAACCCGGACGGGACTCTCAAGAAGTCAACATCTT
GGCACACAGCACTCCGTTTAAGGAAAAAATCTGAGAAAAAAGATAAAGACGGCAAAAGGGAAGGCAAGTTAGAG
AACGGTTATCGGAAGTCACGGGAAGGACTCAGCAACAAGGTATCTGTGAAGCTTCTCAATCCCAACTACATTTA
TGACGTTCCCCCAGAATTCGTCATTCCATTGAGTGAGGTCACGTGTGAGACAGGGGAGACCGTTGTTCTTAGAT
GTCGAGTCTGTGGCCGCCCCAAAGCCTCAATTACCTGGAAGGGCCCTGAACACAACACCTTGAACAACGATGGT
CACTACAGCATCTCCTACAGTGACCTGGGAGAGGCCACGCTGAAGATTGTGGGCGTGACCACGGAAGATGACGG
CATCTACACGTGCATCGCTGTCAATGACATGGGTTCAGCCTCATCATCGGCCAGCCTGAGGGTCCTAGGTCCAG
GGATGGATGGGATCATGGTGACCTGGAAAGACAACTTTGACTCCTTCTACAGTGAAGTGGCTGAGCTTGGCAGG
GGCAGATTCTCTGTCGTTAAGAAATGTGATCAGAAAGGAACCAAGCGAGCAGTGGCCACTAAGTTTGTGAACAA
GAAGTTGATGAAGCGCGACCAGGTCACCCATGAGCTTGGCATCCTGCAGAGCCTCCAGCACCCCCTGCTTGTCG
GCCTCCTCGACACCTTTGAGACCCCCACCAGCTACATCCTGGTCTTAGAAATGGCTGACCAGGGTCGCCTCCTG

# FIGURE 87C

GACTGCGTGGTGCGATGGGGAAGCCTCACTGAAGGGAAGATCAGGGCGCACCTGGGGGAGGTTCTGGAAGCTGT
CCGGTACCTGCACAACTGCAGGATAGCACACCTGGACCTAAAGCCTGAGAATATCCTGGTGGATGAGAGTTTAG
CCAAGCCAACCATCAAACTGGCTGACTTTGGAGATGCTGTTCAGCTCAACACGACCTACTACATCCACCAGTTA
CTGGGGAACCCTGAATTCGCAGCCCCTGAAATCATCCTCGGGAACCCTGTCTCCCTGACCTCGGATACGTGGAG
TGTTGGAGTGCTCACATACGTACTTCTTAGTGGCGTGTCCCCCTTCCTGGATGACAGTGTGGAAGAGACCTGCC
TGAACATTTGCCGCTTAGACTTTAGCTTCCCAGATGACTACTTTAAAGGAGTGAGCCAGAAGGCCAAGGAGTTC
GTGTGCTTCCTCCTGCAGGAGGACCCCGCCAAGCGTCCCTCGGCTGCGCTGGCCCTCCAGGAGCAGTGGCTGCA
GGCCGGCAACGGCAGAAGCACGGGCGTCCTCGACACGTCCAGACTGACTTCCTTCATTGAGCGGCGCAAACACC
AGAATGATGTTCGACCTATCCGTAGCATTAAAAACTTTCTGCAGAGCAGGCTTCTGCCTAGAGTT**TGA**CCTATC
CAGAAGTTCTTTCTCATTCTCTTTCACCTGCCAATCAGCTGTTAATCTGAATTTTCAAGAGAAAACAAGCAAAC
ATAACTGATCAGCTGCCGGTATGTTCATCGTGTGAAATTGCATTCCAAGTGAGCTGTGCTCAGCAGTGCTTGGA
CACAGAGCTGCAAGCTGCGCTGGGGTGGAGGACCGTCACTTACACTCTGCCAAGGACGGAGGTCGCATTGCTGT
ATCACAGTATTTTTTACGGATTTCTG

# FIGURE 88

MSLEQKSQHCKPEEGLDTQEEALGLVGVQAATTEEQEAVSSSSPLVPGTLGEVPAAGSPGPLKSPQGASAIPTA
IDFTLWRQSIKGSSNQEEEGPSTSPDPESVFRAALSKKVADLIHFLLLKY

**N-myristoylation site.**

amino acids 27-32

**Melanoma antigen encoding gene (MAGE) family.**

amino acids 3-124

# FIGURE 89A

MKAMDVLPILKEKVAYLSGGRDKRGGPILTFPARSNHDRIRQEDLRRLISYLACIPSEEVCKRGFTVIVDMRGS
KWDSIKPLLKILQESFPCCIHVALIIKPDNFWQKQRTNFGSSKFEFETNMVSLEGLTKVVDPSQLTPEFDGCLE
YNHEEWIEIRVAFEDYISNATHMLSRLEELQDILAKKELPQDLEGARNMIEEHSQLKKKVIKAPIEDLDLEGQK
LLQRIQSSESFPKKNSGSGNADLQNLLPKVSTMLDRLHSTRQHLHQMWHVRKLKLDQCFQLRLFEQDAEKMFDW
ITHNKGLFLNSYTEIGTSHPHAMELQTQHNHFAMNCMNVYVNINRIMSVANRLVESGHYASQQIRQIASQLEQE
WKAFAAALDERSTLLDMSSIFHQKAEKYMSNVDSWCKACGEVDLPSELQDLEDAIHHHQGIYEHITLAYSEVSQ
DGKSLLDKLQRPLTPGSSDSLTASANYSKAVHHVLDVIHEVLHHQRHVRTIWQHRKVRLHQRLQLCVFQQEVQQ
VLDWIENHGEAFLSKHTGVGKSLHRARALQKRHEDFEEVAQNTYTNADKLLEAAEQLAQTGECDPEEIYQAAHQ
LEDRIQDFVRRVEQRKILLDMSVSFHTHVKELWTWLEELQKELLDDVYAESVEAVQDLIKRFGQQQTTLQVTV
NVIKEGEDLIQQLRDSAISSNKTPHNSSINHIETVLQQLDEAQSQMEELFQERKIKLELFLHVRIFERDAIDII
SDLESWNDELSQQMNDFDTEDLTIAEQRLQHHADKALTMNNLTFDVIHQGQDLLQYVNEVQASGVELLCDRDVD
MATRVQDLLEFLHEKQQELDLAAEQHRKHLEQCVQLRHLQAEVKQVLGWIRNGESMLNAGLITASSLQEAEQLQ
REHEQFQHAIEKTHQSALQVQQKAEAMLQANHYDMDMIRDCAEKVASHWQQLMLKMEDRLKLVNASVAFYKTSE
QVCSVLESLEQEYKREEDWCGGADKLGPNSETDHVTPMISKHLEQKEAFLKACTLARRNADVFLKYLHRNSVNM
PGMVTHIKAPEQQVKNILNELFQRENRVLHYWTMRKRRLDQCQQYVVFERSAKQALEWIHDNGEFYLSTHTSTG
SSIQHTQELLKEHEEFQITAKQTKERVKLLIQLADGFCEKGHAHAAEIKKCVTAVDKRYRDFSLRMEKYRTSLE
KALGISSDSNKSSKSLQLDIIPASIPGSEVKLRDAAHELNEEKRKSARRKEFIMAELIQTEKAYVRDLRECMDT
YLWEMTSGVEEIPPGIVNKELIIFGNMQEIYEFHNNIFLKELEKYEQLPEDVGHCFVTWADKFQMYVTYCKNKP
DSTQLILEHAGSYFDEIQQRHGLANSISSYLIKPVQRITKYQLLLKELLTCCEEGKGEIKDGLEVMLSVPKRAN
DAMHLSMLEGFDENIESQGELILQESFQVWDPKTLIRKGRERHLFLFEMSLVFSKEVKDSSGRSKYLYKSKLFT
SELGVTEHVEGDPCKFALWVGRTPTSDNKIVLKASSIENKQDWIKHIREVIQERTIHLKGALKEPIHIPKTAPA
TRQKGRRDGEDLDSQGDGSSQPDTISIASRTSQNTLDSDKLSGGCELTVVIHDFTACNSNELTIRRGQTVEVLE
RPHDKPDWCLVRTTDRSPAAEGLVPCGSLCIAHSRSSMEMEGIFNHKDSLSVSSNDASPPASVASLQPHMIGAQ
SSPGPKRPGNTLRKWLTSPVRRLSSGKADGHVKKLAHKHKKSREVRKSADAGSQKDSDDSAATPQDETVEERGR
NEGLSSGTLSKSSSSGMQSCGEEEGEEGADAVPLPPPMAIQQHSLLQPDSQDDKASSRLLVRPTSSETPSAAEL
VSAIEELVKSKMALEDRPSSLLVDQGDSSSPSFNPSDNSLLSSSSPIDEMEERKSSSLKRRHYVLQELVETERD
YVRDLGYVVEGYMALMKEDGVPDDMKGKDKIVFGNIHQIYDWHRDFFLGELEKCLEDPEKLGSLFVKHERRLHM
YIAYCQNKPKSEHIVSEYIDTFFEDLKQRLGHRLQLTDLLIKPVQRIMKYQLLLKDFLKYSKKASLDTSELERA
VEVMCIVPRRCNDMMNVGRLQGFDGKIVAQGKLLLQDTFLVTDQDAGLLPRCRERRIFLFEQIVIFSEPLDKKK
GFSMPGFLFKNSIKVSCLCLEENVENDPCKFALTSRTGDVVETFILHSSSPSVRQTWIHEINQILENQRNFLNA
LTSPIEYQRNHSGGGGGGGSGAAAGVGAAAAAGPPVAAAATVAAPAAAAAPPARAGAGPPGSPSLSDTTPPCWS
PLQPRARQRQTRCQSESSSSSNISTMLVTHDYTAVKEDEINVYQGEVVQILASNQQNMFLVFRAATDQCPAAEG
WIPGFVLGHTSAVIVENPDGTLKKSTSWHTALRLRKKSEKKDKDGKREGKLENGYRKSREGLSNKVSVKLLNPN
YIYDVPPEFVIPLSEVTCETGETVVLRCRVCGRPKASITWKGPEHNTLNNDGHYSISYSDLGEATLKIVGVTTE
DDGIYTCIAVNDMGSASSSASLRVLGPGMDGIMVTWKDNFDSFYSEVAELGRGRFSVVKKCDQKGTKRAVATKF
VNKKLMKRDQVTHELGILQSLQHPLLVGLLDTFETPTSYILVLEMADQGRLLDCVVRWGSLTEGKIRAHLGEVL
EAVRYLHNCRIAHLDLKPENILVDESLAKPTIKLADFGDAVQLNTTYYIHQLLGNPEFAAPEIILGNPVSLTSD
TWSVGVLTYVLLSGVSPFLDDSVEETCLNICRLDFSFPDDYFKGVSQKAKEFVCFLLQEDPAKRPSAALALQEQ
WLQAGNGRSTGVLDTSRLTSFIERRKHQNDVRPIRSIKNFLQSRLLPRV

Transmembrane domain.
amino acids 2243-2263

N-glycosylation sites.
amino acids 167-170, 470-473, 692-695, 781-784, 952-955, 1194-1197, 2230-2233, 2316-2319, 2708-2711

Glycosaminoglycan attachment sites.
amino acids 238-241, 2232-2235

cAMP- and cGMP-dependent protein kinase phosphorylation sites.
amino acids 235-238, 1227-1230, 1723-1726, 1903-1906, 2060-2063, 2391-2394, 2403-2406, 2801-2804

# FIGURE 89B

Tyrosine kinase phosphorylation sites.
amino acids 348-355, 633-640, 2330-2337, 2553-2560

N-myristoylation sites.
amino acids 239-244, 302-307, 460-465, 536-541, 874-879, 1354-1359, 1598-1603, 1650-1655, 1779-1784, 1792-1797, 2233-2238, 2234-2239, 2235-2240, 2236-2241, 2237-2242, 2238-2243, 2239-2244, 2241-2246, 2245-2250, 2247-2252, 2519-2524, 2530-2535, 2547-2552, 2581-2586, 2606-2611, 2618-2623, 2730-2735, 2817-2822, 2823-2828

Amidation sites.
amino acids 1558-1561, 2412-2415

Leucine zipper pattern.
amino acids 1850-1871

Serine/Threonine protein kinases active-site signature.
amino acids 2675-2687

RhoGEF domains.
amino acids 1237-1407, 1914-2085

Protein kinase domain.
amino acids 2560-2814

PH domains.
amino acids 1421-1532, 2099-2212

Spectrin repeats.
amino acids 159-279, 281-387, 507-613, 614-725, 848-953, 1079-1185

Immunoglobulin domain.
amino acids 2463-2525

# FIGURE 90

CCACGTCCGGGGTGCCGAGCCAACTTTCCTGCGTCCATGCAGCCCCGCCGGCAACGGCTGCCCGCTCCCTGGTC
CGGGCCCAGGGGCCCGCGCCCCACCGCCCCGCTGCTCGCGCTGCTGCTGTTGCTCGCCCCGGTGGCGGCGCCCG
CGGGGTCCGGGGGCCCCGACGACCCTGGGCAGCCTCAGGATGCTGGGGTCCCGCGCAGGCTCCTGCAGCAGAAG
GCGCGCGCGGCGCTTCACTTCTTCAACTTCCGGTCCGGCTCGCCCAGCGCGCTGCGAGTGCTGGCCGAGGTGCA
GGAGGGCCGCGCGTGGATTAATCCAAAAGAGGGATGTAAAGTTCACGTGGTCTTCAGCACAGAGCGCTACAACC
CAGAGTCTTTACTTCAGGAAGGTGAGGGACGTTTGGGGAAATGTTCTGCTCGAGTGTTTTTCAAGAATCAGAAA
CCCAGACCAACCATCAATGTAACTTGTACACGGCTCATCGAGAAAAAGAAAAGACAACAAGAGGATTACCTGCT
TTACAAGCAAATGAAGCAACTGAAAAACCCCTTGGAAATAGTCAGCATACCTGATAATCATGGACATATTGATC
CCTCTCTGAGACTCATCTGGGATTTGGCTTTCCTTGGAAGCTCTTACGTGATGTGGGAAATGACAACACAGGTG
TCACACTACTACTTGGCACAGCTCACTAGTGTGAGGCAGTGGGTAAGAAAAACCTGAAAATTAACTTGTGCCAC
AAGAGTTACAATCAAAGTGGTCTCCTTAGACTGAATTCATGTGAACTTCTAATTTCATATCAAGAGTTGTAATC
ACATTTATTTCAATAAATATGTGAGTTCCTGC

# FIGURE 91

GAATTCCATTGTGTTGGGGCCCTGGGGGCGGAGGGGAGGGGCCCACCACGGCCTTATTTCCGCGAGCGCCGGCA
CTGCCCGCTCCGAGCCCGTGTCTGTCGGGTGCCGAGCCAACTTTCCTGCGTCCATGCAGCCCCGCCGGCAACGG
CTGCCCGCTCCCTGGTCCGGGCCCAGGGGCCCGCGCCCCACCGCCCCGCTGCTCGCGCTGCTGCTGTTGCTCGC
CCCGGTGGCGGCGCCCGCGGGGTCCGGGGACCCCGACGACCCTGGGCAGCCTCAGGATGCTGGGGTCCCGCGCA
GGCTCCTGCAGCAGGCGGCGCGCGCGGCGCTTCACTTCTTCAACTTCCGGTCCGGCTCGCCCAGCGCGCTGCGA
GTGCTGGCCGAGGTGCAGGAGGGCCGCGCGTGGATTAATCCAAAAGAGGGATGTAAAGTTCACGTGGTCTTCAG
CACAGAGCGCTACAACCCAGAGTCTTTACTTCAGGAAGGTGAGGGACGTTTGGGGAAATGTTCTGCTCGAGTGT
TTTTCAAGAATCAGAAACCCAGACCAACTATCAATGTAACTTGTACACGGCTCATCGAGAAAAAGAAAAGACAA
CAAGAGGATTACCTGCTTTACAAGCAAATGAAGCAACTGAAAAACCCCTTGGAAATAGTCAGCATACCTGATAA
TCATGGACATATTGATCCCTCTCTGAGACTCATCTGGGATTTGGCTTTCCTTGGAAGCTCTTACGTGATGTGGG
AAATGACAACACAGGTGTCACACTACTACTTGGCACAGCTCACTAGTGTGAGGCAGTGGAAAACTAATGATGAT
ACAATTGATTTTGATTATACTGTTCTACTTCATGAATTATCAACACAGGAAATAATTCCCTGTCGCATTCACTT
GGTCTGGTACCCTGGCAAACCTCTTAAAGTGAAGTACCACTGTCAAGAGCTACAGACACCAGAAGAAGCCTCCG
GAACTGAAGAAGGATCAGCTGTAGTACCAACAGAGCTTAGTAATTTCTAAAAAGAAAAAATGATCTTTTTCCGA
CTTCTAAACAAGTGACTATACTAGCATAAATCATTCTTCTAGTAAAACAGCTAAGGTATAGACATTCTAATAAT
TTGGGAAAACCTATGATTACAAGTAAAAACTCAGAAATGCAAAGATGTTGGTTTTTTGTTTCTCAGTCTGCTTT
AGCTTTTAACTCTGGAAGCGCATGCACACTGAACTCTGCTCAGTGCTAAACAGTCACCAGCAGGTTCCTCAGGG
TTTCAGCCCTAAAATGTAAAACCTGGATAATCAGTGTATGTTGCACCAGAATCAGCATTTTTTTTTTAACTGCA
AAAAATGATGGTCTCATCTCTGAATTTATATTTCTCATTCTTTTGAACATACTATAGCTAATATATTTTATGTT
GCTAAATTGCTTCTATCTAGCATGTTAAACAAAGATAATATACTTTCGATGAAAGTAAATTATAGGAAAAAAAT
TAACTGTTTTAAAAAGAACTTGATTATGTTTTATGATTTCAGGCAAGTATTCATTTTTAACTTGCTACCTACTT
TTAAATAAATGTTTACATTTCTAAAAAAAAAAAAAAAA

# FIGURE 92

MQPRRQRLPAPWSGPRGPRPTAPLLALLLLLAPVAAPAGSGGPDDPGQPQDAGVPRRLLQQKARAALHFFNFRS
GSPSALRVLAEVQEGRAWINPKEGCKVHVVFSTERYNPESLLQEGEGRLGKCSARVFFKNQKPRPTINVTCTRL
IEKKKRQQEDYLLYKQMKQLKNPLEIVSIPDNHGHIDPSLRLIWDLAFLGSSYVMWEMTTQVSHYYLAQLTSVR
QWVRKT

Transmembrane domain (type II).
amino acids 17-37

N-glycosylation site.
amino acids 142-145

Tyrosine kinase phosphorylation site.
amino acids 154-162

N-myristoylation site.
amino acids 75-80

# FIGURE 93

MQPRRQRLPAPWSGPRGPRPTAPLLALLLLLAPVAAPAGSGDPDDPGQPQDAGVPRRLLQQAARAALHFFNFRS
GSPSALRVLAEVQEGRAWINPKEGCKVHVVFSTERYNPESLLQEGEGRLGKCSARVFFKNQKPRPTINVTCTRL
IEKKKRQQEDYLLYKQMKQLKNPLEIVSIPDNHGHIDPSLRLIWDLAFLGSSYVMWEMTTQVSHYYLAQLTSVR
QWKTNDDTIDFDYTVLLHELSTQEIIPCRIHLVWYPGKPLKVKYHCQELQTPEEASGTEEGSAVVPTELSNF

Signal sequence.
amino acids 1-36

N-glycosylation site.
amino acids 142-145

Tyrosine kinase phosphorylation site.
amino acids 154-162

N-myristoylation sites.
amino acids 75-80, 279-284

# FIGURE 94

CGCGCCGTGCGTCCGCGCCCGGCCGCCAGGTGCCCCAGTAGCCCGACCGCCGAGATGCCCAGCCCGCCGGGGCT
CCGGGCGCTATGGCTTTGCGCCGCGCTGTGCGCTTCCCGGAGGGCCGGCGGCGCCCCCCAGCCCGGCCCGGGGC
CCACCGCCTGCCCGGCCCCCTGCCACTGCCAGGAGGACGGCATCATGCTGTCTGCCGACTGCTCTGAGCTCGGG
CTGTCCGCCGTTCCGGGGGACCTGGACCCCCTGACGGCTTACCTGGACCTCAGCATGAACAACCTCACAGAGCT
TCAGCCTGGCCTCTTCCACCACCTGCGCTTCTTGGAGGAGCTGCGTCTCTCTGGGAACCATCTCTCACACATCC
CAGGACAAGCATTCTCTGGTCTCTACAGCCTGAAAATCCTGATGCTGCAGAACAATCAGCTGGGAGGAATCCCC
GCAGAGGCGCTGTGGGAGCTGCCGAGCCTGCAGTCGCTGCGCCTAGATGCCAACCTCATCTCCCTGGTCCCGGA
GAGGAGCTTTGAGGGGCTGTCCTCCCTCCGCCACCTCTGGCTGGACGACAATGCACTCACGGAGATCCCTGTCA
GGGCCCTCAACAACCTCCCTGCCCTGCAGGCCATGACCCTGGCCCTCAACCGCATCAGCCACATCCCCGACTAC
GCGTTCCAGAATCTCACCAGCCTTGTGGTGCTGCATTTGCATAACAACCGCATCCAGCATCTGGGGACCCACAG
CTTCGAGGGGCTGCACAATCTGGAGACACTAGACCTGAATTATAACAAGCTGCAGGAGTTCCCTGTGGCCATCC
GGACCCTGGGCAGACTGCAGGAACTGGGGTTCCATAACAACAACATCAAGGCCATCCCAGAAAAGGCCTTCATG
GGGAACCCTCTGCTACAGACGATACACTTTTATGATAACCCAATCCAGTTTGTGGGAAGATCGGCATTCCAGTA
CCTGCCTAAACTCCACACACTATCTCTGAATGGTGCCATGGACATCCAGGAGTTTCCAGATCTCAAAGGCACCA
CCAGCCTGGAGATCCTGACCCTGACCCGCGCAGGCATCCGGCTGCTCCCATCGGGGATGTGCCAACAGCTGCCC
AGGCTCCGAGTCCTGGAACTGTCTCACAATCAAATTGAGGAGCTGCCCAGCCTGCACAGGTGTCAGAAATTGGA
GGAAATCGGCCTCCAACACAACCGCATCTGGGAAATTGGAGCTGACACCTTCAGCCAGCTGAGCTCCCTGCAAG
CCCTGGATCTTAGCTGGAACGCCATCCGGTCCATCCACCCTGAGGCCTTCTCCACCCTGCACTCCCTGGTCAAG
CTGGACCTGACAGACAACCAGCTGACCACACTGCCCCTGGCTGGACTTGGGGGCTTGATGCATCTGAAGCTCAA
AGGGAACCTTGCTCTCTCCCAGGCCTTCTCCAAGGACAGTTTCCCAAAACTGAGGATCCTGGAGGTGCCTTATG
CCTACCAGTGCTGTCCCTATGGGATGTGTGCCAGCTTCTTCAAGGCCTCTGGGCAGTGGGAGGCTGAAGACCTT
CACCTTGATGATGAGGAGTCTTCAAAAAGGCCCCTGGGCCTCCTTGCCAGACAAGCAGAGAACCACTATGACCA
GGACCTGGATGAGCTCCAGCTGGAGATGGAGGACTCAAAGCCACACCCCAGTGTCCAGTGTAGCCCTACTCCAG
GCCCCTTCAAGCCCTGTGAGTACCTCTTTGAAAGCTGGGGCATCCGCCTGGCCGTGTGGGCCATCGTGTTGCTC
TCCGTGCTCTGCAATGGACTGGTGCTGCTGACCGTGTTCGCTGGCGGGCCTGCCCCCCTGCCCCCGGTCAAGTT
TGTGGTAGGTGCGATTGCAGGCGCCAACACCTTGACTGGCATTTCCTGTGGCCTTCTAGCCTCAGTCGATGCCC
TGACCTTTGGTCAGTTCTCTGAGTACGGAGCCCGCTGGGAGACGGGGCTAGGCTGCCGGGCCACTGGCTTCCTG
GCAGTACTTGGGTCGGAGGCATCGGTGCTGCTGCTCACTCTGGCCGCAGTGCAGTGCAGCGTCTCCGTCTCCTG
TGTCCGGGCCTATGGGAAGTCCCCCTCCCTGGGCAGCGTTCGAGCAGGGGTCCTAGGCTGCCTGGCACTGGCAG
GGCTGGCCGCCGCACTGCCCCTGGCCTCAGTGGGAGAATACGGGGCCTCCCCACTCTGCCTGCCCTACGCGCCA
CCTGAGGGTCAGCCAGCAGCCCTGGGCTTCACCGTGGCCCTGGTGATGATGAACTCCTTCTGTTTCCTGGTCGT
GGCCGGTGCCTACATCAAACTGTACTGTGACCTGCCGCGGGGCGACTTTGAGGCCGTGTGGGACTGCGCCATGG
TGAGGCACGTGGCCTGGCTCATCTTCGCAGACGGGCTCCTCTACTGTCCCGTGGCCTTCCTCAGCTTCGCCTCC
ATGCTGGGCCTCTTCCCTGTCACGCCCGAGGCCGTCAAGTCTGTCCTGCTGGTGGTGCTGCCCCTGCCTGCCTG
CCTCAACCCACTGCTGTACCTGCTCTTCAACCCCCACTTCCGGGATGACCTTCGGCGGCTTCGGCCCCGCGCAG
GGGACTCAGGGCCCCTAGCCTATGCTGCGGCCGGGGAGCTGGAGAAGAGCTCCTGTGATTCTACCCAGGCCCTG
GTAGCCTTCTCTGATGTGGATCTCATTCTGGAAGCTTCTGAAGCTGGGCGGCCCCCTGGGCTGGAGACCTATGG
CTTCCCCTCAGTGACCCTCATCTCCTGTCAGCAGCCAGGGGCCCCCAGGCTGGAGGGCAGCCATTGTGTAGAGC
CAGAGGGGAACCACTTTGGGAACCCCCAACCCTCCATGGATGGAGAACTGCTGCTGAGGGCAGAGGGATCTACG
CCAGCAGGTGGAGGCTTGTCAGGGGGTGGCGGCTTTCAGCCCTCTGGCTTGGCCTTTGCTTCACACGTGTAAAT
ATCCCTCCCCATTCTTCTCTTCCCCTCTCTTCCCTTTCCTCTCTCCCCCTCGGTGAATGATGGCTGCTTCTAAA
ACAAATACAACCAAAACTCAGCAGTGTGATCTATAGCAGGATGGCCCAGTACCTGGCTCCACTGATCACCTCTC
TCCTGTGACCATCACCAACGGGTGCCTCTTGGCCTGGCTTTCCCTTGGCCTTCCTCAGCTTCACCTTGATACTG
GGCCTCTTCCTTGTCATGTCTGAAGCTGTGGACCARAGACCTGGACTTTTGTCTGCTTAAGGGAAATGAGGGAA
GTAAAGACAGTGAAGGGGTGGAGGGTTGATCAGGGCACAGTGGACAGGGAGACCTCACARAAAAGGCCTGGAA
GGKGATTTCCCGTGTGACTCATGGRTAGGAWACAAAATGTGTTCCATGTACCATTAATCTTGACATATGCCATG
CATAAARACTTCCTATTAAAATAAGCTTTGGRAGAGATT

# FIGURE 95

MPSPPGLRALWLCAALCASRRAGGAPQPGPGPTACPAPCHCQEDGIMLSADCSELGLSAVPGDLDPLTAYLDLS
MNNLTELQPGLFHHLRFLEELRLSGNHLSHIPGQAFSGLYSLKILMLQNNQLGGIPAEALWELPSLQSLRLDAN
LISLVPERSFEGLSSLRHLWLDDNALTEIPVRALNNLPALQAMTLALNRISHIPDYAFQNLTSLVVLHLHNNRI
QHLGTHSFEGLHNLETLDLNYNKLQEFPVAIRTLGRLQELGFHNNNIKAIPEKAFMGNPLLQTIHFYDNPIQFV
GRSAFQYLPKLHTLSLNGAMDIQEFPDLKGTTSLEILTLTRAGIRLLPSGMCQQLPRLRVLELSHNQIEELPSL
HRCQKLEEIGLQHNRIWEIGADTFSQLSSLQALDLSWNAIRSIHPEAFSTLHSLVKLDLTDNQLTTLPLAGLGG
LMHLKLKGNLALSQAFSKDSFPKLRILEVPYAYQCCPYGMCASFFKASGQWEAEDLHLDDEESSKRPLGLLARQ
AENHYDQDLDELQLEMEDSKPHPSVQCSPTPGPFKPCEYLFESWGIRLAVWAIVLLSVLCNGLVLLTVFAGGPA
PLPPVKFVVGAIAGANTLTGISCGLLASVDALTFGQFSEYGARWETGLGCRATGFLAVLGSEASVLLLTLAAVQ
CSVSVSCVRAYGKSPSLGSVRAGVLGCLALAGLAAALPLASVGEYGASPLCLPYAPPEGQPAALGFTVALVMMN
SFCFLVVAGAYIKLYCDLPRGDFEAVWDCAMVRHVAWLIFADGLLYCPVAFLSFASMLGLFPVTPEAVKSVLLV
VLPLPACLNPLLYLLFNPHFRDDLRRLRPRAGDSGPLAYAAAGELEKSSCDSTQALVAFSDVDLILEASEAGRP
PGLETYGFPSVTLISCQQPGAPRLEGSHCVEPEGNHFGNPQPSMDGELLLRAEGSTPAGGGLSGGGGFQPSGLA
FASHV

Signal sequence.

amino acids 1-24

Transmembrane domains.

amino acids 564-584, 604-624, 646-666, 686-706, 730-750, 771-791, 809-829

N-glycosylation sites.

amino acids 77-80, 208-211

Glycosaminoglycan attachment site.

amino acids 951-954

Tyrosine kinase phosphorylation sites.

amino acids 469-475, 517-523

N-myristoylation sites.

amino acids 45-50, 99-104, 107-112, 127-132, 380-385, 483-488, 493-498, 563-568, 602-607, 612-617, 616-621, 641-646, 652-657, 684-689, 689-694, 698-703, 725-730, 922-927, 942-947, 947-952, 948-953, 949-954, 960-965

Microbodies C-terminal targeting signal.

amino acids 965-968

Cell attachment sequence.

amino acids 760-762

Leucine zipper patterns.

amino acids 57-79, 64-86, 71-93

Leucine rich repeats.

amino acids 67-90, 91-114, 115-138, 139-162, 163-186, 187-210, 211-234, 235-257, 258-281, 282-305, 306-328, 329-352, 353-374, 375-398, 399-422, 423-446

Leucine rich repeat N-terminal domain.

amino acids 34-65

Europäisches Patentamt
European Patent Office
Office européen des brevets

# EUROPEAN SEARCH REPORT

Application Number
EP 08 01 8727

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/61471 A (INCYTE PHARMA INC [US]; TANG Y TOM [US]; LAL PREETI [US]; HILLMAN JENN) 2 December 1999 (1999-12-02)<br>* the whole document *<br>----- | 1-15 | INV.<br>A61K31/00<br>A61P35/00<br>A61K47/48 |
| X | MARIANI L ET AL: "Glioma cell motility is associated with reduced transcription of proapoptotic and proliferation genes: a cDNA microarray analysis"<br>JOURNAL OF NEURO-ONCOLOGY, KLUWER, BOSTON, US,<br>vol. 53, no. 2, 1 June 2001 (2001-06-01), pages 161-176, XP003018417<br>ISSN: 0167-594X<br>* the whole document *<br>----- | 1-15 | |
| A | WO 01/45730 A (IMMUNEX CORP [US]; WILEY STEVEN R [US]) 28 June 2001 (2001-06-28)<br>* the whole document *<br>----- | 1-15 | |
| A | WO 01/88546 A (UNIV MICHIGAN [US]; HANASH SAMIR M [US]; RICKMAN DAVID [US]; TYAGI RAC) 22 November 2001 (2001-11-22)<br>* the whole document *<br>----- | 1-15 | TECHNICAL FIELDS SEARCHED (IPC)<br>C07K<br>A61K |
| A | WO 99/33869 A (CORIXA CORP [US])<br>8 July 1999 (1999-07-08)<br>* the whole document *<br>----- | 1-15 | |
| A | ANTAL-SZALMÁS P ET AL: "A novel flow cytometric assay to quantify soluble CD14 concentration in human serum."<br>CYTOMETRY 1 OCT 2001,<br>vol. 45, no. 2,<br>1 October 2001 (2001-10-01), pages 115-123, XP002479326<br>ISSN: 0196-4763<br>* the whole document *<br>-----<br>-/-- | 1-15 | |

The present search report has been drawn up for all claims

| Place of search | Date of completion of the search | Examiner |
|---|---|---|
| Munich | 20 April 2009 | Hermann, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 08 01 8727

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE UniProt [Online]<br>1 August 1988 (1988-08-01), "Monocyte differentiation antigen CD14 precursor (Myeloid cell-specific leucine-rich glycoprotein) [Contains: Monocyte differentiation antigen CD14, urinary form; Monocyte differentiation antigen CD14, membrane- bound form]."<br>XP002479327<br>retrieved from EBI accession no. UNIPROT:P08571<br>Database accession no. P08571<br>* the whole document *<br>-& FERRERO E ET AL: "NUCLEOTIDE SEQUENCE OF THE GENE ENCODING THE MONOCYTE DIFFERENTIATION ANTIGEN, CD14" NUCLEIC ACIDS RESEARCH, OXFORD UNIVERSITY PRESS, SURREY, GB,<br>vol. 16, no. 9,<br>1 January 1988 (1988-01-01), page 4173, XP002916988<br>ISSN: 0305-1048<br>* the whole document *<br>----- | 1-15 | |
| A | DATABASE Geneseq [Online]<br>3 January 1997 (1997-01-03), "Myelomonocytic differentiation antigen CD14."<br>XP002479328<br>retrieved from EBI accession no. GSP:AAW05316<br>Database accession no. AAW05316<br>* the whole document *<br>-& US 5 543 303 A (GOYERT SANNA M [US])<br>6 August 1996 (1996-08-06)<br>* the whole document *<br>----- | 1-15 | TECHNICAL FIELDS SEARCHED (IPC) |

The present search report has been drawn up for all claims

| Place of search | Date of completion of the search | Examiner |
|---|---|---|
| Munich | 20 April 2009 | Hermann, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 08 01 8727

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2009

| Patent document cited in search report | Publication date | Patent family member(s) | Publication date |
|---|---|---|---|
| WO 9961471 A | 02-12-1999 | AU 4409099 A | 13-12-1999 |
| | | CA 2329072 A1 | 02-12-1999 |
| | | EP 1080194 A2 | 07-03-2001 |
| | | JP 2002516082 T | 04-06-2002 |
| | | US 2007054312 A1 | 08-03-2007 |
| WO 0145730 A | 28-06-2001 | AU 782067 B2 | 30-06-2005 |
| | | AU 2731501 A | 03-07-2001 |
| | | CA 2394015 A1 | 28-06-2001 |
| | | EP 1239869 A2 | 18-09-2002 |
| | | JP 2003517829 T | 03-06-2003 |
| | | NZ 520171 A | 29-07-2005 |
| WO 0188546 A | 22-11-2001 | AU 6171001 A | 26-11-2001 |
| | | US 6680172 B1 | 20-01-2004 |
| WO 9933869 A | 08-07-1999 | AU 2010699 A | 19-07-1999 |
| | | CA 2316397 A1 | 08-07-1999 |
| | | EP 1992640 A1 | 19-11-2008 |
| | | EP 1042360 A2 | 11-10-2000 |
| | | JP 2002507387 T | 12-03-2002 |
| US 5543303 A | 06-08-1996 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4275149 A
- US 4816567 A
- US 5641870 A
- EP 404097 A
- WO 9311161 A
- US 5556762 A
- US 5750373 A
- US 4708871 A
- US 4833092 A
- US 5223409 A
- US 5403484 A
- US 5571689 A
- US 5663143 A
- WO 8403506 A
- WO 8403564 A
- WO 0000823 A
- WO 0039585 A
- US 5500362 A
- US 5821337 A
- WO 9845479 A
- US 4933294 A
- WO 9105264 A
- US 5401638 A
- US 5545806 A
- US 5569825 A
- US 5591669 A
- US 5545807 A
- WO 9717852 A
- US 5565332 A
- US 5573905 A
- US 5567610 A
- US 5229275 A
- US 5869046 A
- WO 9316185 A
- US 5571894 A
- US 5587458 A
- WO 9616673 A
- US 5837234 A
- WO 9802463 A
- WO 9308829 A
- WO 9404690 A
- US 5731168 A
- US 4676980 A
- WO 9100360 A
- WO 92200373 A
- EP 03089 A
- US 5739277 A
- WO 9411026 A
- US 3896111 A
- US 4151042 A
- US 4137230 A
- US 4248870 A
- US 4256746 A
- US 4260608 A
- US 4265814 A
- US 4294757 A
- US 4307016 A
- US 4308268 A
- US 4308269 A
- US 4309428 A
- US 4313946 A
- US 4315929 A
- US 4317821 A
- US 4322348 A
- US 4331598 A
- US 4361650 A
- US 4364866 A
- US 4424219 A
- US 4450254 A
- US 4362663 A
- US 4371533 A
- US 5208020 A
- US 5416064 A
- EP 0425235 B1
- US 5712374 A
- US 5714586 A
- US 5739116 A
- US 5767285 A
- US 5770701 A
- US 5770710 A
- US 5773001 A
- US 5877296 A
- US 5053394 A
- WO 9321232 A
- US 4485045 A
- US 4544545 A
- WO 9738731 A
- US 5013556 A
- WO 9534683 A
- US 5627024 A
- US 5766905 A
- WO 9814277 A
- WO 9820169 A
- WO 9820159 A
- WO 9820036 A
- WO 9735196 A
- WO 9746251 A
- WO 9747314 A

- WO 9709446 A
- US 5498538 A
- US 5432018 A
- WO 9815833 A
- US 5723286 A
- US 5580717 A
- US 5427908 A
- US 5498530 A
- US 5770434 A
- US 5734018 A
- US 5698426 A
- US 5763192 A
- US 5723323 A
- WO 8101145 A
- WO 8807378 A
- US 4975278 A
- US 5364934 A
- WO 8705330 A
- US 4640835 A
- US 4496689 A
- US 4301144 A
- US 4670417 A
- US 4791192 A
- US 4179337 A
- US 5428130 A
- WO 8905859 A
- US 4399216 A
- DD 266710
- US 4946783 A
- US 5648237 A
- US 5789199 A
- US 5840523 A, Simmons
- EP 139383 A
- US 4943529 A
- EP 402226 A
- EP 183070 A
- EP 244234 A
- EP 394538 A
- WO 9100357 A
- US 5010182 A
- EP 362179 A
- WO 9013646 A
- EP 36776 A
- EP 73657 A
- GB 2211504 A
- EP 117060 A
- EP 117058 A
- US 4767704 A
- US 4657866 A
- US 4927762 A
- US 4560655 A
- US 5122469 A
- WO 9003430 A
- WO 8700195 A
- US RE30985 E
- US 3773919 A
- EP 0600517 A
- EP 616812 A
- WO 9607321 A
- US 4892538 A
- US 5283187 A
- WO 9325673 A
- WO 9106629 A
- US 3687808 A
- US 4469863 A
- US 4476301 A
- US 5023243 A
- US 5177196 A
- US 5188897 A
- US 5264423 A
- US 5276019 A
- US 5278302 A
- US 5286717 A
- US 5321131 A
- US 5399676 A
- US 5405939 A
- US 5453496 A
- US 5455233 A
- US 5466677 A
- US 5476925 A
- US 5519126 A
- US 5536821 A
- US 5541306 A
- US 5550111 A
- US 5563253 A
- US 5571799 A
- US 5587361 A
- US 5194599 A
- US 5565555 A
- US 5527899 A
- US 5721218 A
- US 5672697 A
- US 5625050 A
- US 5034506 A
- US 5166315 A
- US 5185444 A
- US 5214134 A
- US 5216141 A
- US 5235033 A
- US 5264562 A
- US 5264564 A
- US 5405938 A
- US 5434257 A
- US 5470967 A
- US 5489677 A
- US 5541307 A
- US 5561225 A
- US 5596086 A
- US 5602240 A
- US 5610289 A
- US 5608046 A
- US 5618704 A
- US 5623070 A
- US 5663312 A
- US 5633360 A
- US 5677437 A

- US 5792608 A
- US 5646269 A
- US 5677439 A
- US 5539082 A
- US 5714331 A
- US 5719262 A
- WO 9839352 A
- WO 9914226 A
- US 4981957 A
- US 5118800 A
- US 5319080 A
- US 5359044 A
- US 5393878 A
- US 5446137 A
- US 5466786 A
- US 5514785 A
- US 5519134 A
- US 5567811 A
- US 5576427 A
- US 5591722 A
- US 5597909 A
- US 5610300 A
- US 5627053 A
- US 5639873 A
- US 5646265 A
- US 5658873 A
- US 5670633 A
- US 5792747 A
- US 5700920 A
- US 4845205 A
- US 5130302 A
- US 5134066 A
- US 5175273 A
- US 5367066 A
- US 5432272 A
- US 5457187 A
- US 5459255 A
- US 5484908 A
- US 5502177 A
- US 5525711 A
- US 5552540 A
- US 5587469 A
- US 5594121 A
- US 5596091 A
- US 5614617 A
- US 5645985 A
- US 5830653 A
- US 5763588 A
- US 6005096 A
- US 5681941 A
- US 5750692 A
- US 33413099 A
- US 4828979 A
- US 4948882 A
- US 5218105 A
- US 5525465 A
- US 5541313 A
- US 5545730 A
- US 5552538 A
- US 5578717 A
- US 5580731 A
- US 5591584 A
- US 5109124 A
- US 5118802 A
- US 5138045 A
- US 5414077 A
- US 5486603 A
- US 5512439 A
- US 5578718 A
- US 4587044 A
- US 4605735 A
- US 4667025 A
- US 4762779 A
- US 4789737 A
- US 4824941 A
- US 4835263 A
- US 4876335 A
- US 4904582 A
- US 4958013 A
- US 5082830 A
- US 5112963 A
- US 5214136 A
- US 5245022 A
- US 5254469 A
- US 5258506 A
- US 5262536 A
- US 5272250 A
- US 5292873 A
- US 5317098 A
- US 5371241 A
- US 5391723 A
- US 5416203 A
- US 5451463 A
- US 5510475 A
- US 5512667 A
- US 5565552 A
- US 5567810 A
- US 5574142 A
- US 5585481 A
- US 5587371 A
- US 5595726 A
- US 5597696 A
- US 5599923 A
- US 5599928 A
- US 5688941 A
- US 5013830 A
- US 5149797 A
- US 5220007 A
- US 5256775 A
- US 5366878 A
- US 5403711 A
- US 5491133 A
- US 5565350 A
- US 5623065 A
- US 5652355 A
- US 5652356 A

- US 5700922 A
- US 5108921 A
- US 5354844 A
- US 5416016 A
- US 5459127 A
- US 5521291 A
- US 5543158 A
- US 5547932 A
- US 5583020 A
- US 5591721 A
- US 4426330 A
- US 4534899 A
- US 5213804 A
- US 5227170 A
- US 5264221 A
- US 5356633 A
- US 5395619 A
- US 5417978 A
- US 5462854 A
- US 5469854 A
- US 5512295 A
- US 5527528 A
- US 5534259 A
- US 5543152 A
- US 5556948 A
- US 5580575 A
- US 5595756 A
- WO 9010048 A
- WO 9013641 A
- WO 9104753 A
- WO 9010448 A
- US 4736866 A
- US 4870009 A
- WO 9733551 A
- US 0110482 W
- EP 307247 A

**Non-patent literature cited in the description**


- **BORING et al.** *CA Cancel J. Clin.,* 1993, vol. 43, 7
- **NIELSEN et al.** *Prot. Eng.,* 1997, vol. 10, 1-6
- **VONHEINJE et al.** *Nucl. Acids. Res.,* 1986, vol. 14, 4683-4690
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **KABAT et al.** Sequences of Proteins of Immunological Interest. 1991
- **CHOTHIA ; LESK.** *J. Mol_Biol.,* 1987, vol. 196, 901-917
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **MORRISON et al.** *Proc. Natl. Acad. Sci. USA,* 1984, vol. 81, 6851-6855
- **ZAPATA et al.** *Protein Eng.,* 1995, vol. 8 (10), 1057-1062
- **PLUCKTHUN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **GEYSEN et al.** *Proc. Natl. Acad. Sci. U.S.A.,* 1984, vol. 81, 3998-4002
- **GEYSEN et al.** *Proc. Natl. Acad. Sci. U.S.A.,* 1985, vol. 82, 178-182
- **GEYSEN et al.** *Synthetic Peptides as Antigens,* 1986, 130-149
- **GEYSEN et al.** *J. Immunol. Meth.,* 1987, vol. 102, 259-274
- **SCHOOFS et al.** *J. Immunol.,* 1988, vol. 140, 611-616
- **CWIRLA, S. E. et al.** *Proc. Natl. Acad. Sci. USA,* 1990, vol. 87, 6378
- **LOWMAN, H.B. et al.** *Biochemistry,* vol. 30, 10832
- **CLACKSON, T. et al.** *Nature,* vol. 352, 624
- **MARKS, J. D. et al.** *J. Mol. Biol.,* 1991, vol. 222, 581
- **KANG, A.S. et al.** *Proc. Natl. Acad. Sci. USA,* vol. 88, 8363
- **SMITH, G. P.** *Current Opin. Biotechnol.,* 1991, vol. 2, 668
- **RAVETCH ; KINET.** *Annu. Rev. Immunol.,* 1991, vol. 9, 457-92
- **CLYNES et al.** *USA,* 1998, vol. 95, 652-656
- **DAËRON.** *Annu. Rev. Immunol.,* 1997, vol. 15, 203-234
- **RAVETCH ; KINET.** *Annu. Rev. Immunol.,* 1991, vol. 9, 457-492
- **CAPEL et al.** *Immunomethods,* 1995, vol. 4, 25-34
- **DE HAAS et al.** *J. Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249
- **GAZZANO-SANTORO et al.** *J. Immunol. Methods,* 1996, vol. 202, 163
- **MOORE et al.** *Cytotechnology,* 1995, vol. 17, 1-11

- **SIAS et al.** *J. Immunol. Methods,* 1990, vol. 132, 73-80
- Cell cycle regulation, oncogenes, and antineoplastic drugs. **MURAKAMI et al.** The Molecular Basis of Cancer. WB Saunders, 1995, 13
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON et al.** *Anal. Biochem.,* 1980, vol. 107, 220
- **SKERRA et al.** *Curr. Opinion in Immunol.,* 1993, vol. 5, 256-262
- **PLÜCKTHUN.** *Immunol. Revs.,* 1992, vol. 130, 151-188
- **MCCAFFERTY et al.** *Nature,* 1990, vol. 348, 552-554
- **MARKS et al.** *Bio/Technology,* 1992, vol. 10, 779-783
- **WATERHOUSE et al.** *Nuc. Acids. Res.,* 1993, vol. 21, 2265-2266
- **MORRISON et al.** *Proc. Natl Acad. Sci. USA,* 1984, vol. 81, 6851
- **RIECHMANN et al.** *Nature,* 1988, vol. 332, 323-327
- **VERHOEYEN et al.** *Science,* 1988, vol. 239, 1534-1536
- **SIMS et al.** *J. Immunol,* 1993, vol. 151, 2296
- **CHOTHIA et al.** *J. Mol. Biol.,* 1987, vol. 196, 901
- **CARTER et al.** *Proc. Natl. Acad. Sci. USA,* 1992, vol. 89, 4285
- **PRESTA et al.** *J. Immunol.,* 1993, vol. 151, 2623
- **JAKOBOVITS et al.** *Proc. Natl. Acad. Sci. USA,* 1993, vol. 90, 2551
- **JAKOBOVITS et al.** *Nature,* 1993, vol. 362, 255-258
- **BRUGGEMANN et al.** *Year in Immuno.,* 1993, vol. 7, 33
- **MCCAFFERTY et al.** *Nature,* 1990, vol. 348, 552-553
- **JOHNSON, KEVIN S. ; CHISWELL, DAVID J.** *Current Opinion in Structural Biology,* 1993, vol. 3, 564-571
- **CLACKSON et al.** *Nature,* vol. 352, 624-628
- **GRIFFITH et al.** *EMBO J.,* vol. 12, 725-734
- **MORIMOTO et al.** *Journal of Biochemical and Biophysical Methods,* 1992, vol. 24, 107-117
- **BRENNAN et al.** *Science,* 1985, vol. 229, 81
- **CARTER et al.** *Bio/Technology,* 1992, vol. 10, 163-167
- Antibody Engineering
- **MILLSTEIN et al.** *Nature,* 1983, vol. 305, 537-539
- **TRAUNECKE et al.** *EMBO J.,* 1991, vol. 10, 3655-3659
- **SURESH et al.** *Methods in Enzymology,* 1986, vol. 121, 210
- **SHALABY et al.** *J. Exp. Med.,* 1992, vol. 175, 217-225
- **KOSTELNY et al.** *J. Immunol,* 1992, vol. 148 (5), 1547-1553
- **GRUBER et al.** *J. Immunol.,* 1994, vol. 152, 5368
- **TUTT et al.** *J. Immunol.,* 1991, vol. 147, 60
- **CARON et al.** *J. Exp Med.,* 1992, vol. 176, 1191-1195
- **SHOPES, B.** *J. Immunol.,* 1992, vol. 148, 2918-2922
- **WOLFF et al.** *Cancer Research,* 1993, vol. 53, 2560-2565
- **STEVENSON et al.** *Anti-Cancer Drug Design,* 1989, vol. 3, 219-230
- **VITETTA et al.** *Science,* 1987, vol. 238, 1098
- **LIU et al.** *Proc. Natl. Acad. Sci. USA,* 1996, vol. 93, 8618-8623
- **CHAR et al.** *Cancer Research,* 1992, vol. 52, 127-131
- **CHARI et al.** *Cancer Research,* 1992, vol. 52, 127-131
- **CARLSSON et al.** *Biochem. J.,* 1978, vol. 173, 723-737
- **HINMAN et al.** *Cancer Research,* 1993, vol. 53, 3336-3342
- **LODE et al.** *Cancer Research,* 1998, vol. 58, 2925-2928
- **FRAKER et al.** *Biochem. Biophys. Res. Commun,* 1978, vol. 80, 49-57
- **EPSTEIN et al.** *Proc. Natl. Acad. Sci. USA,* 1985, vol. 82, 3688
- **HWANG et al.** *Proc. Natl Acad. Sci. USA,* 1980, vol. 77, 4030
- **MARTIN et al.** *J. Biol. Chem,* 1982, vol. 257, 286-288
- **GABIZON et al.** *J. National Cancer Inst.,* 1989, vol. 81 (19), 1484
- **GEYSEN et al.** *J. Immunol.,* 1987, vol. 102, 259-274
- **LOWMAN, H.B. et al.** *Biochemistry,* 1991, vol. 30, 10832
- **CLACKSON, T. et al.** *Nature,* 1991, vol. 352, 624
- **KANG, A.S. et al.** *Proc. Natl. Acad. Sci. USA,* 1991, vol. 88, 8363
- **SMITH, G.** *Current Opin. Biotechnol.,* 1991, vol. 2, 668
- **SCOTT, J.K. ; SMITH, G. P.** *Science,* 1990, vol. 249, 386
- **REN, Z-J. et al.** *Gene,* 1998, vol. 215, 439

- **ZHU, Z.** *CAN,* 1997, vol. 33, 534
- **JIANG.** *J. metal,* 1997, vol. 128, 44380
- **REN, Z-J. et al.** *CAN,* 1997, vol. 127, 215644
- **REN, Z-J.** *Protein Sci.,* 1996, vol. 5, 1833
- **EFIMOV, V. P. et al.** *Virus Gene,* 1995, vol. 10, 173
- **SMITH, G. P ; D SCOTT, J.K.** *Methods in Enzymology,* 1993, vol. 217, 228-257
- **LI et al.** *Mol Biotech.,* 1998, vol. 9, 187
- Antibodies, A Laboratory Manual. Cold Spring Harbor Laboratory, 1988
- **MASSEY.** *Nature,* 1987, vol. 328, 457-458
- **NEUBERGER et al.** *Nature,* 1984, vol. 312, 604-608
- **CUNNINGHAM ; WELLS.** *Science,* 1989, vol. 244, 1081-1085
- **CREIGHTON.** The Proteins. W.H. Freeman & Co., N.Y,
- **CHOTHIA.** *J. Mol. Biol.,* 1976, vol. 150, 1
- **T.E. CREIGHTON.** Proteins: Structure and Molecular Properties. W.H. Freeman & Co, 1983, 79-86
- **APLIN ; WRISTON.** *CRC Crit. Rev. Biochem.,* 1981, 259-306
- **HAKIMUDDIN et al.** *Arch. Biochem. Biophys.,* 1987, vol. 259, 52
- **EDGE et al.** *Anal. Biochem.,* 1981, vol. 118, 131
- **THOTAKURA et al.** *Meth. Enzymol.,* 1987, vol. 138, 350
- Remington's Pharmaceutical Sciences. 1980
- **FIELD et al.** *Mol. Cell. Biol.,* 1988, vol. 8, 2159-2165
- **EVAN et al.** *Molecular and Cellular Biology,* 1985, vol. 5, 3610-3616
- **PABORSKY et al.** *Protein Engineering,* 1990, vol. 3 (6), 547-553
- **HOPP et al.** *BioTechnoloey,* 1988, 1204-1210
- **MARTIN.** *Science,* 1992, vol. 255, 192-194
- **SKINNER et al.** *J. Biol. Chem.,* 1991, vol. 266, 15163-15166
- **FREYERMUTH et al.** *Proc. Natl. Acad. Sci. USA,* 1990, vol. 87, 6393-6397
- **STEWART et al.** Solid-Phase Peptide Synthesis. W.H. Freeman Co, 1989
- **MERRIFIELD.** *J. Am. Chem. Soc.,* 1963, vol. 85, 2149-2154
- **SAMBROOK et al.** Molecular Cloning: A Laboratory Manual. Cold Spring Harbor Laboratory Press, 1989
- **DIEFFENBACH et al.** PCR Primer: A Laboratory Manual. Cold Spring Harbor Laboratory Press, 1995
- Mammalian Cell Biotechnology: a Practical Approach. IRL Press, 1991
- **SHAW et al.** *Gene,* 1983, vol. 23, 315
- **GRAHAM ; VAN DER EB.** *Virology,* 1978, vol. 52, 456-457
- **VAN SOLINGEN et al.** *J. Bact.,* 1977, vol. 130, 946
- **HSIAO et al.** *Proc. Natl. Acad. Sci. (USA),* 1979, vol. 76, 3829
- **KEOWN et al.** *Methods in Enzymology,* 1990, vol. 185, 527-537
- **MANSOUR et al.** *Nature,* 1988, vol. 336, 348-352
- **BEACH ; NURSE.** *Nature,* 1981, vol. 290, 140
- **FLEER et al.** *Bio/TechnologY,* 1991, vol. 9, 968-975
- **LOUVENCOURT et al.** *J. Bacteriol.,* 1983, vol. 154 (2), 737-742
- **VAN DEN BERG et al.** *Bio/Technoiogy,* 1990, vol. 8, 135
- **SREEKRISHNA et al.** *J. Basic Microbiol.,* 1988, vol. 28, 265-278
- **CASE et al.** *Proc. Natl. Acad. Sci. USA,* 1979, vol. 76, 5259-5263
- **BALLANCE et al.** *Biochem. Bionhys. Res. Commun.,* 1983, vol. 112, 284-289
- **TILBUM et al.** *Gene,* 1983, vol. 26, 205-221
- **YELTON et al.** *Proc. Natl. Acad. Sci. USA,* 1984, vol. 81, 1470-1474
- **KELLY ; HYNES.** *EMBO J.,* vol. 4, 475-479
- **C. ANTHONY.** *The Biochemistry of Methylotrophs,* 1982, vol. 269
- **GRAHAM et al.** *J. Gen Virol.,* 1977, vol. 36, 59
- **URLAUB et al.** *Proc. Natl. Acad. Sci. USA,* 1980, vol. 77, 4216
- **MATHER.** *Biol. Reprod.,* 1980, vol. 23, 243-251
- **MATHER et al.** *Annals N.Y. Acad. Sci.,* 1982, vol. 383, 44-68
- **STINCHCOMB et al.** *Nature,* 1979, vol. 282, 39
- **KINGSMAN et al.** *Gene,* 1979, vol. 7, 141
- **TSCHEMPER et al.** *Gene,* 1980, vol. 10, 157
- **JONES.** *Genetics,* 1977, vol. 85, 12
- **CHANG et al.** *Nature,* 1978, vol. 275, 615
- **GOEDDEL et al.** *Nature,* 1979, vol. 281, 544
- **GOEDDEL.** *Nucleic Acids Res.,* 1980, vol. 8, 4057
- **DEBOER et al.** *Proc. Natl. Acad. Sci. USA,* 1983, vol. 80, 21-25
- **HITZEMAN et al.** *J. Biol. Chem.,* 1980, vol. 255, 2073
- **HESS et al.** *J. Adv. Enzyme Reg.,* 1968, vol. 7, 149
- **HOLLAND.** *Biochemistry,* 1978, vol. 17, 4900
- **GETHING et al.** *Nature,* 1981, vol. 293, 620-625
- **MANTEI et al.** *Nature,* 1979, vol. 281, 40-46
- **HAM et al.** *Meth. Enz.,* 1979, vol. 58, 44
- **BARNES et al.** *Anal. Biochem.,* 1980, vol. 102, 255

- **THOMAS.** *Proc. Natl. Acad. Sci. USA,* 1980, vol. 77, 5201-5205
- **DEUTSCHER.** *Methods in Enzymology,* 1990, vol. 182
- **SCOPES.** Protein Purification: Principles and Practice. Springer-Verlag, 1982
- **CARTER et al.** *Bio/Teclmology,* 1992, vol. 10, 163-167
- **LINDMARK et al.** *J. Immunol. Meth.,* 1983, vol. 62, 1-13
- **GUS et al.** *EMBO J.,* 1986, vol. 5, 15671575
- **ANDERSON et al.** *Science,* 1992, vol. 256, 808-813
- **NIELSEN et al.** *Science,* 1981, vol. 254, 1497-1500
- **MARTIN et al.** *Helv. Chim. Acta,* 1995, vol. 78, 486-504
- The Concise Encyclopedia Of Polymer Science And Engineering. John Wiley & Sons, 858-859
- **SANGHVI et al.** Antisense Research and Applications. CRC Press, 1983, 276-278
- **LETSINGER et al.** *Proc. Natl. Acad. Sci. USA,* 1989, vol. 86, 6553-6556
- **MANOHARAN et al.** *Bioorg. Med. Chem. Let,* 1994, vol. 4, 1053-1060
- **MANOHARAN et al.** *Ann. N.Y. Acad. Sci.,* 1992, vol. 660, 306-309
- **MANOHARAN et al.** *Bioorg. Med. Chem. Let.,* 1993, vol. 3, 2765-2770
- **OBERHAUSER et al.** *Nucl. Acids Res.,* vol. 20, 533-538
- **SAISON-BEHMOARAS et al.** *EMBO J.,* vol. 10, 1111-1118
- **KABANOV et al.** *FEBS Lett.,* vol. 259, 327-330
- **SVINARCHUK et al.** *Biochimie,* vol. 75, 49-54
- **MANOHARAN et al.** *Tetrahedron Lett.,* 1995, vol. 36, 3651-3654
- **SHEA et al.** *Nucl. Acids Res.,* vol. 18, 3777-3783
- **MANOHARAN et al.** *Nucleosides & Nucleotides,* vol. 14, 969-973
- **MISBRA et al.** *Biochim. Biophys. Acta,* 1995, vol. 1264, 229-237
- **THOMAS ; CAPECCHI.** *Cell,* 1987, vol. 51, 503
- **LI et al.** *Cell,* 1992, vol. 69, 915
- **BRADLEY.** Teratocarcinomas and Embryonic Stem Cells: A Practical Approach. 1987, 113-152
- **ZAMECNIK et al.** *Proc. Natl. Acad. Sci. USA,* 1986, vol. 83, 4143-4146
- **DZAU et al.** *Trends in Biotechnology,* 1993, vol. 11, 205-210
- **WU et al.** *J. Biol. Chem.,* 1987, vol. 262, 4429-4432
- **WAGNER et al.** *Proc. Natl. Acad. Sci. USA,* 1990, vol. 87, 3410-3414
- **FIELDS ; SONG.** *Nature,* 1989, vol. 340, 245-246
- **CHIEN et al.** *Proc. Natl. Acad. Sci. USA,* 1981, vol. 88, 9578-9582
- **CHEVRAY ; NATHANS.** *Proc. Natl. Acad. Sci. USA,* 1991, vol. 89, 5789-5793
- **ROSSI.** *Current Biology,* 1994, vol. 4, 469-471
- **MARASCO et al.** *Proc. Natl. Acad. Sci. USA,* 1993, vol. 90, 7889-7893
- **LU ; GILLETT.** *Cell Vision,* 1994, vol. 1, 169-176
- **O'REILLEY et al.** Baculovirus expression vectors: A Laboratory Manual. Oxford University Press, 1994
- **RUPERT et al.** *Nature,* 1993, vol. 362, 175-179

Sequence Listing

<110> GENENTECH, INC.

<120> COMPOSITIONS AND METHODS FOR THE DIAGNOSIS AND TREATMENT OF TUMOR

<130> CPS/FP6580922

<140> Divisional of EP 02806285.9
<141> 2002-12-30

<150> US 60/345,444
<151> 2002-01-02

<150> US 60/351,885
<151> 2002-01-25

<150> US 60/360,066
<151> 2002-02-25

<150> US 60/362,004
<151> 2002-03-05

<150> US 60/366,869
<151> 2002-03-20

<150> US 60/366,284
<151> 2002-03-21

<150> US 60/368,679
<151> 2002-03-28

<150> US 60/404,809
<151> 2002-08-19

<150> US 60/405,645
<151> 2002-08-21

<160> 95

<210> 1
<211> 142
<212> DNA
<213> Homo sapien

<400> 1
agaaactcaa gattgactca tgaggacctg aagggtgaca tcccaggagg 50

ggcctctgaa atttcccaca ccccagcgcc tgtgctgagg actccctcca 100

tgtggcccca ggtgccacca ataaaaatcc tacagaaaat tc 142

<210> 2
<211> 1346
<212> DNA
<213> Homo sapien

<400> 2
ctggaagccg gcgggtgccg ctgtgtagga aagaagctaa agcacttcca 50

gagcctgtcc ggagctcaga ggttcggaag acttatcgac catggagcgc 100

gcgtcctgct tgttgctgct gctgctgccg ctggtgcacg tctctgcgac 150

cacgccagaa ccttgtgagc tggacgatga agatttccgc tgcgtctgca 200

acttctccga acctcagccc gactggtccg aagccttcca gtgtgtgtct 250

gcagtagagg tggagatcca tgccggcggt ctcaacctag agccgtttct 300

aaagcgcgtc gatgcggacg ccgacccgcg gcagtatgct gacacggtca 350

aggctctccg cgtgcggcgg ctcacagtgg gagccgcaca ggttcctgct 400

cagctactgg taggcgccct gcgtgtgcta gcgtactccc gcctcaagga 450

actgacgctc gaggacctaa agataaccgg caccatgcct ccgctgcctc 500

tggaagccac aggacttgca ctttccagct tgcgcctacg caacgtgtcg 550

tgggcgacag ggcgttcttg gctcgccgag ctgcagcagt ggctcaagcc 600

aggcctcaag gtactgagca ttgcccaagc acactcgcct gccttttcct 650

gcgaacaggt tcgcgccttc ccggccctta ccagcctaga cctgtctgac 700

aatcctggac tgggcgaacg cggactgatg gcggctctct gtccccacaa 750

gttcccggcc atccagaatc tagcgctgcg caacacagga atggagacgc 800

ccacaggcgt gtgcgccgca ctggcggcgg caggtgtgca gccccacagc 850

ctagacctca gccacaactc gctgcgcgcc accgtaaacc ctagcgctcc 900

gagatgcatg tggtccagcg ccctgaactc cctcaatctg tcgttcgctg 950

ggctggaaca ggtgcctaaa ggactgccag ccaagctcag agtgctcgat 1000

ctcagctgca acagactgaa cagggcgccg cagcctgacg agctgcccga 1050

ggtggataac ctgacactgg acgggaatcc cttcctggtc cctggaactg 1100

ccctccccca cgagggctca atgaactccg gcgtggtccc agcctgtgca 1150

cgttcgaccc tgtcggtggg ggtgtcggga accctggtgc tcctccaagg 1200

ggcccggggc tttgcctaag atccaagaca gaataatgaa tggactcaaa 1250

ctgccttggc ttcaggggag tcccgtcagg acgttgagga cttttcgacc 1300

aattcaaccc tttgccccac ctttattaaa atcttaaaca acaaaa 1346

<210> 3
<211> 1110
<212> DNA
<213> Homo sapien

<400> 3
gggcgggcct cacccgcttc gagtcctcgg gcttccccca cccggcccgt 50

gggggagtat ctgtcctgcc gccttcgccc acgccctgca ctccgggacc 100

gtccctgcgc gctctgggcg accatggccc gcggggctgc gctggcgctg 150

ctgctcttcg gcctgctggg tgttctggtc gccgccccgg atggtggttt 200

cgatttatct gatgcccttc ctgacaatga aaacaagaaa cccactgcaa 250

tccccaagaa acccagtgct ggggatgact ttgacttagg agatgctgtt 300

gttgatggag aaaatgacga cccacgacca ccgaacccac ccaaaccgat 350

gccaaatcca aaccccaacc accctagttc ctccggtagc ttttcagatg 400

ctgaccttgc ggatggcgtt tcaggtggag aaggaaaagg aggcagtgat 450

ggtggaggca gccacaggaa agaaggggaa gaggccgacg ccccaggcgt 500

gatccccggg attgtggggg ctgtcgtggt cgccgtggct ggagccatct 550

ctagcttcat tgcttaccag aaaaagaagc tatgcttcaa agaaaatgca 600

gaacaagggg aggtggacat ggagagccac cggaatgcca acgcagagcc 650

agctgttcag cgtactcttt tagagaaata gaagattgtc ggcagaaaca 700

gcccaggcgt tggcagcagg gttagaacag ctgcctgagg ctcctccctg 750

aaggacacct gcctgagagc agagatggag gccttctgtt cacggcggat 800

tctttgtttt aatcttgcga tgtgctttgc ttgttgctgg gcggatgatg 850

tttactaacg atgaatttta catccaaagg gggataggca cttggacccc 900

cattctccaa ggcccggggg ggcggtttcc catgggatgt gaaaggctgg 950

ccattattaa gtccctgtaa ctcaaatgtc aaccccaccg aggcaccccc 1000

ccgtccccca gaatcttggc tgtttacaaa tcacgtgtcc atcgagcacg 1050

tctgaaaccc ctggtagccc cgacttcttt ttaattaaaa taaggtaagc 1100

ccttcaattt 1110

<210> 4
<211> 604
<212> DNA
<213> Homo sapien

<400> 4
ccacgcgtcc gcgctgcgcc acatcccacc ggcccttaca ctgtggtgtc 50

cagcagcatc cggcttcatg gggggacttg aaccctgcag caggctcctg 100

ctcctgcctc tcctgctggc tgtaagtggt ctccgtcctg tccaggccca 150

ggcccagagc gattgcagtt gctctacggt gagcccgggc gtgctggcag 200

ggatcgtgat gggagacctg gtgctgacag tgctcattgc cctggccgtg 250

tacttcctgg gccggctggt ccctcggggg cgaggggctg cggaggcagc 300

gacccggaaa cagcgtatca ctgagaccga gtcgccttat caggagctcc 350

agggtcagag gtcggatgtc tacagcgacc tcaacacaca gaggccgtat 400

tacaaatgag cccgaatcat gacagtcagc aacatgatac ctggatccag 450

ccattcctga agcccaccct gcacctcatt ccaactccta ccgcgataca 500

gacccacaga gtgccatccc tgagagacca gaccgctccc caatactctc 550

ctaaaataaa catgaagcac aaaaaaaaaa aaaaaaaaaa aaaaaaaaaa 600

aaaa 604

<210> 5
<211> 669
<212> DNA
<213> Homo sapien

<220>
<221> Unsure
<222> 641
<223> Unknown base

<400> 5
ttcttttgga aaaccaaaca tgctttattt catttttttc acaatttatt 50

taaacatctc acatatacaa aataggtaca atttaatttt tctgcttgcc 100

caagaaacaa agcttctgtg gaaccatgga agaagatgaa aatgagactg 150

gcaaagaaca aatgctgaat ctgaagaaga ggacaacttt gggcaaataa 200

tctgcatact tttaattggg aataagatgg aaaatatgaa tgctaaatca 250

aatttttaa aaaatacacc acacgataca actcaataca ggagtatttc 300

ttctcaaatt cttctagcac catcaacatt cttcaagtat ctgaaatact 350

attaattagc acctttgtat tatgaacaaa acaaaacaag gacctcagtt 400

catctctgtc taggtcagca cctaacaatg tggatcacac tcatgggaaa 450

gtgttttgag gtagtttaaa cctttggaag tttgggtttt aaacttccct 500

ctgtggaaga tattcaaaag ccacaagtgg tgcaaatgtt tatggttttt 550

atttttcaat ttttattttg gttttcttac aaaggttgac atttttcata 600

acaggtgtaa gagtgttgaa aaaaaaattt caatttttgg ngggaacggg 650

ggaaggagtt aatgaaact 669

<210> 6
<211> 3636
<212> DNA
<213> Homo sapien

<400> 6

```
ggacaaaagg gtgaaagagg cctcccgggg ttacaaggtg tcattgggtt 50
tcctggaatg caaggacctg aggggccaca gggaccacca ggacaaaagg 100
gtgatactgg agaaccagga ctacctggaa caaaagggac aagaggacct 150
ccgggagcat ctggctaccc tggaaaccca ggacttcccg gaattcctgg 200
ccaagacggc ccgccaggcc ccccaggtat tccaggatgc aatggcacaa 250
agggggagag agggccgctc gggcctcctg gcttgcctgg tttcgcagga 300
aaccccggac caccaggctt accagggatg aagggtgatc caggtgagat 350
acttggccat gtgcccggga tgctgttgaa aggtgaaaga ggatttcccg 400
gaatcccagg gactccaggc ccaccaggac tgccagggct tcaaggtcct 450
gttgggcctc caggatttac cggaccacca ggtcccccag gccctcccgg 500
ccctccaggt gaaaagggac aaatgggctt aagttttcaa ggaccaaaag 550
gtgacaaggg tgaccaaggg gtcagtgggc ctccaggagt accaggacaa 600
gctcaagttc aagaaaaagg agacttcgcc accaagggag aaaagggcca 650
aaaaggtgaa cctggatttc aggggatgcc aggggtcgga gagaaaggtg 700
aacccggaaa accaggaccc agaggcaaac ccggaaaaga tggtgacaaa 750
ggggaaaaag ggagtcccgg ttttcctggt gaacccgggt acccaggact 800
cataggccgc cagggcccgc agggagaaaa gggtgaagca ggtcctcctg 850
gcccacctgg aattgttata ggcacaggac ctttgggaga aaaaggagag 900
aggggctacc ctggaactcc ggggccaaga ggagagccag gcccaaaagg 950
tttcccagga ctaccaggcc aacccggacc tccaggcctc cctgtacctg 1000
ggcaggctgg tgcccctggc ttccctggtg aaagaggaga aaaaggtgac 1050
cgaggatttc ctggtacatc tctgccagga ccaagtggaa gagatgggct 1100
cccgggtcct cctggttccc ctgggccccc tgggcagcct ggctacacaa 1150
atggaattgt ggaatgtcag cccggacctc caggtgacca gggtcctcct 1200
ggaattccag ggcagccagg atttataggc gaaattggag agaaaggtca 1250
aaaaggagag agttgcctca tctgtgatat agacggatat cgggggcctc 1300
ccgggccaca gggacccccg ggagaaatag gtttcccagg gcagccaggg 1350
gccaagggcg acagaggttt gcctggcaga gatggtgttg caggagtgcc 1400
aggccctcaa ggtacaccag ggctgatagg ccagccagga gccaaggggg 1450
agcctggtga gttttatttc gacttgcggc tcaaaggtga caaaggagac 1500
```

ccaggctttc caggacagcc cggcatgcca gggagagcgg gttctcctgg 1550
aagagatggc catccgggtc ttcctggccc caagggctcg ccgggttctg 1600
taggattgaa aggagagcgt ggcccccctg gaggagttgg attcccaggc 1650
agtcgtggtg acaccggccc ccctgggcct ccaggatatg gtcctgctgg 1700
tcccattggt gacaaaggac aagcaggctt tcctggaggc cctggatccc 1750
caggcctgcc aggtccaaag ggtgaaccag gaaaaattgt tcctttacca 1800
ggcccccctg gagcagaagg actgccgggg tccccaggct tcccaggtcc 1850
ccaaggagac cgaggctttc ccggaacccc aggaaggcca ggcctgccag 1900
gagagaaggg cgctgtgggc cagccaggca ttggatttcc agggcccccc 1950
ggccccaaag gtgttgacgg cttacctgga gacatggggc caccggggac 2000
tccaggtcgc ccgggattta atggcttacc tgggaaccca ggtgtgcagg 2050
gccagaaggg agagcctgga gttggtctac cgggactcaa aggtttgcca 2100
ggtcttcccg gcattcctgg cacacccggg gagaagggga gcattggggt 2150
accaggcgtt cctggagaac atggagcgat cggacccccct gggcttcagg 2200
ggatcagagg tgaaccggga cctcctggat tgccaggctc cgtggggtct 2250
ccaggagttc caggaatagg cccccctgga gctaggggtc cccctggagg 2300
acagggacca ccggggttgt caggccctcc tggaataaaa ggagagaagg 2350
gtttccccgg attccctgga ctggacatgc cgggccctaa aggagataaa 2400
ggggctcaag gactccctgg cataacggga cagtcggggc tccctggcct 2450
tcctggacag caggggggctc ctgggattcc tgggtttcca ggttccaagg 2500
gagaaatggg cgtcatgggg acccccgggc agccgggctc accaggacca 2550
tggggtgctc ctggattacc gggtgaaaaa ggggaccatg gctttccggg 2600
ctcctcagga cccaggggag accctggctt gaaaggtgat aagggggatg 2650
tcggtctccc tggcaagcct ggctccatgg ataaggtgga catgggcagc 2700
atgaagggcc agaaaggaga ccaaggagag aaaggacaaa ttggaccaat 2750
tggtgagaag ggatcccgag gagaccctgg gaccccagga gtgcctggaa 2800
aggacgggca ggcaggacag cctgggcagc caggacctaa aggtgatcca 2850
ggtataagtg gaaccccagg tgctccagga cttccgggac caaaaggatc 2900
tgttggtgga atgggcttgc caggaacacc tggagagaaa ggtgtgcctg 2950
gcatccctgg cccacaaggt tcacctggct tacctggaga caaaggtgca 3000

aaaggagaga aagggcaggc aggcccacct ggcataggca tcccagggct 3050

gcgaggtgaa aagggagatc aagggatagc gggtttccca ggaagccctg 3100

gagagaaggg agaaaaagga agcattggga tcccaggaat gccagggtcc 3150

ccaggcctta aagggtctcc cgggagtgtt ggctatccag gaagtcctgg 3200

gctacctgga gaaaaaggtg acaaaggcct cccaggattg gatggcatcc 3250

ctggtgtcaa aggagaagca ggtcttcctg ggactcctgg ccccacaggc 3300

ccagctggcc agaaagggga gccaggcagt gatggaatcc cggggtcagc 3350

aggagagaag ggtgaaccag gtctaccagg aagaggattc ccagggtttc 3400

caggggccaa aggagacaaa ggttcaaagg gtgaggtggg tttcccagga 3450

ttagccggga gcccaggaat tcctggatcc aaaggagagc aaggattcat 3500

gggtcctccg gggccccagg gacagccggg gttaccggga tccccaggcc 3550

atgcaacgga ggggcccaaa ggagaccgcg gacctcaggg ccagcctggc 3600

ctgccaggac ttccgggacc catggggcct ccaggg 3636

<210> 7
<211> 2212
<212> DNA
<213> Homo sapien

<400> 7
ggggaacgag gcccacctgg gagcccagga cttcaggggt tcccaggcat 50

cacacccccct tccaacatct ctggggcacc tggtgacaaa ggggcgccag 100

ggatatttgg cctgaaaggt tatcggggcc caccagggcc accaggttct 150

gctgctcttc ctggaagcaa aggtgacaca gggaacccag gagctccagg 200

aaccccaggg accaaaggat gggccgggga ctccgggccc cagggcaggc 250

ctggtgtgtt tggtctccca ggagaaaaag ggcccagggg tgaacaaggc 300

ttcatgggga acactggacc caccggggcg gtgggcgaca gaggccccaa 350

gggacccaag ggagacccag gattccctgg tgcccccggg actgtgggag 400

ccccccgggat tgcaggaatc ccccagaaga ttgccatcca accagggaca 450

gtgggtcccc aggggaggcg aggcccccct ggggcaccgg gggagatcgg 500

gccccagggc ccccccggag aaccaggttt tcgtggggct ccagggaaag 550

ctgggcccca aggaagaggt ggtgtgtctg ctgttcccgg cttccgggga 600

gatgaaggac ccataggcca ccaggggccg attggccaag aaggtgcacc 650

aggccgtcca gggagcccgg gcctgccggg tatgccaggc cgcagcgtca 700

gcatcggcta cctcctggtg aagcacagcc agacggacca ggagcccatg 750

tgcccggtgg gcatgaacaa actctggagt ggatacagcc tgctgtactt 800
cgagggccag gagaaggcgc acaaccagga cctggggctg gcgggctcct 850
gcctggcgcg gttcagcacc atgcccttcc tgtactgcaa ccctggtgat 900
gtctgctact atgccagccg gaacgacaag tcctactggc tctctaccac 950
tgcgccgctg cccatgatgc ccgtggccga ggacgagatc aagccctaca 1000
tcagccgctg ttctgtgtgt gaggccccgg ccatcgccat cgcggtccac 1050
agtcaggatg tctccatccc acactgccca gctgggtggc ggagtttgtg 1100
gatcggatat tccttcctca tgcacacggc ggcgggagac gaaggcggtg 1150
gccaatcact ggtgtcaccg ggcagctgtc tagaggactt ccgcgccaca 1200
ccattcatcg aatgcaatgg aggccgcggc acctgccact actacgccaa 1250
caagtacagc ttctggctga ccaccattcc cgagcagagc ttccagggct 1300
cgccctccgc cgacacgctc aaggccggcc tcatccgcac acacatcagc 1350
cgctgccagg tgtgcatgaa gaacctgtga gccggcgcgt gccaggaagg 1400
gccattttgg tgcttattct taacttatta cctcaggtgc caaccaaaaa 1450
ttggttttat ttttttctta aaaaaaaaaa aaagtctacc aaaggaattt 1500
gcatccagca gcagcactta gacctgccag ccactgtcac cgagcgggtg 1550
caagcactcg gggtccctgg aggcaagccc tgcccacaga aagccaggag 1600
cagccctggc ccccatcagc cctgctacga cgcaccgcct gaaggcacag 1650
ctaaccactt cgcacacacc catgtaacca ctgcactttc caatgccaca 1700
gacaactcac attgttcaac tccttctcgg ggtgggacag acgagacaac 1750
agcacacagg cagccagccg tggccagagg ctcgaggggc tcaggggctc 1800
aggcacccgt ccccacacga gggccccgtg ggtggcctgg ccctgctttc 1850
tacgccaatg ttatgccagc tccatgttct cccaaatacc gttgatgtga 1900
attattttaa aggcaaaact gtgctcttta ttttaaaaaa cactgataat 1950
cacactgcgg taggtcattc ttttgccaca tccctataga ccactgggtt 2000
tggcaaaact caggcagaag tggagacctt tctagacatc attgtcagcc 2050
ttgctacttg aaggtacacc ccatagggtc ggaggtgctg tccccactgc 2100
cccaccttgt ccctgagatt taacccctcc actgctgggg gtgagctgta 2150
ctcttctgac tgccccctcc tgtgtaacga ctacaaaata aaacttggtt 2200
ctgaatattt tt 2212

<210> 8
<211> 5510
<212> DNA
<213> Homo sapien

<400> 8
agccggccgt ggtggctccg tgcgtccgag cgtccgtccg cgccgtcggc 50

catggccaag cgctccaggg gccccgggcg ccgctgcctg ttggcgctcg 100

tgctgttctg cgcctggggg acgctggccg tggtggccca gaagccgggc 150

gcagggtgtc cgagccgctg cctgtgcttc cgcaccaccg tgcgctgcat 200

gcatctgctg ctggaggccg tgcccgccgt ggcgccgcag acctccatcc 250

tagatcttcg ctttaacaga atcagagaga tccaacctgg ggcattcagg 300

cggctgagga acttgaacac attgcttctc aataataatc agatcaagag 350

gatacctagt ggagcatttg aagacttgga aaatttaaaa tatctctatc 400

tgtacaagaa tgagatccag tcaattgaca ggcaagcatt taagggactt 450

gcctctctag agcaactata cctgcacttt aatcagatag aaactttgga 500

cccagattcg ttccagcatc tcccgaagct cgagaggcta tttttgcata 550

acaaccggat tacacattta gttccaggga catttaatca cttggaatct 600

atgaagagat tgcgactgga ctcaaacaca cttcactgcg actgtgaaat 650

cctgtggttg gcggatttgc tgaaaaccta cgcggagtcg gggaacgcgc 700

aggcagcggc catctgtgaa tatcccagac gcatccaggg acgctcagtg 750

gcaaccatca ccccggaaga gctgaactgt gaaaggcccc ggatcacctc 800

cgagccccag gacgcagatg tgacctcggg gaacaccgtg tacttcacct 850

gcagagccga aggcaacccc aagcctgaga tcatctggct gcgaaacaat 900

aatgagctga gcatgaagac agattcccgc ctaaacttgc tggacgatgg 950

gaccctgatg atccagaaca cacaggagac agaccagggt atctaccagt 1000

gcatggcaaa gaacgtggcc ggagaggtga agacgcaaga ggtgaccctc 1050

aggtacttcg ggtctccagc tcgacccact tttgtaatcc agccacagaa 1100

tacagaggtg ctggttgggg agagcgtcac gctggagtgc agcgccacag 1150

gccacccccc gccgcggatc tcctggacga gaggtgaccg cacacccttg 1200

ccagttgacc cgcgggtgaa catcacgcct tctggcgggc tttacataca 1250

gaacgtcgta caggggggaca gcggagagta tgcgtgctct gcgaccaaca 1300

acattgacag cgtccatgcc accgctttca tcatcgtcca ggctcttcct 1350

cagttcactg tgacgcctca ggacagagtc gttattgagg gccagaccgt 1400

ggatttccag tgtgaagcca agggcaaccc gccgcccgtc atcgcctgga 1450
ccaagggagg gagccagctc tccgtggacc ggcggcacct ggtcctgtca 1500
tcgggaacac ttagaatctc tggtgttgcc ctccacgacc agggccagta 1550
cgaatgccag gctgtcaaca tcatcggctc ccagaaggtc gtggcccacc 1600
tgactgtgca gcccagagtc accccagtgt ttgccagcat tcccagcgac 1650
acaacagtgg aggtgggcgc caatgtgcag ctcccgtgca gctcccaggg 1700
cgagcccgag ccagccatca cctggaacaa ggatggggtt caggtgacag 1750
aaagtggaaa atttcacatc agccctgaag gattcttgac catcaatgac 1800
gttggccctg cagacgcagg tcgctatgag tgtgtggccc ggaacaccat 1850
tgggtcggcc tcggtgagca tggtgctcag tgtgaacgtt cctgacgtca 1900
gtcgaaatgg agatccgttt gtagctacct ccatcgtgga agcgattgcg 1950
actgttgaca gagctataaa ctcaacccga acacatttgt ttgacagccg 2000
tcctcgttct ccaaatgatt tgctggcctt gttccggtat ccgagggatc 2050
cttacacagt tgaacaggca cgggcgggag aaatctttga acggacattg 2100
cagctcattc aggagcatgt acagcatggc ttgatggtcg acctcaacgg 2150
aacaagttac cactacaacg acctggtgtc tccacagtac ctgaacctca 2200
tcgcaaacct gtcgggctgt accgcccacc ggcgcgtgaa caactgctcg 2250
gacatgtgct tccaccagaa gtaccggacg cacgacggca cctgtaacaa 2300
cctgcagcac cccatgtggg gcgcctcgct gaccgccttc gagcgcctgc 2350
tgaaatccgt gtacgagaat ggcttcaaca cccctcgggg catcaacccc 2400
caccgactgt acaacgggca cgcccttccc atgccgcgcc tggtgtccac 2450
caccctgatc gggacggaga ccgtcacacc cgacgagcag ttcacccaca 2500
tgctgatgca gtggggccag ttcctggacc acgacctcga ctccacggtg 2550
gtggccctga gccaggcacg cttctccgac ggacagcact gcagcaacgt 2600
gtgcagcaac gacccccccct gcttctctgt catgatcccc cccaatgact 2650
cccgggccag gagcggggcc cgctgcatgt tcttcgtgcg ctccagccct 2700
gtgtgcggca gcggcatgac ttcgctgctc atgaactccg tgtacccgcg 2750
ggagcagatc aaccagctca cctcctacat cgacgcatcc aacgtgtacg 2800
ggagcacgga gcatgaggcc cgcagcatcc gcgacctggc cagccaccgc 2850
ggcctgctgc ggcagggcat cgtgcagcgg tccgggaagc cgctgctccc 2900

cttcgccacc gggccgccca cggagtgcat gcgggacgag aacgagagcc 2950
ccatcccctg cttcctggcc ggggaccacc gcgccaacga gcagctgggc 3000
ctgaccagca tgcacacgct gtggttccgc gagcacaacc gcattgccac 3050
ggagctgctc aagctgaacc cgcactggga cggcgacacc atctactatg 3100
agaccaggaa gatcgtgggt gcggagatcc agcacatcac ctaccagcac 3150
tggctcccga agatcctggg ggaggtgggc atgaggacgc tgggagagta 3200
ccacggctac gaccccggca tcaatgctgg catcttcaac gccttcgcca 3250
ccgcggcctt caggtttggc cacacgcttg tcaacccact gctttaccgg 3300
ctggacgaga acttccagcc cattgcacaa gatcacctcc cccttcacaa 3350
agctttcttc tctcccttcc ggattgtgaa tgagggcggc atcgatccgc 3400
ttctcagggg gctgttcggg gtggcgggga aaatgcgtgt gccctcgcag 3450
ctgctgaaca cggagctcac ggagcggctg ttctccatgg cacacacggt 3500
ggctctggac ctggcggcca tcaacatcca gcggggccgg gaccacggga 3550
tcccacccta ccacgactac agggtctact gcaatctatc ggcggcacac 3600
acgttcgagg acctgaaaaa tgagattaaa aaccctgaga tccgggagaa 3650
actgaaaagg ttgtatggct cgacactcaa catcgacctg tttccggcgc 3700
tcgtggtgga ggacctggtg cctggcagcc ggctgggccc caccctgatg 3750
tgtcttctca gcacacagtt caagcgcctg cgagatgggg acaggttgtg 3800
gtatgagaac cctggggtgt tctccccggc ccagctgact cagatcaagc 3850
agacgtcgct ggccaggatc ctatgcgaca acgcggacaa catcacccgg 3900
gtgcagagcg acgtgttcag ggtggcggag ttccctcacg gctacggcag 3950
ctgtgacgag atccccaggg tggacctccg ggtgtggcag gactgctgtg 4000
aagactgtag gaccaggggg cagttcaatg ccttttccta tcatttccga 4050
ggcagacggt ctcttgagtt cagctaccag gaggacaagc cgaccaagaa 4100
aacaagacca cggaaaatac ccagtgttgg gagacagggg gaacatctca 4150
gcaacagcac ctcagccttc agcacacgct cagatgcatc tgggacaaat 4200
gacttcagag agtttgttct ggaaatgcag aagaccatca cagacctcag 4250
aacacagata aagaaacttg aatcacggct cagtaccaca gagtgcgtgg 4300
atgccggggg cgaatctcac gccaacaaca ccaagtggaa aaaagatgca 4350
tgcaccattt gtgaatgcaa agacgggcag gtcacctgct tcgtggaagc 4400
ttgcccccct gccacctgtg ctgtccccgt gaacatccca ggggcctgct 4450

gtccagtctg cttacagaag agggcggagg aaaagcccta ggctcctggg 4500

aggctcctca gagtttgtct gctgtgccat cgtgagatcg ggtggccgat 4550

ggcagggagc tgcggactgc agaccaggaa acacccagaa ctcgtgacat 4600

ttcatgacaa cgtccagctg gtgctgttac agaaggcagt gcaggaggct 4650

tccaaccaga gcatctgcgg agaaggaggc acagcaggtg cctgaaggga 4700

agcaggcagg agtcctagct tcacgttaga cttctcaggt ttttatttaa 4750

ttcttttaaa atgaaaaatt ggtgctacta ttaaattgca cagttgaatc 4800

atttaggcgc ctaaattggt tttgcctccc aacaccattt ctttttaaat 4850

aaagcaggat acctctatat gtcagccttg ccttgttcag atgccaggag 4900

ccggcagacc tgtcacccgc aggtggggtg agtctcggag ctgccagagg 4950

ggctcaccga aatcggggtt ccatcacaag ctatgtttaa aaagaaaatt 5000

ggtgtttggc aaacggaaca gaacctttga tgagagcgtt cacagggaca 5050

ctgtctgggg gtgcagtgca agcccccggc ctcttccctg ggaacctctg 5100

aactcctcct tcctctgggc tctctgtaac atttcaccac acgtcagcat 5150

ctaatcccaa gacaaacatt cccgctgctc gaagcagctg tatagcctgt 5200

gactctccgt gtgtcagctc cttccacacc tgattagaac attcataagc 5250

cacatttaga aacagatttg ctttcagctg tcacttgcac acatactgcc 5300

tagttgtgaa ccaaatgtga aaaaacctcc ttcatcccat tgtgtatctg 5350

atacctgccg agggccaagg gtgtgtgttg acaacgccgc tcccagccgg 5400

ccctggttgc gtccacgtcc tgaacaagag ccgcttccgg atggctcttc 5450

ccaagggagg aggagctcaa gtgtcgggaa ctgtctaact tcaggttgtg 5500

tgagtgcgtt 5510

<210> 9
<211> 10478
<212> DNA
<213> Homo sapien

<220>
<221> Unsure
<222> 6765
<223> Unknown base

<400> 9
caaacatgtc agctgttact ggaagtggcc tggcctctat ttatcttcct 50

gatcctgatc tctgttcggc tgagctaccc accctatgaa caacatgaat 100

gccattttcc aaataaagcc atgccctctg caggaacact tccttgggtt 150

caggggatta tctgtaatgc caacaacccc tgtttccgtt acccgactcc 200
tggggaggct cccggagttg ttggaaactt taacaaatcc attgtggctc 250
gcctgttctc agatgctcgg aggcttcttt tatacagcca gaaagacacc 300
agcatgaagg acatgcgcaa agttctgaga acattacagc agatcaagaa 350
atccagctca aacttgaagc ttcaagattt cctggtggac aatgaaacct 400
tctctgggtt cctgtatcac aacctctctc tcccaaagtc tactgtggac 450
aagatgctga gggctgatgt cattctccac aaggtatttt tgcaaggcta 500
ccagttacat ttgacaagtc tgtgcaatgg atcaaaatca gaagagatga 550
ttcaacttgg tgaccaagaa gtttctgagc tttgtggcct accaagggag 600
aaactggctg cagcagagcg agtacttcgt tccaacatgg acatcctgaa 650
gccaatcctg agaacactaa actctacatc tcccttcccg agcaaggagc 700
tggccgaagc cacaaaaaca ttgctgcata gtcttgggac tctggcccag 750
gagctgttca gcatgagaag ctggagtgac atgcgacagg aggtgatgtt 800
tctgaccaat gtgaacagct ccagctcctc cacccaaatc taccaggctg 850
tgtctcgtat tgtctgcggg catcccgagg gaggggggct gaagatcaag 900
tctctcaact ggtatgagga caacaactac aaagccctct ttggaggcaa 950
tggcactgag gaagatgctg aaaccttcta tgacaactct acaactcctt 1000
actgcaatga tttgatgaag aatttggagt ctagtcctct ttcccgcatt 1050
atctggaaag ctctgaagcc gctgctcgtt gggaagatcc tgtatacacc 1100
tgacactcca gccacaaggc aggtcatggc tgaggtgaac aagaccttcc 1150
aggaactggc tgtgttccat gatctggaag gcatgtggga ggaactcagc 1200
cccaagatct ggaccttcat ggagaacagc caagaaatgg accttgtccg 1250
gatgctgttg gacagcaggg acaatgacca cttttgggaa cagcagttgg 1300
atggcttaga ttggacagcc caagacatcg tggcgttttt ggccaagcac 1350
ccagaggatg tccagtccag taatggttct gtgtacacct ggagagaagc 1400
tttcaacgag actaaccagg caatccggac catatctcgc ttcatggagt 1450
gtgtcaacct gaacaagcta gaacccatag caacagaagt ctggctcatc 1500
aacaagtcca tggagctgct ggatgagagg aagttctggg ctggtattgt 1550
gttcactgga attactccag gcagcattga gctgccccat catgtcaagt 1600
acaagatccg aatggacatt gacaatgtgg agaggacaaa taaaatcaag 1650

gatgggtact gggaccctgg tcctcgagct gacccctttg aggacatgcg 1700
gtacgtctgg gggggcttcg cctacttgca ggatgtggtg gagcaggcaa 1750
tcatcagggt gctgacgggc accgagaaga aaactggtgt ctatatgcaa 1800
cagatgccct atccctgtta cgttgatgac atctttctgc gggtgatgag 1850
ccggtcaatg cccctcttca tgacgctggc ctggatttac tcagtggctg 1900
tgatcatcaa gggcatcgtg tatgagaagg aggcacggct gaaagagacc 1950
atgcggatca tgggcctgga caacagcatc ctctggttta gctggttcat 2000
tagtagcctc attcctcttc ttgtgagcgc tggcctgcta gtggtcatcc 2050
tgaagttagg aaacctgctg ccctacagtg atcccagcgt ggtgtttgtc 2100
ttcctgtccg tgtttgctgt ggtgacaatc ctgcagtgct tcctgattag 2150
cacactcttc tccagagcca acctggcagc agcctgtggg ggcatcatct 2200
acttcacgct gtacctgccc tacgtcctgt gtgtggcatg gcaggactac 2250
gtgggcttca cactcaagat cttcgctagc ctgctgtctc ctgtggcttt 2300
tgggtttggc tgtgagtact ttgccctttt tgaggagcag ggcattggag 2350
tgcagtggga caacctgttt gagagtcctg tggaggaaga tggcttcaat 2400
ctcaccactt cggtctccat gatgctgttt gacaccttcc tctatggggt 2450
gatgacctgg tacattgagg ctgtctttcc aggccagtac ggaattccca 2500
ggccctggta ttttccttgc accaagtcct actggtttgg cgaggaaagt 2550
gatgagaaga gccaccctgg ttccaaccag aagagaatat cagaaatctg 2600
catggaggag gaacccaccc acttgaagct gggcgtgtcc attcagaacc 2650
tggtaaaagt ctaccgagat gggatgaagg tggctgtcga tggcctggca 2700
ctgaattttt atgagggcca gatcacctcc ttcctgggcc acaatggagc 2750
ggggaagacg accaccatgt caatcctgac cgggttgttc cccccgacct 2800
cgggcaccgc ctacatcctg ggaaaagaca ttcgctctga gatgagcacc 2850
atccggcaga acctgggggt ctgtccccag cataacgtgc tgtttgacat 2900
gctgactgtc gaagaacaca tctggttcta tgcccgcttg aaagggctct 2950
ctgagaagca cgtgaaggcg gagatggagc agatggccct ggatgttggt 3000
ttgccatcaa gcaagctgaa aagcaaaaca agccagctgt caggtggaat 3050
gcagagaaag ctatctgtgg ccttggcctt tgtcggggga tctaaggttg 3100
tcattctgga tgaacccaca gctggtgtgg acccttactc ccgcagggga 3150
atatgggagc tgctgctgaa ataccgacaa ggccgcacca ttattctctc 3200

tacacaccac atggatgaag cggacgtcct gggggacagg attgccatca 3250
tctcccatgg gaagctgtgc tgtgtgggct cctccctgtt tctgaagaac 3300
cagctgggaa caggctacta cctgaccttg gtcaagaaag atgtggaatc 3350
ctccctcagt tcctgcagaa acagtagtag cactgtgtca tacctgaaaa 3400
aggaggacag tgtttctcag agcagttctg atgctggcct gggcagcgac 3450
catgagagtg acacgctgac catcgatgtc tctgctatct ccaacctcat 3500
caggaagcat gtgtctgaag cccggctggt ggaagacata gggcatgagc 3550
tgacctatgt gctgccatat gaagctgcta aggagggagc ctttgtggaa 3600
ctctttcatg agattgatga ccggctctca gacctgggca tttctagtta 3650
tggcatctca gagacgaccc tggaagaaat attcctcaag gtggccgaag 3700
agagtggggt ggatgctgag acctcagatg gtaccttgcc agcaagacga 3750
aacaggcggg ccttcgggga caagcagagc tgtcttcgcc cgttcactga 3800
agatgatgct gctgatccaa atgattctga catagaccca gaatccagag 3850
agacagactt gctcagtggg atggatggca aagggtccta ccaggtgaaa 3900
ggctggaaac ttacacagca acagtttgtg gcccttttgt ggaagagact 3950
gctaattgcc agacggagtc ggaaaggatt ttttgctcag attgtcttgc 4000
cagctgtgtt tgtctgcatt gcccttgtgt tcagcctgat cgtgccaccc 4050
tttggcaagt accccagcct ggaacttcag ccctggatgt acaacgaaca 4100
gtacacattt gtcagcaatg atgctcctga ggacacggga accctggaac 4150
tcttaaacgc cctcaccaaa gaccctggct tcgggacccg ctgtatggaa 4200
ggaaacccaa tcccagacac gccctgccag gcaggggagg aagagtggac 4250
cactgcccca gttccccaga ccatcatgga cctcttccag aatgggaact 4300
ggacaatgca gaacccttca cctgcatgcc agtgtagcag cgacaaaatc 4350
aagaagatgc tgcctgtgtg tcccccaggg gcaggggggc tgcctcctcc 4400
acaaagaaaa caaaacactg cagatatcct tcaggacctg acaggaagaa 4450
acatttcgga ttatctggtg aagacgtatg tgcagatcat agccaaaagc 4500
ttaaagaaca agatctgggt gaatgagttt aggtatggcg gcttttccct 4550
gggtgtcagt aatactcaag cacttcctcc gagtcaagaa gttaatgatg 4600
ccaccaaaca aatgaagaaa cacctaaagc tggccaagga cagttctgca 4650
gatcgatttc tcaacagctt gggaagattt atgacaggac tggacaccag 4700

aaataatgtc aaggtgtggt tcaataacaa gggctggcat gcaatcagct 4750
ctttcctgaa tgtcatcaac aatgccattc tccgggccaa cctgcaaaag 4800
ggagagaacc ctagccatta tggaattact gctttcaatc atcccctgaa 4850
tctcaccaag cagcagctct cagaggtggc tccgatgacc acatcagtgg 4900
atgtccttgt gtccatctgt gtcatctttg caatgtcctt cgtcccagcc 4950
agctttgtcg tattcctgat ccaggagcgg gtcagcaaag caaaacacct 5000
gcagttcatc agtggagtga agcctgtcat ctactggctc tctaattttg 5050
tctgggatat gtgcaattac gttgtccctg ccacactggt cattatcatc 5100
ttcatctgct tccagcagaa gtcctatgtg tcctccacca atctgcctgt 5150
gctagccctt ctacttttgc tgtatgggtg gtcaatcaca cctctcatgt 5200
acccagcctc ctttgtgttc aagatcccca gcacagccta tgtggtgctc 5250
accagcgtga acctcttcat tggcattaat ggcagcgtgg ccacctttgt 5300
gctggagctg ttcaccgaca ataagctgaa taatatcaat gatatcctga 5350
agtccgtgtt cttgatcttc ccacattttt gcctgggacg agggctcatc 5400
gacatggtga aaaaccaggc aatggctgat gccctggaaa ggtttgggga 5450
gaatcgcttt gtgtcaccat tatcttggga cttggtggga cgaaacctct 5500
tcgccatggc cgtggaaggg gtggtgttct tcctcattac tgttctgatc 5550
cagtacagat tcttcatcag gcccagacct gtaaatgcaa agctatctcc 5600
tctgaatgat gaagatgaag atgtgaggcg ggaaagacag agaattcttg 5650
atggtggagg ccagaatgac atcttagaaa tcaaggagtt gacgaagata 5700
tatagaagga agcggaagcc tgctgttgac aggatttgcg tgggcattcc 5750
tcctggtgag tgctttgggc tcctgggagt taatggggct ggaaaatcat 5800
caactttcaa gatgttaaca ggagatacca ctgttaccag aggagatgct 5850
ttccttaaca gaaatagtat cttatcaaac atccatgaag tacatcagaa 5900
catgggctac tgccctcagt ttgatgccat cacagagctg ttgactggga 5950
gagaacacgt ggagttcttt gcccttttga gaggagtccc agagaaagaa 6000
gttggcaagg ttggtgagtg ggcgattcgg aaactgggcc tcgtgaagta 6050
tggagaaaaa tatgctggta actatagtgg aggcaacaaa cgcaagctct 6100
ctacagccat ggctttgatc ggcgggcctc ctgtggtgtt tctggatgaa 6150
cccaccacag gcatggatcc caaagcccgg cggttcttgt ggaattgtgc 6200
cctaagtgtt gtcaaggagg ggagatcagt agtgcttaca tctcatagta 6250

tggaagaatg tgaagctctt tgcactagga tggcaatcat ggtcaatgga 6300
aggttcaggt gccttggcag tgtccagcat ctaaaaaata ggtttggaga 6350
tggttataca atagttgtac gaatagcagg gtccaacccg gacctgaagc 6400
ctgtccagga tttctttgga cttgcatttc ctggaagtgt tccaaaagag 6450
aaacaccgga acatgctaca ataccagctt ccatcttcat tatcttctct 6500
ggccaggata ttcagcatcc tctcccagag caaaaagcga ctccacatag 6550
aagactactc tgtttctcag acaacacttg accaagtatt tgtgaacttt 6600
gccaaggacc aaagtgatga tgaccactta aaagacctct cattacacaa 6650
aaaccagaca gtagtggacg ttgcagttct cacatctttt ctacaggatg 6700
agaaagtgaa agaaagctat gtatgaagaa tcctgttcat acggggtggc 6750
tgaaagtaaa gaggnactag actttccttt gcaccatgtg aagtgttgtg 6800
gagaaaagag ccagaagttg atgtgggaag aagtaaactg gatactgtac 6850
tgatactatt caatgcaatg caattcaatg caatgaaaac aaaattccat 6900
tacaggggca gtgcctttgt agcctatgtc ttgtatggct ctcaagtgaa 6950
agacttgaat ttagtttttt acctatacct atgtgaaact ctattatgga 7000
acccaatgga catatgggtt tgaactcaca cttttttttt ttttttgttc 7050
ctgtgtattc tcattggggt tgcaacaata attcatcaag taatcatggc 7100
cagcgattat tgatcaaaat caaaaggtaa tgcacatcct cattcactaa 7150
gccatgccat gcccaggaga ctggtttccc ggtgacacat ccattgctgg 7200
caatgagtgt gccagagtta ttagtgccaa gtttttcaga aagtttgaag 7250
caccatggtg tgtcatgctc acttttgtga aagctgctct gctcagagtc 7300
tatcaacatt gaatatcagt tgacagaatg gtgccatgcg tggctaacat 7350
cctgctttga ttccctctga taagctgttc tggtggcagt aacatgcaac 7400
aaaaatgtgg gtgtctctag gcacgggaaa cttggttcca ttgttatatt 7450
gtcctatgct tcgagccatg ggtctacagg gtcatcctta tgagactctt 7500
aaatatactt agatcctggt aagaggcaaa gaatcaacag ccaaactgct 7550
ggggctgcaa gctgctgaag ccagggcatg ggattaaaga gattgtgcgt 7600
tcaaacctag ggaagcctgt gcccatttgt cctgactgtc tgctaacatg 7650
gtacactgca tctcaagatg tttatctgac acaagtgtat tatttctggc 7700
tttttgaatt aatctagaaa atgaaaagat ggagttgtat tttgacaaaa 7750

atgtttgtac tttttaatgt tatttggaat tttaagttct atcagtgact 7800
tctgaatcct tagaatggcc tctttgtaga accctgtggt atagaggagt 7850
atggccactg ccccactatt tttattttct tatgtaagtt tgcatatcag 7900
tcatgactag tgcctagaaa gcaatgtgat ggtcaggatc tcatgacatt 7950
atatttgagt ttctttcaga tcatttagga tactcttaat ctcacttcat 8000
caatcaaata ttttttgagt gtatgctgta gctgaaagag tatgtacgta 8050
cgtataagac tagagagata ttaagtctca gtacacttcc tgtgccatgt 8100
tattcagctc actggtttac aaatataggt tgtcttgtgg ttgtaggagc 8150
ccactgtaac aatactgggc agcctttttt ttttttttta attgcaacaa 8200
tgcaaaagcc aagaaagtat aagggtcaca agtctaaaca atgaattctt 8250
caacagggaa aacagctagc ttgaaaactt gctgaaaaac acaacttgtg 8300
tttatggcat ttagtacctt caaataattg gctttgcaga tattggatac 8350
cccattaaat ctgacagtct caaatttttc atctcttcaa tcactagtca 8400
agaaaaatat aaaaacaaca aatacttcca tatggagcat ttttcagagt 8450
tttctaaccc agtcttattt ttctagtcag taaacatttg taaaaatact 8500
gtttcactaa tacttactgt taactgtctt gagagaaaag aaaaatatga 8550
gagaactatt gtttggggaa gttcaagtga tctttcaata tcattactaa 8600
cttcttccac tttttccaaa atttgaatat taacgctaaa ggtgtaagac 8650
ttcagatttc aaattaatct ttctatattt tttaaattta cagaatatta 8700
tataacccac tgctgaaaaa gaaaaaaatg attgttttag aagttaaagt 8750
caatattgat tttaaatata agtaatgaag gcatatttcc aataactagt 8800
gatatggcat cgttgcattt tacagtatct tcaaaaatac agaatttata 8850
gaataatttc tcctcattta atatttttca aaatcaaagt tatggtttcc 8900
tcattttact aaaatcgtat tctaattctt cattatagta aatctatgag 8950
caactcctta cttcggttcc tctgatttca aggccatatt ttaaaaaatc 9000
aaaaggcact gtgaactatt ttgaagaaaa cacaacattt taatacagat 9050
tgaaaggacc tcttctgaag ctagaaacaa tctatagtta tacatcttca 9100
ttaatactgt gttacctttt aaaatagtaa ttttttacat tttcctgtgt 9150
aaacctaatt gtggtagaaa tttttaccaa ctctatactc aatcaagcaa 9200
aatttctgta tattccctgt ggaatgtacc tatgtgagtt tcagaaattc 9250
tcaaaatacg tgttcaaaaa tttctgcttt tgcatctttg ggacacctca 9300

gaaaacttat taacaactgt gaatatgaga aatacagaag aaaataataa 9350

gccctctata cataaatgcc cagcacaatt cattgttaaa aaacaaccaa 9400

acctcacact actgtatttc attatctgta ctgaaagcaa atgctttgtg 9450

actattaaat gttgcacatc attcattcac tgtatagtaa tcattgacta 9500

aagccatttg tctgtgtttt cttcttgtgg ttgtatatat caggtaaaat 9550

attttccaaa gagccatgtg tcatgtaata ctgaaccact ttgatattga 9600

gacattaatt tgtacccttg ttattatcta ctagtaataa tgtaatactg 9650

tagaaatatt gctctaattc ttttcaaaat tgttgcatcc cccttagaat 9700

gtttctattt ccataaggat ttaggtatgc tattatccct tcttataccc 9750

taagatgaag ctgtttttgt gctctttgtt catcattggc cctcattcca 9800

agcactttac gctgtctgta atgggatcta tttttgcact ggaatatctg 9850

agaattgcaa aactagacaa aagtttcaca acagatttct aagttaaatc 9900

attttcatta aaaggaaaaa agaaaaaaaa ttttgtatgt caataacttt 9950

atatgaagta ttaaaatgca tatttctatg ttgtaatata atgagtcaca 10000

aaataaagct gtgacagttc tgttggtcta cagaaattta cttttgtgca 10050

tttgtggcac cacctactgt tgaagggtta taaagccatt agaaaagtag 10100

aggggaagtg atttggatca aaaggaaaaa ctttagaaaa gattcagatg 10150

ttcccttaat cataaaagag aactgagggg actacttgaa aataaaaggt 10200

tgttttgtat tttcatgttg gttaagatac tgagtaactg gtattaagtg 10250

ttagaggttt ttagataaat attctgctta atgattatga agctgcactg 10300

agatttctga aaatgctctg tagctgagct tatttaataa atgttcactt 10350

ggtatagggg aagctacaaa ggcagccttc agtgtccttt tgtttattca 10400

accaaaaata taaggacaca atgtagcagt tatactggga aggtgctggg 10450

ggtggtggca atggtgagca ggaaggcg 10478

<210> 10
<211> 1793
<212> DNA
<213> Homo sapien

<400> 10
cagaccccga ccccgacccg gaccccgagc ctgccggcgg ctcccgtccc 50

ggccccgcgg tccccgggct ccgcgccctg ctgccggcgc gggctttcct 100

ctgctctctc aaaggccgcc tcctgctggc cgagtcgggt ctctcattca 150

tcacttttat ctgctatgtg gcgtcctcag catctgcctt cctcacagcg 200
cctctgctgg agttcctgct ggccttgtac ttcctctttg ctgatgccat 250
gcagctgaat gacaagtggc agggcttgtg ctggcccatg atggacttcc 300
tgcgctgtgt caccgcggcc ctcatctact ttgctatctc catcacggcc 350
atcgccaagt actcggatgg ggcttccaaa gccgctgggg tgtttggctt 400
ctttgctacc atcgtgtttg caactgattt ctacctgatc tttaacgacg 450
tggccaaatt cctcaaacaa ggggactctg cagatgagac cacagcccac 500
aagacagaag aagagaattc cgactcggac tctgactgaa ggcctggcgg 550
gtgccttggc aacctgagcc acacaggcct ccacccctgt gcctcacagg 600
ggtcgctggc gttggagcgg aggcctggac ttctgagttg cagagggggc 650
tgcggacaca gcaggccccc tacagcctca ggttctgcct gagcccagcc 700
taccaggctt gcccctcagc tcagcactgt tgaccacgct gcgtatgagg 750
gcatcttggg tatcccactc cttctcccca tttctgtccc acaggccttc 800
agccctttaa cgtctctgcc aaaaaccagc acaaggagac aaagcagagc 850
cttgtctgta tctgggcagc aggtgttcca tgctgctagg tggcgggggt 900
cgggggtctt ctgtttcact aacaggaaca aagacagaaa ccatgacagg 950
gctgccccgc caggccccgg tgggtttgtc tgcacttggt gctcctgccc 1000
acaccagcca ctttggtgac aatgaccctt ccaagaatct ttggttcaag 1050
gagcaccagt tccctcttca ttcttgaagc agggagaaat tgacctttgc 1100
cttgtcgccc aggaagtggg gctcggcacc cataactaac acctcccacc 1150
cttggaaacc atgtcttctg gggtgagat gaccattctg ggtctaagac 1200
tgtttcaaag aagagctcat agactgactg gtccagaaga cagagggtac 1250
aacagtggca tcacagtgac agtgtcatgg ggagctgggc gggcccagcc 1300
aaaccctcct tcttcctaga gcccagccag caggcaggag ttcctggacc 1350
ctcaggacag tgaacttcca gacctcaggg caggtctatg ggccactgca 1400
ggagatgaga ccagccttct gtgttcacct aacgatttat actgtgtatc 1450
tgtctttgat ggaattttgt aactttttat attttttat gcaaaagcag 1500
cttcttaaca gatggcattt tctgtgactc taggcctcac aaaagagcca 1550
gagttctgga cccatgtttg gagcatttgt agccttattc tcttgcgtgt 1600
gaatctctta ccctgaaaaa aagccataat gaattaagcc agactgacca 1650
cttgcttgga gtgtgtgctt gaaaaaacca gagcaatact gttgggtatt 1700

gtatcaggct tcagtacaaa ctggtaacac caatgtggat cctgacagct 1750

ttcagtttta gcaaaaatac acgtgaaatc tgaaaaaaaa aaa 1793

<210> 11
<211> 939
<212> DNA
<213> Homo sapien

<400> 11
tcggccgaga tgtctcgctc cgtggcctta gctgtgctcg cgctactctc 50

tctttctggc ctggaggcta tccagcgtac tccaaagatt caggtttact 100

cacgtcatcc agcagagaat ggaaagtcaa atttcctgaa ttgctatgtg 150

tctgggtttc atccatccga cattgaagtt gacttactga agaatggaga 200

gagaattgaa aaagtggagc attcagactt gtctttcagc aaggactggt 250

ctttctatct cttgtactac actgaattca cccccactga aaaagatgag 300

tatgcctgcc gtgtgaacca tgtgactttg tcacagccca agatagttaa 350

gtgggatcga gacatgtaag cagcatcatg gaggtttgaa gatgccgcat 400

ttggattgga tgaattccaa attctgcttg cttgcttttt aatattgata 450

tgcttataca cttacacttt atgcacaaaa tgtagggtta taataatgtt 500

aacatggaca tgatcttctt tataattcta ctttgagtgc tgtctccatg 550

tttgatgtat ctgagcaggt tgctccacag gtagctctag gagggctggc 600

aacttagagg tggggagcag agaattctct tatccaacat caacatcttg 650

gtcagatttg aactcttcaa tctcttgcac tcaaagcttg ttaagatagt 700

taagcgtgca taagttaact tccaatttac atactctgct tagaatttgg 750

gggaaaattt agaaatataa ttgacaggat tattggaaat ttgttataat 800

gaatgaaaca ttttgtcata taagattcat atttacttct tatacatttg 850

ataaagtaag gcatggttgt ggttaatctg gtttattttt gttccacaag 900

ttaaataaat cataaaactt gaaaaaaaaa aaaaaaaaa 939

<210> 12
<211> 2443
<212> DNA
<213> Homo sapien

<400> 12
agctggctca gggcgtccgc taggctcgga cgacctgctg agcctcccaa 50

accgcttcca taaggctttg ctttccaact tcagctacag tgttagctaa 100

gtttggaaag aaggaaaaaa gaaaatccct gggccccttt tcttttgttc 150

tttgccaaag tcgtcgttgt agtctttttg cccaaggctg ttgtgttttt 200
agaggtgcta tctccagttc cttgcactcc tgttaacaag cacctcagcg 250
agagcagcag cagcgatagc agccgcagaa gagccagcgg ggtcgcctag 300
tgtcatgacc agggcgggag atcacaaccg ccagagagga tgctgtggat 350
ccttggccga ctacctgacc tctgcaaaat tccttctcta ccttggtcat 400
tctctctcta cttggggaga tcggatgtgg cactttgcgg tgtctgtgtt 450
tctggtagag ctctatggaa acagcctcct tttgacagca gtctacgggc 500
tggtggtggc agggtctgtt ctggtcctgg gagccatcat cggtgactgg 550
gtggacaaga atgctagact taaagtggcc cagacctcgc tggtggtaca 600
gaatgtttca gtcatcctgt gtggaatcat cctgatgatg gttttcttac 650
ataaacatga rcttctgacc atgtaccatg gatgggttct cacttcctgc 700
tatatcctga tcatcactat tgcaaatatt gcaaatttgg ccagtactgc 750
tactgcaatc acaatccaaa gggattggat tgttgttgtt gcaggagaag 800
acagaagcaa actagcaaat atgaatgcca caatacgaag gattgaccag 850
ttaaccaaca tcttagcccc catggctgtt ggccagatta tgacatttgg 900
ctccccagtc atcggctgtg gctttatttc gggatggaac ttggtatcca 950
tgtgcgtgga gtacgtcctg ctctggaagg tttaccagaa aaccccagct 1000
ctagctgtga aagctggtct taaagaagag gaaactgaat tgaaacagct 1050
gaatttacac aaagatactg agccaaaacc cctggaggga actcatctaa 1100
tgggtgtgaa ggactctaac atccatgagc ttgaacatga gcaagagcct 1150
acttgtgcct cccagatggc tgagcccttc cgtaccttcc gagatggatg 1200
ggtctcctac tacaaccagc ctgtgtttct ggctggcatg ggtcttgctt 1250
tcctttatat gactgtcctg ggctttgact gcatcaccac agggtacgcc 1300
tacactcagg gactgagtgg ttccatcctc agtattttga tgggagcatc 1350
agctataact ggaataatgg gaactgtagc ttttacttgg ctacgtcgaa 1400
aatgtggttt ggttcggaca ggtctgatct caggattggc acagctttcc 1450
tgtttgatct tgtgtgtgat ctctgtattc atgcctggaa gcccccctgga 1500
cttgtccgtt tctccttttg aagatatccg atcaaggttc attcaaggag 1550
agtcaattac acctaccaag atacctgaaa ttacaactga aatatacatg 1600
tctaatgggt ctaattctgc taatattgtc ccggagacaa gtcctgaatc 1650
tgtgcccata atctctgtca gtctgctgtt tgcaggcgtc attgctgcta 1700

gaatcggtct ttggtccttt gatttaactg tgacacagtt gctgcaagaa 1750

aatgtaattg aatctgaaag aggcattata aatggtgtac agaactccat 1800

gaactatctt cttgatcttc tgcatttcat catggtcatc ctggctccaa 1850

atcctgaagc ttttggcttg ctcgtattga tttcagtctc ctttgtggca 1900

atgggccaca ttatgtattt ccgatttgcc caaaatactc tgggaaacaa 1950

gctctttgct tgcggtcctg atgcaaaaga agttaggaag gaaaatcaag 2000

caaatacatc tgttgtttga gacagtttaa ctgttgctat cctgttacta 2050

gattatatag agcacatgtg cttattttgt actgcagaat tccaataaat 2100

ggctgggtgt tttgctctgt ttttaccaca gctgtgcctt gagaactaaa 2150

agctgtttag gaaacctaag tcagcagaaa ttaactgatt aatttccctt 2200

atgttgaggc atggraaaaa aattggraaa aggaaaaact cagttttaaa 2250

tacgggagac tataatggat aacactgrat tcccctattt ctcatgagta 2300

gatacaatct tacgtaaaag agtggttagt cacgtgaatt cagttatcat 2350

ttgacagatt cttatctgta ctagaattca gatatgtcag ttttctgcaa 2400

aactcactct tgttcaagac tagctaattt atttttttgc atc 2443

<210> 13
<211> 2232
<212> DNA
<213> Homo sapien

<400> 13
cttccccttc tctgccctgc tccaggcacc aggctctttc cccttcagtg 50

tctcagagga ggggacggca gcaccatgga cccccgcttg tccactgtcc 100

gccagacctg ctgctgcttc aatgtccgca tcgcaaccac cgccctggcc 150

atctaccatg tgatcatgag cgtcttgttg ttcatcgagc actcagtaga 200

ggtggcccat ggcaaggcgt cctgcaagct ctcccagatg ggctacctca 250

ggatcgctga cctgatctcc agcttcctgc tcatcaccat gctcttcatc 300

atcagcctga gcctactgat cggcgtagtc aagaaccggg agaagtacct 350

gctgcccttc ctgtccctgc aaatcatgga ctatctcctg tgcctgctca 400

ccctgctggg ctcctacatt gagctgcccg cctacctcaa gttggcctcc 450

cggagccgtg ctagctcctc caagttcccc ctgatgacgc tgcagctgct 500

ggacttctgc ctgagcatcc tgaccctctg cagctcctac atggaagtgc 550

ccacctatct caacttcaag tccatgaacc acatgaatta cctccccagc 600

caggaggata tgcctcataa ccagttcatc aagatgatga tcatcttttc 650
catcgccttc atcactgtcc ttatcttcaa ggtctacatg ttcaagtgcg 700
tgtggcggtg ctacagattg atcaagtgca tgaactcggt ggaggagaag 750
agaaactcca agatgctcca gaaggtggtc ctgccgtcct acgaggaagc 800
cctgtctttg ccatcgaaga ccccagaggg gggcccagca ccacccccat 850
actcagaggt gtgaccctcg ccaggcccca gccccagtgc tgggaggggt 900
ggagctgcct cataatctgc ttttttgctt tggtggcccc tgtggcctgg 950
gtgggccctc ccgcccctcc ctggcaggac aatctgcttg tgtctccctc 1000
gctggcctgc tcctcctgca gggcctgtga gctgctcaca actgggtcaa 1050
cgctttaggc tgagtcactc ctcgggtctc tccataattc agcccaacaa 1100
tgcttggttt atttcaatca gctctgacac ttgtttagac gattggccat 1150
tctaaagttg gtgagtttgt caagcaacta tcgacttgat cagttcagcc 1200
aagcaactga caaatcaaaa acccacttgt cagttcagta aaataatttg 1250
gtcaaacaac agtctattgc attgatttat aaatagttgt cagttcacat 1300
agcaatttaa tcaagtaatc attaattagt taccccctat atataaatat 1350
atgtaatcaa tttcttcaaa tagcttgctt acatgataat caattagcca 1400
accatgagtc atttagaata gtgataaata gaatacacag aatagtgatg 1450
aaattcaatt taaaaaatca cgttagcctc caaaccattt aattcaaatg 1500
aacccatcaa ctggatgcca actctggcga atgtaggacc tctgagtggc 1550
tgtataattg ttaattcaaa tgaaattcat ttaaacagtt gacaaactgt 1600
cattcaacaa ttagctccag gaaataacag ttatttcatc ataaaacagt 1650
cccttcaaac acacaattgt tctgctgaag agttgtcatc aacaatccaa 1700
tgctcaccta ttcagttgct ctgtggtcag tgtggctgca tagcagtgga 1750
ttccatgaaa ggagtcattt tagtgatgag ctgccagtcc attcccaggc 1800
caggctgtcg ctggccatcc attcagtcga ttcagtcata ggcgaatctg 1850
ttctgcccga ggcttgtggt caagcaaaaa ttcagccctg aaatcaggca 1900
catctgttcg ttggactaaa cccacaggtt agttcagtca aagcaggcaa 1950
ccccccttgtg ggcactgacc ctgccactgg ggtcatggcg gttgtggcag 2000
ctggggaggt ttggccccaa cagccctcct gtgcctgctt ccctgtgtgt 2050
cggggtcctc cagggagctg acccagaggt ggaggccacg gaggcagggt 2100
ctctggggac tgtcgggggg tacagaggga gaaggctctg caagagctcc 2150

ctggcaatac ccccttgtgt aattgctttg tgtgcgacag ggaggaagtt 2200

tcaataaagc aacaacaagc ttcaaggaat tc 2232

<210> 14
<211> 4249
<212> DNA
<213> Homo sapien

<400> 14
gggaaagcga ggagccgcgg cggcgtggag ccggcgggcc cgggcggggg 50

ctccccggag ccctaccacc ccaccctggg catctacgcc cgctgcatcc 100

ggaacccagg ggtgcagcac ttccagcggg acacgctgtg cgggccctac 150

gccgagagct tcggcgagat cgccagcggc ttctggcagg ccacagctat 200

tttcctggct gtgggaatct ttattctctg catggtggcc ttggtgtccg 250

tcttcaccat gtgtgtacag agcatcatga agaaaagcat cttcaatgtc 300

tgtgggctgt tgcaaggaat tgcaggtcta ttccttatcc tcggtttgat 350

actctaccct gctggctggg gttgccagaa ggccatagac tactgtggac 400

attatgcatc tgcctacaaa cctggagact gctcccttgg ctgggccttt 450

tataccgcca ttgggggcac agtcctcact ttcatctgtg ctgtcttctc 500

tgcacaagca gaaattgcaa cctctagtga caaagtacag gaagaaattg 550

aagaggggaa aaacctgatc tgcctccttt agtttggaag agacaatgcc 600

attttctccc ttgagtaatc ttgtgaaaca gtccacagtt tcatcatttg 650

agtcaagtgg agaactaacc tttacctacc aaagccacgt tccacggccc 700

gaggcttaaa caggaccaat gagaggccac atccagctac gcaaagttac 750

tggacatgcg gtctgcagtg cacattataa ggaatggaac atgaaaatag 800

tatataatcc tagacctgga gttgccaagt tctgtcagac tccatctccc 850

ccaggttcaa tgatggatga taatctaaat cattagggca gcagtttctc 900

tggtaacgga agagaccgtc cgccagatct gcaggctgtt tctgctccaa 950

cactgcttgc ttgtgagcat ctctgcctca gaatggggtt ttgggttgga 1000

gttcttgttt tcctctgttc tttcaagttg tctccaacga acagaaaact 1050

ataaacttac tggggacagg atgtgtgcta aagggcacag caagacactg 1100

tcttttgctt agctgaccaa aggggtcagc agggatggcg tggagtcatg 1150

ctgtggaact tattctaggc tgaatcctag ggtaaggtgg atcaactgaa 1200

ctgtcactcc agagatttta gaaatttgag taaagaaaca ataaggacct 1250

atacaatcat atgagaacaa aaatatgaaa tcttgctagt gaagacgtat 1300
tttttcttct tcccagcagc caggctagca ccagttctgg cccagtctcc 1350
tcttcttctg gagatcacat gtttttcttc taaggttagg attgtgcttt 1400
gactgcgaaa ggaaacctca ctgtttcctc cttccaggga ctgaggtctc 1450
caagctagct gtggcttatg cagatgttca ctgggaggac ctgccagaat 1500
ctcggcactt ggggggagac ctttactccc agtttggtga ccatgctgta 1550
gtcagctcta tttccaatcc cgacagtagc agaatggcat tctacaacaa 1600
aaagaagcta gttatgggag ttaagttttt gtagttactg gtgttgatcc 1650
tgaaagcaga ctgagataac attaaattgc tgcaactgaa gaactgcagc 1700
caagacctta attccaggaa agcacagagg acaaagttaa ttcaaaaaga 1750
ggcgctagat caaggtcaca gcactgccta cacctgttta caaaaagaat 1800
caaataccac tatgaataag gattcagggg tttttaatct actttccata 1850
aattaccaat atcactgatt caggaagata gtatctcaga atgaccagag 1900
cagcacagaa acaagctact ctgacattat gggagcttca aaattgtatc 1950
atgatacaga aacactcctt agcactttaa gaaagtgaga tggaactgcc 2000
agatttctgg aaggagaaaa agtgtaggta tttgggttca ttaatctgct 2050
cacttgagga ctttgttttg aaaaagtacc ttctgtggac aaggtattgt 2100
gctaccagct atacaaccct gacttcagag tttgcaacct tgccctgagt 2150
gaatcatgtt aaagctgtct gagtctaaag caccgtatct tggtgcagaa 2200
cagataatta tacagagatg gaatgggaca accgcagttt tactacattc 2250
tggtgtttgg cctatatgag aaaccatctt ctcacagatt aagggctaag 2300
ggcaaaaggg gtgggaggtg tggaactagc cttaatgagt ttcccattcc 2350
tgaaccaaaa ttcaaagtga gtgagatgta aatcctgtga ttttggtgaa 2400
gaaaaaaacg ggtatcttca tagcagccta ggaaacctta accatatctc 2450
taacaccaca cagaaagagg ctggaggagc cactggacaa agcttctgtc 2500
tctgtgtgta catttataat gttctaacca agtctcaaac cttgatgaaa 2550
aacacaaaat tttccataa acttatcaga agactcactt ttctttcttt 2600
cttggataga gaaaccattt tctgacacta ggtttacaat ctcagtgtcc 2650
ttacaagtta agtcctaagc tcacaggatc ctccgagcat gtccatcacc 2700
tgctctttgg ctaaggtggc agtgtacctc tagatcaacc tgggaacagt 2750
cacaagggag tgtgacttct tggccataat aaactcactc gatagtgttt 2800

atgttattaa tctgaatgca acagaagaca aaagcacagg catgcacaca 2850
cacagaaccc caaaccacta aaaactacct aaacactgac ttagtaaata 2900
gtaaaaaggt aatgttggga cttttaaacc ttgaatccat tagccaggct 2950
tgggatgaaa ggaccatcta aaatcatgct agtctaaacc atgctcttcc 3000
acacagctgt ttaaaaacca ctgggtatga ggaatatgct agaaagaaat 3050
gttaaaaata gattgttggc tcacacttat ttttctaata aataggacca 3100
ttattactac caggaaagtc ttatttattt tgcctgaaat tggcttaaag 3150
aaagtctcat gacgggatgg gatgggctgc gcttctcaat gaactctgag 3200
gcagaaatat ttgccttgga ttctgtggat tctttaaacc tgtgtgctaa 3250
taattcaaac aatgttgcat taattgtata agggtttttg tatagttttc 3300
aaacatctgt ggtgtaatga tctttgttaa acatatattc tgtaaagtgc 3350
catagtcttt ttttatgtgt agcatattta aaaatatata tgtatattat 3400
acatacacaa gtttgtgtga aagatgtgca ataacaaagg tgtatgtatg 3450
ttttgttgtt ttgttttgga aactggacag gagtcaaaac agggatgttt 3500
gtttctgttt tggcaaagga gagttccaca tttttgcctt catggcttat 3550
tcagtaaccc ataattttaa tgctacacaa atcttatgtg aagaaaagac 3600
tggtatgaaa tcatttttc ctgggtctaa aataatcgct agtgttatgt 3650
caaagttaag cccgcacgcc aggcccagtt aatgctagtc tttcatgtga 3700
aatgtgaagc tgccatgttg ccttttctct tagtaggata actagtagct 3750
ggtacataat cactgaggag ctatttctta acatgctttt atagaccatg 3800
ctaatgctag accagtattt aagggctaat ctcacacctc cttagctgta 3850
agagtctggc ttagaacaga cctctctgtg caataacttg tggccactgg 3900
aaatccctgg gccggcattt gtattggggt tgcaatgact cccaagggcc 3950
aaaagagtta aaggcacgac tgggatttct tctgagactg tggtgaaact 4000
ccttccaagg ctgagggggt cagtaggtgc tctggaggga ctcggcacca 4050
cttgatattc aacagccact tgagccaaat ataaaattgt atttacagct 4100
gatggactca atttgagcct tcaaacttgt agttatccta ttatattgta 4150
aactaataca ttgtctagca ttgatttggt tcctgtgcat atgtattttc 4200
actatgtgct cccctcccca gatcttaatt aaaccagatt ttgcaattc 4249

<210> 15
<211> 95

<212> DNA
<213> Homo sapien

<400> 15
ttcagaagtg taattacttt aaaatacact acttccactt ttgtaagtat 50

tttacattta tgtatatatt ctatagtgga agcagaaatt ctctc 95

<210> 16
<211> 2879
<212> DNA
<213> Homo sapien

<400> 16
catttgctat gaatattctc tataacaaag caagacaaat ttagcagcac 50

ttcattgcat ctggatgggg gagagagctg gacaatttct tgctaacaag 100

agatggttaa ctgccctcac ctcagccgtg aattctgcac acctcgcatc 150

cggggcaaca cctgcttctg ctgtgacctc tacaactgtg gcaaccgggt 200

ggagatcact ggtgggtact acgaatacat cgatgtcagc agttgccaag 250

atatcatcca cctctaccac ctgctctggt ctgccaccat cctcaacatt 300

gttggcctgt tcctgggcat catcactgcc gctgtccttg gaggctttaa 350

ggacatgaac ccaactctcc cagcactgaa ctgttctgtt gaaaataccc 400

atccaacagt ttcttactat gctcatcccc aagtggcatc ctacaatacc 450

tactaccata gccctcctca cctgccacca tattctgctt atgactttca 500

gcattccggt gtctttccat cctcccctcc ctctggactt tctgatgagc 550

cccagtctgc ctctccctca cccagctaca tgtggtcctc aagtgcaccg 600

ccccgttact ctccacccta ctatccacct tttgaaaagc caccacctta 650

cagtccctaa agaggaatgc ctgctggcta ttgagattat tgtggctttt 700

gtatttctgc ttcagtggaa gtgtgtaggg tacaaaattt aaagtgtgac 750

tcttatgcat aaagttttac aatggcctgc caggctaggg aaagataggg 800

acgaagctta ttcattatta gtgcagagca ggggtggtca ggctgaacgc 850

agcacagaag ggcagctcac attctctaag caagactggg gagccagccc 900

agcaagaagc ttgtttggac ttgcattacc ctatgctcca cctctgtatt 950

cagcagaagt gtggttgcca tctttttcac tttatgtaaa ggagtgttgc 1000

cctcgggccc ttggcagatt gccaccccag cacctaggtt gaagcacctg 1050

gtttataggc cctatctttc cctacccta aagtcagtcc ctaaggacaa 1100

tttcccagct gatggggcta cacagtagtt ccaatacaga gagttctggc 1150

taagattttg tttgcttgtg tctggatgtt gaaaaagact gcccgtatct 1200

cttactcctt ccttctctgt gagtattgta aaaatggctg ttgtgatcac 1250
tcagctcagc ttttgttatt ggtacctcct aaagggaaaa gtgcaatatt 1300
cttgcatctt cagtagtggg gaacaggatg tattgttccg gaaacactga 1350
aatacacagc aacatgtgag atgttttaag tagatcactt aggagacagt 1400
ggttctacta catgttgcat tattacaaaa tacatttgct acaggagata 1450
taaatcttat ggttgtaatt cagagtttaa aaatgttata aattaggttc 1500
ttgggtcgtg atatgaattg ttactaatct ttgtgactat ttaatcttca 1550
aatattgtgc ttaaccccag caatccgcac gtatcctgca ccccacccca 1600
aaagagtcat ctgtatttta atgccactgg tcttatcggt ccttttgtct 1650
gttgagacca gtcatgacag cattcaagat tatgaaagtg ttacaatgcc 1700
gcttcaagtc tgcaaaacct caaacgtagc caacttgaca aatatttaag 1750
tgttacggca gatttaaaat ccatctggca caccgtggta ggtatttgta 1800
cagttctttt aattacacat agctttaaac catcaacctg atgagtttaa 1850
agcttttgca cccatgcctt cacttcagaa tgaacacctt cattgtgatc 1900
ttatgttaac ctgagaattg atttaaagga agattgataa tcctatactt 1950
tataacgtaa aaatacaggg gctacaggag ggtacctaat tagacagttc 2000
tccaaacaca gaacacacac tggaaaattt tccggccaat tttgctacct 2050
cccaacttga tggattagag gtagcgcata tgctggtgct cccatctacc 2100
ttgtagacac ttagccatca agaatcaagg cacaagaagt gcactctctc 2150
attaacagta aatgtttgca agatattcag tttaactttc agcatcatga 2200
atgttcttat ccagattttg aatccgaaaa actataatcc ttttatgtta 2250
tacaaaatta ctatgatttt ttacagttct gagcatatta aaattctact 2300
ggatttcaaa aagagactaa tacccaactg actaactaaa caaatatcaa 2350
cttgtaatac tcaatgaatt tttttgccat ttacatttga ccgttggctt 2400
tagtgaatgt ccatatttaa ttttttaagg caccattaca cagtttatcc 2450
tacatttatc acatttctta aagtgttaag attctatggc tcatttctat 2500
gtatttttct tactttacaa aataacctga aacagtatag attttgtaac 2550
acttaatttg agcagctttt ttattacatt gaattatata aagtgcatgt 2600
taccttagaa aaattagtat ttgctgcttt actcttttgc aaaacatttg 2650
ctgtaatgaa tggatttgta tttccaatat gtatcttgac tgcattttgt 2700

aatatttact gctttattcc taattctgct ttaaagtact gaactgggca 2750

tgaaacatta aaatattaat ccagaaactg tataaactgg atgttgctta 2800

aaatctgtat cactgccatg ttgaaaattc agactgcttt tgtgatgttt 2850

caaatgaata aaactatcct cccctcgtt 2879

<210> 17
<211> 1110
<212> DNA
<213> Homo sapien

<400> 17
ccaatcgccc ggtgcggtgg tgcagggtct cgggctagtc atggcgtccc 50

cgtctcggag actgcagact aaaccagtca ttacttgttt caagagcgtt 100

ctgctaatct acacttttat tttctggatc actggcgtta tccttcttgc 150

agttggcatt tggggcaagg tgagcctgga gaattacttt tctcttttaa 200

atgagaaggc caccaatgtc cccttcgtgc tcattgctac tggtaccgtc 250

attattcttt tgggcacctt tggttgtttt gctacctgcc gagcttctgc 300

atggatgcta aaactgtatg caatgtttct gactctcgtt tttttggtcg 350

aactggtcgc tgccatcgta ggatttgttt tcagacatga gattaagaac 400

agctttaaga ataattatga gaaggctttg aagcagtata actctacagg 450

agattataga agccatgcag tagacaagat ccaaaatacg ttgcattgtt 500

gtggtgtcac cgattataga gattggacag atactaatta ttactcagaa 550

aaaggatttc ctaagagttg ctgtaaactt gaagattgta ctccacagag 600

agatgcagac aaagtaaaca atgaaggttg ttttataaag gtgatgacca 650

ttatagagtc agaaatggga gtcgttgcag gaatttcctt tggagttgct 700

tgcttccaac tgattggaat ctttctcgcc tactgcctct ctcgtgccat 750

aacaaataac cagtatgaga tagtgtaacc caatgtatct gtgggcctat 800

tcctctctac ctttaaggac atttagggtc ccccctgtga attagaaagt 850

tgcttggctg gagaactgac aacactactt actgatagac caaaaaacta 900

caccagtagg ttgattcaat caagatgtat gtagacctaa aactacacca 950

ataggctgat tcaatcaaga tccgtgctcg cagtgggctg attcaatcaa 1000

gatgtatgtt tgctatgttc taagtccacc ttctatccca ttcatgttag 1050

atcgttgaaa ccctgtatcc ctctgaaaca ctggaagagc tagtaaattg 1100

taaatgaagt 1110

<210> 18

<211> 951
<212> DNA
<213> Homo sapien

<400> 18
gtgcactatg gctcggggct cgctgcgccg gttgctgcgg ctcctcgtgc 50

tggggctctg gctggcgttg ctgcgctccg tggccgggga gcaagcgcca 100

ggcaccgccc cctgctcccg cggcagctcc tggagcgcgg acctggacaa 150

gtgcatggac tgcgcgtctt gcagggcgcg accgcacagc gacttctgcc 200

tgggctgcgc tgcagcacct cctgccccct tccggctgct ttggcccatc 250

cttgggggcg ctctgagcct gaccttcgtg ctggggctgc tttctggctt 300

tttggtctgg agacgatgcc gcaggagaga gaagttcacc acccccatag 350

aggagaccgg cggagagggc tgcccagctg tggcgctgat ccagtgacaa 400

tgtgccccct gccagccggg gctcgcccac tcatcattca ttcatccatt 450

ctagagccag tctctgcctc ccagacgcgg cgggagccaa gctcctccaa 500

ccacaagggg ggtgggggc ggtgaatcac ctctgaggcc tgggcccagg 550

gttcagggga accttccaag gtgtctggtt gccctgcctc tggctccaga 600

acagaaaggg agcctcacgc tggctcacac aaaacagctg acactgacta 650

aggaactgca gcatttgcac aggggagggg ggtgccctcc ttcctagagg 700

ccctgggggc caggctgact tgggggcag acttgacact aggccccact 750

cactcagatg tcctgaaatt ccaccacggg ggtcaccctg gggggttagg 800

gacctatttt taacactagg gggctggccc actaggaggg ctggccctaa 850

gatacagacc cccccaactc cccaaagcgg ggaggagata tttattttgg 900

ggagagtttg gaggggaggg agaatttatt aataaaagaa tctttaactt 950

t 951

<210> 19
<211> 4577
<212> DNA
<213> Homo sapien

<400> 19
gctacaatcc atctggtctc ctccagctcc ttctttctgc aacatgggga 50

agaacaaact ccttcatcca agtctggttc ttctcctctt ggtcctcctg 100

cccacagacg cctcagtctc tggaaaaccg cagtatatgg ttctggtccc 150

ctccctgctc cacactgaga ccactgagaa gggctgtgtc cttctgagct 200

acctgaatga gacagtgact gtaagtgctt ccttggagtc tgtcagggga 250

aacaggagcc tcttcactga cctggaggcg gagaatgacg tactccactg 300
tgtcgccttc gctgtcccaa agtcttcatc caatgaggag gtaatgttcc 350
tcactgtcca agtgaaagga ccaacccaag aatttaagaa gcggaccaca 400
gtgatggtta agaacgagga cagtctggtc tttgtccaga cagacaaatc 450
aatctacaaa ccagggcaga cagtgaaatt tcgtgttgtc tccatggatg 500
aaaactttca ccccctgaat gagttgattc cactagtata cattcaggat 550
cccaaaggaa atcgcatcgc acaatggcag agtttccagt tagagggtgg 600
cctcaagcaa ttttcttttc ccctctcatc agagcccttc cagggctcct 650
acaaggtggt ggtacagaag aaatcaggtg gaaggacaga gcaccctttc 700
accgtggagg aatttgttct tcccaagttt gaagtacaag taacagtgcc 750
aaagataatc accatcttgg aagaagagat gaatgtatca gtgtgtggcc 800
tatacacata tgggaagcct gtccctggac atgtgactgt gagcatttgc 850
agaaagtata gtgacgcttc cgactgccac ggtgaagatt cacaggcttt 900
ctgtgagaaa ttcagtggac agctaaacag ccatggctgc ttctatcagc 950
aagtaaaaac caaggtcttc cagctgaaga ggaaggagta tgaaatgaaa 1000
cttcacactg aggcccagat ccaagaagaa ggaacagtgg tggaattgac 1050
tggaaggcag tccagtgaaa tcacaagaac cataaccaaa ctctcatttg 1100
tgaaagtgga ctcacacttt cgacagggaa ttcccttctt tgggcaggtg 1150
cgcctagtag atgggaaagg cgtccctata ccaaataaag tcatattcat 1200
cagaggaaat gaagcaaact attactccaa tgctaccacg gatgagcatg 1250
gccttgtaca gttctctatc aacaccacca acgttatggg tacctctctt 1300
actgttaggg tcaattacaa ggatcgtagt ccctgttacg gctaccagtg 1350
ggtgtcagaa gaacacgaag aggcacatca cactgcttat cttgtgttct 1400
ccccaagcaa gagctttgtc caccttgagc ccatgtctca tgaactaccc 1450
tgtggccata ctcagacagt ccaggcacat tatattctga atggaggcac 1500
cctgctgggg ctgaagaagc tctcctttta ttatctgata atggcaaagg 1550
gaggcattgt ccgaactggg actcatggac tgcttgtgaa gcaggaagac 1600
atgaagggcc atttttccat ctcaatccct gtgaagtcag acattgctcc 1650
tgtcgctcgg ttgctcatct atgctgtttt acctaccggg gacgtgattg 1700
gggattctgc aaaatatgat gttgaaaatt gtctggccaa caaggtggat 1750
ttgagcttca gcccatcaca aagtctccca gcctcacacg cccacctgcg 1800

agtcacagcg gctcctcagt ccgtctgcgc cctccgtgct gtggaccaaa 1850
gcgtgctgct catgaagcct gatgctgagc tctcggcgtc ctcggtttac 1900
aacctgctac cagaaaagga cctcactggc ttccctgggc ctttgaatga 1950
ccaggacgat gaagactgca tcaatcgtca taatgtctat attaatggaa 2000
tcacatatac tccagtatca agtacaaatg aaaaggatat gtacagcttc 2050
ctagaggaca tgggcttaaa ggcattcacc aactcaaaga ttcgtaaacc 2100
caaaatgtgt ccacagcttc aacagtatga aatgcatgga cctgaaggtc 2150
tacgtgtagg tttttatgag tcagatgtaa tgggaagagg ccatgcacgc 2200
ctggtgcatg ttgaagagcc tcacacggag accgtacgaa agtacttccc 2250
tgagacatgg atctgggatt tggtggtggt aaactcagca ggggtggctg 2300
aggtaggagt aacagtccct gacaccatca ccgagtggaa ggcaggggcc 2350
ttctgcctgt ctgaagatgc tggacttggt atctcttcca ctgcctctct 2400
ccgagccttc cagcccttct ttgtggagct tacaatgcct tactctgtga 2450
ttcgtggaga ggccttcaca ctcaaggcca cggtcctaaa ctaccttccc 2500
aaatgcatcc gggtcagtgt gcagctggaa gcctctcccg ccttccttgc 2550
tgtcccagtg gagaaggaac aagcgcctca ctgcatctgt gcaaacgggc 2600
ggcaaactgt gtcctgggca gtaaccccaa agtcattagg aaatgtgaat 2650
ttcactgtga gcgcagaggc actagagtct caagagctgt gtgggactga 2700
ggtgccttca gttcctgaac acggaaggaa agacacagtc atcaagcctc 2750
tgttggttga acctgaagga ctagagaagg aaacaacatt caactcccta 2800
ctttgtccat caggtggtga ggtttctgaa gaattatccc tgaaactgcc 2850
accaaatgtg gtagaagaat ctgcccgagc ttctgtctca gttttgggag 2900
acatattagg ctctgccatg caaaacacac aaaatcttct ccagatgccc 2950
tatggctgtg gagagcagaa tatggtcctc tttgctccta acatctatgt 3000
actggattat ctaaatgaaa cacagcagct tactccagag gtcaagtcca 3050
aggccattgg ctatctcaac actggttacc agagacagtt gaactacaaa 3100
cactatgatg gctcctacag cacctttggg gagcgatatg gcaggaacca 3150
gggcaacacc tggctcacag cctttgttct gaagactttt gcccaagctc 3200
gagcctacat cttcatcgat gaagcacaca ttacccaagc cctcatatgg 3250
ctctcccaga ggcagaagga caatggctgt ttcaggagct ctgggtcact 3300

gctcaacaat gccataaagg gaggagtaga agatgaagtg accctctccg 3350

cctatatcac catcgccctt ctggagattc ctctcacagt cactcaccct 3400

gttgtccgca atgccctgtt ttgcctggag tcagcctgga agacagcaca 3450

agaaggggac catggcagcc atgtatatac caaagcactg ctggcctatg 3500

cttttgccct ggcaggtaac caggacaaga ggaaggaagt actcaagtca 3550

cttaatgagg aagctgtgaa gaaagacaac tctgtccatt gggagcgccc 3600

tcagaaaccc aaggcaccag tggggcattt ttacgaaccc caggctccct 3650

ctgctgaggt ggagatgaca tcctatgtgc tcctcgctta tctcacggcc 3700

cagccagccc caacctcgga ggacctgacc tctgcaacca acatcgtgaa 3750

gtggatcacg aagcagcaga atgcccaggg cggtttctcc tccacccagg 3800

acacagtggt ggctctccat gctctgtcca aatatggagc cgccacattt 3850

accaggactg ggaaggctgc acaggtgact atccagtctt cagggacatt 3900

ttccagcaaa ttccaagtgg acaacaacaa tcgcctgtta ctgcagcagg 3950

tctcattgcc agagctgcct ggggaataca gcatgaaagt gacaggagaa 4000

ggatgtgtct acctccagac ctccttgaaa tacaatattc tcccagaaaa 4050

ggaagagttc ccctttgctt taggagtgca gactctgcct caaacttgtg 4100

atgaacccaa agcccacacc agcttccaaa tctccctaag tgtcagttac 4150

acagggagcc gctctgcctc caacatggcg atcgttgatg tgaagatggt 4200

ctctggcttc attcccctga agccaacagt gaaaatgctt gaaagatcta 4250

accatgtgag ccggacagaa gtcagcagca accatgtctt gatttacctt 4300

gataaggtgt caaatcagac actgagcttg ttcttcacgg ttctgcaaga 4350

tgtcccagta agagatctca aaccagccat agtgaaagtc tatgattact 4400

acgagacgga tgagtttgca atcgctgagt acaatgctcc ttgcagcaaa 4450

gatcttggaa atgcttgaag accacaaggc tgaaaagtgc tttgctggag 4500

tcctgttctc tgagctccac agaagacacg tgtttttgta tctttaaaga 4550

cttgatgaat aaacactttt tctggtc 4577

<210> 20
<211> 2463
<212> DNA
<213> Homo sapien

<400> 20
cgaaagatgg cggcggaaac gctgctgtcc agtttgttag gactgctgct 50

tctgggactc ctgttacccg caagtctgac cggcggtgtc gggagcctga 100

```
acctggagga gctgagtgag atgcgttatg ggatcgagat cctgccgttg 150
cctgtcatgg gagggcagag ccaatcttcg gacgtggtga ttgtctcctc 200
taagtacaaa cagcgctatg agtgtcgcct gccagctgga gctattcact 250
tccagcgtga aagggaggag gaaacacctg cttaccaagg gcctgggatc 300
cctgagttgt tgagcccaat gagagatgct ccctgcttgc tgaagacaaa 350
ggactggtgg acatatgaat tctgttatgg acgccacatc cagcaatacc 400
acatggaaga ttcagagatc aaaggtgaag tcctctatct cggctactac 450
caatcagcct tcgactggga tgatgaaaca gccaaggcct ccaagcagca 500
tcgtcttaaa cgctaccaca gccagaccta tggcaatggg tccaagtgcg 550
accttaatgg gaggccccgg gaggccgagg ttcggttcct ctgtgacgag 600
ggtgcaggta tctctgggga ctacatcgat cgcgtggacg agcccttgtc 650
ctgctcttat gtgctgacca ttcgcactcc tcggctctgc ccccaccctc 700
tcctccggcc cccacccagt gctgcaccgc aggccatcct ctgtcaccct 750
tccctacagc ctgaggagta catggcctac gttcagaggc aagccgactc 800
aaagcagtat ggagataaaa tcatagagga gctgcaagat ctaggccccc 850
aagtgtggag tgagaccaag tctggggtgg caccccaaaa gatggcaggt 900
gcgagcccga ccaaggatga cagtaaggac tcagatttct ggaagatgct 950
taatgagcca gaggaccagg ccccaggagg ggaggaggtg ccggctgagg 1000
agcaggaccc aagccctgag gcagcagatt cagcttctgg tgctcccaat 1050
gattttcaga acaacgtgca ggtcaaagtc attcgaagcc ctgcggattt 1100
gattcgattc atagaggagc tgaaaggtgg aacaaaaaag gggaagccaa 1150
atataggcca agagcagcct gtggatgatg ctgcagaagt ccctcagagg 1200
gaaccagaga aggaaagggg tgatccagaa cggcagagag agatggaaga 1250
agaggaggat gaggatgagg atgaggatga agatgaggat gaacggcagt 1300
tactgggaga atttgagaag gaactggaag ggatcctgct tccgtcagac 1350
cgagaccggc tccgttcgga gacagagaaa gagctggacc cagatgggct 1400
gaagaaggag tcagagcggg atcgggcaat gctggctctc acatccactc 1450
tcaacaaact catcaaaaga ctggaggaaa aacagagtcc agagctggtg 1500
aagaagcaca agaaaaagag ggttgtcccc aaaaagcctc ccccatcacc 1550
ccaacctaca gggaaaattg agatcaaaat tgtccgccca tgggctgaag 1600
```

ggactgaaga gggtgcacgt tggctgactg atgaggacac gagaaacctc 1650

aaggagatct tcttcaatat cttggtgccg ggagctgaag aggcccagaa 1700

ggaacgccag cggcagaaag agctggagag caattaccgc cgggtgtggg 1750

gctctccagg tggggagggc acaggggacc tggacgaatt tgacttctga 1800

gaccaacact acacttgacc cttcacggaa tccagactct tcctggactg 1850

gcttgcctcc tccccacctc cccaccctgg aacccctgag ggccaaacag 1900

cagagtggag ctgagctgtg gacctctcgg gcaactctgt gggtgtgggg 1950

gccctgggtg aatgctgctg cccctgctgg cagccacctt gagacctcac 2000

cgggcctgtg atatttgctc tcctgaactc tcactcaatc ctcttcctct 2050

cctctgtggc ttttcctgtt attgtcccct aatgatagga tattccctgc 2100

tgcctacctg gagattcagt aggatctttt gagtggaggt gggtagagag 2150

agcaaggagg gcaggacact tagcaggcac tgagcaagca ggcccccacc 2200

tgcccttagt gatgtttgga gtcgttttac cctcttctat tgaattgcct 2250

tgggatttcc ttctcccttt ccctgcccac cctgtcccct acaatttgtg 2300

cttctgagtt gaggagcctt cacctctgtt gctgaggaaa tggtagaatg 2350

ctgcctatca cctccagcac aatcccagtg aaaaaggtgt gaagcaccca 2400

ccatgttctt gaacaatcag gtttctaaat aaacaactgg accatcaaaa 2450

aaaaaaaaaa aaa 2463

<210> 21
<211> 900
<212> DNA
<213> Homo sapien

<400> 21
gcggcgggag aggaacgcgc agccagcctt gggaagccca ggcccggcag 50

ccatggcggt ggaaggagga atgaaatgtg tgaagttctt gctctacgtc 100

ctcctgctgg ccttttgcgc ctgtgcagtg ggactgattg ccgtgggtgt 150

cggggcacag cttgtcctga gtcagaccat aatccagggg gctacccctg 200

gctctctgtt gccagtggtc atcatcgcag tgggtgtctt cctcttcctg 250

gtggcttttg tgggctgctg cggggcctgc aaggagaact attgtcttat 300

gatcacgttt gccatctttc tgtctcttat catgttggtg gaggtggccg 350

cagccattgc tggctatgtg tttagagata aggtgatgtc agagtttaat 400

aacaacttcc ggcagcagat ggagaattac ccgaaaaaca accacactgc 450

ttcgatcctg gacaggatgc aggcagattt taagtgctgt ggggctgcta 500

actacacaga ttgggagaaa atcccttcca tgtcgaagaa ccgagtcccc 550

gactcctgct gcattaatgt tactgtgggc tgtgggatta atttcaacga 600

gaaggcgatc cataaggagg gctgtgtgga gaagattggg ggctggctga 650

ggaaaaatgt gctggtggta gctgcagcag cccttggaat tgcttttgtc 700

gaggttttgg gaattgtctt tgcctgctgc ctcgtgaaga gtatcagaag 750

tggctacgag gtgatgtagg ggtctggtct cctcagcctc ctcatctggg 800

ggagtggaat agtatcctcc aggtttttca attaaacgga ttattttttc 850

agaccgaaaa aaaaaaaaaa aaaaaaaaaa aaaaaaaaaa aaaaaaaaaa 900

<210> 22
<211> 1192
<212> DNA
<213> Homo sapien

<400> 22
cgcgcccccc agtcccgcac ccgttcggcc caggctaagt tagccctcac 50

catgccggtc aaaggaggca ccaagtgcat caaatacctg ctgttcggat 100

ttaacttcat cttctggctt gccgggattg ctgtccttgc cattggacta 150

tggctccgat tcgactctca gaccaagagc atcttcgagc aagaaactaa 200

taataataat tccagcttct acacaggagt ctatattctg atcggagccg 250

gcgccctcat gatgctggtg ggcttcctgg gctgctgcgg ggctgtgcag 300

gagtcccagt gcatgctggg actgttcttc ggcttcctct tggtgatatt 350

cgccattgaa atagctgcgg ccatctgggg atattcccac aaggatgagg 400

tgattaagga agtccaggag ttttacaagg acacctacaa caagctgaaa 450

accaaggatg agccccagcg ggaaacgctg aaagccatcc actatgcgtt 500

gaactgctgt ggtttggctg gggcgtgga acagtttatc tcagacatct 550

gccccaagaa ggacgtactc gaaaccttca ccgtgaagtc ctgtcctgat 600

gccatcaaag aggtcttcga caataaattc cacatcatcg gcgcagtggg 650

catcggcatt gccgtggtca tgatatttgg catgatcttc agtatgatct 700

tgtgctgtgc tatccgcagg aaccgcgaga tggtctagag tcagcttaca 750

tccctgagca ggaaagttta cccatgaaga ttggtgggat tttttgtttg 800

tttgttttgt tttgtttgtt gtttgttgtt tgttttttttg ccactaattt 850

tagtattcat tctgcattgc tagataaaag ctgaagttac tttatgtttg 900

tcttttaatg cttcattcaa tattgacatt tgtagttgag cgggggggttt 950

ggtttgcttg gtttatattt ttcagttgtt tgtttttgct tgttatatta 1000

agcagaaatc ctgcaatgaa aggtactata tttgctagac tctagacaag 1050

atattgtaca taaaagaatt tttttgtctt taaatagata caaatgtcta 1100

tcaactttaa tcaagttgta acttatattg aagacaattt gatacataat 1150

aaaaaattat gacaatgaaa aaaaaaaaaa aaaaaaaaaa gg 1192

<210> 23
<211> 375
<212> PRT
<213> Homo sapien

<400> 23
Met Glu Arg Ala Ser Cys Leu Leu Leu Leu Leu Leu Pro Leu Val
1 5 10 15

His Val Ser Ala Thr Thr Pro Glu Pro Cys Glu Leu Asp Asp Glu
20 25 30

Asp Phe Arg Cys Val Cys Asn Phe Ser Glu Pro Gln Pro Asp Trp
35 40 45

Ser Glu Ala Phe Gln Cys Val Ser Ala Val Glu Val Glu Ile His
50 55 60

Ala Gly Gly Leu Asn Leu Glu Pro Phe Leu Lys Arg Val Asp Ala
65 70 75

Asp Ala Asp Pro Arg Gln Tyr Ala Asp Thr Val Lys Ala Leu Arg
80 85 90

Val Arg Arg Leu Thr Val Gly Ala Ala Gln Val Pro Ala Gln Leu
95 100 105

Leu Val Gly Ala Leu Arg Val Leu Ala Tyr Ser Arg Leu Lys Glu
110 115 120

Leu Thr Leu Glu Asp Leu Lys Ile Thr Gly Thr Met Pro Pro Leu
125 130 135

Pro Leu Glu Ala Thr Gly Leu Ala Leu Ser Ser Leu Arg Leu Arg
140 145 150

Asn Val Ser Trp Ala Thr Gly Arg Ser Trp Leu Ala Glu Leu Gln
155 160 165

Gln Trp Leu Lys Pro Gly Leu Lys Val Leu Ser Ile Ala Gln Ala
170 175 180

His Ser Pro Ala Phe Ser Cys Glu Gln Val Arg Ala Phe Pro Ala
185 190 195

Leu Thr Ser Leu Asp Leu Ser Asp Asn Pro Gly Leu Gly Glu Arg
200 205 210

Gly Leu Met Ala Ala Leu Cys Pro His Lys Phe Pro Ala Ile Gln
215 220 225

```
Asn Leu Ala Leu Arg Asn Thr Gly Met Glu Thr Pro Thr Gly Val
                230                 235                 240

Cys Ala Ala Leu Ala Ala Ala Gly Val Gln Pro His Ser Leu Asp
                245                 250                 255

Leu Ser His Asn Ser Leu Arg Ala Thr Val Asn Pro Ser Ala Pro
                260                 265                 270

Arg Cys Met Trp Ser Ser Ala Leu Asn Ser Leu Asn Leu Ser Phe
                275                 280                 285

Ala Gly Leu Glu Gln Val Pro Lys Gly Leu Pro Ala Lys Leu Arg
                290                 295                 300

Val Leu Asp Leu Ser Cys Asn Arg Leu Asn Arg Ala Pro Gln Pro
                305                 310                 315

Asp Glu Leu Pro Glu Val Asp Asn Leu Thr Leu Asp Gly Asn Pro
                320                 325                 330

Phe Leu Val Pro Gly Thr Ala Leu Pro His Glu Gly Ser Met Asn
                335                 340                 345

Ser Gly Val Val Pro Ala Cys Ala Arg Ser Thr Leu Ser Val Gly
                350                 355                 360

Val Ser Gly Thr Leu Val Leu Leu Gln Gly Ala Arg Gly Phe Ala
                365                 370                 375
```

<210> 24
<211> 185
<212> PRT
<213> Homo sapien

<400> 24

```
Met Ala Arg Gly Ala Ala Leu Ala Leu Leu Leu Phe Gly Leu Leu
  1               5                  10                  15

Gly Val Leu Val Ala Ala Pro Asp Gly Gly Phe Asp Leu Ser Asp
                 20                  25                  30

Ala Leu Pro Asp Asn Glu Asn Lys Lys Pro Thr Ala Ile Pro Lys
                 35                  40                  45

Lys Pro Ser Ala Gly Asp Asp Phe Asp Leu Gly Asp Ala Val Val
                 50                  55                  60

Asp Gly Glu Asn Asp Asp Pro Arg Pro Pro Asn Pro Pro Lys Pro
                 65                  70                  75

Met Pro Asn Pro Asn Pro Asn His Pro Ser Ser Ser Gly Ser Phe
                 80                  85                  90

Ser Asp Ala Asp Leu Ala Asp Gly Val Ser Gly Gly Glu Gly Lys
                 95                 100                 105

Gly Gly Ser Asp Gly Gly Gly Ser His Arg Lys Glu Gly Glu Glu
                110                 115                 120

Ala Asp Ala Pro Gly Val Ile Pro Gly Ile Val Gly Ala Val Val
```

```
                125                 130                 135

Val Ala Val Ala Gly Ala Ile Ser Ser Phe Ile Ala Tyr Gln Lys
                140                 145                 150

Lys Lys Leu Cys Phe Lys Glu Asn Ala Glu Gln Gly Glu Val Asp
                155                 160                 165

Met Glu Ser His Arg Asn Ala Asn Ala Glu Pro Ala Val Gln Arg
                170                 175                 180

Thr Leu Leu Glu Lys
                185

<210> 25
<211> 113
<212> PRT
<213> Homo sapien

<400> 25
Met Gly Gly Leu Glu Pro Cys Ser Arg Leu Leu Leu Leu Pro Leu
1               5                   10                  15

Leu Leu Ala Val Ser Gly Leu Arg Pro Val Gln Ala Gln Ala Gln
                20                  25                  30

Ser Asp Cys Ser Cys Ser Thr Val Ser Pro Gly Val Leu Ala Gly
                35                  40                  45

Ile Val Met Gly Asp Leu Val Leu Thr Val Leu Ile Ala Leu Ala
                50                  55                  60

Val Tyr Phe Leu Gly Arg Leu Val Pro Arg Gly Arg Gly Ala Ala
                65                  70                  75

Glu Ala Ala Thr Arg Lys Gln Arg Ile Thr Glu Thr Glu Ser Pro
                80                  85                  90

Tyr Gln Glu Leu Gln Gly Gln Arg Ser Asp Val Tyr Ser Asp Leu
                95                  100                 105

Asn Thr Gln Arg Pro Tyr Tyr Lys
                110

<210> 26
<211> 1212
<212> PRT
<213> Homo sapien

<400> 26
Gly Gln Lys Gly Glu Arg Gly Leu Pro Gly Leu Gln Gly Val Ile
1               5                   10                  15

Gly Phe Pro Gly Met Gln Gly Pro Glu Gly Pro Gln Gly Pro Pro
                20                  25                  30

Gly Gln Lys Gly Asp Thr Gly Glu Pro Gly Leu Pro Gly Thr Lys
                35                  40                  45

Gly Thr Arg Gly Pro Pro Gly Ala Ser Gly Tyr Pro Gly Asn Pro
                50                  55                  60
```

```
Gly Leu Pro Gly Ile Pro Gly Gln Asp Gly Pro Pro Gly Pro Pro
                65                  70                  75

Gly Ile Pro Gly Cys Asn Gly Thr Lys Gly Glu Arg Gly Pro Leu
                80                  85                  90

Gly Pro Pro Gly Leu Pro Gly Phe Ala Gly Asn Pro Gly Pro Pro
                95                  100                 105

Gly Leu Pro Gly Met Lys Gly Asp Pro Gly Glu Ile Leu Gly His
                110                 115                 120

Val Pro Gly Met Leu Leu Lys Gly Glu Arg Gly Phe Pro Gly Ile
                125                 130                 135

Pro Gly Thr Pro Gly Pro Pro Gly Leu Pro Gly Leu Gln Gly Pro
                140                 145                 150

Val Gly Pro Pro Gly Phe Thr Gly Pro Pro Gly Pro Pro Gly Pro
                155                 160                 165

Pro Gly Pro Pro Gly Glu Lys Gly Gln Met Gly Leu Ser Phe Gln
                170                 175                 180

Gly Pro Lys Gly Asp Lys Gly Asp Gln Gly Val Ser Gly Pro Pro
                185                 190                 195

Gly Val Pro Gly Gln Ala Gln Val Gln Glu Lys Gly Asp Phe Ala
                200                 205                 210

Thr Lys Gly Glu Lys Gly Gln Lys Gly Glu Pro Gly Phe Gln Gly
                215                 220                 225

Met Pro Gly Val Gly Glu Lys Gly Glu Pro Gly Lys Pro Gly Pro
                230                 235                 240

Arg Gly Lys Pro Gly Lys Asp Gly Asp Lys Gly Glu Lys Gly Ser
                245                 250                 255

Pro Gly Phe Pro Gly Glu Pro Gly Tyr Pro Gly Leu Ile Gly Arg
                260                 265                 270

Gln Gly Pro Gln Gly Glu Lys Gly Glu Ala Gly Pro Pro Gly Pro
                275                 280                 285

Pro Gly Ile Val Ile Gly Thr Gly Pro Leu Gly Glu Lys Gly Glu
                290                 295                 300

Arg Gly Tyr Pro Gly Thr Pro Gly Pro Arg Gly Glu Pro Gly Pro
                305                 310                 315

Lys Gly Phe Pro Gly Leu Pro Gly Gln Pro Gly Pro Pro Gly Leu
                320                 325                 330

Pro Val Pro Gly Gln Ala Gly Ala Pro Gly Phe Pro Gly Glu Arg
                335                 340                 345

Gly Glu Lys Gly Asp Arg Gly Phe Pro Gly Thr Ser Leu Pro Gly
                350                 355                 360
```

```
Pro Ser Gly Arg Asp Gly Leu Pro Gly Pro Pro Gly Ser Pro Gly
                365                 370                 375

Pro Pro Gly Gln Pro Gly Tyr Thr Asn Gly Ile Val Glu Cys Gln
                380                 385                 390

Pro Gly Pro Pro Gly Asp Gln Gly Pro Pro Gly Ile Pro Gly Gln
                395                 400                 405

Pro Gly Phe Ile Gly Glu Ile Gly Glu Lys Gly Gln Lys Gly Glu
                410                 415                 420

Ser Cys Leu Ile Cys Asp Ile Asp Gly Tyr Arg Gly Pro Pro Gly
                425                 430                 435

Pro Gln Gly Pro Pro Gly Glu Ile Gly Phe Pro Gly Gln Pro Gly
                440                 445                 450

Ala Lys Gly Asp Arg Gly Leu Pro Gly Arg Asp Gly Val Ala Gly
                455                 460                 465

Val Pro Gly Pro Gln Gly Thr Pro Gly Leu Ile Gly Gln Pro Gly
                470                 475                 480

Ala Lys Gly Glu Pro Gly Glu Phe Tyr Phe Asp Leu Arg Leu Lys
                485                 490                 495

Gly Asp Lys Gly Asp Pro Gly Phe Pro Gly Gln Pro Gly Met Pro
                500                 505                 510

Gly Arg Ala Gly Ser Pro Gly Arg Asp Gly His Pro Gly Leu Pro
                515                 520                 525

Gly Pro Lys Gly Ser Pro Gly Ser Val Gly Leu Lys Gly Glu Arg
                530                 535                 540

Gly Pro Pro Gly Gly Val Gly Phe Pro Gly Ser Arg Gly Asp Thr
                545                 550                 555

Gly Pro Pro Gly Pro Pro Gly Tyr Gly Pro Ala Gly Pro Ile Gly
                560                 565                 570

Asp Lys Gly Gln Ala Gly Phe Pro Gly Gly Pro Gly Ser Pro Gly
                575                 580                 585

Leu Pro Gly Pro Lys Gly Glu Pro Gly Lys Ile Val Pro Leu Pro
                590                 595                 600

Gly Pro Pro Gly Ala Glu Gly Leu Pro Gly Ser Pro Gly Phe Pro
                605                 610                 615

Gly Pro Gln Gly Asp Arg Gly Phe Pro Gly Thr Pro Gly Arg Pro
                620                 625                 630

Gly Leu Pro Gly Glu Lys Gly Ala Val Gly Gln Pro Gly Ile Gly
                635                 640                 645

Phe Pro Gly Pro Pro Gly Pro Lys Gly Val Asp Gly Leu Pro Gly
                650                 655                 660

Asp Met Gly Pro Pro Gly Thr Pro Gly Arg Pro Gly Phe Asn Gly
```

665 670 675

Leu Pro Gly Asn Pro Gly Val Gln Gly Gln Lys Gly Glu Pro Gly
680 685 690

Val Gly Leu Pro Gly Leu Lys Gly Leu Pro Gly Leu Pro Gly Ile
695 700 705

Pro Gly Thr Pro Gly Glu Lys Gly Ser Ile Gly Val Pro Gly Val
710 715 720

Pro Gly Glu His Gly Ala Ile Gly Pro Pro Gly Leu Gln Gly Ile
725 730 735

Arg Gly Glu Pro Gly Pro Pro Gly Leu Pro Gly Ser Val Gly Ser
740 745 750

Pro Gly Val Pro Gly Ile Gly Pro Pro Gly Ala Arg Gly Pro Pro
755 760 765

Gly Gly Gln Gly Pro Pro Gly Leu Ser Gly Pro Pro Gly Ile Lys
770 775 780

Gly Glu Lys Gly Phe Pro Gly Phe Pro Gly Leu Asp Met Pro Gly
785 790 795

Pro Lys Gly Asp Lys Gly Ala Gln Gly Leu Pro Gly Ile Thr Gly
800 805 810

Gln Ser Gly Leu Pro Gly Leu Pro Gly Gln Gln Gly Ala Pro Gly
815 820 825

Ile Pro Gly Phe Pro Gly Ser Lys Gly Glu Met Gly Val Met Gly
830 835 840

Thr Pro Gly Gln Pro Gly Ser Pro Gly Pro Trp Gly Ala Pro Gly
845 850 855

Leu Pro Gly Glu Lys Gly Asp His Gly Phe Pro Gly Ser Ser Gly
860 865 870

Pro Arg Gly Asp Pro Gly Leu Lys Gly Asp Lys Gly Asp Val Gly
875 880 885

Leu Pro Gly Lys Pro Gly Ser Met Asp Lys Val Asp Met Gly Ser
890 895 900

Met Lys Gly Gln Lys Gly Asp Gln Gly Glu Lys Gly Gln Ile Gly
905 910 915

Pro Ile Gly Glu Lys Gly Ser Arg Gly Asp Pro Gly Thr Pro Gly
920 925 930

Val Pro Gly Lys Asp Gly Gln Ala Gly Gln Pro Gly Gln Pro Gly
935 940 945

Pro Lys Gly Asp Pro Gly Ile Ser Gly Thr Pro Gly Ala Pro Gly
950 955 960

Leu Pro Gly Pro Lys Gly Ser Val Gly Gly Met Gly Leu Pro Gly
965 970 975

Thr Pro Gly Glu Lys Gly Val Pro Gly Ile Pro Gly Pro Gln Gly
980 985 990

Ser Pro Gly Leu Pro Gly Asp Lys Gly Ala Lys Gly Glu Lys Gly
995 1000 1005

Gln Ala Gly Pro Pro Gly Ile Gly Ile Pro Gly Leu Arg Gly Glu
1010 1015 1020

Lys Gly Asp Gln Gly Ile Ala Gly Phe Pro Gly Ser Pro Gly Glu
1025 1030 1035

Lys Gly Glu Lys Gly Ser Ile Gly Ile Pro Gly Met Pro Gly Ser
1040 1045 1050

Pro Gly Leu Lys Gly Ser Pro Gly Ser Val Gly Tyr Pro Gly Ser
1055 1060 1065

Pro Gly Leu Pro Gly Glu Lys Gly Asp Lys Gly Leu Pro Gly Leu
1070 1075 1080

Asp Gly Ile Pro Gly Val Lys Gly Glu Ala Gly Leu Pro Gly Thr
1085 1090 1095

Pro Gly Pro Thr Gly Pro Ala Gly Gln Lys Gly Glu Pro Gly Ser
1100 1105 1110

Asp Gly Ile Pro Gly Ser Ala Gly Glu Lys Gly Glu Pro Gly Leu
1115 1120 1125

Pro Gly Arg Gly Phe Pro Gly Phe Pro Gly Ala Lys Gly Asp Lys
1130 1135 1140

Gly Ser Lys Gly Glu Val Gly Phe Pro Gly Leu Ala Gly Ser Pro
1145 1150 1155

Gly Ile Pro Gly Ser Lys Gly Glu Gln Gly Phe Met Gly Pro Pro
1160 1165 1170

Gly Pro Gln Gly Gln Pro Gly Leu Pro Gly Ser Pro Gly His Ala
1175 1180 1185

Thr Glu Gly Pro Lys Gly Asp Arg Gly Pro Gln Gly Gln Pro Gly
1190 1195 1200

Leu Pro Gly Leu Pro Gly Pro Met Gly Pro Pro Gly
1205 1210

<210> 27
<211> 459
<212> PRT
<213> Homo sapien

<400> 27
Gly Glu Arg Gly Pro Pro Gly Ser Pro Gly Leu Gln Gly Phe Pro
1 5 10 15

Gly Ile Thr Pro Pro Ser Asn Ile Ser Gly Ala Pro Gly Asp Lys
20 25 30

Gly Ala Pro Gly Ile Phe Gly Leu Lys Gly Tyr Arg Gly Pro Pro
35 40 45

Gly Pro Pro Gly Ser Ala Ala Leu Pro Gly Ser Lys Gly Asp Thr
50 55 60

Gly Asn Pro Gly Ala Pro Gly Thr Pro Gly Thr Lys Gly Trp Ala
65 70 75

Gly Asp Ser Gly Pro Gln Gly Arg Pro Gly Val Phe Gly Leu Pro
80 85 90

Gly Glu Lys Gly Pro Arg Gly Glu Gln Gly Phe Met Gly Asn Thr
95 100 105

Gly Pro Thr Gly Ala Val Gly Asp Arg Gly Pro Lys Gly Pro Lys
110 115 120

Gly Asp Pro Gly Phe Pro Gly Ala Pro Gly Thr Val Gly Ala Pro
125 130 135

Gly Ile Ala Gly Ile Pro Gln Lys Ile Ala Ile Gln Pro Gly Thr
140 145 150

Val Gly Pro Gln Gly Arg Arg Gly Pro Pro Gly Ala Pro Gly Glu
155 160 165

Ile Gly Pro Gln Gly Pro Pro Gly Glu Pro Gly Phe Arg Gly Ala
170 175 180

Pro Gly Lys Ala Gly Pro Gln Gly Arg Gly Gly Val Ser Ala Val
185 190 195

Pro Gly Phe Arg Gly Asp Glu Gly Pro Ile Gly His Gln Gly Pro
200 205 210

Ile Gly Gln Glu Gly Ala Pro Gly Arg Pro Gly Ser Pro Gly Leu
215 220 225

Pro Gly Met Pro Gly Arg Ser Val Ser Ile Gly Tyr Leu Leu Val
230 235 240

Lys His Ser Gln Thr Asp Gln Glu Pro Met Cys Pro Val Gly Met
245 250 255

Asn Lys Leu Trp Ser Gly Tyr Ser Leu Leu Tyr Phe Glu Gly Gln
260 265 270

Glu Lys Ala His Asn Gln Asp Leu Gly Leu Ala Gly Ser Cys Leu
275 280 285

Ala Arg Phe Ser Thr Met Pro Phe Leu Tyr Cys Asn Pro Gly Asp
290 295 300

Val Cys Tyr Tyr Ala Ser Arg Asn Asp Lys Ser Tyr Trp Leu Ser
305 310 315

Thr Thr Ala Pro Leu Pro Met Met Pro Val Ala Glu Asp Glu Ile
320 325 330

Lys Pro Tyr Ile Ser Arg Cys Ser Val Cys Glu Ala Pro Ala Ile

```
                335                 340                 345

Ala Ile Ala Val His Ser Gln Asp Val Ser Ile Pro His Cys Pro
                350                 355                 360

Ala Gly Trp Arg Ser Leu Trp Ile Gly Tyr Ser Phe Leu Met His
                365                 370                 375

Thr Ala Ala Gly Asp Glu Gly Gly Gly Gln Ser Leu Val Ser Pro
                380                 385                 390

Gly Ser Cys Leu Glu Asp Phe Arg Ala Thr Pro Phe Ile Glu Cys
                395                 400                 405

Asn Gly Gly Arg Gly Thr Cys His Tyr Tyr Ala Asn Lys Tyr Ser
                410                 415                 420

Phe Trp Leu Thr Thr Ile Pro Glu Gln Ser Phe Gln Gly Ser Pro
                425                 430                 435

Ser Ala Asp Thr Leu Lys Ala Gly Leu Ile Arg Thr His Ile Ser
                440                 445                 450

Arg Cys Gln Val Cys Met Lys Asn Leu
                455

<210> 28
<211> 1496
<212> PRT
<213> Homo sapien

<400> 28
Ser Arg Pro Trp Trp Leu Arg Ala Ser Glu Arg Pro Ser Ala Pro
1               5                   10                  15

Ser Ala Met Ala Lys Arg Ser Arg Gly Pro Gly Arg Arg Cys Leu
                20                  25                  30

Leu Ala Leu Val Leu Phe Cys Ala Trp Gly Thr Leu Ala Val Val
                35                  40                  45

Ala Gln Lys Pro Gly Ala Gly Cys Pro Ser Arg Cys Leu Cys Phe
                50                  55                  60

Arg Thr Thr Val Arg Cys Met His Leu Leu Leu Glu Ala Val Pro
                65                  70                  75

Ala Val Ala Pro Gln Thr Ser Ile Leu Asp Leu Arg Phe Asn Arg
                80                  85                  90

Ile Arg Glu Ile Gln Pro Gly Ala Phe Arg Arg Leu Arg Asn Leu
                95                  100                 105

Asn Thr Leu Leu Leu Asn Asn Asn Gln Ile Lys Arg Ile Pro Ser
                110                 115                 120

Gly Ala Phe Glu Asp Leu Glu Asn Leu Lys Tyr Leu Tyr Leu Tyr
                125                 130                 135

Lys Asn Glu Ile Gln Ser Ile Asp Arg Gln Ala Phe Lys Gly Leu
                140                 145                 150
```

```
Ala Ser Leu Glu Gln Leu Tyr Leu His Phe Asn Gln Ile Glu Thr
                155                 160                 165

Leu Asp Pro Asp Ser Phe Gln His Leu Pro Lys Leu Glu Arg Leu
                170                 175                 180

Phe Leu His Asn Asn Arg Ile Thr His Leu Val Pro Gly Thr Phe
                185                 190                 195

Asn His Leu Glu Ser Met Lys Arg Leu Arg Leu Asp Ser Asn Thr
                200                 205                 210

Leu His Cys Asp Cys Glu Ile Leu Trp Leu Ala Asp Leu Leu Lys
                215                 220                 225

Thr Tyr Ala Glu Ser Gly Asn Ala Gln Ala Ala Ala Ile Cys Glu
                230                 235                 240

Tyr Pro Arg Arg Ile Gln Gly Arg Ser Val Ala Thr Ile Thr Pro
                245                 250                 255

Glu Glu Leu Asn Cys Glu Arg Pro Arg Ile Thr Ser Glu Pro Gln
                260                 265                 270

Asp Ala Asp Val Thr Ser Gly Asn Thr Val Tyr Phe Thr Cys Arg
                275                 280                 285

Ala Glu Gly Asn Pro Lys Pro Glu Ile Ile Trp Leu Arg Asn Asn
                290                 295                 300

Asn Glu Leu Ser Met Lys Thr Asp Ser Arg Leu Asn Leu Leu Asp
                305                 310                 315

Asp Gly Thr Leu Met Ile Gln Asn Thr Gln Glu Thr Asp Gln Gly
                320                 325                 330

Ile Tyr Gln Cys Met Ala Lys Asn Val Ala Gly Glu Val Lys Thr
                335                 340                 345

Gln Glu Val Thr Leu Arg Tyr Phe Gly Ser Pro Ala Arg Pro Thr
                350                 355                 360

Phe Val Ile Gln Pro Gln Asn Thr Glu Val Leu Val Gly Glu Ser
                365                 370                 375

Val Thr Leu Glu Cys Ser Ala Thr Gly His Pro Pro Pro Arg Ile
                380                 385                 390

Ser Trp Thr Arg Gly Asp Arg Thr Pro Leu Pro Val Asp Pro Arg
                395                 400                 405

Val Asn Ile Thr Pro Ser Gly Gly Leu Tyr Ile Gln Asn Val Val
                410                 415                 420

Gln Gly Asp Ser Gly Glu Tyr Ala Cys Ser Ala Thr Asn Asn Ile
                425                 430                 435

Asp Ser Val His Ala Thr Ala Phe Ile Ile Val Gln Ala Leu Pro
                440                 445                 450
```

Gln Phe Thr Val Thr Pro Gln Asp Arg Val Val Ile Glu Gly Gln
455 460 465

Thr Val Asp Phe Gln Cys Glu Ala Lys Gly Asn Pro Pro Pro Val
470 475 480

Ile Ala Trp Thr Lys Gly Gly Ser Gln Leu Ser Val Asp Arg Arg
485 490 495

His Leu Val Leu Ser Ser Gly Thr Leu Arg Ile Ser Gly Val Ala
500 505 510

Leu His Asp Gln Gly Gln Tyr Glu Cys Gln Ala Val Asn Ile Ile
515 520 525

Gly Ser Gln Lys Val Val Ala His Leu Thr Val Gln Pro Arg Val
530 535 540

Thr Pro Val Phe Ala Ser Ile Pro Ser Asp Thr Thr Val Glu Val
545 550 555

Gly Ala Asn Val Gln Leu Pro Cys Ser Ser Gln Gly Glu Pro Glu
560 565 570

Pro Ala Ile Thr Trp Asn Lys Asp Gly Val Gln Val Thr Glu Ser
575 580 585

Gly Lys Phe His Ile Ser Pro Glu Gly Phe Leu Thr Ile Asn Asp
590 595 600

Val Gly Pro Ala Asp Ala Gly Arg Tyr Glu Cys Val Ala Arg Asn
605 610 615

Thr Ile Gly Ser Ala Ser Val Ser Met Val Leu Ser Val Asn Val
620 625 630

Pro Asp Val Ser Arg Asn Gly Asp Pro Phe Val Ala Thr Ser Ile
635 640 645

Val Glu Ala Ile Ala Thr Val Asp Arg Ala Ile Asn Ser Thr Arg
650 655 660

Thr His Leu Phe Asp Ser Arg Pro Arg Ser Pro Asn Asp Leu Leu
665 670 675

Ala Leu Phe Arg Tyr Pro Arg Asp Pro Tyr Thr Val Glu Gln Ala
680 685 690

Arg Ala Gly Glu Ile Phe Glu Arg Thr Leu Gln Leu Ile Gln Glu
695 700 705

His Val Gln His Gly Leu Met Val Asp Leu Asn Gly Thr Ser Tyr
710 715 720

His Tyr Asn Asp Leu Val Ser Pro Gln Tyr Leu Asn Leu Ile Ala
725 730 735

Asn Leu Ser Gly Cys Thr Ala His Arg Arg Val Asn Asn Cys Ser
740 745 750

Asp Met Cys Phe His Gln Lys Tyr Arg Thr His Asp Gly Thr Cys

755 760 765

Asn Asn Leu Gln His Pro Met Trp Gly Ala Ser Leu Thr Ala Phe
770 775 780

Glu Arg Leu Leu Lys Ser Val Tyr Glu Asn Gly Phe Asn Thr Pro
785 790 795

Arg Gly Ile Asn Pro His Arg Leu Tyr Asn Gly His Ala Leu Pro
800 805 810

Met Pro Arg Leu Val Ser Thr Thr Leu Ile Gly Thr Glu Thr Val
815 820 825

Thr Pro Asp Glu Gln Phe Thr His Met Leu Met Gln Trp Gly Gln
830 835 840

Phe Leu Asp His Asp Leu Asp Ser Thr Val Val Ala Leu Ser Gln
845 850 855

Ala Arg Phe Ser Asp Gly Gln His Cys Ser Asn Val Cys Ser Asn
860 865 870

Asp Pro Pro Cys Phe Ser Val Met Ile Pro Pro Asn Asp Ser Arg
875 880 885

Ala Arg Ser Gly Ala Arg Cys Met Phe Phe Val Arg Ser Ser Pro
890 895 900

Val Cys Gly Ser Gly Met Thr Ser Leu Leu Met Asn Ser Val Tyr
905 910 915

Pro Arg Glu Gln Ile Asn Gln Leu Thr Ser Tyr Ile Asp Ala Ser
920 925 930

Asn Val Tyr Gly Ser Thr Glu His Glu Ala Arg Ser Ile Arg Asp
935 940 945

Leu Ala Ser His Arg Gly Leu Leu Arg Gln Gly Ile Val Gln Arg
950 955 960

Ser Gly Lys Pro Leu Leu Pro Phe Ala Thr Gly Pro Pro Thr Glu
965 970 975

Cys Met Arg Asp Glu Asn Glu Ser Pro Ile Pro Cys Phe Leu Ala
980 985 990

Gly Asp His Arg Ala Asn Glu Gln Leu Gly Leu Thr Ser Met His
995 1000 1005

Thr Leu Trp Phe Arg Glu His Asn Arg Ile Ala Thr Glu Leu Leu
1010 1015 1020

Lys Leu Asn Pro His Trp Asp Gly Asp Thr Ile Tyr Tyr Glu Thr
1025 1030 1035

Arg Lys Ile Val Gly Ala Glu Ile Gln His Ile Thr Tyr Gln His
1040 1045 1050

Trp Leu Pro Lys Ile Leu Gly Glu Val Gly Met Arg Thr Leu Gly
1055 1060 1065

```
Glu Tyr His Gly Tyr Asp Pro Gly Ile Asn Ala Gly Ile Phe Asn
                1070                1075                1080

Ala Phe Ala Thr Ala Ala Phe Arg Phe Gly His Thr Leu Val Asn
                1085                1090                1095

Pro Leu Leu Tyr Arg Leu Asp Glu Asn Phe Gln Pro Ile Ala Gln
                1100                1105                1110

Asp His Leu Pro Leu His Lys Ala Phe Phe Ser Pro Phe Arg Ile
                1115                1120                1125

Val Asn Glu Gly Gly Ile Asp Pro Leu Leu Arg Gly Leu Phe Gly
                1130                1135                1140

Val Ala Gly Lys Met Arg Val Pro Ser Gln Leu Leu Asn Thr Glu
                1145                1150                1155

Leu Thr Glu Arg Leu Phe Ser Met Ala His Thr Val Ala Leu Asp
                1160                1165                1170

Leu Ala Ala Ile Asn Ile Gln Arg Gly Arg Asp His Gly Ile Pro
                1175                1180                1185

Pro Tyr His Asp Tyr Arg Val Tyr Cys Asn Leu Ser Ala Ala His
                1190                1195                1200

Thr Phe Glu Asp Leu Lys Asn Glu Ile Lys Asn Pro Glu Ile Arg
                1205                1210                1215

Glu Lys Leu Lys Arg Leu Tyr Gly Ser Thr Leu Asn Ile Asp Leu
                1220                1225                1230

Phe Pro Ala Leu Val Val Glu Asp Leu Val Pro Gly Ser Arg Leu
                1235                1240                1245

Gly Pro Thr Leu Met Cys Leu Leu Ser Thr Gln Phe Lys Arg Leu
                1250                1255                1260

Arg Asp Gly Asp Arg Leu Trp Tyr Glu Asn Pro Gly Val Phe Ser
                1265                1270                1275

Pro Ala Gln Leu Thr Gln Ile Lys Gln Thr Ser Leu Ala Arg Ile
                1280                1285                1290

Leu Cys Asp Asn Ala Asp Asn Ile Thr Arg Val Gln Ser Asp Val
                1295                1300                1305

Phe Arg Val Ala Glu Phe Pro His Gly Tyr Gly Ser Cys Asp Glu
                1310                1315                1320

Ile Pro Arg Val Asp Leu Arg Val Trp Gln Asp Cys Cys Glu Asp
                1325                1330                1335

Cys Arg Thr Arg Gly Gln Phe Asn Ala Phe Ser Tyr His Phe Arg
                1340                1345                1350

Gly Arg Arg Ser Leu Glu Phe Ser Tyr Gln Glu Asp Lys Pro Thr
                1355                1360                1365
```

Lys Lys Thr Arg Pro Arg Lys Ile Pro Ser Val Gly Arg Gln Gly
1370 1375 1380

Glu His Leu Ser Asn Ser Thr Ser Ala Phe Ser Thr Arg Ser Asp
1385 1390 1395

Ala Ser Gly Thr Asn Asp Phe Arg Glu Phe Val Leu Glu Met Gln
1400 1405 1410

Lys Thr Ile Thr Asp Leu Arg Thr Gln Ile Lys Lys Leu Glu Ser
1415 1420 1425

Arg Leu Ser Thr Thr Glu Cys Val Asp Ala Gly Gly Glu Ser His
1430 1435 1440

Ala Asn Asn Thr Lys Trp Lys Lys Asp Ala Cys Thr Ile Cys Glu
1445 1450 1455

Cys Lys Asp Gly Gln Val Thr Cys Phe Val Glu Ala Cys Pro Pro
1460 1465 1470

Ala Thr Cys Ala Val Pro Val Asn Ile Pro Gly Ala Cys Cys Pro
1475 1480 1485

Val Cys Leu Gln Lys Arg Ala Glu Glu Lys Pro
1490 1495

<210> 29
<211> 2201
<212> PRT
<213> Homo sapien

<400> 29
Met Pro Ser Ala Gly Thr Leu Pro Trp Val Gln Gly Ile Ile Cys
1 5 10 15

Asn Ala Asn Asn Pro Cys Phe Arg Tyr Pro Thr Pro Gly Glu Ala
20 25 30

Pro Gly Val Val Gly Asn Phe Asn Lys Ser Ile Val Ala Arg Leu
35 40 45

Phe Ser Asp Ala Arg Arg Leu Leu Leu Tyr Ser Gln Lys Asp Thr
50 55 60

Ser Met Lys Asp Met Arg Lys Val Leu Arg Thr Leu Gln Gln Ile
65 70 75

Lys Lys Ser Ser Ser Asn Leu Lys Leu Gln Asp Phe Leu Val Asp
80 85 90

Asn Glu Thr Phe Ser Gly Phe Leu Tyr His Asn Leu Ser Leu Pro
95 100 105

Lys Ser Thr Val Asp Lys Met Leu Arg Ala Asp Val Ile Leu His
110 115 120

Lys Val Phe Leu Gln Gly Tyr Gln Leu His Leu Thr Ser Leu Cys
125 130 135

Asn Gly Ser Lys Ser Glu Glu Met Ile Gln Leu Gly Asp Gln Glu

140 145 150

Val Ser Glu Leu Cys Gly Leu Pro Arg Glu Lys Leu Ala Ala Ala
155 160 165

Glu Arg Val Leu Arg Ser Asn Met Asp Ile Leu Lys Pro Ile Leu
170 175 180

Arg Thr Leu Asn Ser Thr Ser Pro Phe Pro Ser Lys Glu Leu Ala
185 190 195

Glu Ala Thr Lys Thr Leu Leu His Ser Leu Gly Thr Leu Ala Gln
200 205 210

Glu Leu Phe Ser Met Arg Ser Trp Ser Asp Met Arg Gln Glu Val
215 220 225

Met Phe Leu Thr Asn Val Asn Ser Ser Ser Ser Ser Thr Gln Ile
230 235 240

Tyr Gln Ala Val Ser Arg Ile Val Cys Gly His Pro Glu Gly Gly
245 250 255

Gly Leu Lys Ile Lys Ser Leu Asn Trp Tyr Glu Asp Asn Asn Tyr
260 265 270

Lys Ala Leu Phe Gly Gly Asn Gly Thr Glu Glu Asp Ala Glu Thr
275 280 285

Phe Tyr Asp Asn Ser Thr Thr Pro Tyr Cys Asn Asp Leu Met Lys
290 295 300

Asn Leu Glu Ser Ser Pro Leu Ser Arg Ile Ile Trp Lys Ala Leu
305 310 315

Lys Pro Leu Leu Val Gly Lys Ile Leu Tyr Thr Pro Asp Thr Pro
320 325 330

Ala Thr Arg Gln Val Met Ala Glu Val Asn Lys Thr Phe Gln Glu
335 340 345

Leu Ala Val Phe His Asp Leu Glu Gly Met Trp Glu Glu Leu Ser
350 355 360

Pro Lys Ile Trp Thr Phe Met Glu Asn Ser Gln Glu Met Asp Leu
365 370 375

Val Arg Met Leu Leu Asp Ser Arg Asp Asn Asp His Phe Trp Glu
380 385 390

Gln Gln Leu Asp Gly Leu Asp Trp Thr Ala Gln Asp Ile Val Ala
395 400 405

Phe Leu Ala Lys His Pro Glu Asp Val Gln Ser Ser Asn Gly Ser
410 415 420

Val Tyr Thr Trp Arg Glu Ala Phe Asn Glu Thr Asn Gln Ala Ile
425 430 435

Arg Thr Ile Ser Arg Phe Met Glu Cys Val Asn Leu Asn Lys Leu
440 445 450

```
Glu Pro Ile Ala Thr Glu Val Trp Leu Ile Asn Lys Ser Met Glu
                455                 460                 465

Leu Leu Asp Glu Arg Lys Phe Trp Ala Gly Ile Val Phe Thr Gly
                470                 475                 480

Ile Thr Pro Gly Ser Ile Glu Leu Pro His His Val Lys Tyr Lys
                485                 490                 495

Ile Arg Met Asp Ile Asp Asn Val Glu Arg Thr Asn Lys Ile Lys
                500                 505                 510

Asp Gly Tyr Trp Asp Pro Gly Pro Arg Ala Asp Pro Phe Glu Asp
                515                 520                 525

Met Arg Tyr Val Trp Gly Gly Phe Ala Tyr Leu Gln Asp Val Val
                530                 535                 540

Glu Gln Ala Ile Ile Arg Val Leu Thr Gly Thr Glu Lys Lys Thr
                545                 550                 555

Gly Val Tyr Met Gln Gln Met Pro Tyr Pro Cys Tyr Val Asp Asp
                560                 565                 570

Ile Phe Leu Arg Val Met Ser Arg Ser Met Pro Leu Phe Met Thr
                575                 580                 585

Leu Ala Trp Ile Tyr Ser Val Ala Val Ile Ile Lys Gly Ile Val
                590                 595                 600

Tyr Glu Lys Glu Ala Arg Leu Lys Glu Thr Met Arg Ile Met Gly
                605                 610                 615

Leu Asp Asn Ser Ile Leu Trp Phe Ser Trp Phe Ile Ser Ser Leu
                620                 625                 630

Ile Pro Leu Leu Val Ser Ala Gly Leu Leu Val Val Ile Leu Lys
                635                 640                 645

Leu Gly Asn Leu Leu Pro Tyr Ser Asp Pro Ser Val Val Phe Val
                650                 655                 660

Phe Leu Ser Val Phe Ala Val Val Thr Ile Leu Gln Cys Phe Leu
                665                 670                 675

Ile Ser Thr Leu Phe Ser Arg Ala Asn Leu Ala Ala Ala Cys Gly
                680                 685                 690

Gly Ile Ile Tyr Phe Thr Leu Tyr Leu Pro Tyr Val Leu Cys Val
                695                 700                 705

Ala Trp Gln Asp Tyr Val Gly Phe Thr Leu Lys Ile Phe Ala Ser
                710                 715                 720

Leu Leu Ser Pro Val Ala Phe Gly Phe Gly Cys Glu Tyr Phe Ala
                725                 730                 735

Leu Phe Glu Glu Gln Gly Ile Gly Val Gln Trp Asp Asn Leu Phe
                740                 745                 750
```

Glu Ser Pro Val Glu Glu Asp Gly Phe Asn Leu Thr Thr Ser Val
755 760 765

Ser Met Met Leu Phe Asp Thr Phe Leu Tyr Gly Val Met Thr Trp
770 775 780

Tyr Ile Glu Ala Val Phe Pro Gly Gln Tyr Gly Ile Pro Arg Pro
785 790 795

Trp Tyr Phe Pro Cys Thr Lys Ser Tyr Trp Phe Gly Glu Glu Ser
800 805 810

Asp Glu Lys Ser His Pro Gly Ser Asn Gln Lys Arg Ile Ser Glu
815 820 825

Ile Cys Met Glu Glu Glu Pro Thr His Leu Lys Leu Gly Val Ser
830 835 840

Ile Gln Asn Leu Val Lys Val Tyr Arg Asp Gly Met Lys Val Ala
845 850 855

Val Asp Gly Leu Ala Leu Asn Phe Tyr Glu Gly Gln Ile Thr Ser
860 865 870

Phe Leu Gly His Asn Gly Ala Gly Lys Thr Thr Thr Met Ser Ile
875 880 885

Leu Thr Gly Leu Phe Pro Pro Thr Ser Gly Thr Ala Tyr Ile Leu
890 895 900

Gly Lys Asp Ile Arg Ser Glu Met Ser Thr Ile Arg Gln Asn Leu
905 910 915

Gly Val Cys Pro Gln His Asn Val Leu Phe Asp Met Leu Thr Val
920 925 930

Glu Glu His Ile Trp Phe Tyr Ala Arg Leu Lys Gly Leu Ser Glu
935 940 945

Lys His Val Lys Ala Glu Met Glu Gln Met Ala Leu Asp Val Gly
950 955 960

Leu Pro Ser Ser Lys Leu Lys Ser Lys Thr Ser Gln Leu Ser Gly
965 970 975

Gly Met Gln Arg Lys Leu Ser Val Ala Leu Ala Phe Val Gly Gly
980 985 990

Ser Lys Val Val Ile Leu Asp Glu Pro Thr Ala Gly Val Asp Pro
995 1000 1005

Tyr Ser Arg Arg Gly Ile Trp Glu Leu Leu Leu Lys Tyr Arg Gln
1010 1015 1020

Gly Arg Thr Ile Ile Leu Ser Thr His His Met Asp Glu Ala Asp
1025 1030 1035

Val Leu Gly Asp Arg Ile Ala Ile Ile Ser His Gly Lys Leu Cys
1040 1045 1050

Cys Val Gly Ser Ser Leu Phe Leu Lys Asn Gln Leu Gly Thr Gly

1055 1060 1065

Tyr Tyr Leu Thr Leu Val Lys Lys Asp Val Glu Ser Ser Leu Ser
1070 1075 1080

Ser Cys Arg Asn Ser Ser Ser Thr Val Ser Tyr Leu Lys Lys Glu
1085 1090 1095

Asp Ser Val Ser Gln Ser Ser Ser Asp Ala Gly Leu Gly Ser Asp
1100 1105 1110

His Glu Ser Asp Thr Leu Thr Ile Asp Val Ser Ala Ile Ser Asn
1115 1120 1125

Leu Ile Arg Lys His Val Ser Glu Ala Arg Leu Val Glu Asp Ile
1130 1135 1140

Gly His Glu Leu Thr Tyr Val Leu Pro Tyr Glu Ala Ala Lys Glu
1145 1150 1155

Gly Ala Phe Val Glu Leu Phe His Glu Ile Asp Asp Arg Leu Ser
1160 1165 1170

Asp Leu Gly Ile Ser Ser Tyr Gly Ile Ser Glu Thr Thr Leu Glu
1175 1180 1185

Glu Ile Phe Leu Lys Val Ala Glu Glu Ser Gly Val Asp Ala Glu
1190 1195 1200

Thr Ser Asp Gly Thr Leu Pro Ala Arg Arg Asn Arg Arg Ala Phe
1205 1210 1215

Gly Asp Lys Gln Ser Cys Leu Arg Pro Phe Thr Glu Asp Asp Ala
1220 1225 1230

Ala Asp Pro Asn Asp Ser Asp Ile Asp Pro Glu Ser Arg Glu Thr
1235 1240 1245

Asp Leu Leu Ser Gly Met Asp Gly Lys Gly Ser Tyr Gln Val Lys
1250 1255 1260

Gly Trp Lys Leu Thr Gln Gln Gln Phe Val Ala Leu Leu Trp Lys
1265 1270 1275

Arg Leu Leu Ile Ala Arg Arg Ser Arg Lys Gly Phe Phe Ala Gln
1280 1285 1290

Ile Val Leu Pro Ala Val Phe Val Cys Ile Ala Leu Val Phe Ser
1295 1300 1305

Leu Ile Val Pro Pro Phe Gly Lys Tyr Pro Ser Leu Glu Leu Gln
1310 1315 1320

Pro Trp Met Tyr Asn Glu Gln Tyr Thr Phe Val Ser Asn Asp Ala
1325 1330 1335

Pro Glu Asp Thr Gly Thr Leu Glu Leu Leu Asn Ala Leu Thr Lys
1340 1345 1350

Asp Pro Gly Phe Gly Thr Arg Cys Met Glu Gly Asn Pro Ile Pro
1355 1360 1365

Asp Thr Pro Cys Gln Ala Gly Glu Glu Glu Trp Thr Thr Ala Pro
1370 1375 1380

Val Pro Gln Thr Ile Met Asp Leu Phe Gln Asn Gly Asn Trp Thr
1385 1390 1395

Met Gln Asn Pro Ser Pro Ala Cys Gln Cys Ser Ser Asp Lys Ile
1400 1405 1410

Lys Lys Met Leu Pro Val Cys Pro Pro Gly Ala Gly Gly Leu Pro
1415 1420 1425

Pro Pro Gln Arg Lys Gln Asn Thr Ala Asp Ile Leu Gln Asp Leu
1430 1435 1440

Thr Gly Arg Asn Ile Ser Asp Tyr Leu Val Lys Thr Tyr Val Gln
1445 1450 1455

Ile Ile Ala Lys Ser Leu Lys Asn Lys Ile Trp Val Asn Glu Phe
1460 1465 1470

Arg Tyr Gly Gly Phe Ser Leu Gly Val Ser Asn Thr Gln Ala Leu
1475 1480 1485

Pro Pro Ser Gln Glu Val Asn Asp Ala Thr Lys Gln Met Lys Lys
1490 1495 1500

His Leu Lys Leu Ala Lys Asp Ser Ser Ala Asp Arg Phe Leu Asn
1505 1510 1515

Ser Leu Gly Arg Phe Met Thr Gly Leu Asp Thr Arg Asn Asn Val
1520 1525 1530

Lys Val Trp Phe Asn Asn Lys Gly Trp His Ala Ile Ser Ser Phe
1535 1540 1545

Leu Asn Val Ile Asn Asn Ala Ile Leu Arg Ala Asn Leu Gln Lys
1550 1555 1560

Gly Glu Asn Pro Ser His Tyr Gly Ile Thr Ala Phe Asn His Pro
1565 1570 1575

Leu Asn Leu Thr Lys Gln Gln Leu Ser Glu Val Ala Pro Met Thr
1580 1585 1590

Thr Ser Val Asp Val Leu Val Ser Ile Cys Val Ile Phe Ala Met
1595 1600 1605

Ser Phe Val Pro Ala Ser Phe Val Val Phe Leu Ile Gln Glu Arg
1610 1615 1620

Val Ser Lys Ala Lys His Leu Gln Phe Ile Ser Gly Val Lys Pro
1625 1630 1635

Val Ile Tyr Trp Leu Ser Asn Phe Val Trp Asp Met Cys Asn Tyr
1640 1645 1650

Val Val Pro Ala Thr Leu Val Ile Ile Ile Phe Ile Cys Phe Gln
1655 1660 1665

Gln Lys Ser Tyr Val Ser Ser Thr Asn Leu Pro Val Leu Ala Leu
1670 1675 1680

Leu Leu Leu Leu Tyr Gly Trp Ser Ile Thr Pro Leu Met Tyr Pro
1685 1690 1695

Ala Ser Phe Val Phe Lys Ile Pro Ser Thr Ala Tyr Val Val Leu
1700 1705 1710

Thr Ser Val Asn Leu Phe Ile Gly Ile Asn Gly Ser Val Ala Thr
1715 1720 1725

Phe Val Leu Glu Leu Phe Thr Asp Asn Lys Leu Asn Asn Ile Asn
1730 1735 1740

Asp Ile Leu Lys Ser Val Phe Leu Ile Phe Pro His Phe Cys Leu
1745 1750 1755

Gly Arg Gly Leu Ile Asp Met Val Lys Asn Gln Ala Met Ala Asp
1760 1765 1770

Ala Leu Glu Arg Phe Gly Glu Asn Arg Phe Val Ser Pro Leu Ser
1775 1780 1785

Trp Asp Leu Val Gly Arg Asn Leu Phe Ala Met Ala Val Glu Gly
1790 1795 1800

Val Val Phe Phe Leu Ile Thr Val Leu Ile Gln Tyr Arg Phe Phe
1805 1810 1815

Ile Arg Pro Arg Pro Val Asn Ala Lys Leu Ser Pro Leu Asn Asp
1820 1825 1830

Glu Asp Glu Asp Val Arg Arg Glu Arg Gln Arg Ile Leu Asp Gly
1835 1840 1845

Gly Gly Gln Asn Asp Ile Leu Glu Ile Lys Glu Leu Thr Lys Ile
1850 1855 1860

Tyr Arg Arg Lys Arg Lys Pro Ala Val Asp Arg Ile Cys Val Gly
1865 1870 1875

Ile Pro Pro Gly Glu Cys Phe Gly Leu Leu Gly Val Asn Gly Ala
1880 1885 1890

Gly Lys Ser Ser Thr Phe Lys Met Leu Thr Gly Asp Thr Thr Val
1895 1900 1905

Thr Arg Gly Asp Ala Phe Leu Asn Arg Asn Ser Ile Leu Ser Asn
1910 1915 1920

Ile His Glu Val His Gln Asn Met Gly Tyr Cys Pro Gln Phe Asp
1925 1930 1935

Ala Ile Thr Glu Leu Leu Thr Gly Arg Glu His Val Glu Phe Phe
1940 1945 1950

Ala Leu Leu Arg Gly Val Pro Glu Lys Glu Val Gly Lys Val Gly
1955 1960 1965

Glu Trp Ala Ile Arg Lys Leu Gly Leu Val Lys Tyr Gly Glu Lys

1970 1975 1980

Tyr Ala Gly Asn Tyr Ser Gly Gly Asn Lys Arg Lys Leu Ser Thr
1985 1990 1995

Ala Met Ala Leu Ile Gly Gly Pro Pro Val Val Phe Leu Asp Glu
2000 2005 2010

Pro Thr Thr Gly Met Asp Pro Lys Ala Arg Arg Phe Leu Trp Asn
2015 2020 2025

Cys Ala Leu Ser Val Val Lys Glu Gly Arg Ser Val Val Leu Thr
2030 2035 2040

Ser His Ser Met Glu Glu Cys Glu Ala Leu Cys Thr Arg Met Ala
2045 2050 2055

Ile Met Val Asn Gly Arg Phe Arg Cys Leu Gly Ser Val Gln His
2060 2065 2070

Leu Lys Asn Arg Phe Gly Asp Gly Tyr Thr Ile Val Val Arg Ile
2075 2080 2085

Ala Gly Ser Asn Pro Asp Leu Lys Pro Val Gln Asp Phe Phe Gly
2090 2095 2100

Leu Ala Phe Pro Gly Ser Val Pro Lys Glu Lys His Arg Asn Met
2105 2110 2115

Leu Gln Tyr Gln Leu Pro Ser Ser Leu Ser Ser Leu Ala Arg Ile
2120 2125 2130

Phe Ser Ile Leu Ser Gln Ser Lys Lys Arg Leu His Ile Glu Asp
2135 2140 2145

Tyr Ser Val Ser Gln Thr Thr Leu Asp Gln Val Phe Val Asn Phe
2150 2155 2160

Ala Lys Asp Gln Ser Asp Asp Asp His Leu Lys Asp Leu Ser Leu
2165 2170 2175

His Lys Asn Gln Thr Val Val Asp Val Ala Val Leu Thr Ser Phe
2180 2185 2190

Leu Gln Asp Glu Lys Val Lys Glu Ser Tyr Val
2195 2200

<210> 30
<211> 178
<212> PRT
<213> Homo sapien

<400> 30
Asp Pro Asp Pro Asp Pro Asp Pro Glu Pro Ala Gly Gly Ser Arg
1 5 10 15

Pro Gly Pro Ala Val Pro Gly Leu Arg Ala Leu Leu Pro Ala Arg
20 25 30

Ala Phe Leu Cys Ser Leu Lys Gly Arg Leu Leu Leu Ala Glu Ser
35 40 45

Gly Leu Ser Phe Ile Thr Phe Ile Cys Tyr Val Ala Ser Ser Ala
50 55 60

Ser Ala Phe Leu Thr Ala Pro Leu Leu Glu Phe Leu Leu Ala Leu
65 70 75

Tyr Phe Leu Phe Ala Asp Ala Met Gln Leu Asn Asp Lys Trp Gln
80 85 90

Gly Leu Cys Trp Pro Met Met Asp Phe Leu Arg Cys Val Thr Ala
95 100 105

Ala Leu Ile Tyr Phe Ala Ile Ser Ile Thr Ala Ile Ala Lys Tyr
110 115 120

Ser Asp Gly Ala Ser Lys Ala Ala Gly Val Phe Gly Phe Phe Ala
125 130 135

Thr Ile Val Phe Ala Thr Asp Phe Tyr Leu Ile Phe Asn Asp Val
140 145 150

Ala Lys Phe Leu Lys Gln Gly Asp Ser Ala Asp Glu Thr Thr Ala
155 160 165

His Lys Thr Glu Glu Glu Asn Ser Asp Ser Asp Ser Asp
170 175

<210> 31
<211> 119
<212> PRT
<213> Homo sapien

<400> 31
Met Ser Arg Ser Val Ala Leu Ala Val Leu Ala Leu Leu Ser Leu
1 5 10 15

Ser Gly Leu Glu Ala Ile Gln Arg Thr Pro Lys Ile Gln Val Tyr
20 25 30

Ser Arg His Pro Ala Glu Asn Gly Lys Ser Asn Phe Leu Asn Cys
35 40 45

Tyr Val Ser Gly Phe His Pro Ser Asp Ile Glu Val Asp Leu Leu
50 55 60

Lys Asn Gly Glu Arg Ile Glu Lys Val Glu His Ser Asp Leu Ser
65 70 75

Phe Ser Lys Asp Trp Ser Phe Tyr Leu Leu Tyr Tyr Thr Glu Phe
80 85 90

Thr Pro Thr Glu Lys Asp Glu Tyr Ala Cys Arg Val Asn His Val
95 100 105

Thr Leu Ser Gln Pro Lys Ile Val Lys Trp Asp Arg Asp Met
110 115

<210> 32
<211> 571
<212> PRT

<213> Homo sapien

<400> 32

```
Met Thr Arg Ala Gly Asp His Asn Arg Gln Arg Gly Cys Cys Gly
1               5                   10                  15

Ser Leu Ala Asp Tyr Leu Thr Ser Ala Lys Phe Leu Leu Tyr Leu
                20                  25                  30

Gly His Ser Leu Ser Thr Trp Gly Asp Arg Met Trp His Phe Ala
                35                  40                  45

Val Ser Val Phe Leu Val Glu Leu Tyr Gly Asn Ser Leu Leu Leu
                50                  55                  60

Thr Ala Val Tyr Gly Leu Val Val Ala Gly Ser Val Leu Val Leu
                65                  70                  75

Gly Ala Ile Ile Gly Asp Trp Val Asp Lys Asn Ala Arg Leu Lys
                80                  85                  90

Val Ala Gln Thr Ser Leu Val Val Gln Asn Val Ser Val Ile Leu
                95                  100                 105

Cys Gly Ile Ile Leu Met Met Val Phe Leu His Lys His Glu Leu
                110                 115                 120

Leu Thr Met Tyr His Gly Trp Val Leu Thr Ser Cys Tyr Ile Leu
                125                 130                 135

Ile Ile Thr Ile Ala Asn Ile Ala Asn Leu Ala Ser Thr Ala Thr
                140                 145                 150

Ala Ile Thr Ile Gln Arg Asp Trp Ile Val Val Val Ala Gly Glu
                155                 160                 165

Asp Arg Ser Lys Leu Ala Asn Met Asn Ala Thr Ile Arg Arg Ile
                170                 175                 180

Asp Gln Leu Thr Asn Ile Leu Ala Pro Met Ala Val Gly Gln Ile
                185                 190                 195

Met Thr Phe Gly Ser Pro Val Ile Gly Cys Gly Phe Ile Ser Gly
                200                 205                 210

Trp Asn Leu Val Ser Met Cys Val Glu Tyr Val Leu Leu Trp Lys
                215                 220                 225

Val Tyr Gln Lys Thr Pro Ala Leu Ala Val Lys Ala Gly Leu Lys
                230                 235                 240

Glu Glu Glu Thr Glu Leu Lys Gln Leu Asn Leu His Lys Asp Thr
                245                 250                 255

Glu Pro Lys Pro Leu Glu Gly Thr His Leu Met Gly Val Lys Asp
                260                 265                 270

Ser Asn Ile His Glu Leu Glu His Glu Gln Glu Pro Thr Cys Ala
                275                 280                 285

Ser Gln Met Ala Glu Pro Phe Arg Thr Phe Arg Asp Gly Trp Val
```

290 295 300

Ser Tyr Tyr Asn Gln Pro Val Phe Leu Ala Gly Met Gly Leu Ala
305 310 315

Phe Leu Tyr Met Thr Val Leu Gly Phe Asp Cys Ile Thr Thr Gly
320 325 330

Tyr Ala Tyr Thr Gln Gly Leu Ser Gly Ser Ile Leu Ser Ile Leu
335 340 345

Met Gly Ala Ser Ala Ile Thr Gly Ile Met Gly Thr Val Ala Phe
350 355 360

Thr Trp Leu Arg Arg Lys Cys Gly Leu Val Arg Thr Gly Leu Ile
365 370 375

Ser Gly Leu Ala Gln Leu Ser Cys Leu Ile Leu Cys Val Ile Ser
380 385 390

Val Phe Met Pro Gly Ser Pro Leu Asp Leu Ser Val Ser Pro Phe
395 400 405

Glu Asp Ile Arg Ser Arg Phe Ile Gln Gly Glu Ser Ile Thr Pro
410 415 420

Thr Lys Ile Pro Glu Ile Thr Thr Glu Ile Tyr Met Ser Asn Gly
425 430 435

Ser Asn Ser Ala Asn Ile Val Pro Glu Thr Ser Pro Glu Ser Val
440 445 450

Pro Ile Ile Ser Val Ser Leu Leu Phe Ala Gly Val Ile Ala Ala
455 460 465

Arg Ile Gly Leu Trp Ser Phe Asp Leu Thr Val Thr Gln Leu Leu
470 475 480

Gln Glu Asn Val Ile Glu Ser Glu Arg Gly Ile Ile Asn Gly Val
485 490 495

Gln Asn Ser Met Asn Tyr Leu Leu Asp Leu Leu His Phe Ile Met
500 505 510

Val Ile Leu Ala Pro Asn Pro Glu Ala Phe Gly Leu Leu Val Leu
515 520 525

Ile Ser Val Ser Phe Val Ala Met Gly His Ile Met Tyr Phe Arg
530 535 540

Phe Ala Gln Asn Thr Leu Gly Asn Lys Leu Phe Ala Cys Gly Pro
545 550 555

Asp Ala Lys Glu Val Arg Lys Glu Asn Gln Ala Asn Thr Ser Val
560 565 570

Val

<210> 33
<211> 262

<212> PRT
<213> Homo sapien

<400> 33

```
Met Asp Pro Arg Leu Ser Thr Val Arg Gln Thr Cys Cys Cys Phe
  1               5                  10                  15

Asn Val Arg Ile Ala Thr Thr Ala Leu Ala Ile Tyr His Val Ile
                 20                  25                  30

Met Ser Val Leu Leu Phe Ile Glu His Ser Val Glu Val Ala His
                 35                  40                  45

Gly Lys Ala Ser Cys Lys Leu Ser Gln Met Gly Tyr Leu Arg Ile
                 50                  55                  60

Ala Asp Leu Ile Ser Ser Phe Leu Leu Ile Thr Met Leu Phe Ile
                 65                  70                  75

Ile Ser Leu Ser Leu Leu Ile Gly Val Val Lys Asn Arg Glu Lys
                 80                  85                  90

Tyr Leu Leu Pro Phe Leu Ser Leu Gln Ile Met Asp Tyr Leu Leu
                 95                 100                 105

Cys Leu Leu Thr Leu Leu Gly Ser Tyr Ile Glu Leu Pro Ala Tyr
                110                 115                 120

Leu Lys Leu Ala Ser Arg Ser Arg Ala Ser Ser Ser Lys Phe Pro
                125                 130                 135

Leu Met Thr Leu Gln Leu Leu Asp Phe Cys Leu Ser Ile Leu Thr
                140                 145                 150

Leu Cys Ser Ser Tyr Met Glu Val Pro Thr Tyr Leu Asn Phe Lys
                155                 160                 165

Ser Met Asn His Met Asn Tyr Leu Pro Ser Gln Glu Asp Met Pro
                170                 175                 180

His Asn Gln Phe Ile Lys Met Met Ile Ile Phe Ser Ile Ala Phe
                185                 190                 195

Ile Thr Val Leu Ile Phe Lys Val Tyr Met Phe Lys Cys Val Trp
                200                 205                 210

Arg Cys Tyr Arg Leu Ile Lys Cys Met Asn Ser Val Glu Glu Lys
                215                 220                 225

Arg Asn Ser Lys Met Leu Gln Lys Val Val Leu Pro Ser Tyr Glu
                230                 235                 240

Glu Ala Leu Ser Leu Pro Ser Lys Thr Pro Glu Gly Gly Pro Ala
                245                 250                 255

Pro Pro Pro Tyr Ser Glu Val
                260
```

<210> 34
<211> 193
<212> PRT

<213> Homo sapien

<400> 34

```
Gly Lys Ala Arg Ser Arg Gly Gly Val Glu Pro Ala Gly Pro Gly
1               5                   10                  15

Gly Gly Ser Pro Glu Pro Tyr His Pro Thr Leu Gly Ile Tyr Ala
                20                  25                  30

Arg Cys Ile Arg Asn Pro Gly Val Gln His Phe Gln Arg Asp Thr
                35                  40                  45

Leu Cys Gly Pro Tyr Ala Glu Ser Phe Gly Glu Ile Ala Ser Gly
                50                  55                  60

Phe Trp Gln Ala Thr Ala Ile Phe Leu Ala Val Gly Ile Phe Ile
                65                  70                  75

Leu Cys Met Val Ala Leu Val Ser Val Phe Thr Met Cys Val Gln
                80                  85                  90

Ser Ile Met Lys Lys Ser Ile Phe Asn Val Cys Gly Leu Leu Gln
                95                  100                 105

Gly Ile Ala Gly Leu Phe Leu Ile Leu Gly Leu Ile Leu Tyr Pro
                110                 115                 120

Ala Gly Trp Gly Cys Gln Lys Ala Ile Asp Tyr Cys Gly His Tyr
                125                 130                 135

Ala Ser Ala Tyr Lys Pro Gly Asp Cys Ser Leu Gly Trp Ala Phe
                140                 145                 150

Tyr Thr Ala Ile Gly Gly Thr Val Leu Thr Phe Ile Cys Ala Val
                155                 160                 165

Phe Ser Ala Gln Ala Glu Ile Ala Thr Ser Ser Asp Lys Val Gln
                170                 175                 180

Glu Glu Ile Glu Glu Gly Lys Asn Leu Ile Cys Leu Leu
                185                 190
```

<210> 35
<211> 185
<212> PRT
<213> Homo sapien

<400> 35

```
Met Val Asn Cys Pro His Leu Ser Arg Glu Phe Cys Thr Pro Arg
1               5                   10                  15

Ile Arg Gly Asn Thr Cys Phe Cys Cys Asp Leu Tyr Asn Cys Gly
                20                  25                  30

Asn Arg Val Glu Ile Thr Gly Gly Tyr Tyr Glu Tyr Ile Asp Val
                35                  40                  45

Ser Ser Cys Gln Asp Ile Ile His Leu Tyr His Leu Leu Trp Ser
                50                  55                  60

Ala Thr Ile Leu Asn Ile Val Gly Leu Phe Leu Gly Ile Ile Thr
```

65 70 75

Ala Ala Val Leu Gly Gly Phe Lys Asp Met Asn Pro Thr Leu Pro
80 85 90

Ala Leu Asn Cys Ser Val Glu Asn Thr His Pro Thr Val Ser Tyr
95 100 105

Tyr Ala His Pro Gln Val Ala Ser Tyr Asn Thr Tyr Tyr His Ser
110 115 120

Pro Pro His Leu Pro Pro Tyr Ser Ala Tyr Asp Phe Gln His Ser
125 130 135

Gly Val Phe Pro Ser Ser Pro Pro Ser Gly Leu Ser Asp Glu Pro
140 145 150

Gln Ser Ala Ser Pro Ser Pro Ser Tyr Met Trp Ser Ser Ser Ala
155 160 165

Pro Pro Arg Tyr Ser Pro Pro Tyr Tyr Pro Pro Phe Glu Lys Pro
170 175 180

Pro Pro Tyr Ser Pro
185

<210> 36
<211> 245
<212> PRT
<213> Homo sapien

<220>
<221> X
<222> 233
<223> Unknown base

<400> 36
Met Ala Ser Pro Ser Arg Arg Leu Gln Thr Lys Pro Val Ile Thr
1 5 10 15

Cys Phe Lys Ser Val Leu Leu Ile Tyr Thr Phe Ile Phe Trp Ile
20 25 30

Thr Gly Val Ile Leu Leu Ala Val Gly Ile Trp Gly Lys Val Ser
35 40 45

Leu Glu Asn Tyr Phe Ser Leu Leu Asn Glu Lys Ala Thr Asn Val
50 55 60

Pro Phe Val Leu Ile Ala Thr Gly Thr Val Ile Ile Leu Leu Gly
65 70 75

Thr Phe Gly Cys Phe Ala Thr Cys Arg Ala Ser Ala Trp Met Leu
80 85 90

Lys Leu Tyr Ala Met Phe Leu Thr Leu Val Phe Leu Val Glu Leu
95 100 105

Val Ala Ala Ile Val Gly Phe Val Phe Arg His Glu Ile Lys Asn
110 115 120

Ser Phe Lys Asn Asn Tyr Glu Lys Ala Leu Lys Gln Tyr Asn Ser
125 130 135

Thr Gly Asp Tyr Arg Ser His Ala Val Asp Lys Ile Gln Asn Thr
140 145 150

Leu His Cys Cys Gly Val Thr Asp Tyr Arg Asp Trp Thr Asp Thr
155 160 165

Asn Tyr Tyr Ser Glu Lys Gly Phe Pro Lys Ser Cys Cys Lys Leu
170 175 180

Glu Asp Cys Thr Pro Gln Arg Asp Ala Asp Lys Val Asn Asn Glu
185 190 195

Gly Cys Phe Ile Lys Val Met Thr Ile Ile Glu Ser Glu Met Gly
200 205 210

Val Val Ala Gly Ile Ser Phe Gly Val Ala Cys Phe Gln Leu Ile
215 220 225

Gly Ile Phe Leu Ala Tyr Cys Xaa Ser Arg Ala Ile Thr Asn Asn
230 235 240

Gln Tyr Glu Ile Val
245

<210> 37
<211> 129
<212> PRT
<213> Homo sapien

<400> 37
Met Ala Arg Gly Ser Leu Arg Arg Leu Leu Arg Leu Leu Val Leu
1 5 10 15

Gly Leu Trp Leu Ala Leu Leu Arg Ser Val Ala Gly Glu Gln Ala
20 25 30

Pro Gly Thr Ala Pro Cys Ser Arg Gly Ser Ser Trp Ser Ala Asp
35 40 45

Leu Asp Lys Cys Met Asp Cys Ala Ser Cys Arg Ala Arg Pro His
50 55 60

Ser Asp Phe Cys Leu Gly Cys Ala Ala Ala Pro Pro Ala Pro Phe
65 70 75

Arg Leu Leu Trp Pro Ile Leu Gly Gly Ala Leu Ser Leu Thr Phe
80 85 90

Val Leu Gly Leu Leu Ser Gly Phe Leu Val Trp Arg Arg Cys Arg
95 100 105

Arg Arg Glu Lys Phe Thr Thr Pro Ile Glu Glu Thr Gly Gly Glu
110 115 120

Gly Cys Pro Ala Val Ala Leu Ile Gln
125

<210> 38

<211> 1474
<212> PRT
<213> Homo sapien

<400> 38
Met Gly Lys Asn Lys Leu Leu His Pro Ser Leu Val Leu Leu Leu
1               5                   10                  15

Leu Val Leu Leu Pro Thr Asp Ala Ser Val Ser Gly Lys Pro Gln
                20                  25                  30

Tyr Met Val Leu Val Pro Ser Leu Leu His Thr Glu Thr Thr Glu
                35                  40                  45

Lys Gly Cys Val Leu Leu Ser Tyr Leu Asn Glu Thr Val Thr Val
                50                  55                  60

Ser Ala Ser Leu Glu Ser Val Arg Gly Asn Arg Ser Leu Phe Thr
                65                  70                  75

Asp Leu Glu Ala Glu Asn Asp Val Leu His Cys Val Ala Phe Ala
                80                  85                  90

Val Pro Lys Ser Ser Ser Asn Glu Glu Val Met Phe Leu Thr Val
                95                  100                 105

Gln Val Lys Gly Pro Thr Gln Glu Phe Lys Lys Arg Thr Thr Val
                110                 115                 120

Met Val Lys Asn Glu Asp Ser Leu Val Phe Val Gln Thr Asp Lys
                125                 130                 135

Ser Ile Tyr Lys Pro Gly Gln Thr Val Lys Phe Arg Val Val Ser
                140                 145                 150

Met Asp Glu Asn Phe His Pro Leu Asn Glu Leu Ile Pro Leu Val
                155                 160                 165

Tyr Ile Gln Asp Pro Lys Gly Asn Arg Ile Ala Gln Trp Gln Ser
                170                 175                 180

Phe Gln Leu Glu Gly Gly Leu Lys Gln Phe Ser Phe Pro Leu Ser
                185                 190                 195

Ser Glu Pro Phe Gln Gly Ser Tyr Lys Val Val Val Gln Lys Lys
                200                 205                 210

Ser Gly Gly Arg Thr Glu His Pro Phe Thr Val Glu Glu Phe Val
                215                 220                 225

Leu Pro Lys Phe Glu Val Gln Val Thr Val Pro Lys Ile Ile Thr
                230                 235                 240

Ile Leu Glu Glu Glu Met Asn Val Ser Val Cys Gly Leu Tyr Thr
                245                 250                 255

Tyr Gly Lys Pro Val Pro Gly His Val Thr Val Ser Ile Cys Arg
                260                 265                 270

Lys Tyr Ser Asp Ala Ser Asp Cys His Gly Glu Asp Ser Gln Ala
                275                 280                 285

Phe Cys Glu Lys Phe Ser Gly Gln Leu Asn Ser His Gly Cys Phe
290 295 300

Tyr Gln Gln Val Lys Thr Lys Val Phe Gln Leu Lys Arg Lys Glu
305 310 315

Tyr Glu Met Lys Leu His Thr Glu Ala Gln Ile Gln Glu Glu Gly
320 325 330

Thr Val Val Glu Leu Thr Gly Arg Gln Ser Ser Glu Ile Thr Arg
335 340 345

Thr Ile Thr Lys Leu Ser Phe Val Lys Val Asp Ser His Phe Arg
350 355 360

Gln Gly Ile Pro Phe Phe Gly Gln Val Arg Leu Val Asp Gly Lys
365 370 375

Gly Val Pro Ile Pro Asn Lys Val Ile Phe Ile Arg Gly Asn Glu
380 385 390

Ala Asn Tyr Tyr Ser Asn Ala Thr Thr Asp Glu His Gly Leu Val
395 400 405

Gln Phe Ser Ile Asn Thr Thr Asn Val Met Gly Thr Ser Leu Thr
410 415 420

Val Arg Val Asn Tyr Lys Asp Arg Ser Pro Cys Tyr Gly Tyr Gln
425 430 435

Trp Val Ser Glu Glu His Glu Glu Ala His His Thr Ala Tyr Leu
440 445 450

Val Phe Ser Pro Ser Lys Ser Phe Val His Leu Glu Pro Met Ser
455 460 465

His Glu Leu Pro Cys Gly His Thr Gln Thr Val Gln Ala His Tyr
470 475 480

Ile Leu Asn Gly Gly Thr Leu Leu Gly Leu Lys Lys Leu Ser Phe
485 490 495

Tyr Tyr Leu Ile Met Ala Lys Gly Gly Ile Val Arg Thr Gly Thr
500 505 510

His Gly Leu Leu Val Lys Gln Glu Asp Met Lys Gly His Phe Ser
515 520 525

Ile Ser Ile Pro Val Lys Ser Asp Ile Ala Pro Val Ala Arg Leu
530 535 540

Leu Ile Tyr Ala Val Leu Pro Thr Gly Asp Val Ile Gly Asp Ser
545 550 555

Ala Lys Tyr Asp Val Glu Asn Cys Leu Ala Asn Lys Val Asp Leu
560 565 570

Ser Phe Ser Pro Ser Gln Ser Leu Pro Ala Ser His Ala His Leu
575 580 585

Arg Val Thr Ala Ala Pro Gln Ser Val Cys Ala Leu Arg Ala Val
590 595 600

Asp Gln Ser Val Leu Leu Met Lys Pro Asp Ala Glu Leu Ser Ala
605 610 615

Ser Ser Val Tyr Asn Leu Leu Pro Glu Lys Asp Leu Thr Gly Phe
620 625 630

Pro Gly Pro Leu Asn Asp Gln Asp Asp Glu Asp Cys Ile Asn Arg
635 640 645

His Asn Val Tyr Ile Asn Gly Ile Thr Tyr Thr Pro Val Ser Ser
650 655 660

Thr Asn Glu Lys Asp Met Tyr Ser Phe Leu Glu Asp Met Gly Leu
665 670 675

Lys Ala Phe Thr Asn Ser Lys Ile Arg Lys Pro Lys Met Cys Pro
680 685 690

Gln Leu Gln Gln Tyr Glu Met His Gly Pro Glu Gly Leu Arg Val
695 700 705

Gly Phe Tyr Glu Ser Asp Val Met Gly Arg Gly His Ala Arg Leu
710 715 720

Val His Val Glu Glu Pro His Thr Glu Thr Val Arg Lys Tyr Phe
725 730 735

Pro Glu Thr Trp Ile Trp Asp Leu Val Val Val Asn Ser Ala Gly
740 745 750

Val Ala Glu Val Gly Val Thr Val Pro Asp Thr Ile Thr Glu Trp
755 760 765

Lys Ala Gly Ala Phe Cys Leu Ser Glu Asp Ala Gly Leu Gly Ile
770 775 780

Ser Ser Thr Ala Ser Leu Arg Ala Phe Gln Pro Phe Phe Val Glu
785 790 795

Leu Thr Met Pro Tyr Ser Val Ile Arg Gly Glu Ala Phe Thr Leu
800 805 810

Lys Ala Thr Val Leu Asn Tyr Leu Pro Lys Cys Ile Arg Val Ser
815 820 825

Val Gln Leu Glu Ala Ser Pro Ala Phe Leu Ala Val Pro Val Glu
830 835 840

Lys Glu Gln Ala Pro His Cys Ile Cys Ala Asn Gly Arg Gln Thr
845 850 855

Val Ser Trp Ala Val Thr Pro Lys Ser Leu Gly Asn Val Asn Phe
860 865 870

Thr Val Ser Ala Glu Ala Leu Glu Ser Gln Glu Leu Cys Gly Thr
875 880 885

Glu Val Pro Ser Val Pro Glu His Gly Arg Lys Asp Thr Val Ile

890 895 900

Lys Pro Leu Leu Val Glu Pro Glu Gly Leu Glu Lys Glu Thr Thr
905 910 915

Phe Asn Ser Leu Leu Cys Pro Ser Gly Gly Glu Val Ser Glu Glu
920 925 930

Leu Ser Leu Lys Leu Pro Pro Asn Val Val Glu Glu Ser Ala Arg
935 940 945

Ala Ser Val Ser Val Leu Gly Asp Ile Leu Gly Ser Ala Met Gln
950 955 960

Asn Thr Gln Asn Leu Leu Gln Met Pro Tyr Gly Cys Gly Glu Gln
965 970 975

Asn Met Val Leu Phe Ala Pro Asn Ile Tyr Val Leu Asp Tyr Leu
980 985 990

Asn Glu Thr Gln Gln Leu Thr Pro Glu Val Lys Ser Lys Ala Ile
995 1000 1005

Gly Tyr Leu Asn Thr Gly Tyr Gln Arg Gln Leu Asn Tyr Lys His
1010 1015 1020

Tyr Asp Gly Ser Tyr Ser Thr Phe Gly Glu Arg Tyr Gly Arg Asn
1025 1030 1035

Gln Gly Asn Thr Trp Leu Thr Ala Phe Val Leu Lys Thr Phe Ala
1040 1045 1050

Gln Ala Arg Ala Tyr Ile Phe Ile Asp Glu Ala His Ile Thr Gln
1055 1060 1065

Ala Leu Ile Trp Leu Ser Gln Arg Gln Lys Asp Asn Gly Cys Phe
1070 1075 1080

Arg Ser Ser Gly Ser Leu Leu Asn Asn Ala Ile Lys Gly Gly Val
1085 1090 1095

Glu Asp Glu Val Thr Leu Ser Ala Tyr Ile Thr Ile Ala Leu Leu
1100 1105 1110

Glu Ile Pro Leu Thr Val Thr His Pro Val Val Arg Asn Ala Leu
1115 1120 1125

Phe Cys Leu Glu Ser Ala Trp Lys Thr Ala Gln Glu Gly Asp His
1130 1135 1140

Gly Ser His Val Tyr Thr Lys Ala Leu Leu Ala Tyr Ala Phe Ala
1145 1150 1155

Leu Ala Gly Asn Gln Asp Lys Arg Lys Glu Val Leu Lys Ser Leu
1160 1165 1170

Asn Glu Glu Ala Val Lys Lys Asp Asn Ser Val His Trp Glu Arg
1175 1180 1185

Pro Gln Lys Pro Lys Ala Pro Val Gly His Phe Tyr Glu Pro Gln
1190 1195 1200

Ala Pro Ser Ala Glu Val Glu Met Thr Ser Tyr Val Leu Leu Ala
1205 1210 1215

Tyr Leu Thr Ala Gln Pro Ala Pro Thr Ser Glu Asp Leu Thr Ser
1220 1225 1230

Ala Thr Asn Ile Val Lys Trp Ile Thr Lys Gln Gln Asn Ala Gln
1235 1240 1245

Gly Gly Phe Ser Ser Thr Gln Asp Thr Val Val Ala Leu His Ala
1250 1255 1260

Leu Ser Lys Tyr Gly Ala Ala Thr Phe Thr Arg Thr Gly Lys Ala
1265 1270 1275

Ala Gln Val Thr Ile Gln Ser Ser Gly Thr Phe Ser Ser Lys Phe
1280 1285 1290

Gln Val Asp Asn Asn Asn Arg Leu Leu Leu Gln Gln Val Ser Leu
1295 1300 1305

Pro Glu Leu Pro Gly Glu Tyr Ser Met Lys Val Thr Gly Glu Gly
1310 1315 1320

Cys Val Tyr Leu Gln Thr Ser Leu Lys Tyr Asn Ile Leu Pro Glu
1325 1330 1335

Lys Glu Glu Phe Pro Phe Ala Leu Gly Val Gln Thr Leu Pro Gln
1340 1345 1350

Thr Cys Asp Glu Pro Lys Ala His Thr Ser Phe Gln Ile Ser Leu
1355 1360 1365

Ser Val Ser Tyr Thr Gly Ser Arg Ser Ala Ser Asn Met Ala Ile
1370 1375 1380

Val Asp Val Lys Met Val Ser Gly Phe Ile Pro Leu Lys Pro Thr
1385 1390 1395

Val Lys Met Leu Glu Arg Ser Asn His Val Ser Arg Thr Glu Val
1400 1405 1410

Ser Ser Asn His Val Leu Ile Tyr Leu Asp Lys Val Ser Asn Gln
1415 1420 1425

Thr Leu Ser Leu Phe Phe Thr Val Leu Gln Asp Val Pro Val Arg
1430 1435 1440

Asp Leu Lys Pro Ala Ile Val Lys Val Tyr Asp Tyr Tyr Glu Thr
1445 1450 1455

Asp Glu Phe Ala Ile Ala Glu Tyr Asn Ala Pro Cys Ser Lys Asp
1460 1465 1470

Leu Gly Asn Ala

<210> 39
<211> 597
<212> PRT

<213> Homo sapien

<400> 39
Met Ala Ala Glu Thr Leu Leu Ser Ser Leu Leu Gly Leu Leu Leu
1               5                   10                  15

Leu Gly Leu Leu Leu Pro Ala Ser Leu Thr Gly Gly Val Gly Ser
                20                  25                  30

Leu Asn Leu Glu Glu Leu Ser Glu Met Arg Tyr Gly Ile Glu Ile
                35                  40                  45

Leu Pro Leu Pro Val Met Gly Gly Gln Ser Gln Ser Ser Asp Val
                50                  55                  60

Val Ile Val Ser Ser Lys Tyr Lys Gln Arg Tyr Glu Cys Arg Leu
                65                  70                  75

Pro Ala Gly Ala Ile His Phe Gln Arg Glu Arg Glu Glu Glu Thr
                80                  85                  90

Pro Ala Tyr Gln Gly Pro Gly Ile Pro Glu Leu Leu Ser Pro Met
                95                  100                 105

Arg Asp Ala Pro Cys Leu Leu Lys Thr Lys Asp Trp Trp Thr Tyr
                110                 115                 120

Glu Phe Cys Tyr Gly Arg His Ile Gln Gln Tyr His Met Glu Asp
                125                 130                 135

Ser Glu Ile Lys Gly Glu Val Leu Tyr Leu Gly Tyr Tyr Gln Ser
                140                 145                 150

Ala Phe Asp Trp Asp Asp Glu Thr Ala Lys Ala Ser Lys Gln His
                155                 160                 165

Arg Leu Lys Arg Tyr His Ser Gln Thr Tyr Gly Asn Gly Ser Lys
                170                 175                 180

Cys Asp Leu Asn Gly Arg Pro Arg Glu Ala Glu Val Arg Phe Leu
                185                 190                 195

Cys Asp Glu Gly Ala Gly Ile Ser Gly Asp Tyr Ile Asp Arg Val
                200                 205                 210

Asp Glu Pro Leu Ser Cys Ser Tyr Val Leu Thr Ile Arg Thr Pro
                215                 220                 225

Arg Leu Cys Pro His Pro Leu Leu Arg Pro Pro Pro Ser Ala Ala
                230                 235                 240

Pro Gln Ala Ile Leu Cys His Pro Ser Leu Gln Pro Glu Glu Tyr
                245                 250                 255

Met Ala Tyr Val Gln Arg Gln Ala Asp Ser Lys Gln Tyr Gly Asp
                260                 265                 270

Lys Ile Ile Glu Glu Leu Gln Asp Leu Gly Pro Gln Val Trp Ser
                275                 280                 285

Glu Thr Lys Ser Gly Val Ala Pro Gln Lys Met Ala Gly Ala Ser

290 295 300

Pro Thr Lys Asp Asp Ser Lys Asp Ser Asp Phe Trp Lys Met Leu
305 310 315

Asn Glu Pro Glu Asp Gln Ala Pro Gly Gly Glu Glu Val Pro Ala
320 325 330

Glu Glu Gln Asp Pro Ser Pro Glu Ala Ala Asp Ser Ala Ser Gly
335 340 345

Ala Pro Asn Asp Phe Gln Asn Asn Val Gln Val Lys Val Ile Arg
350 355 360

Ser Pro Ala Asp Leu Ile Arg Phe Ile Glu Glu Leu Lys Gly Gly
365 370 375

Thr Lys Lys Gly Lys Pro Asn Ile Gly Gln Glu Gln Pro Val Asp
380 385 390

Asp Ala Ala Glu Val Pro Gln Arg Glu Pro Glu Lys Glu Arg Gly
395 400 405

Asp Pro Glu Arg Gln Arg Glu Met Glu Glu Glu Glu Asp Glu Asp
410 415 420

Glu Asp Glu Asp Glu Asp Glu Asp Glu Arg Gln Leu Leu Gly Glu
425 430 435

Phe Glu Lys Glu Leu Glu Gly Ile Leu Leu Pro Ser Asp Arg Asp
440 445 450

Arg Leu Arg Ser Glu Thr Glu Lys Glu Leu Asp Pro Asp Gly Leu
455 460 465

Lys Lys Glu Ser Glu Arg Asp Arg Ala Met Leu Ala Leu Thr Ser
470 475 480

Thr Leu Asn Lys Leu Ile Lys Arg Leu Glu Glu Lys Gln Ser Pro
485 490 495

Glu Leu Val Lys Lys His Lys Lys Lys Arg Val Val Pro Lys Lys
500 505 510

Pro Pro Pro Ser Pro Gln Pro Thr Gly Lys Ile Glu Ile Lys Ile
515 520 525

Val Arg Pro Trp Ala Glu Gly Thr Glu Glu Gly Ala Arg Trp Leu
530 535 540

Thr Asp Glu Asp Thr Arg Asn Leu Lys Glu Ile Phe Phe Asn Ile
545 550 555

Leu Val Pro Gly Ala Glu Glu Ala Gln Lys Glu Arg Gln Arg Gln
560 565 570

Lys Glu Leu Glu Ser Asn Tyr Arg Arg Val Trp Gly Ser Pro Gly
575 580 585

Gly Glu Gly Thr Gly Asp Leu Asp Glu Phe Asp Phe
590 595

<210> 40
<211> 238
<212> PRT
<213> Homo sapien

<400> 40

```
Met Ala Val Glu Gly Gly Met Lys Cys Val Lys Phe Leu Leu Tyr
1               5                   10                  15

Val Leu Leu Leu Ala Phe Cys Ala Cys Ala Val Gly Leu Ile Ala
                20                  25                  30

Val Gly Val Gly Ala Gln Leu Val Leu Ser Gln Thr Ile Ile Gln
                35                  40                  45

Gly Ala Thr Pro Gly Ser Leu Leu Pro Val Val Ile Ile Ala Val
                50                  55                  60

Gly Val Phe Leu Phe Leu Val Ala Phe Val Gly Cys Cys Gly Ala
                65                  70                  75

Cys Lys Glu Asn Tyr Cys Leu Met Ile Thr Phe Ala Ile Phe Leu
                80                  85                  90

Ser Leu Ile Met Leu Val Glu Val Ala Ala Ala Ile Ala Gly Tyr
                95                  100                 105

Val Phe Arg Asp Lys Val Met Ser Glu Phe Asn Asn Asn Phe Arg
                110                 115                 120

Gln Gln Met Glu Asn Tyr Pro Lys Asn Asn His Thr Ala Ser Ile
                125                 130                 135

Leu Asp Arg Met Gln Ala Asp Phe Lys Cys Cys Gly Ala Ala Asn
                140                 145                 150

Tyr Thr Asp Trp Glu Lys Ile Pro Ser Met Ser Lys Asn Arg Val
                155                 160                 165

Pro Asp Ser Cys Cys Ile Asn Val Thr Val Gly Cys Gly Ile Asn
                170                 175                 180

Phe Asn Glu Lys Ala Ile His Lys Glu Gly Cys Val Glu Lys Ile
                185                 190                 195

Gly Gly Trp Leu Arg Lys Asn Val Leu Val Val Ala Ala Ala Ala
                200                 205                 210

Leu Gly Ile Ala Phe Val Glu Val Leu Gly Ile Val Phe Ala Cys
                215                 220                 225

Cys Leu Val Lys Ser Ile Arg Ser Gly Tyr Glu Val Met
                230                 235
```

<210> 41
<211> 228
<212> PRT
<213> Homo sapien

<400> 41

```
Met Pro Val Lys Gly Gly Thr Lys Cys Ile Lys Tyr Leu Leu Phe
 1               5                  10                  15

Gly Phe Asn Phe Ile Phe Trp Leu Ala Gly Ile Ala Val Leu Ala
                20                  25                  30

Ile Gly Leu Trp Leu Arg Phe Asp Ser Gln Thr Lys Ser Ile Phe
                35                  40                  45

Glu Gln Glu Thr Asn Asn Asn Asn Ser Ser Phe Tyr Thr Gly Val
                50                  55                  60

Tyr Ile Leu Ile Gly Ala Gly Ala Leu Met Met Leu Val Gly Phe
                65                  70                  75

Leu Gly Cys Cys Gly Ala Val Gln Glu Ser Gln Cys Met Leu Gly
                80                  85                  90

Leu Phe Phe Gly Phe Leu Leu Val Ile Phe Ala Ile Glu Ile Ala
                95                  100                 105

Ala Ala Ile Trp Gly Tyr Ser His Lys Asp Glu Val Ile Lys Glu
                110                 115                 120

Val Gln Glu Phe Tyr Lys Asp Thr Tyr Asn Lys Leu Lys Thr Lys
                125                 130                 135

Asp Glu Pro Gln Arg Glu Thr Leu Lys Ala Ile His Tyr Ala Leu
                140                 145                 150

Asn Cys Cys Gly Leu Ala Gly Gly Val Glu Gln Phe Ile Ser Asp
                155                 160                 165

Ile Cys Pro Lys Lys Asp Val Leu Glu Thr Phe Thr Val Lys Ser
                170                 175                 180

Cys Pro Asp Ala Ile Lys Glu Val Phe Asp Asn Lys Phe His Ile
                185                 190                 195

Ile Gly Ala Val Gly Ile Gly Ile Ala Val Val Met Ile Phe Gly
                200                 205                 210

Met Ile Phe Ser Met Ile Leu Cys Cys Ala Ile Arg Arg Asn Arg
                215                 220                 225

Glu Met Val
```

<210> 42
<211> 1064
<212> DNA
<213> Homo sapien

<220>
<221> Unsure
<222> 552-579
<223> Unknown base

<400> 42
agccttcctg ctccgagtct ctgcacctcc ctcaggagcc tgtcagcctg 50

gccctcgtga gaggggcgcc agcccagcag cctgctctgg ggcaccctcc 100

cctacctgaa gggcacaggg tttcgggagt tttccaccat gactattgcc 150

ctgctgggtt ttgccatatt cttgctccat tgtgcgacct gtgagaagcc 200

tctagaaggg attctctcct cctctgcttg gcacttcaca cactcccatt 250

acaatgccac catctatgaa aattcttctc ccaagaccta tgtggagagc 300

ttcgagaaaa tgggcatcta cctcgcggag ccacagtggg cagtgaggta 350

ccggatcatc tctggggatg tggccaatgt atttaaaact gaggagtatg 400

tggtgggcaa cttctgcttc ctaagaataa ggacaaagag cagcaacaca 450

gctcttctga acagagaggt gcgagacagc tacaccctca tcatccaagc 500

cacagagaag accttggagt tggaagcttt gacccgtgtg gtggtccaca 550

tnnnnnnnnn nnnnnnnnnn nnnnnnnnng ctgatctagg ccagaatgct 600

gagttctatt atgcctttaa cacaaggtca gagatgtttg ccatccatcc 650

caccagcggt gtggtcactg tggctgggaa gcttaacgtc acctggcgag 700

gaaagcatga gctccaggtg ctagctgtgg accgcatgcg gaaaatctct 750

gagggcaatg ggtttggcag cctggctgca cttgtggttc atgtggagcc 800

tgccctcagg aagcccccag ccattgcttc agtggtggtg actccaccag 850

acagcaatga tggtaccacc tatgccactg tactggtcga tgcaaatagc 900

tcaggagctg aagtggagtc agtggaagtt gttggtggtg accctggaaa 950

gcacttcaaa gccatcaagt cttatgcccg gagcaatgag ttcagtttgg 1000

tgtctgtcaa agacatcaac tggatggagt accttcatgg gttcaacctc 1050

agcctccagg ccag 1064

<210> 43
<211> 6611
<212> DNA
<213> Homo sapien

<400> 43
tgactgcatc acctggtctg tgaattttcc attagaagct tggtgtgctg 50

ttaggtgaaa gacttgctca gctatgcgtc attgggtttt atcaacatat 100

aggcgaaaaa aatcctggtc tctgagtgta cagctgagat gaaaatttct 150

tttattggag gaagtattga gtgtgtgctc tcaaatgcgg cctcagttga 200

gtagtgcatt cctgagtttt ggaagcaaat ttgcaaacaa ttgagagtcg 250

tacagtgggt gttctaactg gattcaggtt ttttctaatg taattttttc 300

acacgtaaat taaaaagttt agaaatgtca cacataactt cataacactt 350

tatggagaaa tggttgtact tttaattttt ttctttttat ttatactcca 400
actgactgag cagaggttgt acttctaaat aactttgtgg aagtttttag 450
taccataatt tttataattt tcattccagt cctttgatat ttatgacagt 500
acttctgaag cgcttactga gtgccggaca ctgttgtaag tgctttacgg 550
aacttgactt tttttttttt ttgagacgga ctctcgctct gtcgcccagg 600
ctggagtgca gtggtgcagt ggctcgatct cggctcactg ccacctctcc 650
ctcatggttt caaacacttc tcctgcctca gcctcccagg tagccaggat 700
tatagccgcc cgccaccact cccgactaat ttttattttgt atgttctttt 750
ttagtagaga cggaggagtt tcaccatgtt ggccaggctg gtatcgacct 800
cctgacctca agtgatgtgt ccatctcggc ctcccaaggt gctggaatta 850
caggtgtgag ccactgtgct cggcctacct tttttttttg ttttttgttt 900
ttttgaaaag gagtttcgct cttgtccagg ctggagtata atggtgcgat 950
ctcagctcac cgcaatctcc gcctcccaga ttcaagcgat tctcctgcct 1000
cagcctcctc aggagctggg attacaggcg cccaccgcca tgcccggcta 1050
atttttgtat ttttagtaga gacggggttt cactatattg gccaggctgg 1100
tctcgaactg ctgacctcaa gtaatccgcc tgcctcagcc tcccaaagtg 1150
ctgggattac agacgtgatc caccaggatc acaccaggcc gcgcctggcc 1200
tgctttcatt ttaaaagtca aatttgtcat ccgcctcagt gcttgtaatc 1250
ttttctgagt gagatactga aatttgcagt ttcgttttgc ttgcacttgt 1300
tcactggacc agtagtcact gttaaatgta aaagtatcta cttcctctga 1350
aagtttttta ttcctttatt tcctgcctgg gcttgtcctc caccctacat 1400
gtatgcgtag tagatttagt gtttgttatc ctaaccttta ggtttaggga 1450
ttgactgggt ttctgacttt ttatttggcc aatgaggacg atacagaaaa 1500
tgaagcattg gtcattatca cattttaacg ctgaaaaagt aagaaggaca 1550
accccggaat aaaatgatat cagtatcaag ataaaagttt ggaatgggag 1600
aaaaattctc aaagcctgaa agaaaatctg tagttacttt tggtgacgct 1650
gtccagttcc cacaatgtat cattccttat ctgaaactag acatcctctg 1700
cagccagaag aacaagaagt aggcattgac cccttgtcca gttactctaa 1750
caagtctgga ggagattcaa ataaaaatgg aagaagaaca agttctactt 1800
tagactctga agggactttt aattcctata ggaaagaatg ggaagaacta 1850

tttgtaaaca acaattactt ggcaacaata aggcagaagg ggattaatgg 1900
gcagctgaga agcagcaggt tccgcagcat ttgctggaag ctatttcttt 1950
gtgttcttcc tcaagacaaa agtcaatgga taagtagaat tgaagaatta 2000
agagcatggt atagcaacat taaagaaata catattacca acccgaggaa 2050
ggttgttggc caacaagatt tgatgatcaa taatcctctt tcacaggatg 2100
aagggagtct ttggaacaaa ttcttccaag ataaagaact tcgatcaatg 2150
attgaacaag atgtcaaaag aacgtttcct gaaatgcagt tttccagca 2200
agaaaatgtg agaaaaattc ttacagatgt tcttttctgt tatgccagag 2250
aaaacgagca gttgctttat aaacagggca tgcacgaact gttagcacct 2300
atagtctttg tccttcactg tgaccaccaa gcttttctac atgccagtga 2350
gtctgcacag cccagtgagg aaatgaaaac tgtcttgaac cctgagtatc 2400
tggaacatga tgcctatgca gtgttctcac aacttatgga aactgctgaa 2450
ccttggtttt caacttttga gcatgatggt cagaagggga aagaaacact 2500
gatgactccc attccctttg ctagaccaca agatttaggg ccaacaattg 2550
ctattgttac taaagtcaac cagatccagg atcatctact gaagaagcat 2600
gatattgagc tttacatgca cttgaacaga ctagaaattg caccacagat 2650
atatgggtta aggtgggtgc ggctgctatt tggacgagag ttccccctgc 2700
aggaccttct ggtggtctgg gatgccttgt ttgcagacgg cctcagcctg 2750
ggtttagtag attatatctt cgtagccatg ttactttaca tccgagatgc 2800
tttgatctct agtaactacc agacctgtct cggccttctg atgcattacc 2850
cattcatcgg ggatgtacac tcactgattc ttaaggctct gttccttaga 2900
gatccaaaga gaaatccaag accagtgact tatcaattcc atccaaattt 2950
agattattac aaagcacgag gagcagacct catgaataaa agccggacca 3000
atgccaaagg tgctccccctg aatataaata aggtctctaa tagcctgatt 3050
aattttggaa gaaagttgat ttccccagca atggctccag gcagtgcagg 3100
tggccctgta cctggaggca acagcagtag ctcctcctct gttgtaattc 3150
ctaccaggac ctcagcagag gccccaagcc atcacttgca acagcaacag 3200
cagcagcaga ggctgatgaa atcagaaagc atgcctgtgc aattgaacaa 3250
agggctaagt tctaaaaaca tcagttcatc tccaagcgtt gagagtttgc 3300
ctggaggaag agaattcact ggctctccac cttcatctgc tactaaaaaa 3350
gattcctttt ttagcaacat ctcacgttct cgctcacaca gcaaaactat 3400

gggcagaaaa gaatctgaag aagaattaga agcccaaatt tccttccttc 3450
aagggcagtt gaatgacctg gatgccatgt gcaaatactg tgcaaaggtg 3500
atggacactc atcttgtaaa tattcaagat gtgatattac aagaaaattt 3550
ggaaaaagaa gatcaaattc tggtttccct ggcaggatta aaacagatca 3600
aagacattct aaaaggttcc ctgcgtttta accagagcca gctagaggcc 3650
gaagagaacg aacagatcac cattgcggac aaccactact gctccagcgg 3700
ccagggccag ggccgaggcc aaggccagag cgttcaaatg tcaggggcca 3750
ttaaacaggc ctcttcagaa acgccagggt gcactgatag agggaattcc 3800
gatgacttca tcctgatttc caaagatgat gatgggagca gtgccagggg 3850
ctccttctcc ggccaggccc agcctcttcg caccctcaga agcacctctg 3900
ggaaaagcca ggccccagtc tgctccccac tggtgttctc agatccactg 3950
atgggcccag cctcagcttc ctccagcaac cccagctcca gtcctgatga 4000
cgacagcagc aaggactctg gcttcaccat tgtgagtccc ctggacatct 4050
gaccacagtg cccagtcctg ccccacaggg atctagccac ccttcagtgg 4100
ccccaaggcc agactgaggc tcatccagtg gagaaccttc ttaaaccact 4150
gcttccttcc cggcatgcat ttggcattgg tccagccctt tgaaacccct 4200
tagagagaag catatatggc cacaaagcac agaggcttag gtttgccaca 4250
tgcagacagg gctttctggg cccttaccta atccccaccc gactcttgct 4300
ctgagttaga gctgagttac gtacccagta tcacactcac agttagaaaa 4350
gaccgaatca caatttagaa tcacttttcc tctgtcccct tctccccagc 4400
taagaatgtg tggcacctcc atcagttata cttagaagga gcagaaatag 4450
ttattttcgt atcttctatc cctcaaagca tcagacatgg gaaaattggt 4500
ttataccaag aaagcttcct ctgtggaaat ctgtctcagc ctactttatt 4550
cctgcattgg gaagccatat cgcagagcta aatgcaatag aatgaaccag 4600
aactagtgga ttccagggct gggggaaaaa aaaaaaagaa aaaacctcat 4650
tactgacctc tcaaagttat aaggatctct gcaaacagga tctaagctta 4700
ggaataatat ttaggtgtga tatagtgtta gatttttttg atgtattaaa 4750
gaatgcatct ccaatcctta ggccatatca actttggcca tcaatatctc 4800
tccttaaaca attatatttc acctttttaga atctttcata gccagaaaac 4850
aagattactg taagccagtt ttagctgcac tgatttcaaa agatataaga 4900

atattactat ccttcaaatg gaaaatgcga ccttgacttt atgggataaa 4950
catctttcag acagtcagtt ttctagtcag gtttctctgg tttcagagct 5000
gtatatacct gtcaactgag gaataaaggg aaaaacccaa gttcattccc 5050
acccaaagtc agaatccctc attggcctta aggtagcagt cataagacag 5100
agaattggac ctagagtccc ttctgtgggg aataaggata cctagagaac 5150
attccacatg ccaagaggat gcaggatttc tacacaaccc cttcccttct 5200
tggaagtcaa gtgtaggtac tgcagggcct gtgctcagct gtgaaccccg 5250
tatcctgggc cccactgccg ggaccgggtc tgacatgcca gtgccttcct 5300
gggctgagca cagattagag actctccccc ttgtcagtca gcaccttagg 5350
aaaccatgat gggcacagag catcacatga gctgtttctc tccttaaaga 5400
agatccctgg aaaggatgct tttcctctcc tttgcctgcg caggaattct 5450
aacaggagtg ggtgaggatg gcagagggac acagtgcctg tctcgcctcc 5500
atcagggaga gcagccatgc cagggatgac tagctctttg agcctgtcct 5550
cagaggatgg cgaggcagcc gggcagtgga ggccttcatg gtaacaaatg 5600
aaagctcagt atagaggaac agacactgtt tacgtccctc ccactgctaa 5650
ccttatatat ctctatagac aaatgtgata atgacatgat ttcccacctg 5700
ccctccaaga aaatggtgac tcactctcaa gtcagctact gtagagaggg 5750
ttctaattgg ttctgcaatt tgctcttaaa ctctagcagg gaactctcct 5800
cttaccacat cagcatgtaa ggtgaataat aactctggtt ttgccagaca 5850
gcaggttgtc tgaccttcaa ccactgggca attgcctggc agatgcacac 5900
agtagctccc tggcttctgg ctctgagtgt tcctctcagc acctctgagt 5950
aagctgctgc caagcacata tccctatgac aacactttgt aaaagccgcg 6000
gggcccccat acagcgagtg accttgcaac tgtgcagggt tgccattggt 6050
cactttctca ccttgggaag gtgtcagtgt tttcagttct aaggtaagag 6100
gtgtagagct gttcccacca gggctctggg acagactgga aaggaccaca 6150
gacctggcca tccctgggca gcagggccag tgtcacctgc tgacctctag 6200
tatttccttt gccctagagc tagagtcatg atagctgagg gtcactcgcc 6250
ctgcaagagt cactaggcac ccaccatgcc aataaggctc tccgctggct 6300
ccctgcagtt ggctgggtgt ttaatagtca ctgaaaactc ccagccctgc 6350
tgcacactag aggcaggtcc tctcggtcct ctccatcctg tgcttctgtg 6400
gcccccagca agctcaccgc ctccttggag gagagagaca tacaaggaca 6450

gtgggtcatg ggtagtacca gcctcaaatt cccacaggct catactcaga 6500

caattgtatt actgccttat gtttttaag tgtttttta aattcttcat 6550

agttgagtat tatttgcaat tttattagtt acagtgctat taaagaatat 6600

gtgctccttt t 6611

<210> 44
<211> 1982
<212> DNA
<213> Homo sapien

<400> 44
tagagaaggc agacgcatcc cgaactcgct ggaggacaag gctcagctct 50

tgccaggcca aattgagaca tgtctgacac aagcgagagt ggtgcaggtc 100

taactcgctt ccaggctgaa gcctcagaaa aggacagtag ctcgatgatg 150

cagactctgt tgacagtgac ccagaatgtg gaggtcccag agacaccgaa 200

ggcctcaaag gcactggagg tctcagagga tgtgaaggtc tcaaaagcct 250

ctggggtctc aaaggccaca gaggtctcaa agaccccaga ggctcgggag 300

gcacctgcca cccaggcctc atctactact cagctgactg atacccaggt 350

tctggcagct gaaaacaaga gtctagcagc tgacaccaag aaacagaatg 400

ctgacccgca ggctgtgaca atgcctgcca ctgagaccaa aaaggtcagc 450

catgtggctg atacaaaggt caatacaaag gctcaggaga ctgaggctgc 500

accctctcag gccccagcag atgaacctga gcctgagagt gcagctgccc 550

agtctcagga gaatcaggat actcggccca aggtcaaagc caagaaagcc 600

cgaaaggtga agcatctgga tggggaagag gatggcagca gtgatcagag 650

tcaggcttct ggaaccacag gtggccgaag ggtctcaaag gccctaatgg 700

cctcaatggc ccgcagggct tcaaggggtc ccatagcctt ttgggcccgc 750

agggcatcaa ggactcggtt ggctgcttgg gcccggagag ccttgctctc 800

cctgagatca cctaaagccc gtaggggcaa ggctcgccgt agagctgcca 850

agctccagtc atcccaagag cctgaagcac caccacctcg ggatgtggcc 900

cttttgcaag ggagggcaaa tgatttggtg aagtaccttt tggctaaaga 950

ccagacgaag attcccatca agcgctcgga catgctgaag gacatcatca 1000

aagaatacac tgatgtgtac cccgaaatca ttgaacgagc aggctattcc 1050

ttggagaagg tatttgggat tcaattgaag gaaattgata agaatgacca 1100

cttgtacatt cttctcagca ccttagagcc cactgatgca ggcatactgg 1150

gaacgactaa ggactcaccc aagctgggtc tgctcatggt gcttcttagc 1200

atcatcttca tgaatggaaa tcggtccagt gaggctgtca tctgggaggt 1250

gctgcgcaag ttggggctgc gccctgggat acatcattca ctctttgggg 1300

acgtgaagaa gctcatcact gatgagtttg tgaagcagaa gtacctggac 1350

tatgccagag tccccaatag caatccccct gaatatgagt tcttctgggg 1400

cctgcgctct tactatgaga ccagcaagat gaaagtcctc aagtttgcct 1450

gcaaggtaca aaagaaggat cccaaggaat gggcagctca gtaccgagag 1500

gcgatggaag cggatttgaa ggctgcagct gaggctgcag ctgaagccaa 1550

ggctagggcc gagattagag ctcgaatggg cattgggctc ggctcggaga 1600

atgctgccgg gccctgcaac tgggacgaag ctgatatcgg accctgggcc 1650

aaagcccgga tccaggcggg agcagaagct aaagccaaag cccaagagag 1700

tggcagtgcc agcactggtg ccagtaccag taccaataac agtgccagtg 1750

ccagtgccag caccagtggt ggcttcagtg ctggtgccag cctgaccgcc 1800

actctcacat ttgggctctt cgctggcctt ggtggagctg gtgccagcac 1850

cagtggcagc tctggtgcct gtggtttctc ctacaagtga gattttagat 1900

attgttaatc ctgccagtct ttctcttcaa gccagggtgc atcctcagaa 1950

acctatccaa cacagcactc taggcagcca ct 1982

<210> 45
<211> 801
<212> DNA
<213> Homo sapien

<400> 45
cgccgcggcg atgccggagg agggttcggg ctgctcggtg cggcgcaggc 50

cctatgggtg cgtcctgcgg gctgctttgg tcccattggt cgcgggcttg 100

gtgatctgcc tcgtggtgtg catccagcgc ttcgcacagg ctcagcagca 150

gctgccgctc gagtcacttg ggtgggacgt agctgagctg cagctgaatc 200

acacaggacc tcagcaggac cccaggctat actggcaggg gggcccagca 250

ctgggccgct ccttcctgca tggaccagag ctggacaagg ggcagctacg 300

tatccatcgt gatggcatct acatggtaca catccaggtg acgctggcca 350

tctgctcctc cacgacggcc tccaggcacc accccaccac cctggccgtg 400

ggaatctgct ctcccgcctc ccgtagcatc agcctgctgc gtctcagctt 450

ccaccaaggt tgtaccattg cctcccagcg cctgacgccc ctggcccgag 500

gggacacact ctgcaccaac ctcactggga cacttttgcc ttcccgaaac 550

actgatgaga ccttctttgg agtgcagtgg gtgcgcccct gaccactgct 600

gctgattagg gttttttaaa ttttatttta ttttatttaa gttcaagaga 650

aaaagtgtac acacaggggc cacccggggt tggggtggga gtgtggtggg 700

gggtagtggt ggcaggacaa gagaaggcat tgagcttttt ctttcatttt 750

cctattaaaa aatacaaaaa tccaaaaaaa aaaaaaaaaa aaaaaaaaaa 800

a 801

<210> 46
<211> 690
<212> DNA
<213> Homo sapien

<400> 46
cagcacatcc cgctctgggc tttaaacgtg acccctcgcc tcgactcgcc 50

ctgccctgtg aaaatgttgg tgcttcttgc tttcatcatc gccttccaca 100

tcacctctgc agccttgctg ttcattgcca ccgtcgacaa tgcctggtgg 150

gtaggagatg agttttttgc agatgtctgg agaatatgta ccaacaacac 200

gaattgcaca gtcatcaatg acagctttca agagtactcc acgctgcagg 250

cggtccaggc caccatgatc ctctccacca ttctctgctg catcgccttc 300

ttcatcttcg tgctccagct cttccgcctg aagcagggag agaggtttgt 350

cctaacctcc atcatccagc taatgtcatg tctgtgtgtc atgattgcgg 400

cctccattta tacagacagg cgtgaagaca ttcacgacaa aaacgcgaaa 450

ttctatcccg tgaccagaga aggcagctac ggctactcct acatcctggc 500

gtgggtggcc ttcgcctgca ccttcatcag cggcatgatg tacctgatac 550

tgaggaagcg caaatagagt tccggagctg ggttgcttct gctgcagtac 600

agaatccaca ttcagataac cattttgtat ataatcatta ttttttgagg 650

tttttctagc aaaccgtatt gtttccttta aaagccaaaa 690

<210> 47
<211> 1823
<212> DNA
<213> Homo sapien

<400> 47
gcgcggagct gggagtggct tcgccatggc tgtgagaagg gactccgtgt 50

ggaagtactg ctggggtgtt ttgatggttt tatgcagaac tgcgatttcc 100

aaatcgatag ttttagagcc tatctattgg aattcctcga actccaaatt 150

tctacctgga caaggactgg tactataccc acagatagga gacaaattgg 200

atattatttg ccccaaagtg gactctaaaa ctgttggcca gtatgaatat 250
tataaagttt atatggttga taaagaccaa gcagacagat gcactattaa 300
gaaggaaaat acccctctcc tcaactgtgc caaaccagac caagatatca 350
aattcaccat caagtttcaa gaattcagcc ctaacctctg ggtctagaa 400
tttcagaaga acaaagatta ttacattata tctacatcaa atgggtcttt 450
ggagggcctg gataaccagg agggaggggt gtgccagaca agagccatga 500
agatcctcat gaaagttgga caagatgcaa gttctgctgg atcaaccagg 550
aataaagatc caacaagacg tccagaacta gaagctggta caaatggaag 600
aagttcgaca acaagtccct ttgtaaaacc aaatccaggt tctagcacag 650
acggcaacag cgccggacat tcggggaaca acatcctcgg ttccgaagtg 700
gccttatttg cagggattgc ttcaggatgc atcatcttca tcgtcatcat 750
catcacgctg gtggtcctct tgctgaagta ccggaggaga cacaggaagc 800
actcgccgca gcacacgacc acgctgtcgc tcagcacact ggccacaccc 850
aagcgcagcg gcaacaacaa cggctcagag cccagtgaca ttatcatccc 900
gctaaggact gcggacagcg tcttctgccc tcactacgag aaggtcagcg 950
gggactacgg gcacccggtg tacatcgtcc aggagatgcc cccgcagagc 1000
ccggcgaaca tttactacaa ggtctgagag ggaccctggt ggtacctgtg 1050
ctttcccaga ggacacctaa tgtcccgatg cctcccttga gggtttgaga 1100
gcccgcgtgc tggagaattg actgaagcac agcaccgggg gagagggaca 1150
ctcctcctcg gaagagcccg tcgcgctgga cagcttacct agtcttgtag 1200
cattcggcct tggtgaacac acacgctccc tggaagctgg aagactgtgc 1250
agaagacgcc cattcggact gctgtgccgc gtcccacgtc tcctcctcga 1300
agccatgtgc tgcggtcact caggcctctg cagaagccaa gggaagacag 1350
tggtttgtgg acgagagggc tgtgagcatc ctggcaggtg ccccaggatg 1400
ccacgcctgg aagggccggc ttctgcctgg ggtgcatttc ccccgcagtg 1450
cataccggac ttgtcacacg gacctcgggc tagttaaggt gtgcaaagat 1500
ctctagagtt tagtccttac tgtctcactc gttctgttac ccagggctct 1550
gcagcacctc acctgagacc tccactccac atctgcatca ctcatggaac 1600
actcatgtct ggagtcccct cctccagccg ctggcaacaa cagcttcagt 1650
ccatgggtaa tccgttcata gaaattgtgt ttgctaacaa ggtgcccttt 1700
agccagatgc taggctgtct gcgaagaagg ctaggagttc atagaaggga 1750

gtggggctgg ggaaagggct ggctgcaatt gcagctcact gctgctgcct 1800

ctgaaacaga aagttggaaa gga 1823

<210> 48
<211> 1100
<212> DNA
<213> Homo sapien

<400> 48
ggccgcggga gaggaggcca tgggcgcgcg cggggcgctg ctgctggcgc 50

tgctgctggc tcgggctgga ctcaggaagc cggagtcgca ggaggcggcg 100

ccgttatcag gaccatgcgg ccgacgggtc atcacgtcgc gcatcgtggg 150

tggagaggac gccgaactcg ggcgttggcc gtggcagggg agcctgcgcc 200

tgtgggattc ccacgtatgc ggagtgagcc tgctcagcca ccgctgggca 250

ctcacggcgg cgcactgctt tgaaacctat agtgacctta gtgatccctc 300

cgggtggatg gtccagtttg gccagctgac ttccatgcca tccttctgga 350

gcctgcaggc ctactacacc cgttacttcg tatcgaatat ctatctgagc 400

cctcgctacc tggggaattc accctatgac attgccttgg tgaagctgtc 450

tgcacctgtc acctacacta aacacatcca gcccatctgt ctccaggcct 500

ccacatttga gtttgagaac cggacagact gctgggtgac tggctggggg 550

tacatcaaag aggatgaggc actgccatct ccccacaccc tccaggaagt 600

tcaggtcgcc atcataaaca actctatgtg caaccacctc ttcctcaagt 650

acagtttccg caaggacatc tttggagaca tggtttgtgc tggcaacgcc 700

caaggcggga aggatgcctg cttcggtgac tcaggtggac ccttggcctg 750

taacaagaat ggactgtggt atcagattgg agtcgtgagc tggggagtgg 800

gctgtggtcg gcccaatcgg cccggtgtct acaccaatat cagccaccac 850

tttgagtgga tccagaagct gatggcccag agtggcatgt cccagccaga 900

cccctcctgg ccactactct tttccctct tctctgggct ctcccactcc 950

tggggccggt ctgagcctac ctgagcccat gcagcctggg gccactgcca 1000

agtcaggccc tggttctctt ctgtcttgtt tggtaataaa cacattccag 1050

ttgatgcctt gcagggcatt cttcaaaaaa aaaaaaaaaa aaaaaaaaaa 1100

<210> 49
<211> 2063
<212> DNA
<213> Homo sapien

<400> 49

gagagaggca gcagcttgct cagcggacaa ggatgctggg cgtgagggac 50
caaggcctgc cctgcactcg ggcctcctcc agccagtgct gaccagggac 100
ttctgacctg ctggccagcc aggacctgtg tggggaggcc ctcctgctgc 150
cttggggtga caatctcagc tccaggctac agggagaccg ggaggatcac 200
agagccagca tgttacagga tcctgacagt gatcaacctc tgaacagcct 250
cgatgtcaaa cccctgcgca aaccccgtat ccccatggag accttcagaa 300
aggtggggat ccccatcatc atagcactac tgagcctggc gagtatcatc 350
attgtggttg tcctcatcaa ggtgattctg gataaatact acttcctctg 400
cgggcagcct ctccacttca tcccgaggaa gcagctgtgt gacggagagc 450
tggactgtcc cttgggggag gacgaggagc actgtgtcaa gagcttcccc 500
gaagggcctg cagtggcagt ccgcctctcc aaggaccgat ccacactgca 550
ggtgctggac tcggccacag ggaactggtt ctctgcctgt ttcgacaact 600
tcacagaagc tctcgctgag acagcctgta ggcagatggg ctacagcaga 650
gctgtggaga ttggcccaga ccaggatctg gatgttgttg aaatcacaga 700
aaacagccag gagcttcgca tgcggaactc aagtgggccc tgtctctcag 750
gctccctggt ctccctgcac tgtcttgcct gtgggaagag cctgaagacc 800
ccccgtgtgg tgggtgggga ggaggcctct gtggattctt ggccttggca 850
ggtcagcatc cagtacgaca aacagcacgt ctgtggaggg agcatcctgg 900
acccccactg ggtcctcacg gcagcccact gcttcaggaa acataccgat 950
gtgttcaact ggaaggtgcg ggcaggctca gacaaactgg gcagcttccc 1000
atccctggct gtggccaaga tcatcatcat tgaattcaac cccatgtacc 1050
ccaaagacaa tgacatcgcc ctcatgaagc tgcagttccc actcactttc 1100
tcaggcacag tcaggcccat ctgtctgccc ttctttgatg aggagctcac 1150
tccagccacc ccactctgga tcattggatg gggctttacg aagcagaatg 1200
gagggaagat gtctgacata ctgctgcagg cgtcagtcca ggtcattgac 1250
agcacacggt gcaatgcaga cgatgcgtac caggggggaag tcaccgagaa 1300
gatgatgtgt gcaggcatcc cggaaggggg tgtggacacc tgccagggtg 1350
acagtggtgg gcccctgatg taccaatctg accagtggca tgtggtgggc 1400
atcgttagct ggggctatgg ctgcgggggc ccgagcaccc caggagtata 1450
caccaaggtc tcagcctatc tcaactggat ctacaatgtc tggaaggctg 1500
agctgtaatg ctgctgcccc tttgcagtgc tgggagccgc ttccttcctg 1550

ccctgcccac ctggggatcc cccaaagtca gacacagagc aagagtcccc 1600

ttgggtacac ccctctgccc acagcctcag catttcttgg agcagcaaag 1650

ggcctcaatt cctgtaagag accctcgcag cccagaggcg cccagaggaa 1700

gtcagcagcc ctagctcggc cacacttggt gctcccagca tcccagggag 1750

agacacagcc cactgaacaa ggtctcaggg gtattgctaa gccaagaagg 1800

aactttccca cactactgaa tggaagcagg ctgtcttgta aaagcccaga 1850

tcactgtggg ctggagagga gaaggaaagg gtctgcgcca gccctgtccg 1900

tcttcaccca tccccaagcc tactagagca agaaaccagt tgtaatataa 1950

aatgcactgc cctactgttg gtatgactac cgttacctac tgttgtcatt 2000

gttattacag ctatggccac tattattaaa gagctgtgta acatctctgg 2050

caaaaaaaaa aaa 2063

<210> 50
<211> 2692
<212> DNA
<213> Homo sapien

<400> 50
cccgggtcga cccacgcgtc cggggagaaa ggatggccgg cctggcggcg 50

cggttggtcc tgctagctgg ggcagcggcg ctggcgagcg gctcccaggg 100

cgaccgtgag ccggtgtacc gcgactgcgt actgcagtgc gaagagcaga 150

actgctctgg gggcgctctg aatcacttcc gctcccgcca gccaatctac 200

atgagtctag caggctggac ctgtcgggac gactgtaagt atgagtgtat 250

gtgggtcacc gttgggctct acctccagga aggtcacaaa gtgcctcagt 300

tccatggcaa gtggcccttc tcccggttcc tgttctttca agagccggca 350

tcggccgtgg cctcgtttct caatggcctg gccagcctgg tgatgctctg 400

ccgctaccgc accttcgtgc cagcctcctc ccccatgtac cacacctgtg 450

tggccttcgc ctgggtgtcc ctcaatgcat ggttctggtc cacagtcttc 500

cacaccaggg acactgacct cacagagaaa atggactact tctgtgcctc 550

cactgtcatc ctacactcaa tctacctgtg ctgcgtcagg accgtggggc 600

tgcagcaccc agctgtggtc agtgccttcc gggctctcct gctgctcatg 650

ctgaccgtgc acgtctccta cctgagcctc atccgcttcg actatggcta 700

caacctggtg gccaacgtgg ctattggcct ggtcaacgtg gtgtggtggc 750

tggcctggtg cctgtggaac cagcggcggc tgcctcacgt gcgcaagtgc 800

gtggtggtgg tcttgctgct gcaggggctg tccctgctcg agctgcttga 850

cttcccaccg ctcttctggg tcctggatgc ccatgccatc tggcacatca 900

gcaccatccc tgtccacgtc ctctttttca gctttctgga agatgacagc 950

ctgtacctgc tgaaggaatc agaggacaag ttcaagctgg actgaagacc 1000

ttggagcgag tctgccccag tggggatcct gcccccgccc tgctggcctc 1050

ccttctcccc tcaacccttg agatgatttt ctcttttcaa cttcttgaac 1100

ttggacatga aggatgtggg cccagaatca tgtggccagc ccaccccctg 1150

ttggccctca ccagccttgg agtctgttct agggaaggcc tcccagcatc 1200

tgggactcga gagtgggcag cccctctacc tcctggagct gaactggggt 1250

ggaactgagt gtgttcttag ctctaccggg aggacagctg cctgtttcct 1300

ccccaccagc ctcctcccca catccccagc tgcctggctg ggtcctgaag 1350

ccctctgtct acctgggaga ccagggacca caggccttag ggatacaggg 1400

ggtccccttc tgttaccacc ccccaccctc ctccaggaca ccactaggtg 1450

gtgctggatg cttgttcttt ggccagccaa ggttcacggc gattctcccc 1500

atgggatctt gagggaccaa gctgctggga ttgggaagga gtttcaccct 1550

gaccgttgcc ctagccaggt tcccaggagg cctcaccata ctccctttca 1600

gggccagggc tccagcaagc ccagggcaag gatcctgtgc tgctgtctgg 1650

ttgagagcct gccaccgtgt gtcgggagtg tgggccaggc tgagtgcata 1700

ggtgacaggg ccgtgagcat gggcctgggt gtgtgtgagc tcaggcctag 1750

gtgcgcagtg tggagacggg tgttgtcggg gaagaggtgt ggcttcaaag 1800

tgtgtgtgtg caggggggtgg gtgtgttagc gtgggttagg ggaacgtgtg 1850

tgcgcgtgct ggtgggcatg tgagatgagt gactgccggt gaatgtgtcc 1900

acagttgaga ggttggagca ggatgaggga atcctgtcac catcaataat 1950

cacttgtgga gcgccagctc tgcccaagac gccacctggg cggacagcca 2000

ggagctctcc atggccaggc tgcctgtgtg catgttccct gtctggtgcc 2050

cctttgcccg cctcctgcaa acctcacagg gtccccacac aacagtgccc 2100

tccagaagca gcccctcgga ggcagaggaa ggaaaatggg gatggctggg 2150

gctctctcca tcctcctttt ctccttgcct tcgcatggct ggccttcccc 2200

tccaaaacct ccattcccct gctgccagcc cctttgccat agcctgattt 2250

tggggaggag gaaggggcga tttgagggag aaggggagaa agcttatggc 2300

tgggtctggt ttcttccctt cccagagggt cttactgttc cagggtggcc 2350

ccagggcagg caggggccac actatgcctg tgccctggta aaggtgaccc 2400

ctgccattta ccagcagccc tggcatgttc ctgccccaca ggaatagaat 2450

ggagggagct ccagaaactt tccatcccaa aggcagtctc cgtggttgaa 2500

gcagactgga tttttgctct gcccctgacc ccttgtccct ctttgaggga 2550

ggggagctat gctaggactc caacctcagg gactcgggtg gcctgcgcta 2600

gcttcttttg atactgaaaa cttttaaggt gggagggtgg caagggatgt 2650

gcttaataaa tcaattccaa gcctcaaaaa aaaaaaaaaa aa 2692

<210> 51
<211> 1098
<212> DNA
<213> Homo sapien

<400> 51
cggcacgagg gtcccgcgcg ctcctccgac ccgctccgct ccgctccgct 50

cggccccgcg ccgcccgtca acatgatccg ctgcggcctg gcctgcgagc 100

gctgccgctg gatcctgccc ctgctcctac tcagcgccat cgccttcgac 150

atcatcgcgc tggccggccg cggctggttg cagtctagcg accacggcca 200

gacgtcctcg ctgtggtgga aatgctccca agagggcggc ggcagcgggt 250

cctacgagga gggctgtcag agcctcatgg agtacgcgtg ggggtagagca 300

gcggctgcca tgctcttctg tggcttcatc atcctggtga tctgtttcat 350

cctctccttc ttcgccctct gtggacccca gatgcttgtc ttcctgagag 400

tgattggagg tctccttgcc ttggctgctg tgttccagat catctccctg 450

gtaatttacc ccgtgaagta cacccagacc ttcacccttc atgccaaccc 500

tgctgtcact tacatctata actgggccta cggctttggg tgggcagcca 550

cgattatcct gattggctgt gccttcttct tctgctgcct cctcaactac 600

gaagatgacc ttctgggcaa tgccaagccc aggtacttct acacatctgc 650

ctaacttggg aatgaatgtg ggagaaaatc gctgctgctg agatggactc 700

cagaagaaga aactgtttct ccaggcgact ttgaacccat tttttggcag 750

tgttcatatt attaaactag tcaaaaatgc taaaataatt tgggagaaaa 800

tattttttaa gtagtgttat agtttcatgt ttatctttta ttatgttttg 850

tgaagttgtg tcttttcact aattacctat actatgccaa tatttcctta 900

tatctatcca taacatttat actacatttg taagagaata tgcacgtgaa 950

acttaacact ttataaggta aaaatgaggt ttccaagatt taataatctg 1000

atcaagttct tgttatttcc aaatagaatg gactcggtct gttaagggct 1050

aaggagaaga ggaagataag gttaaaagtt gttaatgacc aaacattc 1098

<210> 52
<211> 3325
<212> DNA
<213> Homo sapien

<400> 52
gaacgcttgt gtctaactga tgctcctaat gcggaagccc ctgaaaggcg 50

gttgtggtgc aaaggaaaac ccacaggcca aggaatggga agaccaaggt 100

tgacacttgt ttgtcaagtg tcaataatca tctctgcccg ggacctcagc 150

atgaacaacc tcacagagct tcagcctggc ctcttccacc acctgcgctt 200

cttggaggag ctgcgtctct ctgggaacca tctctcacac atcccaggac 250

aagcattctc tggtctctac agcctgaaaa tcctgatgct gcagaacaat 300

cagctgggag gaatccccgc agaggcgctg tgggagctgc cgagcctgca 350

gtcgctgcgc ctagatgcca acctcatctc cctggtcccg gagaggagct 400

ttgaggggct gtcctccctc cgccacctct ggctggacga caatgcactc 450

acggagatcc ctgtcagggc cctcaacaac ctccctgccc tgcaggccat 500

gaccctggcc ctcaaccgca tcagccacat ccccgactac gcgttccaga 550

atctcaccag ccttgtggtg ctgcatttgc ataacaaccg catccagcat 600

ctggggaccc acagcttcga ggggctgcac aatctggaga cactagacct 650

gaattataac aagctgcagg agttccctgt ggccatccgg accctgggca 700

gactgcagga actggggttc cataacaaca acatcaaggc catcccagaa 750

aaggccttca tggggaaccc tctgctacag acgatacact tttatgataa 800

cccaatccag tttgtgggaa gatcggcatt ccagtacctg cctaaactcc 850

acacactatc tctgaatggt gccatggaca tccaggagtt tccagatctc 900

aaaggcacca ccagcctgga gatcctgacc ctgacccgcg caggcatccg 950

gctgctccca tcggggatgt gccaacagct gcccaggctc cgagtcctgg 1000

aactgtctca caatcaaatt gaggagctgc ccagcctgca caggtgtcag 1050

aaattggagg aaatcggcct ccaacacaac cgcatctggg aaattggagc 1100

tgacaccttc agccagctga gctccctgca agccctggat cttagctgga 1150

acgccatccg gtccatccac cctgaggcct tctccaccct gcactccctg 1200

gtcaagctgg acctgacaga caaccagctg accacactgc ccctggctgg 1250

acttgggggc ttgatgcatc tgaagctcaa agggaacctt gctctctccc 1300

aggccttctc caaggacagt ttcccaaaac tgaggatcct ggaggtgcct 1350
tatgcctacc agtgctgtcc ctatgggatg tgtgccagct tcttcaaggc 1400
ctctgggcag tgggaggctg aagaccttca ccttgatgat gaggagtctt 1450
caaaaaggcc cctgggcctc cttgccagac aagcagagaa ccactatgac 1500
caggacctgg atgagctcca gctggagatg gaggactcaa agccacaccc 1550
cagtgtccag tgtagcccta ctccaggccc cttcaagccc tgtgagtacc 1600
tctttgaaag ctggggcatc cgcctggccg tgtgggccat cgtgttgctc 1650
tccgtgctct gcaatggact ggtgctgctg accgtgttcg ctggcgggcc 1700
tgcccccctg cccccggtca agtttgtggt aggtgcgatt gcaggcgcca 1750
acaccttgac tggcatttcc tgtggccttc tagcctcagt cgatgccctg 1800
acctttggtc agttctctga gtacggagcc cgctgggaga cggggctagg 1850
ctgccgggcc actggcttcc tggcagtact tgggtcggag gcatcggtgc 1900
tgctgctcac tctggccgca gtgcagtgca gcgtctccgt ctcctgtgtc 1950
cgggcctatg ggaagtcccc ctccctgggc agcgttcgag caggggtcct 2000
aggctgcctg gcactggcag ggctggccgc cgcactgccc ctggcctcag 2050
tgggagaata cggggcctcc ccactctgcc tgccctacgc gccacctgag 2100
ggtcagccag cagccctggg cttcaccgtg gccctggtga tgatgaactc 2150
cttctgtttc ctggtcgtgg ccggtgccta catcaaactg tactgtgacc 2200
tgccgcgggg cgactttgag gccgtgtggg actgcgccat ggtgaggcac 2250
gtggcctggc tcatcttcgc agacgggctc ctctactgtc ccgtggcctt 2300
cctcagcttc gcctccatgc tgggcctctt ccctgtcacg cccgaggccg 2350
tcaagtctgt cctgctggtg gtgctgcccc tgcctgcctg cctcaaccca 2400
ctgctgtacc tgctcttcaa cccccacttc cgggatgacc ttcggcggct 2450
tcggccccgc gcaggggact cagggcccct agcctatgct gcggccgggg 2500
agctggagaa gagctcctgt gattctaccc aggccctggt agccttctct 2550
gatgtggatc tcattctgga agcttctgaa gctgggcggc cccctgggct 2600
ggagacctat ggcttcccct cagtgaccct catctcctgt cagcagccag 2650
gggcccccag gctggagggc agccattgtg tagagccaga ggggaaccac 2700
tttgggaacc cccaaccctc catggatgga gaactgctgc tgagggcaga 2750
gggatctacg ccagcaggtg gaggcttgtc agggggtggc ggctttcagc 2800

cctctggctt ggcctttgct tcacacgtgt aaatatccct ccccattctt 2850

ctcttcccct ctcttccctt tcctctctcc ccctcggtga atgatggctg 2900

cttctaaaac aaatacaacc aaaactcagc agtgtgatct atagcaggat 2950

ggcccagtac ctggctccac tgatcacctc tctcctgtga ccatcaccaa 3000

cgggtgcctc ttggcctggc tttcccttgg ccttcctcag cttcaccttg 3050

atactgggcc tcttccttgt catgtctgaa gctgtggacc agagacctgg 3100

acttttgtct gcttaaggga aatgagggaa gtaaagacag tgaaggggtg 3150

gagggttgat cagggcacag tggacaggga gacctcacag agaaaggcct 3200

ggaaggtgat ttcccgtgtg actcatggat aggatacaaa atgtgttcca 3250

tgtaccatta atcttgacat atgccatgca taaagacttc ctattaaaat 3300

aagctttgga agagaaaaaa aaaaa 3325

<210> 53
<211> 1939
<212> DNA
<213> Homo sapien

<400> 53
cgcctccgcc ttcggaggct gacgcgcccg ggcgccgttc caggcctgtg 50

cagggcggat cggcagccgc ctggcggcga tccagggcgg tgcggggcct 100

gggcgggagc cgggaggcgc ggccggcatg gaggcgctgc tgctgggcgc 150

ggggttgctg ctgggcgctt acgtgcttgt ctactacaac ctggtgaagg 200

ccccgccgtg cggcggcatg ggcaacctgc ggggccgcac ggccgtggtc 250

acgggcgcca acagcggcat cggaaagatg acggcgctgg agctggcgcg 300

ccggggagcg cgcgtggtgc tggcctgccg cagccaggag cgcggggagg 350

cggctgcctt cgacctccgc caggagagtg ggaacaatga ggtcatcttc 400

atggccttgg acttggccag tctggcctcg gtgcgggcct ttgccactgc 450

ctttctgagc tctgagccac ggttggacat cctcatccac aatgccggta 500

tcagttcctg tggccggacc cgtgaggcgt ttaacctgct gcttcgggtg 550

aaccatatcg gtccctttct gctgacacat ctgctgctgc cttgcctgaa 600

ggcatgtgcc cctagccgcg tggtggtggt agcctcagct gcccactgtc 650

ggggacgtct tgacttcaaa cgcctggacc gcccagtggt gggctggcgg 700

caggagctgc gggcatatgc tgacactaag ctggctaatg tactgtttgc 750

ccgggagctc gccaaccagc ttgaggccac tggcgtcacc tgctatgcag 800

cccacccagg gcctgtgaac tcggagctgt tcctgcgcca tgttcctgga 850

tggctgcgcc cacttttgcg cccattggct tggctggtgc tccgggcacc 900

aagagggggt gcccagacac ccctgtattg tgctctacaa gagggcatcg 950

agcccctcag tgggagatat tttgccaact gccatgtgga agaggtgcct 1000

ccagctgccc gagacgaccg ggcagcccat cggctatggg aggccagcaa 1050

gaggctggca gggcttgggc ctggggagga tgctgaaccc gatgaagacc 1100

cccagtctga ggactcagag gccccatctt ctctaagcac cccccaccct 1150

gaggagccca cagtttctca accttacccc agccctcaga gctcaccaga 1200

tttgtctaag atgacgcacc gaattcaggc taaagttgag cctgagatcc 1250

agctctccta accctcaggc caggatgctt gccatggcac ttcatggtcc 1300

ttgaaaacct cggatgtgtg tgaggccatg ccctggacac tgacgggttt 1350

gtgatcttga cctccgtggt tactttctgg ggccccaagc tgtgccctgg 1400

acatctcttt tcctggttga aggaataatg ggtgattatt tcttcctgag 1450

agtgacagta accccagatg gagagatagg ggtatgctag acactgtgct 1500

tctcggaaat ttggatgtag tattttcagg ccccaccctt attgattctg 1550

atcagctctg gagcagaggc agggagtttg caatgtgatg cactgccaac 1600

attgagaatt agtgaactga tccctttgca accgtctagc taggtagtta 1650

aattaccccc atgttaatga agcggaatta ggctcccgag ctaagggact 1700

cgcctagggt ctcacagtga gtaggaggag ggcctgggat ctgaacccaa 1750

gggtctgagg ccagggccga ctgccgtaag atgggtgctg agaagtgagt 1800

cagggcaggg cagctggtat cgaggtgccc catgggagta aggggacgcc 1850

ttccgggcgg atgcagggct ggggtcatct gtatctgaag cccctcggaa 1900

taaagcgcgt tgaccgccaa aaaaaaaaaa aaaaaaaaa 1939

<210> 54
<211> 1484
<212> DNA
<213> Homo sapien

<400> 54
gaatttgtag aagacagcgg cgttgccatg gcggcgtctc tggggcaggt 50

gttggctctg gtgctggtgg ccgctctgtg gggtggcacg cagccgctgc 100

tgaagcgggc ctccgccggc ctgcagcggg ttcatgagcc gacctgggcc 150

cagcagttgc tacaggagat gaagaccctc ttcttgaata ctgagtacct 200

gatgcccttt ctcctcaacc agtgtggatc ccttctctat tacctcacct 250

tggcatcgac agatctgacc ctggctgtgc ccatctgtaa ctctctggct 300

atcatcttca cactgattgt tgggaaggcc cttggagaag atattggtgg 350

aaaacgtaag ttagactact gcgagtgcgg gacgcagctc tgtggatctc 400

gacatacctg tgttagttcc ttcccagaac ccatctcccc agagtgggtg 450

aggacacggc cttttcccat cctgcccttt cctctgcagc tgttttgctt 500

ccttgtggcc atcagagttc ccttcccctg gacagtctgg agaaagacag 550

aggctggggt ttgggattga agaccagacc ccatctgagc ccttcctcca 600

gccctgtacc agctcctact ggcatggctg agctcagacc ctcctgattt 650

ctgcctatta tcccaggagc agttgctggc atggtgctca ccgtgatagg 700

aatttcactc tgcatcacaa gctcagtgag taagacccag gggcaacagt 750

ctaccctttg agtgggccga acccacttcc agctctgctg cctccaggaa 800

gcccctgggc catgaagtgc tggcagtgag cggatggacc tagcacttcc 850

cctctctggc cttagcttcc tcctctctta tggggataac agctacctca 900

tggatcacaa taagagaaca agagtgaaag agttttgtaa ccttcaagtg 950

ctgttcagct gcggggattt agcacaggag actctacgct caccctcagc 1000

aacctttctg ccccagcagc tctcttcctg ctaacatctc aggctcccag 1050

cccagccacc attactgtgg cctgatctgg actatcatgg tggcaggttc 1100

catggactgc agaactccag ctgcatggaa agggccagct gcagactttg 1150

agccagaaat gcaaacggga ggcctctggg actcagtcag agcgctttgg 1200

ctgaatgagg ggtggaaccg agggaagaag gtgcgtcgga gtggcagatg 1250

caggaaatga gctgtctatt agccttgcct gccccaccca tgaggtaggc 1300

agaaatcctc actgccagcc cctcttaaac aggtagagag ctgtgagccc 1350

cagccccacc tgactccagc acacctggcg agtagtagct gtcaataaat 1400

ctatgtaaac agacaaaaaa aaaaaaaaaa aaaaaaaaaa aaaaaaaaaa 1450

aaaaaaaaaa aaaaaaaaaa aaaaaaaaaa aaaa 1484

<210> 55
<211> 5479
<212> DNA
<213> Homo sapien

<400> 55
cggcgaacag acgttctttc tcctccatgc agttacacaa aaggagggct 50

acggaaacta aaagtttcgg ggcctctggc tcggtgtgtg gagaaaagag 100

aaaacctgga gacgggatat gaagatcaat gatgcagact gatggtcttg 150

```
atgaagctgg gcatttataa ctagattcat taaggaatac aaagaaaata 200
cttaaaggga tcaataatgg tgtcttctgg ttgcagaatg cgaagtctgt 250
ggtttatcat tgtaatcagc ttcttaccaa atacagaagg tttcagcaga 300
gcagctttac catttgggct ggtgaggcga gaattatcct gtgaaggtta 350
ttctatagat ctgcgatgcc cgggcagtga tgtcatcatg attgagagcg 400
ctaactatgg tcggacggat gacaagattt gtgatgctga cccatttcag 450
atggagaata cagactgcta cctccccgat gccttcaaaa ttatgactca 500
aaggtgcaac aatcgaacac agtgtatagt agttactggg tcagatgtgt 550
ttcctgatcc atgtcctgga acatacaaat accttgaagt ccaatatgaa 600
tgtgtccctt acatttttgt gtgtcctggg accttgaaag caattgtgga 650
ctcaccatgt atatatgaag ctgaacaaaa ggcgggtgct tggtgcaagg 700
accctcttca ggctgcagat aaaatttatt tcatgccctg gactccctat 750
cgtaccgata ctttaataga atatgcttct ttagaagatt tccaaaatag 800
tcgccaaaca acaacatata aacttccaaa tcgagtagat ggtactggat 850
ttgtggtgta tgatggtgct gtcttcttta acaaagaaag aacgaggaat 900
attgtgaaat ttgacttgag gactagaatt aagagtggcg aggccataat 950
taactatgcc aactaccatg atacctcacc atacagatgg ggaggaaaga 1000
ctgatatcga cctagcagtt gatgaaaatg gtttatgggt catttacgcc 1050
actgaacaga acaatggaat gatagttatt agccagctga atccatacac 1100
tcttcgattt gaagcaacgt gggagactgt atacgacaaa cgtgccgcat 1150
caaatgcttt tatgatatgc ggagtcctct atgtggttag gtcagtttat 1200
caagacaatg aaagtgaaac aggcaagaac tcaattgatt acatttataa 1250
tacccgatta aaccgaggag aatatgtaga cgttcccttc cccaaccagt 1300
atcagtatat tgctgcagtg gattacaatc caagagataa ccaactttac 1350
gtgtggaaca ataacttcat tttacgatat tctctggagt ttggtccacc 1400
tgatcctgcc caagtgccta ccacagctgt gacaataact tcttcagctg 1450
agctgttcaa aaccataata tcaaccacaa gcactacttc acagaaaggc 1500
cccatgagca caactgtagc tggatcacag gaaggaagca aagggacaaa 1550
accacctcca gcagtttcta caaccaaaat tccacctata acaaatattt 1600
ttcccctgcc agagagattc tgtgaagcat tagactccaa ggggataaag 1650
```

tggcctcaga cacaaagggg aatgatggtt gaacgaccat gccctaaggg 1700
aacaagagga actgcctcat atctctgcat gatttccact ggaacatgga 1750
accctaaggg ccccgatctt agcaactgta cctcacactg ggtgaatcag 1800
ctggctcaga agatcagaag cggagaaaat gctgctagtc ttgccaatga 1850
actggctaaa cataccaaag ggccagtgtt tgctggggat gtaagttctt 1900
cagtgagatt gatggagcag ttggtggaca tccttgatgc acagctgcag 1950
gaactgaaac ctagtgaaaa agattcagct ggacggagtt ataacaaggc 2000
aattgttgac acagtggaca accttctgag acctgaagct ttggaatcat 2050
ggaaacatat gaattcttct gaacaagcac atactgcaac aatgttactc 2100
gatacattgg aagaaggagc ttttgtccta gctgacaatc ttttagaacc 2150
aacaagggtc tcaatgccca cagaaaatat tgtcctggaa gttgccgtac 2200
tcagtacaga aggacagatc caagacttta aatttcctct gggcatcaaa 2250
ggagcaggca gctcaatcca actgtccgca aataccgtca aacagaacag 2300
caggaatggg cttgcaaagt tggtgttcat catttaccgg agcctgggac 2350
agttccttag tacagaaaat gcaaccatta aactgggtgc tgattttatt 2400
ggtcgtaata gcaccattgc agtgaactct cacgtcattt cagtttcaat 2450
caataaagag tccagccgag tatacctgac tgatcctgtg ctttttaccc 2500
tgccacacat tgatcctgac aattatttca atgcaaactg ctccttctgg 2550
aactactcag agagaactat gatgggatat tggtctaccc agggctgcaa 2600
gctggttgac actaataaaa ctcgaacaac gtgtgcatgc agccacctaa 2650
ccaattttgc aattctcatg gcccacaggg aaattgcata taaagatggc 2700
gttcatgaat tacttcttac agtcatcacc tgggtgggaa ttgtcatttc 2750
ccttgtttgc ctggctatct gcatcttcac cttctgcttt ttccgtggcc 2800
tacagagtga ccgaaatact attcacaaga acctttgtat caaccttttc 2850
attgctgaat ttattttcct aataggcatt gataagacaa aatatgcgat 2900
tgcatgccca atatttgcag gacttctaca ctttttcttt ttggcagctt 2950
ttgcttggat gtgcctagaa ggtgtgcagc tctacctaat gttagttgaa 3000
gtttttgaaa gtgaatattc aaggaaaaaa tattactatg ttgctggtta 3050
cttgtttcct gccacagtgg ttggagtttc agctgctatt gactataaga 3100
gctatggaac agaaaaagct tgctggcttc atgttgataa ctactttata 3150
tggagcttca ttggacctgt taccttcatt attctgctaa atattatctt 3200

cttggtgatc acattgtgca aaatggtgaa gcattcaaac actttgaaac 3250
cagattctag caggttggaa aacattaagt cttgggtgct tggcgctttc 3300
gctcttctgt gtcttcttgg cctcacctgg tcctttgggt tgctttttat 3350
taatgaggag actattgtga tggcatatct cttcactata tttaatgctt 3400
tccagggagt gttcattttc atctttcact gtgctctcca aaagaaagta 3450
cgaaaagaat atggcaagtg cttcagacac tcatactgct gtggaggcct 3500
cccaactgag agtccccaca gttcagtgaa ggcatcaacc accagaacca 3550
gtgctcgcta ttcctctggc acacagagtc gtataagaag aatgtggaat 3600
gatactgtga gaaaacaatc agaatcttct tttatctcag gtgacatcaa 3650
tagcacttca acacttaatc aaggacattc actgaacaat gccagggata 3700
caagtgccat ggatactcta ccgctaaatg gtaattttaa caacagctac 3750
tcgctgcaca agggtgacta taatgacagc gtgcaagttg tggactgtgg 3800
actaagtctg aatgatactg cttttgagaa aatgatcatt tcagaattag 3850
tgcacaacaa cttacggggc agcagcaaga ctcacaacct cgagctcacg 3900
ctaccagtca aacctgtgat tggaggtagc agcagtgaag atgatgctat 3950
tgtggcagat gcttcatctt taatgcacag cgacaaccca gggctggagc 4000
tccatcacaa agaactcgag gcaccactta ttcctcagcg gactcactcc 4050
cttctgtacc aaccccagaa gaaagtgaag tccgagggaa ctgacagcta 4100
tgtctcccaa ctgacagcag aggctgaaga tcacctacag tcccccaaca 4150
gagactctct ttatacaagc atgcccaatc ttagagactc tccctatccg 4200
gagagcagcc ctgacatgga agaagacctc tctccctcca ggaggagtga 4250
gaatgaggac atttactata aaagcatgcc aaatcttgga gctggccatc 4300
agcttcagat gtgctaccag atcagcaggg gcaatagtga tggttatata 4350
atccccatta acaaagaagg gtgtattcca gaaggagatg ttagagaagg 4400
acaaatgcag ctggttacaa gtctttaatc atacagctaa ggaattccaa 4450
gggccacatg cgagtattaa taaataaaga caccattggc ctgacgcagc 4500
tccctcaaac tctgcttgaa gagatgactc ttgacctgtg gttctctggt 4550
gtaaaaaaga tgactgaacc ttgcagttct gtgaattttt ataaaacata 4600
caaaaacttt gtatatacac agagtatact aaagtgaatt atttgttaca 4650
aagaaaagag atgccagcca ggtattttaa gattctgctg ctgtttagag 4700

aaattgtgaa acaagcaaaa caaaactttc cagccatttt actgcagcag 4750

tctgtgaact aaatttgtaa atatggctgc accatttttg taggcctgca 4800

ttgtattata tacaagacgt aggctttaaa atcctgtggg acaaatttac 4850

tgtaccttac tattcctgac aagacttgga aaagcaggag agatattctg 4900

catcagtttg cagttcactg caaatctttt acattaaggc aaagattgaa 4950

aacatgctta accactagca atcaagccac aggccttatt tcatatgttt 5000

cctcaactgt acaatgaact attctcatga aaaatggcta aagaaattat 5050

attttgttct attgctaggg taaaataaat acatttgtgt ccaactgaaa 5100

tataattgtc attaaaataa ttttaaagag tgaagaaaat attgtgaaaa 5150

gctcttggtt gcacatgtta tgaaatgttt tttcttacac tttgtcatgg 5200

taagttctac tcattttcac ttcttttcca ctgtatacag tgttctgctt 5250

tgacaaagtt agtctttatt acttacattt aaatttctta ttgccaaaag 5300

aacgtgtttt atggggagaa acaaactctt tgaagccagt tatgtcatgc 5350

cttgcacaaa agtgatgaaa tctagaaaag attgtgtgtc acccctgttt 5400

attcttgaac agagggcaaa gagggcactg ggcacttctc acaaactttc 5450

tagtgaacaa aaggtgccta ttcttttt 5479

<210> 56
<211> 1434
<212> DNA
<213> Homo sapien

<400> 56
gcatagatga atgtatcagt ggatggatag ttggctagat gggtgggttg 50

gtggatgaat ggcagagctt gcacctgcca gtccatctga catcaaagcc 100

agtgtctcta atggtgacac caccctcctc tgcagcagga ggcagagctg 150

tgggatgaat gaggttcgcc aggtctccct tacctatcct gggtccccag 200

ctccttctca ctctcttccc ttgcagcctc gaagcggagg atccctgtgt 250

cccagccggg catggccgac ccccaccagc ttttcgatga cacaagttca 300

gcccagagcc ggggctatgg ggcccagcgg gcacctggtg gcctgagtta 350

tcctgcagcc tctcccacgc cccatgcagc cttcctggct gacccggtgt 400

ccaacatggc catggcctat gggagcagcc tggccgcgca gggcaaggag 450

ctggtggata agaacatcga ccgcttcatc cccatcacca agctcaagta 500

ttactttgct gtggacacca tgtatgtggg cagaaagctg ggcctgctgt 550

tcttccccta cctacaccag gactgggaag tgcagtacca acaggacacc 600

ccggtggccc cccgctttga cgtcaatgcc ccggacctct acattccagc 650

aatggctttc atcacctacg ttttggtggc tggtcttgcg ctggggaccc 700

aggataggtt ctccccagac ctcctggggc tgcaagcgag ctcagccctg 750

gcctggctga ccctggaggt gctggccatc ctgctcagcc tctatctggt 800

cactgtcaac accgacctca ccaccatcga cctggtggcc ttcttgggct 850

acaaatatgt cgggatgatt ggcggggtcc tcatgggcct gctcttcggg 900

aagattggct actacctggt gctgggctgg tgctgcgtag ccatctttgt 950

gttcatgatc cggacgctgc ggctgaagat cttggcagac gcagcagctg 1000

agggggtccc ggtgcgtggg gcccggaacc agctgcgcat gtacctgacc 1050

atggcggtgg cggcggcgca gcctatgctc atgtactggc tcaccttcca 1100

cctggtgcgg tgagcgcgcc cgctgaacct cccgctgctg ctgctgctgc 1150

tgggggccac tgtggccgcc gaactcatct cctgcctgca ggccccaagg 1200

tccaccctgt ctggccacag gcaccgcctc catcccatgt cccgcccagc 1250

cccgcccccca acccaaggtg ctgagagatc tccagctgca caggccaccg 1300

ccccagggcg tggccgctgt tacagaaaca ataaaccctg atgggcatgg 1350

caaaaaaaaa aaaaaaaaaa aaaaaaaaaa aaaaaaaaaa aaaaaaaaaa 1400

aaaaaaaaaa aaaaaaaaaa aaaaaaaaaa aaga 1434

<210> 57
<211> 1414
<212> DNA
<213> Homo sapien

<400> 57
cttccacgcc cgagggcatc gcgctggcct acggcagcct cctgctcatg 50

gcgctgctgc ccatcttctt cggcgccctg cgctccgtac gctgcgcccg 100

cggcaagaat gcttcagaca tgcctgaaac aatcaccagc cgggatgccg 150

cccgcttccc catcatcgcc agctgcacac tcttggggct ctacctcttt 200

ttcaaaatat tctcccagga gtacatcaac ctcctgctgt ccatgtattt 250

cttcgtgctg ggaatcctgg ccctgtccca caccatcagc cccttcatga 300

ataagttttt tccagccagc tttccaaatc gacagtacca gctgctcttc 350

acacagggtt ctggggaaaa caaggaagag atcatcaatt atgaatttga 400

caccaaggac ctggtgtgcc tgggcctgag cagcatcgtt ggcgtctggt 450

acctgctgag gaagcactgg attgccaaca acctttttgg cctggccttc 500

tcccttaatg gagtagagct cctgcacctc aacaatgtca gcactggctg 550

catcctgctg ggcggactct tcatctacga tgtcttctgg gtatttggca 600

ccaatgtgat ggtgacagtg gccaagtcct tcgaggcacc aataaaattg 650

gtgtttcccc aggatctgct gcagaaaggc ctcgaagcaa acaactttgc 700

catgctggga cttggagatg tcgtcattcc agggatcttc attgccttgc 750

tgctgcgctt tgacatcagc ttgaagaaga atacccacac ctacttctac 800

accagctttg cagcctacat cttcggcctg ggccttacca tcttcatcat 850

gcacatcttc aagcatgctc agcctgccct cctatacctg gtccccgcct 900

gcatcggttt tcctgtcctg gtggcgctgg ccaagggaga agtgacagag 950

atgttcagtt atgaggagtc aaatcctaag gatccagcgg cagtgacaga 1000

atccaaagag ggaacagagg catcagcatc gaaggggctg gagaagaaag 1050

agaaatgatg cagctggtgc ccgagcctct cagggccaga ccagacagat 1100

gggggctggg cccacacagg cgtgcaccgg tagagggcac aggaggccaa 1150

gggcagctcc aggacagggc agggggcagc aggatacctc cagccaggcc 1200

tctgtggcct ctgtttcctt ctccctttct tggccctcct ctgctcctcc 1250

ccacaccctg caggcaaaag aaacccccag cttcccccct ccccgggagc 1300

caggtgggaa aagtgggtgt gatttttaga ttttgtattg tggactgatt 1350

ttgcctcaca ttaaaaactc atcccatggc cagggcgggc cactgtaaaa 1400

aaaaaaaaaa aaaa 1414

<210> 58
<211> 308
<212> PRT
<213> Homo sapien

<220>
<221> X
<222> 138-147
<223> Unknown base

<400> 58

```
Met Thr Ile Ala Leu Leu Gly Phe Ala Ile Phe Leu Leu His Cys
  1               5                  10                  15

Ala Thr Cys Glu Lys Pro Leu Glu Gly Ile Leu Ser Ser Ser Ala
                 20                  25                  30

Trp His Phe Thr His Ser His Tyr Asn Ala Thr Ile Tyr Glu Asn
                 35                  40                  45

Ser Ser Pro Lys Thr Tyr Val Glu Ser Phe Glu Lys Met Gly Ile
                 50                  55                  60
```

Tyr Leu Ala Glu Pro Gln Trp Ala Val Arg Tyr Arg Ile Ile Ser
65 70 75

Gly Asp Val Ala Asn Val Phe Lys Thr Glu Glu Tyr Val Val Gly
80 85 90

Asn Phe Cys Phe Leu Arg Ile Arg Thr Lys Ser Ser Asn Thr Ala
95 100 105

Leu Leu Asn Arg Glu Val Arg Asp Ser Tyr Thr Leu Ile Ile Gln
110 115 120

Ala Thr Glu Lys Thr Leu Glu Leu Glu Ala Leu Thr Arg Val Val
125 130 135

Val His Xaa Xaa Xaa Xaa Xaa Xaa Xaa Xaa Xaa Xaa Ala Asp Leu
140 145 150

Gly Gln Asn Ala Glu Phe Tyr Tyr Ala Phe Asn Thr Arg Ser Glu
155 160 165

Met Phe Ala Ile His Pro Thr Ser Gly Val Val Thr Val Ala Gly
170 175 180

Lys Leu Asn Val Thr Trp Arg Gly Lys His Glu Leu Gln Val Leu
185 190 195

Ala Val Asp Arg Met Arg Lys Ile Ser Glu Gly Asn Gly Phe Gly
200 205 210

Ser Leu Ala Ala Leu Val Val His Val Glu Pro Ala Leu Arg Lys
215 220 225

Pro Pro Ala Ile Ala Ser Val Val Val Thr Pro Pro Asp Ser Asn
230 235 240

Asp Gly Thr Thr Tyr Ala Thr Val Leu Val Asp Ala Asn Ser Ser
245 250 255

Gly Ala Glu Val Glu Ser Val Glu Val Val Gly Gly Asp Pro Gly
260 265 270

Lys His Phe Lys Ala Ile Lys Ser Tyr Ala Arg Ser Asn Glu Phe
275 280 285

Ser Leu Val Ser Val Lys Asp Ile Asn Trp Met Glu Tyr Leu His
290 295 300

Gly Phe Asn Leu Ser Leu Gln Ala
305

<210> 59
<211> 795
<212> PRT
<213> Homo sapien

<400> 59
Met Tyr His Ser Leu Ser Glu Thr Arg His Pro Leu Gln Pro Glu
1 5 10 15

Glu Gln Glu Val Gly Ile Asp Pro Leu Ser Ser Tyr Ser Asn Lys

20 25 30

Ser Gly Gly Asp Ser Asn Lys Asn Gly Arg Arg Thr Ser Ser Thr
35 40 45

Leu Asp Ser Glu Gly Thr Phe Asn Ser Tyr Arg Lys Glu Trp Glu
50 55 60

Glu Leu Phe Val Asn Asn Asn Tyr Leu Ala Thr Ile Arg Gln Lys
65 70 75

Gly Ile Asn Gly Gln Leu Arg Ser Ser Arg Phe Arg Ser Ile Cys
80 85 90

Trp Lys Leu Phe Leu Cys Val Leu Pro Gln Asp Lys Ser Gln Trp
95 100 105

Ile Ser Arg Ile Glu Glu Leu Arg Ala Trp Tyr Ser Asn Ile Lys
110 115 120

Glu Ile His Ile Thr Asn Pro Arg Lys Val Val Gly Gln Gln Asp
125 130 135

Leu Met Ile Asn Asn Pro Leu Ser Gln Asp Glu Gly Ser Leu Trp
140 145 150

Asn Lys Phe Phe Gln Asp Lys Glu Leu Arg Ser Met Ile Glu Gln
155 160 165

Asp Val Lys Arg Thr Phe Pro Glu Met Gln Phe Phe Gln Gln Glu
170 175 180

Asn Val Arg Lys Ile Leu Thr Asp Val Leu Phe Cys Tyr Ala Arg
185 190 195

Glu Asn Glu Gln Leu Leu Tyr Lys Gln Gly Met His Glu Leu Leu
200 205 210

Ala Pro Ile Val Phe Val Leu His Cys Asp His Gln Ala Phe Leu
215 220 225

His Ala Ser Glu Ser Ala Gln Pro Ser Glu Glu Met Lys Thr Val
230 235 240

Leu Asn Pro Glu Tyr Leu Glu His Asp Ala Tyr Ala Val Phe Ser
245 250 255

Gln Leu Met Glu Thr Ala Glu Pro Trp Phe Ser Thr Phe Glu His
260 265 270

Asp Gly Gln Lys Gly Lys Glu Thr Leu Met Thr Pro Ile Pro Phe
275 280 285

Ala Arg Pro Gln Asp Leu Gly Pro Thr Ile Ala Ile Val Thr Lys
290 295 300

Val Asn Gln Ile Gln Asp His Leu Leu Lys Lys His Asp Ile Glu
305 310 315

Leu Tyr Met His Leu Asn Arg Leu Glu Ile Ala Pro Gln Ile Tyr
320 325 330

Gly Leu Arg Trp Val Arg Leu Leu Phe Gly Arg Glu Phe Pro Leu
335 340 345

Gln Asp Leu Leu Val Val Trp Asp Ala Leu Phe Ala Asp Gly Leu
350 355 360

Ser Leu Gly Leu Val Asp Tyr Ile Phe Val Ala Met Leu Leu Tyr
365 370 375

Ile Arg Asp Ala Leu Ile Ser Ser Asn Tyr Gln Thr Cys Leu Gly
380 385 390

Leu Leu Met His Tyr Pro Phe Ile Gly Asp Val His Ser Leu Ile
395 400 405

Leu Lys Ala Leu Phe Leu Arg Asp Pro Lys Arg Asn Pro Arg Pro
410 415 420

Val Thr Tyr Gln Phe His Pro Asn Leu Asp Tyr Tyr Lys Ala Arg
425 430 435

Gly Ala Asp Leu Met Asn Lys Ser Arg Thr Asn Ala Lys Gly Ala
440 445 450

Pro Leu Asn Ile Asn Lys Val Ser Asn Ser Leu Ile Asn Phe Gly
455 460 465

Arg Lys Leu Ile Ser Pro Ala Met Ala Pro Gly Ser Ala Gly Gly
470 475 480

Pro Val Pro Gly Gly Asn Ser Ser Ser Ser Ser Ser Val Val Ile
485 490 495

Pro Thr Arg Thr Ser Ala Glu Ala Pro Ser His His Leu Gln Gln
500 505 510

Gln Gln Gln Gln Gln Arg Leu Met Lys Ser Glu Ser Met Pro Val
515 520 525

Gln Leu Asn Lys Gly Leu Ser Ser Lys Asn Ile Ser Ser Ser Pro
530 535 540

Ser Val Glu Ser Leu Pro Gly Gly Arg Glu Phe Thr Gly Ser Pro
545 550 555

Pro Ser Ser Ala Thr Lys Lys Asp Ser Phe Phe Ser Asn Ile Ser
560 565 570

Arg Ser Arg Ser His Ser Lys Thr Met Gly Arg Lys Glu Ser Glu
575 580 585

Glu Glu Leu Glu Ala Gln Ile Ser Phe Leu Gln Gly Gln Leu Asn
590 595 600

Asp Leu Asp Ala Met Cys Lys Tyr Cys Ala Lys Val Met Asp Thr
605 610 615

His Leu Val Asn Ile Gln Asp Val Ile Leu Gln Glu Asn Leu Glu
620 625 630

Lys Glu Asp Gln Ile Leu Val Ser Leu Ala Gly Leu Lys Gln Ile
635 640 645

Lys Asp Ile Leu Lys Gly Ser Leu Arg Phe Asn Gln Ser Gln Leu
650 655 660

Glu Ala Glu Glu Asn Glu Gln Ile Thr Ile Ala Asp Asn His Tyr
665 670 675

Cys Ser Ser Gly Gln Gly Gln Gly Arg Gly Gln Gly Gln Ser Val
680 685 690

Gln Met Ser Gly Ala Ile Lys Gln Ala Ser Ser Glu Thr Pro Gly
695 700 705

Cys Thr Asp Arg Gly Asn Ser Asp Asp Phe Ile Leu Ile Ser Lys
710 715 720

Asp Asp Asp Gly Ser Ser Ala Arg Gly Ser Phe Ser Gly Gln Ala
725 730 735

Gln Pro Leu Arg Thr Leu Arg Ser Thr Ser Gly Lys Ser Gln Ala
740 745 750

Pro Val Cys Ser Pro Leu Val Phe Ser Asp Pro Leu Met Gly Pro
755 760 765

Ala Ser Ala Ser Ser Ser Asn Pro Ser Ser Ser Pro Asp Asp Asp
770 775 780

Ser Ser Lys Asp Ser Gly Phe Thr Ile Val Ser Pro Leu Asp Ile
785 790 795

<210> 60
<211> 606
<212> PRT
<213> Homo sapien

<400> 60
Met Ser Asp Thr Ser Glu Ser Gly Ala Gly Leu Thr Arg Phe Gln
1 5 10 15

Ala Glu Ala Ser Glu Lys Asp Ser Ser Ser Met Met Gln Thr Leu
20 25 30

Leu Thr Val Thr Gln Asn Val Glu Val Pro Glu Thr Pro Lys Ala
35 40 45

Ser Lys Ala Leu Glu Val Ser Glu Asp Val Lys Val Ser Lys Ala
50 55 60

Ser Gly Val Ser Lys Ala Thr Glu Val Ser Lys Thr Pro Glu Ala
65 70 75

Arg Glu Ala Pro Ala Thr Gln Ala Ser Ser Thr Thr Gln Leu Thr
80 85 90

Asp Thr Gln Val Leu Ala Ala Glu Asn Lys Ser Leu Ala Ala Asp
95 100 105

Thr Lys Lys Gln Asn Ala Asp Pro Gln Ala Val Thr Met Pro Ala

110 115 120

Thr Glu Thr Lys Lys Val Ser His Val Ala Asp Thr Lys Val Asn
125 130 135

Thr Lys Ala Gln Glu Thr Glu Ala Ala Pro Ser Gln Ala Pro Ala
140 145 150

Asp Glu Pro Glu Pro Glu Ser Ala Ala Ala Gln Ser Gln Glu Asn
155 160 165

Gln Asp Thr Arg Pro Lys Val Lys Ala Lys Lys Ala Arg Lys Val
170 175 180

Lys His Leu Asp Gly Glu Glu Asp Gly Ser Ser Asp Gln Ser Gln
185 190 195

Ala Ser Gly Thr Thr Gly Gly Arg Arg Val Ser Lys Ala Leu Met
200 205 210

Ala Ser Met Ala Arg Arg Ala Ser Arg Gly Pro Ile Ala Phe Trp
215 220 225

Ala Arg Arg Ala Ser Arg Thr Arg Leu Ala Ala Trp Ala Arg Arg
230 235 240

Ala Leu Leu Ser Leu Arg Ser Pro Lys Ala Arg Arg Gly Lys Ala
245 250 255

Arg Arg Arg Ala Ala Lys Leu Gln Ser Ser Gln Glu Pro Glu Ala
260 265 270

Pro Pro Pro Arg Asp Val Ala Leu Leu Gln Gly Arg Ala Asn Asp
275 280 285

Leu Val Lys Tyr Leu Leu Ala Lys Asp Gln Thr Lys Ile Pro Ile
290 295 300

Lys Arg Ser Asp Met Leu Lys Asp Ile Ile Lys Glu Tyr Thr Asp
305 310 315

Val Tyr Pro Glu Ile Ile Glu Arg Ala Gly Tyr Ser Leu Glu Lys
320 325 330

Val Phe Gly Ile Gln Leu Lys Glu Ile Asp Lys Asn Asp His Leu
335 340 345

Tyr Ile Leu Leu Ser Thr Leu Glu Pro Thr Asp Ala Gly Ile Leu
350 355 360

Gly Thr Thr Lys Asp Ser Pro Lys Leu Gly Leu Leu Met Val Leu
365 370 375

Leu Ser Ile Ile Phe Met Asn Gly Asn Arg Ser Ser Glu Ala Val
380 385 390

Ile Trp Glu Val Leu Arg Lys Leu Gly Leu Arg Pro Gly Ile His
395 400 405

His Ser Leu Phe Gly Asp Val Lys Lys Leu Ile Thr Asp Glu Phe
410 415 420

Val Lys Gln Lys Tyr Leu Asp Tyr Ala Arg Val Pro Asn Ser Asn
425 430 435

Pro Pro Glu Tyr Glu Phe Phe Trp Gly Leu Arg Ser Tyr Tyr Glu
440 445 450

Thr Ser Lys Met Lys Val Leu Lys Phe Ala Cys Lys Val Gln Lys
455 460 465

Lys Asp Pro Lys Glu Trp Ala Ala Gln Tyr Arg Glu Ala Met Glu
470 475 480

Ala Asp Leu Lys Ala Ala Ala Glu Ala Ala Ala Glu Ala Lys Ala
485 490 495

Arg Ala Glu Ile Arg Ala Arg Met Gly Ile Gly Leu Gly Ser Glu
500 505 510

Asn Ala Ala Gly Pro Cys Asn Trp Asp Glu Ala Asp Ile Gly Pro
515 520 525

Trp Ala Lys Ala Arg Ile Gln Ala Gly Ala Glu Ala Lys Ala Lys
530 535 540

Ala Gln Glu Ser Gly Ser Ala Ser Thr Gly Ala Ser Thr Ser Thr
545 550 555

Asn Asn Ser Ala Ser Ala Ser Ala Ser Thr Ser Gly Gly Phe Ser
560 565 570

Ala Gly Ala Ser Leu Thr Ala Thr Leu Thr Phe Gly Leu Phe Ala
575 580 585

Gly Leu Gly Gly Ala Gly Ala Ser Thr Ser Gly Ser Ser Gly Ala
590 595 600

Cys Gly Phe Ser Tyr Lys
605

<210> 61
<211> 193
<212> PRT
<213> Homo sapien

<400> 61
Met Pro Glu Glu Gly Ser Gly Cys Ser Val Arg Arg Arg Pro Tyr
1 5 10 15

Gly Cys Val Leu Arg Ala Ala Leu Val Pro Leu Val Ala Gly Leu
20 25 30

Val Ile Cys Leu Val Val Cys Ile Gln Arg Phe Ala Gln Ala Gln
35 40 45

Gln Gln Leu Pro Leu Glu Ser Leu Gly Trp Asp Val Ala Glu Leu
50 55 60

Gln Leu Asn His Thr Gly Pro Gln Gln Asp Pro Arg Leu Tyr Trp
65 70 75

Gln Gly Gly Pro Ala Leu Gly Arg Ser Phe Leu His Gly Pro Glu
80 85 90

Leu Asp Lys Gly Gln Leu Arg Ile His Arg Asp Gly Ile Tyr Met
95 100 105

Val His Ile Gln Val Thr Leu Ala Ile Cys Ser Ser Thr Thr Ala
110 115 120

Ser Arg His His Pro Thr Thr Leu Ala Val Gly Ile Cys Ser Pro
125 130 135

Ala Ser Arg Ser Ile Ser Leu Leu Arg Leu Ser Phe His Gln Gly
140 145 150

Cys Thr Ile Ala Ser Gln Arg Leu Thr Pro Leu Ala Arg Gly Asp
155 160 165

Thr Leu Cys Thr Asn Leu Thr Gly Thr Leu Leu Pro Ser Arg Asn
170 175 180

Thr Asp Glu Thr Phe Phe Gly Val Gln Trp Val Arg Pro
185 190

<210> 62
<211> 167
<212> PRT
<213> Homo sapien

<400> 62
Met Leu Val Leu Leu Ala Phe Ile Ile Ala Phe His Ile Thr Ser
1 5 10 15

Ala Ala Leu Leu Phe Ile Ala Thr Val Asp Asn Ala Trp Trp Val
20 25 30

Gly Asp Glu Phe Phe Ala Asp Val Trp Arg Ile Cys Thr Asn Asn
35 40 45

Thr Asn Cys Thr Val Ile Asn Asp Ser Phe Gln Glu Tyr Ser Thr
50 55 60

Leu Gln Ala Val Gln Ala Thr Met Ile Leu Ser Thr Ile Leu Cys
65 70 75

Cys Ile Ala Phe Phe Ile Phe Val Leu Gln Leu Phe Arg Leu Lys
80 85 90

Gln Gly Glu Arg Phe Val Leu Thr Ser Ile Ile Gln Leu Met Ser
95 100 105

Cys Leu Cys Val Met Ile Ala Ala Ser Ile Tyr Thr Asp Arg Arg
110 115 120

Glu Asp Ile His Asp Lys Asn Ala Lys Phe Tyr Pro Val Thr Arg
125 130 135

Glu Gly Ser Tyr Gly Tyr Ser Tyr Ile Leu Ala Trp Val Ala Phe
140 145 150

Ala Cys Thr Phe Ile Ser Gly Met Met Tyr Leu Ile Leu Arg Lys

155 160 165

Arg Lys

<210> 63
<211> 333
<212> PRT
<213> Homo sapien

<400> 63
Met Ala Val Arg Arg Asp Ser Val Trp Lys Tyr Cys Trp Gly Val
1 5 10 15

Leu Met Val Leu Cys Arg Thr Ala Ile Ser Lys Ser Ile Val Leu
20 25 30

Glu Pro Ile Tyr Trp Asn Ser Ser Asn Ser Lys Phe Leu Pro Gly
35 40 45

Gln Gly Leu Val Leu Tyr Pro Gln Ile Gly Asp Lys Leu Asp Ile
50 55 60

Ile Cys Pro Lys Val Asp Ser Lys Thr Val Gly Gln Tyr Glu Tyr
65 70 75

Tyr Lys Val Tyr Met Val Asp Lys Asp Gln Ala Asp Arg Cys Thr
80 85 90

Ile Lys Lys Glu Asn Thr Pro Leu Leu Asn Cys Ala Lys Pro Asp
95 100 105

Gln Asp Ile Lys Phe Thr Ile Lys Phe Gln Glu Phe Ser Pro Asn
110 115 120

Leu Trp Gly Leu Glu Phe Gln Lys Asn Lys Asp Tyr Tyr Ile Ile
125 130 135

Ser Thr Ser Asn Gly Ser Leu Glu Gly Leu Asp Asn Gln Glu Gly
140 145 150

Gly Val Cys Gln Thr Arg Ala Met Lys Ile Leu Met Lys Val Gly
155 160 165

Gln Asp Ala Ser Ser Ala Gly Ser Thr Arg Asn Lys Asp Pro Thr
170 175 180

Arg Arg Pro Glu Leu Glu Ala Gly Thr Asn Gly Arg Ser Ser Thr
185 190 195

Thr Ser Pro Phe Val Lys Pro Asn Pro Gly Ser Ser Thr Asp Gly
200 205 210

Asn Ser Ala Gly His Ser Gly Asn Asn Ile Leu Gly Ser Glu Val
215 220 225

Ala Leu Phe Ala Gly Ile Ala Ser Gly Cys Ile Ile Phe Ile Val
230 235 240

Ile Ile Ile Thr Leu Val Val Leu Leu Leu Lys Tyr Arg Arg Arg
245 250 255

His Arg Lys His Ser Pro Gln His Thr Thr Thr Leu Ser Leu Ser
260 265 270

Thr Leu Ala Thr Pro Lys Arg Ser Gly Asn Asn Asn Gly Ser Glu
275 280 285

Pro Ser Asp Ile Ile Ile Pro Leu Arg Thr Ala Asp Ser Val Phe
290 295 300

Cys Pro His Tyr Glu Lys Val Ser Gly Asp Tyr Gly His Pro Val
305 310 315

Tyr Ile Val Gln Glu Met Pro Pro Gln Ser Pro Ala Asn Ile Tyr
320 325 330

Tyr Lys Val

<210> 64
<211> 314
<212> PRT
<213> Homo sapien

<400> 64
Met Gly Ala Arg Gly Ala Leu Leu Leu Ala Leu Leu Leu Ala Arg
1 5 10 15

Ala Gly Leu Arg Lys Pro Glu Ser Gln Glu Ala Ala Pro Leu Ser
20 25 30

Gly Pro Cys Gly Arg Arg Val Ile Thr Ser Arg Ile Val Gly Gly
35 40 45

Glu Asp Ala Glu Leu Gly Arg Trp Pro Trp Gln Gly Ser Leu Arg
50 55 60

Leu Trp Asp Ser His Val Cys Gly Val Ser Leu Leu Ser His Arg
65 70 75

Trp Ala Leu Thr Ala Ala His Cys Phe Glu Thr Tyr Ser Asp Leu
80 85 90

Ser Asp Pro Ser Gly Trp Met Val Gln Phe Gly Gln Leu Thr Ser
95 100 105

Met Pro Ser Phe Trp Ser Leu Gln Ala Tyr Tyr Thr Arg Tyr Phe
110 115 120

Val Ser Asn Ile Tyr Leu Ser Pro Arg Tyr Leu Gly Asn Ser Pro
125 130 135

Tyr Asp Ile Ala Leu Val Lys Leu Ser Ala Pro Val Thr Tyr Thr
140 145 150

Lys His Ile Gln Pro Ile Cys Leu Gln Ala Ser Thr Phe Glu Phe
155 160 165

Glu Asn Arg Thr Asp Cys Trp Val Thr Gly Trp Gly Tyr Ile Lys
170 175 180

Glu Asp Glu Ala Leu Pro Ser Pro His Thr Leu Gln Glu Val Gln
185 190 195

Val Ala Ile Ile Asn Asn Ser Met Cys Asn His Leu Phe Leu Lys
200 205 210

Tyr Ser Phe Arg Lys Asp Ile Phe Gly Asp Met Val Cys Ala Gly
215 220 225

Asn Ala Gln Gly Gly Lys Asp Ala Cys Phe Gly Asp Ser Gly Gly
230 235 240

Pro Leu Ala Cys Asn Lys Asn Gly Leu Trp Tyr Gln Ile Gly Val
245 250 255

Val Ser Trp Gly Val Gly Cys Gly Arg Pro Asn Arg Pro Gly Val
260 265 270

Tyr Thr Asn Ile Ser His His Phe Glu Trp Ile Gln Lys Leu Met
275 280 285

Ala Gln Ser Gly Met Ser Gln Pro Asp Pro Ser Trp Pro Leu Leu
290 295 300

Phe Phe Pro Leu Leu Trp Ala Leu Pro Leu Leu Gly Pro Val
305 310

<210> 65
<211> 432
<212> PRT
<213> Homo sapien

<400> 65
Met Leu Gln Asp Pro Asp Ser Asp Gln Pro Leu Asn Ser Leu Asp
1 5 10 15

Val Lys Pro Leu Arg Lys Pro Arg Ile Pro Met Glu Thr Phe Arg
20 25 30

Lys Val Gly Ile Pro Ile Ile Ile Ala Leu Leu Ser Leu Ala Ser
35 40 45

Ile Ile Ile Val Val Val Leu Ile Lys Val Ile Leu Asp Lys Tyr
50 55 60

Tyr Phe Leu Cys Gly Gln Pro Leu His Phe Ile Pro Arg Lys Gln
65 70 75

Leu Cys Asp Gly Glu Leu Asp Cys Pro Leu Gly Glu Asp Glu Glu
80 85 90

His Cys Val Lys Ser Phe Pro Glu Gly Pro Ala Val Ala Val Arg
95 100 105

Leu Ser Lys Asp Arg Ser Thr Leu Gln Val Leu Asp Ser Ala Thr
110 115 120

Gly Asn Trp Phe Ser Ala Cys Phe Asp Asn Phe Thr Glu Ala Leu
125 130 135

Ala Glu Thr Ala Cys Arg Gln Met Gly Tyr Ser Arg Ala Val Glu

```
                140                 145                 150

Ile Gly Pro Asp Gln Asp Leu Asp Val Val Glu Ile Thr Glu Asn
                155                 160                 165

Ser Gln Glu Leu Arg Met Arg Asn Ser Ser Gly Pro Cys Leu Ser
                170                 175                 180

Gly Ser Leu Val Ser Leu His Cys Leu Ala Cys Gly Lys Ser Leu
                185                 190                 195

Lys Thr Pro Arg Val Val Gly Gly Glu Glu Ala Ser Val Asp Ser
                200                 205                 210

Trp Pro Trp Gln Val Ser Ile Gln Tyr Asp Lys Gln His Val Cys
                215                 220                 225

Gly Gly Ser Ile Leu Asp Pro His Trp Val Leu Thr Ala Ala His
                230                 235                 240

Cys Phe Arg Lys His Thr Asp Val Phe Asn Trp Lys Val Arg Ala
                245                 250                 255

Gly Ser Asp Lys Leu Gly Ser Phe Pro Ser Leu Ala Val Ala Lys
                260                 265                 270

Ile Ile Ile Ile Glu Phe Asn Pro Met Tyr Pro Lys Asp Asn Asp
                275                 280                 285

Ile Ala Leu Met Lys Leu Gln Phe Pro Leu Thr Phe Ser Gly Thr
                290                 295                 300

Val Arg Pro Ile Cys Leu Pro Phe Phe Asp Glu Glu Leu Thr Pro
                305                 310                 315

Ala Thr Pro Leu Trp Ile Ile Gly Trp Gly Phe Thr Lys Gln Asn
                320                 325                 330

Gly Gly Lys Met Ser Asp Ile Leu Leu Gln Ala Ser Val Gln Val
                335                 340                 345

Ile Asp Ser Thr Arg Cys Asn Ala Asp Asp Ala Tyr Gln Gly Glu
                350                 355                 360

Val Thr Glu Lys Met Met Cys Ala Gly Ile Pro Glu Gly Gly Val
                365                 370                 375

Asp Thr Cys Gln Gly Asp Ser Gly Gly Pro Leu Met Tyr Gln Ser
                380                 385                 390

Asp Gln Trp His Val Val Gly Ile Val Ser Trp Gly Tyr Gly Cys
                395                 400                 405

Gly Gly Pro Ser Thr Pro Gly Val Tyr Thr Lys Val Ser Ala Tyr
                410                 415                 420

Leu Asn Trp Ile Tyr Asn Val Trp Lys Ala Glu Leu
                425                 430
```

<210> 66
<211> 320

<212> PRT
<213> Homo sapien

<400> 66
```
Met Ala Gly Leu Ala Ala Arg Leu Val Leu Leu Ala Gly Ala Ala
1               5                   10                  15

Ala Leu Ala Ser Gly Ser Gln Gly Asp Arg Glu Pro Val Tyr Arg
                20                  25                  30

Asp Cys Val Leu Gln Cys Glu Glu Gln Asn Cys Ser Gly Gly Ala
                35                  40                  45

Leu Asn His Phe Arg Ser Arg Gln Pro Ile Tyr Met Ser Leu Ala
                50                  55                  60

Gly Trp Thr Cys Arg Asp Asp Cys Lys Tyr Glu Cys Met Trp Val
                65                  70                  75

Thr Val Gly Leu Tyr Leu Gln Glu Gly His Lys Val Pro Gln Phe
                80                  85                  90

His Gly Lys Trp Pro Phe Ser Arg Phe Leu Phe Phe Gln Glu Pro
                95                  100                 105

Ala Ser Ala Val Ala Ser Phe Leu Asn Gly Leu Ala Ser Leu Val
                110                 115                 120

Met Leu Cys Arg Tyr Arg Thr Phe Val Pro Ala Ser Ser Pro Met
                125                 130                 135

Tyr His Thr Cys Val Ala Phe Ala Trp Val Ser Leu Asn Ala Trp
                140                 145                 150

Phe Trp Ser Thr Val Phe His Thr Arg Asp Thr Asp Leu Thr Glu
                155                 160                 165

Lys Met Asp Tyr Phe Cys Ala Ser Thr Val Ile Leu His Ser Ile
                170                 175                 180

Tyr Leu Cys Cys Val Arg Thr Val Gly Leu Gln His Pro Ala Val
                185                 190                 195

Val Ser Ala Phe Arg Ala Leu Leu Leu Leu Met Leu Thr Val His
                200                 205                 210

Val Ser Tyr Leu Ser Leu Ile Arg Phe Asp Tyr Gly Tyr Asn Leu
                215                 220                 225

Val Ala Asn Val Ala Ile Gly Leu Val Asn Val Val Trp Trp Leu
                230                 235                 240

Ala Trp Cys Leu Trp Asn Gln Arg Arg Leu Pro His Val Arg Lys
                245                 250                 255

Cys Val Val Val Val Leu Leu Leu Gln Gly Leu Ser Leu Leu Glu
                260                 265                 270

Leu Leu Asp Phe Pro Pro Leu Phe Trp Val Leu Asp Ala His Ala
                275                 280                 285
```

Ile Trp His Ile Ser Thr Ile Pro Val His Val Leu Phe Phe Ser
290 295 300

Phe Leu Glu Asp Asp Ser Leu Tyr Leu Leu Lys Glu Ser Glu Asp
305 310 315

Lys Phe Lys Leu Asp
320

<210> 67
<211> 193
<212> PRT
<213> Homo sapien

<400> 67
Met Ile Arg Cys Gly Leu Ala Cys Glu Arg Cys Arg Trp Ile Leu
1 5 10 15

Pro Leu Leu Leu Leu Ser Ala Ile Ala Phe Asp Ile Ile Ala Leu
20 25 30

Ala Gly Arg Gly Trp Leu Gln Ser Ser Asp His Gly Gln Thr Ser
35 40 45

Ser Leu Trp Trp Lys Cys Ser Gln Glu Gly Gly Gly Ser Gly Ser
50 55 60

Tyr Glu Glu Gly Cys Gln Ser Leu Met Glu Tyr Ala Trp Gly Arg
65 70 75

Ala Ala Ala Ala Met Leu Phe Cys Gly Phe Ile Ile Leu Val Ile
80 85 90

Cys Phe Ile Leu Ser Phe Phe Ala Leu Cys Gly Pro Gln Met Leu
95 100 105

Val Phe Leu Arg Val Ile Gly Gly Leu Leu Ala Leu Ala Ala Val
110 115 120

Phe Gln Ile Ile Ser Leu Val Ile Tyr Pro Val Lys Tyr Thr Gln
125 130 135

Thr Phe Thr Leu His Ala Asn Pro Ala Val Thr Tyr Ile Tyr Asn
140 145 150

Trp Ala Tyr Gly Phe Gly Trp Ala Ala Thr Ile Ile Leu Ile Gly
155 160 165

Cys Ala Phe Phe Phe Cys Cys Leu Leu Asn Tyr Glu Asp Asp Leu
170 175 180

Leu Gly Asn Ala Lys Pro Arg Tyr Phe Tyr Thr Ser Ala
185 190

<210> 68
<211> 915
<212> PRT
<213> Homo sapien

<400> 68
Met Gly Arg Pro Arg Leu Thr Leu Val Cys Gln Val Ser Ile Ile

1 5 10 15

Ile Ser Ala Arg Asp Leu Ser Met Asn Asn Leu Thr Glu Leu Gln
20 25 30

Pro Gly Leu Phe His His Leu Arg Phe Leu Glu Glu Leu Arg Leu
35 40 45

Ser Gly Asn His Leu Ser His Ile Pro Gly Gln Ala Phe Ser Gly
50 55 60

Leu Tyr Ser Leu Lys Ile Leu Met Leu Gln Asn Asn Gln Leu Gly
65 70 75

Gly Ile Pro Ala Glu Ala Leu Trp Glu Leu Pro Ser Leu Gln Ser
80 85 90

Leu Arg Leu Asp Ala Asn Leu Ile Ser Leu Val Pro Glu Arg Ser
95 100 105

Phe Glu Gly Leu Ser Ser Leu Arg His Leu Trp Leu Asp Asp Asn
110 115 120

Ala Leu Thr Glu Ile Pro Val Arg Ala Leu Asn Asn Leu Pro Ala
125 130 135

Leu Gln Ala Met Thr Leu Ala Leu Asn Arg Ile Ser His Ile Pro
140 145 150

Asp Tyr Ala Phe Gln Asn Leu Thr Ser Leu Val Val Leu His Leu
155 160 165

His Asn Asn Arg Ile Gln His Leu Gly Thr His Ser Phe Glu Gly
170 175 180

Leu His Asn Leu Glu Thr Leu Asp Leu Asn Tyr Asn Lys Leu Gln
185 190 195

Glu Phe Pro Val Ala Ile Arg Thr Leu Gly Arg Leu Gln Glu Leu
200 205 210

Gly Phe His Asn Asn Asn Ile Lys Ala Ile Pro Glu Lys Ala Phe
215 220 225

Met Gly Asn Pro Leu Leu Gln Thr Ile His Phe Tyr Asp Asn Pro
230 235 240

Ile Gln Phe Val Gly Arg Ser Ala Phe Gln Tyr Leu Pro Lys Leu
245 250 255

His Thr Leu Ser Leu Asn Gly Ala Met Asp Ile Gln Glu Phe Pro
260 265 270

Asp Leu Lys Gly Thr Thr Ser Leu Glu Ile Leu Thr Leu Thr Arg
275 280 285

Ala Gly Ile Arg Leu Leu Pro Ser Gly Met Cys Gln Gln Leu Pro
290 295 300

Arg Leu Arg Val Leu Glu Leu Ser His Asn Gln Ile Glu Glu Leu
305 310 315

Pro Ser Leu His Arg Cys Gln Lys Leu Glu Glu Ile Gly Leu Gln
320 325 330

His Asn Arg Ile Trp Glu Ile Gly Ala Asp Thr Phe Ser Gln Leu
335 340 345

Ser Ser Leu Gln Ala Leu Asp Leu Ser Trp Asn Ala Ile Arg Ser
350 355 360

Ile His Pro Glu Ala Phe Ser Thr Leu His Ser Leu Val Lys Leu
365 370 375

Asp Leu Thr Asp Asn Gln Leu Thr Thr Leu Pro Leu Ala Gly Leu
380 385 390

Gly Gly Leu Met His Leu Lys Leu Lys Gly Asn Leu Ala Leu Ser
395 400 405

Gln Ala Phe Ser Lys Asp Ser Phe Pro Lys Leu Arg Ile Leu Glu
410 415 420

Val Pro Tyr Ala Tyr Gln Cys Cys Pro Tyr Gly Met Cys Ala Ser
425 430 435

Phe Phe Lys Ala Ser Gly Gln Trp Glu Ala Glu Asp Leu His Leu
440 445 450

Asp Asp Glu Glu Ser Ser Lys Arg Pro Leu Gly Leu Leu Ala Arg
455 460 465

Gln Ala Glu Asn His Tyr Asp Gln Asp Leu Asp Glu Leu Gln Leu
470 475 480

Glu Met Glu Asp Ser Lys Pro His Pro Ser Val Gln Cys Ser Pro
485 490 495

Thr Pro Gly Pro Phe Lys Pro Cys Glu Tyr Leu Phe Glu Ser Trp
500 505 510

Gly Ile Arg Leu Ala Val Trp Ala Ile Val Leu Leu Ser Val Leu
515 520 525

Cys Asn Gly Leu Val Leu Leu Thr Val Phe Ala Gly Gly Pro Ala
530 535 540

Pro Leu Pro Pro Val Lys Phe Val Val Gly Ala Ile Ala Gly Ala
545 550 555

Asn Thr Leu Thr Gly Ile Ser Cys Gly Leu Leu Ala Ser Val Asp
560 565 570

Ala Leu Thr Phe Gly Gln Phe Ser Glu Tyr Gly Ala Arg Trp Glu
575 580 585

Thr Gly Leu Gly Cys Arg Ala Thr Gly Phe Leu Ala Val Leu Gly
590 595 600

Ser Glu Ala Ser Val Leu Leu Leu Thr Leu Ala Ala Val Gln Cys
605 610 615

Ser Val Ser Val Ser Cys Val Arg Ala Tyr Gly Lys Ser Pro Ser
620 625 630

Leu Gly Ser Val Arg Ala Gly Val Leu Gly Cys Leu Ala Leu Ala
635 640 645

Gly Leu Ala Ala Ala Leu Pro Leu Ala Ser Val Gly Glu Tyr Gly
650 655 660

Ala Ser Pro Leu Cys Leu Pro Tyr Ala Pro Pro Glu Gly Gln Pro
665 670 675

Ala Ala Leu Gly Phe Thr Val Ala Leu Val Met Met Asn Ser Phe
680 685 690

Cys Phe Leu Val Val Ala Gly Ala Tyr Ile Lys Leu Tyr Cys Asp
695 700 705

Leu Pro Arg Gly Asp Phe Glu Ala Val Trp Asp Cys Ala Met Val
710 715 720

Arg His Val Ala Trp Leu Ile Phe Ala Asp Gly Leu Leu Tyr Cys
725 730 735

Pro Val Ala Phe Leu Ser Phe Ala Ser Met Leu Gly Leu Phe Pro
740 745 750

Val Thr Pro Glu Ala Val Lys Ser Val Leu Leu Val Val Leu Pro
755 760 765

Leu Pro Ala Cys Leu Asn Pro Leu Leu Tyr Leu Leu Phe Asn Pro
770 775 780

His Phe Arg Asp Asp Leu Arg Arg Leu Arg Pro Arg Ala Gly Asp
785 790 795

Ser Gly Pro Leu Ala Tyr Ala Ala Ala Gly Glu Leu Glu Lys Ser
800 805 810

Ser Cys Asp Ser Thr Gln Ala Leu Val Ala Phe Ser Asp Val Asp
815 820 825

Leu Ile Leu Glu Ala Ser Glu Ala Gly Arg Pro Pro Gly Leu Glu
830 835 840

Thr Tyr Gly Phe Pro Ser Val Thr Leu Ile Ser Cys Gln Gln Pro
845 850 855

Gly Ala Pro Arg Leu Glu Gly Ser His Cys Val Glu Pro Glu Gly
860 865 870

Asn His Phe Gly Asn Pro Gln Pro Ser Met Asp Gly Glu Leu Leu
875 880 885

Leu Arg Ala Glu Gly Ser Thr Pro Ala Gly Gly Gly Leu Ser Gly
890 895 900

Gly Gly Gly Phe Gln Pro Ser Gly Leu Ala Phe Ala Ser His Val
905 910 915

<210> 69

<211> 377
<212> PRT
<213> Homo sapien

<400> 69
Met Glu Ala Leu Leu Leu Gly Ala Gly Leu Leu Leu Gly Ala Tyr
1               5                   10                  15

Val Leu Val Tyr Tyr Asn Leu Val Lys Ala Pro Pro Cys Gly Gly
            20                  25                  30

Met Gly Asn Leu Arg Gly Arg Thr Ala Val Val Thr Gly Ala Asn
        35                  40                  45

Ser Gly Ile Gly Lys Met Thr Ala Leu Glu Leu Ala Arg Arg Gly
    50                  55                  60

Ala Arg Val Val Leu Ala Cys Arg Ser Gln Glu Arg Gly Glu Ala
65                  70                  75

Ala Ala Phe Asp Leu Arg Gln Glu Ser Gly Asn Asn Glu Val Ile
80                  85                  90

Phe Met Ala Leu Asp Leu Ala Ser Leu Ala Ser Val Arg Ala Phe
95                  100                 105

Ala Thr Ala Phe Leu Ser Ser Glu Pro Arg Leu Asp Ile Leu Ile
110                 115                 120

His Asn Ala Gly Ile Ser Ser Cys Gly Arg Thr Arg Glu Ala Phe
125                 130                 135

Asn Leu Leu Leu Arg Val Asn His Ile Gly Pro Phe Leu Leu Thr
140                 145                 150

His Leu Leu Leu Pro Cys Leu Lys Ala Cys Ala Pro Ser Arg Val
155                 160                 165

Val Val Val Ala Ser Ala Ala His Cys Arg Gly Arg Leu Asp Phe
170                 175                 180

Lys Arg Leu Asp Arg Pro Val Val Gly Trp Arg Gln Glu Leu Arg
185                 190                 195

Ala Tyr Ala Asp Thr Lys Leu Ala Asn Val Leu Phe Ala Arg Glu
200                 205                 210

Leu Ala Asn Gln Leu Glu Ala Thr Gly Val Thr Cys Tyr Ala Ala
215                 220                 225

His Pro Gly Pro Val Asn Ser Glu Leu Phe Leu Arg His Val Pro
230                 235                 240

Gly Trp Leu Arg Pro Leu Leu Arg Pro Leu Ala Trp Leu Val Leu
245                 250                 255

Arg Ala Pro Arg Gly Gly Ala Gln Thr Pro Leu Tyr Cys Ala Leu
260                 265                 270

Gln Glu Gly Ile Glu Pro Leu Ser Gly Arg Tyr Phe Ala Asn Cys
275                 280                 285

His Val Glu Glu Val Pro Pro Ala Ala Arg Asp Asp Arg Ala Ala
290 295 300

His Arg Leu Trp Glu Ala Ser Lys Arg Leu Ala Gly Leu Gly Pro
305 310 315

Gly Glu Asp Ala Glu Pro Asp Glu Asp Pro Gln Ser Glu Asp Ser
320 325 330

Glu Ala Pro Ser Ser Leu Ser Thr Pro His Pro Glu Glu Pro Thr
335 340 345

Val Ser Gln Pro Tyr Pro Ser Pro Gln Ser Ser Pro Asp Leu Ser
350 355 360

Lys Met Thr His Arg Ile Gln Ala Lys Val Glu Pro Glu Ile Gln
365 370 375

Leu Ser

<210> 70
<211> 180
<212> PRT
<213> Homo sapien

<400> 70
Met Ala Ala Ser Leu Gly Gln Val Leu Ala Leu Val Leu Val Ala
1 5 10 15

Ala Leu Trp Gly Gly Thr Gln Pro Leu Leu Lys Arg Ala Ser Ala
20 25 30

Gly Leu Gln Arg Val His Glu Pro Thr Trp Ala Gln Gln Leu Leu
35 40 45

Gln Glu Met Lys Thr Leu Phe Leu Asn Thr Glu Tyr Leu Met Pro
50 55 60

Phe Leu Leu Asn Gln Cys Gly Ser Leu Leu Tyr Tyr Leu Thr Leu
65 70 75

Ala Ser Thr Asp Leu Thr Leu Ala Val Pro Ile Cys Asn Ser Leu
80 85 90

Ala Ile Ile Phe Thr Leu Ile Val Gly Lys Ala Leu Gly Glu Asp
95 100 105

Ile Gly Gly Lys Arg Lys Leu Asp Tyr Cys Glu Cys Gly Thr Gln
110 115 120

Leu Cys Gly Ser Arg His Thr Cys Val Ser Ser Phe Pro Glu Pro
125 130 135

Ile Ser Pro Glu Trp Val Arg Thr Arg Pro Phe Pro Ile Leu Pro
140 145 150

Phe Pro Leu Gln Leu Phe Cys Phe Leu Val Ala Ile Arg Val Pro
155 160 165

Phe Pro Trp Thr Val Trp Arg Lys Thr Glu Ala Gly Val Trp Asp
170 175 180

<210> 71
<211> 1403
<212> PRT
<213> Homo sapien

<400> 71
Met Val Ser Ser Gly Cys Arg Met Arg Ser Leu Trp Phe Ile Ile
1 5 10 15

Val Ile Ser Phe Leu Pro Asn Thr Glu Gly Phe Ser Arg Ala Ala
20 25 30

Leu Pro Phe Gly Leu Val Arg Arg Glu Leu Ser Cys Glu Gly Tyr
35 40 45

Ser Ile Asp Leu Arg Cys Pro Gly Ser Asp Val Ile Met Ile Glu
50 55 60

Ser Ala Asn Tyr Gly Arg Thr Asp Asp Lys Ile Cys Asp Ala Asp
65 70 75

Pro Phe Gln Met Glu Asn Thr Asp Cys Tyr Leu Pro Asp Ala Phe
80 85 90

Lys Ile Met Thr Gln Arg Cys Asn Asn Arg Thr Gln Cys Ile Val
95 100 105

Val Thr Gly Ser Asp Val Phe Pro Asp Pro Cys Pro Gly Thr Tyr
110 115 120

Lys Tyr Leu Glu Val Gln Tyr Glu Cys Val Pro Tyr Ile Phe Val
125 130 135

Cys Pro Gly Thr Leu Lys Ala Ile Val Asp Ser Pro Cys Ile Tyr
140 145 150

Glu Ala Glu Gln Lys Ala Gly Ala Trp Cys Lys Asp Pro Leu Gln
155 160 165

Ala Ala Asp Lys Ile Tyr Phe Met Pro Trp Thr Pro Tyr Arg Thr
170 175 180

Asp Thr Leu Ile Glu Tyr Ala Ser Leu Glu Asp Phe Gln Asn Ser
185 190 195

Arg Gln Thr Thr Thr Tyr Lys Leu Pro Asn Arg Val Asp Gly Thr
200 205 210

Gly Phe Val Val Tyr Asp Gly Ala Val Phe Phe Asn Lys Glu Arg
215 220 225

Thr Arg Asn Ile Val Lys Phe Asp Leu Arg Thr Arg Ile Lys Ser
230 235 240

Gly Glu Ala Ile Ile Asn Tyr Ala Asn Tyr His Asp Thr Ser Pro
245 250 255

Tyr Arg Trp Gly Gly Lys Thr Asp Ile Asp Leu Ala Val Asp Glu

260 265 270

Asn Gly Leu Trp Val Ile Tyr Ala Thr Glu Gln Asn Asn Gly Met
275 280 285

Ile Val Ile Ser Gln Leu Asn Pro Tyr Thr Leu Arg Phe Glu Ala
290 295 300

Thr Trp Glu Thr Val Tyr Asp Lys Arg Ala Ala Ser Asn Ala Phe
305 310 315

Met Ile Cys Gly Val Leu Tyr Val Val Arg Ser Val Tyr Gln Asp
320 325 330

Asn Glu Ser Glu Thr Gly Lys Asn Ser Ile Asp Tyr Ile Tyr Asn
335 340 345

Thr Arg Leu Asn Arg Gly Glu Tyr Val Asp Val Pro Phe Pro Asn
350 355 360

Gln Tyr Gln Tyr Ile Ala Ala Val Asp Tyr Asn Pro Arg Asp Asn
365 370 375

Gln Leu Tyr Val Trp Asn Asn Asn Phe Ile Leu Arg Tyr Ser Leu
380 385 390

Glu Phe Gly Pro Pro Asp Pro Ala Gln Val Pro Thr Thr Ala Val
395 400 405

Thr Ile Thr Ser Ser Ala Glu Leu Phe Lys Thr Ile Ile Ser Thr
410 415 420

Thr Ser Thr Thr Ser Gln Lys Gly Pro Met Ser Thr Thr Val Ala
425 430 435

Gly Ser Gln Glu Gly Ser Lys Gly Thr Lys Pro Pro Pro Ala Val
440 445 450

Ser Thr Thr Lys Ile Pro Pro Ile Thr Asn Ile Phe Pro Leu Pro
455 460 465

Glu Arg Phe Cys Glu Ala Leu Asp Ser Lys Gly Ile Lys Trp Pro
470 475 480

Gln Thr Gln Arg Gly Met Met Val Glu Arg Pro Cys Pro Lys Gly
485 490 495

Thr Arg Gly Thr Ala Ser Tyr Leu Cys Met Ile Ser Thr Gly Thr
500 505 510

Trp Asn Pro Lys Gly Pro Asp Leu Ser Asn Cys Thr Ser His Trp
515 520 525

Val Asn Gln Leu Ala Gln Lys Ile Arg Ser Gly Glu Asn Ala Ala
530 535 540

Ser Leu Ala Asn Glu Leu Ala Lys His Thr Lys Gly Pro Val Phe
545 550 555

Ala Gly Asp Val Ser Ser Ser Val Arg Leu Met Glu Gln Leu Val
560 565 570

Asp Ile Leu Asp Ala Gln Leu Gln Glu Leu Lys Pro Ser Glu Lys
575 580 585

Asp Ser Ala Gly Arg Ser Tyr Asn Lys Ala Ile Val Asp Thr Val
590 595 600

Asp Asn Leu Leu Arg Pro Glu Ala Leu Glu Ser Trp Lys His Met
605 610 615

Asn Ser Ser Glu Gln Ala His Thr Ala Thr Met Leu Leu Asp Thr
620 625 630

Leu Glu Glu Gly Ala Phe Val Leu Ala Asp Asn Leu Leu Glu Pro
635 640 645

Thr Arg Val Ser Met Pro Thr Glu Asn Ile Val Leu Glu Val Ala
650 655 660

Val Leu Ser Thr Glu Gly Gln Ile Gln Asp Phe Lys Phe Pro Leu
665 670 675

Gly Ile Lys Gly Ala Gly Ser Ser Ile Gln Leu Ser Ala Asn Thr
680 685 690

Val Lys Gln Asn Ser Arg Asn Gly Leu Ala Lys Leu Val Phe Ile
695 700 705

Ile Tyr Arg Ser Leu Gly Gln Phe Leu Ser Thr Glu Asn Ala Thr
710 715 720

Ile Lys Leu Gly Ala Asp Phe Ile Gly Arg Asn Ser Thr Ile Ala
725 730 735

Val Asn Ser His Val Ile Ser Val Ser Ile Asn Lys Glu Ser Ser
740 745 750

Arg Val Tyr Leu Thr Asp Pro Val Leu Phe Thr Leu Pro His Ile
755 760 765

Asp Pro Asp Asn Tyr Phe Asn Ala Asn Cys Ser Phe Trp Asn Tyr
770 775 780

Ser Glu Arg Thr Met Met Gly Tyr Trp Ser Thr Gln Gly Cys Lys
785 790 795

Leu Val Asp Thr Asn Lys Thr Arg Thr Thr Cys Ala Cys Ser His
800 805 810

Leu Thr Asn Phe Ala Ile Leu Met Ala His Arg Glu Ile Ala Tyr
815 820 825

Lys Asp Gly Val His Glu Leu Leu Leu Thr Val Ile Thr Trp Val
830 835 840

Gly Ile Val Ile Ser Leu Val Cys Leu Ala Ile Cys Ile Phe Thr
845 850 855

Phe Cys Phe Phe Arg Gly Leu Gln Ser Asp Arg Asn Thr Ile His
860 865 870

Lys Asn Leu Cys Ile Asn Leu Phe Ile Ala Glu Phe Ile Phe Leu
875 880 885

Ile Gly Ile Asp Lys Thr Lys Tyr Ala Ile Ala Cys Pro Ile Phe
890 895 900

Ala Gly Leu Leu His Phe Phe Phe Leu Ala Ala Phe Ala Trp Met
905 910 915

Cys Leu Glu Gly Val Gln Leu Tyr Leu Met Leu Val Glu Val Phe
920 925 930

Glu Ser Glu Tyr Ser Arg Lys Lys Tyr Tyr Tyr Val Ala Gly Tyr
935 940 945

Leu Phe Pro Ala Thr Val Val Gly Val Ser Ala Ala Ile Asp Tyr
950 955 960

Lys Ser Tyr Gly Thr Glu Lys Ala Cys Trp Leu His Val Asp Asn
965 970 975

Tyr Phe Ile Trp Ser Phe Ile Gly Pro Val Thr Phe Ile Ile Leu
980 985 990

Leu Asn Ile Ile Phe Leu Val Ile Thr Leu Cys Lys Met Val Lys
995 1000 1005

His Ser Asn Thr Leu Lys Pro Asp Ser Ser Arg Leu Glu Asn Ile
1010 1015 1020

Lys Ser Trp Val Leu Gly Ala Phe Ala Leu Leu Cys Leu Leu Gly
1025 1030 1035

Leu Thr Trp Ser Phe Gly Leu Leu Phe Ile Asn Glu Glu Thr Ile
1040 1045 1050

Val Met Ala Tyr Leu Phe Thr Ile Phe Asn Ala Phe Gln Gly Val
1055 1060 1065

Phe Ile Phe Ile Phe His Cys Ala Leu Gln Lys Lys Val Arg Lys
1070 1075 1080

Glu Tyr Gly Lys Cys Phe Arg His Ser Tyr Cys Cys Gly Gly Leu
1085 1090 1095

Pro Thr Glu Ser Pro His Ser Ser Val Lys Ala Ser Thr Thr Arg
1100 1105 1110

Thr Ser Ala Arg Tyr Ser Ser Gly Thr Gln Ser Arg Ile Arg Arg
1115 1120 1125

Met Trp Asn Asp Thr Val Arg Lys Gln Ser Glu Ser Ser Phe Ile
1130 1135 1140

Ser Gly Asp Ile Asn Ser Thr Ser Thr Leu Asn Gln Gly His Ser
1145 1150 1155

Leu Asn Asn Ala Arg Asp Thr Ser Ala Met Asp Thr Leu Pro Leu
1160 1165 1170

Asn Gly Asn Phe Asn Asn Ser Tyr Ser Leu His Lys Gly Asp Tyr

1175 1180 1185

Asn Asp Ser Val Gln Val Val Asp Cys Gly Leu Ser Leu Asn Asp
1190 1195 1200

Thr Ala Phe Glu Lys Met Ile Ile Ser Glu Leu Val His Asn Asn
1205 1210 1215

Leu Arg Gly Ser Ser Lys Thr His Asn Leu Glu Leu Thr Leu Pro
1220 1225 1230

Val Lys Pro Val Ile Gly Gly Ser Ser Ser Glu Asp Asp Ala Ile
1235 1240 1245

Val Ala Asp Ala Ser Ser Leu Met His Ser Asp Asn Pro Gly Leu
1250 1255 1260

Glu Leu His His Lys Glu Leu Glu Ala Pro Leu Ile Pro Gln Arg
1265 1270 1275

Thr His Ser Leu Leu Tyr Gln Pro Gln Lys Lys Val Lys Ser Glu
1280 1285 1290

Gly Thr Asp Ser Tyr Val Ser Gln Leu Thr Ala Glu Ala Glu Asp
1295 1300 1305

His Leu Gln Ser Pro Asn Arg Asp Ser Leu Tyr Thr Ser Met Pro
1310 1315 1320

Asn Leu Arg Asp Ser Pro Tyr Pro Glu Ser Ser Pro Asp Met Glu
1325 1330 1335

Glu Asp Leu Ser Pro Ser Arg Arg Ser Glu Asn Glu Asp Ile Tyr
1340 1345 1350

Tyr Lys Ser Met Pro Asn Leu Gly Ala Gly His Gln Leu Gln Met
1355 1360 1365

Cys Tyr Gln Ile Ser Arg Gly Asn Ser Asp Gly Tyr Ile Ile Pro
1370 1375 1380

Ile Asn Lys Glu Gly Cys Ile Pro Glu Gly Asp Val Arg Glu Gly
1385 1390 1395

Gln Met Gln Leu Val Thr Ser Leu
1400

<210> 72
<211> 283
<212> PRT
<213> Homo sapien

<400> 72
Met Ala Asp Pro His Gln Leu Phe Asp Asp Thr Ser Ser Ala Gln
1 5 10 15

Ser Arg Gly Tyr Gly Ala Gln Arg Ala Pro Gly Gly Leu Ser Tyr
20 25 30

Pro Ala Ala Ser Pro Thr Pro His Ala Ala Phe Leu Ala Asp Pro
35 40 45

Val Ser Asn Met Ala Met Ala Tyr Gly Ser Ser Leu Ala Ala Gln
50 55 60

Gly Lys Glu Leu Val Asp Lys Asn Ile Asp Arg Phe Ile Pro Ile
65 70 75

Thr Lys Leu Lys Tyr Tyr Phe Ala Val Asp Thr Met Tyr Val Gly
80 85 90

Arg Lys Leu Gly Leu Leu Phe Phe Pro Tyr Leu His Gln Asp Trp
95 100 105

Glu Val Gln Tyr Gln Gln Asp Thr Pro Val Ala Pro Arg Phe Asp
110 115 120

Val Asn Ala Pro Asp Leu Tyr Ile Pro Ala Met Ala Phe Ile Thr
125 130 135

Tyr Val Leu Val Ala Gly Leu Ala Leu Gly Thr Gln Asp Arg Phe
140 145 150

Ser Pro Asp Leu Leu Gly Leu Gln Ala Ser Ser Ala Leu Ala Trp
155 160 165

Leu Thr Leu Glu Val Leu Ala Ile Leu Leu Ser Leu Tyr Leu Val
170 175 180

Thr Val Asn Thr Asp Leu Thr Thr Ile Asp Leu Val Ala Phe Leu
185 190 195

Gly Tyr Lys Tyr Val Gly Met Ile Gly Gly Val Leu Met Gly Leu
200 205 210

Leu Phe Gly Lys Ile Gly Tyr Tyr Leu Val Leu Gly Trp Cys Cys
215 220 225

Val Ala Ile Phe Val Phe Met Ile Arg Thr Leu Arg Leu Lys Ile
230 235 240

Leu Ala Asp Ala Ala Ala Glu Gly Val Pro Val Arg Gly Ala Arg
245 250 255

Asn Gln Leu Arg Met Tyr Leu Thr Met Ala Val Ala Ala Ala Gln
260 265 270

Pro Met Leu Met Tyr Trp Leu Thr Phe His Leu Val Arg
275 280

<210> 73
<211> 336
<212> PRT
<213> Homo sapien

<400> 73
Met Ala Leu Leu Pro Ile Phe Phe Gly Ala Leu Arg Ser Val Arg
1 5 10 15

Cys Ala Arg Gly Lys Asn Ala Ser Asp Met Pro Glu Thr Ile Thr
20 25 30

Ser Arg Asp Ala Ala Arg Phe Pro Ile Ile Ala Ser Cys Thr Leu
35 40 45

Leu Gly Leu Tyr Leu Phe Phe Lys Ile Phe Ser Gln Glu Tyr Ile
50 55 60

Asn Leu Leu Leu Ser Met Tyr Phe Phe Val Leu Gly Ile Leu Ala
65 70 75

Leu Ser His Thr Ile Ser Pro Phe Met Asn Lys Phe Phe Pro Ala
80 85 90

Ser Phe Pro Asn Arg Gln Tyr Gln Leu Leu Phe Thr Gln Gly Ser
95 100 105

Gly Glu Asn Lys Glu Glu Ile Ile Asn Tyr Glu Phe Asp Thr Lys
110 115 120

Asp Leu Val Cys Leu Gly Leu Ser Ser Ile Val Gly Val Trp Tyr
125 130 135

Leu Leu Arg Lys His Trp Ile Ala Asn Asn Leu Phe Gly Leu Ala
140 145 150

Phe Ser Leu Asn Gly Val Glu Leu Leu His Leu Asn Asn Val Ser
155 160 165

Thr Gly Cys Ile Leu Leu Gly Gly Leu Phe Ile Tyr Asp Val Phe
170 175 180

Trp Val Phe Gly Thr Asn Val Met Val Thr Val Ala Lys Ser Phe
185 190 195

Glu Ala Pro Ile Lys Leu Val Phe Pro Gln Asp Leu Leu Gln Lys
200 205 210

Gly Leu Glu Ala Asn Asn Phe Ala Met Leu Gly Leu Gly Asp Val
215 220 225

Val Ile Pro Gly Ile Phe Ile Ala Leu Leu Leu Arg Phe Asp Ile
230 235 240

Ser Leu Lys Lys Asn Thr His Thr Tyr Phe Tyr Thr Ser Phe Ala
245 250 255

Ala Tyr Ile Phe Gly Leu Gly Leu Thr Ile Phe Ile Met His Ile
260 265 270

Phe Lys His Ala Gln Pro Ala Leu Leu Tyr Leu Val Pro Ala Cys
275 280 285

Ile Gly Phe Pro Val Leu Val Ala Leu Ala Lys Gly Glu Val Thr
290 295 300

Glu Met Phe Ser Tyr Glu Glu Ser Asn Pro Lys Asp Pro Ala Ala
305 310 315

Val Thr Glu Ser Lys Glu Gly Thr Glu Ala Ser Ala Ser Lys Gly
320 325 330

Leu Glu Lys Lys Glu Lys

335

<210> 74
<211> 5069
<212> DNA
<213> Homo sapien

<400> 74
gggcgcagag gaggaaaggg agcaggcgca gggggactgg aaaggcagca 50

tgcgctcgcc aggagcaacc tcggcgccca gggtctgagg ctgcagcccc 100

agttcgccat tgtgagccgc cgccggggga gtccgctagc gcagccgtgc 150

ccccgagtcc ccgtccgcgc agcgatgggg cacctgccca cggggataca 200

cggcgcccgc cgcctcctgc ctctgctctg gctctttgtg ctgttcaaga 250

atgctacagc tttccatgta actgtccaag atgataataa catcgttgtc 300

tcattagaag cttcagacgt catcagtcca gcatctgtgt atgttgtgaa 350

gataactggt gaatccaaaa attatttctt cgaatttgag gaattcaaca 400

gcactttgcc tcctcctgtt attttcaagg ccagttatca tggcctttat 450

tatataatca ctctggtagt ggtaaatgga aatgtggtga ccaagccatc 500

cagatcaatc actgtgttaa caaaacctct acctgtaacc agtgtttcca 550

tatatgacta taaaccttct cctgaaacag gagtcctgtt tgaaatacat 600

tatccagaaa aatataacgt tttcacaaga gtgaacatta gctactggga 650

aggtaaagac ttccggacaa tgctatataa agatttcttt aagggaaaaa 700

cagtatttaa tcactggctg ccaggaatgt gttatagtaa tatcaccttt 750

cagctggtat ctgaggcaac ttttaataaa agtacccttg ttgagtacag 800

tggtgtcagt cacgaaccca aacagcacag aactgcccct tatccacctc 850

aaaatatttc cgttcgtatc gtaaacttga acaaaaacaa ctgggaagaa 900

cagagtggca atttcccaga agaatccttc atgagatcac aagatacaat 950

aggaaaagaa aaactcttcc attttacaga agaaacccct gaaattccct 1000

cgggcaacat ttcttccggt tggcctgatt ttaatagcag tgactatgaa 1050

actacgtctc agccatattg gtgggacagt gcatctgcag ctcctgaaag 1100

tgaagatgaa tttgtcagcg tacttcccat ggaatacgaa aataacagta 1150

cactcagtga gacagagaag tcaacatcag gctctttctc ctttttccct 1200

gtgcaaatga tattgacctg gttaccaccc aaaccaccca ctgcttttga 1250

tgggttccat atccatattg aacgagaaga gaactttact gaatatttga 1300

tggtggatga agaagcacat gaatttgttg cagaactgaa ggaacctggg 1350

aaatataagt tatctgtgac aacctttagt tcctcaggat cttgtgaaac 1400
tcgaaaaagt cagtcagcaa aatcactcag cttttatatc agtccttcag 1450
gagagtggat tgaagaactg accgagaagc cgcagcacgt gagtgtccac 1500
gttttaagct caaccactgc cttgatgtcc tggacatctt cccaagagaa 1550
ctacaacagc accattgtgt ctgtggtgtc gctgacctgc cagaaacaaa 1600
aggagagcca gaggcttgaa aagcagtact gcactcaggt gaactcaagc 1650
aaacctatta ttgaaaatct ggttcctggt gcccagtacc aggttgtaat 1700
atacctaagg aaaggccctt tgattggacc accttcagat cctgtgacat 1750
ttgctattgt tcccacagga ataaaggatt taatgctcta tcctttgggt 1800
cctacggccg tggttctgag ctggaccaga ccttatttag gcgtgttcag 1850
aaaatacgtg gttgaaatgt tttatttcaa ccctgctaca atgacatcag 1900
agtggaccac ctactatgaa atagcagcaa ctgtttcctt aactgcatcc 1950
gtgagaatag ctaatctgct gccagcatgg tactacaact tccgggttac 2000
catggtgacg tggggagatc cagaattgag ctgctgtgac agctctacca 2050
tcagcttcat aacagcccca gtggctccgg aaatcacttc tgtggaatat 2100
ttcaacagtc tgttatatat cagttggaca tatggggatg atacaacgga 2150
cttgtcccat tctagaatgc ttcactggat ggtggttgca gaaggaaaaa 2200
agaaaattaa aaagagtgta acacgcaatg tcatgactgc aattctcagc 2250
ttgcctccag gcgacatcta taacctctca gtaactgctt gtactgaaag 2300
aggaagtaat acctccatgc tccgccttgt caagctagaa ccagctccac 2350
ccaaatcact cttcgcagtg aacaaaaccc agacttcagt gactttgctg 2400
tgggtggaag agggagtagc tgatttcttt gaagttttct gtcaacaagt 2450
tggctccagt cagaaaacca aacttcagga accagttgct gtttcttccc 2500
atgtcgtgac catctccagc cttcttcctg ccactgccta caattgtagt 2550
gtcaccagct ttagccatga cagccccagt gtccctacgt tcatagccgt 2600
ctcaacaatg gttacagaga tgaatcccaa tgtggtagtg atctccgtgc 2650
tggccatcct tagcacactt ttaattggac tgttgcttgt taccctcatt 2700
attcttagga aaaagcatct gcagatggct agggagtgtg gagctggtac 2750
atttgtcaat tttgcatcct tagagaggga tggaaagctt ccatacaact 2800
ggagtaaaaa tggtttaaag aagaggaaac tgacaaaccc ggttcaactg 2850

gatgactttg atgcctatat taaggatatg gccaaagact ctgactataa 2900
attttctctt cagtttgagg agttgaaatt gattggactg gatatcccac 2950
actttgctgc agatcttcca ctgaatcgat gtaaaaaccg ttacacaaac 3000
atcctaccat atgacttcag ccgtgtgaga ttagtctcca tgaatgaaga 3050
ggaaggtgca gactacatca atgccaacta tattcctgga tacaactcac 3100
cccaggagta tattgccacc caggggccac tgcctgaaac cagaaatgac 3150
ttctggaaga tggtcctgca acaaaagtct cagattattg tcatgctcac 3200
tcagtgtaat gagaaaagga gggtgaaatg tgaccattac tggccattca 3250
cggaagaacc tatagcctat ggagacatca ctgtggagat gatttcagag 3300
gaagagcagg acgactgggc ctgtagacac ttccggatca actatgctga 3350
cgagatgcag gatgtgatgc attttaacta cactgcatgg cctgatcatg 3400
gtgtgcccac agcaaatgct gcagaaagta tcctgcagtt tgtacacatg 3450
gtccgacagc aagctaccaa gagcaaaggt cccatgatca ttcactgcag 3500
tgctggcgtg ggacggacag gaacattcat tgccctggac aggctcttgc 3550
agcacattcg ggatcatgag tttgttgaca tcttagggct ggtgtcagaa 3600
atgaggtcat accggatgtc tatggtacag acagaggagc agtacatttt 3650
tatccatcag tgtgtgcaac tgatgtggat gaagaagaag cagcagttct 3700
gcatcagtga tgtcatatac gagaatgtta gcaagtccta gttcagaatc 3750
cggagcagag aggacatgat gtgcgcccat cctcccttgc ttccagattg 3800
ttttagtggg ccctgatggt catttttcta aacagaggcc ctgctttgta 3850
atatgtggcc aaggagataa tttatctcac agaagcaccg ggaagactta 3900
gccttaaaga gcctacagtg tccttttgga ctctttcact tcgggacatt 3950
taataatgga ccaaattcaa cagaacacca ggaaggtcaa gacgctctcc 4000
aaagggcagg aagtacagca cttccgaaga gtttagttgg ccctttgctg 4050
gttgggctga gtttttttatt tttaagtgtt tgtttttcag tgcaataatt 4100
tttgtgtgtg tgtgattctt atcagaaagt tgaattgttt tctgcctaca 4150
ccgttcatca gccccataac ccaggaagga acaggcattg ttagcatcag 4200
attatacctc attattaaaa ggaggcatgg ccacacatga agaaatggtc 4250
attctacttc aaagaaattg agccagcact atctgtactc caacattacc 4300
ggatctggat tggggaggtt ggtcagggaa gagaggggtt ctacccacag 4350
atcaactgtg taatctttta ctattcaagc tataattcag cttcaaagta 4400

gagtagaaaa aaaattgtct taactgttct agttcttgat ggttttcttc 4450

cttattaaca gttggtgttt cttccttggc ccttttggac taatgttact 4500

gtccaagttc tttctcaaga aaccacatct ggttcagaag agtgtcaagt 4550

tggactcttt gaactctgtt gctgtctgag caatcgtggt gcctagactt 4600

tgcattcctt gttctgttga cctgcataca tgtgagagct atttctttaa 4650

gaactatata ggctgtgaaa acgcactttc tttcccccaa agagctggga 4700

atttatgaag ttatggcaat gaactgcagc atgctgggac aattatttga 4750

ctactttttt ttgtaatatt gtcaaatgtc tctatggatt ctgacagaga 4800

tttctttttg ttttgttatt cttttggttg tcagtttcat tttaacgagt 4850

gtaactagta acattttatt ctttggattt tgtataatta cagtacatga 4900

ttgtgtattg tgacatgaat gctgtcaaaa tgacattgat ggcattgtga 4950

agcctgttac tttgtgtcac ttcctgataa ataagaggtg atgacatgga 5000

tatacaacag aaaacacttt gagttgaaag taaacacaag ctggctgctt 5050

ccctgtggca actgtggct 5069

<210> 75
<211> 3743
<212> DNA
<213> Homo sapien

<400> 75
gcaaaggtga ctggcttcag tgaaggtgtg gtggatagtg tcaaaggtgg 50

gttttccagc ttctcccagg ccacccattc agcagcaggc gctgtagtct 100

caaagcccag agagattgcc tcactcattc ggaacaaatt tggcagtgca 150

gacaacatcc ccaacctgaa ggactcttta gaggaagggc aagtggatga 200

tgcggggaag gctttgggag tgatttcaaa ctttcagtct agcccaaaat 250

atggtagtga agaagattgt tctagtgcca cttcaggctc agtgggagcc 300

aacagcacca cagggggcat cgctgtagga gcatccagct ccaaaacaaa 350

caccctggac atgcagagct caggatttga tgcactacta catgagatcc 400

aggagatccg ggaaacccag gccagactag aggaatcctt tgagactctc 450

aaggaacatt atcagaggga ctattcctta ataatgcaga ccttacagga 500

ggagcgatat agatgtgaac gattggaaga acagctaaat gacctaacag 550

agctccacca gaatgaaatc ttgaacttga agcaggaact ggcaagcatg 600

gaagaaaaaa tcgcgtatca gtcctatgaa cgggcccggg acatccagga 650

ggccctggag gcatgccaga cgcgcatctc caagatggag ctgcagcagc 700
agcagcagca ggtggtgcag ctagaagggc tggagaatgc cactgcccgg 750
aaccttctgg gcaaactcat caacatcctc ctggctgtca tggcagtcct 800
tttggtcttt gtctccactg tagccaactg tgtggtcccc ctcatgaaga 850
ctcgcaacag gacgttcagc actttattcc ttgtggtttt tattgccttt 900
ctctggaagc actgggacgc cctcttcagc tatgtggaac ggttcttttc 950
atcccctaga tgatgctggc acagaaggca ttgttcccta ccctctggcg 1000
agtgcatgca gcagagagtt agacagcaac ttacctactc tgaagttttc 1050
tacaacaaaa aaagagttga gtgaatctgt ttacatttag aataatgttt 1100
ttttcttcaa gagacgcaat tgcaatagta ttttttagat tttatccaag 1150
aagtttttttg ggcgaaaatc ttggatcatt tttatgtagc atgattttcc 1200
ttgggatgca aatcttaaaa cagtccttta atatgaacca acaatctgga 1250
gcacaccgaa gggcaatcta aattgtggct tgaaggactg cactaaaacc 1300
cactaaaaag atgcgaaaac ctgatgaggg caaaccagtt aaacctaaca 1350
ccctgccttg tctgggctca tcacctctcc ctatcccaga ctaactttac 1400
tgtgaaatcc taccacattc catgtctgaa tttttggatt cggggtggat 1450
tttcgttgtc cgtggaagaa cacatggatc tctctggctt tctcacccaa 1500
gttggccact tacgctaatc ctggaagtat gatcactttt gaacctgccc 1550
cttaaccttg acgaggatac aaaagtgaaa gcatcatccc ccaaaggatc 1600
actgcacagt cctactacag tatttttaag tagccctcta aatacttaat 1650
tttaagcaaa atcccttggc cgcactttta aggttttttt atatgtgtat 1700
agttaccaac ctaaaaataa aaaatccgaa cagcatactt gaagaatgta 1750
atactcaaac tctcagtgct tccttatggt ttctaatagg attttttatt 1800
attgttatta ttattattgg gttttttggg acagggttgg gagggtcttt 1850
tatttttcct ttgaaataaa gaagtgatgt ttttaaatga agaaatgtgt 1900
ggatatttaa gtgtgctgct ccctcttgtc ttgaaacagt ttgagtaaga 1950
aagtcttgct gtaaatgctg ccctctgccg cctttgtttt gagatgcagt 2000
ttaaactccc tctggctgct gctgctgctt tttggtgtcc cgacatacct 2050
acgccccccgt tttatgggtt tggcttagtt gaagaggaaa gggttgtgca 2100
aggagagcag gaggctgttt ccaaaaacca gtgtagtagg atagggattt 2150
tttttttttt ttttgcccca agaaaacgtt cacccagtga tcttgggctg 2200

gggttgtctt taggaaaagt tgagactata agagtcataa ataagtcctt 2250
gtgtttcctt aatttatttt gttaacaccc ctaattacaa ccaaagtgat 2300
gatgtggagt cttctgtctt cattttggcc ccagcattct taatttcaaa 2350
gctttattct gtctgcctaa gagaatcaac caaaggtgat tctcctaaag 2400
agcagtgaag gaaatgtcag gttagcagga cccaagtttt gggtgtgaaa 2450
tgttgccagc ttcctataat gtaaacggac ttgttaacct aacctaatta 2500
tgctcagtgg acttctatag atggttttga aaaatgaact gagctgcctt 2550
cccgcatcgc ataaccagtt ccatcatcct ggtggaactt gaacatttag 2600
agtttatcta gagagcttgg ttaatctttc catattattt gtagtattgg 2650
tcacaaatgc tgttccctct tagcctcatt ctgtgcaacc aagtgcatat 2700
aagatgccct gaaaagagta acaaagtatg ctttgcctgt ttccacttac 2750
caggaaattc cttcagaact agattagcat tgccctgcct gtctgaaagg 2800
acagtttacc taatggtgcc agcctccttt tgctttggca agctggattt 2850
ctcagagcca gcatgttgtt tccataacta ctttgatatt ttaactcagg 2900
tactccagtc ttcaccccaa cctcagctga ttgtagtaca cctgctagct 2950
ctgttgcccc ctcaaaactg cacccagagc agggccacaa gggtgctttt 3000
ttttctttaa aaaaaaaaaa attagaacca attcatgttc atgccaaaaa 3050
caaattgtcc ccaagcctat atgtattaaa atgttaactt tgcctaaaaa 3100
tattgcagtg actttttagg caggagtgcc aaaggacact atgaactttt 3150
tgaactgaca gtttctccta actttctgct ttagcgtaat tgctcagagt 3200
agagagcccc cacaaagtta tttaaaagat gccctagcag caatccacca 3250
gtttttctaa gctagaacct ttgagtcccc caaactgcct gaagacttaa 3300
gttttgtggg cactggaagt cactttgata gatggattga aactgttcct 3350
atttgccctg ggacggtttc tatctatcaa aggaaggttt tcacctgtag 3400
aaagccccct gcctccagcc aaatagtccc atgctgactt tctatcttcc 3450
tttctcaaac tgtcttagga aggaccttca gtgcagatca ggtgcagtaa 3500
tggctttctt gtcccttaat tattcaccag acccagaagt tgtacgcatt 3550
taatgctgtt tgtaaccatg catctgtttt cattctttgc tgtacctttt 3600
gctgcccatc ctgttacttt tgagtttctt tcattgtggt tgttcttggg 3650
ttcttttgtc ttgtcagagc tcttctataa cctcgctcta atggcttaac 3700

agttgttctg ggtggaaacg tcccctcatt tgaatgctcc tct 3743

<210> 76
<211> 5263
<212> DNA
<213> Homo sapien

<220>
<221> Unsure
<222> 848, 1060, 1248, 1377, 2310, 2319, 2839
<223> Unknown base

<400> 76
agtggaagga gcaggcgctt gagctcgagc gacggcgctg gcggagacgc 50

cggctgctcc tcccctcccc gccggtatta atctctggag aagacacatc 100

cacagttagc actttcttca gatgctgacg ctcggtgaac agttgccttt 150

ggtcacaaga tttagaagac acagtgtcca tcctcccaga ttggatctct 200

ttttcatatg gatcttctgt ttctatgtct ttttaaaaaa taactttttg 250

ggaaaccttt tggattacaa ctgttcatcc tcacctatgc aaagaaaggg 300

aagctattgc tgggattttg aggagctttt cctaaaagga ttgtacacct 350

tagaagtgct taaggaagag tgatgaagat aggcatgaag ccttcgtctc 400

acagctgcat gcgtagtcac tgttgaagca aatgcctacc taatttgaca 450

ctcttggtgt gtttaaaaaa tttttttgag tttgcaaata agcatattaa 500

gtctactgat ggagccttcg ggcagtgaac agttatttga ggaccctgat 550

cctggaggca aatcccaaga tgcagaggcc agaaagcaga cagaatcaga 600

acaaaaattg tctaaaatga cccacaatgc tttggagaac attaacgtga 650

ttggccaagg cttgaagcat ctcttccagc accagcgcag gaggtcatca 700

gtgtctccac atgatgtgca gcaaattcag gcagatccag aacctgaaat 750

ggatctggaa agccagaacg catgtgctga gattgatggt gtccccaccc 800

accccacagc tctgaatcgt gtcctgcagc agattcgagt gccacccnag 850

atgaagagag ggacaagctt gcatagtagg cggggcaagc cagaggcccc 900

aaagggaagt ccccaaatca acaggaagtc tggtcaggag atgacagctg 950

ttatgcagtc aggccgaccc atgtcttcat ccacaactga tgcacctacc 1000

ggctctgcta tgatggaaat agcttgtgct gctgctgctg ctgctgctgc 1050

atgtctaccn ggagaggagg gaactgcgga gcggatcgaa cggttggaag 1100

taagcagcct tgcccaaaca tccagtgcag tggcctccag taccgatggc 1150

agcatccaca cagactctgt ggatggaaca ccagaccctc agcgcacaaa 1200

ggctgccatt gctcacctgc agcagaagat cctgaagctc acagaacnaa 1250
tcaagattgc acaaacagcc cgggacgaca acgttgctga atacttgaag 1300
cttgccaaca gtgcagacaa acagcaggct gcccgcatca agcaagtctt 1350
tgagaagaag aaccagaaat ctgcccnaac tatcctccag ctgcaaaaga 1400
aacttgagca ctaccacagg aagctcagag aggtagagca gaatgggatc 1450
ccccggcagc caaaggatgt cttcagggac atgcaccagg gtctgaagga 1500
tgtaggagca aaggtgactg gcttcagtga aggtgtggtg gatagtgtca 1550
aaggtgggtt ttccagcttc tcccaggcca cccattcagc agcaggcgct 1600
gtagtctcaa agcccagaga gattgcctca ctcattcgga acaaatttgg 1650
cagtgcagac aacatcccca acctgaagga ctctttagag gaagggcaag 1700
tggatgatgc ggggaaggct ttgggagtga tttcaaactt tcagtctagc 1750
ccaaaatatg gtagtgaaga agattgttct agtgccactt caggctcagt 1800
gggagccaac agcaccacag gggcatcgc tgtaggagca tccagctcca 1850
aaacaaacac cctggacatg cagagctcag gatttgatgc actactacat 1900
gagatccagg agatccggga aacccaggcc agactagagg aatcctttga 1950
gactctcaag gaacattatc agagggacta ttccttaata atgcagacct 2000
tacaggagga gcgatataga tgtgaacgat tggaagaaca gctaaatgac 2050
ctaacagagc tccaccagaa tgaaatcttg aacttgaagc aggaactggc 2100
aagcatggaa gaaaaaatcg cgtatcagtc ctatgaacgg gcccgggaca 2150
tccaggaggc cctggaggca tgccagacgc gcatctccaa gatggagctg 2200
cagcagcagc agcagcaggt ggtgcagcta gaagggctgg agaatgccac 2250
tgcccggaac cttctgggca aactcatcaa catcctcctg gctgtcatgg 2300
cagtcctttn ggtctttgnc tccactgtag ccaactgtgt ggtcccctc 2350
atgaagactc gcaacaggac gttcagcact ttattccttg tggtttttat 2400
tgcctttctc tggaagcact gggacgccct cttcagctat gtggaacggt 2450
tcttttcatc ccctagatga tgctggcaca gaaggcattg ttccctaccc 2500
tctggcgagt gcatgcagca gagagttaga cagcaactta cctactctga 2550
agttttctac aacaaaaaaa gagttgagtg aatctgttta catttagaat 2600
aatgtttttt tcttcaagag acgcaattgc aatagtattt tttagatttt 2650
atccaagaag ttttttgggc gaaaatcttg gatcattttt atgtagcatg 2700
attttccttg ggatgcaaat cttaaaacag tcctttaata tgaaccaaca 2750

atctggagca caccgaaggg caatctaaat tgtggcttga aggactgcac 2800
taaaacccac taaaaagatg cgaaaacctg atgagggcna accagttaaa 2850
cctaacaccc tgccttgtct gggctcatca cctctcccta tcccagacta 2900
actttactgt gaaatcctac acattccatg tctgaatttt tggattcggg 2950
gtggattttc gttgtccgtg gaagaacaca tggatctctc tggctttctc 3000
acccaagttg gccacttacg ctaatcctgg aagtatgatc acttttgaac 3050
ctgcccctta accttgacga ggatacaaaa gtgaaagcat catcccccaa 3100
aggatcactg cacagtccta ctacagtatt tttaagtagc cctctaaata 3150
cttaatttta agcaaaatcc cttggccgca cttttaaggt ttttttatat 3200
gtgtatagtt accaacctaa aaataaaaaa tccgaacagc atacttgaag 3250
aatgtaatac tcaaactctc agtgcttcct tatggtttct aataggattt 3300
tttattattg ttattattat tattgggttt ttttggacag ggttgggagg 3350
gtcttttatt tttcctttga aataaagaag tgatgttttt aaatgaagaa 3400
atgtgtggat atttaagtgt gctgctccct cttgtcttga aacagtttga 3450
gtaagaaagt cttgctgtaa atgctgccct ctgccgcctt tgttttgaga 3500
tgcagtttaa actccctctg gctgctgctg ctgctttttg gtgtcccgac 3550
atacctacgc ccccgtttta tgggtttggc ttagttgaag aggaaagggt 3600
tgtgcaagga gagcaggagg ctgtttccaa aaaccagtgt agtaggatag 3650
ggattttttt tttttttttg ccccaagaaa acgttcaccc agtgatcttg 3700
ggctggggtt gtctttagga aaagttgaga ctataagagt cataaataag 3750
tccttgtgtt tccttaattt attttgttaa cacccctaat tacaaccaaa 3800
gtgatgatgt ggagtcttct gtcttcattt tggccccagc attcttaatt 3850
tcaaagcttt attctgtctg cctaagagaa tcaaccaaag gtgattctcc 3900
taaagagcag tgaaggaaat gtcaggttag caggacccaa gttttgggtg 3950
tgaaatgttg ccagcttcct ataatgtaaa cggacttgtt aacctaacct 4000
aattatgctc agtggacttc tatagatggt tttgaaaaat gaactgagct 4050
gccttcccgc atcgcataac cagttccatc atcctggtgg aacttgaaca 4100
tttagagttt atctagagag cttggttaat ctttccatat tatttgtagt 4150
attggtcaca aatgctgttc cctcttagcc tcattctgtg caaccaagtg 4200
catataagat gccctgaaaa gagtaacaaa gtatgctttg cctgtttcca 4250

cttaccagga aattccttca gaactagatt agcattgccc tgcctgtctg 4300

aaaggacagt ttacctaatg gtgccagcct ccttttgctt tggcaagctg 4350

gatttctcag agccagcatg ttgtttccat aactactttg atattttaac 4400

tcaggtactc cagtcttcac cccaacctca gctgattgta gtacacctgc 4450

tagctctgtt gccccctcaa aactgcaccc agagcagggc cacaagggtg 4500

cttttttttct ttaaaaaaaa aaaaattaga accaattcat gttcatgcca 4550

aaaacaaatt gtccccaagc ctatatgtat taaaatgtta actttgccta 4600

aaaatattgc agtgactttt taggcaggag tgccaaagga cactatgaac 4650

tttttgaact gacagtttct cctaactttc tgctttagcg taattgctca 4700

gagtagagag cccccacaaa gttatttaaa agatgcccta gcagcaatcc 4750

accagttttt ctaagctaga acctttgagt cccccaaact gcctgaagac 4800

ttaagttttg tgggcactgg aagtcacttt gatagatgga ttgaaactgt 4850

tcctatttgc cctgggacgg tttctatcta tcaaaggaag gttttcacct 4900

gtagaaagcc ccctgcctcc agccaaatag tcccatgctg actttctatc 4950

ttcctttctc aaactgtctt aggaaggacc ttcagtgcag atcaggtgca 5000

gtaatggctt tcttgtccct taattattca ccagacccag aagttgtacg 5050

catttaatgc tgtttgtaac catgcatctg ttttcattct ttgctgtacc 5100

ttttgctgcc catcctgtta cttttgagtt tctttcattg tggttgttct 5150

tgggttcttt tgtcttgtca gagctcttct ataacctcgc tctaatggct 5200

taacagttgt tctgggtgga aacgtcccct catttgaatg ctcctctaaa 5250

aaaaaaaaaa aaa 5263

<210> 77
<211> 5132
<212> DNA
<213> Homo sapien

<400> 77
tattagccaa gctaagttac tcttttgcct cctgttgtta ctcaagtctt 50

ttctcttctg tccttctgcc agccttaccc cactccttaa tcctctgaac 100

cagcaaacca ttgccaagtt ctgatgcaaa gtggtttata ggcctgactg 150

gaccagacta aaagtgttca aaatagcaag caacaaggag cagaaatcca 200

tattagaatg ggatatggac tatatttata ttggtacaga atgccttcaa 250

taaagagttg tgagttgtgt aggtgagttg ccatggagct acaaatatga 300

gttgatattc tgaaatccta gacagccatc tccaaggtta agaaaaatcc 350

ttatgcactc acttgcaaag atatccacag catgctcttg gagcgccgcc 400
ggccgggagg cgaaggatgc aggcggctcc gcgcgccggc tgcggggcag 450
cgctcctgct gtggattgtc agcagctgcc tctgcagagc ctggacggct 500
ccctccacgt cccaaaaatg tgatgagcca cttgtctctg gactccccca 550
tgtggctttc agcagctcct cctccatctc tggtagctat tctcccggct 600
atgccaagat aaacaagaga ggaggtgctg gggatggtc tccatcagac 650
agcgaccatt atcaatggct tcaggttgac tttggcaatc ggaagcagat 700
cagtgccatt gcaacccaag gaaggtatag cagctcagat tgggtgaccc 750
aataccggat gctctacagc gacacaggga gaaactggaa accctatcat 800
caagatggga atatctgggc atttcccgga aacattaact ctgacggtgt 850
ggtccggcac gaattacagc atccgattat tgcccgctat gtgcgcatag 900
tgcctctgga ttggaatgga gaaggtcgca ttggactcag aattgaagtt 950
tatggctgtt cttactgggc tgatgttatc aactttgatg gccatgttgt 1000
attaccatat agattcagaa acaagaagat gaaaacactg aaagatgtca 1050
ttgccttgaa ctttaagacg tctgaaagtg aaggagtaat cctgcacgga 1100
gaaggacagc aaggagatta cattaccttg gaactgaaaa aagccaagct 1150
ggtcctcagt ttaaacttag gaagcaacca gcttggcccc atatatggcc 1200
acacatcagt gatgacagga agtttgctgg atgaccacca ctggcactct 1250
gtggtcattg agcgccaggg gcggagcatt aacctcactc tggacaggag 1300
catgcagcac ttccgtacca atggagagtt tgactacctg gacttggact 1350
atgagataac ctttggaggc atccctttct ctggcaagcc cagctccagc 1400
agtagaaaga atttcaaagg ctgcatggaa agcatcaact acaatggcgt 1450
caacattact gatcttgcca gaaggaagaa attagagccc tcaaatgtgg 1500
gaaatttgag cttttcttgt gtggaaccct atacggtgcc tgtctttttc 1550
aacgctacaa gttacctgga ggtgcccgga cggcttaacc aggacctgtt 1600
ctcagtcagt ttccagttta ggacatggaa ccccaatggt ctcctggtct 1650
tcagtcactt tgcggataat ttgggcaatg tggagattga cctcactgaa 1700
agcaaagtgg gtgttcacat caacatcaca cagaccaaga tgagccaaat 1750
cgatatttcc tcaggttctg ggttgaatga tggacagtgg cacgaggttc 1800
gcttcctagc caaggaaaat tttgctattc tcaccatcga tggagatgaa 1850

gcatcagcag ttcgaactaa tagtcccctt caagttaaaa ctggcgagaa 1900
gtactttttt ggaggttttc tgaaccagat gaataactca agtcactctg 1950
tccttcagcc ttcattccaa ggatgcatgc agctcattca agtggacgat 2000
caacttgtaa atttatacga agtggcacaa aggaagccgg gaagtttcgc 2050
gaatgtcagc attgacatgt gtgcgatcat agacagatgt gtgcccaatc 2100
actgtgagca tggtggaaag tgctcgcaaa catgggacag cttcaaatgc 2150
acttgtgatg agacaggata cagtggggcc acctgccaca actctatcta 2200
cgagccttcc tgtgaagcct acaaacacct aggacagaca tcaaattatt 2250
actggataga tcctgatggc agcggacctc tggggcctct gaaagtttac 2300
tgcaacatga cagaggacaa agtgtggacc atagtgtctc atgacttgca 2350
gatgcagacg cctgtggtcg gctacaaccc agaaaaatac tcagtgacac 2400
agctcgttta cagcgcctcc atggaccaga taagtgccat cactgacagt 2450
gccgagtact gcgagcagta tgtctcctat ttctgcaaga tgtcaagatt 2500
gttgaacacc ccagatggaa gcccttacac ttggtgggtt ggcaaagcca 2550
acgagaagca ctactactgg ggaggctctg ggcctggaat ccagaaatgt 2600
gcctgcggca tcgaacgcaa ctgcacagat cccaagtact actgtaactg 2650
cgacgcggac tacaagcaat ggaggaagga tgctggtttc ttatcataca 2700
aagatcacct gccagtgagc caagtggtgg ttggagatac tgaccgtcaa 2750
ggctcagaag ccaaattgag cgtaggtcct ctgcgctgcc aaggagacag 2800
gaattattgg aatgccgcct ctttcccaaa cccatcctcc tacctgcact 2850
tctctacttt ccaaggggaa actagcgctg acatttcttt ctacttcaaa 2900
acattaaccc cctggggagt gtttcttgaa aatatgggaa aggaagattt 2950
catcaagctg gagctgaagt ctgccacaga agtgtccttt tcatttgatg 3000
tgggaaatgg gccagtagag attgtagtga ggtcaccaac ccctctcaac 3050
gatgaccagt ggcaccgggt cactgcagag aggaatgtca agcaggccag 3100
cctacaggtg gaccggctac cgcagcagat ccgcaaggcc ccaacagaag 3150
gccacacccg cctggagctc tacagccagt tatttgtggg tggtgctggg 3200
ggccagcagg gcttcctggg ctgcatccgc tccttgagga tgaatggggt 3250
gacacttgac ctggaggaaa gagcaaaggt cacatctggg ttcatatccg 3300
gatgctcggg ccattgcacc agctatggaa caaactgtga aaatggaggc 3350
aaatgcctag agagatacca cggttactcc tgcgattgct ctaatactgc 3400

atatgatgga acattttgca acaaagatgt tggtgcattt tttgaagaag 3450
ggatgtggct acgatataac tttcaggcac cagcaacaaa tgccagagac 3500
tccagcagca gagtagacaa cgctcccgac cagcagaact cccacccgga 3550
cctggcacag gaggagatcc gcttcagctt cagcaccacc aaggcgccct 3600
gcattctcct ctacatcagc tccttcacca cagacttctt ggcagtcctc 3650
gtcaaaccca ctggaagctt acagattcga tacaacctgg gtggcacccg 3700
agagccatac aatattgacg tagaccacag gaacatggcc aatggacagc 3750
cccacagtgt caacatcacc cgccacgaga agaccatctt tctcaagctc 3800
gatcattatc cttctgtgag ttaccatctg ccaagttcat ccgacaccct 3850
cttcaattct cccaagtcgc tctttctggg aaaagttata gaaacaggga 3900
aaattgacca agagattcac aaatacaaca ccccaggatt cactggttgc 3950
ctctccagag tccagttcaa ccagatcgcc cctctcaagg ccgccttgag 4000
gcagacaaac gcctcggctc acgtccacat ccagggcgag ctggtggagt 4050
ccaactgcgg ggcctcgccg ctgaccctct cccccatgtc gtccgccacc 4100
gacccctggc acctggatca cctggattca gccagtgcag attttccata 4150
taatccagga caaggccaag ctataagaaa tggagtcaac agaaactcgg 4200
ctatcattgg aggcgtcatt gctgtggtga ttttcaccat cctgtgcacc 4250
ctggtcttcc tgatccggta catgttccgc cacaagggca cctaccatac 4300
caacgaagca aagggggcgg agtcggcaga gagcgcggac gccgccatca 4350
tgaacaacga ccccaacttc acagagacca ttgatgaaag caaaaaggaa 4400
tggctcattt gaggggtggc tacttggcta tgggataggg aggagggaat 4450
tactagggag gagagaaagg gacaaaagca ccctgcttca tactcttgag 4500
cacatcctta aaatatcagc acaagttggg ggaggcaggc aatggaatat 4550
aatggaatat tcttgagact gatcacaaaa aaaaaaaaaa ccttttttaat 4600
atttctttat agctgagttt tcccttctgt atcaaaacaa aataatacaa 4650
aaaatgcttt tagagtttaa gcaatggttg aaatttgtag gtactatctg 4700
tcttattttg tgtgtgttta gaggtgttct aaagacccgt ggtaacaggg 4750
caagttttct acgtttttaa gagcccttag aacgtgggta ttttttttct 4800
tgagaaaagc taatgcacct acagatggcc cccaacattc tcttcctttt 4850
gcttctagtc aaccttaatg ggctgttaca gaaactagtt cgtgtttata 4900

tactatttcc tttgatgtcc tataagtcgg aaaagaaagg ggcaaagaga 4950

acctattatt tgccagtttt taagcagagc tcaatctatg ccagctctct 5000

ggcatctggg gttcctgact gataccagca gttgaaggaa gagagtgcat 5050

ggcacctggt gtgtaacgac acaatcagca caactggaga gaggcattaa 5100

agaaccaggg aaggtagttt gatttttcat tg 5132

<210> 78
<211> 4627
<212> DNA
<213> Homo sapien

<400> 78
tcacttgcct gatatttcca gtgtcagagg gacacagcca acgtggggtc 50

ccttctaggc tgacagccgc tctccagcca ctgccgcgag cccgtctgct 100

cccgccctgc ccgtgcactc tccgcagccg ccctccgcca agccccagcg 150

cccgctccca tcgccgatga ccgcggggag gaggatggag atgctctgtg 200

ccggcagggt ccctgcgctg ctgctctgcc tgggtttcca tcttctacag 250

gcagtcctca gtacaactgt gattccatca tgtatcccag gagagtccag 300

tgataactgc acagctttag ttcagacaga agacaatcca cgtgtggctc 350

aagtgtcaat aacaaagtgt agctctgaca tgaatggcta ttgtttgcat 400

ggacagtgca tctatctggt ggacatgagt caaaactact gcaggtgtga 450

agtgggttat actggtgtcc gatgtgaaca cttcttttta accgtccacc 500

aacctttaag caaagagtat gtggctttga ccgtgattct tattattttg 550

tttcttatca cagtcgtcgg ttccacatat tatttctgca gatggtacag 600

aaatcgaaaa agtaaagaac caaagaagga atatgagaga gttacctcag 650

gggatccaga gttgccgcaa gtctgaatgg cgccatcaaa cttatgggca 700

gggataacag tgtgcctggt taatattaat attccatttt attaataata 750

tttatgttgg gtcaagtgtt aggtcaataa cactgtattt taatgtactt 800

gaaaaatgtt tttatttttg ttttattttt gacagactat ttgctaatgt 850

ataatgtgca gaaaatattt aatatcaaaa gaaaattgat atttttatac 900

aagtaatttc ctgagctaaa tgcttcattg aaagcttcaa agtttatatg 950

cctggtgcac agtgcttaga agtaagcaat tcccaggtca tagctcaaga 1000

attgttagca aatgacagat ttctgtaagc ctatatatat agtcaaatcg 1050

atttagtaag tatgtttttt atgttcctca aatcagtgat aattggtttg 1100

actgtaccat ggtttgatat gtagttggca ccatggtatc atatattaaa 1150

acaataatgc aattagaatt tgggagaagc aaatataggt cctgtgttaa 1200
acactacaca tttgaaacaa gctaaccctg gggagtctat ggtctcttca 1250
ctcaggtctc agctataatt ctgttatatg aggggcagtg gacagttccc 1300
tatgccaact cacgactcct acaggtacta gtcactcatc taccagattc 1350
tgcctatgta aaatgaattg aaaaacaatt ttctgtaatc ttttatttaa 1400
gtagtgggca tttcatagct tcacaatgtt cctttttgt atattacaac 1450
atttatgtga ggtaattatt gctcaacaga caattagaaa aaagtccaca 1500
cttgaagcct aaatttgtgc tttttaagaa tatttttaga ctatttcttt 1550
ttataggggc tttgctgaat tctaacatta aatcacagcc caaaatttga 1600
tggactaatt attattttaa aatatatgaa gacaataatt ctacatgttg 1650
tcttaagatg gaaatacagt tatttcatct tttattcaag gaagttttaa 1700
ctttaataca gctcagtaaa tggcttcttc tagaatgtaa agttatgtat 1750
ttaaagttgt atcttgacac aggaaatggg aaaaaactta aaaattaata 1800
tggtgtattt ttccaaatga aaaatctcaa ttgaaagctt ttaaaatgta 1850
gaaacttaaa cacaccttcc tgtggaggct gagatgaaaa ctagggctca 1900
ttttcctgac atttgtttat tttttggaag agacaaagat ttcttctgca 1950
ctctgagccc ataggtctca gagagttaat aggagtattt ttgggctatt 2000
gcataaggag ccactgctgc caccactttt ggattttatg ggaggctcct 2050
tcatcgaatg ctaaaccttt gagtagagtc tccctggatc acataccagg 2100
tcagggagga tctgttcttc ctctacgttt atcctggcat gtgctagggt 2150
aaacgaaggc ataataagcc atggctgacc tctggagcac caggtgccag 2200
gacttgtctc catgtgtatc catgcattat ataccctggt gcaatcacac 2250
gactgtcatc taaagtcctg gccctggccc ttactattag gaaaataaac 2300
agacaaaaac aagtaaatat atatggtcct atacatattg tatatatatt 2350
catatacaaa catgtatgta tacatgacct taatggatca tagaattgca 2400
gtcatttggt gctctgctaa ccatttatat aaaacttaaa aacaagagaa 2450
aagaaaaatc aattagatct aaacagttat ttctgtttcc tatttaatat 2500
agctgaagtc aaaatatgta agaacacatt ttaaatactc tacttacagt 2550
tggccctctg tggttagttc cacatctgtg gattcaacca accaaggacg 2600
gaaaatgctt aaaaaataat acaacaacaa caaaaaatac attataacaa 2650

ctatttactt ttttttttt ctttttgaga tggagtctcg ctctgttgcc 2700

caggttggag tgcagtggca cgatctcggc tcactgcaac ctcacctccc 2750

gggttcaaga gatcctcctg cctcagcctc ctgagcagct gggactacag 2800

gcgcatgcca ccatgcccag ctaattttg tattttagt agaggcgggg 2850

tttcaccatg ttggccagga tggtctcaat ctcctaacct tgagatccac 2900

cctccacagc ctcccaaact gctgggatta caggcgtgag ccaccgcacg 2950

tagcatttac attaggtatt acaagtaatg taaagatgat ttaagtatac 3000

aggaggatgt gaataggtta tatgcaagca ctatgccctt ttatataagt 3050

gacttgaaca tctgtgcccg attttagtat gtgcaggggg gcgatctggg 3100

aatcagtccc ctgtggatac caaggtacaa ctgtatttat taacgcttac 3150

tagatgtgag gagagtctga atattttcag tgatcttggc tgtttcaaaa 3200

aaatctattg acttttcaat aaatcagctg caatccattt atttcattta 3250

caaaagattt attgtaagcc tctcaatctt ggtttttcag ttgatcttaa 3300

gcatgtcaat tcataaaaac aagtcatttt tgtatttttc atctttaaga 3350

atgcttaaaa aagctaatcc ctaaaatagt tagatctttg taaatgcata 3400

ttaaataata aagtatgacc cacattactt tttatgggtg aaaataagac 3450

aaaaataata gttttagtga ggatggtgct gagtaaacat aaaaactgat 3500

ttgctctcag ctgatgtgtc ctgtacacag tgggaagatt ttagttcaca 3550

cttagtctaa ctccccatt ttacagattt ctcactatat atatttctag 3600

aaggggctat gcatattcaa tgtattgaga accaaagcaa ccacaaatgc 3650

ataaatgcat aatttatggt cttcaaccaa ggccacataa taacccagtt 3700

aacttactct ttaaccagga atattaagtt ctataactag tactcaaggt 3750

ttaaccttaa aattaagatt tccttaacct taaccttaaa attgatatta 3800

tattaaacat acataataca atgtaactcc actgttctcc tgaatatttt 3850

ttgctctaat ctctctgccg aaagtcaaag tgatgggaga attggtatac 3900

tggtatgact acgtcttaag tcagattttt atttatgagt ctttgagact 3950

aaattcaatc accaccaggt atcaaatcaa cttttatgca gcaaatatat 4000

gattctagtg tctgactttt gttaaattca gtaatgcagt ttttaaaaac 4050

ctgtatctga cccactttgt aatttttgct ccaatatcca ttctgtagac 4100

ttttgaaaaa aaagttttta atttgatgcc caatatattc tgaccgttaa 4150

aaaattcttg ttcatatggg agaaggggga gtaatgactt gtacaaacag 4200

tatttctggt gtatatttta atgtttttaa aaagagtaat ttcatttaaa 4250

tatctgttat tcaaatttga tgatgttaaa tgtaatataa tgtattttct 4300

ttttattttg cactctgtaa ttgcactttt taagtttgaa gagccatttt 4350

ggtaaacggt ttttattaaa gatgctatgg aacataaagt tgtattgcat 4400

gcaatttaaa gtaacttatt tgactatgaa tattatcgga ttactgaatt 4450

gtatcaattt gtttgtgttc aatatcagct ttgataattg tgtaccttaa 4500

gatattgaag gagaaaatag ataatttaca agatattatt aatttttatt 4550

tatttttctt gggaattgaa aaaaattgaa ataaataaaa atgcattgaa 4600

catcttgcat tcaaaatctt cactgac 4627

<210> 79
<211> 1188
<212> PRT
<213> Homo sapien

<400> 79
Met Gly His Leu Pro Thr Gly Ile His Gly Ala Arg Arg Leu Leu
1               5                   10                  15

Pro Leu Leu Trp Leu Phe Val Leu Phe Lys Asn Ala Thr Ala Phe
                20                  25                  30

His Val Thr Val Gln Asp Asp Asn Asn Ile Val Val Ser Leu Glu
                35                  40                  45

Ala Ser Asp Val Ile Ser Pro Ala Ser Val Tyr Val Val Lys Ile
                50                  55                  60

Thr Gly Glu Ser Lys Asn Tyr Phe Phe Glu Phe Glu Glu Phe Asn
                65                  70                  75

Ser Thr Leu Pro Pro Pro Val Ile Phe Lys Ala Ser Tyr His Gly
                80                  85                  90

Leu Tyr Tyr Ile Ile Thr Leu Val Val Val Asn Gly Asn Val Val
                95                  100                 105

Thr Lys Pro Ser Arg Ser Ile Thr Val Leu Thr Lys Pro Leu Pro
                110                 115                 120

Val Thr Ser Val Ser Ile Tyr Asp Tyr Lys Pro Ser Pro Glu Thr
                125                 130                 135

Gly Val Leu Phe Glu Ile His Tyr Pro Glu Lys Tyr Asn Val Phe
                140                 145                 150

Thr Arg Val Asn Ile Ser Tyr Trp Glu Gly Lys Asp Phe Arg Thr
                155                 160                 165

Met Leu Tyr Lys Asp Phe Phe Lys Gly Lys Thr Val Phe Asn His
                170                 175                 180

Trp Leu Pro Gly Met Cys Tyr Ser Asn Ile Thr Phe Gln Leu Val
185 190 195

Ser Glu Ala Thr Phe Asn Lys Ser Thr Leu Val Glu Tyr Ser Gly
200 205 210

Val Ser His Glu Pro Lys Gln His Arg Thr Ala Pro Tyr Pro Pro
215 220 225

Gln Asn Ile Ser Val Arg Ile Val Asn Leu Asn Lys Asn Asn Trp
230 235 240

Glu Glu Gln Ser Gly Asn Phe Pro Glu Glu Ser Phe Met Arg Ser
245 250 255

Gln Asp Thr Ile Gly Lys Glu Lys Leu Phe His Phe Thr Glu Glu
260 265 270

Thr Pro Glu Ile Pro Ser Gly Asn Ile Ser Ser Gly Trp Pro Asp
275 280 285

Phe Asn Ser Ser Asp Tyr Glu Thr Thr Ser Gln Pro Tyr Trp Trp
290 295 300

Asp Ser Ala Ser Ala Ala Pro Glu Ser Glu Asp Glu Phe Val Ser
305 310 315

Val Leu Pro Met Glu Tyr Glu Asn Asn Ser Thr Leu Ser Glu Thr
320 325 330

Glu Lys Ser Thr Ser Gly Ser Phe Ser Phe Phe Pro Val Gln Met
335 340 345

Ile Leu Thr Trp Leu Pro Pro Lys Pro Pro Thr Ala Phe Asp Gly
350 355 360

Phe His Ile His Ile Glu Arg Glu Glu Asn Phe Thr Glu Tyr Leu
365 370 375

Met Val Asp Glu Glu Ala His Glu Phe Val Ala Glu Leu Lys Glu
380 385 390

Pro Gly Lys Tyr Lys Leu Ser Val Thr Thr Phe Ser Ser Ser Gly
395 400 405

Ser Cys Glu Thr Arg Lys Ser Gln Ser Ala Lys Ser Leu Ser Phe
410 415 420

Tyr Ile Ser Pro Ser Gly Glu Trp Ile Glu Glu Leu Thr Glu Lys
425 430 435

Pro Gln His Val Ser Val His Val Leu Ser Ser Thr Thr Ala Leu
440 445 450

Met Ser Trp Thr Ser Ser Gln Glu Asn Tyr Asn Ser Thr Ile Val
455 460 465

Ser Val Val Ser Leu Thr Cys Gln Lys Gln Lys Glu Ser Gln Arg
470 475 480

Leu Glu Lys Gln Tyr Cys Thr Gln Val Asn Ser Ser Lys Pro Ile

485 490 495

Ile Glu Asn Leu Val Pro Gly Ala Gln Tyr Gln Val Val Ile Tyr
500 505 510

Leu Arg Lys Gly Pro Leu Ile Gly Pro Pro Ser Asp Pro Val Thr
515 520 525

Phe Ala Ile Val Pro Thr Gly Ile Lys Asp Leu Met Leu Tyr Pro
530 535 540

Leu Gly Pro Thr Ala Val Val Leu Ser Trp Thr Arg Pro Tyr Leu
545 550 555

Gly Val Phe Arg Lys Tyr Val Val Glu Met Phe Tyr Phe Asn Pro
560 565 570

Ala Thr Met Thr Ser Glu Trp Thr Thr Tyr Tyr Glu Ile Ala Ala
575 580 585

Thr Val Ser Leu Thr Ala Ser Val Arg Ile Ala Asn Leu Leu Pro
590 595 600

Ala Trp Tyr Tyr Asn Phe Arg Val Thr Met Val Thr Trp Gly Asp
605 610 615

Pro Glu Leu Ser Cys Cys Asp Ser Ser Thr Ile Ser Phe Ile Thr
620 625 630

Ala Pro Val Ala Pro Glu Ile Thr Ser Val Glu Tyr Phe Asn Ser
635 640 645

Leu Leu Tyr Ile Ser Trp Thr Tyr Gly Asp Asp Thr Thr Asp Leu
650 655 660

Ser His Ser Arg Met Leu His Trp Met Val Val Ala Glu Gly Lys
665 670 675

Lys Lys Ile Lys Lys Ser Val Thr Arg Asn Val Met Thr Ala Ile
680 685 690

Leu Ser Leu Pro Pro Gly Asp Ile Tyr Asn Leu Ser Val Thr Ala
695 700 705

Cys Thr Glu Arg Gly Ser Asn Thr Ser Met Leu Arg Leu Val Lys
710 715 720

Leu Glu Pro Ala Pro Pro Lys Ser Leu Phe Ala Val Asn Lys Thr
725 730 735

Gln Thr Ser Val Thr Leu Leu Trp Val Glu Glu Gly Val Ala Asp
740 745 750

Phe Phe Glu Val Phe Cys Gln Gln Val Gly Ser Ser Gln Lys Thr
755 760 765

Lys Leu Gln Glu Pro Val Ala Val Ser Ser His Val Val Thr Ile
770 775 780

Ser Ser Leu Leu Pro Ala Thr Ala Tyr Asn Cys Ser Val Thr Ser
785 790 795

Phe Ser His Asp Ser Pro Ser Val Pro Thr Phe Ile Ala Val Ser
800 805 810

Thr Met Val Thr Glu Met Asn Pro Asn Val Val Val Ile Ser Val
815 820 825

Leu Ala Ile Leu Ser Thr Leu Leu Ile Gly Leu Leu Leu Val Thr
830 835 840

Leu Ile Ile Leu Arg Lys Lys His Leu Gln Met Ala Arg Glu Cys
845 850 855

Gly Ala Gly Thr Phe Val Asn Phe Ala Ser Leu Glu Arg Asp Gly
860 865 870

Lys Leu Pro Tyr Asn Trp Ser Lys Asn Gly Leu Lys Lys Arg Lys
875 880 885

Leu Thr Asn Pro Val Gln Leu Asp Asp Phe Asp Ala Tyr Ile Lys
890 895 900

Asp Met Ala Lys Asp Ser Asp Tyr Lys Phe Ser Leu Gln Phe Glu
905 910 915

Glu Leu Lys Leu Ile Gly Leu Asp Ile Pro His Phe Ala Ala Asp
920 925 930

Leu Pro Leu Asn Arg Cys Lys Asn Arg Tyr Thr Asn Ile Leu Pro
935 940 945

Tyr Asp Phe Ser Arg Val Arg Leu Val Ser Met Asn Glu Glu Glu
950 955 960

Gly Ala Asp Tyr Ile Asn Ala Asn Tyr Ile Pro Gly Tyr Asn Ser
965 970 975

Pro Gln Glu Tyr Ile Ala Thr Gln Gly Pro Leu Pro Glu Thr Arg
980 985 990

Asn Asp Phe Trp Lys Met Val Leu Gln Gln Lys Ser Gln Ile Ile
995 1000 1005

Val Met Leu Thr Gln Cys Asn Glu Lys Arg Arg Val Lys Cys Asp
1010 1015 1020

His Tyr Trp Pro Phe Thr Glu Glu Pro Ile Ala Tyr Gly Asp Ile
1025 1030 1035

Thr Val Glu Met Ile Ser Glu Glu Glu Gln Asp Asp Trp Ala Cys
1040 1045 1050

Arg His Phe Arg Ile Asn Tyr Ala Asp Glu Met Gln Asp Val Met
1055 1060 1065

His Phe Asn Tyr Thr Ala Trp Pro Asp His Gly Val Pro Thr Ala
1070 1075 1080

Asn Ala Ala Glu Ser Ile Leu Gln Phe Val His Met Val Arg Gln
1085 1090 1095

Gln Ala Thr Lys Ser Lys Gly Pro Met Ile Ile His Cys Ser Ala
1100 1105 1110

Gly Val Gly Arg Thr Gly Thr Phe Ile Ala Leu Asp Arg Leu Leu
1115 1120 1125

Gln His Ile Arg Asp His Glu Phe Val Asp Ile Leu Gly Leu Val
1130 1135 1140

Ser Glu Met Arg Ser Tyr Arg Met Ser Met Val Gln Thr Glu Glu
1145 1150 1155

Gln Tyr Ile Phe Ile His Gln Cys Val Gln Leu Met Trp Met Lys
1160 1165 1170

Lys Lys Gln Gln Phe Cys Ile Ser Asp Val Ile Tyr Glu Asn Val
1175 1180 1185

Ser Lys Ser

<210> 80
<211> 320
<212> PRT
<213> Homo sapien

<400> 80
Ala Lys Val Thr Gly Phe Ser Glu Gly Val Val Asp Ser Val Lys
1 5 10 15

Gly Gly Phe Ser Ser Phe Ser Gln Ala Thr His Ser Ala Ala Gly
20 25 30

Ala Val Val Ser Lys Pro Arg Glu Ile Ala Ser Leu Ile Arg Asn
35 40 45

Lys Phe Gly Ser Ala Asp Asn Ile Pro Asn Leu Lys Asp Ser Leu
50 55 60

Glu Glu Gly Gln Val Asp Asp Ala Gly Lys Ala Leu Gly Val Ile
65 70 75

Ser Asn Phe Gln Ser Ser Pro Lys Tyr Gly Ser Glu Glu Asp Cys
80 85 90

Ser Ser Ala Thr Ser Gly Ser Val Gly Ala Asn Ser Thr Thr Gly
95 100 105

Gly Ile Ala Val Gly Ala Ser Ser Ser Lys Thr Asn Thr Leu Asp
110 115 120

Met Gln Ser Ser Gly Phe Asp Ala Leu Leu His Glu Ile Gln Glu
125 130 135

Ile Arg Glu Thr Gln Ala Arg Leu Glu Glu Ser Phe Glu Thr Leu
140 145 150

Lys Glu His Tyr Gln Arg Asp Tyr Ser Leu Ile Met Gln Thr Leu
155 160 165

Gln Glu Glu Arg Tyr Arg Cys Glu Arg Leu Glu Glu Gln Leu Asn

170 175 180

Asp Leu Thr Glu Leu His Gln Asn Glu Ile Leu Asn Leu Lys Gln
185 190 195

Glu Leu Ala Ser Met Glu Glu Lys Ile Ala Tyr Gln Ser Tyr Glu
200 205 210

Arg Ala Arg Asp Ile Gln Glu Ala Leu Glu Ala Cys Gln Thr Arg
215 220 225

Ile Ser Lys Met Glu Leu Gln Gln Gln Gln Gln Gln Val Val Gln
230 235 240

Leu Glu Gly Leu Glu Asn Ala Thr Ala Arg Asn Leu Leu Gly Lys
245 250 255

Leu Ile Asn Ile Leu Leu Ala Val Met Ala Val Leu Leu Val Phe
260 265 270

Val Ser Thr Val Ala Asn Cys Val Val Pro Leu Met Lys Thr Arg
275 280 285

Asn Arg Thr Phe Ser Thr Leu Phe Leu Val Val Phe Ile Ala Phe
290 295 300

Leu Trp Lys His Trp Asp Ala Leu Phe Ser Tyr Val Glu Arg Phe
305 310 315

Phe Ser Ser Pro Arg
320

<210> 81
<211> 653
<212> PRT
<213> Homo sapien

<220>
<221> X
<222> 114, 247, 290, 601, 604
<223> Unknown base

<400> 81
Met Glu Pro Ser Gly Ser Glu Gln Leu Phe Glu Asp Pro Asp Pro
1 5 10 15

Gly Gly Lys Ser Gln Asp Ala Glu Ala Arg Lys Gln Thr Glu Ser
20 25 30

Glu Gln Lys Leu Ser Lys Met Thr His Asn Ala Leu Glu Asn Ile
35 40 45

Asn Val Ile Gly Gln Gly Leu Lys His Leu Phe Gln His Gln Arg
50 55 60

Arg Arg Ser Ser Val Ser Pro His Asp Val Gln Gln Ile Gln Ala
65 70 75

Asp Pro Glu Pro Glu Met Asp Leu Glu Ser Gln Asn Ala Cys Ala
80 85 90

Glu Ile Asp Gly Val Pro Thr His Pro Thr Ala Leu Asn Arg Val
95 100 105

Leu Gln Gln Ile Arg Val Pro Pro Xaa Met Lys Arg Gly Thr Ser
110 115 120

Leu His Ser Arg Arg Gly Lys Pro Glu Ala Pro Lys Gly Ser Pro
125 130 135

Gln Ile Asn Arg Lys Ser Gly Gln Glu Met Thr Ala Val Met Gln
140 145 150

Ser Gly Arg Pro Met Ser Ser Ser Thr Thr Asp Ala Pro Thr Gly
155 160 165

Ser Ala Met Met Glu Ile Ala Cys Ala Ala Ala Ala Ala Ala Ala
170 175 180

Ala Cys Leu Pro Gly Glu Glu Gly Thr Ala Glu Arg Ile Glu Arg
185 190 195

Leu Glu Val Ser Ser Leu Ala Gln Thr Ser Ser Ala Val Ala Ser
200 205 210

Ser Thr Asp Gly Ser Ile His Thr Asp Ser Val Asp Gly Thr Pro
215 220 225

Asp Pro Gln Arg Thr Lys Ala Ala Ile Ala His Leu Gln Gln Lys
230 235 240

Ile Leu Lys Leu Thr Glu Xaa Ile Lys Ile Ala Gln Thr Ala Arg
245 250 255

Asp Asp Asn Val Ala Glu Tyr Leu Lys Leu Ala Asn Ser Ala Asp
260 265 270

Lys Gln Gln Ala Ala Arg Ile Lys Gln Val Phe Glu Lys Lys Asn
275 280 285

Gln Lys Ser Ala Xaa Thr Ile Leu Gln Leu Gln Lys Lys Leu Glu
290 295 300

His Tyr His Arg Lys Leu Arg Glu Val Glu Gln Asn Gly Ile Pro
305 310 315

Arg Gln Pro Lys Asp Val Phe Arg Asp Met His Gln Gly Leu Lys
320 325 330

Asp Val Gly Ala Lys Val Thr Gly Phe Ser Glu Gly Val Val Asp
335 340 345

Ser Val Lys Gly Gly Phe Ser Ser Phe Ser Gln Ala Thr His Ser
350 355 360

Ala Ala Gly Ala Val Val Ser Lys Pro Arg Glu Ile Ala Ser Leu
365 370 375

Ile Arg Asn Lys Phe Gly Ser Ala Asp Asn Ile Pro Asn Leu Lys
380 385 390

Asp Ser Leu Glu Glu Gly Gln Val Asp Asp Ala Gly Lys Ala Leu

395 400 405

Gly Val Ile Ser Asn Phe Gln Ser Ser Pro Lys Tyr Gly Ser Glu
410 415 420

Glu Asp Cys Ser Ser Ala Thr Ser Gly Ser Val Gly Ala Asn Ser
425 430 435

Thr Thr Gly Gly Ile Ala Val Gly Ala Ser Ser Ser Lys Thr Asn
440 445 450

Thr Leu Asp Met Gln Ser Ser Gly Phe Asp Ala Leu Leu His Glu
455 460 465

Ile Gln Glu Ile Arg Glu Thr Gln Ala Arg Leu Glu Glu Ser Phe
470 475 480

Glu Thr Leu Lys Glu His Tyr Gln Arg Asp Tyr Ser Leu Ile Met
485 490 495

Gln Thr Leu Gln Glu Glu Arg Tyr Arg Cys Glu Arg Leu Glu Glu
500 505 510

Gln Leu Asn Asp Leu Thr Glu Leu His Gln Asn Glu Ile Leu Asn
515 520 525

Leu Lys Gln Glu Leu Ala Ser Met Glu Glu Lys Ile Ala Tyr Gln
530 535 540

Ser Tyr Glu Arg Ala Arg Asp Ile Gln Glu Ala Leu Glu Ala Cys
545 550 555

Gln Thr Arg Ile Ser Lys Met Glu Leu Gln Gln Gln Gln Gln Gln
560 565 570

Val Val Gln Leu Glu Gly Leu Glu Asn Ala Thr Ala Arg Asn Leu
575 580 585

Leu Gly Lys Leu Ile Asn Ile Leu Leu Ala Val Met Ala Val Leu
590 595 600

Xaa Val Phe Xaa Ser Thr Val Ala Asn Cys Val Val Pro Leu Met
605 610 615

Lys Thr Arg Asn Arg Thr Phe Ser Thr Leu Phe Leu Val Val Phe
620 625 630

Ile Ala Phe Leu Trp Lys His Trp Asp Ala Leu Phe Ser Tyr Val
635 640 645

Glu Arg Phe Phe Ser Ser Pro Arg
650

<210> 82
<211> 1331
<212> PRT
<213> Homo sapien

<400> 82
Met Gln Ala Ala Pro Arg Ala Gly Cys Gly Ala Ala Leu Leu Leu
1 5 10 15

Trp Ile Val Ser Ser Cys Leu Cys Arg Ala Trp Thr Ala Pro Ser
20 25 30

Thr Ser Gln Lys Cys Asp Glu Pro Leu Val Ser Gly Leu Pro His
35 40 45

Val Ala Phe Ser Ser Ser Ser Ser Ile Ser Gly Ser Tyr Ser Pro
50 55 60

Gly Tyr Ala Lys Ile Asn Lys Arg Gly Gly Ala Gly Gly Trp Ser
65 70 75

Pro Ser Asp Ser Asp His Tyr Gln Trp Leu Gln Val Asp Phe Gly
80 85 90

Asn Arg Lys Gln Ile Ser Ala Ile Ala Thr Gln Gly Arg Tyr Ser
95 100 105

Ser Ser Asp Trp Val Thr Gln Tyr Arg Met Leu Tyr Ser Asp Thr
110 115 120

Gly Arg Asn Trp Lys Pro Tyr His Gln Asp Gly Asn Ile Trp Ala
125 130 135

Phe Pro Gly Asn Ile Asn Ser Asp Gly Val Val Arg His Glu Leu
140 145 150

Gln His Pro Ile Ile Ala Arg Tyr Val Arg Ile Val Pro Leu Asp
155 160 165

Trp Asn Gly Glu Gly Arg Ile Gly Leu Arg Ile Glu Val Tyr Gly
170 175 180

Cys Ser Tyr Trp Ala Asp Val Ile Asn Phe Asp Gly His Val Val
185 190 195

Leu Pro Tyr Arg Phe Arg Asn Lys Lys Met Lys Thr Leu Lys Asp
200 205 210

Val Ile Ala Leu Asn Phe Lys Thr Ser Glu Ser Glu Gly Val Ile
215 220 225

Leu His Gly Glu Gly Gln Gln Gly Asp Tyr Ile Thr Leu Glu Leu
230 235 240

Lys Lys Ala Lys Leu Val Leu Ser Leu Asn Leu Gly Ser Asn Gln
245 250 255

Leu Gly Pro Ile Tyr Gly His Thr Ser Val Met Thr Gly Ser Leu
260 265 270

Leu Asp Asp His His Trp His Ser Val Val Ile Glu Arg Gln Gly
275 280 285

Arg Ser Ile Asn Leu Thr Leu Asp Arg Ser Met Gln His Phe Arg
290 295 300

Thr Asn Gly Glu Phe Asp Tyr Leu Asp Leu Asp Tyr Glu Ile Thr
305 310 315

```
Phe Gly Gly Ile Pro Phe Ser Gly Lys Pro Ser Ser Ser Ser Arg
                320                 325                 330

Lys Asn Phe Lys Gly Cys Met Glu Ser Ile Asn Tyr Asn Gly Val
                335                 340                 345

Asn Ile Thr Asp Leu Ala Arg Arg Lys Lys Leu Glu Pro Ser Asn
                350                 355                 360

Val Gly Asn Leu Ser Phe Ser Cys Val Glu Pro Tyr Thr Val Pro
                365                 370                 375

Val Phe Phe Asn Ala Thr Ser Tyr Leu Glu Val Pro Gly Arg Leu
                380                 385                 390

Asn Gln Asp Leu Phe Ser Val Ser Phe Gln Phe Arg Thr Trp Asn
                395                 400                 405

Pro Asn Gly Leu Leu Val Phe Ser His Phe Ala Asp Asn Leu Gly
                410                 415                 420

Asn Val Glu Ile Asp Leu Thr Glu Ser Lys Val Gly Val His Ile
                425                 430                 435

Asn Ile Thr Gln Thr Lys Met Ser Gln Ile Asp Ile Ser Ser Gly
                440                 445                 450

Ser Gly Leu Asn Asp Gly Gln Trp His Glu Val Arg Phe Leu Ala
                455                 460                 465

Lys Glu Asn Phe Ala Ile Leu Thr Ile Asp Gly Asp Glu Ala Ser
                470                 475                 480

Ala Val Arg Thr Asn Ser Pro Leu Gln Val Lys Thr Gly Glu Lys
                485                 490                 495

Tyr Phe Phe Gly Gly Phe Leu Asn Gln Met Asn Asn Ser Ser His
                500                 505                 510

Ser Val Leu Gln Pro Ser Phe Gln Gly Cys Met Gln Leu Ile Gln
                515                 520                 525

Val Asp Asp Gln Leu Val Asn Leu Tyr Glu Val Ala Gln Arg Lys
                530                 535                 540

Pro Gly Ser Phe Ala Asn Val Ser Ile Asp Met Cys Ala Ile Ile
                545                 550                 555

Asp Arg Cys Val Pro Asn His Cys Glu His Gly Gly Lys Cys Ser
                560                 565                 570

Gln Thr Trp Asp Ser Phe Lys Cys Thr Cys Asp Glu Thr Gly Tyr
                575                 580                 585

Ser Gly Ala Thr Cys His Asn Ser Ile Tyr Glu Pro Ser Cys Glu
                590                 595                 600

Ala Tyr Lys His Leu Gly Gln Thr Ser Asn Tyr Tyr Trp Ile Asp
                605                 610                 615

Pro Asp Gly Ser Gly Pro Leu Gly Pro Leu Lys Val Tyr Cys Asn
```

620 625 630

Met Thr Glu Asp Lys Val Trp Thr Ile Val Ser His Asp Leu Gln
635 640 645

Met Gln Thr Pro Val Val Gly Tyr Asn Pro Glu Lys Tyr Ser Val
650 655 660

Thr Gln Leu Val Tyr Ser Ala Ser Met Asp Gln Ile Ser Ala Ile
665 670 675

Thr Asp Ser Ala Glu Tyr Cys Glu Gln Tyr Val Ser Tyr Phe Cys
680 685 690

Lys Met Ser Arg Leu Leu Asn Thr Pro Asp Gly Ser Pro Tyr Thr
695 700 705

Trp Trp Val Gly Lys Ala Asn Glu Lys His Tyr Tyr Trp Gly Gly
710 715 720

Ser Gly Pro Gly Ile Gln Lys Cys Ala Cys Gly Ile Glu Arg Asn
725 730 735

Cys Thr Asp Pro Lys Tyr Tyr Cys Asn Cys Asp Ala Asp Tyr Lys
740 745 750

Gln Trp Arg Lys Asp Ala Gly Phe Leu Ser Tyr Lys Asp His Leu
755 760 765

Pro Val Ser Gln Val Val Val Gly Asp Thr Asp Arg Gln Gly Ser
770 775 780

Glu Ala Lys Leu Ser Val Gly Pro Leu Arg Cys Gln Gly Asp Arg
785 790 795

Asn Tyr Trp Asn Ala Ala Ser Phe Pro Asn Pro Ser Ser Tyr Leu
800 805 810

His Phe Ser Thr Phe Gln Gly Glu Thr Ser Ala Asp Ile Ser Phe
815 820 825

Tyr Phe Lys Thr Leu Thr Pro Trp Gly Val Phe Leu Glu Asn Met
830 835 840

Gly Lys Glu Asp Phe Ile Lys Leu Glu Leu Lys Ser Ala Thr Glu
845 850 855

Val Ser Phe Ser Phe Asp Val Gly Asn Gly Pro Val Glu Ile Val
860 865 870

Val Arg Ser Pro Thr Pro Leu Asn Asp Asp Gln Trp His Arg Val
875 880 885

Thr Ala Glu Arg Asn Val Lys Gln Ala Ser Leu Gln Val Asp Arg
890 895 900

Leu Pro Gln Gln Ile Arg Lys Ala Pro Thr Glu Gly His Thr Arg
905 910 915

Leu Glu Leu Tyr Ser Gln Leu Phe Val Gly Gly Ala Gly Gly Gln
920 925 930

Gln Gly Phe Leu Gly Cys Ile Arg Ser Leu Arg Met Asn Gly Val
935 940 945

Thr Leu Asp Leu Glu Glu Arg Ala Lys Val Thr Ser Gly Phe Ile
950 955 960

Ser Gly Cys Ser Gly His Cys Thr Ser Tyr Gly Thr Asn Cys Glu
965 970 975

Asn Gly Gly Lys Cys Leu Glu Arg Tyr His Gly Tyr Ser Cys Asp
980 985 990

Cys Ser Asn Thr Ala Tyr Asp Gly Thr Phe Cys Asn Lys Asp Val
995 1000 1005

Gly Ala Phe Phe Glu Glu Gly Met Trp Leu Arg Tyr Asn Phe Gln
1010 1015 1020

Ala Pro Ala Thr Asn Ala Arg Asp Ser Ser Ser Arg Val Asp Asn
1025 1030 1035

Ala Pro Asp Gln Gln Asn Ser His Pro Asp Leu Ala Gln Glu Glu
1040 1045 1050

Ile Arg Phe Ser Phe Ser Thr Thr Lys Ala Pro Cys Ile Leu Leu
1055 1060 1065

Tyr Ile Ser Ser Phe Thr Thr Asp Phe Leu Ala Val Leu Val Lys
1070 1075 1080

Pro Thr Gly Ser Leu Gln Ile Arg Tyr Asn Leu Gly Gly Thr Arg
1085 1090 1095

Glu Pro Tyr Asn Ile Asp Val Asp His Arg Asn Met Ala Asn Gly
1100 1105 1110

Gln Pro His Ser Val Asn Ile Thr Arg His Glu Lys Thr Ile Phe
1115 1120 1125

Leu Lys Leu Asp His Tyr Pro Ser Val Ser Tyr His Leu Pro Ser
1130 1135 1140

Ser Ser Asp Thr Leu Phe Asn Ser Pro Lys Ser Leu Phe Leu Gly
1145 1150 1155

Lys Val Ile Glu Thr Gly Lys Ile Asp Gln Glu Ile His Lys Tyr
1160 1165 1170

Asn Thr Pro Gly Phe Thr Gly Cys Leu Ser Arg Val Gln Phe Asn
1175 1180 1185

Gln Ile Ala Pro Leu Lys Ala Ala Leu Arg Gln Thr Asn Ala Ser
1190 1195 1200

Ala His Val His Ile Gln Gly Glu Leu Val Glu Ser Asn Cys Gly
1205 1210 1215

Ala Ser Pro Leu Thr Leu Ser Pro Met Ser Ser Ala Thr Asp Pro
1220 1225 1230

Trp His Leu Asp His Leu Asp Ser Ala Ser Ala Asp Phe Pro Tyr
1235 1240 1245

Asn Pro Gly Gln Gly Gln Ala Ile Arg Asn Gly Val Asn Arg Asn
1250 1255 1260

Ser Ala Ile Ile Gly Gly Val Ile Ala Val Val Ile Phe Thr Ile
1265 1270 1275

Leu Cys Thr Leu Val Phe Leu Ile Arg Tyr Met Phe Arg His Lys
1280 1285 1290

Gly Thr Tyr His Thr Asn Glu Ala Lys Gly Ala Glu Ser Ala Glu
1295 1300 1305

Ser Ala Asp Ala Ala Ile Met Asn Asn Asp Pro Asn Phe Thr Glu
1310 1315 1320

Thr Ile Asp Glu Ser Lys Lys Glu Trp Leu Ile
1325 1330

<210> 83
<211> 169
<212> PRT
<213> Homo sapien

<400> 83
Met Thr Ala Gly Arg Arg Met Glu Met Leu Cys Ala Gly Arg Val
1 5 10 15

Pro Ala Leu Leu Leu Cys Leu Gly Phe His Leu Leu Gln Ala Val
20 25 30

Leu Ser Thr Thr Val Ile Pro Ser Cys Ile Pro Gly Glu Ser Ser
35 40 45

Asp Asn Cys Thr Ala Leu Val Gln Thr Glu Asp Asn Pro Arg Val
50 55 60

Ala Gln Val Ser Ile Thr Lys Cys Ser Ser Asp Met Asn Gly Tyr
65 70 75

Cys Leu His Gly Gln Cys Ile Tyr Leu Val Asp Met Ser Gln Asn
80 85 90

Tyr Cys Arg Cys Glu Val Gly Tyr Thr Gly Val Arg Cys Glu His
95 100 105

Phe Phe Leu Thr Val His Gln Pro Leu Ser Lys Glu Tyr Val Ala
110 115 120

Leu Thr Val Ile Leu Ile Ile Leu Phe Leu Ile Thr Val Val Gly
125 130 135

Ser Thr Tyr Tyr Phe Cys Arg Trp Tyr Arg Asn Arg Lys Ser Lys
140 145 150

Glu Pro Lys Lys Glu Tyr Glu Arg Val Thr Ser Gly Asp Pro Glu
155 160 165

Leu Pro Gln Val

<210> 84
<211> 2207
<212> DNA
<213> Homo sapien

<220>
<221> N
<222> 1823-1854
<223> Unknown base

<400> 84
tcggctcgcg gctttctgat tatgcagaac ttaaatctat gcctcagtga 50

cccatacagc attccagttc ctatcaccta ctgtcttgtc cctatacttg 100

cagcagttgt ccagggttat tctttgtctg tattagaatt ttttttcagg 150

ttgcttaagg aatcttgcag atacttgtga caaagaatca taaatgctgt 200

tgttaaactg aataatgaat tgagtcccaa atgttcgtgc taattaatgc 250

tttttgagtt ggagatgaaa tgagagtaat atcatcaagc tgtggattaa 300

agttatcctc aaagccccat catctacaaa aagaatagga caggaactgc 350

ctttgtgcag gtgcaagacc atgttacttt tgagcagtga gcttgagatg 400

tctgggatac aaattgggtt ccctattaac tactaatcat tccttttttt 450

tctttcacct tcagccactc acaactgacc ttcactacta ttacatcctg 500

gagctgtcgt tttattggtc tttgatgttt tctcagttca ctgatatcaa 550

aagaaaggac tttggcatta tgttcctgca ccaccttgta tctattttct 600

tgattacctt ttcatatgtc aacaatatgg cccgagtagg aacgctggtc 650

ctttgtcttc atgattcagc tgatgctctt ctggaggctg ccaaaatggc 700

aaattatgcc aagtttcaga aaatgtgtga tctcctgttt gttatgtttg 750

ccgtggtttt tatcaccaca cgactgggta tatttcctct ctgggtgtta 800

aataccacat tatttgaaag ctgggagatc gttggacctt acccttcctg 850

gtgggttttt aacctactgc tattgctagt acaagggttg aactgcttct 900

ggtcttactt gattgtgaaa atagcttgca aagctgtttc aagaggcaag 950

gtgtccaagg atgatcgaag tgatattgag tctagctcag atgaggagga 1000

ctcagaacct ccgggaaaga atccccacac tgcgacaacc accaatggga 1050

ccagtggtac caacgggtat ctcctgactg gctcctgctc catggatgat 1100

taattactca aaactacaag tcccaagcaa agtgaactat ttgttcctgg 1150

aagtatttaa taagttgcaa atgcagttcc tttcataata tctcagcacc 1200

agaaacaaaa attaagatta tcaaagcatt ttgaatagtg cactgccatg 1250

tgtcctgtct gtgaatgaag aagaattacc attctctctt tgtaggcatg 1300

ctgtatgtaa ttgacacaag ggaacagtat ttgcatttgt actgtcttag 1350

aatattattt atttttttgt atttgtaaat ctgtggacaa aagagggttt 1400

cctcactcct tttactcact gggctcatga cagtgaagga gatgctccat 1450

ctgcttctcc ccctttctct tgctgtagtc caatgtgcta tgagcatcag 1500

cttactttgt cacttagagc aagcaaaacc cagtgcaaga gtctcgttca 1550

gctctaaata ggtttgcttt cttttagtta cagtgcccat tttgaaattg 1600

cctatacagt cttagtgacc atttaaaccg gacgaactag gtgtttaatt 1650

ttcactcttc atgttcaatt agcagttcaa attaaagaag atggttattg 1700

gagaactttt ttgaatggtt ttgtattaaa ttgctttgaa atagatttca 1750

tttcttgtgc acacagccaa gatttcttca atgggtgtga gctagttgag 1800

ggttaacctt gtaggttgca gannnnnnnn nnnnnnnnnn nnnnnnnnnn 1850

nnnngatgag gtcagtgctc tgattttgaa ggaggatatt cactgaagct 1900

catagttata aacaaggaaa tcactgttaa gaatgggaat ttgtcctgtg 1950

ttctgggaat aacataaaga gagcaactga tttcagccag gttttgccac 2000

taccctataa ttagtgcagt cttatgttat aaaagaaaga agttaactat 2050

atttggggac aaaaaaatat ttcaagagtt gataaagatt acctgtgcag 2100

tgcagagcac tttaatgcaa ccagctttca agaaaaagcc ctatctagta 2150

cttgatgttg atgtttttat tttgctgagc aaaataaagc caatgggaga 2200

aggacaa 2207

<210> 85
<211> 192
<212> PRT
<213> Homo sapien

<400> 85
Met Phe Ser Gln Phe Thr Asp Ile Lys Arg Lys Asp Phe Gly Ile
1               5                   10                  15

Met Phe Leu His His Leu Val Ser Ile Phe Leu Ile Thr Phe Ser
                20                  25                  30

Tyr Val Asn Asn Met Ala Arg Val Gly Thr Leu Val Leu Cys Leu
                35                  40                  45

His Asp Ser Ala Asp Ala Leu Leu Glu Ala Ala Lys Met Ala Asn
                50                  55                  60

Tyr Ala Lys Phe Gln Lys Met Cys Asp Leu Leu Phe Val Met Phe

65 70 75

Ala Val Val Phe Ile Thr Thr Arg Leu Gly Ile Phe Pro Leu Trp
80 85 90

Val Leu Asn Thr Thr Leu Phe Glu Ser Trp Glu Ile Val Gly Pro
95 100 105

Tyr Pro Ser Trp Trp Val Phe Asn Leu Leu Leu Leu Leu Val Gln
110 115 120

Gly Leu Asn Cys Phe Trp Ser Tyr Leu Ile Val Lys Ile Ala Cys
125 130 135

Lys Ala Val Ser Arg Gly Lys Val Ser Lys Asp Asp Arg Ser Asp
140 145 150

Ile Glu Ser Ser Ser Asp Glu Glu Asp Ser Glu Pro Pro Gly Lys
155 160 165

Asn Pro His Thr Ala Thr Thr Thr Asn Gly Thr Ser Gly Thr Asn
170 175 180

Gly Tyr Leu Leu Thr Gly Ser Cys Ser Met Asp Asp
185 190

<210> 86
<211> 375
<212> DNA
<213> Homo sapien

<400> 86
atgtctcttg agcagaagag tcagcactgc aagcctgagg aaggccttga 50

cacccaagaa gaggccctgg gcctggtggg tgtgcaggct gccactactg 100

aggagcagga ggctgtgtcc tcctcctctc ctctggtccc aggcaccctg 150

ggggaggtgc ctgctgctgg gtcaccaggt cctctcaaga gtcctcaggg 200

agcctccgcc atccccactg ccatcgattt cactctatgg aggcaatcca 250

ttaagggctc cagcaaccaa gaagaggagg ggccaagcac ctcccctgac 300

ccagagtctg tgttccgagc agcactcagt aagaaggtgg ctgacttgat 350

tcattttctg ctcctcaagt attaa 375

<210> 87
<211> 8906
<212> DNA
<213> Homo sapien

<400> 87
gaggcggcca aggacctggc cgacatcgcg gccttcttcc gatccgggtt 50

tcgaaaaaac gatgaaatga aagctatgga tgttttacca attttgaagg 100

aaaaagttgc atacctttca ggtgggagag ataaacgtgg aggtcccatt 150

ttaacgtttc cggcccgcag caatcatgac agaatacgac aggaggatct 200

caggagactc atttcctatc tagcctgtat tcccagcgag gaggtctgca 250
agcgtggctt cacggtgatc gtggacatgc gtgggtccaa gtgggactcc 300
atcaagcccc ttctgaagat cctgcaggag tccttcccct gctgcatcca 350
tgtggccctg atcatcaagc cagacaactt ctggcagaaa cagaggacta 400
attttggcag ttctaaattt gaatttgaga caaatatggt ctctttagaa 450
ggccttacca aagtagttga tccttctcag ctaactcctg agtttgatgg 500
ctgcctggaa tacaaccacg aagaatggat tgaaatcaga gttgcttttg 550
aagactacat tagcaatgcc acccacatgc tgtctcggct ggaggaactt 600
caggacatcc tagctaagaa ggagctgcct caggatttag agggggctcg 650
gaatatgatc gaggaacatt ctcagctgaa gaagaaggtg attaaggccc 700
ccatcgagga cctggatttg gagggacaga agctgcttca gaggatacag 750
agcagtgaaa gctttcccaa aaagaactca ggctcaggca atgcggacct 800
gcagaacctc ttgcccaagg tgtccaccat gctggaccgg ctgcactcga 850
cacggcagca tctgcaccag atgtggcatg tgaggaagct gaagctggac 900
cagtgcttcc agctgaggct gtttgaacag gatgctgaga agatgtttga 950
ctggatcaca cacaacaaag gcctgtttct aaacagctac acagagattg 1000
ggaccagcca ccctcatgcc atggagcttc agacgcagca caatcacttt 1050
gccatgaact gtatgaacgt gtatgtaaat ataaaccgca tcatgtcggt 1100
ggccaatcgt ctggtggagt ctggccacta tgcctcgcag cagatcaggc 1150
agatcgcgag tcagctggag caggagtgga aggcgtttgc ggcagccctg 1200
gatgagcgga gcaccttgct ggacatgtcc tccattttcc accagaaggc 1250
cgaaaagtat atgagcaacg tggattcatg gtgtaaagct tgcggtgagg 1300
tagaccttcc ctcagagctg caggacctag aagatgccat tcatcaccac 1350
cagggaatat atgaacatat cactcttgct tattctgagg tcagccaaga 1400
tgggaagtcg ctccttgaca agctccagcg gcccttgact cccggcagct 1450
ccgattccct gacagcctct gccaactact ccaaggccgt gcaccatgtc 1500
ctggatgtca tccacgaggt gctgcaccac cagcggcacg tgagaacaat 1550
ctggcaacac cgcaaggtcc ggctgcatca gaggctgcag ctgtgtgttt 1600
tccagcagga agttcagcag gtgctagact ggatcgagaa ccacggagaa 1650
gcatttctga gcaaacatac aggtgtgggg aaatctcttc atcgggccag 1700

agcattgcag aaacgtcatg aagattttga agaagtggca cagaacacat 1750

acaccaatgc ggataaatta ctggaagcag cagaacagct ggctcagact 1800

ggggaatgtg accccgaaga gatttatcag gctgcccatc agctggaaga 1850

ccggattcaa gatttcgttc ggcgtgttga gcagcgaaag atcctactgg 1900

acatgtcagt gtcctttcac acccatgtga aagagctgtg gacgtggctg 1950

gaggagctgc agaaggagct gctggacgac gtgtatgccg agtcggtgga 2000

ggccgtgcag gacctcatca agcgctttgg ccagcagcag cagaccaccc 2050

tgcaggtgac tgtcaacgtg atcaaggaag gggaggacct catccagcag 2100

ctcagggact ctgccatctc cagtaacaag accccccaca acagctccat 2150

caaccacatt gagacggtgc tgcagcagct ggacgaggcg cagtcgcaga 2200

tggaggagct cttccaggag cgcaagatca agctggagct cttcctgcac 2250

gtgcgcatct tcgagaggga cgccatcgac attatctcag acctcgagtc 2300

ttggaatgat gagctttctc agcaaatgaa tgacttcgac acagaagatc 2350

tcacgattgc agagcagcgc ctccagcacc atgcagacaa agccttgacc 2400

atgaacaact tgacttttga cgtcatccac caagggcaag atcttctgca 2450

gtatgtcaat gaggtccagg cctctggtgt ggagctgctg tgtgatagag 2500

atgtagacat ggcaactcgg gtccaggacc tgctggagtt tcttcatgaa 2550

aaacagcagg aattggattt agccgcagag cagcatcgga aacacctgga 2600

gcagtgcgtg cagctgcgcc acctgcaggc agaagtgaaa caggtgctgg 2650

gttggatccg caacggagag tccatgttaa atgccggact tatcacagcc 2700

agctcgttac aagaggcaga gcagctccag cgagagcacg agcagttcca 2750

gcatgccatt gagaaaacac atcagagcgc gctgcaggtg cagcagaagg 2800

cagaagccat gctacaggcc aaccactacg acatggacat gatccgggac 2850

tgcgccgaga aggtggcgtc tcactggcaa cagctcatgc tcaagatgga 2900

agatcgcctc aagctcgtca acgcctctgt cgctttctac aaaacctcag 2950

agcaggtctg cagcgtcctc gagagcctgg aacaggagta caagagagaa 3000

gaagactggt gtggcggggc ggataagctg ggcccaaact ctgagacgga 3050

ccacgtgacg cccatgatca gcaagcacct ggagcagaag gaggcattcc 3100

tgaaggcttg caccettgct cggaggaatg cagacgtctt cctgaaatac 3150

ctgcacagga acagcgtgaa catgccagga atggtgacgc acatcaaagc 3200

tcctgaacag caagtgaaaa atatcttgaa tgaactcttc caacgggaga 3250

acagggtatt gcattactgg accatgagga agagacggct ggaccagtgt 3300
cagcagtacg tggtctttga gaggagtgcc aagcaggctt tggaatggat 3350
ccatgacaat ggcgagttct acctttccac acacacctcc acgggctcca 3400
gtatacagca cacccaggag ctcctgaaag agcacgagga gttccagata 3450
actgcaaagc aaaccaaaga gagagtgaag ctattgatac agctggctga 3500
tggcttttgt gaaaaagggc atgcccatgc ggcagagata aaaaaatgtg 3550
ttactgctgt ggataagagg tacagagatt tctctctgcg gatggagaag 3600
tacaggacct ctttggaaaa agccctgggg atttcttcag attccaacaa 3650
atcgagtaaa agtctccagc tagatatcat tccagccagt atccctggct 3700
cagaggtgaa acttcgagat gctgctcatg aacttaatga agagaagcgg 3750
aaatctgccc gcaggaaaga gttcataatg gctgagctca ttcaaactga 3800
aaaggcttat gtaagagacc tccgggaatg tatggatacg tacctgtggg 3850
aaatgaccag tggcgtggaa gagattccac ctggcattgt aaacaaagaa 3900
ctcatcatct tcggaaacat gcaagaaatc tacgaatttc ataataacat 3950
attcctaaag gagctggaaa aatatgaaca gttgccagag gatgttggac 4000
attgttttgt tacttgggca gacaagtttc agatgtatgt cacatattgc 4050
aaaaataagc ctgattctac tcagctgata ttggaacatg cagggtccta 4100
ttttgacgag atacagcagc gacatggatt agccaattcc atttcttcct 4150
accttattaa accagttcag cgaataacga aatatcagct ccttttaaaa 4200
gagctgctga cgtgctgtga ggaaggaaag ggagagatta aagatggcct 4250
ggaggtgatg ctcagcgtgc cgaagcgagc caatgacgcc atgcacctca 4300
gcatgctgga agggtttgat gaaaacattg agtctcaggg agaactcatc 4350
ctacaggaat ccttccaagt gtgggaccca aaaaccttaa ttcgaaaggg 4400
tcgagaacgg catctcttcc tttttgaaat gtccttagta tttagtaaag 4450
aagtgaaaga ttccagtggg agaagcaagt acctttataa aagcaaattg 4500
tttacctcag agttgggtgt cacagaacat gttgaaggag acccttgcaa 4550
atttgcactg tgggtgggga gaacaccaac ttcagataat aaaattgtcc 4600
ttaaggcttc cagcatagag aacaagcagg actggataaa gcatatccgc 4650
gaagtcatcc aggagcggac gatccacctg aagggagccc tgaaggagcc 4700
cattcacatc cctaagaccg ctcccgccac aagacagaag ggaaggaggg 4750

atggagagga tctggacagc caaggagacg gcagcagcca gcctgatacg 4800
atttccatcg cctcacggac gtctcagaac acgctggaca gcgataagct 4850
ctctggtggc tgtgagctga cagtggtgat ccatgacttc accgcttgca 4900
acagcaacga gctgaccatc cgacggggcc agaccgtgga agttctggag 4950
cggccgcatg acaagcctga ctggtgtctg gtgcggacca ctgaccgctc 5000
cccagcggca gaaggcctgg tcccctgtgg ttcactgtgc atcgcccact 5050
ccagaagtag catggaaatg gagggcatct tcaaccacaa agactcgctc 5100
tccgtctcca gcaatgacgc cagtccaccc gcatccgtgg cttccctcca 5150
gccccacatg atcggggccc agagctcgcc gggccccaag cggccgggca 5200
acaccctgcg caagtggctc accagccccg tgcggcggct cagcagcggc 5250
aaggccgacg ggcacgtgaa gaagctggcg cacaagcaca agaagagccg 5300
cgaggtccgc aagagcgccg acgccggctc gcagaaggac tccgacgaca 5350
gtgcggccac cccgcaggac gagacggtcg aggagagagg ccggaacgag 5400
ggcctgagca gcggtactct ctccaaatcc tcctcctcgg ggatgcagag 5450
ctgtggagaa gaggaaggcg aggagggggc cgacgccgtg cccctgccgc 5500
cacccatggc catccagcag cacagcctcc tccagccaga ctcacaggat 5550
gacaaggcct cttctcggtt attagtccgc cccaccagct ccgaaacacc 5600
gagtgcagcc gagctcgtca gtgcaattga ggaactcgtg aaaagcaaga 5650
tggcactgga ggatcgcccc agctcactcc ttgttgacca gggagatagt 5700
agcagccctt ccttcaaccc ttcggataat tcccttctct cttcctcctc 5750
gcccattgat gagatggaag aaaggaaatc cagctcttta aagagaagac 5800
actacgtttt gcaagaacta gtggagacag agcgtgacta tgtgcgggac 5850
cttggctatg tggttgaggg ctacatggca cttatgaaag aagatggtgt 5900
tcctgatgac atgaaaggaa aagacaaaat tgtgttcggc aacatccatc 5950
agatttacga ctggcacaga gacttttttt taggagagtt agagaagtgc 6000
cttgaagatc cagaaaaact aggatccctt tttgttaaac acgagagaag 6050
gttgcacatg tacatagctt attgtcaaaa taaaccaaag tctgagcaca 6100
ttgtctcaga atacattgat acctttttg aggacttaaa gcagcgtctt 6150
ggccacaggt tacagctcac agatctgttg atcaaaccag tgcagagaat 6200
catgaagtat cagctgttac tgaaggactt cctcaagtat tccaaaaagg 6250
ccagcctgga tacatcagaa ttagagagag ctgtggaagt catgtgcata 6300

gtacccaggc ggtgcaacga catgatgaac gtggggcggc tgcaaggatt 6350
cgacgggaaa atcgttgccc agggtaaact gctcttgcag gacacattct 6400
tggtcacaga ccaagatgca ggacttctgc ctcgctgcag agagaggcgc 6450
atcttcctct ttgagcagat cgtcatattc agcgaaccac ttgataaaaa 6500
gaagggcttc tccatgccgg gattcctgtt taagaacagt atcaaggtga 6550
gttgcctttg cctggaggaa aatgtggaaa atgatccctg taaatttgct 6600
ctgacatcga ggacgggtga cgtggtagag accttcattt tgcattcatc 6650
tagtccaagt gtccggcaaa cttggatcca tgaaatcaac caaattttag 6700
aaaaccagcg caattttta aatgccttga catcgccaat cgagtaccag 6750
aggaaccaca gcgggggcgg cggcggcggc ggcagcgggg cagcggcggg 6800
ggtgggggca gcggcggcgg cggggccccc agtggcggca gcggccacag 6850
tggcggcccc agcagctgcg gcggcgcccc cagcacgagc aggagccggc 6900
cctcccggat cccccagcct gtccgacacc accccccgt gctggtctcc 6950
tctgcagcct cgagccaggc agaggcagac aagatgtcag agtgaaagca 7000
gcagcagtag caacatctcc accatgttgg tgacacacga ttacacggca 7050
gtgaaggagg atgagatcaa cgtctaccaa ggagaggtcg ttcaaattct 7100
ggccagcaac cagcagaaca tgtttctggt gttccgagcc gccactgacc 7150
agtgccccgc agctgagggc tggattccag gctttgtcct gggccacacc 7200
agtgcagtca tcgtggagaa cccggacggg actctcaaga agtcaacatc 7250
ttggcacaca gcactccgtt taaggaaaaa atctgagaaa aaagataaag 7300
acggcaaaag ggaaggcaag ttagagaacg gttatcggaa gtcacgggaa 7350
ggactcagca acaaggtatc tgtgaagctt ctcaatccca actacattta 7400
tgacgttccc ccagaattcg tcattccatt gagtgaggtc acgtgtgaga 7450
caggggagac cgttgttctt agatgtcgag tctgtggccg ccccaaagcc 7500
tcaattacct ggaagggccc tgaacacaac accttgaaca acgatggtca 7550
ctacagcatc tcctacagtg acctgggaga ggccacgctg aagattgtgg 7600
gcgtgaccac ggaagatgac ggcatctaca cgtgcatcgc tgtcaatgac 7650
atgggttcag cctcatcatc ggccagcctg agggtcctag gtccagggat 7700
ggatgggatc atggtgacct ggaaagacaa ctttgactcc ttctacagtg 7750
aagtggctga gcttggcagg ggcagattct ctgtcgttaa gaaatgtgat 7800

cagaaaggaa ccaagcgagc agtggccact aagtttgtga acaagaagtt 7850

gatgaagcgc gaccaggtca cccatgagct tggcatcctg cagagcctcc 7900

agcacccccct gcttgtcggc ctcctcgaca cctttgagac ccccaccagc 7950

tacatcctgg tcttagaaat ggctgaccag ggtcgcctcc tggactgcgt 8000

ggtgcgatgg ggaagcctca ctgaagggaa gatcagggcg cacctggggg 8050

aggttctgga agctgtccgg tacctgcaca actgcaggat agcacacctg 8100

gacctaaagc ctgagaatat cctggtggat gagagtttag ccaagccaac 8150

catcaaactg gctgactttg gagatgctgt tcagctcaac acgacctact 8200

acatccacca gttactgggg aaccctgaat tcgcagcccc tgaaatcatc 8250

ctcgggaacc ctgtctccct gacctcggat acgtggagtg ttggagtgct 8300

cacatacgta cttcttagtg gcgtgtcccc cttcctggat gacagtgtgg 8350

aagagacctg cctgaacatt tgccgcttag actttagctt cccagatgac 8400

tactttaaag gagtgagcca gaaggccaag gagttcgtgt gcttcctcct 8450

gcaggaggac cccgccaagc gtccctcggc tgcgctggcc ctccaggagc 8500

agtggctgca ggccggcaac ggcagaagca cgggcgtcct cgacacgtcc 8550

agactgactt ccttcattga gcggcgcaaa caccagaatg atgttcgacc 8600

tatccgtagc attaaaaact ttctgcagag caggcttctg cctagagttt 8650

gacctatcca gaagttcttt ctcattctct ttcacctgcc aatcagctgt 8700

taatctgaat tttcaagaga aaacaagcaa acataactga tcagctgccg 8750

gtatgttcat cgtgtgaaat tgcattccaa gtgagctgtg ctcagcagtg 8800

cttggacaca gagctgcaag ctgcgctggg gtggaggacc gtcacttaca 8850

ctctgccaag gacggaggtc gcattgctgt atcacagtat tttttacgga 8900

tttctg 8906

<210> 88
<211> 124
<212> PRT
<213> Homo sapien

<400> 88

Met Ser Leu Glu Gln Lys Ser Gln His Cys Lys Pro Glu Glu Gly
1 5 10 15

Leu Asp Thr Gln Glu Glu Ala Leu Gly Leu Val Gly Val Gln Ala
20 25 30

Ala Thr Thr Glu Glu Gln Glu Ala Val Ser Ser Ser Ser Pro Leu
35 40 45

Val Pro Gly Thr Leu Gly Glu Val Pro Ala Ala Gly Ser Pro Gly
50 55 60

Pro Leu Lys Ser Pro Gln Gly Ala Ser Ala Ile Pro Thr Ala Ile
65 70 75

Asp Phe Thr Leu Trp Arg Gln Ser Ile Lys Gly Ser Ser Asn Gln
80 85 90

Glu Glu Glu Gly Pro Ser Thr Ser Pro Asp Pro Glu Ser Val Phe
95 100 105

Arg Ala Ala Leu Ser Lys Lys Val Ala Asp Leu Ile His Phe Leu
110 115 120

Leu Leu Lys Tyr

<210> 89
<211> 2861
<212> PRT
<213> Homo sapien

<400> 89
Met Lys Ala Met Asp Val Leu Pro Ile Leu Lys Glu Lys Val Ala
1 5 10 15

Tyr Leu Ser Gly Gly Arg Asp Lys Arg Gly Gly Pro Ile Leu Thr
20 25 30

Phe Pro Ala Arg Ser Asn His Asp Arg Ile Arg Gln Glu Asp Leu
35 40 45

Arg Arg Leu Ile Ser Tyr Leu Ala Cys Ile Pro Ser Glu Glu Val
50 55 60

Cys Lys Arg Gly Phe Thr Val Ile Val Asp Met Arg Gly Ser Lys
65 70 75

Trp Asp Ser Ile Lys Pro Leu Leu Lys Ile Leu Gln Glu Ser Phe
80 85 90

Pro Cys Cys Ile His Val Ala Leu Ile Ile Lys Pro Asp Asn Phe
95 100 105

Trp Gln Lys Gln Arg Thr Asn Phe Gly Ser Ser Lys Phe Glu Phe
110 115 120

Glu Thr Asn Met Val Ser Leu Glu Gly Leu Thr Lys Val Val Asp
125 130 135

Pro Ser Gln Leu Thr Pro Glu Phe Asp Gly Cys Leu Glu Tyr Asn
140 145 150

His Glu Glu Trp Ile Glu Ile Arg Val Ala Phe Glu Asp Tyr Ile
155 160 165

Ser Asn Ala Thr His Met Leu Ser Arg Leu Glu Glu Leu Gln Asp
170 175 180

Ile Leu Ala Lys Lys Glu Leu Pro Gln Asp Leu Glu Gly Ala Arg

185 190 195

Asn Met Ile Glu Glu His Ser Gln Leu Lys Lys Lys Val Ile Lys
200 205 210

Ala Pro Ile Glu Asp Leu Asp Leu Glu Gly Gln Lys Leu Leu Gln
215 220 225

Arg Ile Gln Ser Ser Glu Ser Phe Pro Lys Lys Asn Ser Gly Ser
230 235 240

Gly Asn Ala Asp Leu Gln Asn Leu Leu Pro Lys Val Ser Thr Met
245 250 255

Leu Asp Arg Leu His Ser Thr Arg Gln His Leu His Gln Met Trp
260 265 270

His Val Arg Lys Leu Lys Leu Asp Gln Cys Phe Gln Leu Arg Leu
275 280 285

Phe Glu Gln Asp Ala Glu Lys Met Phe Asp Trp Ile Thr His Asn
290 295 300

Lys Gly Leu Phe Leu Asn Ser Tyr Thr Glu Ile Gly Thr Ser His
305 310 315

Pro His Ala Met Glu Leu Gln Thr Gln His Asn His Phe Ala Met
320 325 330

Asn Cys Met Asn Val Tyr Val Asn Ile Asn Arg Ile Met Ser Val
335 340 345

Ala Asn Arg Leu Val Glu Ser Gly His Tyr Ala Ser Gln Gln Ile
350 355 360

Arg Gln Ile Ala Ser Gln Leu Glu Gln Glu Trp Lys Ala Phe Ala
365 370 375

Ala Ala Leu Asp Glu Arg Ser Thr Leu Leu Asp Met Ser Ser Ile
380 385 390

Phe His Gln Lys Ala Glu Lys Tyr Met Ser Asn Val Asp Ser Trp
395 400 405

Cys Lys Ala Cys Gly Glu Val Asp Leu Pro Ser Glu Leu Gln Asp
410 415 420

Leu Glu Asp Ala Ile His His His Gln Gly Ile Tyr Glu His Ile
425 430 435

Thr Leu Ala Tyr Ser Glu Val Ser Gln Asp Gly Lys Ser Leu Leu
440 445 450

Asp Lys Leu Gln Arg Pro Leu Thr Pro Gly Ser Ser Asp Ser Leu
455 460 465

Thr Ala Ser Ala Asn Tyr Ser Lys Ala Val His His Val Leu Asp
470 475 480

Val Ile His Glu Val Leu His His Gln Arg His Val Arg Thr Ile
485 490 495

Trp Gln His Arg Lys Val Arg Leu His Gln Arg Leu Gln Leu Cys
500 505 510

Val Phe Gln Gln Glu Val Gln Gln Val Leu Asp Trp Ile Glu Asn
515 520 525

His Gly Glu Ala Phe Leu Ser Lys His Thr Gly Val Gly Lys Ser
530 535 540

Leu His Arg Ala Arg Ala Leu Gln Lys Arg His Glu Asp Phe Glu
545 550 555

Glu Val Ala Gln Asn Thr Tyr Thr Asn Ala Asp Lys Leu Leu Glu
560 565 570

Ala Ala Glu Gln Leu Ala Gln Thr Gly Glu Cys Asp Pro Glu Glu
575 580 585

Ile Tyr Gln Ala Ala His Gln Leu Glu Asp Arg Ile Gln Asp Phe
590 595 600

Val Arg Arg Val Glu Gln Arg Lys Ile Leu Leu Asp Met Ser Val
605 610 615

Ser Phe His Thr His Val Lys Glu Leu Trp Thr Trp Leu Glu Glu
620 625 630

Leu Gln Lys Glu Leu Leu Asp Asp Val Tyr Ala Glu Ser Val Glu
635 640 645

Ala Val Gln Asp Leu Ile Lys Arg Phe Gly Gln Gln Gln Gln Thr
650 655 660

Thr Leu Gln Val Thr Val Asn Val Ile Lys Glu Gly Glu Asp Leu
665 670 675

Ile Gln Gln Leu Arg Asp Ser Ala Ile Ser Ser Asn Lys Thr Pro
680 685 690

His Asn Ser Ser Ile Asn His Ile Glu Thr Val Leu Gln Gln Leu
695 700 705

Asp Glu Ala Gln Ser Gln Met Glu Glu Leu Phe Gln Glu Arg Lys
710 715 720

Ile Lys Leu Glu Leu Phe Leu His Val Arg Ile Phe Glu Arg Asp
725 730 735

Ala Ile Asp Ile Ile Ser Asp Leu Glu Ser Trp Asn Asp Glu Leu
740 745 750

Ser Gln Gln Met Asn Asp Phe Asp Thr Glu Asp Leu Thr Ile Ala
755 760 765

Glu Gln Arg Leu Gln His His Ala Asp Lys Ala Leu Thr Met Asn
770 775 780

Asn Leu Thr Phe Asp Val Ile His Gln Gly Gln Asp Leu Leu Gln
785 790 795

Tyr Val Asn Glu Val Gln Ala Ser Gly Val Glu Leu Leu Cys Asp
800 805 810

Arg Asp Val Asp Met Ala Thr Arg Val Gln Asp Leu Leu Glu Phe
815 820 825

Leu His Glu Lys Gln Gln Glu Leu Asp Leu Ala Ala Glu Gln His
830 835 840

Arg Lys His Leu Glu Gln Cys Val Gln Leu Arg His Leu Gln Ala
845 850 855

Glu Val Lys Gln Val Leu Gly Trp Ile Arg Asn Gly Glu Ser Met
860 865 870

Leu Asn Ala Gly Leu Ile Thr Ala Ser Ser Leu Gln Glu Ala Glu
875 880 885

Gln Leu Gln Arg Glu His Glu Gln Phe Gln His Ala Ile Glu Lys
890 895 900

Thr His Gln Ser Ala Leu Gln Val Gln Gln Lys Ala Glu Ala Met
905 910 915

Leu Gln Ala Asn His Tyr Asp Met Asp Met Ile Arg Asp Cys Ala
920 925 930

Glu Lys Val Ala Ser His Trp Gln Gln Leu Met Leu Lys Met Glu
935 940 945

Asp Arg Leu Lys Leu Val Asn Ala Ser Val Ala Phe Tyr Lys Thr
950 955 960

Ser Glu Gln Val Cys Ser Val Leu Glu Ser Leu Glu Gln Glu Tyr
965 970 975

Lys Arg Glu Glu Asp Trp Cys Gly Gly Ala Asp Lys Leu Gly Pro
980 985 990

Asn Ser Glu Thr Asp His Val Thr Pro Met Ile Ser Lys His Leu
995 1000 1005

Glu Gln Lys Glu Ala Phe Leu Lys Ala Cys Thr Leu Ala Arg Arg
1010 1015 1020

Asn Ala Asp Val Phe Leu Lys Tyr Leu His Arg Asn Ser Val Asn
1025 1030 1035

Met Pro Gly Met Val Thr His Ile Lys Ala Pro Glu Gln Gln Val
1040 1045 1050

Lys Asn Ile Leu Asn Glu Leu Phe Gln Arg Glu Asn Arg Val Leu
1055 1060 1065

His Tyr Trp Thr Met Arg Lys Arg Arg Leu Asp Gln Cys Gln Gln
1070 1075 1080

Tyr Val Val Phe Glu Arg Ser Ala Lys Gln Ala Leu Glu Trp Ile
1085 1090 1095

His Asp Asn Gly Glu Phe Tyr Leu Ser Thr His Thr Ser Thr Gly

1100 1105 1110

Ser Ser Ile Gln His Thr Gln Glu Leu Leu Lys Glu His Glu Glu
1115 1120 1125

Phe Gln Ile Thr Ala Lys Gln Thr Lys Glu Arg Val Lys Leu Leu
1130 1135 1140

Ile Gln Leu Ala Asp Gly Phe Cys Glu Lys Gly His Ala His Ala
1145 1150 1155

Ala Glu Ile Lys Lys Cys Val Thr Ala Val Asp Lys Arg Tyr Arg
1160 1165 1170

Asp Phe Ser Leu Arg Met Glu Lys Tyr Arg Thr Ser Leu Glu Lys
1175 1180 1185

Ala Leu Gly Ile Ser Ser Asp Ser Asn Lys Ser Ser Lys Ser Leu
1190 1195 1200

Gln Leu Asp Ile Ile Pro Ala Ser Ile Pro Gly Ser Glu Val Lys
1205 1210 1215

Leu Arg Asp Ala Ala His Glu Leu Asn Glu Glu Lys Arg Lys Ser
1220 1225 1230

Ala Arg Arg Lys Glu Phe Ile Met Ala Glu Leu Ile Gln Thr Glu
1235 1240 1245

Lys Ala Tyr Val Arg Asp Leu Arg Glu Cys Met Asp Thr Tyr Leu
1250 1255 1260

Trp Glu Met Thr Ser Gly Val Glu Glu Ile Pro Pro Gly Ile Val
1265 1270 1275

Asn Lys Glu Leu Ile Ile Phe Gly Asn Met Gln Glu Ile Tyr Glu
1280 1285 1290

Phe His Asn Asn Ile Phe Leu Lys Glu Leu Glu Lys Tyr Glu Gln
1295 1300 1305

Leu Pro Glu Asp Val Gly His Cys Phe Val Thr Trp Ala Asp Lys
1310 1315 1320

Phe Gln Met Tyr Val Thr Tyr Cys Lys Asn Lys Pro Asp Ser Thr
1325 1330 1335

Gln Leu Ile Leu Glu His Ala Gly Ser Tyr Phe Asp Glu Ile Gln
1340 1345 1350

Gln Arg His Gly Leu Ala Asn Ser Ile Ser Ser Tyr Leu Ile Lys
1355 1360 1365

Pro Val Gln Arg Ile Thr Lys Tyr Gln Leu Leu Leu Lys Glu Leu
1370 1375 1380

Leu Thr Cys Cys Glu Glu Gly Lys Gly Glu Ile Lys Asp Gly Leu
1385 1390 1395

Glu Val Met Leu Ser Val Pro Lys Arg Ala Asn Asp Ala Met His
1400 1405 1410

Leu Ser Met Leu Glu Gly Phe Asp Glu Asn Ile Glu Ser Gln Gly
1415 1420 1425

Glu Leu Ile Leu Gln Glu Ser Phe Gln Val Trp Asp Pro Lys Thr
1430 1435 1440

Leu Ile Arg Lys Gly Arg Glu Arg His Leu Phe Leu Phe Glu Met
1445 1450 1455

Ser Leu Val Phe Ser Lys Glu Val Lys Asp Ser Ser Gly Arg Ser
1460 1465 1470

Lys Tyr Leu Tyr Lys Ser Lys Leu Phe Thr Ser Glu Leu Gly Val
1475 1480 1485

Thr Glu His Val Glu Gly Asp Pro Cys Lys Phe Ala Leu Trp Val
1490 1495 1500

Gly Arg Thr Pro Thr Ser Asp Asn Lys Ile Val Leu Lys Ala Ser
1505 1510 1515

Ser Ile Glu Asn Lys Gln Asp Trp Ile Lys His Ile Arg Glu Val
1520 1525 1530

Ile Gln Glu Arg Thr Ile His Leu Lys Gly Ala Leu Lys Glu Pro
1535 1540 1545

Ile His Ile Pro Lys Thr Ala Pro Ala Thr Arg Gln Lys Gly Arg
1550 1555 1560

Arg Asp Gly Glu Asp Leu Asp Ser Gln Gly Asp Gly Ser Ser Gln
1565 1570 1575

Pro Asp Thr Ile Ser Ile Ala Ser Arg Thr Ser Gln Asn Thr Leu
1580 1585 1590

Asp Ser Asp Lys Leu Ser Gly Gly Cys Glu Leu Thr Val Val Ile
1595 1600 1605

His Asp Phe Thr Ala Cys Asn Ser Asn Glu Leu Thr Ile Arg Arg
1610 1615 1620

Gly Gln Thr Val Glu Val Leu Glu Arg Pro His Asp Lys Pro Asp
1625 1630 1635

Trp Cys Leu Val Arg Thr Thr Asp Arg Ser Pro Ala Ala Glu Gly
1640 1645 1650

Leu Val Pro Cys Gly Ser Leu Cys Ile Ala His Ser Arg Ser Ser
1655 1660 1665

Met Glu Met Glu Gly Ile Phe Asn His Lys Asp Ser Leu Ser Val
1670 1675 1680

Ser Ser Asn Asp Ala Ser Pro Pro Ala Ser Val Ala Ser Leu Gln
1685 1690 1695

Pro His Met Ile Gly Ala Gln Ser Ser Pro Gly Pro Lys Arg Pro
1700 1705 1710

```
Gly Asn Thr Leu Arg Lys Trp Leu Thr Ser Pro Val Arg Arg Leu
                1715                1720                1725

Ser Ser Gly Lys Ala Asp Gly His Val Lys Lys Leu Ala His Lys
                1730                1735                1740

His Lys Lys Ser Arg Glu Val Arg Lys Ser Ala Asp Ala Gly Ser
                1745                1750                1755

Gln Lys Asp Ser Asp Asp Ser Ala Ala Thr Pro Gln Asp Glu Thr
                1760                1765                1770

Val Glu Glu Arg Gly Arg Asn Glu Gly Leu Ser Ser Gly Thr Leu
                1775                1780                1785

Ser Lys Ser Ser Ser Ser Gly Met Gln Ser Cys Gly Glu Glu Glu
                1790                1795                1800

Gly Glu Glu Gly Ala Asp Ala Val Pro Leu Pro Pro Pro Met Ala
                1805                1810                1815

Ile Gln Gln His Ser Leu Leu Gln Pro Asp Ser Gln Asp Asp Lys
                1820                1825                1830

Ala Ser Ser Arg Leu Leu Val Arg Pro Thr Ser Ser Glu Thr Pro
                1835                1840                1845

Ser Ala Ala Glu Leu Val Ser Ala Ile Glu Glu Leu Val Lys Ser
                1850                1855                1860

Lys Met Ala Leu Glu Asp Arg Pro Ser Ser Leu Leu Val Asp Gln
                1865                1870                1875

Gly Asp Ser Ser Ser Pro Ser Phe Asn Pro Ser Asp Asn Ser Leu
                1880                1885                1890

Leu Ser Ser Ser Ser Pro Ile Asp Glu Met Glu Glu Arg Lys Ser
                1895                1900                1905

Ser Ser Leu Lys Arg Arg His Tyr Val Leu Gln Glu Leu Val Glu
                1910                1915                1920

Thr Glu Arg Asp Tyr Val Arg Asp Leu Gly Tyr Val Val Glu Gly
                1925                1930                1935

Tyr Met Ala Leu Met Lys Glu Asp Gly Val Pro Asp Asp Met Lys
                1940                1945                1950

Gly Lys Asp Lys Ile Val Phe Gly Asn Ile His Gln Ile Tyr Asp
                1955                1960                1965

Trp His Arg Asp Phe Phe Leu Gly Glu Leu Glu Lys Cys Leu Glu
                1970                1975                1980

Asp Pro Glu Lys Leu Gly Ser Leu Phe Val Lys His Glu Arg Arg
                1985                1990                1995

Leu His Met Tyr Ile Ala Tyr Cys Gln Asn Lys Pro Lys Ser Glu
                2000                2005                2010

His Ile Val Ser Glu Tyr Ile Asp Thr Phe Phe Glu Asp Leu Lys
```

2015 2020 2025

Gln Arg Leu Gly His Arg Leu Gln Leu Thr Asp Leu Leu Ile Lys
2030 2035 2040

Pro Val Gln Arg Ile Met Lys Tyr Gln Leu Leu Leu Lys Asp Phe
2045 2050 2055

Leu Lys Tyr Ser Lys Lys Ala Ser Leu Asp Thr Ser Glu Leu Glu
2060 2065 2070

Arg Ala Val Glu Val Met Cys Ile Val Pro Arg Arg Cys Asn Asp
2075 2080 2085

Met Met Asn Val Gly Arg Leu Gln Gly Phe Asp Gly Lys Ile Val
2090 2095 2100

Ala Gln Gly Lys Leu Leu Leu Gln Asp Thr Phe Leu Val Thr Asp
2105 2110 2115

Gln Asp Ala Gly Leu Leu Pro Arg Cys Arg Glu Arg Arg Ile Phe
2120 2125 2130

Leu Phe Glu Gln Ile Val Ile Phe Ser Glu Pro Leu Asp Lys Lys
2135 2140 2145

Lys Gly Phe Ser Met Pro Gly Phe Leu Phe Lys Asn Ser Ile Lys
2150 2155 2160

Val Ser Cys Leu Cys Leu Glu Glu Asn Val Glu Asn Asp Pro Cys
2165 2170 2175

Lys Phe Ala Leu Thr Ser Arg Thr Gly Asp Val Val Glu Thr Phe
2180 2185 2190

Ile Leu His Ser Ser Ser Pro Ser Val Arg Gln Thr Trp Ile His
2195 2200 2205

Glu Ile Asn Gln Ile Leu Glu Asn Gln Arg Asn Phe Leu Asn Ala
2210 2215 2220

Leu Thr Ser Pro Ile Glu Tyr Gln Arg Asn His Ser Gly Gly Gly
2225 2230 2235

Gly Gly Gly Gly Ser Gly Ala Ala Ala Gly Val Gly Ala Ala Ala
2240 2245 2250

Ala Ala Gly Pro Pro Val Ala Ala Ala Ala Thr Val Ala Ala Pro
2255 2260 2265

Ala Ala Ala Ala Ala Pro Pro Ala Arg Ala Gly Ala Gly Pro Pro
2270 2275 2280

Gly Ser Pro Ser Leu Ser Asp Thr Thr Pro Pro Cys Trp Ser Pro
2285 2290 2295

Leu Gln Pro Arg Ala Arg Gln Arg Gln Thr Arg Cys Gln Ser Glu
2300 2305 2310

Ser Ser Ser Ser Ser Asn Ile Ser Thr Met Leu Val Thr His Asp
2315 2320 2325

Tyr Thr Ala Val Lys Glu Asp Glu Ile Asn Val Tyr Gln Gly Glu
2330 2335 2340

Val Val Gln Ile Leu Ala Ser Asn Gln Gln Asn Met Phe Leu Val
2345 2350 2355

Phe Arg Ala Ala Thr Asp Gln Cys Pro Ala Ala Glu Gly Trp Ile
2360 2365 2370

Pro Gly Phe Val Leu Gly His Thr Ser Ala Val Ile Val Glu Asn
2375 2380 2385

Pro Asp Gly Thr Leu Lys Lys Ser Thr Ser Trp His Thr Ala Leu
2390 2395 2400

Arg Leu Arg Lys Lys Ser Glu Lys Lys Asp Lys Asp Gly Lys Arg
2405 2410 2415

Glu Gly Lys Leu Glu Asn Gly Tyr Arg Lys Ser Arg Glu Gly Leu
2420 2425 2430

Ser Asn Lys Val Ser Val Lys Leu Leu Asn Pro Asn Tyr Ile Tyr
2435 2440 2445

Asp Val Pro Pro Glu Phe Val Ile Pro Leu Ser Glu Val Thr Cys
2450 2455 2460

Glu Thr Gly Glu Thr Val Val Leu Arg Cys Arg Val Cys Gly Arg
2465 2470 2475

Pro Lys Ala Ser Ile Thr Trp Lys Gly Pro Glu His Asn Thr Leu
2480 2485 2490

Asn Asn Asp Gly His Tyr Ser Ile Ser Tyr Ser Asp Leu Gly Glu
2495 2500 2505

Ala Thr Leu Lys Ile Val Gly Val Thr Thr Glu Asp Asp Gly Ile
2510 2515 2520

Tyr Thr Cys Ile Ala Val Asn Asp Met Gly Ser Ala Ser Ser Ser
2525 2530 2535

Ala Ser Leu Arg Val Leu Gly Pro Gly Met Asp Gly Ile Met Val
2540 2545 2550

Thr Trp Lys Asp Asn Phe Asp Ser Phe Tyr Ser Glu Val Ala Glu
2555 2560 2565

Leu Gly Arg Gly Arg Phe Ser Val Val Lys Lys Cys Asp Gln Lys
2570 2575 2580

Gly Thr Lys Arg Ala Val Ala Thr Lys Phe Val Asn Lys Lys Leu
2585 2590 2595

Met Lys Arg Asp Gln Val Thr His Glu Leu Gly Ile Leu Gln Ser
2600 2605 2610

Leu Gln His Pro Leu Leu Val Gly Leu Leu Asp Thr Phe Glu Thr
2615 2620 2625

Pro Thr Ser Tyr Ile Leu Val Leu Glu Met Ala Asp Gln Gly Arg
2630 2635 2640

Leu Leu Asp Cys Val Val Arg Trp Gly Ser Leu Thr Glu Gly Lys
2645 2650 2655

Ile Arg Ala His Leu Gly Glu Val Leu Glu Ala Val Arg Tyr Leu
2660 2665 2670

His Asn Cys Arg Ile Ala His Leu Asp Leu Lys Pro Glu Asn Ile
2675 2680 2685

Leu Val Asp Glu Ser Leu Ala Lys Pro Thr Ile Lys Leu Ala Asp
2690 2695 2700

Phe Gly Asp Ala Val Gln Leu Asn Thr Thr Tyr Tyr Ile His Gln
2705 2710 2715

Leu Leu Gly Asn Pro Glu Phe Ala Ala Pro Glu Ile Ile Leu Gly
2720 2725 2730

Asn Pro Val Ser Leu Thr Ser Asp Thr Trp Ser Val Gly Val Leu
2735 2740 2745

Thr Tyr Val Leu Leu Ser Gly Val Ser Pro Phe Leu Asp Asp Ser
2750 2755 2760

Val Glu Glu Thr Cys Leu Asn Ile Cys Arg Leu Asp Phe Ser Phe
2765 2770 2775

Pro Asp Asp Tyr Phe Lys Gly Val Ser Gln Lys Ala Lys Glu Phe
2780 2785 2790

Val Cys Phe Leu Leu Gln Glu Asp Pro Ala Lys Arg Pro Ser Ala
2795 2800 2805

Ala Leu Ala Leu Gln Glu Gln Trp Leu Gln Ala Gly Asn Gly Arg
2810 2815 2820

Ser Thr Gly Val Leu Asp Thr Ser Arg Leu Thr Ser Phe Ile Glu
2825 2830 2835

Arg Arg Lys His Gln Asn Asp Val Arg Pro Ile Arg Ser Ile Lys
2840 2845 2850

Asn Phe Leu Gln Ser Arg Leu Leu Pro Arg Val
2855 2860

<210> 90
<211> 846
<212> DNA
<213> Homo sapien

<400> 90
ccacgtccgg ggtgccgagc caactttcct gcgtccatgc agccccgccg 50

gcaacggctg cccgctccct ggtccgggcc caggggcccg cgccccaccg 100

ccccgctgct cgcgctgctg ctgttgctcg ccccggtggc ggcgcccgcg 150

gggtccgggg gccccgacga ccctgggcag cctcaggatg ctggggtccc 200

gcgcaggctc ctgcagcaga aggcgcgcgc ggcgcttcac ttcttcaact 250

tccggtccgg ctcgcccagc gcgctgcgag tgctggccga ggtgcaggag 300

ggccgcgcgt ggattaatcc aaaagaggga tgtaaagttc acgtggtctt 350

cagcacagag cgctacaacc cagagtcttt acttcaggaa ggtgagggac 400

gtttggggaa atgttctgct cgagtgtttt tcaagaatca gaaacccaga 450

ccaaccatca atgtaacttg tacacggctc atcgagaaaa agaaaagaca 500

acaagaggat tacctgcttt acaagcaaat gaagcaactg aaaaacccct 550

tggaaatagt cagcatacct gataatcatg gacatattga tccctctctg 600

agactcatct gggatttggc tttccttgga agctcttacg tgatgtggga 650

aatgacaaca caggtgtcac actactactt ggcacagctc actagtgtga 700

ggcagtgggt aagaaaaacc tgaaaattaa cttgtgccac aagagttaca 750

atcaaagtgg tctccttaga ctgaattcat gtgaacttct aatttcatat 800

caagagttgt aatcacattt atttcaataa atatgtgagt tcctgc 846

<210> 91
<211> 1592
<212> DNA
<213> Homo sapien

<400> 91
gaattccatt gtgttggggc cctgggggcg gaggggaggg gcccaccacg 50

gccttatttc cgcgagcgcc ggcactgccc gctccgagcc cgtgtctgtc 100

gggtgccgag ccaactttcc tgcgtccatg cagccccgcc ggcaacggct 150

gcccgctccc tggtccgggc ccaggggccc gcgccccacc gccccgctgc 200

tcgcgctgct gctgttgctc gccccggtgg cggcgcccgc ggggtccggg 250

gaccccgacg accctgggca gcctcaggat gctggggtcc cgcgcaggct 300

cctgcagcag gcggcgcgcg cggcgcttca cttcttcaac ttccggtccg 350

gctcgcccag cgcgctgcga gtgctggccg aggtgcagga gggccgcgcg 400

tggattaatc caaaagaggg atgtaaagtt cacgtggtct tcagcacaga 450

gcgctacaac ccagagtctt tacttcagga aggtgaggga cgtttgggga 500

aatgttctgc tcgagtgttt ttcaagaatc agaaacccag accaactatc 550

aatgtaactt gtacacggct catcgagaaa aagaaaagac aacaagagga 600

ttacctgctt tacaagcaaa tgaagcaact gaaaaacccc ttggaaatag 650

tcagcatacc tgataatcat ggacatattg atccctctct gagactcatc 700

tgggatttgg ctttccttgg aagctcttac gtgatgtggg aaatgacaac 750

acaggtgtca cactactact tggcacagct cactagtgtg aggcagtgga 800

aaactaatga tgatacaatt gattttgatt atactgttct acttcatgaa 850

ttatcaacac aggaaataat tccctgtcgc attcacttgg tctggtaccc 900

tggcaaacct cttaaagtga agtaccactg tcaagagcta cagacaccag 950

aagaagcctc cggaactgaa gaaggatcag ctgtagtacc aacagagctt 1000

agtaatttct aaaaagaaaa aatgatcttt ttccgacttc taaacaagtg 1050

actatactag cataaatcat tcttctagta aaacagctaa ggtatagaca 1100

ttctaataat ttgggaaaac ctatgattac aagtaaaaac tcagaaatgc 1150

aaagatgttg gtttttttgtt tctcagtctg ctttagcttt taactctgga 1200

agcgcatgca cactgaactc tgctcagtgc taaacagtca ccagcaggtt 1250

cctcagggtt tcagccctaa aatgtaaaac ctggataatc agtgtatgtt 1300

gcaccagaat cagcattttt tttttaactg caaaaaatga tggtctcatc 1350

tctgaattta tatttctcat tcttttgaac atactatagc taatatattt 1400

tatgttgcta aattgcttct atctagcatg ttaaacaaag ataatatact 1450

ttcgatgaaa gtaaattata ggaaaaaaat taactgtttt aaaaagaact 1500

tgattatgtt ttatgatttc aggcaagtat tcatttttaa cttgctacct 1550

acttttaaat aaatgtttac atttctaaaa aaaaaaaaaa aa 1592

<210> 92
<211> 228
<212> PRT
<213> Homo sapien

<400> 92
Met Gln Pro Arg Arg Gln Arg Leu Pro Ala Pro Trp Ser Gly Pro
1 5 10 15

Arg Gly Pro Arg Pro Thr Ala Pro Leu Leu Ala Leu Leu Leu Leu
20 25 30

Leu Ala Pro Val Ala Ala Pro Ala Gly Ser Gly Gly Pro Asp Asp
35 40 45

Pro Gly Gln Pro Gln Asp Ala Gly Val Pro Arg Arg Leu Leu Gln
50 55 60

Gln Lys Ala Arg Ala Ala Leu His Phe Phe Asn Phe Arg Ser Gly
65 70 75

Ser Pro Ser Ala Leu Arg Val Leu Ala Glu Val Gln Glu Gly Arg
80 85 90

Ala Trp Ile Asn Pro Lys Glu Gly Cys Lys Val His Val Val Phe

95 100 105

Ser Thr Glu Arg Tyr Asn Pro Glu Ser Leu Leu Gln Glu Gly Glu
110 115 120

Gly Arg Leu Gly Lys Cys Ser Ala Arg Val Phe Phe Lys Asn Gln
125 130 135

Lys Pro Arg Pro Thr Ile Asn Val Thr Cys Thr Arg Leu Ile Glu
140 145 150

Lys Lys Lys Arg Gln Gln Glu Asp Tyr Leu Leu Tyr Lys Gln Met
155 160 165

Lys Gln Leu Lys Asn Pro Leu Glu Ile Val Ser Ile Pro Asp Asn
170 175 180

His Gly His Ile Asp Pro Ser Leu Arg Leu Ile Trp Asp Leu Ala
185 190 195

Phe Leu Gly Ser Ser Tyr Val Met Trp Glu Met Thr Thr Gln Val
200 205 210

Ser His Tyr Tyr Leu Ala Gln Leu Thr Ser Val Arg Gln Trp Val
215 220 225

Arg Lys Thr

<210> 93
<211> 294
<212> PRT
<213> Homo sapien

<400> 93
Met Gln Pro Arg Arg Gln Arg Leu Pro Ala Pro Trp Ser Gly Pro
1 5 10 15

Arg Gly Pro Arg Pro Thr Ala Pro Leu Leu Ala Leu Leu Leu Leu
20 25 30

Leu Ala Pro Val Ala Ala Pro Ala Gly Ser Gly Asp Pro Asp Asp
35 40 45

Pro Gly Gln Pro Gln Asp Ala Gly Val Pro Arg Arg Leu Leu Gln
50 55 60

Gln Ala Ala Arg Ala Ala Leu His Phe Phe Asn Phe Arg Ser Gly
65 70 75

Ser Pro Ser Ala Leu Arg Val Leu Ala Glu Val Gln Glu Gly Arg
80 85 90

Ala Trp Ile Asn Pro Lys Glu Gly Cys Lys Val His Val Val Phe
95 100 105

Ser Thr Glu Arg Tyr Asn Pro Glu Ser Leu Leu Gln Glu Gly Glu
110 115 120

Gly Arg Leu Gly Lys Cys Ser Ala Arg Val Phe Phe Lys Asn Gln
125 130 135

```
Lys Pro Arg Pro Thr Ile Asn Val Thr Cys Thr Arg Leu Ile Glu
                140                 145                 150

Lys Lys Lys Arg Gln Gln Glu Asp Tyr Leu Leu Tyr Lys Gln Met
                155                 160                 165

Lys Gln Leu Lys Asn Pro Leu Glu Ile Val Ser Ile Pro Asp Asn
                170                 175                 180

His Gly His Ile Asp Pro Ser Leu Arg Leu Ile Trp Asp Leu Ala
                185                 190                 195

Phe Leu Gly Ser Ser Tyr Val Met Trp Glu Met Thr Thr Gln Val
                200                 205                 210

Ser His Tyr Tyr Leu Ala Gln Leu Thr Ser Val Arg Gln Trp Lys
                215                 220                 225

Thr Asn Asp Asp Thr Ile Asp Phe Asp Tyr Thr Val Leu Leu His
                230                 235                 240

Glu Leu Ser Thr Gln Glu Ile Ile Pro Cys Arg Ile His Leu Val
                245                 250                 255

Trp Tyr Pro Gly Lys Pro Leu Lys Val Lys Tyr His Cys Gln Glu
                260                 265                 270

Leu Gln Thr Pro Glu Glu Ala Ser Gly Thr Glu Glu Gly Ser Ala
                275                 280                 285

Val Val Pro Thr Glu Leu Ser Asn Phe
                290
```

<210> 94
<211> 3443
<212> DNA
<213> Homo sapien

<400> 94

```
cgcgccgtgc gtccgcgccc ggccgccagg tgccccagta gcccgaccgc 50

cgagatgccc agcccgccgg ggctccgggc gctatggctt tgcgccgcgc 100

tgtgcgcttc ccggagggcc ggcggcgccc cccagcccgg cccggggccc 150

accgcctgcc cggccccctg ccactgccag gaggacggca tcatgctgtc 200

tgccgactgc tctgagctcg ggctgtccgc cgttccgggg gacctggacc 250

ccctgacggc ttacctggac ctcagcatga acaacctcac agagcttcag 300

cctggcctct tccaccacct gcgcttcttg gaggagctgc gtctctctgg 350

gaaccatctc tcacacatcc caggacaagc attctctggt ctctacagcc 400

tgaaaatcct gatgctgcag aacaatcagc tgggaggaat ccccgcagag 450

gcgctgtggg agctgccgag cctgcagtcg ctgcgcctag atgccaacct 500

catctccctg gtcccggaga ggagctttga ggggctgtcc tccctccgcc 550
```

acctctggct ggacgacaat gcactcacgg agatccctgt cagggccctc 600

aacaacctcc ctgccctgca ggccatgacc ctggccctca accgcatcag 650

ccacatcccc gactacgcgt tccagaatct caccagcctt gtggtgctgc 700

atttgcataa caaccgcatc cagcatctgg ggacccacag cttcgagggg 750

ctgcacaatc tggagacact agacctgaat tataacaagc tgcaggagtt 800

ccctgtggcc atccggaccc tgggcagact gcaggaactg gggttccata 850

acaacaacat caaggccatc ccagaaaagg ccttcatggg gaaccctctg 900

ctacagacga tacactttta tgataaccca atccagtttg tgggaagatc 950

ggcattccag tacctgccta aactccacac actatctctg aatggtgcca 1000

tggacatcca ggagtttcca gatctcaaag gcaccaccag cctggagatc 1050

ctgaccctga cccgcgcagg catccggctg ctcccatcgg ggatgtgcca 1100

acagctgccc aggctccgag tcctggaact gtctcacaat caaattgagg 1150

agctgcccag cctgcacagg tgtcagaaat tggaggaaat cggcctccaa 1200

cacaaccgca tctgggaaat tggagctgac accttcagcc agctgagctc 1250

cctgcaagcc ctggatctta gctggaacgc catccggtcc atccaccctg 1300

aggccttctc caccctgcac tccctggtca agctggacct gacagacaac 1350

cagctgacca cactgcccct ggctggactt gggggcttga tgcatctgaa 1400

gctcaaaggg aaccttgctc tctcccaggc cttctccaag gacagtttcc 1450

caaaactgag gatcctggag gtgccttatg cctaccagtg ctgtccctat 1500

gggatgtgtg ccagcttctt caaggcctct gggcagtggg aggctgaaga 1550

ccttcacctt gatgatgagg agtcttcaaa aaggcccctg ggcctccttg 1600

ccagacaagc agagaaccac tatgaccagg acctggatga gctccagctg 1650

gagatggagg actcaaagcc acaccccagt gtccagtgta gccctactcc 1700

aggccccttc aagccctgtg agtacctctt tgaaagctgg ggcatccgcc 1750

tggccgtgtg ggccatcgtg ttgctctccg tgctctgcaa tggactggtg 1800

ctgctgaccg tgttcgctgg cgggcctgcc cccctgcccc cggtcaagtt 1850

tgtggtaggt gcgattgcag gcgccaacac cttgactggc atttcctgtg 1900

gccttctagc ctcagtcgat gccctgacct ttggtcagtt ctctgagtac 1950

ggagcccgct gggagacggg gctaggctgc cgggccactg gcttcctggc 2000

agtacttggg tcggaggcat cggtgctgct gctcactctg gccgcagtgc 2050

agtgcagcgt ctccgtctcc tgtgtccggg cctatgggaa gtccccctcc 2100
ctgggcagcg ttcgagcagg ggtcctaggc tgcctggcac tggcagggct 2150
ggccgccgca ctgcccctgg cctcagtggg agaatacggg gcctccccac 2200
tctgcctgcc ctacgcgcca cctgagggtc agccagcagc cctgggcttc 2250
accgtggccc tggtgatgat gaactccttc tgtttcctgg tcgtggccgg 2300
tgcctacatc aaactgtact gtgacctgcc gcggggcgac tttgaggccg 2350
tgtgggactg cgccatggtg aggcacgtgg cctggctcat cttcgcagac 2400
gggctcctct actgtcccgt ggccttcctc agcttcgcct ccatgctggg 2450
cctcttccct gtcacgcccg aggccgtcaa gtctgtcctg ctggtggtgc 2500
tgcccctgcc tgcctgcctc aacccactgc tgtacctgct cttcaacccc 2550
cacttccggg atgaccttcg gcggcttcgg ccccgcgcag gggactcagg 2600
gcccctagcc tatgctgcgg ccggggagct ggagaagagc tcctgtgatt 2650
ctacccaggc cctggtagcc ttctctgatg tggatctcat tctggaagct 2700
tctgaagctg ggcggccccc tgggctggag acctatggct tcccctcagt 2750
gaccctcatc tcctgtcagc agccaggggc ccccaggctg gagggcagcc 2800
attgtgtaga gccagagggg aaccactttg ggaaccccca accctccatg 2850
gatggagaac tgctgctgag ggcagaggga tctacgccag caggtggagg 2900
cttgtcaggg ggtggcggct ttcagccctc tggcttggcc tttgcttcac 2950
acgtgtaaat atccctcccc attcttctct tcccctctct tccctttcct 3000
ctctccccct cggtgaatga tggctgcttc taaaacaaat acaaccaaaa 3050
ctcagcagtg tgatctatag caggatggcc cagtacctgg ctccactgat 3100
cacctctctc ctgtgaccat caccaacggg tgcctcttgg cctggctttc 3150
ccttggcctt cctcagcttc accttgatac tgggcctctt ccttgtcatg 3200
tctgaagctg tggaccarag acctggactt ttgtctgctt aagggaaatg 3250
agggaagtaa agacagtgaa ggggtggagg gttgatcagg gcacagtgga 3300
cagggagacc tcacaraaaa aggcctggaa ggkgatttcc cgtgtgactc 3350
atggrtagga wacaaaatgt gttccatgta ccattaatct tgacatatgc 3400
catgcataaa racttcctat taaaataagc tttggragag att 3443

<210> 95
<211> 967
<212> PRT
<213> Homo sapien

<400> 95

Met Pro Ser Pro Pro Gly Leu Arg Ala Leu Trp Leu Cys Ala Ala
1               5                   10                  15

Leu Cys Ala Ser Arg Arg Ala Gly Gly Ala Pro Gln Pro Gly Pro
                20                  25                  30

Gly Pro Thr Ala Cys Pro Ala Pro Cys His Cys Gln Glu Asp Gly
                35                  40                  45

Ile Met Leu Ser Ala Asp Cys Ser Glu Leu Gly Leu Ser Ala Val
                50                  55                  60

Pro Gly Asp Leu Asp Pro Leu Thr Ala Tyr Leu Asp Leu Ser Met
                65                  70                  75

Asn Asn Leu Thr Glu Leu Gln Pro Gly Leu Phe His His Leu Arg
                80                  85                  90

Phe Leu Glu Glu Leu Arg Leu Ser Gly Asn His Leu Ser His Ile
                95                  100                 105

Pro Gly Gln Ala Phe Ser Gly Leu Tyr Ser Leu Lys Ile Leu Met
                110                 115                 120

Leu Gln Asn Asn Gln Leu Gly Gly Ile Pro Ala Glu Ala Leu Trp
                125                 130                 135

Glu Leu Pro Ser Leu Gln Ser Leu Arg Leu Asp Ala Asn Leu Ile
                140                 145                 150

Ser Leu Val Pro Glu Arg Ser Phe Glu Gly Leu Ser Ser Leu Arg
                155                 160                 165

His Leu Trp Leu Asp Asp Asn Ala Leu Thr Glu Ile Pro Val Arg
                170                 175                 180

Ala Leu Asn Asn Leu Pro Ala Leu Gln Ala Met Thr Leu Ala Leu
                185                 190                 195

Asn Arg Ile Ser His Ile Pro Asp Tyr Ala Phe Gln Asn Leu Thr
                200                 205                 210

Ser Leu Val Val Leu His Leu His Asn Asn Arg Ile Gln His Leu
                215                 220                 225

Gly Thr His Ser Phe Glu Gly Leu His Asn Leu Glu Thr Leu Asp
                230                 235                 240

Leu Asn Tyr Asn Lys Leu Gln Glu Phe Pro Val Ala Ile Arg Thr
                245                 250                 255

Leu Gly Arg Leu Gln Glu Leu Gly Phe His Asn Asn Asn Ile Lys
                260                 265                 270

Ala Ile Pro Glu Lys Ala Phe Met Gly Asn Pro Leu Leu Gln Thr
                275                 280                 285

Ile His Phe Tyr Asp Asn Pro Ile Gln Phe Val Gly Arg Ser Ala
                290                 295                 300

Phe Gln Tyr Leu Pro Lys Leu His Thr Leu Ser Leu Asn Gly Ala
305 310 315

Met Asp Ile Gln Glu Phe Pro Asp Leu Lys Gly Thr Thr Ser Leu
320 325 330

Glu Ile Leu Thr Leu Thr Arg Ala Gly Ile Arg Leu Leu Pro Ser
335 340 345

Gly Met Cys Gln Gln Leu Pro Arg Leu Arg Val Leu Glu Leu Ser
350 355 360

His Asn Gln Ile Glu Glu Leu Pro Ser Leu His Arg Cys Gln Lys
365 370 375

Leu Glu Glu Ile Gly Leu Gln His Asn Arg Ile Trp Glu Ile Gly
380 385 390

Ala Asp Thr Phe Ser Gln Leu Ser Ser Leu Gln Ala Leu Asp Leu
395 400 405

Ser Trp Asn Ala Ile Arg Ser Ile His Pro Glu Ala Phe Ser Thr
410 415 420

Leu His Ser Leu Val Lys Leu Asp Leu Thr Asp Asn Gln Leu Thr
425 430 435

Thr Leu Pro Leu Ala Gly Leu Gly Gly Leu Met His Leu Lys Leu
440 445 450

Lys Gly Asn Leu Ala Leu Ser Gln Ala Phe Ser Lys Asp Ser Phe
455 460 465

Pro Lys Leu Arg Ile Leu Glu Val Pro Tyr Ala Tyr Gln Cys Cys
470 475 480

Pro Tyr Gly Met Cys Ala Ser Phe Phe Lys Ala Ser Gly Gln Trp
485 490 495

Glu Ala Glu Asp Leu His Leu Asp Asp Glu Glu Ser Ser Lys Arg
500 505 510

Pro Leu Gly Leu Leu Ala Arg Gln Ala Glu Asn His Tyr Asp Gln
515 520 525

Asp Leu Asp Glu Leu Gln Leu Glu Met Glu Asp Ser Lys Pro His
530 535 540

Pro Ser Val Gln Cys Ser Pro Thr Pro Gly Pro Phe Lys Pro Cys
545 550 555

Glu Tyr Leu Phe Glu Ser Trp Gly Ile Arg Leu Ala Val Trp Ala
560 565 570

Ile Val Leu Leu Ser Val Leu Cys Asn Gly Leu Val Leu Leu Thr
575 580 585

Val Phe Ala Gly Gly Pro Ala Pro Leu Pro Pro Val Lys Phe Val
590 595 600

Val Gly Ala Ile Ala Gly Ala Asn Thr Leu Thr Gly Ile Ser Cys

605 610 615

Gly Leu Leu Ala Ser Val Asp Ala Leu Thr Phe Gly Gln Phe Ser
620 625 630

Glu Tyr Gly Ala Arg Trp Glu Thr Gly Leu Gly Cys Arg Ala Thr
635 640 645

Gly Phe Leu Ala Val Leu Gly Ser Glu Ala Ser Val Leu Leu Leu
650 655 660

Thr Leu Ala Ala Val Gln Cys Ser Val Ser Val Ser Cys Val Arg
665 670 675

Ala Tyr Gly Lys Ser Pro Ser Leu Gly Ser Val Arg Ala Gly Val
680 685 690

Leu Gly Cys Leu Ala Leu Ala Gly Leu Ala Ala Ala Leu Pro Leu
695 700 705

Ala Ser Val Gly Glu Tyr Gly Ala Ser Pro Leu Cys Leu Pro Tyr
710 715 720

Ala Pro Pro Glu Gly Gln Pro Ala Ala Leu Gly Phe Thr Val Ala
725 730 735

Leu Val Met Met Asn Ser Phe Cys Phe Leu Val Val Ala Gly Ala
740 745 750

Tyr Ile Lys Leu Tyr Cys Asp Leu Pro Arg Gly Asp Phe Glu Ala
755 760 765

Val Trp Asp Cys Ala Met Val Arg His Val Ala Trp Leu Ile Phe
770 775 780

Ala Asp Gly Leu Leu Tyr Cys Pro Val Ala Phe Leu Ser Phe Ala
785 790 795

Ser Met Leu Gly Leu Phe Pro Val Thr Pro Glu Ala Val Lys Ser
800 805 810

Val Leu Leu Val Val Leu Pro Leu Pro Ala Cys Leu Asn Pro Leu
815 820 825

Leu Tyr Leu Leu Phe Asn Pro His Phe Arg Asp Asp Leu Arg Arg
830 835 840

Leu Arg Pro Arg Ala Gly Asp Ser Gly Pro Leu Ala Tyr Ala Ala
845 850 855

Ala Gly Glu Leu Glu Lys Ser Ser Cys Asp Ser Thr Gln Ala Leu
860 865 870

Val Ala Phe Ser Asp Val Asp Leu Ile Leu Glu Ala Ser Glu Ala
875 880 885

Gly Arg Pro Pro Gly Leu Glu Thr Tyr Gly Phe Pro Ser Val Thr
890 895 900

Leu Ile Ser Cys Gln Gln Pro Gly Ala Pro Arg Leu Glu Gly Ser
905 910 915

```
His Cys Val Glu Pro Glu Gly Asn His Phe Gly Asn Pro Gln Pro
                920                 925                 930

Ser Met Asp Gly Glu Leu Leu Leu Arg Ala Glu Gly Ser Thr Pro
                935                 940                 945

Ala Gly Gly Gly Leu Ser Gly Gly Gly Gly Phe Gln Pro Ser Gly
                950                 955                 960

Leu Ala Phe Ala Ser His Val
                965
```